# EUROPEAN PATENT APPLICATION

(11) **EP 4 471 023 A1**
(43) Date of publication of application: **04.12.2024**
(21) Application number: 23746280.9
(22) Date of filing: 19.01.2023
(51) Int. Cl.: C07D 401/12, C07D 493/04, A61K 31/513, A61K 31/662, A61P 9/00, A61P 13/00

(54) **COMPOUND SERVING AS MASP-2 INHIBITOR, PHARMACEUTICAL COMPOSITION, PREPARATION METHOD THEREFOR, AND USE THEREOF**

(30) Priority: 28.01.2022 CN 202210116158
(71) Applicant: Wuhan Createrna Science and Technology Co.,Ltd., Wuhan, Hubei 430075 (CN)
(72) Inventor: LOU, Jun, Wuhan, Hubei 430075 (CN); WU, Jingkang, Wuhan, Hubei 430075 (CN); ZHOU, Feng, Wuhan, Hubei 430075 (CN); LIU, Li, Wuhan, Hubei 430075 (CN); DONG, Xiaolong, Wuhan, Hubei 430075 (CN); CHEN, Ying, Wuhan, Hubei 430075 (CN); ZHANG, Liqian, Wuhan, Hubei 430075 (CN); ZHANG, Yihan, Wuhan, Hubei 430075 (CN); CHEN, Yongkai, Wuhan, Hubei 430075 (CN); WANG, Chaodong, Wuhan, Hubei 430075 (CN)
(74) Representative: Gille Hrabal Partnerschaftsgesellschaft mbB Patentanwälte
(86) International application number: PCT/CN2023/073238
(87) International publication number: WO 2023/143401

(57) **Abstract**

The present invention relates to a compound having inhibitory activity for the lectin pathway of complement, a preparation method therefor, and an application thereof. Specifically, the present invention relates to a compound as represented by general formula (I'), a pharmaceutically acceptable salt thereof, a preparation method therefor, and an application thereof as a MASP-2 inhibitor, particularly an application in the preparation of a drug for treating diseases such as IgA nephropathy, TA-TMA, aHUS, and lupus nephritis.

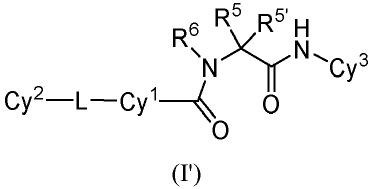

## Description

The present application claims priority to Chinese Patent Application No. 202210116158.X, filed to China National Intellectual Property Administration on Jan. 28, 2022 and entitled "COMPOUND SERVING AS MASP-2 INHIBITOR, PHARMACEUTICAL COMPOSITION, PREPARATION METHOD THEREFOR, AND USE THEREOF" which is incorporated herein by reference in its entirety.

### TECHNICAL FIELD

The present disclosure belongs to the field of medicines, and particularly relates to a compound serving as a MASP-2 inhibitor, a pharmaceutical composition, a preparation method therefor, and use thereof.

### BACKGROUND

In addition to Ig molecules, another class of large molecules involved in immune effects, known as complement molecules, have been found in blood or body fluids. Their chemical components are a group of immunoglobulins with enzymatic activity, accounting for about 10% of the total amount of serum globulins. Complement molecules are extremely unstable. Most complement molecules are heat-sensitive and lose activity within 30 min at 56 °C. Early immunological studies found that this substance is essential in immune hemolytic reactions and bacteriolytic reactions and is therefore designated as the complement. Studies in recent years have shown that the complements are each composed of 11 serum proteins and are designated as C1 to C9 according to the sequence that is based on the time when they are discovered. C1 consists of three subunits, Clq, Clr, and Cls, collectively referred to as the complement system.

The complement system can be activated through three independent yet correlated pathways, i.e., the classical pathway, the alternative pathway, and the lectin pathway. The three activation pathways of the complement system include: the classical pathway: C1q, C1r, C1s, C4, C2, C3, C5, and C6-9; the mannose-binding lectin pathway (MBL pathway): MBL, MASP1-2, C4, C2, C3, C5, and C6-9; and the alternative pathway: C3, factor B, factor D, factor P (properdin), C5, and C6-9.

The lectin pathway activation is initiated by the binding of mannose-binding lectin (MBL), collectin 11 (CL-K1), and ficolins (Ficolin-1, Ficolin-2, and Ficolin-3) in pathogen-associated molecular patterns (PAMPs) (D-mannose, N-acetyl-D-glucosamine, or acetyl). Upon binding to target molecules, MBL, CL-K1, and ficolins form complexes with MBL-associated serine proteases 1 and 2 (MASP-1 and MASP-2) and cleave C4 and C2 to form C3 convertase (C4b2a). Subsequently, the complement cascade is activated, leading to opsonization, phagocytosis, and lysis of target microorganisms by forming membrane attack complexes.

Mannose-binding lectin-associated serine protease 2 (MASP-2) is the effector enzyme of the lectin pathway of the complement system. At present, research projects targeting the lectin pathway of the complement that have witnessed faster progress are all related to antibodies. For example, MASP-2-targeting monoclonal antibody narsoplimab (OMS-721) has been granted breakthrough therapy designation for treating patients with high-risk HSCT-TMA and orphan drug designation for preventing complement-mediated thrombotic microangiopathy (TMA) and treating HSCT-TMA in the United States. In the European Union, it has been granted orphan drug designation for treating hematopoietic stem cell transplantation (HSCT). Currently, narsoplimab is in phase III clinical trials for the treatment of IgA nephropathy (IgAN) and atypical hemolytic uremic syndrome (aHUS). Previously, this drug had also been granted orphan drug designation and breakthrough therapy designation for treating IgAN and fast track designation for treating aHUS in the United States, and orphan drug designation for treating IgAN in the European Union.

MASP-2-dependent complement activation has been considered to contribute to the pathogenesis of many acute and chronic disease states. Therefore, there is a need currently for small molecule compounds suitable for use in the treatment of diseases and conditions associated with the MASP-2 complement pathway.

### SUMMARY OF THE DISLOSURE

In order to alleviate the technical problems described above, the present disclosure provides a compound represented by formula (I'), a racemate, a stereoisomer, a tautomer, a nitrogen oxide, or a pharmaceutically acceptable salt thereof: 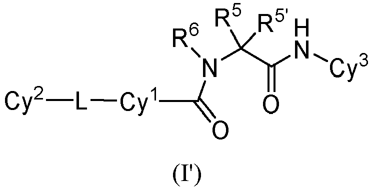 wherein:
Cy³ is selected from 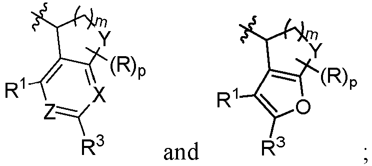
Z is selected from CR² and N;
R¹, R², and R³ are each independently selected from H, halogen, -NH₂, -OH, -NO₂, -CN, C₁₋₆ alkyl, C₁₋₆ aminoalkyl, and C₁₋₆ alkoxy;
X is selected from CR⁴ and N; R⁴ is selected from H, halogen, C₁₋₆ alkyl, and C₁₋₆ alkoxy;
Y is selected from -CH₂-, -C(C₁₋₃ alkyl)₂-, -NH-, -O-, and -S-; m is selected from 0, 1, 2, and 3;
each R is identical or different and is each independently selected from H, halogen, C₁₋₆ alkyl, and C₁₋₆ alkoxy;
p is selected from 0, 1, 2, 3, and 4;
R⁵ is selected from H, C₁₋₆ alkyl, C₂₋₆ alkynyl, C₁₋₆ haloalkyl, and C₃₋₆ cycloalkyl;
R^{5'} is selected from H or C₁₋₆ alkyl; or
R⁵ and R^{5'}, together with the carbon atom attached thereto, form C₃₋₆ cycloalkyl;
R⁶ is selected from H and C₁₋₆ alkyl; or
R⁵ and R⁶, together with the C and N atoms attached thereto, form 3- to 12-membered heterocycloalkyl, wherein the 3- to 12-membered heterocycloalkyl being unsubstituted or optionally substituted with 1, 2, or 3 R^{5a};
each R^{5a} is identical or different and is each independently selected from halogen, C₁₋₆ alkyl, -OH, - NO₂, -CN, oxo (=O), C₂₋₆ alkenyl, C₂₋₆ alkynyl, C₁₋₆ haloalkyl, C₁₋₆ alkoxy, and C₁₋₆ haloalkoxy; or
R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2, or 3 halogens;
Cy¹ is selected from the following groups unsubstituted or optionally substituted with 1, 2, 3, and 4 R⁷: 5- to 8-membered heterocyclyl and 5- to 6-membered heteroaryl;
each R⁷ is identical or different and is each independently selected from H, oxo (=O), halogen, and C₁₋₆ alkyl;
L is selected from -(CR^{a}R^{b})_{q}- and -(CR^{a}R^{b})_{q}-O-; q is selected from 0, 1, and 2, each R^{a} and each R^{b} are identical or different and are each independently selected from H, halogen, and C₁₋₆ alkyl, or R^{a} and R^{b}, together with the carbon atom attached thereto, form C₃₋₆ cycloalkyl;
Cy² is selected from the following groups unsubstituted or optionally substituted with 1, 2, 3, or 4 R⁸: C₆₋₁₀ aryl, 5- to 10-membered heteroaryl, C₃₋₁₀ cycloalkyl, and 5- to 12-membered heterocyclyl;
each R⁸ is identical or different and is each independently selected from -CN, halogen, C₁₋₆ alkyl, C₁₋₆ alkoxy, C₁₋₆ haloalkyl, C₁₋₆ haloalkoxy, C₃₋₁₀ cycloalkyl, C₆₋₁₀ aryl, and 5- to 10-membered heteroaryl. According to an embodiment of the present disclosure, the compound represented by formula (I') has a structure represented by the following formula (I): 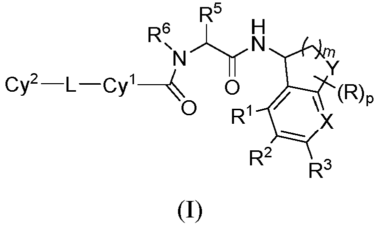 wherein:
   R¹, R², and R³ are each independently selected from H, halogen, -NH₂, -OH, -NO₂, -CN, C₁₋₆ alkyl, C₁₋₆ aminoalkyl, and C₁₋₆ alkoxy;
   X is selected from CR⁴ and N; R⁴ is selected from H, halogen, C₁₋₆ alkyl, and C₁₋₆ alkoxy;
   Y is selected from -CH₂-, -C(C₁₋₃ alkyl)₂-, -NH-, -O-, and -S-; m is selected from 0, 1, 2, and 3;
   each R is identical or different and is each independently selected from H, halogen, C₁₋₆ alkyl, and C₁₋₆ alkoxy;
   p is selected from 0, 1, 2, 3, and 4;
   R⁵ is selected from H, C₁₋₆ alkyl, C₂₋₆ alkynyl, C₁₋₆ haloalkyl, and C₃₋₆ cycloalkyl; R⁶ is selected from H and C₁₋₆ alkyl, or R⁵ and R⁶, together with the C and N atoms attached thereto, form 3- to 12-membered heterocycloalkyl, wherein the 3- to 12- membered heterocycloalkyl being unsubstituted or optionally substituted with 1, 2, or 3 R^{5a};
   each R^{5a} is identical or different and is each independently selected from halogen, C₁₋₆ alkyl, -OH, - NO₂, -CN, oxo (=O), C₂₋₆ alkenyl, C₂₋₆ alkynyl, C₁₋₆ haloalkyl, C₁₋₆ alkoxy, and C₁₋₆ haloalkoxy; or
   R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2, or 3 halogens;
   Cy¹ is selected from the following groups unsubstituted or optionally substituted with 1, 2, 3, or 4 R⁷: 5- to 8-membered heterocyclyl and 5- to 6-membered heteroaryl;
   each R⁷ is identical or different and is each independently selected from H, oxo (=O), halogen, and C₁₋₆ alkyl;
   L is selected from -(CR^{a}R^{b})_{q}- and -(CR^{a}R^{b})_{q}-O-; q is selected from 0, 1, and 2, each R^{a} and each R^{b} are identical or different and are each independently selected from H, halogen, and C₁₋₆ alkyl, or R^{a} and R^{b}, together with the carbon atom attached thereto, form C₃₋₆ cycloalkyl;
   Cy² is selected from the following groups unsubstituted or optionally substituted with 1, 2, 3, or 4 R⁸: C₆₋₁₀ aryl, 5- to 10-membered heteroaryl, C₃₋₁₀ cycloalkyl, and 5- to 12-membered heterocyclyl;
   each R⁸ is identical or different and is each independently selected from -CN, halogen, C₁₋₆ alkyl, C₁₋₆ alkoxy, C₁₋₆ haloalkyl, C₁₋₆ haloalkoxy, C₃₋₁₀ cycloalkyl, C₆₋₁₀ aryl, and 5- to 10-membered heteroaryl.

According to an embodiment of the present disclosure, the compound represented by formula (I') has a structure represented by the following formula (I): 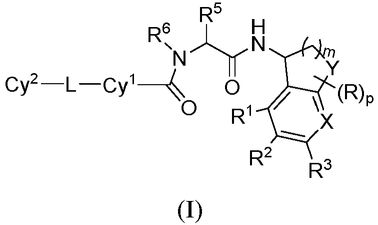 wherein:
R¹, R², and R³ are each independently selected from H, halogen, -NH₂, -OH, -NO₂, -CN, C₁₋₆ alkyl, and C₁₋₆ alkoxy;
X is selected from CR⁴ or N; R⁴ is selected from H, halogen, C₁₋₆ alkyl, and C₁₋₆ alkoxy;
Y is selected from -CH₂-, -NH-, -O-, and -S-; m is selected from 0, 1, 2, and 3;
each R is identical or different and is each independently selected from H, halogen, C₁₋₆ alkyl, and C₁₋₆ alkoxy; p is selected from 0, 1, 2, 3, and 4;
R⁵ and R⁶ are each independently selected from H and C₁₋₆ alkyl, or R⁵ and R⁶, together with the C and N atoms attached thereto, form 3- to 12-membered heterocycloalkyl;
Cy¹ is selected from the following groups unsubstituted or optionally substituted with 1, 2, 3, or 4 R⁷: 5- to 6-membered heterocyclyl, and 5- to 6-membered heteroaryl;
each R⁷ is identical or different and is each independently selected from H, halogen, and C₁₋₆ alkyl;
L represents -(CR^{a}R^{b})ₚ-; p is selected from 0, 1, and 2, each R^{a} and each R^{b} are identical or different and are each independently selected from H, halogen, and C₁₋₆ alkyl, or R^{a} and R^{b}, together with the carbon atom attached thereto, form C₃₋₆ cycloalkyl;
Cy² is selected from the following groups unsubstituted or optionally substituted with 1, 2, 3, or 4 R⁸: C₆₋₁₀ aryl, 5- to 10-membered heteroaryl, C₃₋₁₀ cycloalkyl, and 5- to 12-membered heterocyclyl;
each R⁸ is identical or different and is each independently selected from CN, halogen, C₁₋₆ alkyl, C₁₋₆ alkoxy, C₁₋₆ haloalkyl, C₁₋₆ haloalkoxy, C₃₋₁₀ cycloalkyl, C₆₋₁₀ aryl, and 5- to 10-membered heteroaryl.

According to an embodiment of the present disclosure, when Cy¹, through a chiral carbon atom on its ring, is linked to -C(=O)NR⁶-, the compound represented by formula (I') has a structure represented by the following formula (II): 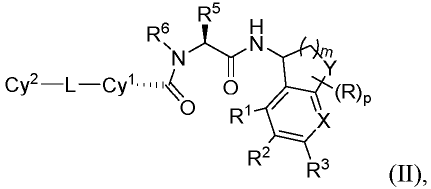 wherein, R⁵ is selected from C₁₋₆ alkyl, C₂₋₆ alkynyl, C₁₋₆ haloalkyl, and C₃₋₆ cycloalkyl; m, p, X, Y, R, R¹, R², R³, R⁶, L, Cy¹, and Cy² are as defined herein.

According to an embodiment of the present disclosure, when Cy¹, through a chiral carbon atom on its ring, is linked to -C(=O)NR⁶-, the compound represented by formula (I) has a structure represented by the following formula (II): 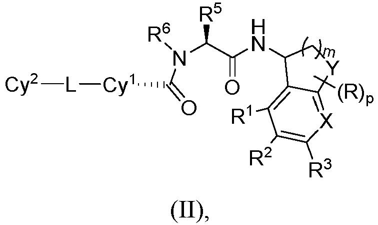 wherein, R⁵ is C₁₋₆ alkyl; m, p, X, Y, R, R¹, R², R³, R⁶, L, Cy¹, and Cy² are as defined herein.

According to an embodiment of the present disclosure, the compound represented by formula (I') has a structure represented by any one of the following structural formulas (I-a)-(I-j'): 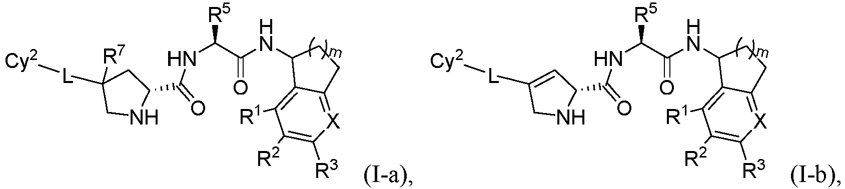 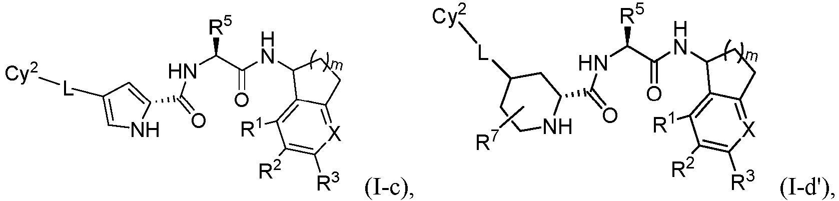 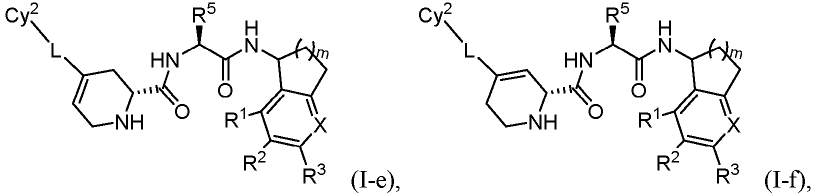 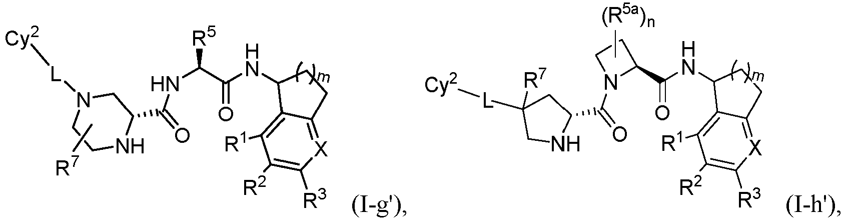 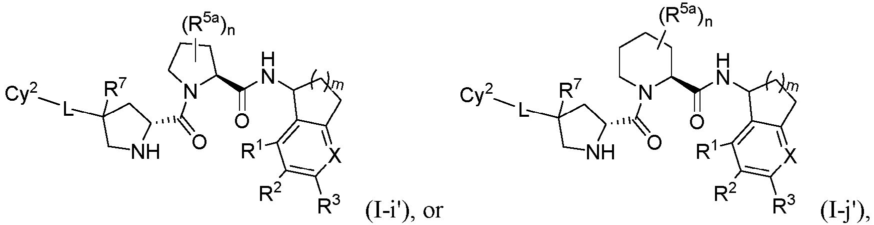 wherein, R⁵ is selected from C₁₋₃ alkyl, C₂₋₃ alkynyl, C₁₋₃ haloalkyl, and C₃₋₄ cycloalkyl; R^{5a} is each independently selected from halogen, C₁₋₃ alkyl, -OH, -NO₂, -CN, oxo (=O), C₁₋₃ haloalkyl, C₁₋₃ alkoxy, and C₁₋₃ haloalkoxy; or R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2, or 3 halogens; n is 0, 1, 2, or 3; m, X, R¹, R², R³, R⁷, L, and Cy² are as defined herein.

According to an embodiment of the present disclosure, the compound represented by formula (I) has a structure represented by any one of the following structural formulas (I-a)-(I-j): 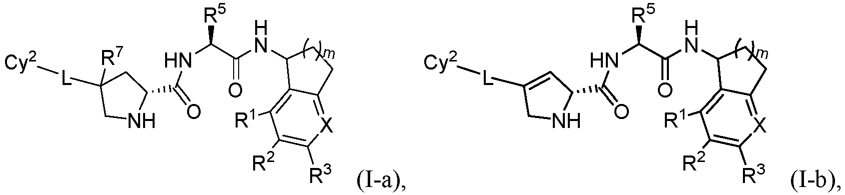 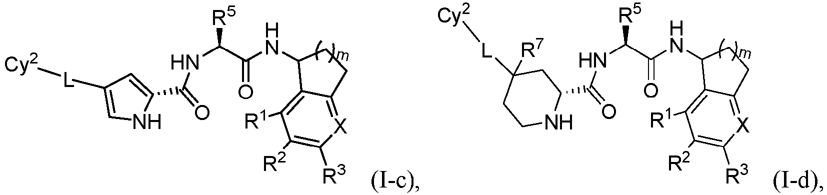 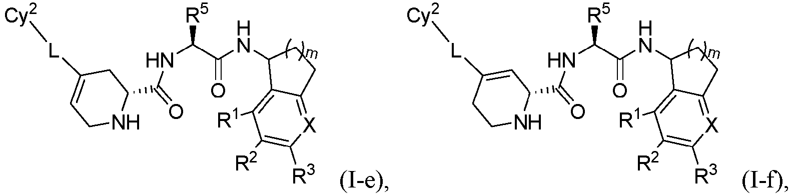 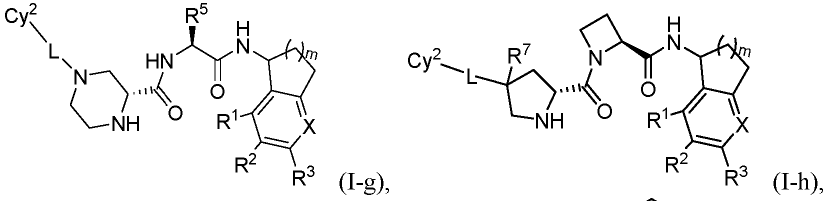 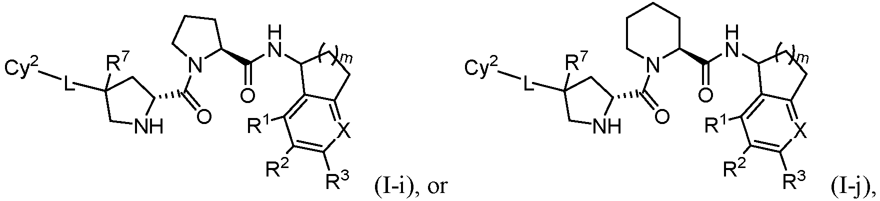 wherein, R⁵ is C₁₋₃ alkyl; m, X, R¹, R², R³, R⁵, R⁷, L, and Cy² are as defined herein.

According to an embodiment of the present disclosure, the compound represented by formula (I') has a structure represented by any one of the following structural formulas (I-l)-(I-u'): 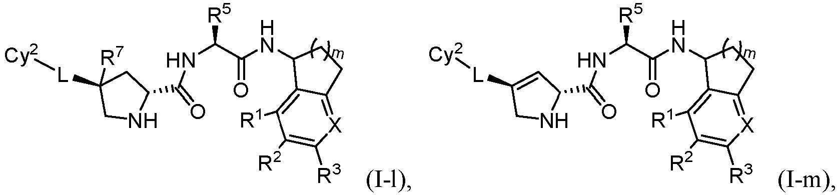 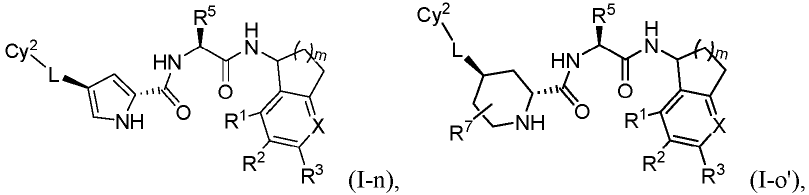 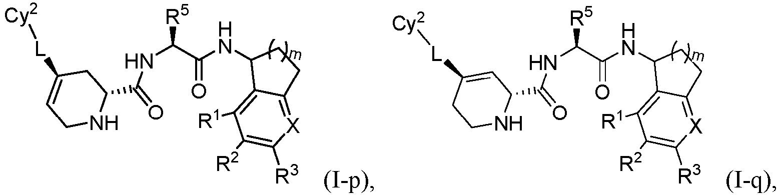 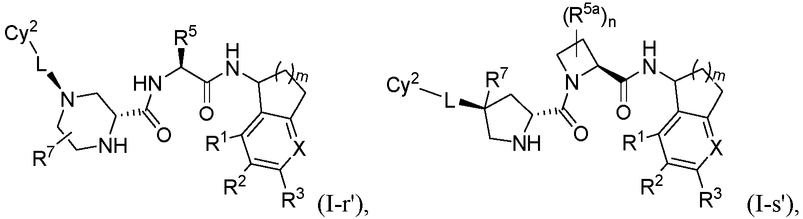 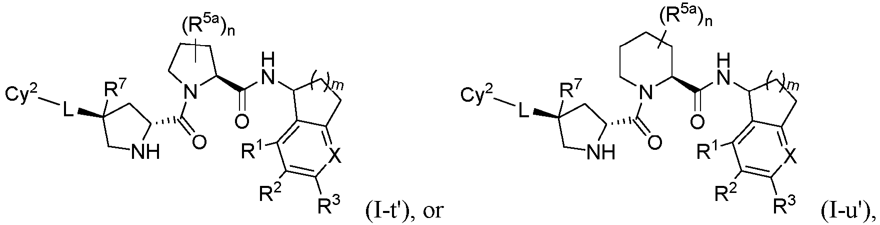 wherein, R⁵ is selected from C₁₋₃ alkyl, C₂₋₃ alkynyl, C₁₋₃ haloalkyl, and C₃₋₄ cycloalkyl; R^{5a} is each independently selected from halogen, C₁₋₃ alkyl, -OH, -NO₂, -CN, oxo (=O), C₁₋₃ haloalkyl, C₁₋₃ alkoxy, and C₁₋₃ haloalkoxy; or R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2, or 3 halogens; n is 0, 1, 2, or 3; m, X, R¹, R², R³, R⁷, L, and Cy² are as defined herein.

According to an embodiment of the present disclosure, the compound represented by formula (I) has a structure represented by any one of the following structural formulas (I-l)-(I-u): 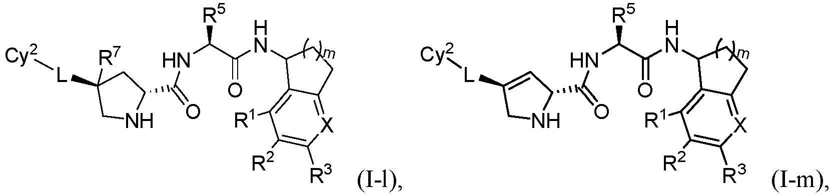 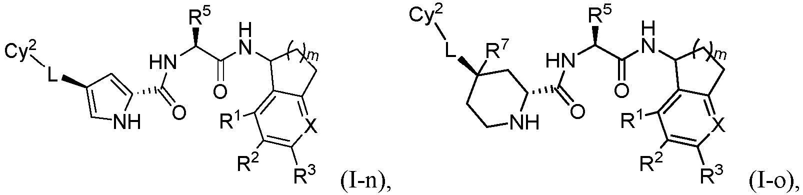 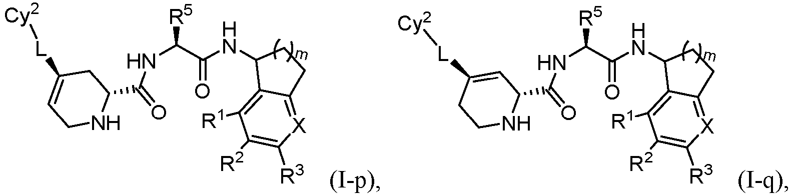 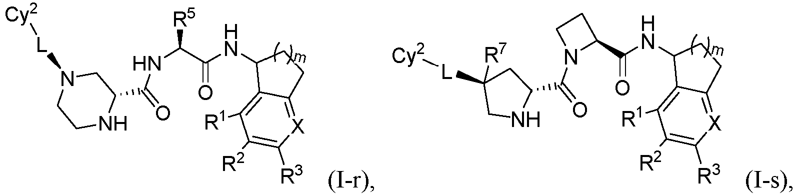 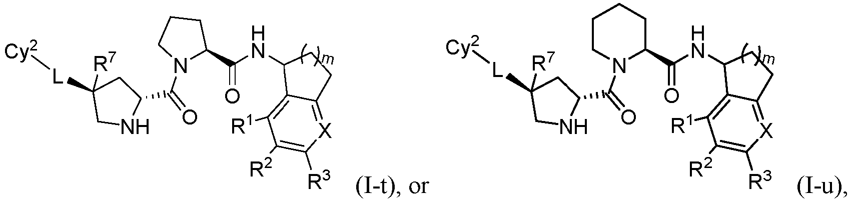 wherein, R⁵ is C₁₋₃ alkyl; m, X, R¹, R², R³, R⁵, R⁷, L, and Cy² are as defined herein.

According to an embodiment of the present disclosure, the compound represented by formula (I') has a structure represented by any one of the following structural formulas (I-v)-(I-z'): 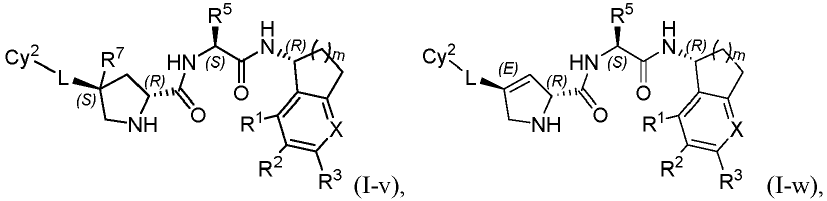 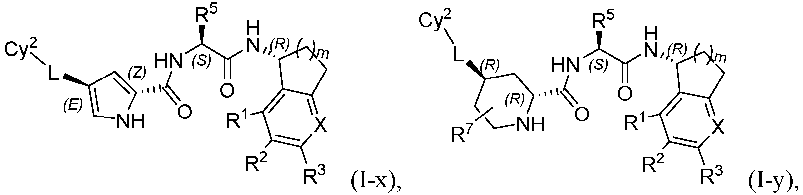 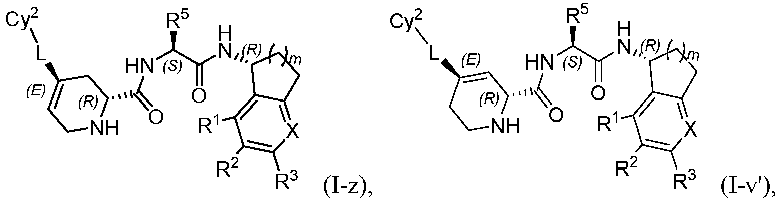 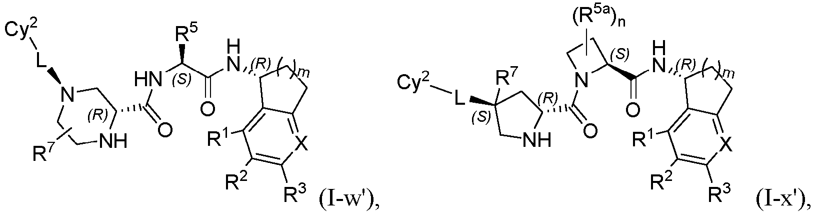 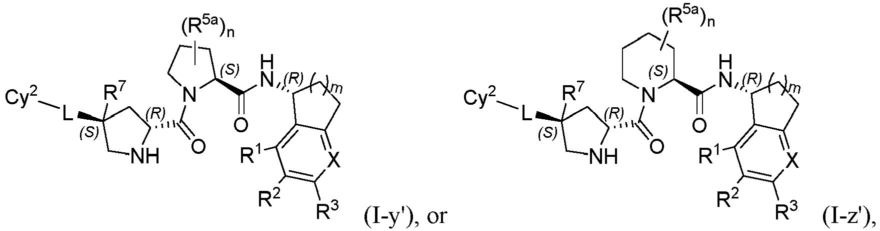 wherein, R⁵ is selected from methyl, ethyl, ethynyl, propynyl, -CH₂F, -CHF₂, -CF₃, and cyclopropyl; R^{5a} is each independently selected from halogen, methyl, ethyl, -CH₂F, -CHF₂, -CF₃, and methoxy; or R^{5a} on the same carbon atom or on two adjacent carbon atoms, together with the carbon atom attached thereto, form cyclopropyl or cyclobutyl, wherein the cyclopropyl and cyclobutyl being unsubstituted or optionally substituted with 1, 2, or 3 halogens; n is 0, 1, 2, or 3; m, X, R¹, R², R³, R⁷, L, and Cy² are as defined herein.

According to an embodiment of the present disclosure, Cy³ is selected from the following structural formulas:

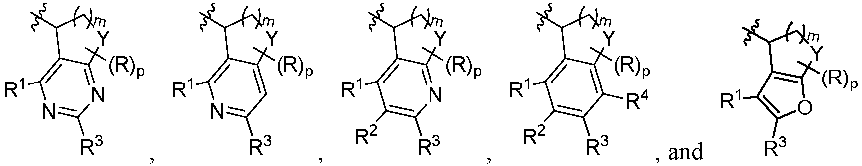

According to an embodiment of the present disclosure, Cy³ is selected from the following structural formulas:

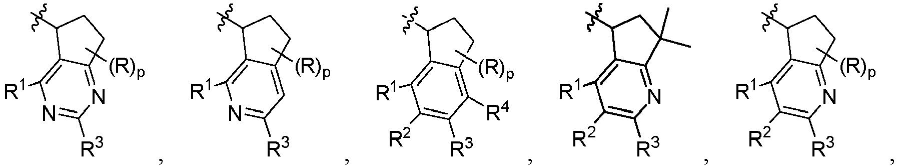

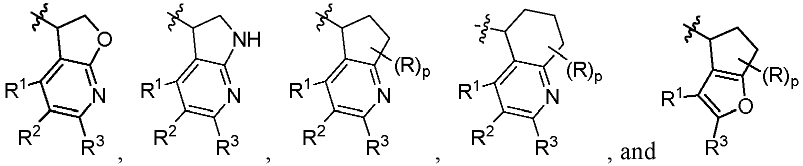

According to an embodiment of the present disclosure, Cy³ is selected from the following structural formulas:

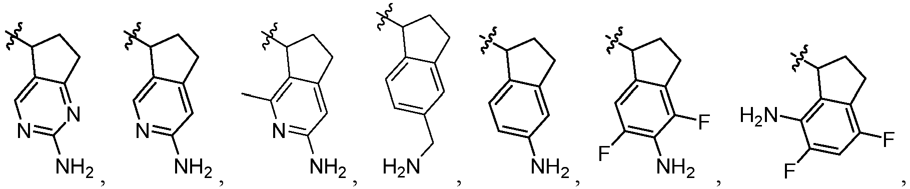

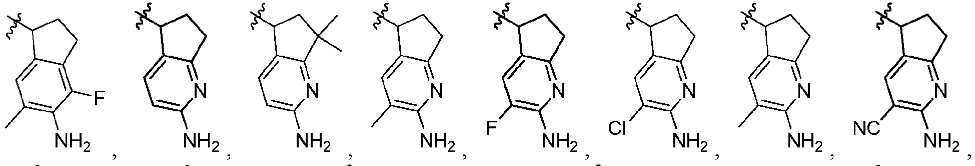

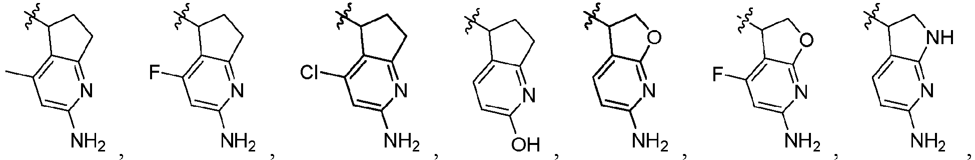

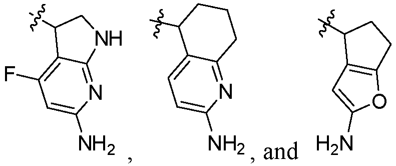

According to an embodiment of the present disclosure, R⁵ is selected from H, C₁₋₃ alkyl, C₂₋₃ alkynyl, C₁₋₃ haloalkyl, and C₃₋₄ cycloalkyl;
R^{5'} is selected from H and C₁₋₃ alkyl; or R⁵ and R^{5'}, together with the carbon atom attached thereto, form C₃₋₄ cycloalkyl.

According to an embodiment of the present disclosure, R⁵ is selected from H, methyl, ethyl, ethynyl, propynyl, -CH₂F, -CHF₂, -CF₃, and cyclopropyl; R^{5'} is selected from H, methyl, and ethyl; or R⁵ and R^{5'}, together with the carbon atom attached thereto, form cyclopropyl.

According to an embodiment of the present disclosure, R¹, R², and R³ are each independently selected from H, F, Cl, Br, -NH₂, -OH, -CN, methyl, ethyl, -CH₂NH₂, and methoxy.

According to an embodiment of the present disclosure, R¹ is selected from H, F, Cl, Br, -NH₂, and methyl.

According to an embodiment of the present disclosure, R² is selected from H, F, Cl, Br, -CN, methyl, ethyl, and methoxy.

According to an embodiment of the present disclosure, R³ is selected from H, F, -NH₂, -OH, -CH₂NH₂, and methoxy.

According to an embodiment of the present disclosure, X is selected from CR⁴ and N; R⁴ is selected from H, halogen, methyl, and ethyl.

According to an embodiment of the present disclosure, Y is selected from -CH₂-, -C(CH₃)₂-, -NH-, and -O-; m is selected from 0, 1, and 2.

According to an embodiment of the present disclosure, each R is identical or different and is independently selected from H, halogen, C₁₋₃ alkyl, and C₁₋₃ alkoxy; p is selected from 0, 1, and 2. According to an embodiment of the present disclosure, R⁵ is selected from H, methyl, and ethyl. According to an embodiment of the present disclosure, R⁶ is selected from H, methyl, and ethyl.

According to an embodiment of the present disclosure, R⁵ and R⁶, together with the C and N atoms attached thereto, form 4-, 5-, or 6-membered heterocycloalkyl, wherein the 4-, 5-, and 6-membered heterocycloalkyl being unsubstituted or optionally substituted with 1, 2, or 3 R^{5a}; R^{5a} is each independently selected from F, Cl, Br, methyl, and ethyl; or R^{5a} on the same carbon atom or on two adjacent carbon atoms, together with the carbon atom attached thereto, form cyclopropyl or cyclobutyl, wherein the cyclopropyl and cyclobutyl being unsubstituted or optionally substituted with 1, 2, or 3 halogens.

According to an embodiment of the present disclosure, R⁵ and R⁶, together with the C and N atoms attached thereto, form 4-, 5-, or 6-membered heterocycloalkyl.

According to an embodiment of the present disclosure, R⁵ and R⁶, together with the C and N atoms attached thereto, form the following structures: 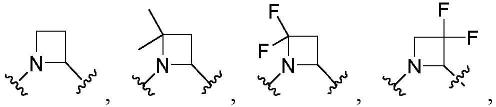 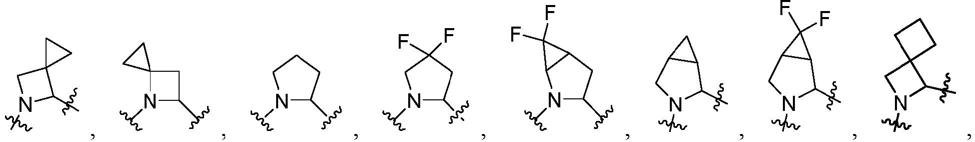 or 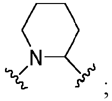 the structures formed are preferably selected from:

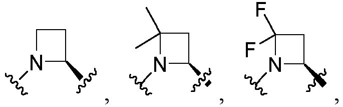

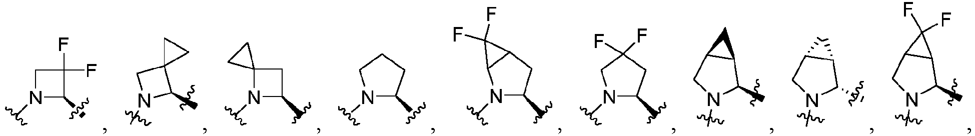

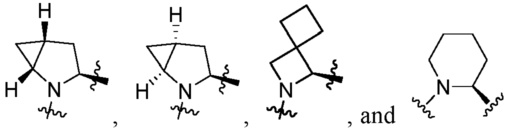

According to an embodiment of the present disclosure, L is absent or selected from -C(R^{a})(R^{b})-, -O-, and -C(R^{a})(R^{b})-O-; R^{a} and R^{b} are each independently H, halogen, methyl, or ethyl, or R^{a} and R^{b}, together with the carbon atom attached thereto, form cyclopropyl or cyclobutyl.

According to an embodiment of the present disclosure, L is absent or selected from -CH₂-, -CH(CH₃)-, -CF₂-, -O-, -CH₂-O-, -CH(CH₃)-O-, and

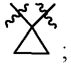

According to an embodiment of the present disclosure, Cy¹ is selected from the following groups unsubstituted or substituted with 1 or 2 R⁷: tetrahydropyrrolyl, 2,5-dihydro-1H-pyrrolyl, pyrrolyl, piperidyl, 1,2,3,6-tetrahydropyridinyl, 1,2,5,6-tetrahydropyridinyl, piperazinyl, 4-azaspiro[2.5]octyl, 1,2-dihydropyridinyl, and 1,4-dihydropyrazinyl; each R⁷ is identical or different and is each independently selected from H, oxo (=O), F, Cl, or -CH₃.

According to an embodiment of the present disclosure, Cy¹ is selected from the following structures: 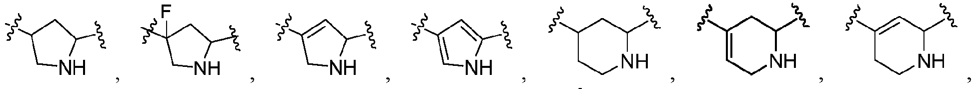 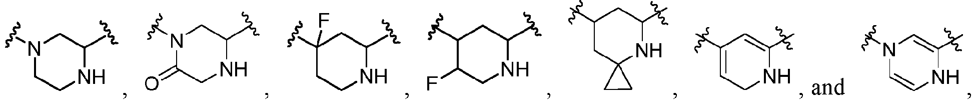 preferably selected from: 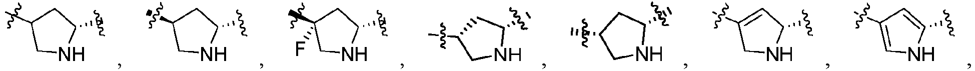 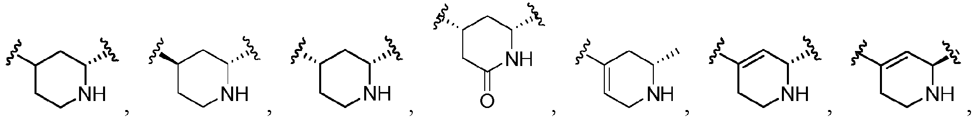 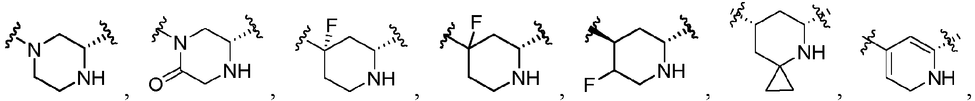 and

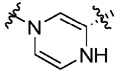

According to an embodiment of the present disclosure, Cy² is selected from the following groups unsubstituted or optionally substituted with 1, 2, 3, or 4 R⁸: C₆₋₁₀ aryl, 5- to 9-membered heteroaryl, and 6- to 11-membered heterocyclyl; each R⁸ is identical or different and is each independently selected from -CN, F, Br, Cl, -CH₂F, -CHF₂, -CF₃, methyl, ethyl, n-propyl, isopropyl, n-butyl, tert-butyl, methoxy, ethoxy, phenyl, pyridinyl, and trifluoromethoxy.

According to an embodiment of the present disclosure, Cy² is selected from the following groups unsubstituted or optionally substituted with 1, 2, 3, or 4 R⁸: C₆₋₁₀ aryl, 6- to 9-membered heteroaryl, and 6- to 11-membered heterocyclyl; each R⁸ is identical or different and is each independently selected from F, Br, Cl, CH₂F, CHF₂, CF₃, methyl, ethyl, n-propyl, isopropyl, n-butyl, tert-butyl, methoxy, ethoxy, phenyl, pyridinyl, and trifluoromethoxy.

According to an embodiment of the present disclosure, Cy² is selected from the following groups unsubstituted or substituted with 1, 2, 3, or 4 R⁸: phenyl, naphthyl, pyrrolyl, pyridinyl, benzothiazolyl, piperidyl, 6-azaspiro[2.5]octyl, 7-azaspiro[3.5]nonyl, 8-azaspiro[4.5]decyl, and 3-azaspiro[5.5]undecyl; each R⁸ is identical or different and is each independently selected from F, Br, Cl, CH₂F, CHF₂, CF₃, methyl, ethyl, n-propyl, isopropyl, n-butyl, tert-butyl, methoxy, ethoxy, phenyl, pyridinyl, and trifluoromethoxy.

According to an embodiment of the present disclosure, Cy² is selected from the following structures: 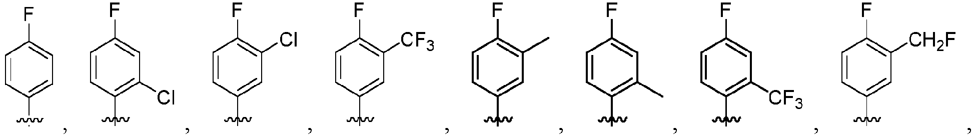 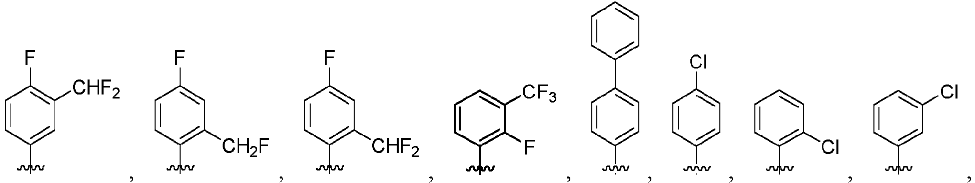 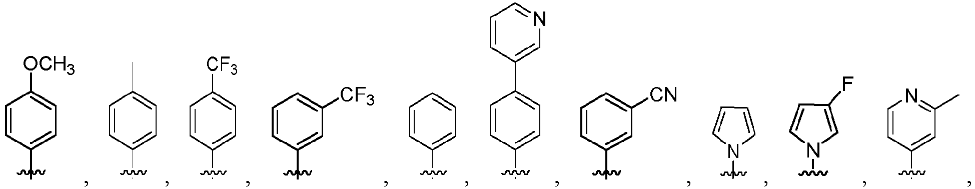 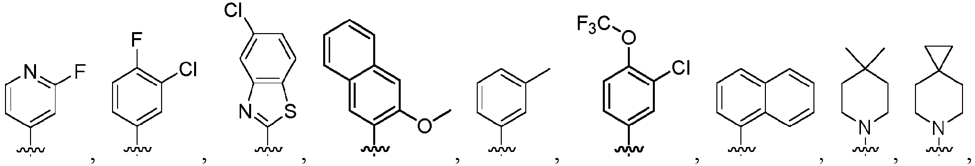 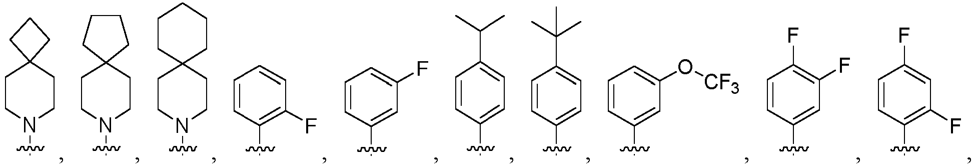 and

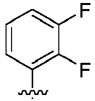

According to an embodiment of the present disclosure, Cy³ is selected from the following structures: 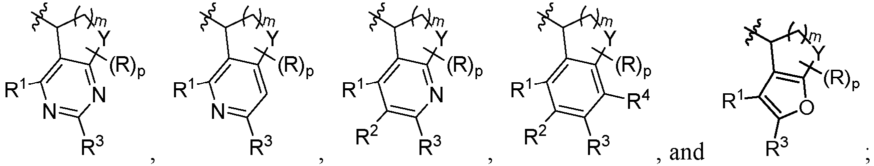
R¹, R², and R³ are each independently selected from H, F, Cl, Br, -NH₂, -OH, -CN, C₁₋₃ alkyl, and C₁₋₃ aminoalkyl;
R⁴ is selected from H, halogen, and C₁₋₃ alkyl;
Y is selected from -CH₂-, -C(CH₃)₂-, -NH-, -O-, and -S-; m is selected from 0, 1, and 2;
each R is identical or different and is each independently selected from H, halogen, and C₁₋₃ alkyl; p is selected from 0, 1, and 2;
R⁵ is selected from H, C₁₋₃ alkyl, C₂₋₃ alkynyl, C₁₋₃ haloalkyl, and C₃₋₄ cycloalkyl;
R^{5'} is selected from H and C₁₋₃ alkyl; or
R⁵ and R^{5'}, together with the carbon atom attached thereto, form C₃₋₄ cycloalkyl;
R⁶ is selected from H and C₁₋₃ alkyl; or
R⁵ and R⁶, together with the C and N atoms attached thereto, form 4-, 5-, or 6-membered heterocycloalkyl, wherein the 4-, 5-, and 6-membered heterocycloalkyl being unsubstituted or optionally substituted with 1, 2, or 3 R^{5a}; R^{5a} is each independently selected from F, Cl, Br, methyl, and ethyl; or R^{5a} on the same carbon atom or on two adjacent carbon atoms, together with the carbon atom attached thereto, form cyclopropyl or cyclobutyl, wherein the cyclopropyl and cyclobutyl being unsubstituted or optionally substituted with 1, 2, or 3 halogens;
L is absent or selected from -C(R^{a})(R^{b})-, -O-, and -C(R^{a})(R^{b})-O-; R^{a} and R^{b} are each independently selected from H, halogen, and C₁₋₃ alkyl, or R^{a} and R^{b}, together with the carbon atom attached thereto, form cyclopropyl;
Cy¹ is selected from the following groups unsubstituted or substituted with 1 or 2 R⁷: tetrahydropyrrolyl, 2,5-dihydro-1H-pyrrolyl, pyrrolyl, piperidyl, 1,2,3,6-tetrahydropyridinyl, 1,2,5,6-tetrahydropyridinyl, piperazinyl, 4-azaspiro[2.5]octyl, 1,2-dihydropyridinyl, and 1,4-dihydropyrazinyl; each R⁷ is identical or different and is each independently selected from H, oxo (=O), F, and Cl;
Cy² is selected from the following groups unsubstituted or substituted with 1, 2, 3, or 4 R⁸: phenyl, naphthyl, pyrrolyl, pyridinyl, benzothiazolyl, piperidyl, 6-azaspiro[2.5]octyl, 7-azaspiro[3.5]nonyl, 8-azaspiro[4.5]decyl, and 3-azaspiro[5.5]undecyl;
each R⁸ is identical or different and is each independently selected from -CN, F, Cl, -CH₂F, -CHF₂, CF₃, methyl, ethyl, n-propyl, isopropyl, tert-butyl, methoxy, phenyl, pyridinyl, and trifluoromethoxy.

According to an embodiment of the present disclosure,
R¹, R², and R³ are each independently selected from H, F, Cl, Br, -NH₂, -OH, and C₁₋₃ alkyl;
X is selected from CR⁴ and N;
R⁴ is selected from H, halogen, and C₁₋₃ alkyl;
Y may be selected from -CH₂- and -NH-;
m is selected from 0, 1, and 2;
each R may be identical or different and is each independently selected from H, halogen, and C₁₋₃ alkyl; p may be selected from 0, 1, and 2;
R⁵ may be selected from H and C₁₋₃ alkyl;
R⁶ may be selected from H and C₁₋₃ alkyl; or
R⁵ and R⁶, together with the C and N atoms attached thereto, form 4-, 5-, or 6-membered heterocycloalkyl;
L may be absent or selected from -C(R^{a})(R^{b})-, R^{a} and R^{b} are each independently H, halogen, or C₁₋₃ alkyl, or R^{a} and R^{b}, together with the carbon atom attached thereto, form cyclopropyl;
Cy¹ may be selected from the following groups unsubstituted or substituted with 1 or 2 R⁷: tetrahydropyrrolyl, 2,5-dihydro-1H-pyrrolyl, pyrrolyl, piperidyl, 1,2,3,6-tetrahydropyridinyl, 1,2,5,6-tetrahydropyridinyl, and piperazinyl; R⁷ is selected from H, F, and Cl;
Cy² may be selected from the following groups unsubstituted or substituted with 1, 2, 3, or 4 R⁸: phenyl, naphthyl, pyridinyl, benzothiazolyl, piperidyl, 6-azaspiro[2.5]octyl, 7-azaspiro[3.5]nonyl, 8-azaspiro[4.5]decyl, and 3-azaspiro[5.5]undecyl;
each R⁸ may be identical or different and is each independently selected from F, Cl, CF₃, methyl, isopropyl, tert-butyl, methoxy, phenyl, pyridinyl, and trifluoromethoxy.

According to an embodiment of the present disclosure, Cy³ is selected from the following structures: 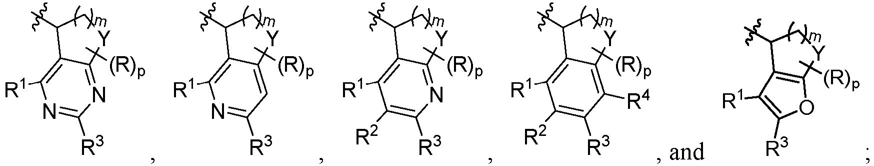
R¹ is selected from H, F, Cl, -NH₂, and methyl;
R² is selected from H, F, Cl, -CN, and methyl;
R³ is selected from H, F, -NH₂, -OH, -CH₂NH₂, and methoxy;
R⁴ is selected from H and F;
Y is selected from -CH₂-, -C(CH₃)₂-, -NH-, and -O-;
m is 1 or 2;
R is selected from H, methyl, and ethyl;
R⁵ is selected from H, methyl, ethyl, ethynyl, propynyl, -CH₂F, -CHF₂, -CF₃, and cyclopropyl;
R^{5'} is selected from H, methyl, and ethyl; or
R⁵ and R^{5'}, together with the carbon atom attached thereto, form cyclopropyl;
R⁶ is H; or
R⁵ and R⁶, together with the C and N atoms attached thereto, form the following structures: 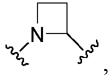 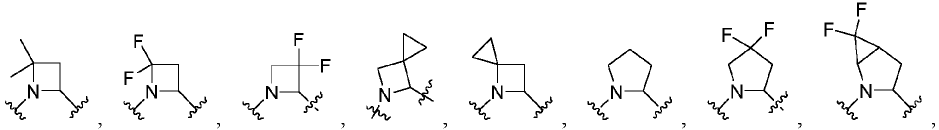 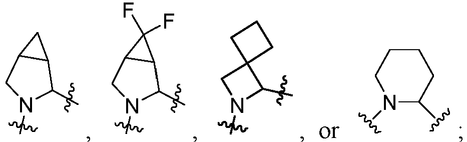 the structures formed are preferably selected from: 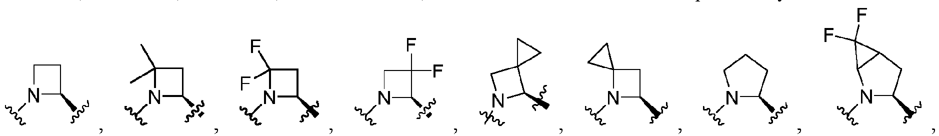 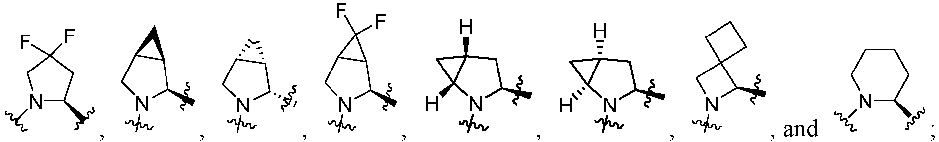
L may be absent or selected from -CH₂-, -CH(CH₃)-, -CF₂-, -O-, -CH₂-O-, and 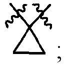
Cy¹ may be selected from the following structures: 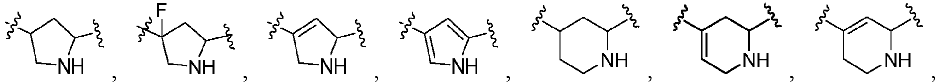 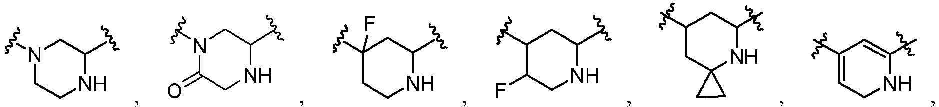 and 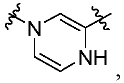
preferably selected from: 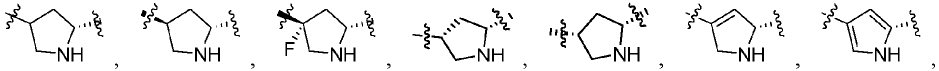 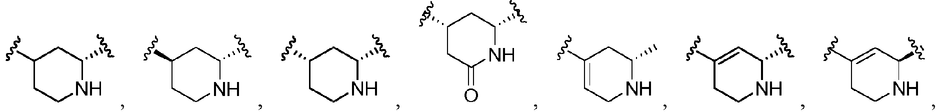 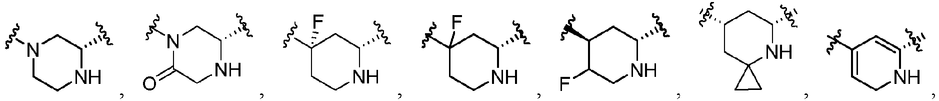 and 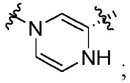 and/or
Cy² may be selected from the following structures:

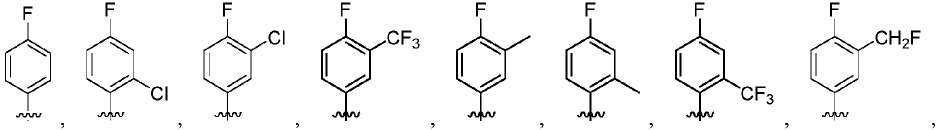

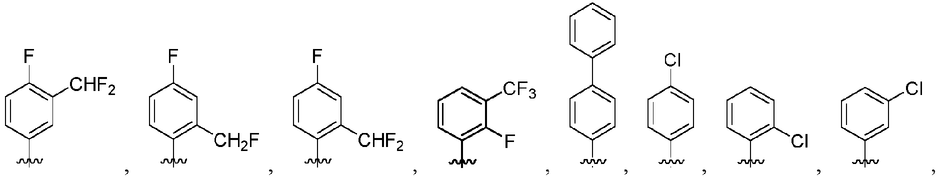

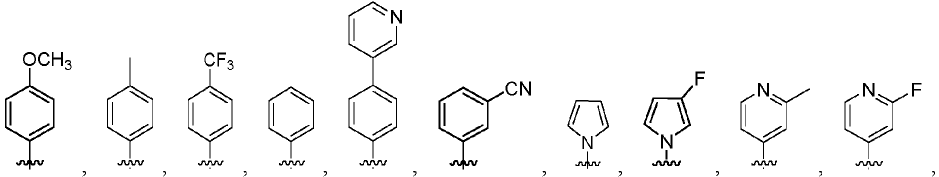

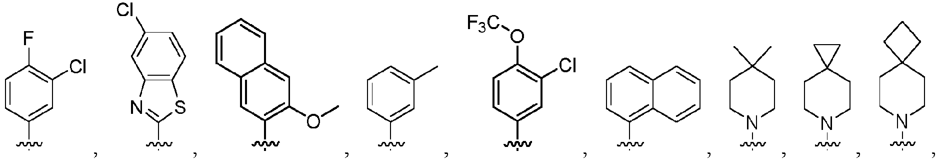

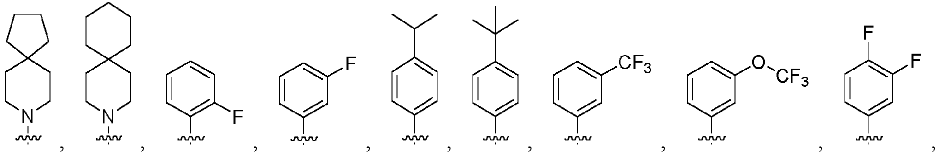

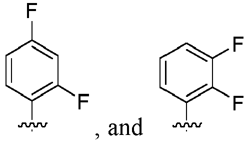

According to an embodiment of the present disclosure, R¹ may be selected from H and F;
R² may be selected from H, F, Cl, and methyl;
R³ may be selected from -NH₂, -OH, and methoxy;
X may be selected from CR⁴ and N; R⁴ is selected from H and F;
Y may be selected from -CH₂-; m is 1 or 2;
R may be selected from H;
R⁵ may be selected from H and methyl;
R⁶ may be selected from H; or
R⁵ and R⁶, together with the C and N atoms attached thereto, form the following structures: 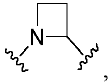 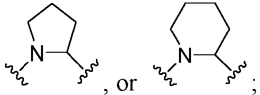 the structures formed are preferably selected from: 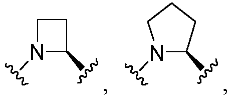 and 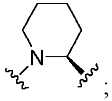
L may be absent or selected from -CH₂-, -CH(CH₃)-, -CF₂-, and 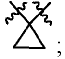 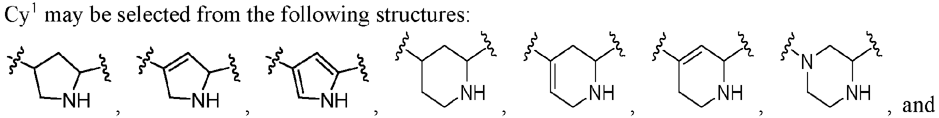 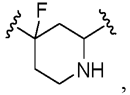
preferably selected from: 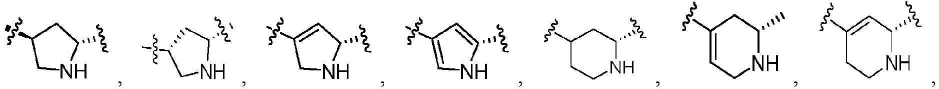 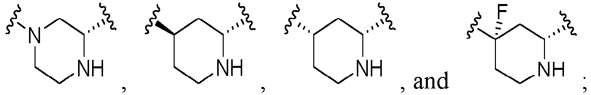 and/or
Cy² may be selected from the following structures:

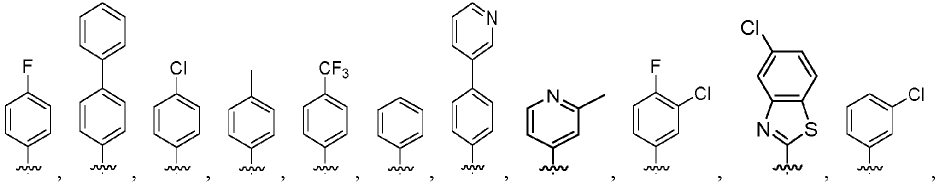

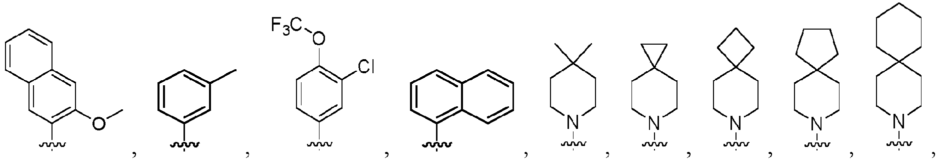

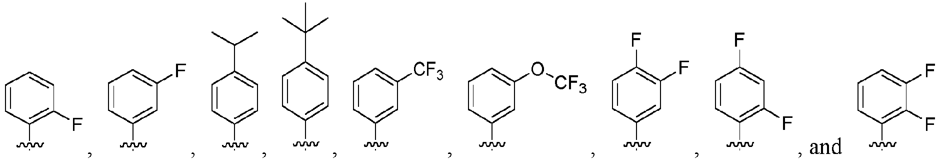

According to an embodiment of the present disclosure, the compound represented by formula (I') is selected from the following structures: 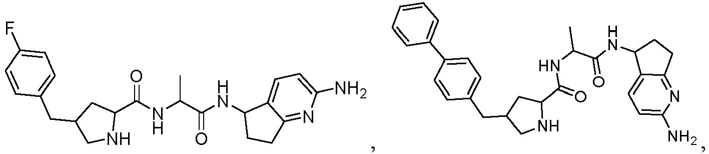 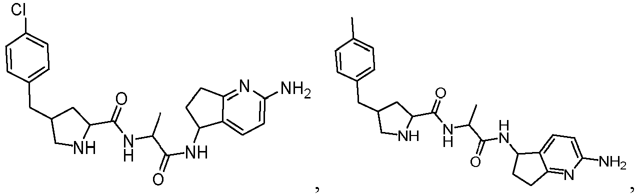 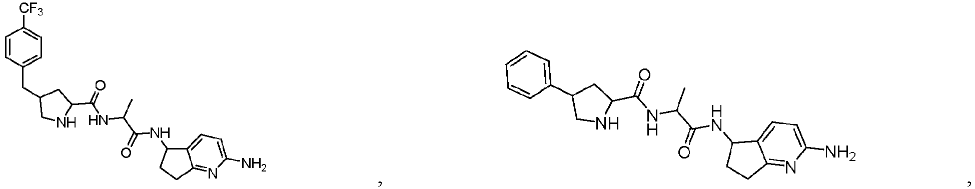 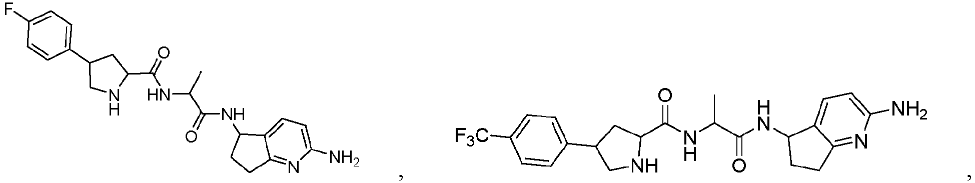 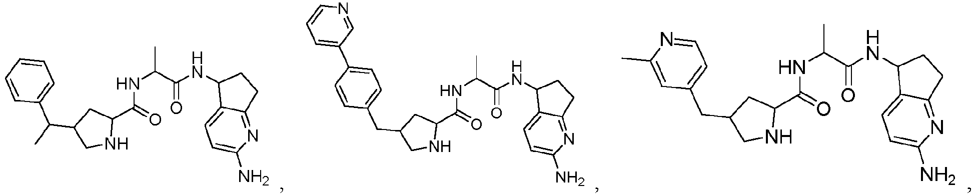 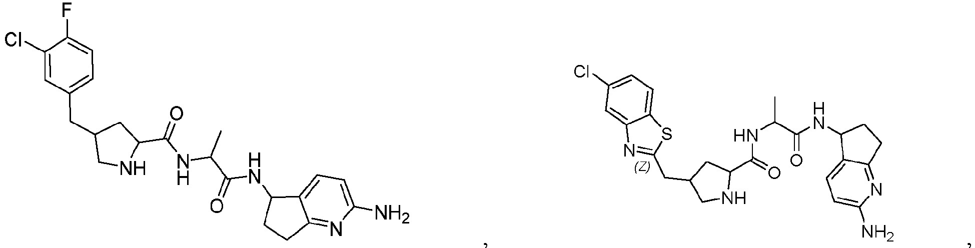 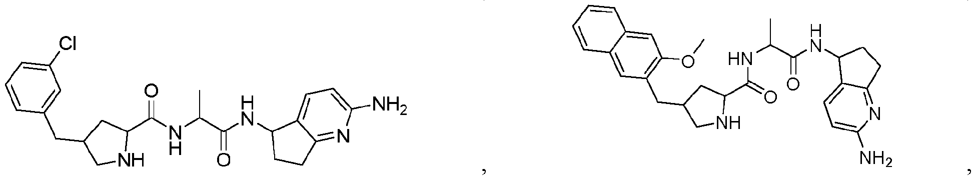 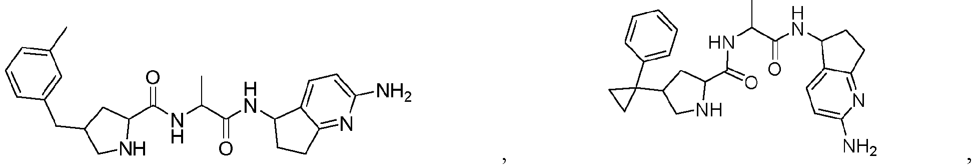 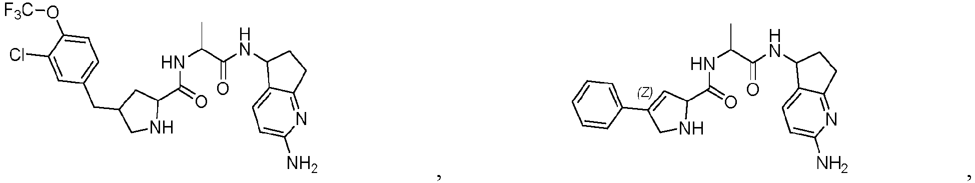 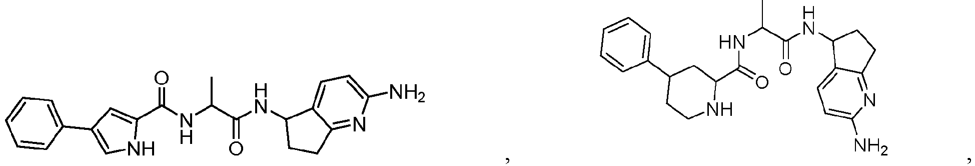 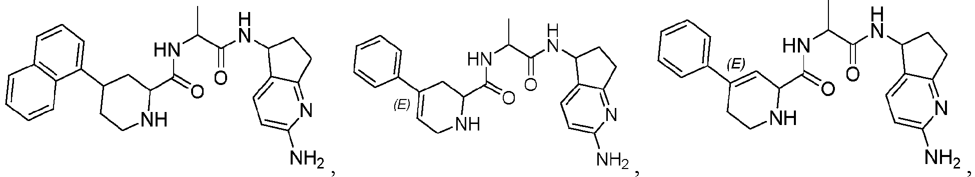 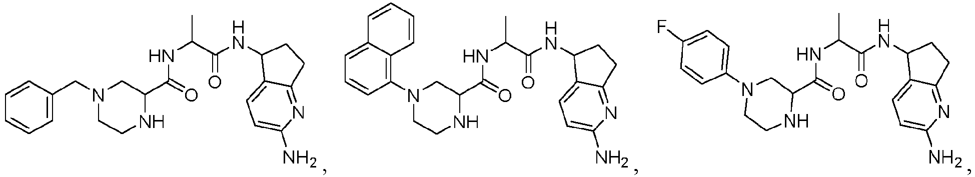 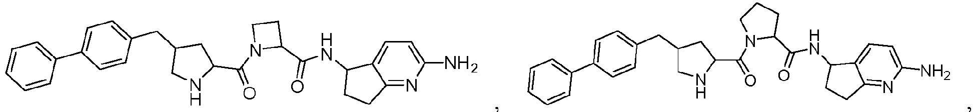 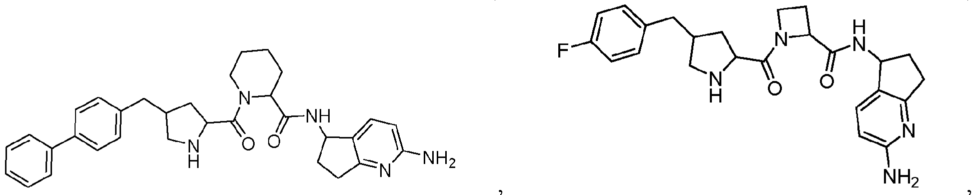 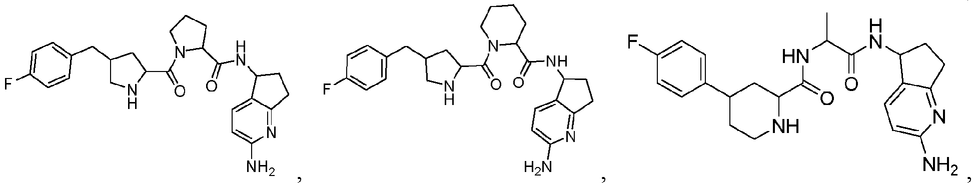 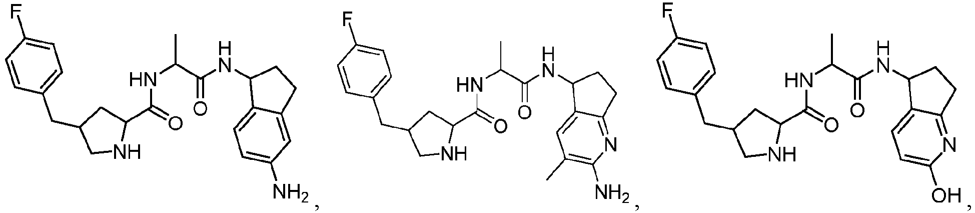 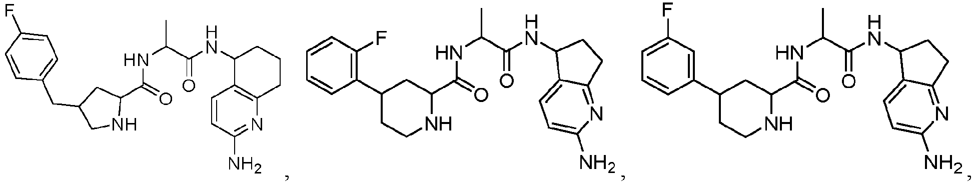 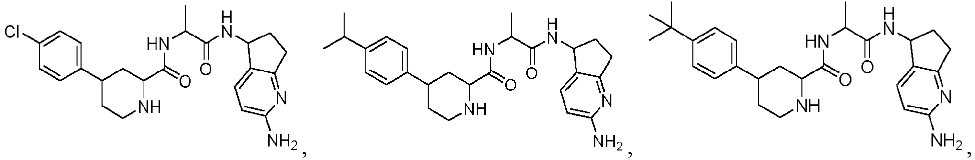 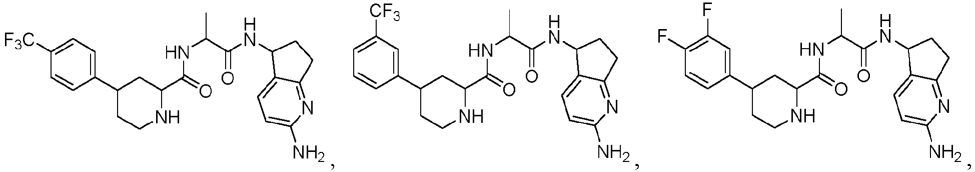 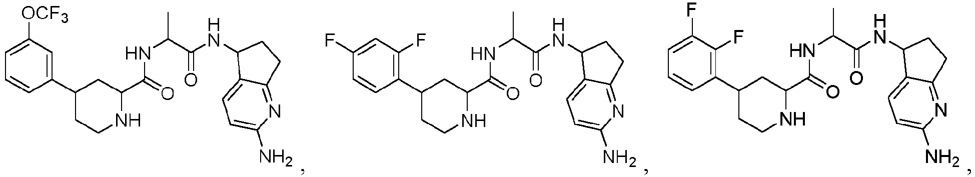 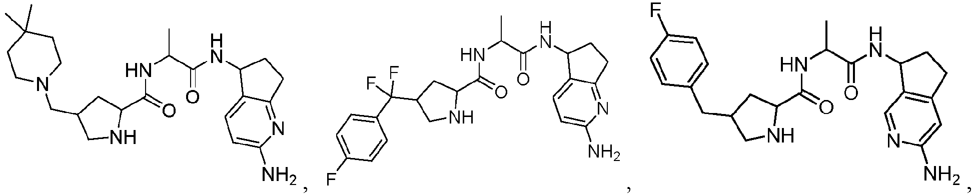 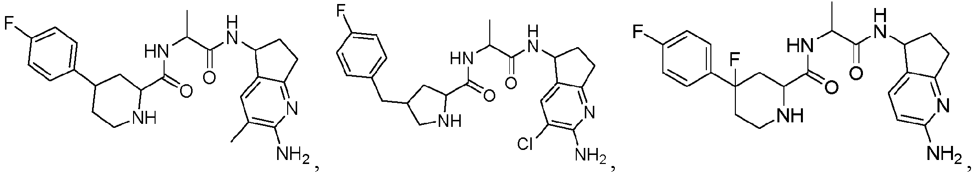 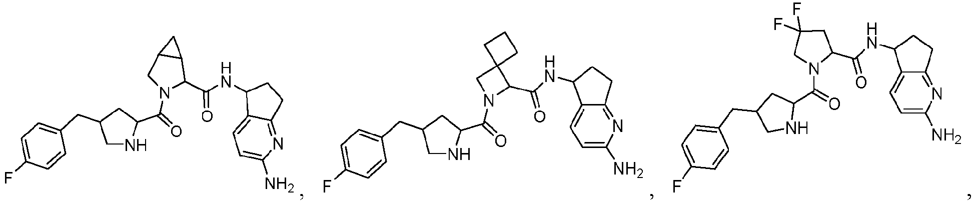 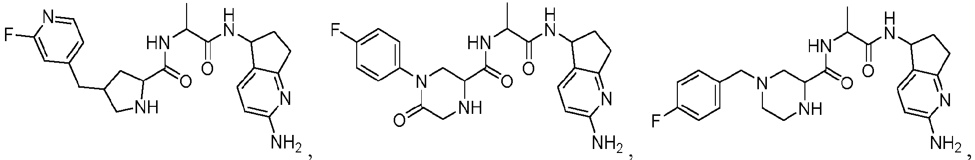 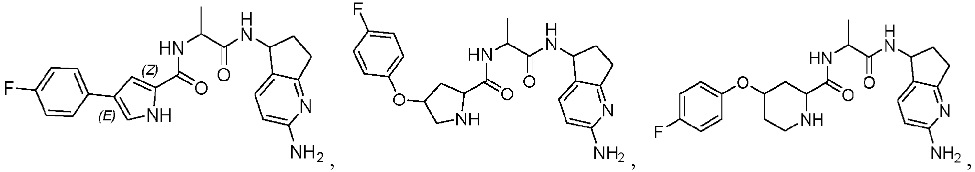 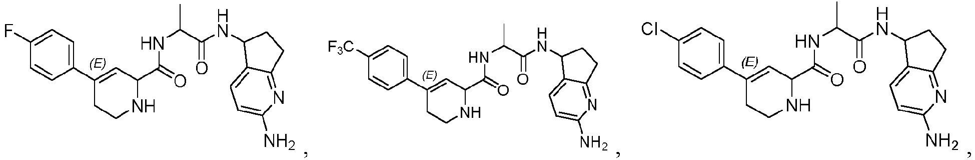 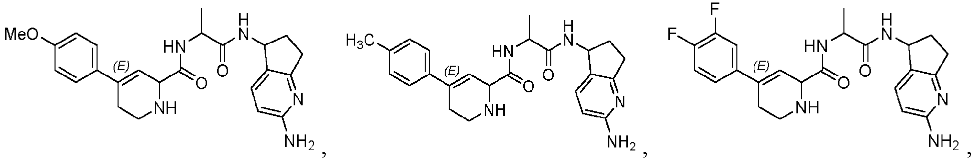 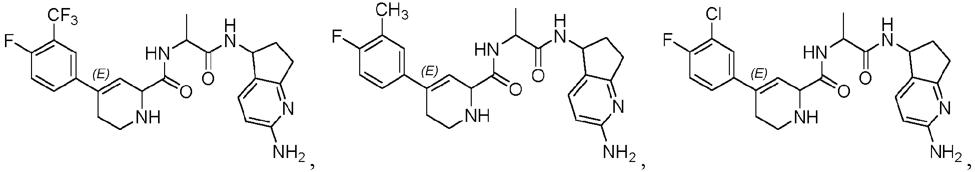 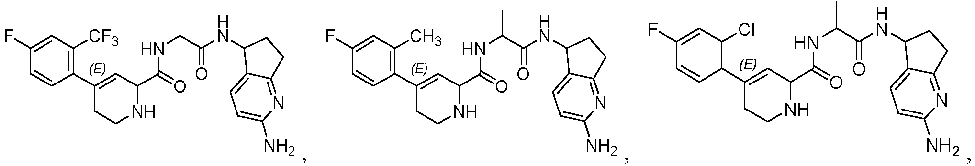 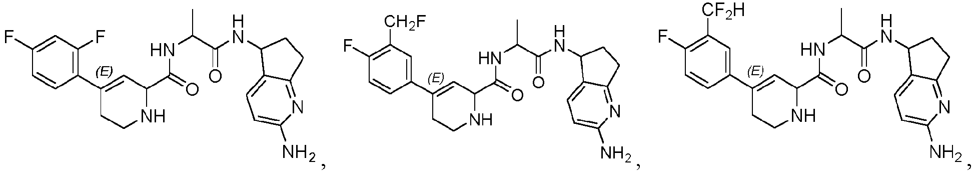 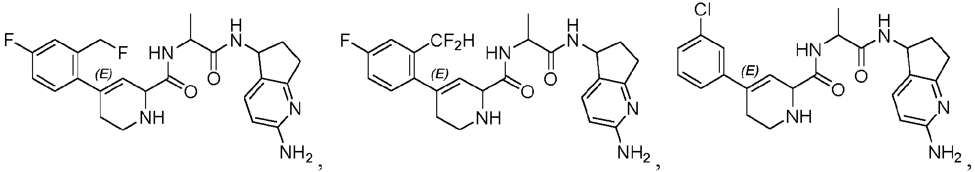 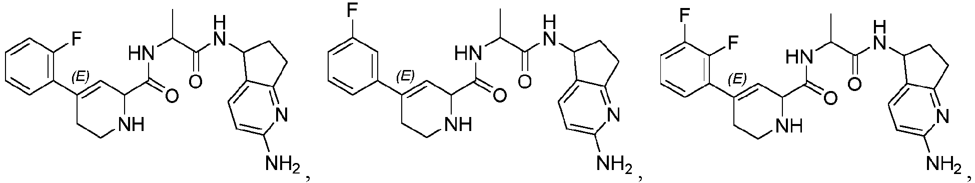 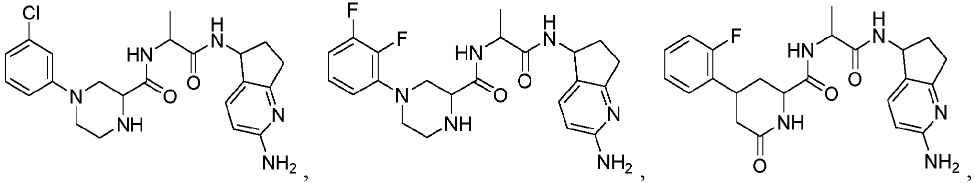 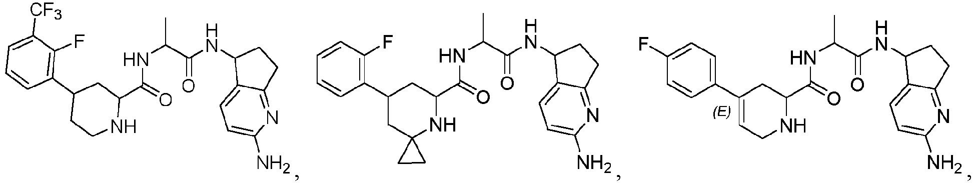 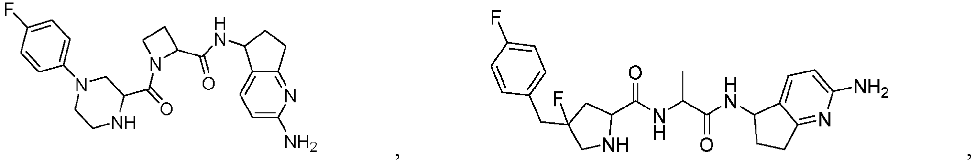 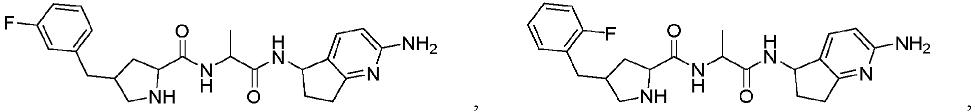 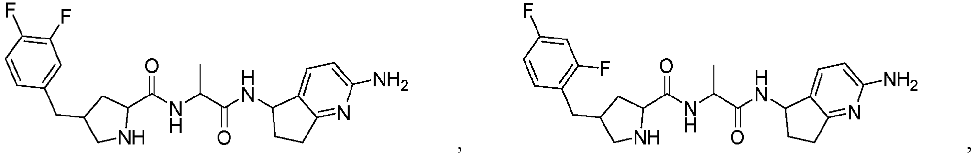 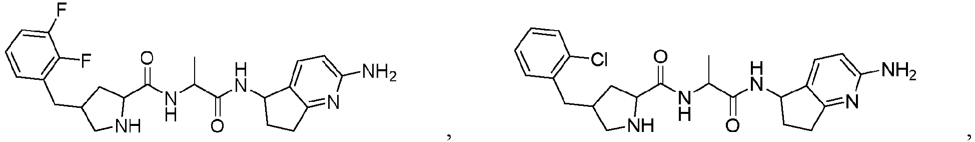 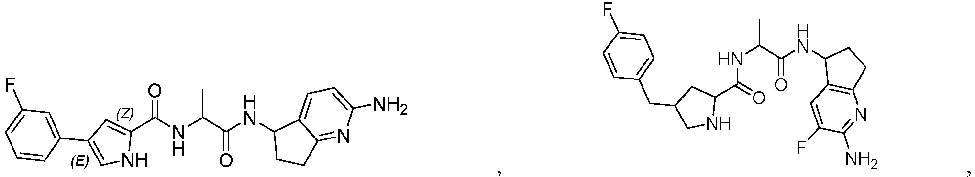 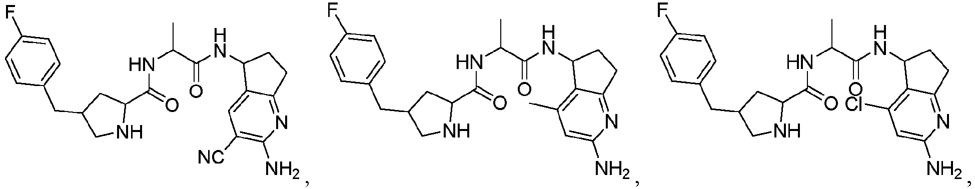 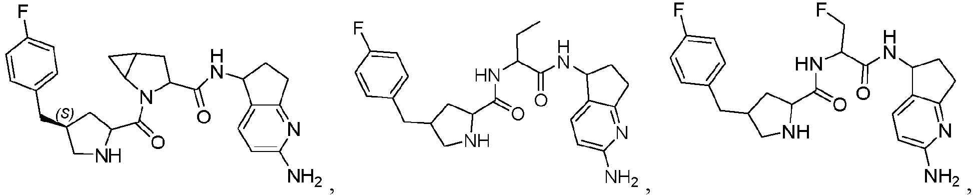 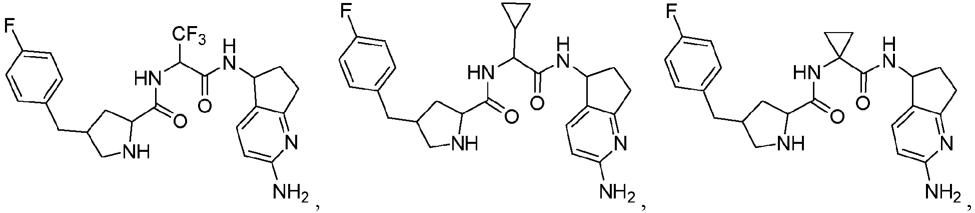 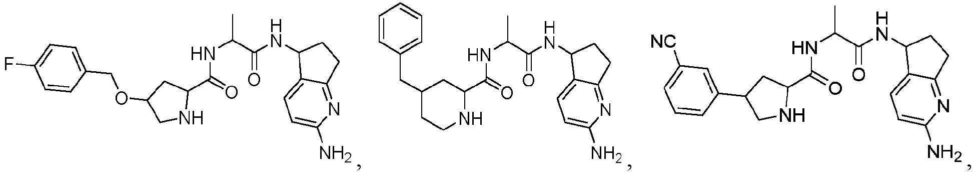 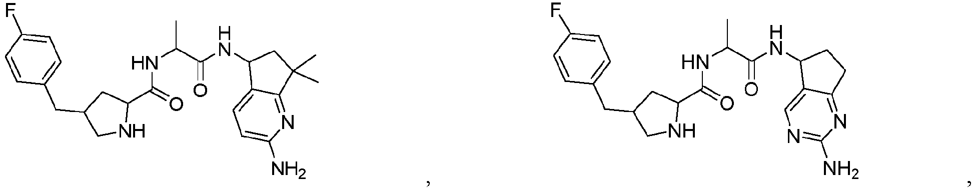 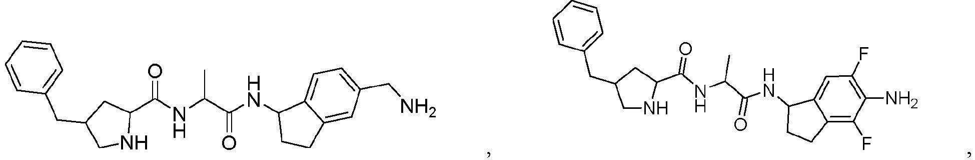 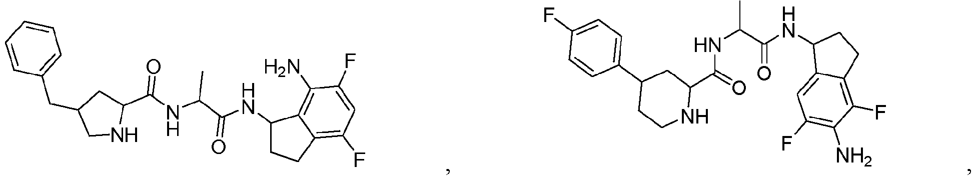 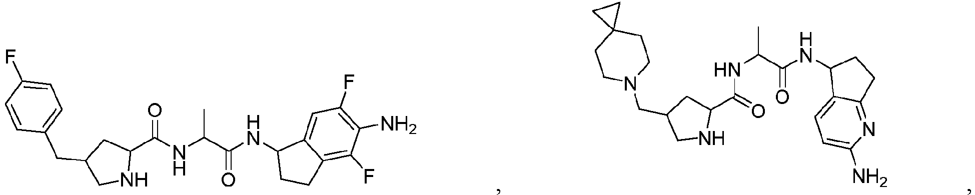 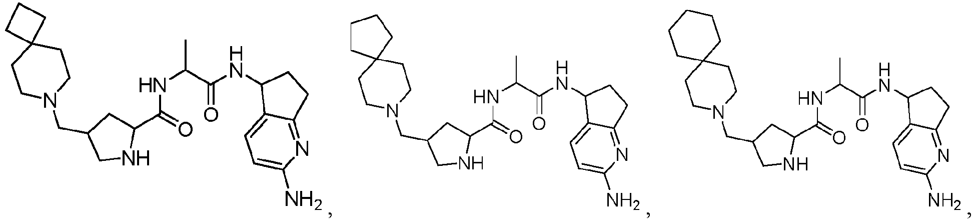 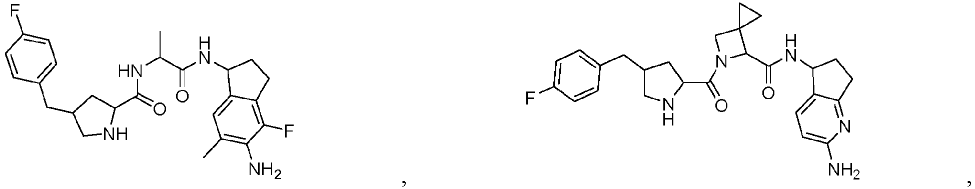 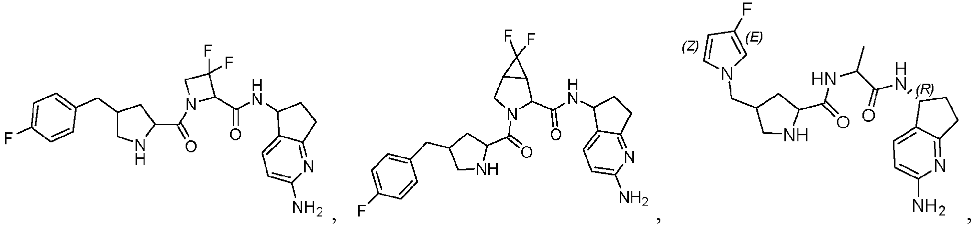 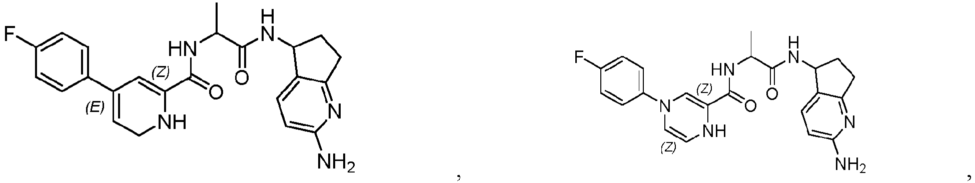 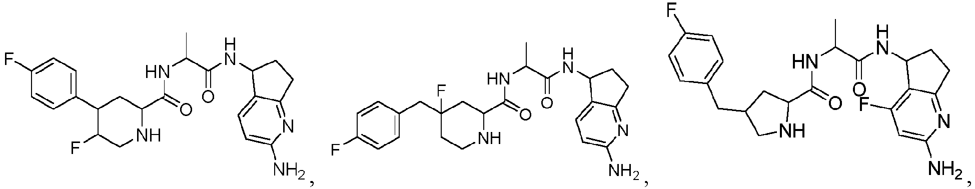 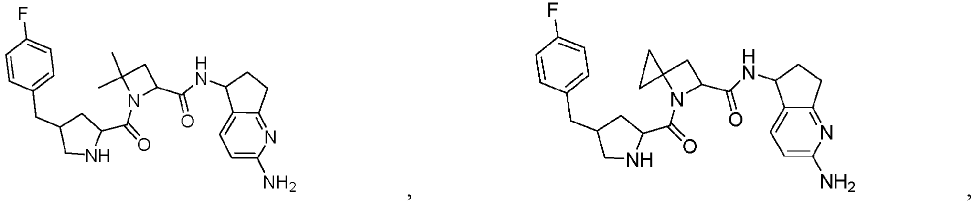 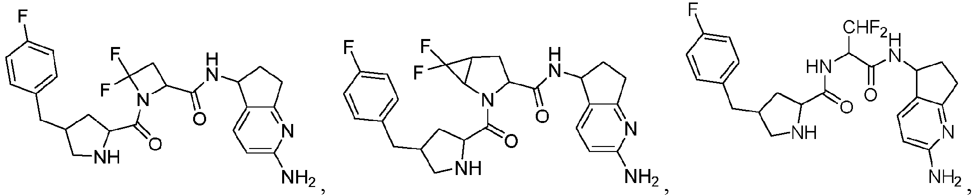 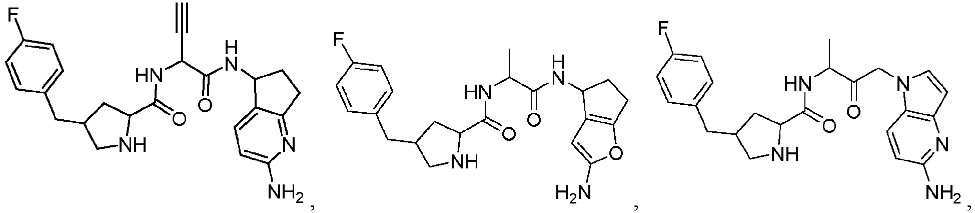 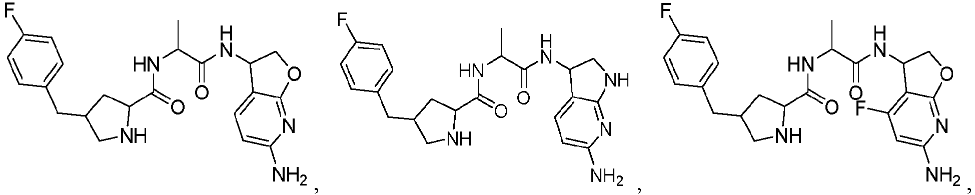 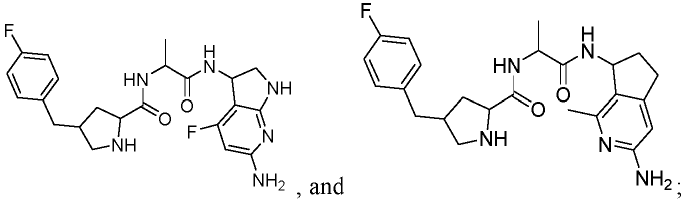 preferably, the compound represented by formula (I') is selected from the following structures:

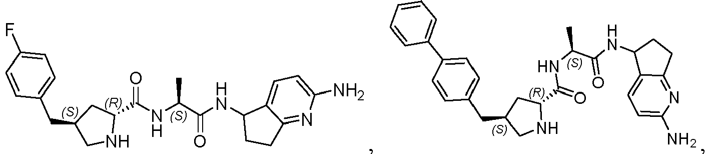

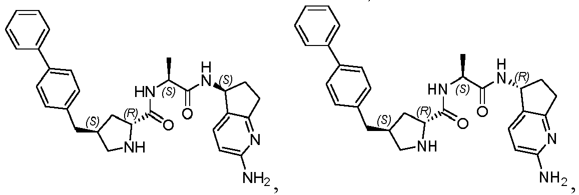

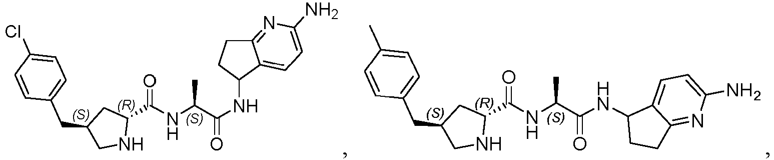

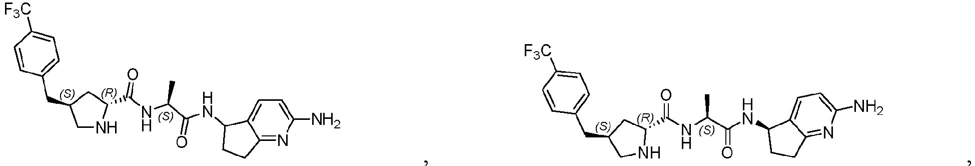

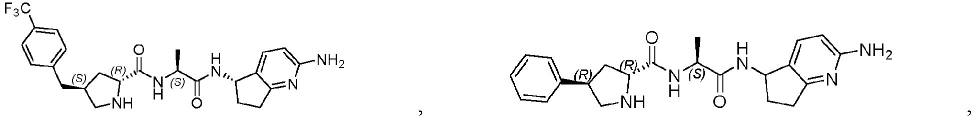

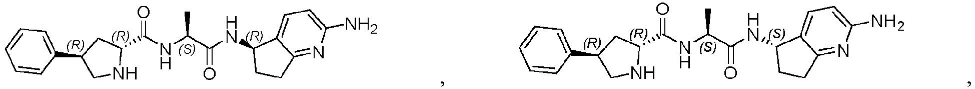

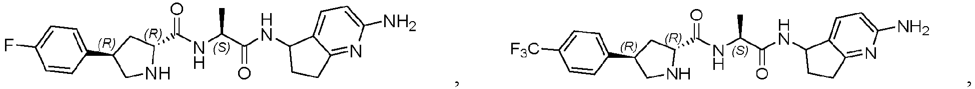

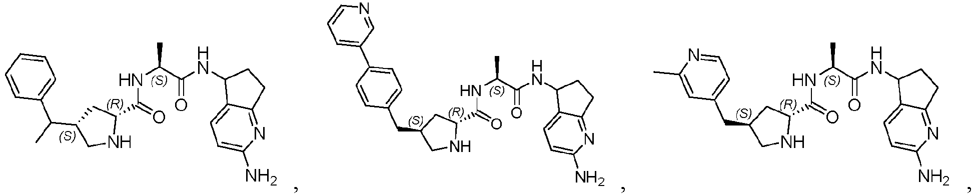

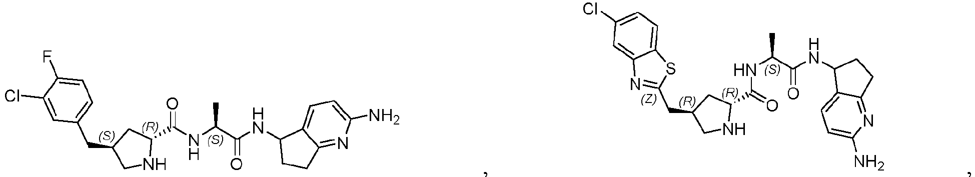

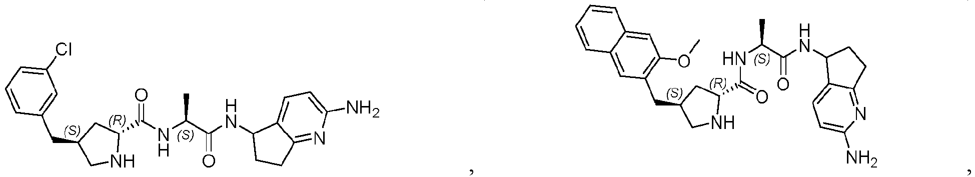

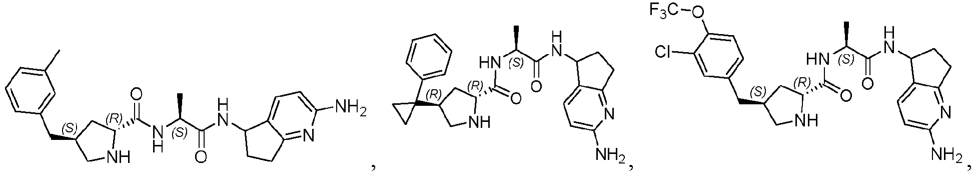

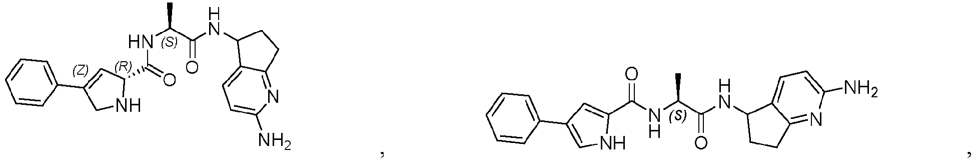

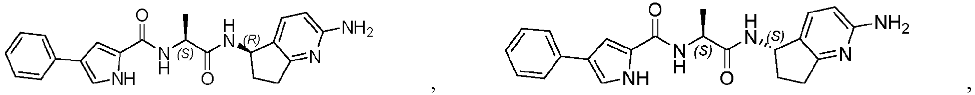

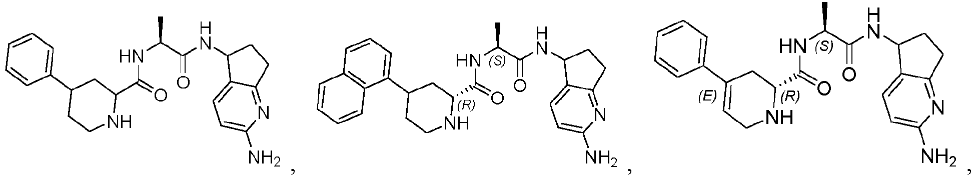

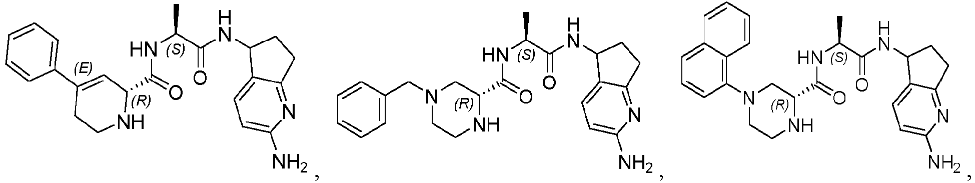

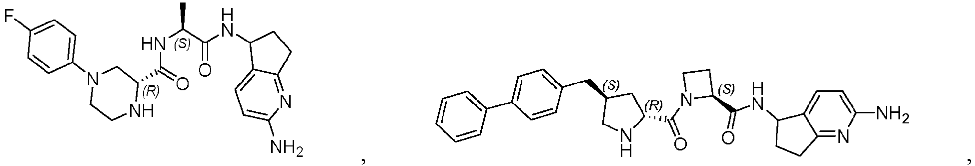

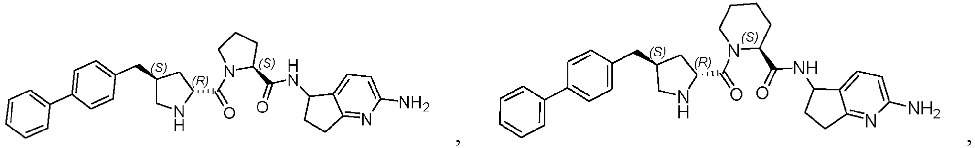

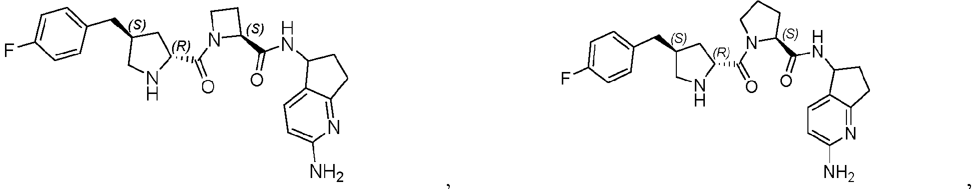

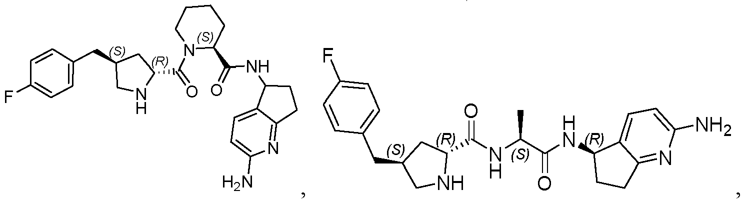

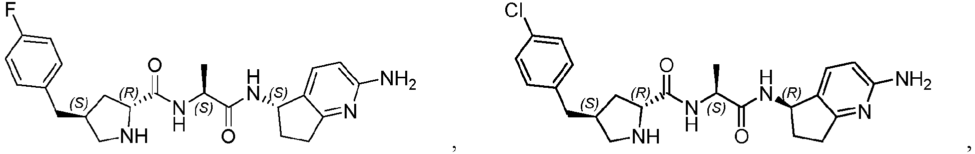

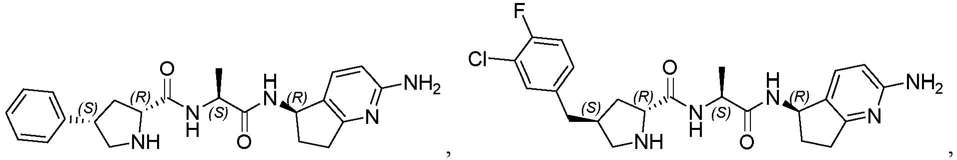

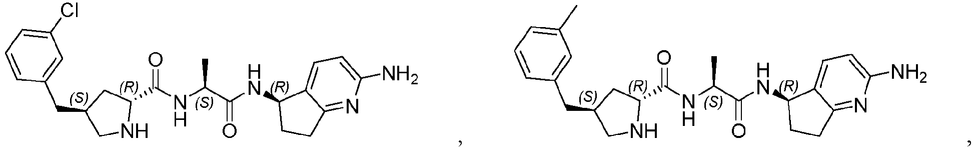

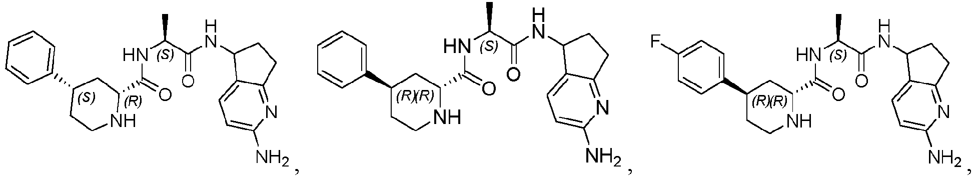

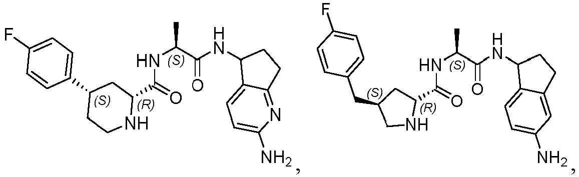

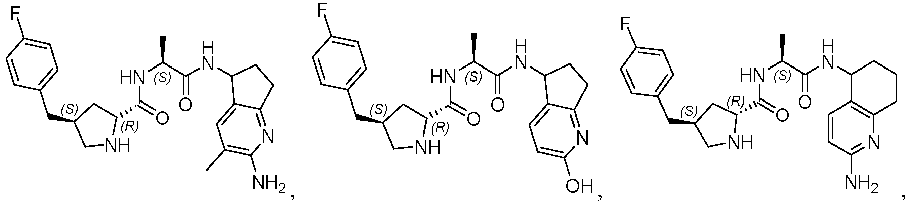

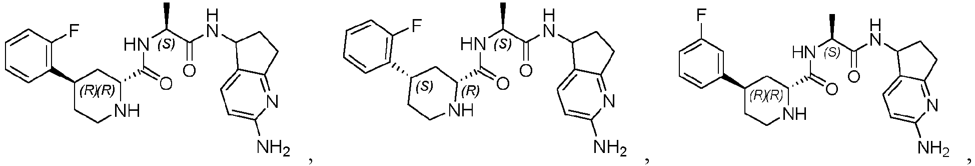

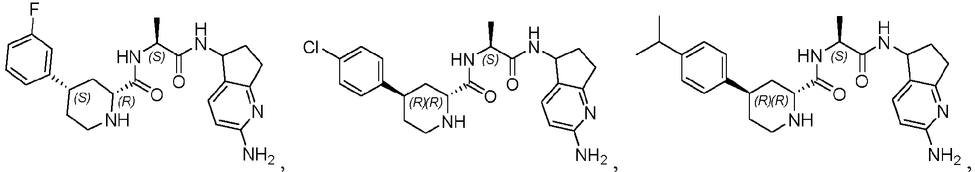

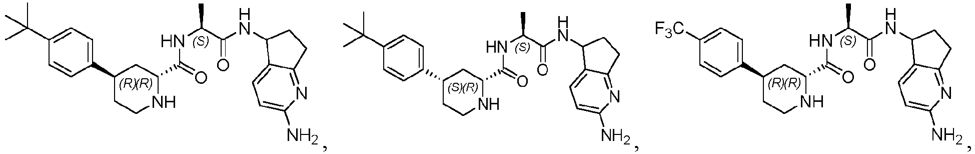

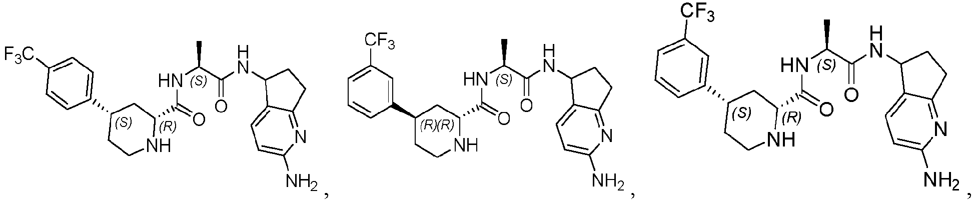

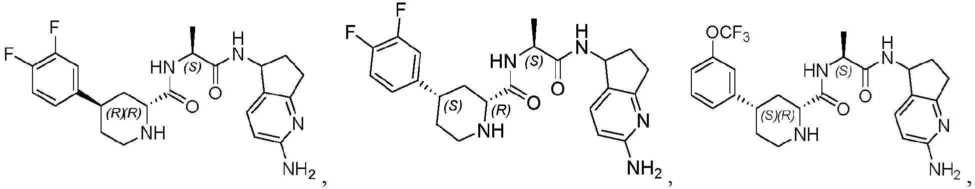

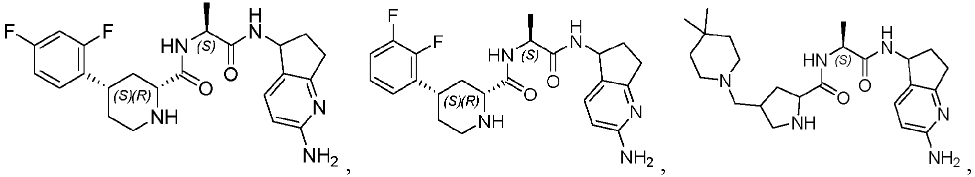

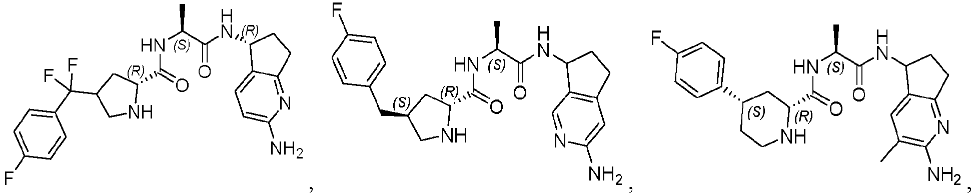

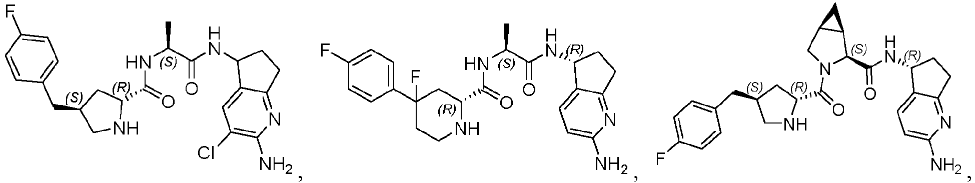

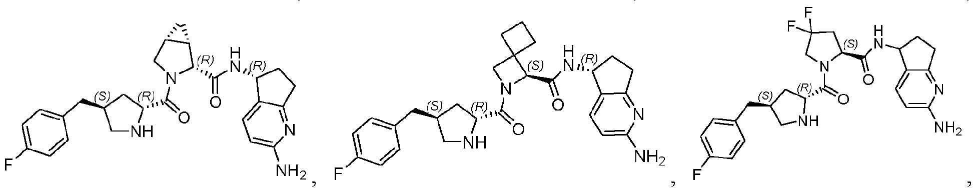

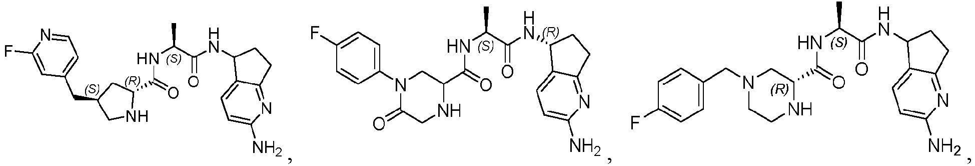

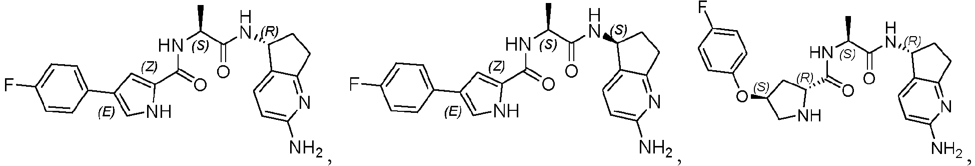

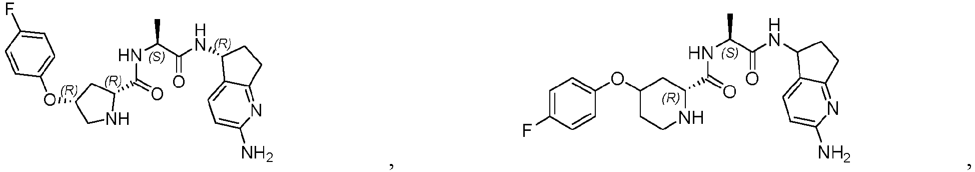

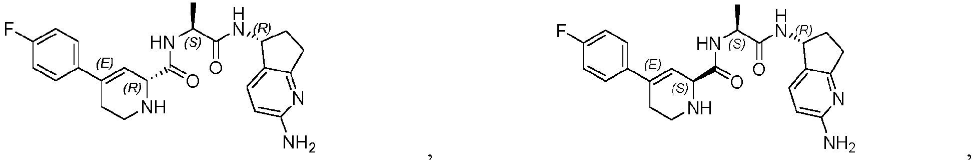

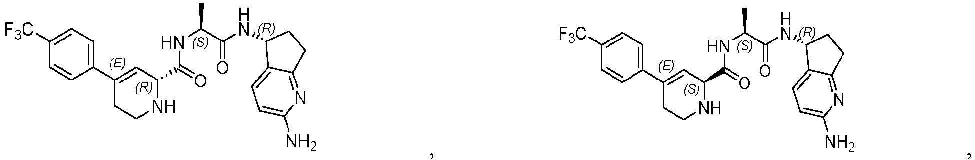

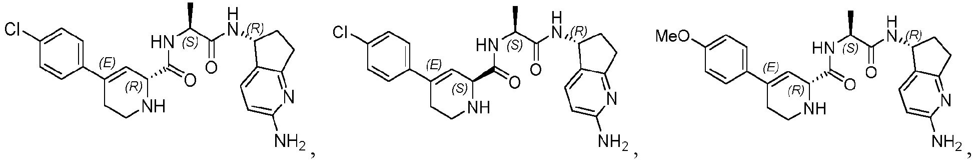

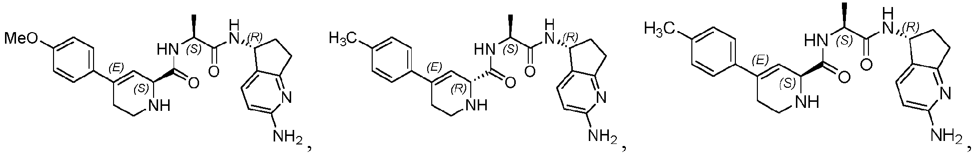

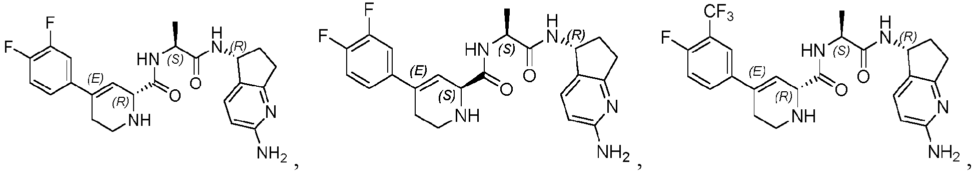

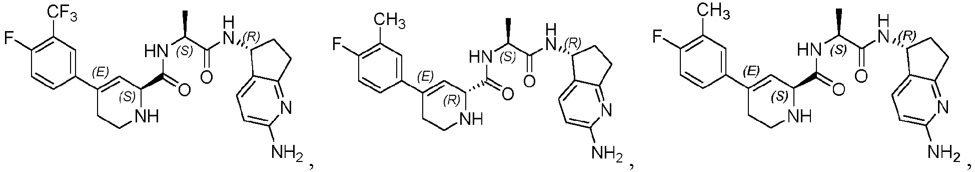

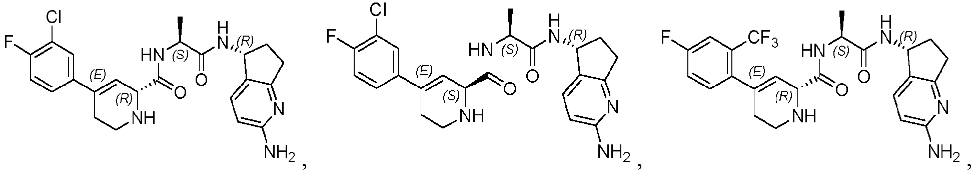

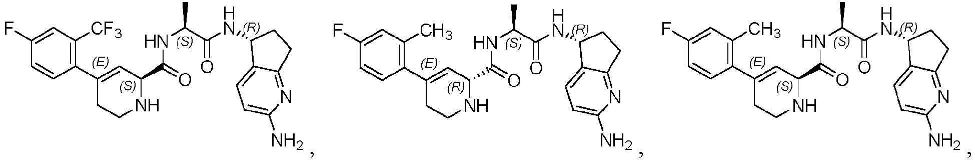

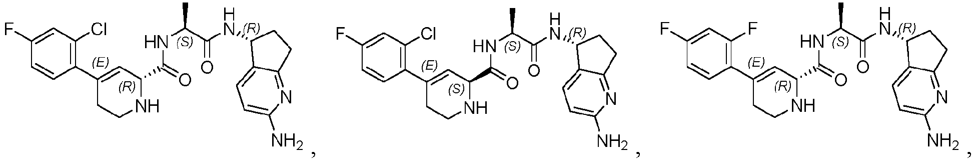

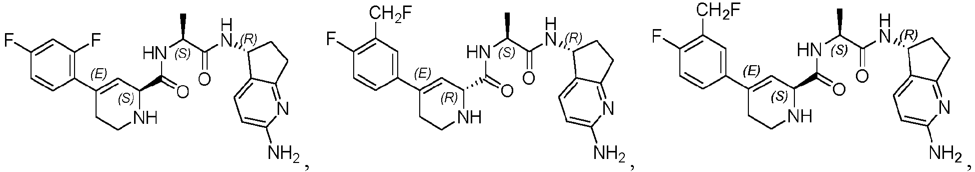

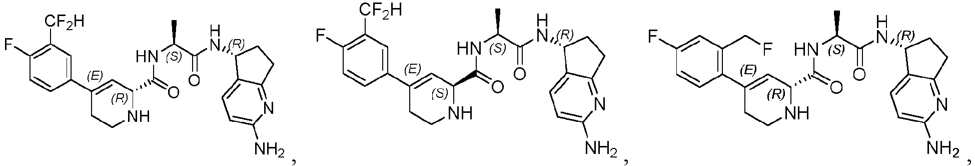

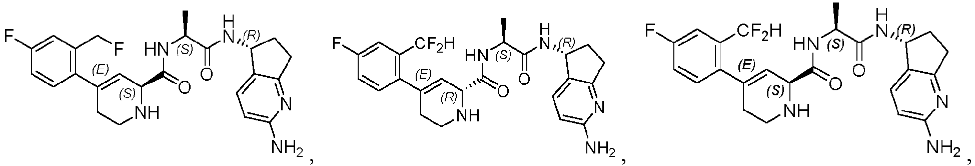

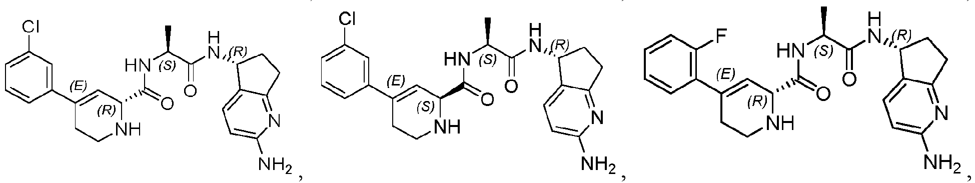

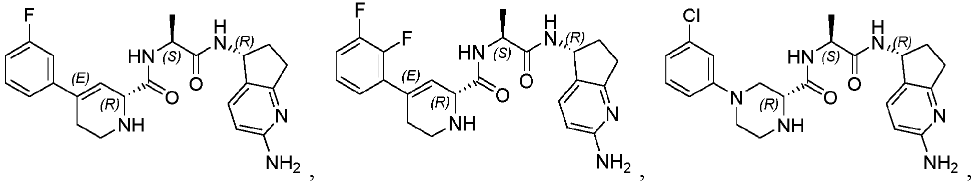

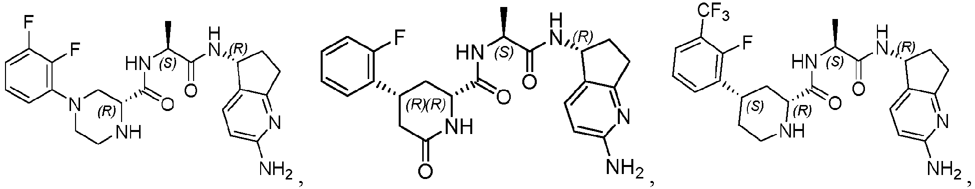

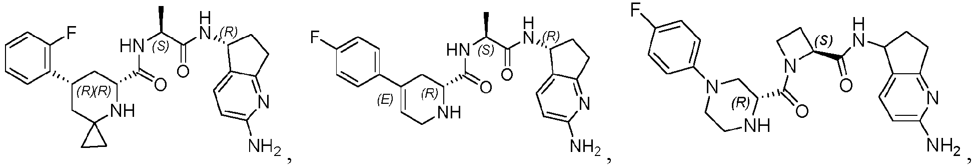

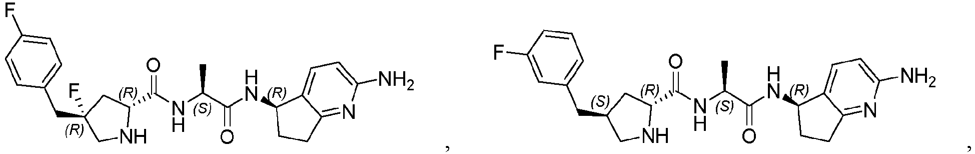

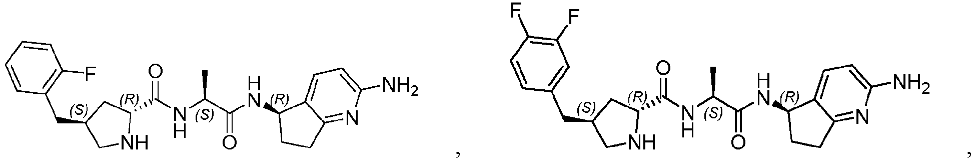

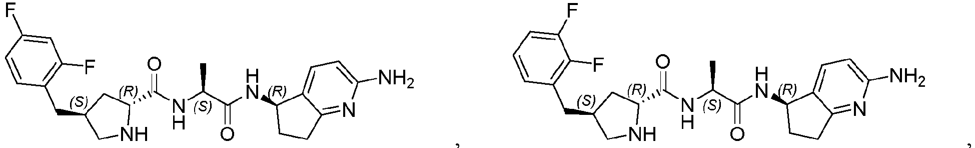

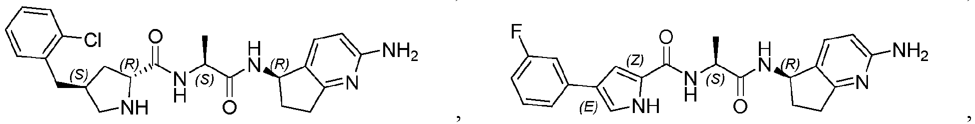

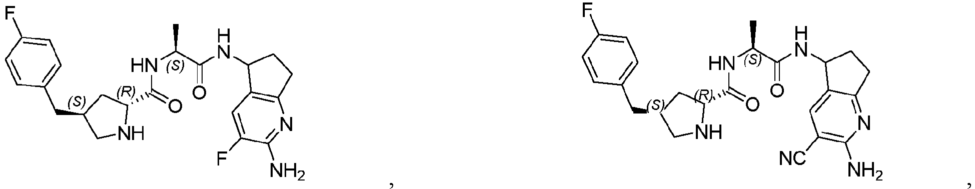

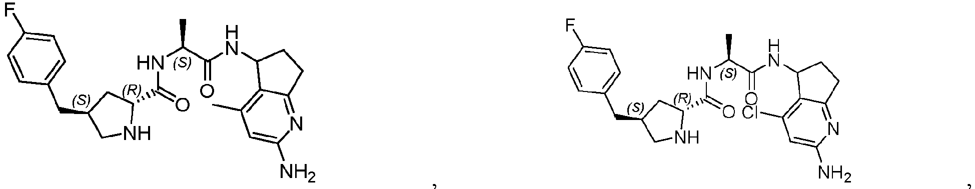

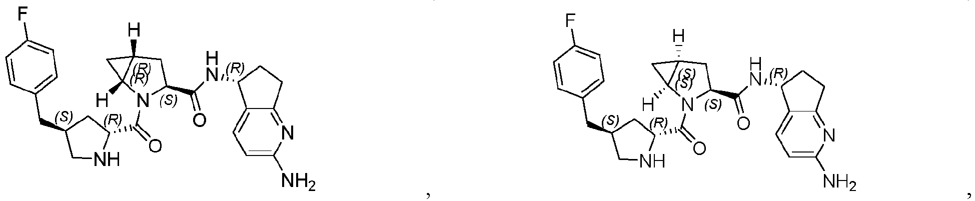

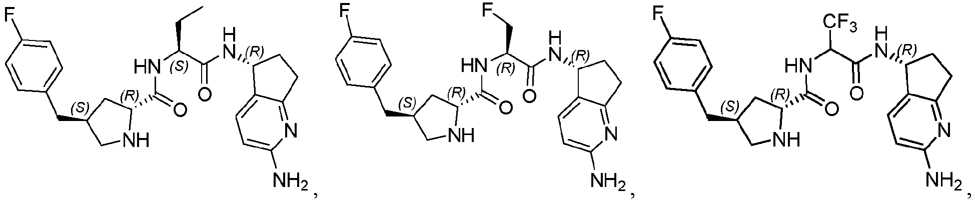

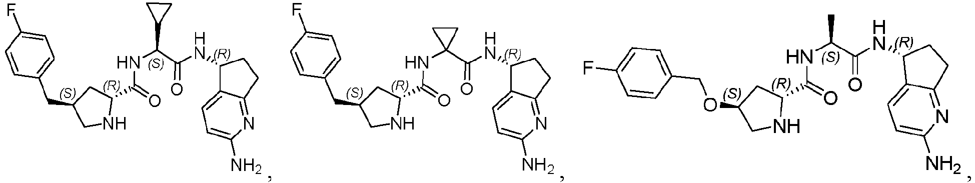

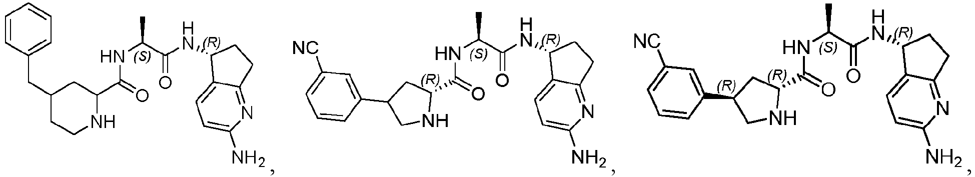

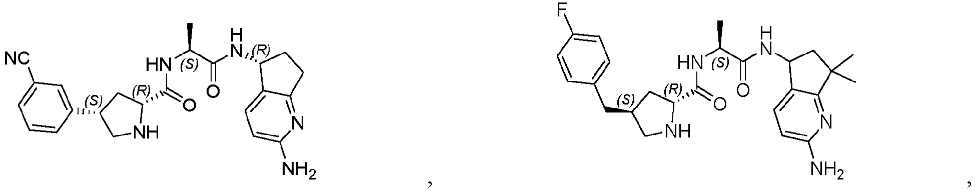

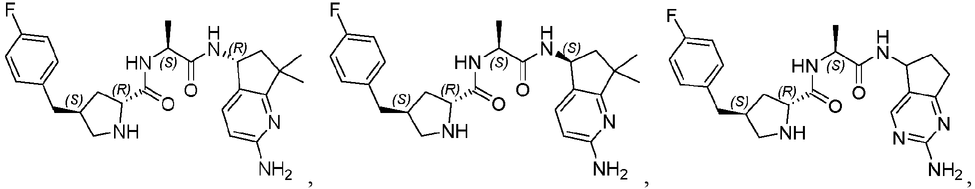

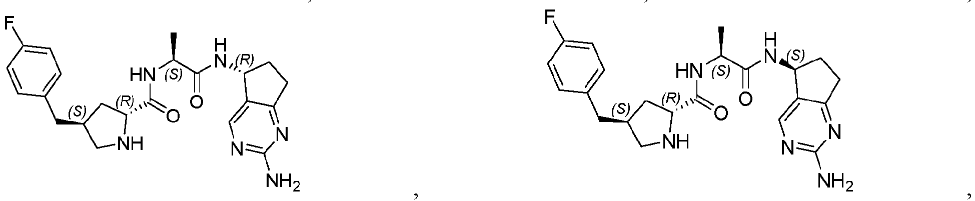

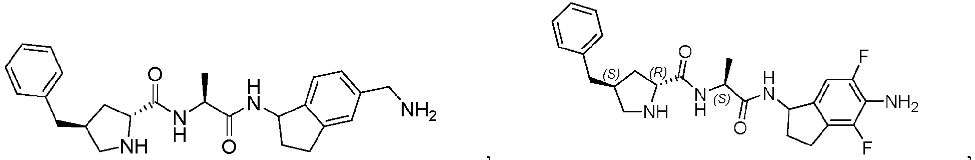

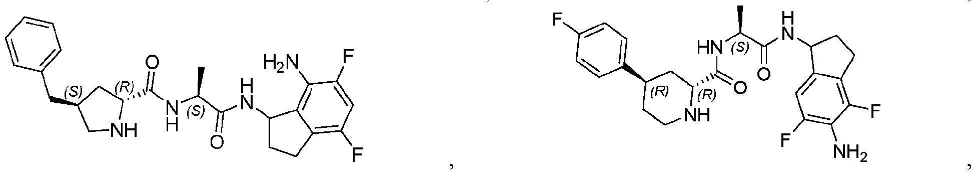

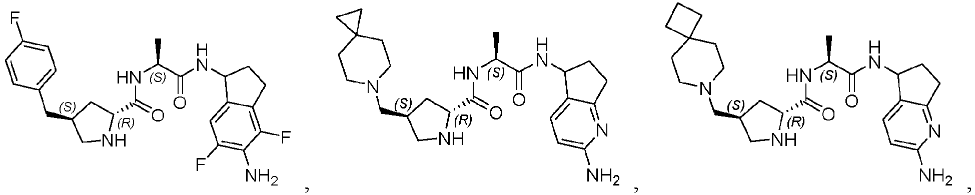

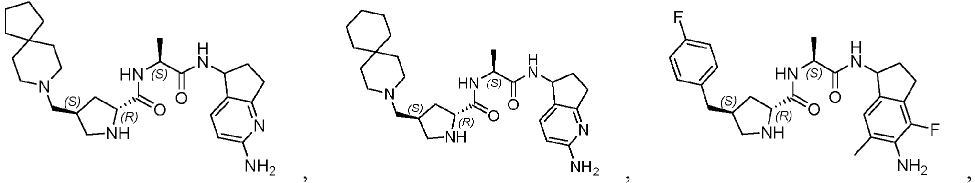

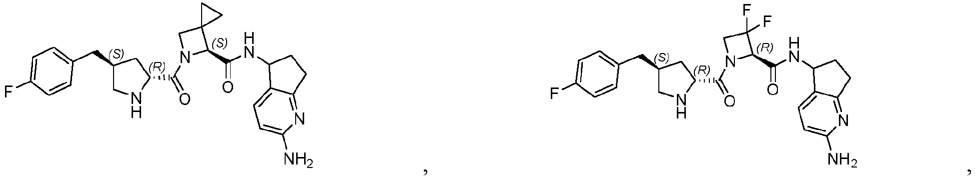

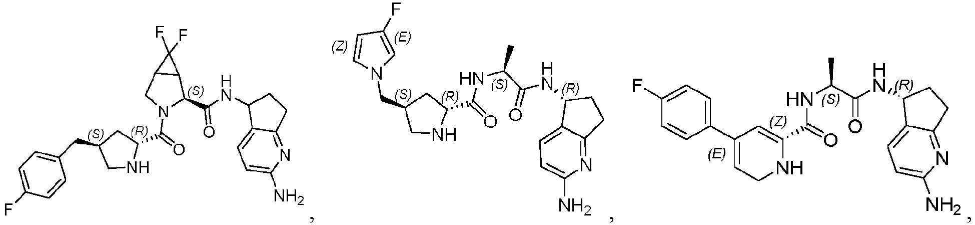

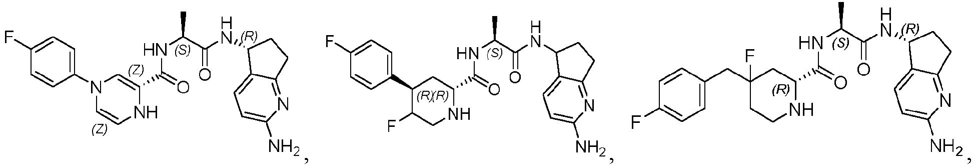

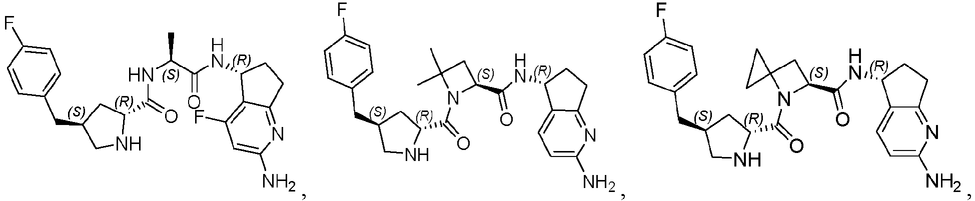

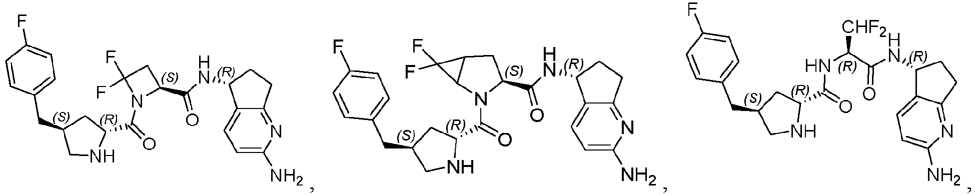

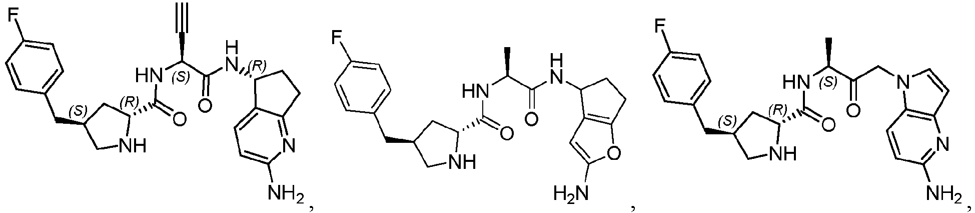

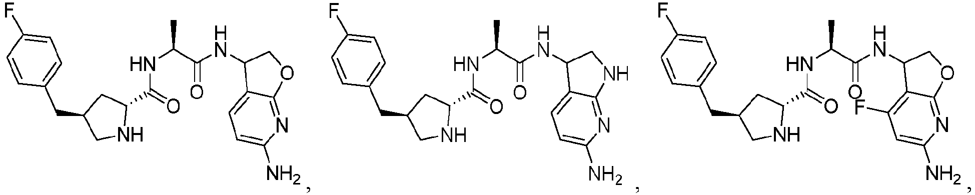

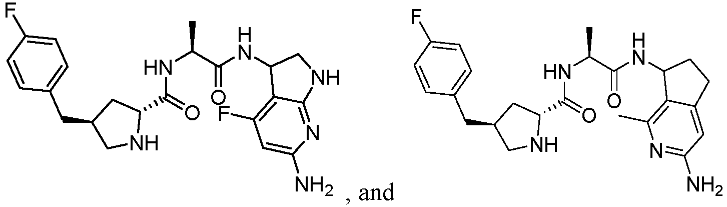

The present disclosure further provides a preparation method for the compound represented by formula (I'), which comprises the following steps:
subjecting compound I-A' and compound I-B' to a condensation reaction to give compound I-1', and then deprotecting the compound I-1' to give the compound represented by formula (I'); 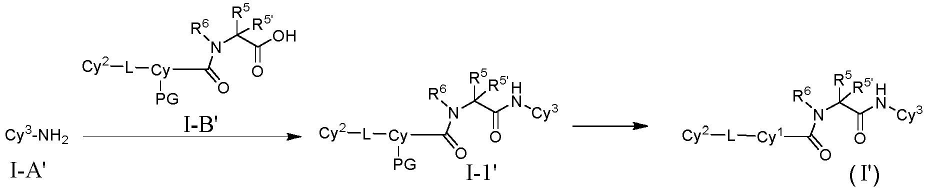 or
subjecting compound I-C' and compound I-D' to a condensation reaction to give compound I-1', and then deprotecting the compound I-1' to give the compound represented by formula (I'); 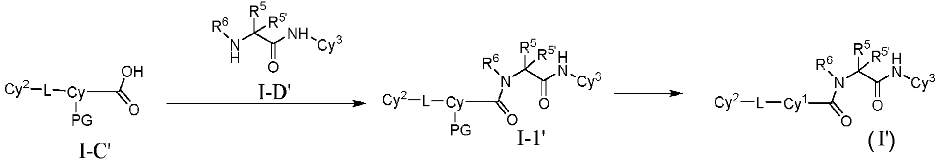
wherein R⁵, R^{5'}, R⁶, L, Cy¹, Cy², and Cy³ are each independently defined as described herein; Cy is a group resulting from protection of an active group in Cy¹ by PG; PG is a protecting group, e.g., an amino protecting group such as tert-butyloxycarbonyl.

The present disclosure further provides a preparation method for the compound represented by formula (I), which comprises the following steps: subjecting compound I-A and compound I-B to a condensation reaction to give compound I-1, and then deprotecting the compound I-1 to give the compound represented by formula (I); 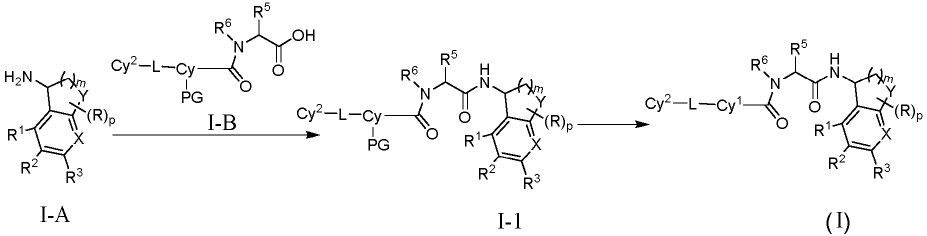 or subjecting compound I-C and compound I-D to a condensation reaction to give compound I-1, and then deprotecting the compound I-1 to give the compound represented by formula (I); 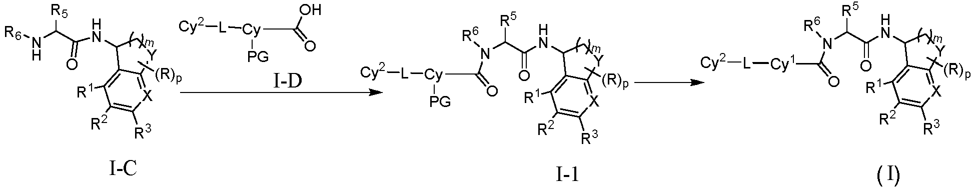 wherein R¹, R², R³, R⁵, R⁶, R, X, Y, L, Cy¹, Cy², m, and p are each independently defined as described herein; Cy is a group resulting from protection of an active group in Cy¹ by PG; PG is a protecting group, e.g., an amino protecting group such as tert-butyloxycarbonyl.

The present disclosure further provides compounds represented by formulas (I-A)-(I-G), racemates, stereoisomers, or hydrochlorides thereof: 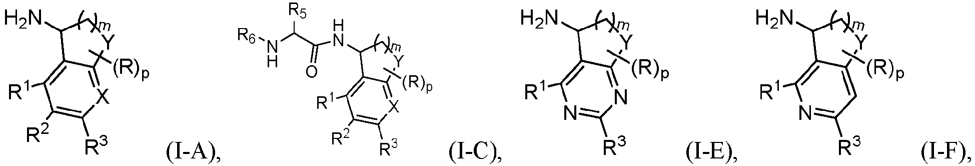 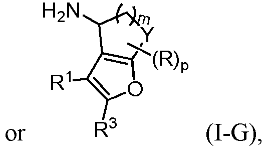
wherein R¹, R², R³, R⁵, R⁶, R, X, Y, m, and p are each independently defined as described herein;
preferably, the compound represented by formula (I-A) is selected from the following compounds:

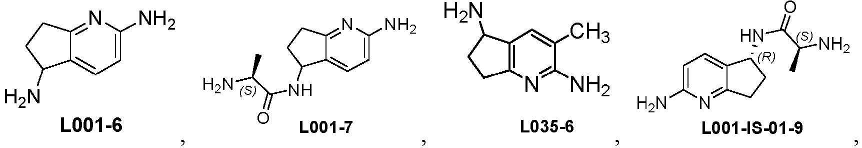

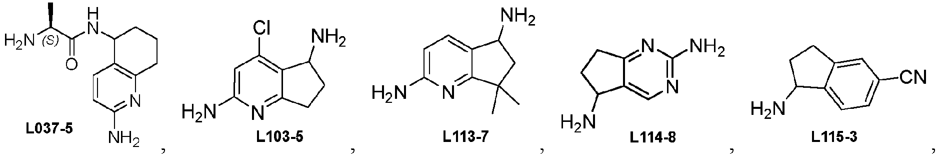

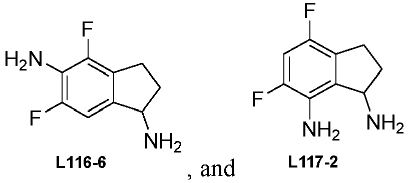

The present disclosure further provides a pharmaceutical composition, which comprises a therapeutically effective amount of at least one of the compounds represented by formula (I'), the racemate, the stereoisomer, the tautomer, the nitrogen oxide or the pharmaceutically acceptable salt thereof.

According to an embodiment of the present disclosure, the pharmaceutical composition further comprises at least one pharmaceutically acceptable excipient.

Those pharmaceutical compositions may be prepared in a manner well known in the art of medicaments and may be administered by a variety of routes depending on whether local or systemic treatment is desired and the region to be treated. The pharmaceutical composition can be administered topically (e.g., by transdermal, dermal, ocular, and mucosal routes, including intranasal, vaginal, and rectal administration routes), pulmonarily (e.g., by inhalation or insufflation of powders or aerosols, including by nebulizer; by intratracheal and intranasal routes), orally, or parenterally. The parenteral administration includes intravenous, intra-arterial, subcutaneous, intraperitoneal or intramuscular injection or infusion; or intracranial (e.g., intrathecal or intracerebroventricular) administration. The pharmaceutical composition may be administered parenterally in a single bolus form, or may be administered by, for example, continuous infusion pump. The pharmaceutical composition and formulation administered topically may include a transdermal patch, an ointment, a lotion, a cream, a gel, a drop, a suppository, a spray, a liquid, a powder formulation, and a pulvis. Conventional pharmaceutical carriers, water, powders or oily bases, thickeners, and the like may be necessary or desirable.

In the preparation of the pharmaceutical composition of the present disclosure, the active ingredient is typically mixed with excipients, diluted with excipients or enclosed within such carriers as capsules, sachets, paper, or containers in other forms. When used as a diluent, the excipient may be a solid, semisolid, or liquid substance that serves as a vehicle, a carrier, or a medium for the active ingredient. Thus, the pharmaceutical composition may be in the following forms: tablets, pills, pulvises, lozenges, sachets, cachets, elixirs, suspensions, emulsions, solution formulations, syrups, aerosols (solid or dissolved in a liquid vehicle); ointments, soft and hard gelatin capsules, suppositories, sterile solutions for injection, and sterile packaged powders containing, for example, up to 10% by weight of active compound.

Certain examples of suitable excipients include lactose, glucose, sucrose, sorbitol, mannitol, starch, acacia, calcium phosphate, alginate, tragacanth, gelatin, calcium silicate, microcrystalline cellulose, polyvinylpyrrolidone, cellulose, water, syrup, and methylcellulose. The excipients may be further selected from: lubricants such as talc, sodium stearate, magnesium stearate, sodium oleate, sodium benzoate, sodium acetate, sodium chloride and mineral oil; wetting agents; emulsifiers and suspending agents; preservatives such as methyl benzoate and hydroxypropyl benzoate; and sweetening agents and flavoring agents. The composition of the present disclosure may be formulated by the methods known in the art so as to provide immediate, sustained, or delayed release of the active ingredient after administration to the patient.

The composition may be formulated in unit dosage form. Each dose contains about 5-1000 mg, more typically about 100-500 mg, of the active ingredient. The term "unit dosage form" refers to physically discrete single dose units suitable for use in human patients and other mammals, each unit containing a predetermined amount of active substance mixed with a suitable pharmaceutical excipient that can produce the desired therapeutic effect by calculation.

The active compound may be effective in a wide range of doses and is generally administered in a pharmaceutically effective amount. However, it will be appreciated that the amount of the compound actually administered is usually determined by a physician, in the light of the relevant circumstances, including the disorder to be treated, the selected route of administration, the compound actually administered; the age, weight, and response of an individual patient; the severity of patient's symptoms and the like.

In preparing solid compositions such as tablets, the main active ingredient is mixed with pharmaceutical excipients to form a solid preformulation composition of a homogeneous mixture comprising the compound of the present disclosure. When describing these preformulation compositions as homogeneous, it means that the active ingredient is generally distributed evenly throughout the composition such that the composition may be readily divided into equally effective unit dosage forms such as tablets, pills and capsules. The solid preformulation is then divided into unit dosage forms of the type described above containing, for example, about 0.1-1000 mg of the active ingredient of the present disclosure.

The tablets or pills of the present disclosure may be coated or compounded to obtain a dosage form affording the advantage of long-acting effect. For example, the tablets or pills contain an inner dose component and an outer dose component, the latter being in the form of an envelope of the former.

The two components may be separated by an enteric coating layer which serves to prevent the disintegration in the stomach to allow the inner component to pass through the duodenum entirely or to be delayed in release. A variety of substances may be used for such enteric coating layers or coatings, including various polymeric acids and mixtures of polymeric acids and such substances as shellac, cetyl alcohol and cellulose acetate.

Liquid forms for oral administration or injection administration in which the compound and pharmaceutical composition of the present disclosure may be incorporated include aqueous solutions, suitable flavoring syrups, aqueous or oil suspensions; and emulsions flavored with edible oils such as cottonseed oil, sesame oil, coconut oil or peanut oil; as well as elixirs and similar pharmaceutical vehicles.

Pharmaceutical compositions for inhalation or insufflation include solution formulations and suspensions in pharmaceutically acceptable aqueous or organic solvents, or mixtures thereof, and pulvises. The liquid or solid compositions may contain suitable pharmaceutically acceptable excipients as described above. In certain embodiments, the pharmaceutical composition is administered by the oral or intranasal or respiratory route for local or systemic effect. The composition may be nebulized using an inert gas. The nebulized solution may be inhaled directly from the nebulizing device or the nebulizing device may be attached to a face mask, tent or an intermittent positive pressure ventilator. The pharmaceutical compositions in the form of a solution, suspension, or powder may be administered orally, or nasally by means of a device which delivers the formulation in a suitable manner.

The amount of the compound or pharmaceutical composition administered to a patient is not fixed and is dependent on the drug administered, the purpose of the administration such as prevention or treatment; the condition of the patient, the route of administration, etc. In therapeutic applications, the composition may be administered to a patient suffering from a disease in an amount sufficient to cure or at least partially arrest the symptoms of the disease and its complications. The effective dose may depend on the disease state being treated and the determination of the attending clinician, the determination depending on factors such as the severity of the disease, the age, weight and general condition of the patient, and the like.

The pharmaceutical composition administered to the patient may be in the form of the pharmaceutical composition as described above. These compositions may be sterilized by conventional sterilization techniques or by filter sterilization. The aqueous solutions may be packaged for use as is, or lyophilized, or the lyophilized formulation is mixed with a sterile aqueous carrier prior to administration. The pH of the compound formulation is usually 3-11, more preferably 5-9, and most preferably 7-8. It will be appreciated that the use of certain excipients, carriers, or stabilizers as described above may result in the formation of a pharmaceutical salt.

The therapeutic dose of the compound of the present disclosure can be determined, for example, according to: the specific use of the treatment, the route of administration of the compound, the health and condition of the patient, and the judgment of the prescriber. The proportion or concentration of the compound of the present disclosure in the pharmaceutical composition may vary depending on a variety of factors including the dose, chemical properties (e.g., hydrophobicity), and the route of administration. For example, the compound of the present disclosure may be provided for parenteral administration by a physiological buffered aqueous solution containing about 0.1-10% w/v of the compound. Certain typical dose ranges from about 1 µg/kg body weight/day to about 1 g/kg body weight/day. In certain embodiments, the dose ranges from about 1 mg/kg body weight/day to about 2000 mg/kg body weight/day, preferably from about 50 mg/kg body weight/day to about 500 mg/kg body weight/day. The dose is likely dependent on such variables as the type and degree of progression of the disease or disorder, the general health condition of a particular patient, the relative biological potency of the compound selected, the excipient formulation, and its route of administration. Effective dose can be extrapolated from dose-response curves derived from *in vitro* or animal model test systems. The present disclosure further provides use of the compound represented by formula (I'), the racemate, the stereoisomer, the tautomer, the nitrogen oxide or the pharmaceutically acceptable salt thereof and the pharmaceutical composition described above for the manufacture of a medicament.

According to an embodiment of the present disclosure, the medicament is used for preventing and/or treating a disease associated with the lectin pathway of complement.

According to an embodiment of the present disclosure, the medicament is a MASP-2 inhibitor.

The present disclosure further provides use of the compound represented by formula (I'), the racemate, the stereoisomer, the tautomer, the nitrogen oxide or the pharmaceutically acceptable salt thereof and the pharmaceutical composition described above for treating and/or preventing a disease associated with the lectin pathway of complement.

The present disclosure further provides a method for treating and/or preventing a disease associated with the lectin pathway of complement, which comprises administering to a patient a therapeutically or prophylactically effective amount of at least one of the compounds represented by formula (I'), the racemate, the stereoisomer, the tautomer, the nitrogen oxide or the pharmaceutically acceptable salt thereof and the pharmaceutical composition described above.

According to an embodiment of the present disclosure, the disease associated with the lectin pathway of complement is selected from thrombotic microangiopathy (TMA), a renal disease, respiratory distress syndrome caused by coronavirus infection, a hematologic system disease, a complication caused by type I or type II diabetes mellitus, a cardiovascular disease or disorder, an inflammatory gastrointestinal disorder, a pulmonary disorder, an ophthalmic disease or disorder, an ocular angiogenic disease or disorder, disseminated intravascular coagulation (DIC) or other complement-mediated coagulation disorders, angiogenesis-dependent cancer or tumor, a peripheral nervous system and/or central nervous system disorder or injury, sepsis or a disorder caused by sepsis, inflammatory response caused by tissue or organ transplantation, ischemia reperfusion injury, graft-versus-host disease, diffuse alveolar hemorrhage (DAH), venous occlusive disease (VOD), a skin disorder, an endocrine disorder, atherosclerosis, or an autoimmune disease; the thrombotic microangiopathy is selected from thrombotic thrombocytopenic purpura (TTP), refractory TTP, Upshaw Schulman syndrome (USS), hemolytic uremic syndrome (HUS), atypical hemolytic uremic syndrome (aHUS), non-Factor H-dependent atypical hemolytic uremic syndrome, aHUS secondary to an infection, plasma therapy-resistant aHUS, TMA secondary to cancer, TMA secondary to chemotherapy, TMA secondary to transplantation, or TMA associated with hematopoietic stem cell transplantation (HSCT-TMA); the renal disease is selected from IgA nephropathy, mesangioproliferative glomerulonephritis, membranous glomerulonephritis, membranoproliferative glomerulonephritis, acute post-infectious glomerulonephritis, C3 glomerulopathy, cryoglobulinemic glomerulonephritis, pauci-immune necrotizing crescentic glomerulonephritis, lupus nephritis, or Henoch-Schonlein purpura nephritis; the respiratory distress syndrome caused by coronavirus infection is selected from respiratory distress syndrome caused by influenza A virus, influenza B virus or influenza C virus and respiratory distress syndrome caused by SARS-COV-2, SARS-COV or MERS-COV; the hematologic system disease is selected from paroxysmal nocturnal hemoglobinuria (PNH), cold agglutinin syndrome, disseminated intravascular coagulation, or autoimmune hemolytic anemia; the inflammatory gastrointestinal disorder is selected from pancreatitis, Crohn's disease, ulcerative colitis, irritable bowel syndrome, or inflammatory bowel disease (IBD); the peripheral nervous system and/or central nervous system disorder or injury is selected from multiple sclerosis (MS), myasthenia gravis (MG), Huntington's disease (HD), amyotrophic lateral sclerosis (ALS), Guillain Barre syndrome, reperfusion following stroke, degenerative discs, cerebral trauma, Parkinson's disease (PD), Alzheimer's disease (AD), Miller-Fisher syndrome, cerebral trauma and/or hemorrhage, traumatic brain injury, demyelination, or meningitis; the autoimmune disease is selected from systemic lupus erythematosus, rheumatoid arthritis, psoriatic arthritis, polymyositis, or psoriasis.

According to an embodiment of the present disclosure, the disease associated with the lectin pathway of complement is selected from a renal disease, thrombotic microangiopathy (TMA), atypical hemolytic uremic syndrome (aHUS), thrombotic microangiopathy (TMA), ischemia reperfusion injury, aHUS secondary to an infection, an ophthalmic disease, an inflammatory gastrointestinal disease, a pulmonary disease, inflammatory response caused by organ or tissue transplantation surgery, a hematological disease, and a complication caused by type I or type II diabetes mellitus, wherein the renal disease is selected from immunoglobulin A nephropathy (IgA nephropathy), C3 glomerulopathy, lupus nephritis, glomerulonephritis, and the like.

### Beneficial Effects

The compounds of the present disclosure can be used for preventing and/or treating a disease associated with the lectin pathway of complement and for manufacturing a medicament for a disease associated with the lectin pathway of complement. The compounds have good inhibitory activity on MASP-2 and excellent pharmacokinetic and pharmacodynamic activity.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should be construed as being within the scope of the specification and/or the claims of the present application.

The terms "Cₙ₋ₘ" and "Cₙ-Cₘ" (wherein n and m are integers) denote groups comprising n to m carbon atoms. Examples include
C₁₋₆, C₁₋₃, and the like. The terms are intended to explicitly disclose each member of the range, i.e., Cₙ, Cₙ₊₁, Cₙ₊₂...Cₘ₋₂, Cₘ₋₁, and Cₘ. For example, C₁₋₆ is intended to disclose C₁, C₂, C₃, C₄, C₅, and C₆. "Cₙ₋ₘ" has the same meaning as "Cₙ-Cₘ".

The term "n-membered" (wherein n is an integer) generally describes a situation that the number of ring-forming atoms is n.

The term "n- to m-membered" (wherein n and m are integers) describes a situation that the number of ring-forming atoms ranges from n to m. For example, piperidyl is an example of a 6-membered heterocycloalkyl ring, pyrazolyl is an example of a 5-membered heteroaryl ring, and pyridinyl is an example of a 6-membered heteroaryl ring.

Unless otherwise stated, the numerical ranges described in the specification and claims shall be construed as at least including each specific integer value therein. For example, the numerical range of "1-20" shall be construed as including each integer value in the numerical range "1-20", i.e., 1, 2, 3, 4, 5, 6, 7, 8, 9, 10, 11, 12, 13, 14, 15, 16, 17, 18, 19, and 20.

Depending on their molecular structure, the compounds of the present disclosure may be chiral (having one or more stereogenic centers). Unless otherwise stated, all stereoisomers, such as enantiomers and diastereoisomers, are desirable. These compounds may therefore be present in a racemic or optically active form. The compounds of the present disclosure encompass isomers with each chiral carbon in R or S configuration, or mixtures and racemates thereof. The compounds of the present disclosure or intermediates thereof may be separated into enantiomers by chemical or physical methods well known to those skilled in the art, or used in such form for synthesis. In the case of racemic amines, diastereoisomers are prepared from mixtures by reaction with optically active resolving agents. Examples of suitable resolving agents are optically active acids such as R- or S-tartaric acid, diacetyltartaric acid, dibenzoyltartaric acid, mandelic acid, malic acid, lactic acid, suitable N-protected amino acids (e.g., N-benzoylproline or N-benzenesulfonylproline), or various optically active camphorsulfonic acids. Enantiomeric resolution by chromatography can be advantageously conducted with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvents containing water or alcohol, for example, hexane/isopropanol/acetonitrile. The corresponding stable isomers can be separated according to known methods, such as extraction, filtration, or column chromatography.

In some embodiments, the compounds of the present disclosure have the (*R*)-configuration. In other embodiments, the compounds have the (*S*)-configuration. In compounds having more than one chiral center, unless otherwise stated, each chiral center in the compounds can independently be (*R*) or (*S*). The compounds described herein may further include tautomeric forms. The tautomeric forms result from the exchange of a single bond with an adjacent double bond and the concomitant migration of protons. The tautomeric forms include prototropic tautomers, which are isomeric protonated states with identical empirical formula and total charge. Examples of prototropic tautomers include keto-enol pair, amide-imidic acid pair, lactam-lactim pair, enamine-imine pair, and ring forms in which a proton may occupy two or more positions of a heterocyclic system, such as 1H- and 3H-imidazole, 1H-, 2Hand 4H-1,2,4-triazole, 1H- and 2H-isoindole, and 1H- and 2H-pyrazole. The tautomeric forms can be in equilibrium or sterically locked into one form by appropriate substitution. The present disclosure is intended to include all such tautomers of the compounds.

The compounds described herein may further include all isotopes of atoms present in the intermediates or final compounds. Isotopes include those atoms having the same atomic number but different mass numbers. For example, isotopes of hydrogen include tritium and deuterium.

The term "pharmaceutically acceptable salt" refers to a salt prepared with the compound of the present disclosure and a relatively non-toxic, pharmaceutically acceptable acid or base. When the compounds of the present disclosure contain a relatively acidic functional group, a base addition salt can be obtained by contacting the original form of such a compound with a sufficient amount of pharmaceutically acceptable base in a pure solution or a suitable inert solvent. Pharmaceutically acceptable base addition salts include, but are not limited to: lithium salt, sodium salt, potassium salt, calcium salt, aluminum salt, magnesium salt, zinc salt, bismuth salt, ammonium salt, and diethanolamine salt. When the compounds of the present disclosure contain a relatively basic functional group, an acid addition salt can be obtained by contacting the original form of such a compound with a sufficient amount of pharmaceutically acceptable acid in a pure solution or a suitable inert solvent. The pharmaceutically acceptable acid includes inorganic acids including, but not limited to: hydrochloric acid, hydrobromic acid, hydroiodic acid, nitric acid, carbonic acid, phosphoric acid, phosphorous acid, sulfuric acid, and the like. The pharmaceutically acceptable acid includes organic acids including, but not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, oleic acid, tannic acid, pantothenic acid, bitartrate, ascorbic acid, gentisic acid, fumaric acid, gluconic acid, saccharic acid, formic acid, ethanesulfonic acid, embonic acid (i.e., 4,4'-methylene-bis(3-hydroxy-2-naphthoic acid)), amino acids (e.g., glutamic acid, arginine), and the like. When the compounds of the present disclosure contain relatively acidic and relatively basic functional groups, they may be converted to base addition salts or acid addition salts. Details can be found in Berge et al., "Pharmaceutical Salts ", Journal of Pharmaceutical Science 66: 1-19 (1977), or, Handbook of Pharmaceutical Salts: Properties, Selection, and Use (P. Heinrich Stahl and Camile G. Wermuth, ed., Wiley-VCH, 2002). In some embodiments, the "pharmaceutically acceptable salt" is hydrochloride.

The term "halogen" or "halo" refers to fluorine, chlorine, bromine, and iodine.

The term "C₁₋₁₀ alkyl" refers to a linear or branched saturated monovalent hydrocarbyl group having 1 to 10 carbon atoms. For example, it refers to linear and branched alkyl groups having 1, 2, 3, 4, 5, 6, 7, 8, 9, or 10 carbon atoms. The term "C₁₋₆ alkyl" refers to a linear or branched alkyl group having 1, 2, 3, 4, 5, or 6 carbon atoms. The term "C₁₋₃ alkyl" refers to a linear or branched alkyl group having 1, 2, or 3 carbon atoms. The alkyl group includes, but is not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof. The term "alkoxy" refers to a group having the formula "-O- alkyl", wherein the alkyl is as defined above. The term "C₁₋₆ alkoxy" referes to a group in which the alkyl group has 1 to 6 carbon atoms. The alkoxy includes, but is not limited to, methoxy, ethoxy, propoxy (e.g., n-propoxy and isopropoxy), tert-butoxy, and the like.

The term "haloalkyl" refers to the alkyl as defined above which is substituted with one or more halogens as defined above. The haloalkyl includes, but is not limited to, monofluoromethyl, difluoromethyl, trifluoromethyl, trichloromethyl, 2,2,2-trifluoroethyl, 1-fluoromethyl-2-fluoroethyl, 3-bromo-2-fluoropropyl, 1-bromomethyl-2-bromoethyl, and the like.

The term "haloalkoxy" refers to the alkoxy as defined above with the alkyl portion substituted with one or more halogens as defined above. The haloalkoxy includes, but is not limited to, trifluoromethoxy, difluoromethoxy, pentafluoroethoxy, and the like.

The term "C₂₋₆ alkenyl" refers to a linear or branched monovalent hydrocarbyl group comprising 2 to 6 carbon atoms and at least one carbon-carbon double bond. The alkenyl includes, but is not limited to, ethenyl, n-propenyl, isopropenyl, n-butenyl, sec-butenyl, and the like.

The term "C₂₋₆ alkynyl" refers to a linear or branched monovalent hydrocarbyl group comprising 2 to 6 carbon atoms and at least one carbon-carbon triple bond. The alkynyl includes, but is not limited to, ethynyl, 1-propynyl, 2-propynyl, 3-butynyl, 2-pentynyl, and 1-butynyl.

The term "C₁₋₆ aminoalkyl" refers to C₁₋₆ alkyl substituted with one or more amino groups. The term "C₁₋₃ aminoalkyl" refers to C₁₋₃ alkyl substituted with one or more amino groups, and the aminoalkyl includes, but is not limited to, aminomethyl, i.e., -CH₂NH₂, and aminoethyl, i.e., -CH₂CH₂NH₂.

The term "C₃₋₁₀ cycloalkyl" refers to a saturated monovalent or polyvalent monocyclic, bicyclic, or tricyclic hydrocarbyl group having 3, 4, 5, 6, 7, 8, 9, or 10 carbon atoms. The bicyclic and tricyclic hydrocarbyl groups include bridged rings, fused rings, and spiro rings. The C₃₋₁₀ cycloalkyl may be selected from C₃₋₆ cycloalkyl or C₃₋₄ cycloalkyl. The C₃₋₁₀ cycloalkyl may be a monocyclic hydrocarbyl group, such as cyclopropyl, cyclobutyl, cyclopentyl, cyclohexyl, cycloheptyl, cyclooctyl, cyclononyl or cyclodecyl, or a bicyclic hydrocarbyl group such as bornyl, hexahydroindolyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, or a tricyclic hydrocarbyl group such as adamantyl.

The term "3- to 12-membered heterocycloalkyl" refers to a saturated monovalent or polyvalent monocyclic, bicyclic, or tricyclic system comprising 3 to 12 ring atoms, and the ring atoms comprise 1, 2, 3, or 4 heteroatoms independently selected from O, S, and N, wherein N and S may also be optionally oxidized to various oxidized forms to form nitrogen oxides, -S(=O)-, or -S(=O)₂-. The "3-to 12-membered heterocycloalkyl" is selected from 3- to 6-membered heterocycloalkyl or 6- to 11-membered heterocycloalkyl. The heterocycloalkyl may be attached to the rest of the molecule through any one of the carbon or nitrogen atoms (if present) on its ring. The heterocycloalkyl may include, but is not limited to: azetidinyl, pyrrolidinyl, piperidyl, 6-azaspiro[2.5]octyl, diazaspiro[3,5]nonyl, 2,6-diazaspiro[3,4]octyl, 7-azaspiro[3.5]nonyl, 8-azaspiro[4.5]decyl, 3-azaspiro[5.5]undecyl, or the like.

The term "3- to 12-membered heterocyclyl" refers to a saturated or unsaturated non-aromatic monovalent or polyvalent ring system; for example, it is a monocyclic ring system comprising 4, 5, 6, or 7 ring atoms, a bicyclic ring system comprising 7, 8, 9, 10, 11, or 12 ring atoms, or a tricyclic ring system comprising 10, 11, or 12 ring atoms, and its ring atoms comprise at least one, e.g., 1, 2, 3, 4, 5, or 6 heteroatoms independently selected from O, S, and N, wherein N and S may also be optionally oxidized to various oxidized forms to form nitrogen oxides, -S(=O)-, or -S(=O)₂-. The bicyclic and tricyclic ring systems include fused rings, bridged rings, or spiro rings. The 3- to 12-membered heterocyclyl may be selected from "3- to 10-membered heterocyclyl", "5- to 12-membered heterocyclyl", or "6- to 11-membered heterocyclyl". The term "3- to 10-membered heterocyclyl" refers to a saturated or unsaturated non-aromatic ring or ring system comprising 3 to 10 ring atoms, and the ring atoms comprise at least one heteroatom selected from O, S, and N. The term "5- to 12-membered heterocyclyl" refers to a saturated or unsaturated non-aromatic ring or ring system comprising 5 to 12 ring atoms, and the ring atoms comprise at least one heteroatom selected from O, S, and N. The term "6- to 11-membered heterocyclyl" refers to a saturated or unsaturated non-aromatic ring or ring system comprising 6 to 11 ring atoms, and the ring atoms comprise at least one heteroatom selected from O, S, and N. In some embodiments, the heterocyclyl may be heterocycloalkyl. The heterocyclyl may be attached to the rest of the molecule through any one of the carbon or nitrogen atoms (if present) on its ring. For example, when 3- to 12-membered heterocyclyl is selected from piperidyl, the nitrogen atom on the piperidyl ring and the carbon atom at the para-position may be attach to the other groups. The heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl, and pyrrolinyl; 6-membered rings such as tetrahydropyranyl, piperidyl, morpholinyl, dithianyl, thiomorpholinyl, piperazinyl, or trithianyl; or 7-membered rings such as diazepanyl. Optionally, the heterocyclyl may be benzo-fused. The heterocyclyl may be bicyclic, for example, but not limited to, 6-azaspiro[2.5]octyl, diazaspiro[3,5]nonyl, 2,6-diazaspiro[3,4]octyl, 7-azaspiro[3.5]nonyl, 8-azaspiro[4.5]decyl or 3-azaspiro[5.5]undecyl, hexahydrocyclopenta[c]pyrrol-2(1H)-yl ring, hexahydropyrrolo[1,2-a]pyrazin-2(1H)-yl ring, 4-azaspiro[2.5]octyl, 1,2-dihydropyridinyl, and 1,4-dihydropyrazinyl. The heterocyclyl may be partially unsaturated, i.e., it may contain one or more double bonds, for example, but not limited to, dihydrofuranyl, dihydropyranyl, 2,5-dihydro-1H-pyrrolyl, 4H-[1,3,4]thiadiazinyl, 1,2,3,5-tetrahydrooxazolyl, or 4H-[1,4]thiazinyl, or it may be benzo-fused, for example, but not limited to, dihydroisoquinolyl.

The term "C₆₋₁₀ aryl" refers to an aromatic monovalent monocyclic, bicyclic, or tricyclic hydrocarbon ring having 6, 7, 8, 9, or 10 carbon atoms, in particular a ring having 6 carbon atoms ("C₆ aryl"), such as phenyl, or a ring having 9 carbon atoms ("C₉ aryl"), such as indanyl or indenyl, or a ring having 10 carbon atoms ("C₁₀ aryl"), such as naphthyl. When the C₆₋₁₀ aryl is substituted, it may be monosubstituted or polysubstituted. In addition, the substitution site is not limited and may be, for example, ortho-substitution, para-substitution, or meta-substitution.

The term "5- to 10-membered heteroaryl" refers to a monovalent or polyvalent monocyclic, bicyclic, or tricyclic aromatic ring system comprising 5 to 10 ring atoms, and the ring atoms comprise 1, 2, 3, 4, or 5 heteroatoms independently selected from N, O, and S. The term "5- to 8-membered heteroaryl" refers to a monovalent or polyvalent monocyclic, bicyclic, or tricyclic aromatic ring system comprising 5, 6, 7, or 8 ring atoms, and in particular, it comprises 5 or 6 carbon atoms and 1 to 3, preferably 1 or 2 heteroatoms independently selected from N, O, and S. The bicyclic and tricyclic ring systems include fused rings. In some embodiments, it may be benzo-fused. When the 5- to 10-membered heteroaryl is substituted, it may be monosubstituted or polysubstituted. In addition, the substitution site is not limited. For example, hydrogen attached to the carbon atom on the heteroaryl ring may be substituted, or hydrogen attached to the heteroatom on the heteroaryl ring may be substituted. The heteroaryl may include, but is not limited to: pyridinyl, pyrazinyl, pyrimidinyl, triazinyl, pyridazinyl, thienyl, furanyl, pyrrolyl, imidazolyl, pyrazolyl, thiazolyl, indoxyl, benzothiazolyl, benzofuranyl, or benzimidazolyl.

The term "spiro rings" refers to a ring system in which two rings share 1 ring-forming atom.

The term "fused rings" refers to a ring system in which two rings share 2 ring-forming atoms.

The term "bridged rings" refers to a ring system in which two rings share more than 2 ring-forming atoms.

The compounds of the present disclosure may be present in the form of a solvate (e.g., hydrate), and the compounds of the present disclosure contain a polar solvent as a structural element of the crystal lattice of the compound, particularly, for example, water, methanol, or ethanol. The amount of the polar solvent, especially water, may be present in a stoichiometric or non-stoichiometric ratio.

The term "patient" refers to any animal including mammals, preferably mice, rats, other rodents, rabbits, dogs, cats, pigs, cattle, sheep, horses, or primates, and most preferably humans.

The term "therapeutically effective amount" refers to the amount of the active compound or drug that causes a biological or medical response that researchers, veterinarians, physicians or other clinicians are looking for in tissues, systems, animals, individuals or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition (i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms). The therapeutically effective amount can be estimated initially from cell culture assays, or the initial dose can be estimated from *in vivo* data. Using these preliminary guidance, those of ordinary skill in the art can determine effective dose for humans. In addition, the toxicity and the therapeutic efficacy of the compounds described herein can also be determined by standard pharmaceutical procedures in cell cultures or experimental animals, e.g., by the measurement of LD₅₀ and ED₅₀.

It should be appreciated by those skilled in the art that resolved chiral compounds can be distinguished by their retention time in the chiral chromatography column. If absolute configurations of the compounds are listed in the examples, they are not meant to correspond one-to-one to the compound numbers, but merely to illustrate two existence forms of absolute configurations. The absolute configuration corresponding to the compound number is subject to the absolute configuration objectively corresponding to the specific retention time.

### DETAILED DESCRIPTION OF THE PREFERRED EMBODIMENT

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the content described above of the present disclosure are encompassed within the protection scope of the present disclosure.

Unless otherwise stated, the starting materials and reagents used in the following examples are all commercially available products or can be prepared by using known methods.

Unless otherwise stated, the terms used in the following detailed description of the experiments represent the following reagents, respectively:
NaBH₄: sodium borohydride; PPh₃: triphenylphosphine; DIAD: diisopropyl azodicarboxylate; m-CPBA: m-chloroperoxybenzoic acid; PyBOP-PF₆: bromotripyrrolidinophosphonium hexafluorophosphate; EDCI: 1-(3-dimethylaminopropyl)-3-ethylcarbodiimide hydrochloride; HOBT: 1-hydroxybenzotriazole; DIEA: N,N-diisopropylethylamine; LiHMDS: lithium bis(trimethylsilyl)amide; LiEt₃BH: lithium triethylborohydride; BF₃-Et₂O: boron trifluoride diethyl ether coordination complex; Pd(Ph₃P)₄: tetrakis(triphenylphosphine)palladium(0); Rh(PPh₃)₃Cl: tris(triphenylphosphine)rhodium chloride; Et₃SiH: triethylsilane; LAH: lithium aluminum hydride; Et₂Zn: diethyl zinc; KHF₂: potassium hydrogen fluoride; PBr₃: phosphorus tribromide; SEM-Cl: 2-(trimethylsilyl)ethoxymethyl chloride; Cu(OAc)₂: copper(II) acetate; EtOAc: ethyl acetate; TEA: triethylamine; THF: tetrahydrofuran; TBAF: tetrabutylammonium fluoride; T₃P: 1-propylphosphoric cyclic anhydride.

### <Preparation Examples>

### Example 1: Synthesis of (2R,4S)-N-((2S)-1-((2-amino-6,7-dihydro-5H-cyclopenta[b]pyridin-5-yl) amino)-1-oxopropan-2-yl)-4-(4-fluorobenzyl)pyrrolidine-2-carboxamide (Compound L001) Hydrochloride

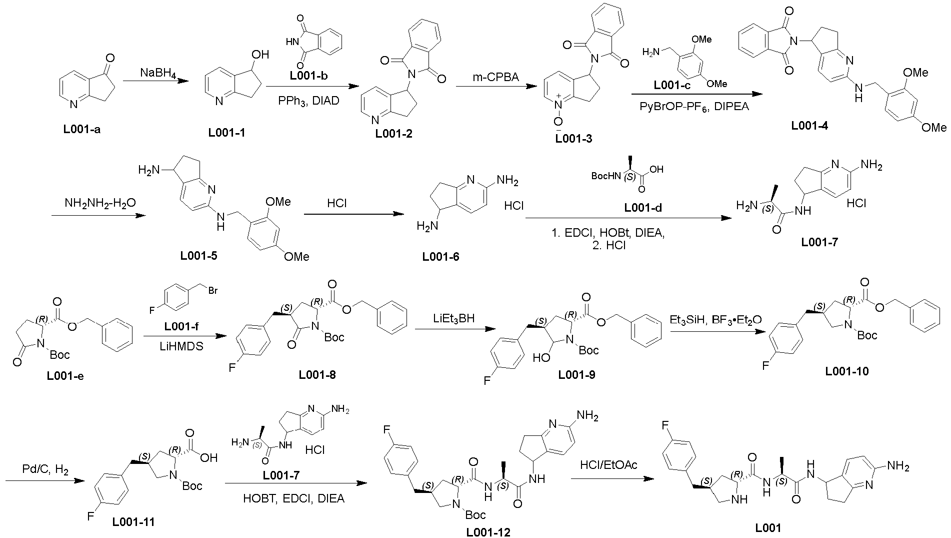

### 1.1: Synthesis of compound L001-1

L001-a (4.0 g, 30.40 mmol) was dissolved in methanol (60 mL) at room temperature, and the mixture was cooled to 0 °C. Sodium borohydride (3.4 g, 91.20 mmol) was added in batches. The reaction liquid was stirred at 0 °C for 0.5 h and then warmed to 25 °C and reacted for 2 h. The reaction liquid was quenched with water (10 mL) and concentrated under reduced pressure to give a crude product. The crude product was purified by silica gel column chromatography to give compound L001-1 (3.8 g, yield: 93.60%).

### 1.2: Synthesis of compound L001-2

L001-1 (3.8 g, 28.11 mmol), triphenylphosphine (8.8 g, 33.74 mmol), and L001-b (4.6 g, 33.74 mmol) were dissolved in tetrahydrofuran (40 mL) at room temperature, and the mixture was cooled to 0 °C. Diisopropyl azodicarboxylate (6.82 g, 33.74 mmol) was then added dropwise and slowly under nitrogen atmosphere. After the dropwise addition was completed, the reaction liquid was warmed to room temperature and reacted for 2 h. The reaction liquid was directly concentrated, and the residue was added with water (100 mL) for dilution. The aqueous phase was adjusted to pH = 3-4 with an diluted aqueous hydrochloric acid solution (1 M) and then washed with ethyl acetate (50 mL × 3). The aqueous phase was adjusted to pH = 8-9 with a saturated aqueous sodium bicarbonate solution and then extracted with dichloromethane (50 mL × 3). The organic phase was dried over anhydrous sodium sulfate and filtered, and the filtrate was concentrated under reduced pressure. The residue was purified by silica gel column chromatography to give compound L001-2 (3.6 g, yield: 48.45%). LC-MS: [M+H]⁺ = 265.05.

### 1.3: Synthesis of compound L001-3

L001-2 (3.6 g, 13.62 mmol) was dissolved in dichloromethane (70 mL), and m-chloroperoxybenzoic acid (3.06 g, 17.71 mmol) was added in batches at 0 °C. The reaction liquid was warmed to 25 °C and reacted for 2 h. The reaction liquid was diluted with dichloromethane (200 mL) and then washed with a saturated aqueous sodium bicarbonate solution (50 mL × 3). The organic phase was dried over anhydrous sodium sulfate and filtered, and the filtrate was concentrated under reduced pressure to give compound L001-3 (3.7 g, crude product), which could be used directly in the next step. LC-MS: [M+H]⁺ = 280.95.

### 1.4: Synthesis of compound L001-4

L001-3 (2.6 g, 9.28 mmol) was dissolved in dichloromethane (50 mL) at room temperature, and L001-c (2.0 g, 12.06 mmol), bromotripyrrolidinophosphonium hexafluorophosphate (7.8 g, 16.70 mmol), and N,N-diisopropylethylamine (3.0 g, 23.19 mmol) were sequentially added. The reaction liquid was stirred at room temperature for 12 h. The reaction liquid was directly concentrated under reduced pressure, and the residue was purified by silica gel column chromatography to give compound L001-4 (2.0 g). LC-MS: [M+H]⁺ = 430.00.

### 1.5: Synthesis of compound L001-5

L001-4 (2.0 g, 4.66 mmol) was dissolved in ethanol (20 mL), and hydrazine hydrate (20 mL, 85%) was added. The reaction liquid was then stirred at 25 °C for 12 h. The reaction liquid was concentrated under reduced pressure, and the residue was added with water (20 mL) for dilution and extracted with dichloromethane (20 mL × 3). The organic phase was dried over anhydrous sodium sulfate and filtered, and the filtrate was concentrated under reduced pressure to give compound L001-5 (1.5 g, crude product), which could be used directly in the next step. LC-MS: [M+H]⁺ = 300.05.

### 1.6: Synthesis of compound L001-6

L001-5 (1.5 g, 5.01 mmol) was dissolved in tetrahydrofuran (20 mL) at room temperature, and concentrated hydrochloric acid (20 mL, 37%) was added. The reaction liquid was then warmed to 70 °C and stirred for 1 h. The reaction liquid was concentrated under reduced pressure, and the residue was added with ethyl acetate/methanol (20 mL, 10/1 (v/v)). The mixture was stirred at room temperature for 1 h. The mixture was filtered, and the filter cake was dried under reduced pressure to give compound L001-6 hydrochloride (600 mg, yield: 64.52%). LC-MS: [M+H]⁺ = 150.20.

### 1.7: Synthesis of compound L001-7

L001-d (1.5 g, 8.08 mmol) was dissolved in N,N-dimethylformamide (20 mL), and 1-(3-dimethylaminopropyl)-3-ethylcarbodiimide hydrochloride (1.7 g, 8.89 mmol) and 1-hydroxybenzotriazole (1.2 g, 8.89 mmol) were sequentially added. The mixture was reacted at 25 °C for 10 min, and then a solution of N,N-diisopropylethylamine (5.2 g, 40.40 mmol) and L001-6 (1.5 g, 8.08 mmol) in N,N-dimethylformamide (10 mL) was added. The reaction liquid was reacted at 25 °C for 30 min. The reaction liquid was poured into water (30 mL) and extracted with ethyl acetate (30 mL × 3). The organic phase was washed with a saturated aqueous ammonium chloride solution (20 mL × 3), dried over anhydrous sodium sulfate, and filtered, and the filtrate was concentrated under reduced pressure. The residue was purified by silica gel column chromatography to give an intermediate. The intermediate (1.5 g, 4.68 mmol) was dissolved in dichloromethane (20 mL), and a solution of hydrogen chloride in 1,4-dioxane (20 mL, 4 M) was added. The reaction liquid was reacted at 25 °C for 30 min. The reaction liquid was directly concentrated under reduced pressure, and the residue was added with dichloromethane (10 mL). The mixture was stirred at room temperature for 30 min and filtered, and the filter cake was dried under reduced pressure to give compound L001-7 hydrochloride (1.3 g), which was used directly in the next step. LC-MS: [M+H]⁺ = 221.15.

### 1.8: Synthesis of compound L001-8

L001-e (1.0 g, 3.13 mmol) was dissolved in tetrahydrofuran (15 mL) at room temperature, and then the mixture was cooled to -70 °C. Lithium bis(trimethylsilyl)amide (3.8 mL, 3.80 mmol, 1 M in tetrahydrofuran) was added dropwise and slowly under nitrogen atmosphere. After the dropwise addition was completed, the mixture was stirred at -70 °C for 0.5 h. L001-f (0.7 g, 3.76 mmol) was then added dropwise and slowly. After the dropwise addition was completed, the reaction liquid was stirred at -70 °C for another 2 h. The reaction liquid was quenched with a saturated aqueous ammonium chloride solution (10 mL) at -70 °C, and after being warmed to room temperature, the system was extracted with ethyl acetate (20 mL × 3). The combined organic phases were dried over anhydrous sodium sulfate and filtered, and the filtrate was concentrated under reduced pressure. The residue was purified by silica gel column chromatography to give compound L001-8 (800 mg, yield: 59.70%).

### 1.9: Synthesis of compound L001-9

L001-8 (800 mg, 1.87 mmol) was dissolved in tetrahydrofuran (10 mL) at room temperature. The reaction liquid was cooled to -70 °C, and lithium triethylborohydride (2 mL, 2.0 mmol, 1 M in tetrahydrofuran) was added dropwise and slowly under nitrogen atmosphere. After the dropwise addition was completed, the reaction liquid was reacted at -70 °C for 0.5 h. The reaction liquid was quenched with a saturated aqueous sodium bicarbonate solution (10 mL) at -70 °C and then extracted with ethyl acetate (10 mL × 3). The organic phase was dried over anhydrous sodium sulfate and filtered, and the filtrate was concentrated under reduced pressure to give compound L001-9 (800 mg, crude product). LC-MS: [M-OH]⁺ = 412.05.

### 1.10: Synthesis of compound L001-10

L001-9 (800 mg, 1.86 mmol) was dissolved in dichloromethane (15 mL) at room temperature, and the reaction liquid was cooled to -70 °C. Triethylsilane (259 mg, 2.34 mmol) and boron trifluoride diethyl ether coordination complex (332 mg, 2.34 mmol) were added dropwise and slowly under nitrogen atmosphere. After the dropwise addition was completed, the reaction liquid was stirred at -70 °C for 0.5 h. The reaction liquid was supplemented with triethylsilane (259 mg, 2.34 mmol) and boron trifluoride diethyl ether coordination complex (332 mg, 2.34 mmol) and stirred at -70 °C for another 1 h. The reaction liquid was quenched by adding a saturated aqueous sodium bicarbonate solution (10 mL) at -70 °C and extracted with dichloromethane (20 mL × 3). The combined organic phases were dried over anhydrous sodium sulfate and filtered, and the filtrate was concentrated under reduced pressure. The residue was purified by silica gel column chromatography to give compound L001-10 (500 mg, yield: 64.94%).

### 1.11: Synthesis of compound L001-11

At room temperature, L001-10 (500 mg, 1.21 mmol) was dissolved in methanol (20 mL), wet palladium on carbon (100 mg, 10% palladium content) was added, and the reaction liquid was purged with hydrogen three times and then stirred at room temperature for 4 h under hydrogen atmosphere. The reaction liquid was directly filtered, and the filtrate was concentrated under reduced pressure to give compound L001-11 (410 mg, crude product), which could be used directly in the next step. LC-MS: [M-tBu+H]⁺ = 268.05.

### 1.12: Synthesis of compound L001-12 hydrochloride

L001-11 (70 mg, 0.22 mmol) was dissolved in N,N-dimethylformamide (2 mL) at room temperature, and 1-(3-dimethylaminopropyl)-3-ethylcarbodiimide hydrochloride (50 mg, 0.26 mmol) and 1-hydroxybenzotriazole (35 mg, 0.26 mmol) were added. The mixture was reacted at 25 °C for 10 min, and then a solution of N,N-diisopropylethylamine (141 mg, 1.09 mmol) and L001-7 (67 mg, 0.26 mmol) in N,N-dimethylformamide (2 mL) was added. The reaction system was reacted at 25 °C for 30 min. The reaction liquid was poured into water (10 mL) and extracted with ethyl acetate (10 mL × 3). The combined organic phases were washed with a saturated aqueous ammonium chloride solution (10 mL × 3), dried over anhydrous sodium sulfate and filtered, and the filtrate was concentrated under reduced pressure. The residue was purified by preparative high performance liquid chromatography (acidic condition) to give compound L001-12 hydrochloride (50 mg, yield: 61.52%). LC-MS: [M+H]⁺ = 526.10.

### 1.13: Synthesis of compound L001 hydrochloride

The L001-12 hydrochloride (50 mg, 0.10 mmol) was dissolved in dichloromethane (2 mL), and a solution of hydrogen chloride in ethyl acetate (2 mL, 4 M) was added. The reaction liquid was stirred at room temperature for 2 h. A solid was precipitated. The reaction liquid was filtered, and the filter cake was dissolved in deionized water (10 mL) and lyophilized to give compound L001 hydrochloride (24.3 mg).

¹H NMR (400 MHz, DMSO-*d₆*): δ 10.52 (s, 1H), 8.89 (s, 1H), 8.50 - 8.64 (m, 2H), 8.03 (s, 2H), 7.71 - 7.64 (m, 1H), 7.48 - 7.28 (m, 3H), 7.20 - 7.11 (m, 2H), 6.84 (d, *J* = 8.0 Hz, 1H), 5.17 - 5.12 (m, 1H), 4.38 (s, 1H), 4.32 - 4.19 (m, 1H), 3.36 - 3.26 (m, 1H), 3.09 - 2.98 (m, 1H), 2.94 - 2.86 (m, 2H), 2.79 - 2.59 (m, 2H), 2.49- 2.40 (m, 2H), 2.10 - 2.02 (m, 1H), 1.98 - 1.86 (m, 3H), 1.23 (d, *J=* 8.0 Hz, 3H); LC-MS: [M+H]⁺ = 426.10.

### Example 2: Synthesis of (2R,4S)-4-([1,1'-biphenyl]-4-ylmethyl)-N-((2S)-1-((2-amino-6,7-dihydro-SH-cyclopenta[b]pyridin-5-yl)amino)-1-oxopropan-2-yl)pyrrolidine-2-carboxamide (Compound L002) and (2R,4S)-4-([1,1'-biphenyl]-4-ylmethyl)-N-((S)-1-(((S)-2-amino-6,7-dihydro-5H-cyclopenta [b]pyridin-5-yl)amino)-1-oxopropan-2-yl)pyrrolidine-2-carboxamide (Compound L002-IS-01 or L002-IS-02) and (2R,4S)-4-([1,1'-biphenyl]-4-ylmethyl)-N-((S)-1-(((R)-2-amino-6,7-dihydro-5H-cyclopenta[b]pyridin-5-yl)amino)-1-oxopropan-2-yl)pyrrolidine-2-carboxamide (Compound L002-IS-02 or L002-IS-01)

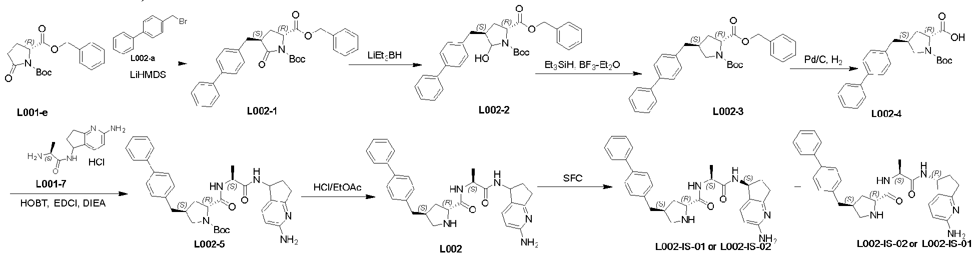

### 2.1: Synthesis of compound L002-1

L001-e (2.0 g, 6.20 mmol) was dissolved in tetrahydrofuran (25 mL) at room temperature, and the reaction liquid was cooled to -78 °C. Lithium bis(trimethylsilyl)amide (7.0 mL, 7.0 mmol, 1 M in tetrahydrofuran) was added dropwise under nitrogen atmosphere. After the dropwise addition was completed, the reaction liquid was stirred at -78 °C for 0.5 h. L002-a (1.8 g, 7.44 mmol) was then added dropwise, and after the dropwise addition was completed, the reaction liquid was stirred at -78 °C for another 2 h. At this temperature, the reaction liquid was quenched by adding water (50 mL), and after being warmed to room temperature, the system was extracted with ethyl acetate (40 mL × 3). The combined organic phases were dried over anhydrous sodium sulfate and filtered, and the filtrate was concentrated under reduced pressure. The residue was purified by silica gel column chromatography to give compound L002-1 (1.8 g, yield: 58%).

### 2.2: Synthesis of compound L002-2

L002-1 (1.8 g, 3.71 mmol) was dissolved in tetrahydrofuran (30 mL) at room temperature, and the reaction liquid was cooled to -78 °C. Lithium triethylborohydride (4.1 mL, 4.10 mmol, 1 M in tetrahydrofuran) was added dropwise and slowly under nitrogen atmosphere. After the dropwise addition was completed, the reaction liquid was reacted at -78 °C for 0.5 h. At this temperature, the reaction liquid was quenched by adding water (30 mL), and after being warmed to room temperature, the system was extracted with ethyl acetate (30 mL × 3). The combined organic phases were dried over anhydrous sodium sulfate and filtered, and the filtrate was concentrated under reduced pressure to give compound L002-2 (1.4 g, crude product), which was used directly in the next step.

### 2.3: Synthesis of compound L002-3

L002-2 (1.4 g, crude product) was dissolved in dichloromethane (20 mL) at room temperature, and the solution was cooled to -78 °C. Triethylsilane (400 mg, 3.44 mmol) and boron trifluoride diethyl ether coordination complex (488 mg, 3.44 mmol) were added dropwise and slowly under nitrogen atmosphere. The reaction liquid was stirred at -78 °C for 0.5 h. The reaction liquid was then supplemented with triethylsilane (400 mg, 3.44 mmol) and boron trifluoride diethyl ether coordination complex (488 mg, 3.44 mmol) and stirred at -78 °C for another 2 h. The reaction liquid was quenched by adding a saturated aqueous sodium bicarbonate solution (30 mL) at -78 °C, and after being warmed to room temperature, the system was extracted with ethyl acetate (30 mL × 3). The combined organic phases were dried over anhydrous sodium sulfate and filtered, and the filtrate was concentrated under reduced pressure. The residue was purified by silica gel column chromatography to give compound L002-3 (900 mg, yield: 50.68%). LC-MS: [M+H]⁺ = 472.59.

### 2.4: Synthesis of compound L002-4

At room temperature, L002-3 (900 mg, 1.91 mmol) was dissolved in ethanol (15 mL) , wet palladium on carbon (100 mg, 10% palladium content) was added, and the reaction liquid was stirred for 3 h under hydrogen atmosphere. The reaction liquid was filtered through celite, and the filtrate was concentrated under reduced pressure to give compound L002-4 (780 mg, crude product), which was used directly in the next step. LC-MS: [M-tBu+H]⁺ = 326.15.

### 2.5: Synthesis of compound L002-5

L002-4 (400 mg, crude product) was dissolved in N,N-dimethylformamide (5 mL), and 1-(3-dimethylaminopropyl)-3-ethylcarbodiimide hydrochloride (223 mg, 1.16 mmol) and 1-hydroxybenzotriazole (157 mg, 1.16 mmol) were added. The reaction liquid was stirred at 25 °C for 10 min, and then a solution of N,N-diisopropylethylamine (677 mg, 5.25 mmol) and L001-7 (324 mg, 1.26 mmol) in N,N-dimethylformamide (5 mL) was added. The reaction liquid was reacted at 25 °C for another 0.5 h. The reaction liquid was poured into water (20 mL) and extracted with ethyl acetate (15 mL × 3). The combined organic phases were washed with a saturated aqueous ammonium chloride solution (20 mL × 3), dried over anhydrous sodium sulfate, and filtered, and the filtrate was concentrated under reduced pressure. The residue was purified by silica gel column chromatography to give compound L002-5 (500 mg, yield: 81.97%). LC-MS: [M+H]⁺ = 584.05.

### 2.6: Synthesis of compound L002

L002-5 (500 mg, 0.85 mmol) was dissolved in dichloromethane (5 mL), and a solution of hydrogen chloride in ethyl acetate (5 mL, 4 M) was added. The reaction liquid was reacted at 25 °C for 0.5 h. The reaction liquid was directly concentrated under reduced pressure, and the residue was purified by preparative high performance liquid chromatography (basic condition) and lyophilized to give compound L002 (200 mg, yield: 48.28%).

¹H NMR (400 MHz, DMSO-*d₆*): δ 8.20 - 8.13 (m, 2H), 7.64 (d, *J =* 8.0 Hz, 2H), 7.57 (d, *J* = 8.0 Hz, 2H), 7.45 (t, *J* = 8.0 Hz, 2H), 7.34 (t, *J* = 8.0 Hz, 1H), 7.27 (dd, *J* = 8.0, 4.0 Hz, 2H), 7.16 - 7.12 (m, 1H), 6.22 (d, *J* = 8.0 Hz, 1H), 5.84 (s, 2H), 5.14 - 5.03 (m, 1H), 4.28 - 4.16 (m, 1H), 3.69 - 3.58 (m, 1H), 3.09 - 3.04 (m, 1H), 2.93 - 2.83 (m, 1H), 2.79 - 2.54 (m, 5H), 2.37 - 2.27 (m, 1H), 2.22 - 2.13 (m, 1H), 1.84 - 1.62 (m, 3H), 1.21-1.18 (m, 3H); LC-MS: [M+H]⁺ = 484.15.

### 2.7: Synthesis of compounds L002-IS-01 and L002-IS-02

Compound L002 (200 mg) was separated by high performance liquid chromatography (basic, CHIRAL ART Amylose-C NEO, 30 × 250 mm, 10 µm, mobile phase, A: n-hexane (0.1% diethylamine), B: isopropanol, flow rate: 30 mL/min) to give compound L002-IS-01 (retention time in the spectrum Rt = 6.184 min, 69.9 mg) and L002-IS-02 (retention time in the spectrum Rt = 7.442 min, 80.6 mg).
L002-IS-01:
   ¹H NMR (400 MHz, DMSO-*d₆*): δ 8.19-8.12 (m, 2H), 7.67 - 7.61 (m, 2H), 7.57 (d, *J* = 8.0 Hz, 2H), 7.45 (t, *J* = 8.0 Hz, 2H), 7.34 (t, *J* = 8.0 Hz, 1H), 7.26 (d, *J* = 8.0 Hz, 2H), 7.11 (d, *J* = 8.0 Hz, 1H), 6.24 (d, *J =* 12.0 Hz, 1H), 5.83 (s, 2H), 5.09 (q, *J =* 8.0 Hz, 1H), 4.22 (p, *J =* 8.0 Hz, 1H), 3.64 (dd, Jₗ =8.0, *J*₂ = 4.0 Hz, 1H), 2.86 (dd, *J*₁= 10.0, *J₂* = 6.8 Hz, 1H), 2.80 - 2.51 (m, 5H), 2.37 - 2.29 (m, 1H), 2.20 - 2.13 (m, 1H), 1.82 - 1.63 (m, 3H), 1.35 - 1.23 (m, 1H), 1.19 (d, *J =* 8.0 Hz, 3H); LC-MS: [M+H]⁺ = 484.15.
L002-IS-02:
   ¹H NMR (400 MHz, DMSO-*d₆*): δ 8.16 (t, *J* = 8.0 Hz, 2H), 7.64 (d, *J* = 8.0 Hz, 2H), 7.57 (d, *J* = 8.0 Hz, 2H), 7.45 (t, *J* = 8.0 Hz, 2H), 7.34 (t, *J* = 8.0 Hz, 1H), 7.27 (d, *J* = 8.0 Hz, 2H), 7.15 (d, *J* = 8.0 Hz, 1H), 6.23 (d, *J* = 8.0 Hz, 1H), 5.84 (s, 2H), 5.07 (q, *J* = 8.0 Hz, 1H), 4.21 (p, *J* = 8.0 Hz, 1H), 3.64 (dd, *J*₁= 8.0, *J*₂ = 4.0 Hz, 1H), 2.89 (dd, *J*₁=8.0, *J*₂ = 4.0 Hz, 1H), 2.78 - 2.55 (m, 5H), 2.38 - 2.27 (m, 1H), 2.22 - 2.19 (m, 1H), 1.83 - 1.61 (m, 3H), 1.27 - 1.21 (m, 1H), 1.19 (d, *J* = 8.0 Hz, 3H); LC-MS:[M+H]⁺= 484.15.

### Example 3: Synthesis of (2R,4S)-N-((2S)-1-((2-amino-6,7-dihydro-5H-cyclopenta[b]pyridin-5-yl) amino)-1-oxopropan-2-yl)-4-(4-chlorobenzyl)pyrrolidine-2-carboxamide (Compound L003) Hydrochloride

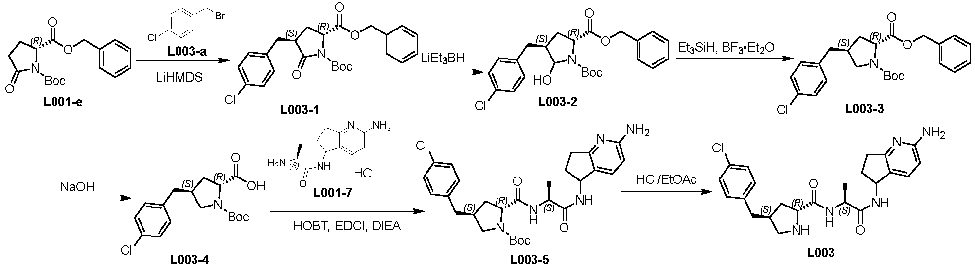

### 3.1: Synthesis of compound L003-1

L001-e (1.0 g, 3.13 mmol) was dissolved in anhydrous tetrahydrofuran (10 mL), and the reaction system was cooled to -78 °C under nitrogen atmosphere. Lithium bis(trimethylsilyl)amide (3.4 mL, 3.44 mmol, 1 M in tetrahydrofuran) was added dropwise and slowly. The reaction liquid was stirred at this temperature for 0.5 h. L003-a (804 mg, 3.91 mmol) was then added dropwise to the reaction liquid with the reaction temperature maintained at -78 °C. After the dropwise addition was completed, the reaction liquid was stirred for another 2 h. At this temperature, the reaction liquid was quenched by adding a saturated aqueous sodium bicarbonate solution (15 mL) and extracted with ethyl acetate (30 mL × 3). The combined organic phases were dried over anhydrous sodium sulfate and filtered, and the filtrate was concentrated under reduced pressure. The residue was purified by silica gel column chromatography to give compound L003-1 (310 mg, yield: 22.4%). LC-MS: [M-Boc+H]⁺ = 344.00.

### 3.2: Synthesis of compound L003-2

L003-1 (310 mg, 0.70 mmol) was dissolved in tetrahydrofuran (4 mL). The reaction liquid was cooled to -78 °C under nitrogen atmosphere, and lithium triethylborohydride (0.77 mL, 0.77 mmol, 1 M in tetrahydrofuran) was added dropwise and slowly. After the dropwise addition was completed, the reaction liquid was stirred at -78 °C for 2 h. At this temperature, the reaction liquid was quenched by adding a saturated aqueous sodium bicarbonatesolution (10 mL) and extracted with ethyl acetate (20 mL × 3). The combined organic phases were dried over anhydrous sodium sulfate and filtered, and the filtrate was concentrated under reduced pressure. The residue was purified by silica gel column chromatography to give compound L003-2 (260 mg, yield: 83.6 %). LC-MS: [M-OH]⁺ = 427.95.

### 3.3: Synthesis of compound L003-3

L003-2 (260 mg, 0.58 mmol) was dissolved in dichloromethane (10 mL), and the reaction system was cooled to -78 °C under nitrogen atmosphere. Triethylsilane (75 mg, 0.64 mmol) and boron trifluoride diethyl ether coordination complex (98.7 mg, 0.70 mmol) were added dropwise and slowly. After the dropwise addition was completed, the reaction liquid was stirred at -78 °C for 2 h. At this temperature, the reaction liquid was quenched by adding a saturated aqueous sodium bicarbonate solution (10 mL) and extracted with ethyl acetate (20 mL × 3). The combined organic phases were dried over anhydrous sodium sulfate and filtered, and the filtrate was concentrated under reduced pressure. The residue was purified by silica gel column chromatography to give compound L003-3 (200 mg, yield: 80.6 %). LC-MS: [M+H]⁺ = 430.05.

### 3.4: Synthesis of compound L003-4

L003-3 (100 mg, 0.23 mmol) was dissolved in methanol (3 mL) and water (3 mL), and then sodium hydroxide (18.6 mg, 0.46 mmol) was added. The reaction liquid was stirred at room temperature for 16 h. The reaction liquid was added with water (5 mL) for dilution and extracted with ethyl acetate (5 mL). The organic phase was discarded, and the aqueous phase was adjusted to pH = 5-6 with formic acid and then extracted with ethyl acetate (15 mL × 3). The combined organic phases were dried over anhydrous sodium sulfate and filtered, and the filtrate was concentrated under reduced pressure to give compound L003-4 (90 mg, crude product), which could be used directly in the next step.

### 3.5: Synthesis of compound L003-5

L003-4 (90 mg, crude product), 1-hydroxybenzotriazole (39 mg, 0.29 mmol), N,N-dimethylformamide (2 mL), and 1-(3-dimethylaminopropyl)-3-ethylcarbodiimide hydrochloride (55 mg, 0.29 mmol) were added to a reaction flask. The reaction liquid was stirred at room temperature for 0.5 h. Meanwhile, L001-7 (92 mg, 0.31 mmol) and N,N-diisopropylethylamine (134.2 mg, 1.04 mmol) were dissolved in N,N-dimethylformamide (2 mL), and the solution was stirred at room temperature for 0.5 h. The solution was then added to the reaction liquid, and the reaction liquid was stirred for another 0.5 h. The reaction liquid was added with water (20 mL) for dilution and extracted with ethyl acetate (30 mL × 3). The organic phases were combined, washed with saturated brine (40 mL), dried over anhydrous sodium sulfate, and filtered, and the filtrate was concentrated under reduced pressure. The residue was purified by high performance liquid chromatography (acidic condition) to give compound L003-5 (58.0 mg, yield: 40.1%). LC-MS: [M+H]⁺ = 542.00.

### 3.6: Synthesis of compound L003 hydrochloride

L003-5 (58 mg, 0.11 mmol) was dissolved in dichloromethane (2 mL), and a solution of hydrogen chloride in ethyl acetate (2 mL, 4 M) was added. The reaction liquid was stirred at room temperature for 0.5 h. A solid was precipitated. The solid was filtered, and the filter cake was washed with dichloromethane (15 mL), dissolved in deionized water (15 mL), and then lyophilized to give compound L003 hydrochloride (36 mg, yield: 76.1%).

¹H NMR (400 MHz, DMSO-*d₆*): δ 10.45-10.37 (m, 1H), 8.86 (d, *J =* 6.4 Hz, 1H), 8.57-8.55 (m, 2H), 7.94 (s, 2H), 7.66 (dd, *J*₁ = 8.0 Hz, *J*₂ = 4.0 Hz, 1H), 7.33 (dd, *J*₁ = 8.0, *J*₂ = 4.0 Hz, 4H), 6.81 (d, *J =* 8.0 Hz, 1H), 5.14 (d, *J* = 4.0 Hz, 1H), 4.42 - 4.17 (m, 2H), 3.27 (s, 1H), 3.09 - 2.82 (m, 3H), 2.77 - 2.64 (m, 2H), 2.48 - 2.35 (m, 2H), 2.06 - 1.90 (m, 3H), 1.24 - 1.22 (m, 4H); LC-MS: [M+H]⁺ = 442.00.

### Example 4: Synthesis of (2R,4S)-N-((2S)-1-((2-amino-6,7-dihydro-5H-cyclopenta[b]pyridin-5-yl) amino)-1-oxopropan-2-yl)-4-(4-methylbenzyl)pyrrolidine-2-carboxamide (Compound L004) Hydrochloride

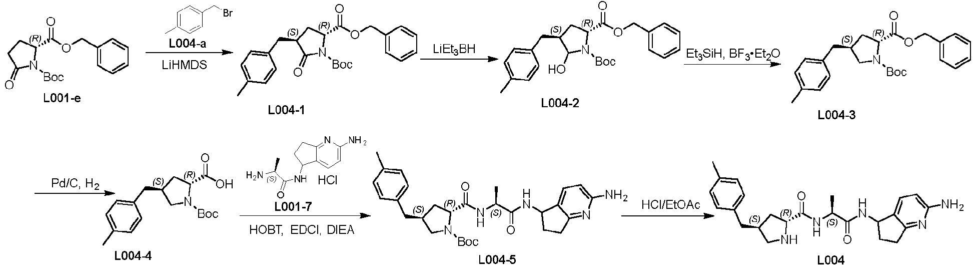

### 4.1: Synthesis of compound L004-1

L001-e (1 g, 3.13 mmol) was added to a reaction flask and dissolved by adding tetrahydrofuran (10 mL) under nitrogen atmosphere. The mixture was placed in a cold bath at -78 °C. Lithium bis(trimethylsilyl)amide (3.2 mL, 3.20 mmol, 1 M in tetrahydrofuran) was added with the temperature maintained at -78 °C, and the mixture was stirred for 0.5 h. L004-a (576 mg, 3.13 mmol) was then added, and the mixture was reacted at -78 °C for another 2 h. A small amount of insoluble substance was formed. The insoluble substance was removed by filtration, and the filtrate was added with water (50 mL) for dilution and extracted with ethyl acetate (50 mL × 3). The combined organic phases were dried over anhydrous sodium sulfate and filtered, and the filtrate was concentrated under reduced pressure. The residue was purified by silica gel column chromatography to give compound L004-1 (650 mg, yield: 49%). LC-MS: [M-Boc+H]⁺ = 324.05.

### 4.2: Synthesis of compound L004-2

L004-1 (600 mg, 1.42 mmol) was added to a reaction flask and dissolved by adding tetrahydrofuran (5 mL) under nitrogen atmosphere. The mixture was placed in a cold bath at -78 °C. Lithium triethylborohydride (1.42 mL, 1.42 mmol) was added with the temperature maintained at -78 °C, and the reaction liquid was stirred for 0.5 h. The reaction liquid was quenched by adding water (50 mL) and extracted with ethyl acetate (50 mL × 3). The combined organic phases were dried over anhydrous sodium sulfate and filtered, and the filtrate was concentrated under reduced pressure to give compound L004-2 (810 mg, crude product). LC-MS: [M-OH]⁺ = 408.05.

### 4.3: Synthesis of compound L004-3

L004-2 (600 mg, 1.41 mmol) was added to a reaction flask and dissolvedby adding dichloromethane (5 mL) under nitrogen atmosphere. The mixture was placed in a cold bath at -78 °C. Triethylsilane (163.9 mg, 1.41 mmol) and boron trifluoride diethyl ether coordination complex (200 mg, 1.41 mmol) were added with the temperature maintained at -78 °C, and the reaction liquid was stirred for 0.5 h. The reaction liquid was supplemented with triethylsilane (164 mg, 1.41 mmol) and boron trifluoride diethyl ether coordination complex (200 mg, 1.41 mmol) and stirred at -78 °C for another 2 h. At this temperature, the reaction liquid was quenched by adding water (50 mL) and extracted with ethyl acetate (50 mL × 3). The combined organic phases were dried over anhydrous sodium sulfate and filtered, and the filtrate was concentrated under reduced pressure. The residue was purified by silica gel column chromatography to give compound L004-3 (402 mg, yield: 70%). LC-MS: [M+H]⁺ = 410.00.

### 4.4: Synthesis of compound L004-4

L004-3 (402 mg, 0.98 mmol) was added to a reaction flask and dissolved by adding methanol (5 mL). Then, wet palladium on carbon (30 mg, 10% palladium content) was added, and the reaction liquid was reacted at room temperature for 3 h under hydrogen atmosphere. The reaction liquid was filtered through celite, and then the filtrate was concentrated under reduced pressure to give compound L004-4 (267 mg, crude product), which could be used directly in the next step. LC-MS: [M-tBu+H]⁺ = 264.05.

### 4.5: Synthesis of compound L004-5

L004-4 (100 mg, 0.31 mmol), 1-hydroxybenzotriazole (47 mg, 0.34 mmol), N,N-dimethylformamide (3 mL), N,N-diisopropylethylamine (170 mg, 1.24 mmol), and 1-(3-dimethylaminopropyl)-3-ethylcarbodiimide hydrochloride (65 mg, 0.34 mmol) were added to a reaction flask, and the mixture was reacted at room temperature for 0.5 h. Compound L001-7 (82 mg, 0.37 mmol) was then added, and the mixture was reacted at room temperature for another 1 h. The mixture was added with water (40 mL) for dilution and extracted with ethyl acetate (30 mL × 3). The combined organic phases were washed with saturated sodium chloride (40 mL), dried over anhydrous sodium sulfate, and filtered, and the filtrate was concentrated under reduced pressure. The residue was purified by preparative high performance liquid chromatography (acidic condition) to give compound L004-5 (57.8 mg, yield: 35.5%). LC-MS: [M+H]⁺ = 522.10.

### 4.6: Synthesis of compound L004 hydrochloride

L004-5 (57.8 mg, 0.11 mmol) was dissolved in dichloromethane (2 mL), and a solution of hydrogen chloride in ethyl acetate (1 mL, 4 M) was added. The mixture was stirred at room temperature for 0.5 h. A solid was precipitated and filtered, and the filter cake was washed with ethyl acetate (10 mL). The filter cake was dissolved by adding water (3 mL) and lyophilized to give compound L004 hydrochloride (40.2 mg, yield: 81.1%).

¹H NMR (400 MHz, DMSO-*d₆*): δ 10.45-10.37 (m, 1H), 8.86 (d, *J =* 6.4 Hz, 1H), 8.57-8.55 (m, 2H), 7.94 (s, 2H), 7.66 (dd, *J*₁ = 8.0 Hz, *J*₂ = 4.0 Hz, 1H), 7.33 (dd, *J*₁ = 8.0, *J*₂ = 4.0 Hz, 4H), 6.81 (d, *J =* 8.0 Hz, 1H), 5.14 (d, *J* = 4.0 Hz, 1H), 4.42 - 4.17 (m, 2H), 3.27 (s, 1H), 3.09 - 2.82 (m, 3H), 2.77 - 2.64 (m, 2H), 2.48 - 2.35 (m, 2H), 2.06 - 1.90 (m, 3H), 1.24 - 1.22 (m, 4H); LC-MS: [M+H]⁺ =422.15.

### Example 5: Synthesis of (2R,4S)-N-((2S)-1-((2-amino-6,7-dihydro-5H-cyclopenta[b]pyridin-5-yl) amino)-1-oxopropan-2-yl)-4-(4-(trifluoromethyl)benzyl)pyrrolidine-2-carboxamide (Compound L005) Hydrochloride and (2R,4S)-N-((S)-1-(((R)-2-amino-6,7-dihydro-5H-cyclopenta[b]pyridin-5-yl)amino)-1-oxopropan-2-yl)-4-(4-(trifluoromethyl)benzyl)pyrrolidine-2-carboxamide (Compound L005-IS-01 or L005-IS-02) Hydrochloride and (2R,4S)-N-((S)-1-(((S)-2-amino-6,7-dihydro-5H-cyclopenta[b]pyridin-5-yl)amino)-1-oxopropan-2-yl)-4-(4-(trifluoromethyl)benzyl)pyrrolidine-2-carboxamide (Compound L005-IS-02 or L005-IS-01) Hydrochloride

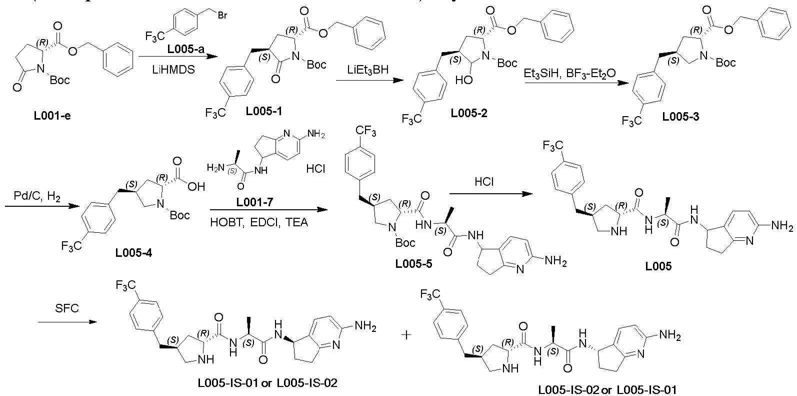

### 5.1: Synthesis of compound L005-1

L001-e (2.0 g, 6.26 mmol) was added to tetrahydrofuran (20 mL) at room temperature, and the reaction liquid was cooled to -78 °C. Lithium bis(trimethylsilyl)amide (6.80 mL, 6.80 mmol, 1 M in tetrahydrofuran) was added dropwise under nitrogen atmosphere. After the dropwise addition was completed, the reaction liquid was stirred at -78 °C for 0.5 h. A solution of L005-a (1.64 g, 6.88 mmol) in tetrahydrofuran (10 mL) was added dropwise to the reaction liquid, and after the dropwise addition was completed, the reaction liquid was stirred at -78 °C for another 2 h. The reaction liquid was quenched by adding an aqueous ammonium chloride solution (10 mL) at -78 °C, and after being warmed to room temperature, the reaction system was extracted with ethyl acetate (50 mL × 3). The combined organic phases were dried over anhydrous sodium sulfate and filtered, and the filtrate was concentrated under reduced pressure. The residue was purified by silica gel column chromatography to give compound L005-1 (1.2 g, yield: 50.6%). LC-MS: [M-Boc+H]⁺ = 377.95.

### 5.2: Synthesis of compound L005-2

L005-1 (1.1 g, 2.30 mmol) was dissolved in tetrahydrofuran (15 mL) at room temperature, and the mixture was cooled to -78 °C. Lithium triethylborohydride (2.5 mL, 2.5 mmol, 1 M in tetrahydrofuran) was added dropwise under nitrogen atmosphere. After the dropwise addition was completed, the reaction liquid was stirred at -78 °C for 0.5 h. The reaction liquid was quenched by adding a saturated aqueous sodium bicarbonate solution (10 mL) at -78 °C, and after being warmed to room temperature, the reaction liquid was extracted with ethyl acetate (30 mL × 3). The combined organic phases were dried over anhydrous sodium sulfate and filtered, and the filtrate was concentrated under reduced pressure to give compound L005-2 (500 mg, crude product), which could be used directly in the next step. LC-MS: [M-OH]⁺ = 461.95.

### 5.3: Synthesis of compound L005-3

L005-2 (500 mg, 1.04 mmol) was dissolved in dichloromethane (5 mL) at room temperature, and the reaction liquid was cooled to -78 °C. Triethylsilane (312 mg, 2.70 mmol) and boron trifluoride diethyl ether coordination complex (384 mg, 2.70 mmol) were added dropwise to the reaction liquid under nitrogen atmosphere, and after the dropwise addition was completed, the reaction liquid was stirred at -78 °C for 1 h. The reaction liquid was quenched by adding a saturated aqueous sodium bicarbonate solution (10 mL) at -78 °C, and after being warmed to room temperature, the system was extracted with ethyl acetate (30 mL × 3). The combined organic phases were dried over anhydrous sodium sulfate and filtered, and the filtrate was concentrated under reduced pressure to give compound L005-3 (450 mg, crude product), which could be used directly in the next step. LC-MS: [M-tBu+H]⁺ = 407.95.

### 5.4: Synthesis of compound L005-4

L005-3 (450 mg, crude product) was dissolved in methanol (5 mL), and wet palladium on carbon (200 mg, 10% palladium content) was added. The reaction liquid was stirred at 20 °C for 1 h under hydrogen atmosphere. The reaction liquid was filtered, and the filtrate was concentrated under reduced pressure to give compound L005-4 (300 mg, crude product), which was used directly in the next step.

### 5.5: Synthesis of compound L005-5

L005-4 (127 mg, crude product), L001-7 (100 mg, 0.34 mmol), 1-(3-dimethylaminopropyl)-3-ethylcarbodiimide hydrochloride (88 mg, 0.46 mmol), 1-hydroxybenzotriazole (69 mg, 0.46 mmol), and triethylamine (93 mg, 0.92 mmol) were dissolved in N,N-dimethylformamide (2 mL), and the reaction liquid was stirred at room temperature for 1 h. The reaction liquid was added with water (20 mL) for dilution and extracted with dichloromethane (20 mL × 3). The combined organic phases were dried over anhydrous sodium sulfate and filtered, and the filtrate was concentrated under reduced pressure to give a crude product. The crude product was purified by preparative high performance liquid chromatography (acidic condition) to give compound L005-5 (150 mg). LC-MS: [M+H]⁺ = 576.10.

### 5.6: Synthesis of compound L005 hydrochloride

L005-5 (150 mg, 0.19 mmol) was dissolved in dichloromethane (1 mL), and a solution of hydrogen chloride in 1,4-dioxane (1 mL, 4 M) was added dropwise. The reaction liquid was stirred at room temperature for 1 h, and a white solid was precipitated. The reaction liquid was filtered, and the filter cake was washed with dichloromethane (3 mL). The filter cake was dissolved in deionized water (10 mL) and lyophilized to give compound L005 hydrochloride (52.0 mg, yield: 57.4%).

¹H NMR (400 MHz, DMSO-*d₆*): δ 10.54 - 10.52 (m, 1H), 8.87 (dd, *J* = 7.2, 4.0 Hz, 1H), 8.73 - 8.42 (m, 2H), 8.04 (s, 2H), 7.66 (dt, *J =* 8.8, 7.2 Hz, 3H), 7.49 (d, *J =* 8.0 Hz, 2H), 6.82 (d, *J =* 8.0 Hz, 1H), 5.13 (d, *J* = 7.2 Hz, 1H), 4.50 - 4.10 (m, 2H), 3.26 (s, 1H), 3.10 - 2.97 (m, 1H), 2.96 - 2.71 (m, 4H), 2.52 (d, *J =* 7.2 Hz, 1H), 2.06 (dt, *J =* 8.8, 6.8 Hz, 1H), 1.99 - 1.83 (m, 2H), 1.21 (d, *J =* 7.2 Hz, 3H); LC-MS: [M+H]⁺ =476.05.

### 5.7: Synthesis of compound L005-IS-01 hydrochloride and L005-IS-02 hydrochloride

L005 hydrochloride (52.0 mg, 0.11 mmol) was separated and purified by supercritical fluid chromatography (SFC) (column: Daicel Chiralpak AD, 50 × 25 mm, 10 µm, mobile phase, A: carbon dioxide, B: ethanol, flow rate: 70 g/min) to give two compounds L005-IS-01 (retention time Rt = 1.660 min, 7.7 mg) and L005-IS-02 (retention time Rt = 1.989 min, 9.8 mg).
L005-IS-01:
   ¹H NMR (400 MHz, DMSO-*d₆*): δ 8.13 (d, *J =* 7.6 Hz, 2H), 7.62 (d, *J=* 8.0 Hz, 2H), 7.40 (d, *J* = 8.0 Hz, 2H), 7.12 (d, *J* = 8.4 Hz, 1H), 6.21 (d, *J* = 8.4 Hz, 1H), 5.82 (s, 2H), 5.05 (dd, *J* = 14.4, 7.6 Hz, 1H), 4.32 - 4.08 (m, 1H), 3.61 - 3.59 (m, 1H), 2.82 - 2.66 (m, 1H), 2.65 - 2.62 (m, 5H), 2.37 - 2.12 (m, 2H), 1.85 - 1.52 (m, 3H), 1.20 - 1.17 (m, 4H); LC-MS: [M+H]⁺ =476.05.
L005-IS-02:
   ¹H NMR (400 MHz, DMSO-*d₆*): δ 8.16 - 8.10 (m, 2H), 7.62 (d, *J =* 8.4 Hz, 2H), 7.39 (d, *J =* 8.0 Hz, 2H), 7.08 (d, *J* = 8.0 Hz, 1H), 6.21 (d, *J* = 8.4 Hz, 1H), 5.81 (s, 2H), 5.07 (dd, *J* = 14.8, 7.6 Hz, 1H), 4.24 - 4.15 (m, 1H), 3.62 - 3.59 (m, 1H), 2.78 - 2.53 (m, 6H), 2.58 - 2.30 (m, 2H), 1.70 - 1.62 (m, 2H), 1.19 (t, *J =* 8.8 Hz, 4H); LC-MS: [M+H]⁺ =476.05.

### Example 6: Synthesis of (2R,4R)-N-((S)-1-(((R)-2-amino-6,7-dihydro-5H-cyclopenta[b]pyridin-5-yl)amino)-1-oxopropan-2-yl)-4-phenylpyrrolidine-2-carboxamide Hydrochloride (Compound L006-IS-01 or L006-IS-02) and (2R,4R)-N-((S)-1-(((S)-2-amino-6,7-dihydro-5H-cyclopenta[b]pyridin-5-yl)amino)-1-oxopropan-2-yl)-4-phenylpyrrolidine-2-carboxamide Hydrochloride (Compound L006-IS-02 or L006-IS-01)

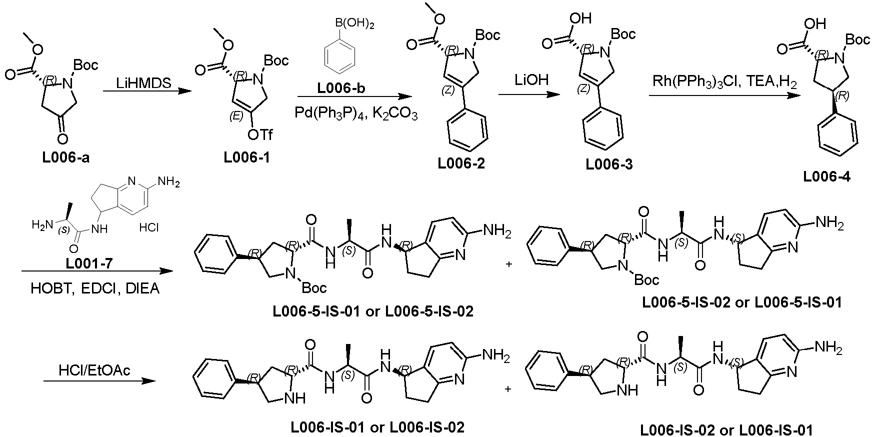

### 6.1: Synthesis of compound L006-1

L006-a (1 g, 4.12 mmol) was added to a reaction flask and dissolved by adding tetrahydrofuran (10 mL) under nitrogen atmosphere. The mixture was placed in a cold bath at -78 °C. Lithium bis(trimethylsilyl)amide (4.9 mL, 4.90 mmol, 1 M in tetrahydrofuran) was added dropwise and slowly with the temperature maintained at -78 °C. After the addition was completed, the reaction liquid was reacted at this temperature for 1 h. A solution of N-(5-chloro-2-pyridinyl)bis(trifluoromethanesulfonamide) (2 g, 5.10 mmol) in tetrahydrofuran (4 mL) was then added dropwise and slowly at -78 °C. After the addition was completed, the reaction liquidwas reacted at this temperature for another 1 h. The reaction liquid was warmed to -20 °C and reacted for 16 h. The reaction liquid was quenched with a saturated aqueous sodium bicarbonate solution (50 mL) and extracted with ethyl acetate (50 mL × 3). The combined organic phases were washed with saturated brine (50 mL), dried over anhydrous sodium sulfate, and filtered, and the filtrate was concentrated under reduced pressure. The residue was purified by silica gel column chromatography to give compound L006-1 (1.1 g, yield: 71%).

### 6.2: Synthesis of compound L006-2

L006-1 (600 mg, 1.60 mmol), phenylboronic acid (235 mg, 1.93 mmol), tetrakis(triphenylphosphine) palladium(0) (185 mg, 0.16 mmol), and potassium carbonate (664 mg, 4.81 mmol) were added to a reaction flask and dissolved by adding 1,4-dioxane (10 mL) and water (2 mL) under nitrogen atmosphere. The reaction liquid was placed in an oil bath at 80 °C and reacted for 16 h. The reaction liquid was cooled to room temperature, added with water (50 mL) for dilution, and extracted with ethyl acetate (50 mL × 3). The combined organic phases were washed with saturated brine (50 mL), dried over anhydrous sodium sulfate, and filtered, and the filtrate was concentrated under reduced pressure.

The residue was purified by silica gel column chromatography to give compound L006-2 (420 mg, yield: 87 %). LC-MS: [M+H]⁺ = 304.10.

### 6.3: Synthesis of compound L006-3

L006-2 (420 mg, 1.36 mmol) and lithium hydroxide monohydrate (873 mg, 20.78 mmol) were added to a reaction flask and dissolved by adding tetrahydrofuran (4 mL), methanol (2 mL), and water (2 mL). The reaction liquid was stirred at room temperature for 2 h. The reaction liquid was adjusted to pH = 5-6 with diluted hydrochloric acid (1 M), added with water (10 mL) for dilution, and extracted with ethyl acetate (20 mL × 3). The combined organic phases were dried over anhydrous sodium sulfate and filtered, and the filtrate was concentrated under reduced pressure to give compound L006-3 (350 mg, yield: 87.1%). LC-MS: [M-tBu+H]⁺ = 234.05.

### 6.4: Synthesis of compound L006-4

L006-3 (350 mg, 1.21 mmol), tris(triphenylphosphine)rhodium chloride (113 mg, 0.12 mmol), triethylamine (123 mg, 1.21 mmol), and methanol (10 mL) were added to a reaction flask. The reaction liquid was purged with hydrogen and stirred at room temperature for 2 days. The reaction liquid was filtered, and the filtrate was adjusted to pH = 10 with an aqueous sodium hydroxide solution (1 M). Ethyl acetate (40 mL) was added for extraction, and the organic phase was washed once with a saturated aqueous sodium bicarbonate solution (20 mL) and then discarded. All aqueous phases were combined, adjusted to pH = about 5-6 with diluted hydrochloric acid (0.5 M), and then extracted with ethyl acetate (20 mL × 3). The combined organic phases were washed with saturated brine (50 mL), dried over anhydrous sodium sulfate, and filtered, and the filtrate was concentrated under reduced pressure to give compound L006-4 (209 mg, yield: 73.2%). LC-MS: [M-Boc+H]⁺ = 192.10.

### 6.5: Synthesis of compounds L006-5-IS-01 and L006-5-IS-02

L006-4 (100 mg, 0.34 mmol), 1-hydroxybenzotriazole (53 mg, 0.38 mmol), N,N-dimethylformamide (3 mL), N,N-diisopropylethylamine (177 mg, 1.37 mmol), and 1-(3-dimethylaminopropyl)-3-ethylcarbodiimide hydrochloride (73 mg, 0.38 mmol) were added to a reaction flask, and the reaction liquid was stirred at room temperature for 0.5 h. L001-7 (91 mg, 0.41 mmol) was then added, and the reaction liquid was reacted at room temperature for another 1 h. The reaction liquid was added with water (50 mL) for dilution and extracted with ethyl acetate (30 mL × 3). The combined organic phases were washed with a saturated aqueous sodium chloride solution (40 mL), dried over anhydrous sodium sulfate, and filtered, and the filtrate was concentrated under reduced pressure. The residue was subjected to preparative high performance liquid chromatography (acidic condition) to give compounds L006-5-IS-01 (retention time Rt = 18.349 min, 20 mg) and L006-5-IS-02 (retention time Rt = 20.877 min, 30 mg). LC-MS: [M+H]⁺ = 494.10.

### 6.6: Synthesis of compound L006-IS-01 hydrochloride

The obtained L006-5-IS-01 (retention time Rt = 18.349 min, 20 mg) was dissolved in dichloromethane (2 mL), and a solution of hydrogen chloride in ethyl acetate (1 mL, 4 M) was added. The reaction liquid was stirred at room temperature for 1 h. A solid was precipitated. The reaction liquid was filtered, and the filter cake was washed with ethyl acetate (10 mL). The solid was dissolved by adding deionized water (5 mL) and lyophilized to give compound L006-IS-01 hydrochloride (13.5 mg).

¹H NMR (400 MHz, CH₃OH-*d₄*): δ 7.85 - 7.75(m, 1H), 7.38 - 7.28(m, 5H), 6.85 - 6.80(m, 1H), 5.33 - 5.29(m, 1H), 4.63 - 4.49(m, 1H), 4.37 - 4.30(m, 1H), 3.84 - 3.76(m, 1H), 3.68 - 3.47(m, 1H), 3.36 - 3.31(m, 1H), 3.17 - 3.09(m, 1H), 3.02 - 2.88(m, 2H), 2.66 - 2.56(m, 1H), 2.22 - 1.99(m, 2H), 1.42(d, *J =* 7.2 Hz, 3H); LC-MS: [M+H]⁺=394.10.

### 6.7: Synthesis of compound L006-IS-02 hydrochloride

The obtained L006-5-IS-02 (retention time Rt = 20.877 min, 30 mg) was dissolved in dichloromethane (2 mL), and a solution of hydrogen chloride in ethyl acetate (1 mL, 4 M) was added. The reaction liquid was stirred at room temperature for 1 h. A solid was precipitated. The reaction liquid was filtered, and the filter cake was washed with ethyl acetate (10 mL). The solid was dissolved by adding deionized water (5 mL) and lyophilized to give compound L006-IS-02 hydrochloride (22.2 mg).

¹H NMR (400 MHz, CH₃OH-*d₄*): ¹H NMR (400 MHz, CH₃OH-*d₄*): δ 7.86 - 7.78(m, 1H), 7.38 - 7.28(m, 5H), 6.85 - 6.82(m, 1H), 5.35 - 5.30(m, 1H), 4.63 - 4.46(m, 1H), 4.39 - 4.28(m, 1H), 3.86 - 3.76(m, 1H), 3.69 - 3.48(m, 1H), 3.38 - 3.35(m, 1H), 3.19 - 3.11(m, 1H), 3.05 - 2.97(m, 1H), 2.67 - 2.50(m, 3H), 2.11 - 1.99(m, 1H), 1.42(d, *J =* 7.2 Hz, 3H); LC-MS: [M+H]⁺ =394.10.

### Example 7: Synthesis of (2R,4R)-N-((2S)-1-((2-amino-6,7-dihydro-5H-cyclopenta[b]pyridin-5-yl) amino)-1-oxopropan-2-yl)-4-(4-fluorophenyl)pyrrolidine-2-carboxamide (Compound L007) Hydrochloride

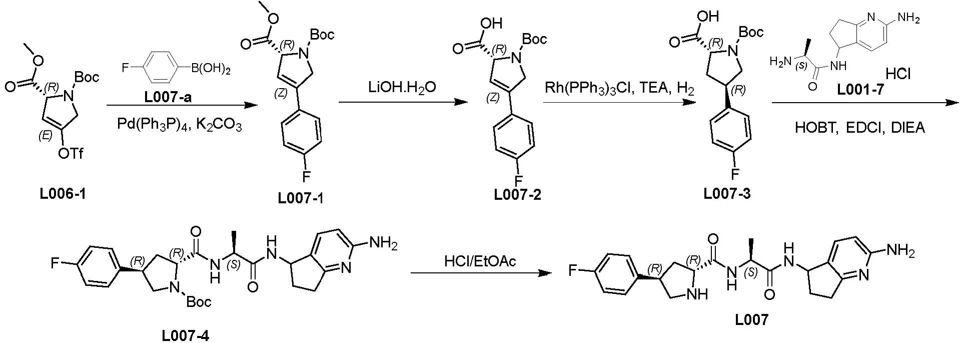

### 7.1: Synthesis of compound L007-1

L006-1 (450 mg, 1.2 mmol), 4-fluorophenylboronic acid (202 mg, 1.44 mmol), tetrakis(triphenylphosphine)palladium(0) (129 mg, 0.12 mmol), and potassium carbonate (497 mg, 3.6 mmol) were added to 1,4-dioxane (10 mL) and water (2 mL) at room temperature. The reaction liquid was reacted at 80 °C for 16 h under nitrogen atmosphere. The reaction liquid was cooled to room temperature, added with water (50 mL) for dilution, and extracted with ethyl acetate (50 mL × 3). The combined organic phases were washed with saturated brine (50 mL), dried over anhydrous sodium sulfate, and filtered, and the filtrate was concentrated under reduced pressure. The residue was purified by silica gel column chromatography to give compound L007-1 (361 mg, yield: 94 %). LC-MS: [M+H]⁺ = 322.05.

### 7.2: Synthesis of compound L007-2

L007-1 (361 mg, 1.12 mmol) and lithium hydroxide monohydrate (708 mg, 16.86 mmol) were added to a reaction flask and dissolved by adding tetrahydrofuran (4 mL), methanol (2 mL), and water (2 mL). The reaction liquidwas stirred at room temperature for 2 h. The reaction liquid was adjusted to pH = 5-6 with diluted hydrochloric acid (1 M), added with water (10 mL) for dilution, and extracted with ethyl acetate (20 mL × 3). The combined organic phases were dried over anhydrous sodium sulfate and filtered, and the filtrate was concentrated under reduced pressure to give compound L007-2 (325 mg, yield: 94%), which could be used directly in the next step. LC-MS: [M-tBu+H]⁺ = 252.05.

### 7.3: Synthesis of compound L007-3

L007-2 (325 mg, 1.06 mmol), tris(triphenylphosphine)rhodium chloride (102 mg, 0.11 mmol), and triethylamine (107 mg, 1.06 mmol) were added to a reaction flask and dissolved by adding methanol (10 mL). The reaction liquid was purged with hydrogen and stirred at room temperature for 4 days. The reaction liquid was filtered, and the filtrate was adjusted to pH = 10 with an aqueous sodium hydroxide solution (1 M) and extracted with ethyl acetate (40 mL). The organic phase was washed once with a saturated aqueous sodium bicarbonate solution (20 mL) and then discarded. The combined aqueous phases were adjusted to pH = 5-6 with diluted hydrochloric acid (0.5 M) and then extracted with ethyl acetate (20 mL × 3). The combined organic phases were washed with saturated brine (50 mL), dried over anhydrous sodium sulfate and filtered, and the filtrate was concentrated under reduced pressure to give compound L007-3 (crude product), which could be used directly in the next step. LC-MS: [M-tBu +H]⁺ = 254.00.

### 7.4: Synthesis of compound L007-4

L007-3 (100 mg, 0.32 mmol), 1-hydroxybenzotriazole (49 mg, 0.36 mmol), N,N-dimethylformamide (2 mL), N,N-diisopropylethylamine (167 mg, 1.29 mmol), and 1-(3-dimethylaminopropyl)-3-ethylcarbodiimide hydrochloride (68 mg, 0.36 mmol) were added to a reaction flask, and the reaction liquid was stirred at room temperature for 0.5 h. L001-7 (86 mg, 0.39 mmol) was then added, and the reaction liquidwas reacted at room temperature for another 1 h. The reaction liquid was added with water (50 mL) for dilution and extracted with ethyl acetate (30 mL × 3). The combined organic phases were washed with a saturated aqueous sodium chloride solution (40 mL), dried over anhydrous sodium sulfate, and filtered, and the filtrate was concentrated under reduced pressure. The residue was purified by preparative high performance liquid chromatography (acidic condition) to give compound L007-4 (40 mg). LC-MS: [M+H]⁺ = 512.05.

### 7.5: Synthesis of compound L007 hydrochloride

L007-4 (40 mg, 0.078 mmol) was dissolved in dichloromethane (2 mL), and a solution of hydrogen chloride in ethyl acetate (1 mL, 4 M) was added. The reaction liquid was stirred at room temperature for 1 h. A solid was precipitated. The reaction liquid was filtered, and the filter cake was washed with ethyl acetate (5 mL), dissolved in deionized water (5 mL), and lyophilized to give compound L007 hydrochloride (5.2 mg).

¹H NMR (400 MHz, CH₃OH-*d₄*): δ 7.87 - 7.73 (m, 1H), 7.64 - 7.55 (m, 1H), 7.37 (dd, *J =* 8.8 Hz, 5.2 Hz, 1H), 7.21 - 7.08 (m, 2H), 6.86 - 6.74 (m, 1H), 5.36 - 5.32 (m, 1.5H), 4.59 - 4.48 (m, 1.5H), 4.37 - 4.29 (m, 1H), 3.80 - 3.69 (m, 1H), 3.35 - 3.33 (m, 1H), 3.16 - 3.13 (m, 1H), 3.06 - 3.00 (m, 1H), 2.91 - 2.84 (m, 0.5H), 2.68 - 2.62 (m, 1H), 2.21 - 1.95 (m, 2.5H), 1.47 - 1.41 (m, 3H); ¹⁹F NMR (400 MHz, CH₃OH-*d₄*): δ -113.09, -116.89; LC-MS: [M+H]⁺ = 412.15.

### Example 8: Synthesis of (2R,4R)-N-((2S)-1-((2-amino-6,7-dihydro-5H-cyclopenta[b]pyridin-5-yl) amino)-1-oxopropan-2-yl)-4-(4-(trifluoromethyl)phenyl)pyrrolidine-2-carboxamide (Compound L008) Hydrochloride

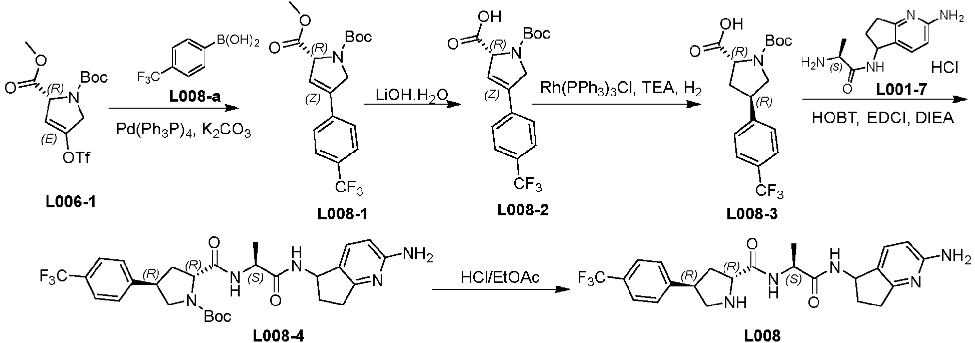

### 8.1: Synthesis of compound L008-1

L006-1 (450 mg, 1.2 mmol), 4-(trifluoromethyl)phenylboronic acid (274 mg, 1.44 mmol), tetrakis(triphenylphosphine)palladium(0) (139 mg, 0.12 mmol), and potassium carbonate (496.5 mg, 3.6 mmol) were added to a reaction flask and dissolved by adding 1,4-dioxane (10 mL) and water (2 mL). The reaction liquid was reacted at 80 °C for 16 h under nitrogen atmosphere. The reaction liquid was cooled to room temperature, added with water (50 mL) for dilution, and extracted with ethyl acetate (50 mL × 3). The combined organic phases were washed with saturated brine (50 mL), dried over anhydrous sodium sulfate, and filtered, and the filtrate was concentrated under reduced pressure.

The residue was purified by silica gel column chromatography to give compound L008-1 (386 mg, yield: 88%). LC-MS: [M-tBu+H]⁺ = 316.00.

### 8.2: Synthesis of compound L008-2

L008-1 (386 mg, 1.04 mmol) and lithium hydroxide monohydrate (16 mg, 16.86 mmol) were added to a reaction flask and dissolved by adding tetrahydrofuran (4 mL), methanol (2 mL), and water (2 mL). The reaction liquid was stirred at room temperature for 2 h. The reaction liquid was adjusted to pH = 5-6 with diluted hydrochloric acid (1 M), added with water (10 mL) for dilution, and extracted with ethyl acetate (20 mL × 3). The combined organic phases were dried over anhydrous sodium sulfate and filtered, and the filtrate was concentrated under reduced pressure to give compound L008-2 (310 mg, yield: 83%). LC-MS: [M-tBu+H]⁺ = 302.00.

### 8.3: Synthesis of compound L008-3

L008-2 (310 mg, 0.87 mmol), tris(triphenylphosphine)rhodium chloride (81 mg, 0.09 mmol), and triethylamine (88 mg, 0.87 mmol) were added to a reaction flask and dissolved by adding methanol (15 mL) and tetrahydrofuran (15 mL). The reaction liquid was stirred at room temperature for 4 days under hydrogen atmosphere. The reaction liquid was filtered, and the filtrate was adjusted to pH = 10 with an aqueous sodium hydroxide solution (1 M) and extracted with ethyl acetate (40 mL). The organic phase was washed once with a saturated aqueous sodium bicarbonate solution (20 mL) and then discarded. All aqueous phases were combined, adjusted to pH = 5-6 with diluted hydrochloric acid (0.5 M), and then extracted with ethyl acetate (20 mL × 3). The combined organic phases were washed with saturated brine (50 mL), dried over anhydrous sodium sulfate, and filtered, and the filtrate was concentrated under reduced pressure to give compound L008-3 (crude product), which could be used directly in the next step. LC-MS: [M-tBu+H]⁺ = 304.00.

### 8.4: Synthesis of compound L008-4

L008-3 (100 mg, 0.28 mmol), 1-hydroxybenzotriazole (42.2 mg, 0.31 mmol), N,N-dimethylformamide (2 mL), N,N-diisopropylethylamine (143.7 mg, 1.11 mmol), and 1-(3-dimethylaminopropyl)-3-ethylcarbodiimide hydrochloride (58.5 mg, 0.31 mmol) were added to a reaction flask, and the reaction liquid was stirred at room temperature for 0.5 h. L001-7 (73.5 mg, 0.33 mmol) was then added, and the reaction liquid was reacted at room temperature for another 1 h. The reaction liquid was added with water (50 mL) for dilution and extracted with ethyl acetate (30 mL × 3). The combined organic phases were washed with a saturated aqueous sodium chloride solution (40 mL), dried over anhydrous sodium sulfate, and filtered, and the filtrate was concentrated under reduced pressure. The residue was purified by preparative high performance liquid chromatography (acidic condition) to give compound L008-4 (35.2 mg). LC-MS: [M+H]⁺ = 562.05.

### 8.5: Synthesis of compound L008 hydrochloride

L008-4 (35.2 mg, 0.063 mmol) was dissolved in dichloromethane (2 mL), and a solution of hydrogen chloride in ethyl acetate (1 mL, 4 M) was added. The reaction liquid was stirred at room temperature for 1 h. A solid was precipitated. The reaction liquid was filtered, and the filter cake was washed with ethyl acetate (5 mL). The filter cake was dissolved in deionized water (5 mL) and lyophilized to give compound L008 hydrochloride (23.7 mg).

¹H NMR (400 MHz, CH₃OH-*d₄*): δ 7.87 - 7.77 (m, 1H), 7.68 (d, *J =* 8.0 Hz, 2H), 7.56 (t, *J =* 8.0 Hz, 2H), 6.85 - 6.80 (m, 1H), 5.35 - 5.28 (m, 1H), 4.55 - 4.49 (m, 1H), 4.40 - 4.28 (m, 1H), 3.88 - 3.78 (m, 2H), 3.42 - 3.37 (m, 1H), 3.17 - 3.09 (m, 1H), 3.05 - 2.89 (m, 2H), 2.67 - 2.63 (m, 1H), 2.29 - 2.02 (m, 2H), 1.44 -1.40 (m, 3H); LC-MS: [M+H]⁺ = 462.05.

### Example 9: Synthesis of (2R,4S)-N-((2S)-1-((2-amino-6,7-dihydro-5H-cyclopenta[b]pyridin-5-yl) amino)-1-oxopropan-2-yl)-4-(1-phenylethyl)pyrrolidine-2-carboxamide (Compound L009) Hydrochloride

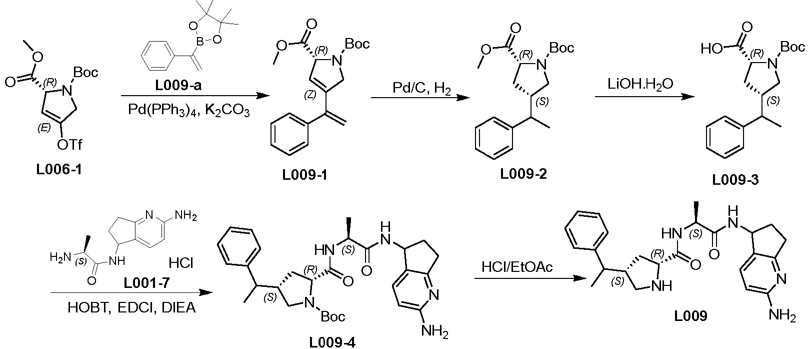

### 9.1: Synthesis of compound L009-1

L006-1 (600 mg, 1.6 mmol), L009-a (442 mg, 1.92 mmol), tetrakis(triphenylphosphine)palladium(0) (185 mg, 0.16 mmol), and potassium carbonate (661.9 mg, 4.8 mmol) were added to a reaction flask and dissolved by adding 1,4-dioxane (10 mL) and water (2 mL) under nitrogen atmosphere. The reaction liquid was reacted at 80 °C for 16 h. The reaction liquid was cooled to room temperature, added with water (50 mL) for dilution, and extracted with ethyl acetate (50 mL × 3). The combined organic phases were washed with saturated brine (50 mL), dried over anhydrous sodium sulfate, and filtered, and the filtrate was concentrated under reduced pressure. The residue was purified by silica gel column chromatography to give compound L009-1 (480 mg, yield: 91%). LC-MS: [M+H]⁺ = 330.10.

### 9.2: Synthesis of compound L009-2

L009-1 (120 mg, 0.4 mmol) was added to a reaction flask and dissolved by adding methanol (10 mL). Wet palladium on carbon (30 mg, 10% palladium content) was then added, and the reaction liquid was reacted at room temperature for 3 h under hydrogen atmosphere. The reaction liquid was filtered through celite, and the filtrate was concentrated under reduced pressure to give compound L009-2 (crude product), which was used directly in the next step. LC-MS: [M+H]⁺ = 334.10.

### 9.3: Synthesis of compound L009-3

L009-2 (122.7 mg, crude product) and lithium hydroxide monohydrate (129.7 mg, 5.4 mmol) were added to a reaction flask and dissolved by adding tetrahydrofuran (4 mL), methanol (2 mL), and water (2 mL). The reaction liquid was stirred at room temperature for 2 h. The reaction liquid was adjusted to pH = 5-6 with diluted hydrochloric acid (1 M), added with water (10 mL) for dilution, and extracted with ethyl acetate (20 mL × 3). The combined organic phases were dried over anhydrous sodium sulfate and filtered, and the filtrate was concentrated under reduced pressure to give compound L009-3 (100 mg) in the form of a colorless oily liquid. LC-MS: [M-tBu+H]⁺ = 264.10.

### 9.4: Synthesis of compound L009-4

L009-3 (100 mg, 0.31 mmol), 1-hydroxybenzotriazole (48 mg, 0.35 mmol), N,N-dimethylformamide (2 mL), N,N-diisopropylethylamine (162 mg, 1.25 mmol) and 1-(3-dimethylaminopropyl)-3-ethylcarbodiimide hydrochloride (66 mg, 0.35 mmol) were added to a reaction flask. The reaction liquid was reacted at room temperature for 0.5 h. L001-7 (83 mg, 0.38 mmol) was further added, and the reaction liquid was reacted at room temperature for another 1 h. The reaction liquid was added with water (40 mL) for dilution, and extracted with ethyl acetate (30 mL × 3). The combined organic phases were washed with a saturated aqueous sodium chloride solution (40 mL), dried over anhydrous sodium sulfate and filtered, and the filtrate was concentrated under reduced pressure. The residue was purified by preparative high performance liquid chromatography (acidic condition) to give compound L009-4 (51.7 mg, yield: 36.7%). LC-MS: [M+H]⁺ = 522.05.

### 9.5: Synthesis of compound L009 hydrochloride

L009-4 (51.7 mg, 0.1 mmol) was dissolved in dichloromethane (2 mL), and a solution of hydrogen chloride in ethyl acetate (1 mL, 4 M) was added at room temperature. The reaction liquid was stirred for 0.5 h, and a solid was precipitated. The reaction liquid was filtered, and the filter cake was washed with ethyl acetate (5 mL). The filter cake was dissolved in deionized water (2 mL) and lyophilized to give compound L009 hydrochloride (35.9 mg, yield: 73.1%).

¹H NMR (400 MHz, CH₃OH-*d₄*): δ 7.71 - 7.59(m, 1H), 7.17(t, *J =* 7.2 Hz, 2H), 7.11 - 7.07(m, 3H), 6.72 - 6.67(m, 1H), 5.18 - 5.09(m, 1H), 4.23 - 4.19(m, 1H), 4.16 - 4.07(m, 1H), 3.52 - 3.48(m, 0.5H), 3.03 - 2.96(m, 1.5H), 2.89 - 2.60(m, 3H), 2.53 - 2.46(m, 3H), 2.05 - 1.87(m, 2H), 1.28(d, *J* = 7.2 Hz, 2H), 1.21 - 1.15(m, 4H); LC-MS: [M+H]⁺ = 422.15.

### Example 10: Synthesis of (2R,4S)-N-((2S)-1-((2-amino-6,7-dihydro-5H-cyclopenta[b]pyridin-5-yl) amino)-1-oxopropan-2-yl)-4-(4-(pyridin-3-yl)benzyl)pyrrolidine-2-carboxamide (Compound L010) Hydrochloride

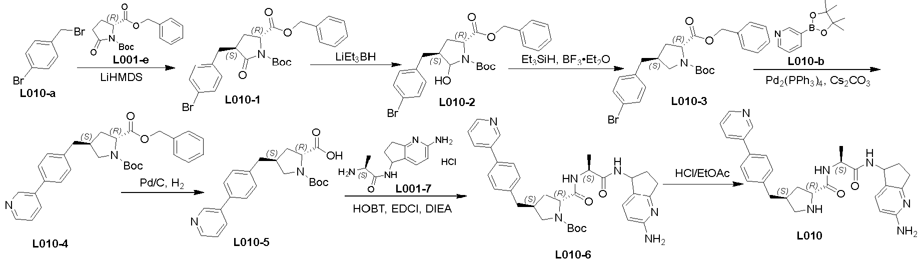

### 10.1: Synthesis of compound L010-1

L001-e (5.1 g, 16.0 mmol) was dissolved in tetrahydrofuran (40 mL), and then cooled to -78 °C under nitrogen atmosphere. Lithium bis(trimethylsilyl)amide (24 mL, 24.0 mmol, 1 M in tetrahydrofuran ) was added slowly. The reaction liquid was stirred at this temperature for 0.5 h. L010-a (4.0 g, 16.0 mmol) was then added slowly, and the reaction liquid was reacted at -78 °C for 2 h. The reaction liquid was quenched with a saturated ammonium chloride solution (30 mL) at -78 °C. The reaction system was warmed to room temperature and extracted with ethyl acetate (30 mL × 3). The combined organic phases were washed with saturated sodium chloride (20 mL), dried over anhydrous sodium sulfate and filtered, and the filtrate was concentrated under reduced pressure. The residue was purified by silica gel column chromatography to give compound L010-1 (2.8 g, yield: 35.8%). LC-MS: [M-Boc+H]⁺ = 387.90

### 10.2: Synthesis of compound L010-2

L010-1 (2.0 g, 4.1 mmol) was dissolved in tetrahydrofuran (25 mL), and the mixture was cooled to - 78 °C under nitrogen atmosphere. Lithium triethylborohydride (4.5 mL, 4.5 mmol, 1 M in tetrahydrofuran) was added slowly, and the mixture was stirred and reacted with the temperature controlled at -78 °C for 2 h. Water (15 mL) was added to quench the reaction, and the mixture was extracted with ethyl acetate (20 mL × 3). The combined organic phases were washed with a saturated aqueous sodium chloride solution (20 mL), dried over anhydrous sodium sulfate and filtered, and the filtrate was concentrated under reduced pressure. The residue was purified by silica gel column chromatography to give compound L010-2 (1.1 g, yield: 54.77%). LC-MS: [M-OH]⁺ = 473.95.

### 10.3: Synthesis of compound L010-3

L010-2 (1.0 g, 2.1 mmol) was dissolved in dichloromethane (10 mL), and the mixture was cooled to - 78 °C under nitrogen atmosphere. Triethylsilane (360 mg, 3.1 mmol) was added slowly, boron trifluoride diethyl ether coordination complex (436 mg, 3.1 mmol) was added dropwise and slowly, and the mixture was stirred and reacted at -78 °C for 2 h. Water (15 mL) was added to quench the reaction at this temperature, and after being warmed to room temperature, the reaction system was extracted with dichloromethane (15 mL × 3). The combined organic phases were washed with a saturated aqueous sodium chloride solution (20 mL), dried over anhydrous sodium sulfate and filtered, and the filtrate was concentrated under reduced pressure. The residue was purified by silica gel column chromatography to give compound L010-3 (400 mg, yield: 41.35%). LC-MS: [M-Boc+H]⁺ = 375.95.

### 10.4: Synthesis of compound L010-4

L010-4 (400 mg, 0.84 mmol), L010-b (181.6 mg, 0.89 mmol), cesium carbonate (550 mg, 1.69 mmol) and tetrakis(triphenylphosphine)palladium(0) (107 mg, 0.08 mmol) were added sequentially to 1,4-dioxane (5 mL) and water (1 mL). The reaction liquid was stirred at 100 °C for 2 h under nitrogen atmosphere. The reaction liquid was cooled to room temperature, and a solid was undissolved. The reaction liquid was filtered, water (10 mL) was added to the filtrate, and the mixture was extracted with ethyl acetate (10 mL × 3). The combined organic phases were washed with a saturated aqueous sodium chloride solution (10 mL), dried over anhydrous sodium sulfate and filtered, and the filtrate was concentrated under reduced pressure. The residue was purified by silica gel column chromatography to give compound L010-4 (300 mg, yield: 75.29%).

### 10.5: Synthesis of compound L010-5

L010-4 (300 mg, 0.63 mmol) was dissolved in methanol (2 mL), and wet palladium on carbon (100 mg, 10% palladium content) was added. The reaction liquid was stirred at 20 °C for 1 h under hydrogen atmosphere. The reaction liquid was filtered through celite, and the filtrate was concentrated under reduced pressure. The residue was purified by silica gel column chromatography to give compound L010-5 (140 mg, yield: 57.66%). LC-MS: [M+H]⁺ = 383.05.

### 10.6: Synthesis of compound L010-6

L010-5 (130 mg, 0.34 mmol) was dissolved in N,N-dimethylformamide (2 mL). 1-Hydroxybenzotriazole (50.5 mg, 0.37 mmol) and 1-(3-dimethylaminopropyl)-3-ethylcarbodiimide hydrochloride (70.1 mg, 0.37 mmol) were added. The reaction liquid was stirred at room temperature for 10 min. A solution of L001-7 (74.9 mg, 0.34 mmol) and N,N-diisopropylethylamine (175.4 mg, 1.36 mmol) in N,N-dimethylformamide (2 mL) was added. The reaction liquid was stirred at room temperature for another 1.5 h. The reaction liquid was directly poured into water (10 mL), and the mixture was extracted with ethyl acetate (15 mL × 3). The combined organic phases were washed with a saturated aqueous ammonium chloride solution (10 mL × 3), dried over anhydrous sodium sulfate and filtered, and the filtrate was concentrated under reduced pressure. The residue was purified by preparative high performance liquid chromatography (basic condition) and lyophilized to give compound L010-6 (49 mg, yield: 24.65%). LC-MS: [M+H]⁺ = 585.15.

### 10.7: Synthesis of compound L010 hydrochloride

L010-6 (49 mg, 0.084 mmol) was dissolved in dichloromethane (2 mL), and a solution of hydrogen chloride in ethyl acetate (1 mL, 4 M) was added at room temperature. The reaction liquid was stirred for 0.5 h, and a solid was precipitated. The reaction liquid was filtered, and the filter cake was washed with ethyl acetate (10 mL). The filter cake was dissolved in deionized water (10 mL) and lyophilized to give compound L010 hydrochloride (18.5 mg, yield: 45.6%).

¹H NMR (400 MHz, DMSO-*d₆*): δ 10.70 (s, 1H), 9.24 (s, 1H), 8.99 (t, *J =* 7.2 Hz, 1H), 8.85 (d, *J =* 6.0 Hz, 2H), 8.70 (t, *J =* 7.6 Hz, 1H), 8.61 (s, 1H), 8.33 - 7.98 (m, 3H), 7.85 (d, *J =* 8.0 Hz, 2H), 7.68 (d, *J =* 8.8 Hz, 1H), 7.48 (d, *J =* 8.0 Hz, 2H), 6.87 (d, *J =* 8.8 Hz, 1H), 5.14 (q, *J =* 7.2 Hz, 1H), 4.43 (s, 1H), 4.1 - 4.19 (m, 1H), 3.30 (s, 1H), 3.12 - 2.99 (m, 1H), 2.98 - 2.73 (m, 4H), 2.61 - 2.51 (m, 1H), 2.48 - 2.38 (m, 1H), 2.16 - 2.07 (m, 1H), 2.01 - 1.90 (m, 2H), 1.23 (d, *J* = 7.2 Hz, 3H); LC-MS: [M+H]⁺ = 485.10.

### Example 11: Synthesis of (2R,4S)-N-((2S)-1-((2-amino-6,7-dihydro-5H-cyclopenta[b]pyridin-5-yl) amino)-1-oxopropan-2-yl)-4-((2-methylpyridin-4-yl)methyl)pyrrolidine-2-carboxamide (Compound L011) Hydrochloride

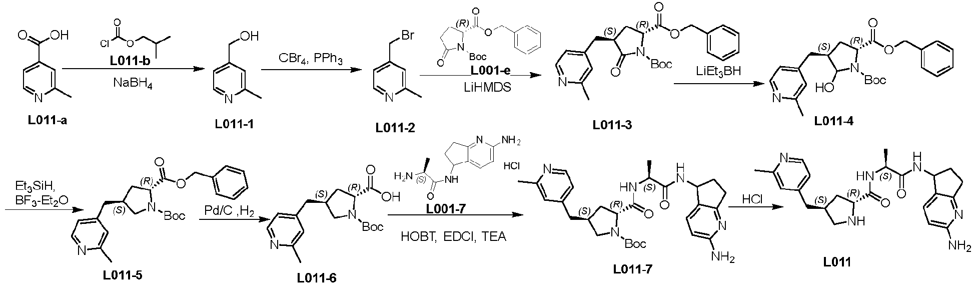

### 11.1: Synthesis of compound L011-1

L011-a (3.0 g, 21.90 mmol) was added to tetrahydrofuran (40 mL). L011-b (3.6 g, 26.30 mmol) was added dropwise at 0 °C. After the dropwise addition was completed, the reaction liquid was warmed to 20 °C and stirred for 0.5 h. A large amount of white solid was precipitated, the solid was filtered, and the filtrate was cooled to 0 °C. A solution of sodium borohydride (1.25 g, 32.90 mmol) in water (5 mL) was added. The reaction liquid was stirred at 0 °C for 0.5 h. The reaction liquid was adjusted to pH = 5-6 with diluted hydrochloric acid (1 M) and extracted with ethyl acetate (20 mL × 3). The organic phase was discarded. The aqueous phase was adjusted to pH = 8 with sodium carbonate and extracted with ethyl acetate (20 mL × 3). The combined organic phases were dried over anhydrous sodium sulfate and filtered, and the filtrate was concentrated under reduced pressure to give compound L011-1 (1.0 g, yield: 38.5%). LC-MS: [M+H]⁺ = 124.20.

### 11.2: Synthesis of compound L011-2

L011-1 (1.3 g, 10.60 mmol) and triphenylphosphine (3.6 g, 13.70 mmol) were added to dichloromethane (20 mL). A solution of tetrabromomethane (4.5 g, 13.70 mmol) in dichloromethane (5 mL) was added dropwise at 0 °C. After the addition was completed, the reaction liquid was stirred at 20 °C for 0.5 h. The reaction liquid was added with water (20 mL) for dilution and extracted with ethyl acetate (20 mL × 3). The organic phase was dried over anhydrous sodium sulfate and filtered. The filtrate was added with toluene (20 mL) and concentrated under reduced pressure to 10 mL. Toluene (20 mL) was added again, and the mixture was concentrated under reduced pressure to 5 mL to give a solution of compound L011-2 in toluene. The solution was used directly in the next step. LC-MS: [M+H]⁺ = 188.00.

### 11.3: Synthesis of compound L011-3

L001-e (2.2 g, 7.00 mmol) was added to tetrahydrofuran (20 mL), and the mixture was cooled to -78 °C. Lithium bis(trimethylsilyl)amide (7.7 mL, 7.70 mmol, 1 M in tetrahydrofuran) was added dropwise under nitrogen atmosphere. After the dropwise addition was completed, the reaction liquid was stirred at -78 °C for 0.5 h. A solution of L011-2 in toluene (~5 mL, the crude product solution obtained in the previous step) was then added dropwise. The reaction liquid was stirred at -78 °C for 1 h. The reaction liquid was quenched by adding an aqueous ammonium chloride solution (10 mL) at -78 °C, and after being warmed to room temperature, the system was extracted with ethyl acetate (10 mL × 3). The combined organic phases were dried over anhydrous sodium sulfate and filtered, and the filtrate was concentrated under reduced pressure. The residue was purified by silica gel column chromatography to give compound L011-3 (1.28 g, yield: 43.1%). LC-MS: [M+H]⁺ = 425.10.

### 11.4: Synthesis of compound L011-4

L011-3 (1.28 g, 3.00 mmol) was added to tetrahydrofuran (15 mL) at room temperature, and the mixture was cooled to -78 °C. Lithium triethylborohydride (3.6 mL, 3.6 mmol, 1 M in tetrahydrofuran) was added dropwise under nitrogen atmosphere. After the dropwise addition was completed, the reaction liquid was stirred at -78 °C for 0.5 h. The reaction liquid was quenched by adding a saturated aqueous sodium bicarbonate solution (10 mL) at -78 °C, and after being warmed to room temperature, the reaction system was extracted with ethyl acetate (10 mL × 3). The combined organic phases were dried over anhydrous sodium sulfate and filtered, and the filtrate was concentrated under reduced pressure to give compound L011-4 (crude product), which was used directly in the next step. LC-MS: [M+H]⁺ = 427.10.

### 11.5: Synthesis of compound L011-5

L011-4 (1.3 g, 3.00 mmol) was dissolved in dichloromethane (20 mL) at room temperature, and the mixture was cooled to -78 °C. Triethylsilane (650 mg, 4.50 mmol) and boron trifluoride diethyl ether coordination complex (522 mg, 4.50 mmol) were added dropwise under nitrogen atmosphere. The reaction liquid was reacted at -78 °C for 1 h. The reaction liquid was quenched by adding a saturated aqueous sodium bicarbonate solution (10 mL) at -78 °C, and after being warmed to room temperature, the system was extracted with ethyl acetate (10 mL × 3). The combined organic phases were dried over anhydrous sodium sulfate and filtered, and the filtrate was concentrated under reduced pressure to give compound L011-5 (crude product), which was used directly in the next step. LC-MS: [M+H]⁺ = 411.10.

### 11.6: Synthesis of compound L011-6

L011-5 (400 mg, 0.98 mmol) and wet palladium on carbon (200 mg, 10% palladium content) were added to methanol (4 mL). The reaction liquid was stirred at room temperature for 0.5 h under hydrogen atmosphere. The reaction liquid was filtered, and the filtrate was concentrated under reduced pressure to give L011-6 (crude product), which was used directly in the next step. LC-MS: [M+H]⁺ = 321.10.

### 11.7: Synthesis of compound L011-7

L011-6 (110 mg, crude product), L001-7 (100 mg, 0.34 mmol), 1-(3-dimethylaminopropyl)-3-ethylcarbodiimide hydrochloride (97 mg, 0.51 mmol), 1-hydroxybenzotriazole (77 mg, 0.51 mmol) and triethylamine (104 mg, 1.02 mmol) were added to dichloromethane (3 mL). The reaction liquid was stirred at room temperature for 0.5 h. The reaction liquid was added with water (20 mL) for dilution and extracted with dichloromethane (10 mL × 3). The combined organic phases were dried over anhydrous sodium sulfate and filtered, and the filtrate was concentrated under reduced pressure. The residue was purified by preparative high performance liquid chromatography (acidic condition) to give compound L011-7 (70 mg, yield: 38.5%). LC-MS: [M+H]⁺ = 523.10.

### 11.8: Synthesis of compound L011 hydrochloride

L011-7 (70 mg, 0.13 mmol) was dissolved in dichloromethane (2 mL), and then a solution of hydrogen chloride in 1,4-dioxane (2 mL, 4 M) was added dropwise at room temperature. The reaction liquid was stirred for 0.5 h. A solid was precipitated. The reaction liquid was filtered, and the filter cake was washed with dichloromethane (5 mL). The filter cake was dissolved in deionized water (10 mL) and lyophilized to give compound L011 hydrochloride (39.0 mg, yield: 63.8%).

¹H NMR (400 MHz, DMSO-*d₆*): δ 10.75 - 10.69 (m, 1H), 8.93 (t, *J =* 6.8 Hz, 1H), 8.71 - 8.58 (m, 3H), 8.05 (s, 2H), 7.86 (s, 1H), 7.80 (d, *J =* 5.6 Hz, 1H), 7.85 - 7.63 (m, 1H), 6.83 (d, *J =* 8.8 Hz, 1H), 5.13 (m, 1H), 4.41 (s, 1H), 4.33 - 4.16 (m, 1H), 3.29 (d, *J =* 4.4 Hz, 2H), 3.10 - 2.97 (m, 1H), 2.96 - 2.84 (m, 3H), 2.70 (s, 3H), 2.62 (s, 1H), 2.46 - 2.32 (m, 1H), 2.15 - 2.04 (m, 1H), 2.02 - 1.87 (m, 2H), 1.22 (d, *J =* 7.2 Hz, 3H); LC-MS: [M+H]⁺ = 423.10.

### Example 12: Synthesis of (2R,4S)-N-((2S)-1-((2-amino-6,7-dihydro-5H-cyclopenta[b]pyridin-5-yl) amino)-1-oxopropan-2-yl)-4-(3-chloro-4-fluorobenzyl)pyrrolidine-2-carboxamide (Compound L012) Hydrochloride

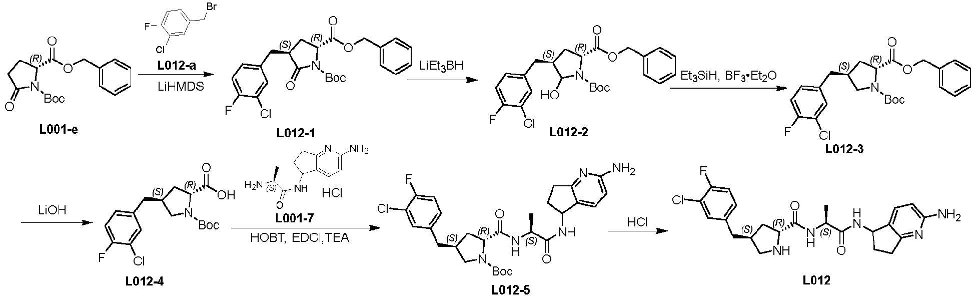

### 12.1: Synthesis of compound L012-1

L001-e (1.5 g, 4.69 mmol) was dissolved in tetrahydrofuran (15 mL) at room temperature, and the mixture was cooled to -78 °C. Lithium bis(trimethylsilyl)amide (5.16 mL, 5.16 mmol, 1 M in tetrahydrofuran) was added dropwise under nitrogen atmosphere. After the dropwise addition was completed, the reaction liquid was stirred at -78 °C for 0.5 h. A solution of L012-a (1.15 g, 5.16 mmol) in tetrahydrofuran (10 mL) was added, and the reaction liquid was stirred at -78 °C for another 2 h. The reaction liquid was quenched by adding an aqueous ammonium chloride solution (10 mL) at this temperature, and after being warmed to room temperature, the system was extracted with ethyl acetate (50 mL × 3). The combined organic phases were dried over anhydrous sodium sulfate and filtered, and the filtrate was concentrated under reduced pressure. The residue was purified by silica gel column chromatography to give compound L012-1 (1.5 g, yield: 69.1%). LC-MS: [M-Boc+H]⁺ = 361.95.

### 12.2: Synthesis of compound L012-2

L012-1 (1.5 g, 3.20 mmol) was dissolved in tetrahydrofuran (25 mL) at room temperature, and the mixture was cooled to -78 °C. Lithium triethylborohydride (3.5 mL, 3.5 mmol, 1 M in tetrahydrofuran) was added dropwise under nitrogen atmosphere. After the dropwise addition was completed, the reaction liquid was stirred at -78 °C for 0.5 h. The reaction liquid was quenched by adding a saturated aqueous sodium bicarbonate solution (10 mL) at this temperature, and after being warmed to room temperature, the system was extracted with ethyl acetate (50 mL × 3). The combined organic phases were dried over anhydrous sodium sulfate and filtered, and the filtrate was concentrated under reduced pressure to give compound L012-2 (crude product), which was used directly in the next step.

### 12.3: Synthesis of compound L012-3

L012-2 (1.5 g, crude product) was dissolved in dichloromethane (15 mL) at room temperature, and the reaction liquid was cooled to -78 °C. Under nitrogen atmosphere, triethylsilane (487 mg, 4.20 mmol) was added, and then boron trifluoride diethyl ether coordination complex (600 mg, 4.20 mmol) was added dropwise. After the dropwise addition was completed, the reaction liquid was stirred at -78 °C for 1 h. The reaction liquid was quenched by adding a saturated aqueous sodium bicarbonate solution (10 mL) at this temperature, and after being warmed to room temperature, the system was extracted with ethyl acetate (50 mL × 3). The combined organic phases were dried over anhydrous sodium sulfate and filtered, and the filtrate was concentrated under reduced pressure to give compound L012-3 (crude product), which was used directly in the next step. LC-MS: [M+H]⁺ = 447.95.

### 12.4: Synthesis of compound L012-4

L012-3 (500 mg, 1.16 mmol) was dissolved in tetrahydrofuran (4 mL) and water (2 mL) at room temperature, and lithium hydroxide monohydrate (180 mg, 4.46 mmol) was added. The reaction liquid was stirred at room temperature for 16 h. The reaction liquid was directly concentrated under reduced pressure, and the residue was dissolved in water (10 mL). The aqueous phase was adjusted to pH = 5-6 with diluted hydrochloric acid (1 M) and extracted with ethyl acetate (20 mL × 3). The combined organic phases were dried over anhydrous sodium sulfate and filtered, and the filtrate was concentrated under reduced pressure to give compound L012-4 (crude product), which was used directly in the next step. LC-MS: [M-tBu+H]⁺ = 301.95.

### 12.5: Synthesis of compound L012-5

L012-4 (170 mg, crude product), L001-7 (140 mg, 0.48 mmol), 1-(3-dimethylaminopropyl)-3-ethylcarbodiimide hydrochloride (137 mg, 0.72 mmol), 1-hydroxybenzotriazole (109 mg, 0.72 mmol) and triethylamine (146 mg, 1.44 mmol) were added to dichloromethane (3 mL). The reaction liquid was stirred at room temperature for 0.5 h. The reaction liquid was added with water (20 mL) for dilution and extracted with dichloromethane (10 mL × 3). The organic phases were combined, dried over anhydrous sodium sulfate and filtered, and the filtrate was concentrated under reduced pressure. The residue was purified by preparative high performance liquid chromatography to give compound L012-5 (100 mg, yield: 37.5%). LC-MS: [M+H]⁺ = 560.00.

### 12.6: Synthesis of compound L012 hydrochloride

L012-5 (100 mg, 0.18 mmol) was dissolved in dichloromethane (1 mL), and a solution of hydrogen chloride in 1,4-dioxane (1 mL, 4 M) was added. The reaction liquid was stirred at room temperature for 1 h. A solid was precipitated. The reaction liquid was filtered, and the filter cake was dissolved in deionized water (10 mL) and lyophilized to give compound L012 hydrochloride (69.7 mg, yield: 78.7%).

¹H NMR (400 MHz, DMSO-*d₆*): δ 10.29 - 10.19 (m, 1H), 8.82 - 8.78 (m, 1 H), 8.54 - 8.52 (m, 2H), 7.94 (s, 2H), 7.66 - 7.65 (m, 1H), 7.48 - 7.45 (m, 1H), 7.34 (t, *J =* 8.8 Hz, 1H), 7.29 - 7.17 (m, 1H), 6.80 *(d, J =* 8.8 Hz, 1H), 5.14 - 5.12 (m, 1H), 4.35 - 4.18 (m, 2H), 3.26 - 3.24 (m, 1H), 2.94 - 2.84 (m, 3H), 2.78 - 2.59 (m, 2H), 2.46 - 2.40 (m, 1H), 2.10 - 1.83 (m, 3H), 1.24 - 1.21 (m, 4H); LC-MS: [M+H]⁺ =459.95.

### Example 13: Synthesis of (2R,4R)-N-((2S)-1-((2-amino-6,7-dihydro-5H-cyclopenta[b]pyridin-5-yl) amino)-1-oxopropan-2-yl)-4-((5-chlorobenzo[d]thiazol-2-yl)methyl)pyrrolidine-2-carboxamide (Compound L013) Hydrochloride

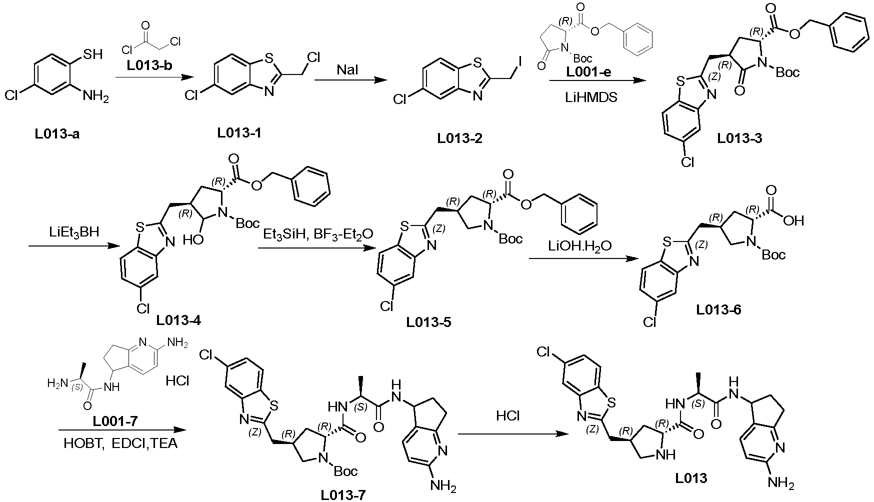

### 13.1: Synthesis of compound L013-1

L013-a (2 g, 12.50 mmol) was added to toluene (15 mL). L013-b (2.8 g, 25.00 mmol) was added dropwise at room temperature. After the dropwise addition was completed, the reaction liquid was stirred at 100 °C for 2 h. The reaction liquid was cooled to room temperature and concentrated under reduced pressure to remove toluene. The residue was added with petroleum ether (100 mL). The mixture was stirred at room temperature for 1 h and filtered, and the filtrate was concentrated under reduced pressure to give compound L013-1 (1.5 g, yield: 55.5%). LC-MS: [M+H]⁺ = 217.95.

### 13.2: Synthesis of compound L013-2

L013-1 (1.5 g, 6.88 mmol) and sodium iodide (2.1 g, 13.76 mmol) were added to tetrahydrofuran (20 mL), and the reaction liquid was stirred at 40 °C for 2 h. The reaction liquid was cooled to room temperature, then added with water (20 mL) for dilution, and extracted with ethyl acetate (20 mL × 3). The combined organic phases were dried over anhydrous sodium sulfate and filtered, and the filtrate was concentrated under reduced pressure to give compound L013-2 (2.05 g, yield: 96.2%). LC-MS: [M+H]⁺= 309.80.

### 13.3: Synthesis of compound L013-3

L001-e (2.11 g, 6.60 mmol) was dissolved in tetrahydrofuran (20 mL) at room temperature, and the reaction liquid was cooled to -78 °C. Lithium bis(trimethylsilyl)amide (7.3 mL, 7.3 mmol, 1 M in tetrahydrofuran) was added dropwise under nitrogen atmosphere, and after the dropwise addition was completed, the reaction liquid was stirred for 0.5 h. A solution of L013-2 (2.05 g, 6.6 mmol) in tetrahydrofuran (10 mL) was then added dropwise, and after the dropwise addition was completed, the reaction liquid was stirred at -78 °C for another 1 h. The reaction liquid was quenched by adding an aqueous ammonium chloride solution (10 mL) at this temperature, and after being warmed to room temperature, the system was extracted with ethyl acetate (10 mL × 3). The combined organic phases were dried over anhydrous sodium sulfate and filtered, and the filtrate was concentrated under reduced pressure. The residue was purified by silica gel column chromatography to give compound L013-3 (2 g, yield: 60.6%). LC-MS: [M+H]⁺ = 500.90.

### 13.4: Synthesis of compound L013-4

L013-3 (1.3 g, 2.60 mmol) was dissolved in tetrahydrofuran (15 mL) at room temperature, and then cooled to -78 °C. Lithium triethylborohydride (2.9 mL, 2.90 mmol, 1 M in tetrahydrofuran) was then added dropwise under nitrogen atmosphere. After the dropwise addition was completed, the reaction liquid was stirred at -78 °C for 0.5 h. The reaction liquid was quenched by adding a saturated aqueous sodium bicarbonate solution (10 mL) at this temperature, and after being warmed to room temperature, the system was extracted with ethyl acetate (10 mL × 3). The combined organic phases were dried over anhydrous sodium sulfate and filtered, and the filtrate was concentrated under reduced pressure to give compound L013-4 (crude product), which was used directly in the next step. LC-MS: [M-OH]⁺ = 484.90.

### 13.5: Synthesis of compound L013-5

L013-4 (1.3 g, crude product) was dissolved in dichloromethane (20 mL) at room temperature and the reaction liquid was cooled to -78 °C. Under nitrogen atmosphere, triethylsilane (382 mg, 3.30 mmol) was added, and boron trifluoride diethyl ether coordination complex (472 mg, 3.30 mmol) was added dropwise. After the dropwise addition was completed, the reaction liquid was stirred at -78 °C for 1 h. The reaction liquid was quenched by adding a saturated aqueous sodium bicarbonate solution (10 mL) at this temperature, and after being warmed to room temperature, the system was extracted with ethyl acetate (10 mL × 3). The combined organic phases were dried over anhydrous sodium sulfate and filtered, and the filtrate was concentrated to give compound L013-5 (crude product), which was used directly in the next step. LC-MS: [M+H]⁺ = 487.00.

### 13.6: Synthesis of compound L013-6

L013-5 (300 mg, 0.61 mmol) and lithium hydroxide monohydrate (100 mg, 2.40 mmol) were added to tetrahydrofuran (6 mL) and water (2 mL). The reaction liquid was stirred at room temperature for 16 h. The reaction liquid was directly concentrated under reduced pressure and added with water (10 mL) for dilution. The aqueous phase was adjusted to pH = 5-6 with hydrochloric acid (1 M) and extracted with ethyl acetate (10 mL × 3). The combined organic phases were dried over anhydrous sodium sulfate and filtered, and the filtrate was concentrated under reduced pressure to give compound L013-6 (150 mg, crude product), which was used directly in the next step. LC-MS: [M+H]⁺= 396.95.

### 13.7: Synthesis of compound L013-7

L013-6 (150 mg, crude product), L001-7 (110 mg, 0.37 mmol), 1-(3-dimethylaminopropyl)-3-ethylcarbodiimide hydrochloride (106 mg, 0.55 mmol), 1-hydroxybenzotriazole (84 mg, 0.55 mmol) and triethylamine (112 mg, 1.10 mmol) were added to dichloromethane (3 mL). The reaction liquid was stirred at room temperature for 0.5 h. The reaction liquid was added with water (20 mL) for dilution and extracted with dichloromethane (10 mL × 3). The combined organic phases were dried over anhydrous sodium sulfate and filtered, and the filtrate was concentrated under reduced pressure. The residue was purified by preparative high performance liquid chromatography (acidic condition) to give compound L013-7 (120 mg, yield: 49.6%). LC-MS: [M+H]⁺ = 599.00.

### 13.8: Synthesis of compound L013 hydrochloride

L013-7 (120 mg, 0.20 mmol) was dissolved in dichloromethane (2 mL). A solution of hydrogen chloride in 1,4-dioxane (4 M, 2 mL) was added dropwise at room temperature, and the reaction liquid was stirred for 0.5 h. A solid was precipitated. The reaction liquid was filtered, and the filter cake was washed with dichloromethane (3 mL). The filter cake was dissolved in deionized water (10 mL) and lyophilized to give compound L013 hydrochloride (50.4 mg, yield: 78.7%).

¹H NMR (400 MHz, DMSO-*d₆*): δ 10.46 - 10.44 (m, 1H), 8.87 (dd, *J*₁= 7.2 Hz, *J*₂ = 2.8 Hz, 1H), 8.72 - 8.53 (m, 2H), 8.11 (d, *J =* 8.8 Hz, 1H), 8.03 - 8. 00 (m, 3H), 7.68 - 7.64 (m, 1H), 7.47 (dd, *J*₁ = 8.8 Hz, *J*₂ = 2.0 Hz, 1H), 6.82 (dd, *J*₁ = 8.8 Hz, *J*₂ = 2.4 Hz, 1H), 5.17 - 5.11 (m, 1H), 4.38 (s, 1H), 4.26 - 4.22 (m, 1H), 3.54 - 3.42 (m, 1H), 3.31 (d, *J =* 7.2 Hz, 2H), 3.13 - 2.96 (m, 2H), 2.85 - 2.82 (m, 2H), 2.47 - 2.37 (m, 1H), 2.12 (m, 2H), 1.98 - 1.87 (m, 1H), 1.22 (d, *J =* 7.2 Hz, 3H); LC-MS: [M+H]⁺= 499.00.

### Example 14: Synthesis of (2R,4S)-N-((2S)-1-((2-amino-6,7-dihydro-5H-cyclopenta[b]pyridin-5-yl) amino)-1-oxopropan-2-yl)-4-(3-chlorobenzyl)pyrrolidine-2-carboxamide (Compound L014) Hydrochloride

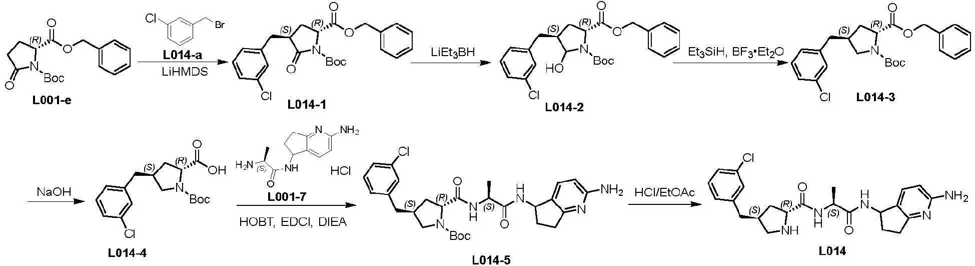

### 14.1: Synthesis of compound L014-1

L001-e (1.0 g, 3.13 mmol) was added to a reaction flask, and under nitrogen atmosphere, dissolved by adding tetrahydrofuran (10 mL), and placed in a cold bath at -78 °C. Lithium bis(trimethylsilyl)amide (3.2 mL, 3.2 mmol, 1 M in tetrahydrofuran) was added with the temperature maintained at -78 °C, and the reaction liquid was stirred for 0.5 h after the addition was completed. L014-a (639 mg, 3.13 mmol) was then added, and the reaction liquid was reacted at -78 °C for another 2 h. A small amount of solid was precipitated. The reaction liquid was quenched with a saturated aqueous ammonium chloride solution (10 mL) at -78 °C, and after being warmed to room temperature, the system was extracted with ethyl acetate (20 mL × 3). The combined organic phases were dried over anhydrous sodium sulfate and filtered, and the filtrate was concentrated under reduced pressure. The residue was purified by silica gel column chromatography to give compound L014-1 (950 mg, yield: 68%). LC-MS: [M-Boc+H]⁺ = 344.00.

### 14.2: Synthesis of compound L014-2

L014-1 (950 mg, 2.14 mmol) was added to a reaction flask, and under nitrogen atmosphere, dissolved by adding tetrahydrofuran (10 mL), and placed in a cold bath at -78 °C. Lithium triethylborohydride (3.2 mL, 3.20 mmol, 1 M in tetrahydrofuran) was added with the temperature maintained at -78 °C, and the reaction liquid was stirred for 0.5 h after the addition was completed. The reaction liquid was quenched by adding water (50 mL) at this temperature, and after being warmed to room temperature, the system was extracted with ethyl acetate (50 mL × 3). The combined organic phases were dried over anhydrous sodium sulfate and filtered, and the filtrate was concentrated under reduced pressure to give compound L014-2 (crude product), which could be used directly in the next step. LC-MS: [M-OH]⁺= 428.05.

### 14.3: Synthesis of compound L014-3

L014-2 (1.1 g crude product) was added to a reaction flask, and under nitrogen atmosphere, dissolved by adding dichloromethane (10 mL), and placed in a cold bath at -78 °C. Triethylsilane (345 mg, 2.97 mmol) and boron trifluoride diethyl ether coordination complex (421 mg, 2.97 mmol) were separately added with the temperature maintained at -78 °C, and the reaction liquid was stirred for 0.5 h after the addition was completed. The reaction liquid was supplemented with triethylsilane (345 mg, 2.97 mmol) and boron trifluoride diethyl ether coordination complex (421 mg, 2.97 mmol) and stirred at - 78 °C for another 2 h. The reaction liquid was quenched by adding water (50 mL) at this temperature, and after being warmed to room temperature, the system was extracted with ethyl acetate (50 mL × 3).

The combined organic phases were dried over anhydrous sodium sulfate and filtered, and the filtrate was concentrated under reduced pressure. The residue was purified by silica gel column chromatography to give compound L014-3 (830 mg, yield: 84%). LC-MS: [M+H]⁺ = 430.05.

### 14.4: Synthesis of compound L014-4

L014-3 (830 mg, 1.93 mmol) and sodium hydroxide (154 mg, 3.87 mmol) were added to a reaction flask and dissolved by adding methanol (10 mL) and water (5 mL). The reaction liquid was reacted at room temperature for 16 h. The reaction liquid was concentrated under reduced pressure until the volume was 2-3 mL, added with water (40 mL) for dilution, adjusted to pH = 5-6 with diluted hydrochloric acid (0.5 M), and extracted with ethyl acetate (30 mL × 3). The combined organic phases were dried over anhydrous sodium sulfate and filtered, and the filtrate was concentrated under reduced pressure to give compound L014-4 (crude product), which could be used directly in the next step. LC-MS: [M+Na]⁺ = 362.00.

### 14.5: Synthesis of compound L014-5

L014-4 (100 mg, crude product), 1-hydroxybenzotriazole (78 mg, 0.32 mmol), N,N-dimethylformamide (2 mL), N,N-diisopropylethylamine (170 mg, 1.18 mmol) and 1-(3-dimethylaminopropyl)-3-ethylcarbodiimide hydrochloride (62 mg, 0.32 mmol) were added to a reaction flask. The reaction liquid was reacted at room temperature for 0.5 h. L001-7 (78 mg, 0.35 mmol) was further added. The reaction liquid was reacted at room temperature for another 1 h. The reaction liquid was added with water (40 mL) for dilution and extracted with ethyl acetate (30 mL × 3). The organic phases were combined, washed with a saturated aqueous sodium chloride solution (40 mL), dried over anhydrous sodium sulfate and filtered, and the filtrate was concentrated under reduced pressure. The residue was purified by preparative high performance liquid chromatography (acidic condition) to give compound L014-5 (16 mg). LC-MS: [M+H]⁺ = 542.00.

### 14.6: Synthesis of compound L014 hydrochloride

L014-5 (16 mg, 0.03 mmol) was dissolved in dichloromethane (2 mL), and a solution of hydrogen chloride in ethyl acetate (1 mL, 4 M) was added at room temperature. The reaction liquid was stirred for 0.5 h, and a white solid was precipitated. The reaction liquid was filtered, and the filter cake was washed with ethyl acetate (10 mL). The filter cake was dissolved in deionized water (2 mL) and lyophilized to give compound L014 hydrochloride (10.5 mg).

¹H NMR (400 MHz, CH₃OH-*d₄*): δ 7.84 -7.76(m, 1H), 7.33 - 7.16(m, 4H), 6.82(d, *J =* 9.2 Hz, 1H), 5.34 - 5.27(m, 1H), 4.47 - 4.45(m, 1H), 4.33 - 4.23(m, 1H), 3.51 - 3.45(m, 1H), 3.16 - 2.96(m, 3H), 2.79 - 2.74(m, 2H), 2.66 - 2.62(m, 2H), 2.20 - 1.02(m, 3H), 1.38(d, *J =* 7.2 Hz, 3H); LC-MS: [M+H]⁺ = 442.05.

### Example 15: Synthesis of (2R,4S)-N-((2S)-1-((2-amino-6,7-dihydro-5H-cyclopenta[b]pyridin-5-yl) amino)-1-oxopropan-2-yl)-4-((3-methoxynaphthalen-2-yl)methyl)pyrrolidine-2-carboxamide (Compound L015) Hydrochloride

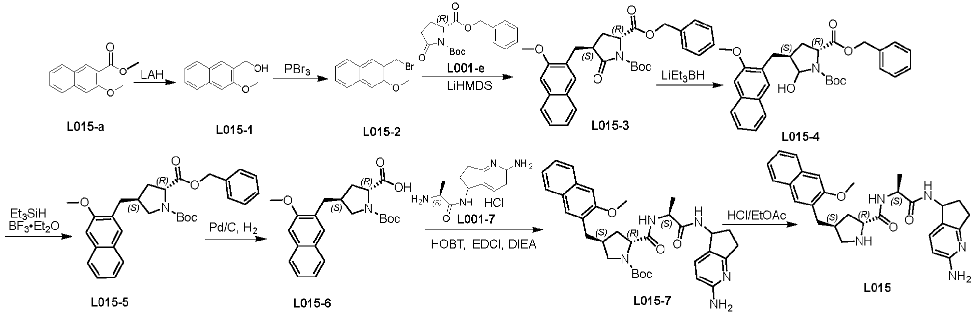

### 15.1: Synthesis of compound L015-1

L015-a (2.0 g, 9.25 mmol) in tetrahydrofuran (10 mL) was added dropwise and slowly to a suspension of lithium aluminum hydride (386 mg, 10.17 mmol) in tetrahydrofuran (10 mL) at 0 °C. After the dropwise addition was completed, the reaction system was warmed to 25 °C and stirred for 3 h. The reaction liquid was cooled to 0 °C, quenched by adding an aqueous sodium hydroxide solution (5 mL, 5 N), added with tetrahydrofuran (30 mL) for dilution, dried over anhydrous sodium sulfate and filtered, and the filtrate was concentrated under reduced pressure to give compound L015-1 (crude product), which could be used directly in the next step.

### 15.2: Synthesis of compound L015-2

L015-1 (1.7 g, crude product) was dissolved in tetrahydrofuran (30 mL) and cooled to 0 °C. A solution of phosphorus tribromide (1.2 g, 4.52 mmol) in tetrahydrofuran (10 mL) was added dropwise and slowly. After the dropwise addition was completed, the reaction system was warmed to 25 °C and stirred for 2.5 h. The reaction liquid was quenched by adding water (20 mL) and extracted with ethyl acetate (20 mL × 3). The combined organic phases were dried over anhydrous sodium sulfate and filtered, and the filtrate was concentrated under reduced pressure. The residue was purified by silica gel column chromatography to give compound L015-2 (1.3 g, yield: 57.3%).

### 15.3: Synthesis of compound L015-3

L001-e (1.3 g, 4.07 mmol) was dissolved in tetrahydrofuran (20 mL). Lithium bis(trimethylsilyl) amide (4.5 mL, 4.5 mmol, 1M in tetrahydrofuran) was added dropwise and slowly at -70 °C under nitrogen atmosphere. After the dropwise addition was completed, the reaction liquid was stirred at -70 °C for 0.5 h. A solution of L015-2 (1.2 g, 4.88 mmol) in tetrahydrofuran (10 mL) was then added dropwise and slowly, and after the dropwise addition was completed, the reaction liquid was stirred at -70 °C for another 2 h. The reaction liquid was quenched by adding a saturated aqueous ammonium chloride solution (20 mL) at this temperature, and after being warmed to room temperature, the system was extracted with ethyl acetate (20 mL × 3). The combined organic phases were dried over anhydrous sodium sulfate and filtered, and the filtrate was concentrated under reduced pressure. The residue was purified by silica gel column chromatography to give compound L015-3 (900 mg, yield: 38.5%). LC-MS: [M-Boc+H]⁺ = 390.00.

### 15.4: Synthesis of compound L015-4

L015-3 (900 mg, 1.84 mmol) was dissolved in tetrahydrofuran (20 mL). Lithium triethylborohydride (2.1 mL, 2.1 mmol, 1 M in tetrahydrofuran) was added dropwise and slowly at -70 °C under nitrogen atmosphere. After the dropwise addition was completed, the reaction liquid was stirred at -70 °C for 2 h. The reaction liquid was quenched by adding a saturated aqueous sodium bicarbonate solution (10 mL) at this temperature, and after being warmed to room temperature, the system was extracted with ethyl acetate (15 mL × 3). The combined organic phases were dried over anhydrous sodium sulfate and filtered, and the filtrate was concentrated under reduced pressure. The residue was purified by silica gel column chromatography to give compound L015-4 (400 mg, yield: 44.3%). LC-MS: [M-OH]⁺ = 474.00.

### 15.5: Synthesis of compound L015-5

L015-5 (400 mg, 0.81 mmol) was dissolved in dichloromethane (10 mL). Triethylsilane (113 mg, 0.98 mmol) and boron trifluoride diethyl ether coordination complex (139 mg, 0.98 mmol) were added at - 70 °C under nitrogen atmosphere, and the reaction liquid was stirred for 0.5 h. The reaction liquid was then supplemented with triethylsilane (113 mg, 0.98 mmol) and boron trifluoride diethyl ether coordination complex (139 mg, 0.98 mmol) and stirred at -70 °C for another 2 h. The reaction liquid was quenched by adding a saturated aqueous sodium bicarbonate solution (10 mL) at this temperature, and after being warmed to room temperature, the system was extracted with dichloromethane (15 mL × 3). The combined organic phases were dried over anhydrous sodium sulfate and filtered, and the filtrate was concentrated under reduced pressure. The residue was purified by silica gel column chromatography to give compound L015-5 (300 mg, yield: 77.5%). LC-MS: [M-tBu+H]⁺ = 420.00.

### 15.6: Synthesis of compound L015-6

L015-5 (300 mg, 0.63 mmol) was dissolved in methanol (10 mL), and wet palladium on carbon (30 mg, 10% palladium content) was added. The reaction liquid was stirred at 25 °C for 1 h under hydrogen atmosphere. The reaction liquid was filtered through celite, and the filtrate was concentrated under reduced pressure to give compound L015-6 (crude product), which could be used directly in the next step. LC-MS: [M-tBu+H]⁺ = 330.00.

### 15.7: Synthesis of compound L015-7

L015-6 (100 mg, crude product) was dissolved in N,N-dimethylformamide (2 mL), and 1-hydroxybenzotriazole (39 mg, 0.29 mmol) and 1-(3-dimethylaminopropyl)-3-ethylcarbodiimide hydrochloride (55 mg, 0.29 mmol) were added. The mixture was stirred at 20 °C for 10 min. A solution of L001-7 (80 mg, 0.31 mmol) and N,N-diisopropylethylamine (167 mg, 1.30 mmol) in N,N-dimethylformamide (2 mL) was then added. The reaction liquid was reacted for 1.5 h. The reaction liquid was poured into water (10 mL) and extracted with ethyl acetate (15 mL × 3). The combined organic phases were washed with a saturated aqueous sodium chloride solution (10 mL × 3), dried over anhydrous sodium sulfate and filtered, and the filtrate was concentrated under reduced pressure. The residue was purified by preparative high performance liquid chromatography (acidic condition) to give compound L015-7 (60 mg, yield: 39.4%). LC-MS: [M+H]⁺ = 588.05.

### 15.8: Synthesis of compound L015 hydrochloride

L015-7 (60 mg, 0.05 mmol) was dissolved in dichloromethane (2 mL), and a solution of hydrogen chloride in ethyl acetate (2 mL, 4 M) was added. The reaction liquid was stirred at 25 °C for 0.5 h. A solid was precipitated in the reaction liquid. The reaction liquid was filtered, and the filter cake was washed with ethyl acetate (5 mL). The filter cake was dissolved in deionized water (15 mL) and lyophilized to give compound L015 hydrochloride (29.2 mg, yield: 58%).

¹H NMR (400 MHz, DMSO-*d₆*): δ 10.27 - 10.16 (m, 1 H), 8.85 (d, *J =* 8.0 Hz, 1H), 8.56 (t, *J =* 8.0 Hz, 2H), 7.97 (s, 1H), 7.89 - 7.65 (m, 2H), 7.45 - 7.41 (m, 4H), 7.37 - 7.33 (m, 2H), 6.82 (dd, *J*₁ =8.8 Hz, *J₂* = 2.0 Hz, 1H), 5.18 - 5.12 (m, 1H), 4.43 - 4.35 (m, 1H), 4.34 - 4.19 (m, 1H), 3.92 (s, 3H), 3.36 - 3.28 (m, 1H), 3.13 - 2.78 (m, 5H), 2.66 - 2.63 (m, 1H), 2.48 - 2.41 (m, 1H), 2.16 - 2.08 (m, 1H), 1.97 - 1.88 (m, 2H), 1.23 (d, *J =* 8.0 Hz, 3H); LC/MS(ESI+) m/z: [M+H]⁺ =488.10.

### Example 16: Synthesis of (2R,4S)-N-((2S)-1-((2-amino-6,7-dihydro-5H-cyclopenta[b]pyridin-5-yl) amino)-1-oxopropan-2-yl)-4-(3-methylbenzyl)pyrrolidine-2-carboxamide (Compound L016) Hydrochloride

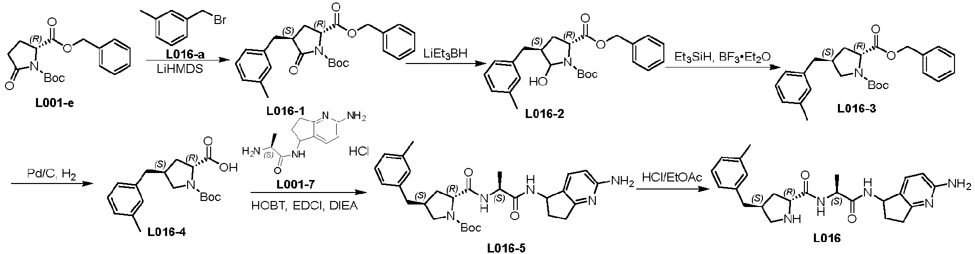

### 16.1: Synthesis of compound L016-1

L001-e (1.0 g, 3.13 mmol) was dissolved in tetrahydrofuran (10 mL). Lithium bis(trimethylsilyl) amide (3.2 mL, 3.2 mmol, 1 M in tetrahydrofuran) was added dropwise at -78 °C under nitrogen atmosphere, and after the addition was completed, the mixture was stirred at -78 °C for 0.5 h. L016-a (576 mg, 3.13 mmol) was added to the reaction system, and after the addition was completed, the reaction liquid was reacted at -78 °C for 2 h. The reaction liquid was quenched by adding water (50 mL) and extracted with ethyl acetate (40 mL × 3). The combined organic phases were dried over anhydrous sodium sulfate and filtered, and the filtrate was concentrated under reduced pressure. The residue was purified by silica gel column chromatography to give compound L016-1 (697 mg, yield: 53%). LC-MS [M-Boc+H]⁺ = 324.0.

### 16.2: Synthesis of compound L016-2

L016-1 (697 mg, 1.65 mmol) was dissolved in tetrahydrofuran (10 mL). Lithium triethylborohydride (2.5 mL, 2.5 mmol, 1 M in tetrahydrofuran) was added dropwise at -78 °C under nitrogen atmosphere, and after the dropwise addition was completed, the reaction liquid was stirred at -78 °C for 0.5 h. At this temperature, the reaction liquid was quenched by adding water (50 mL) and extracted with ethyl acetate (40 mL × 3). The combined organic phases were dried over anhydrous sodium sulfate and filtered, and the filtrate was concentrated under reduced pressure to give compound L016-2 (crude product), which was used directly in the next step.

### 16.3: Synthesis of compound L016-3

L016-2 (810 mg, crude product) was dissolved in dichloromethane (10 mL). Triethylsilane (284 mg, 2.45 mmol) and boron trifluoride diethyl ether coordination complex (347 mg, 2.45 mmol) were added at -78 °C under nitrogen atmosphere. After the addition was completed, the reaction liquid was stirred at -78 °C for 0.5 h. The reaction liquid was supplemented with triethylsilane (284 mg, 2.45 mmol) and boron trifluoride diethyl ether coordination complex (347 mg, 2.45 mmol) and stirred at -78 °C for another 2 h. At this temperature, the reaction liquid was quenched by adding water (50 mL) and extracted with ethyl acetate (40 mL × 3). The combined organic phases were dried over anhydrous sodium sulfate and filtered, and the filtrate was concentrated under reduced pressure. The residue was purified by silica gel column chromatography to give compound L016-3 (432 mg, yield: 56%). LC-MS [M-Boc+H]⁺ = 310.1.

### 16.4: Synthesis of compound L016-4

L016-3 (432 mg, 1.93 mmol) was dissolved in methanol (10 mL), and wet palladium on carbon (20 mg, 10% palladium content) was added. The reaction liquid was stirred at room temperature for 3 h under hydrogen atmosphere. The reaction liquid was filtered through celite, and the filtrate was concentrated under reduced pressure to give compound L016-4 (307 mg, crude product), which was used directly in the next step.

### 16.5: Synthesis of compound L016-5

L016-4 (100 mg, 0.31 mmol), 1-hydroxybenzotriazole (48 mg, 0.35 mmol), N,N-dimethylformamide (2 mL), N,N-diisopropylethylamine (162 mg, 1.25 mmol) and 1-(3-dimethylaminopropyl)-3-ethylcarbodiimide hydrochloride (66 mg, 0.35 mmol) were added to a reaction flask. The mixture was stirred at room temperature for 0.5 h. L001-7 (83 mg, 0.38 mmol) was then added. The reaction liquid was reacted at room temperature for 1 h. The reaction liquid was added with water (40 mL) for dilution and extracted with ethyl acetate (30 mL × 3). The combined organic phases were washed with saturated sodium chloride (40 mL), dried over anhydrous sodium sulfate and filtered, and the filtrate was concentrated under reduced pressure. The residue was purified by preparative high performance liquid chromatography (basic condition) to give compound L016-5 (25.2 mg). LC-MS [M+H]⁺= 522.1.

### 16.6: Synthesis of compound L016 hydrochloride

L016-5 (25.2 mg, 0.05 mmol) was dissolved in dichloromethane (2 mL), and a solution of hydrogen chloride in ethyl acetate (1 mL, 4 M) was added. The mixture was stirred at room temperature for 0.5 h, and a solid was precipitated. The reaction system was filtered, and the filter cake was washed with ethyl acetate (5 mL). The filter cake was dissolved in water (15 mL) and lyophilized to give compound L016 hydrochloride (17.1 mg, yield: 71.8%).

¹H NMR (400 MHz, CH₃OH-*d₄*): δ 7.84 - 7.76 (m, 1H), 7.19 (t, *J =* 7.2 Hz, 1H), 7.05 - 6.98 (m, 3H), 6.83 (d, *J =* 8.8 Hz, 1H), 5.30 (m, 1H), 4.48 - 4.23 (m, 2H), 3.49 - 3.40 (m, 1H), 3.13 - 3.11 (m, 1H), 3.06 - 2.96 (m, 2H), 2.78 - 2.73 (m, 2H), 2.68 - 2.47 (m, 2H), 2.32 (s, 3H), 2.21 - 1.97 (m, 3H), 1.39 - 1.36(m, 3H); LC-MS [M+H]⁺= 422.2.

### Example 17: Synthesis of (2R,4R)-N-((2S)-1-((2-amino-6,7-dihydro-5H-cyclopenta[b]pyridin-5-yl) amino)-1-oxopropan-2-yl)-4-(1-phenylcyclopropyl)pyrrolidine-2-carboxamide (Compound L017)

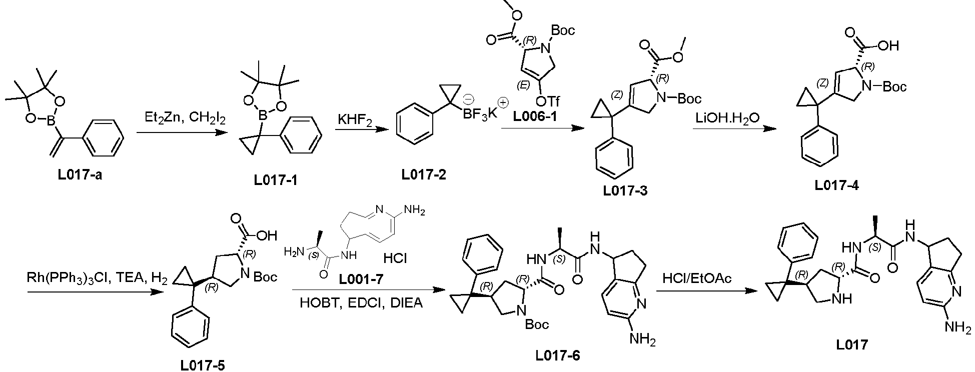

### 17.1: Synthesis of compound L017-1

Diiodomethane (5.9 g, 21.72 mmol) was added to dichloromethane (40 mL) at room temperature. The mixture was cooled to 0 °C and stirred for 5 min under nitrogen atmosphere. A solution of diethyl zinc in toluene (10.8 mL, 10.8 mmol, 1 M in toluene) was added slowly, and the reaction liquid was stirred at 0 °C for 0.5 h. A solution of L017-a (1 g, 4.35 mmol) in dichloromethane (10 mL) was then added dropwise and slowly. The reaction liquid was stirred at 0 °C for another 0.5 h, warmed to room temperature and stirred for 16 h. The reaction liquid was quenched by adding a saturated aqueous ammonium chloride solution (50 mL), and the mixture was stirred for 3-5 min. The mixture was extracted with dichloromethane (50 mL × 3). The combined organic phases were washed with saturated brine (50 mL), dried over anhydrous sodium sulfate and filtered, and the filtrate was concentrated under reduced pressure. The residue was purified by silica gel column chromatography to give compound L017-1 (979 mg, yield: 92%). LC-MS: [M+H]⁺ = 245.15.

### 17.2: Synthesis of compound L017-2

L017-1 (900 mg, 3.69 mmol), potassium hydrogen fluoride (1.73 g, 22.1 mmol) and methanol (30 mL) were added to a 100 mL sealed tube at room temperature, and the reaction liquid was stirred at 80 °C for 5 h. The reaction liquid was cooled to room temperature and concentrated under reduced pressure to about 10 mL. A solid was precipitated, and the reaction liquid was filtered. The filter cake was placed in a round-bottom flask. A diethyl ether/n-heptane solution (40 mL, 1/1 (v/v)) was added, and the suspension was stirred at room temperature for 0.5 h. The suspension was filtered and the filter cake was washed with diethyl ether (30 mL). The washed filter cake was then added to acetonitrile (40 mL). The mixture was stirred at 60 °C for 5 min and filtered while hot. The filter cake was dried under reduced pressure to give compound L017-2 (471 mg, yield: 57%).

### 17.3: Synthesis of compound L017-3

L017-2 (471 mg, 2.10 mmol), L006-1 (790 mg, 2.10 mmol), mesylate[n-butyl di(1-adamantyl) phosphine](2-amino-1,1'-biphenyl-2-yl)palladium(II) (70 mg, 0.11 mmol) and cesium carbonate (1.37 g, 4.20 mmol) were dissolved in toluene (20 mL) and water (2 mL). The reaction liquid was purged with nitrogen for 15 min and reacted at 95 °C for 2 h. The reaction liquid was cooled to room temperature, concentrated under reduced pressure to remove toluene, added with water (40 mL) for dilution, and extracted with ethyl acetate (30 mL × 3). The combined organic phases were washed with saturated brine (50 mL), dried over anhydrous sodium sulfate and filtered, and the filtrate was concentrated under reduced pressure. The residue was purified by silica gel column chromatography to give compound L017-3 (270 mg, yield: 37%). LC-MS: [M-tBu+H]⁺ = 288.05.

### 17.4: Synthesis of compound L017-4

L017-3 (270 mg, 0.79 mmol) and lithium hydroxide monohydrate (485.6 mg, 11.8 mmol) were dissolved in tetrahydrofuran (4 mL), methanol (2 mL) and water (2 mL). The reaction liquid was stirred at room temperature for 2 h. The reaction liquid was adjusted to pH = 5-6 with diluted hydrochloric acid (1 M), added with water (20 mL) for dilution, and extracted with ethyl acetate (20 mL × 3). The combined organic phases were dried over anhydrous sodium sulfate and filtered, and the filtrate was concentrated under reduced pressure to give compound L017-4 (157 mg). LC-MS: [M-tBu+H]⁺ = 274.05.

### 17.5: Synthesis of compound L017-5

L017-4 (157 mg, 0.48 mmol), tris(triphenylphosphine)rhodium chloride (44.6 mg, 0.05 mmol) and triethylamine (48.6 mg, 0.48 mmol) were dissolved in methanol (15 mL) and tetrahydrofuran (15 mL) at room temperature. The reaction liquid was stirred at room temperature for 4 days under hydrogen atmosphere. The reaction liquid was filtered, and the filtrate was adjusted to pH = 10 with an aqueous sodium hydroxide solution (1 M), and then extracted with ethyl acetate (40 mL). The organic phase was washed with a saturated aqueous sodium bicarbonate solution (20 mL). The organic phase was discarded. All aqueous phases were combined, adjusted to pH = 5-6 with diluted hydrochloric acid (1 M), and then extracted with ethyl acetate (20 mL × 3). The combined organic phases were washed with saturated brine (50 mL), dried over anhydrous sodium sulfate and filtered, and the filtrate was concentrated under reduced pressure to give compound L017-5 (140 mg, crude product), which was used directly in the next step. LC-MS: [M-tBu+H]⁺ = 276.00.

### 17.6: Synthesis of compound L017-6

L017-5 (140 mg, crude product) and 1-hydroxybenzotriazole (59 mg, 0.42 mmol) were dissolved in N,N-dimethylformamide (2 mL) at room temperature. N,N-diisopropylethylamine (217 mg, 1.68 mmol) and 1-(3-dimethylaminopropyl)-3-ethylcarbodiimide hydrochloride (81 mg, 0.42 mmol) were added. The mixture was stirred at room temperature for 0.5 h. L001-7 (111 mg, 0.5 mmol) was then added. The reaction liquid was reacted at room temperature for 1 h. The reaction liquid was added with water (50 mL) for dilution and extracted with ethyl acetate (30 mL × 3). The combined organic phases were washed with saturated sodium chloride (40 mL), dried over anhydrous sodium sulfate and filtered, and the filtrate was concentrated under reduced pressure. The residue was purified by preparative high performance liquid chromatography (acidic condition) to give compound L017-6 (120 mg, yield: 53.6%). LC-MS: [M+H]⁺ = 534.00.

### 17.7: Synthesis of compound L017

L017-6 (120 mg, 0.225 mmol) was dissolved in dichloromethane (2 mL), and a solution of hydrogen chloride in ethyl acetate (1 mL, 4 M) was added. The reaction liquid was stirred at room temperature for 1 h. A solid was precipitated. The reaction liquid was filtered, and the filter cake was washed with ethyl acetate (10 mL). The crude product obtained by filtration was purified by preparative high performance liquid chromatography (basic condition) to give compound L017 (19.1 mg).

¹H NMR (400 MHz, DMSO-*d₆*): δ 8.91- 8.90 (m, 1H), 8.58 - 8.51 (m, 1H), 7.97 (s, 2H), 7.68 - 7.62 (m, 1H), 7.35 - 7.23 (m, 5H), 6.80 (d, *J=* 8.8 Hz, 1H), 5.30 (s, 0.5H), 5.16 - 5.09 (m, 1H), 4.97 (s, 0.5H), 4.38 - 4.36 (m, 1H), 4.23 - 4.17 (m, 1H), 3.83 - 3.81 (m, 1H), 3.05 - 2.98 (s, 1H), 2.93 - 2.85 (m, 1H), 2.45 - 2.42 (m, 1H), 2.35 (s, 1H), 2.01 - 1.74 (m, 2H), 1.33 (t, *J =* 7.2 Hz, 2H), 1.22 - 1.07 (m, 6H), 0.80 - 0.72 (m, 1H); LC-MS: [M+H]⁺ = 434.10.

### Example 18: Synthesis of (2R,4S)-N-((2S)-1-((2-amino-6,7-dihydro-5H-cyclopenta[b]pyridin-5-yl) amino)-1-oxopropan-2-yl)-4-(3-chloro-4-(trifluoromethoxy)benzyl)pyrrolidine-2-carboxamide (Compound L018)

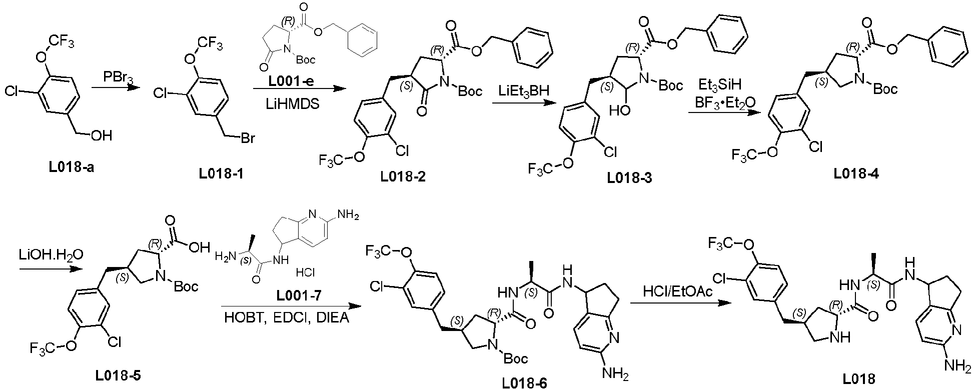

### 18.1: Synthesis of compound L018-1

L018-a (2.0 g, 8.52 mmol) was dissolved in dichloromethane (30 mL), and a solution of phosphorus tribromide (1.2 g, 4.52 mmol) in dichloromethane (10 mL) was added dropwise and slowly at 0 °C. After the dropwise addition was completed, the reaction liquid was stirred at 0 °C for 1 h. The reaction liquid was quenched by adding water (20 mL) and extracted with ethyl acetate (20 mL × 3). The combined organic phases were dried over anhydrous sodium sulfate and filtered, and the filtrate was concentrated under reduced pressure. The residue was purified by silica gel column chromatography to give compound L018-1 (1.5 g, yield: 59.1%).

### 18.2: Synthesis of compound L018-2

L001-e (1.5 g, 4.70 mmol) was dissolved in tetrahydrofuran (20 mL), and the mixture was cooled to - 70 °C. Lithium bis(trimethylsilyl)amide (5.2 mL, 5.2 mmol, 1 M in tetrahydrofuran) was added dropwise and slowly under nitrogen atmosphere, and after the dropwise addition was completed, the reaction liquid was stirred at -70 °C for 0.5 h. A solution of L018-1 (1.5 g, 5.17 mmol) in tetrahydrofuran (10 mL) was then added dropwise, and after the dropwise addition was completed, the reaction system was stirred at -70 °C for another 2 h. The reaction liquid was quenched with a saturated aqueous ammonium chloride solution (20 mL) at this temperature, and after being warmed to room temperature, the system was extracted with ethyl acetate (20 mL × 3). The combined organic phases were dried over anhydrous sodium sulfate and filtered, and the filtrate was concentrated under reduced pressure. The residue was purified by silica gel column chromatography to give compound L018-2 (1.2 g, yield: 48%). LC-MS: [M-Boc+H]⁺ = 427.90.

### 18.3: Synthesis of compound L018-3

L018-2 (1.2 g, 2.27 mmol) was dissolved in tetrahydrofuran (20 mL) and cooled to -70 °C. Lithium triethylborohydride (2.5 mL, 2.5 mmol, 1 M in tetrahydrofuran) was added dropwise and slowly under nitrogen atmosphere. After the dropwise addition was completed, the reaction liquid was stirred at -70 °C for 2 h. The reaction liquid was quenched by adding a saturated aqueous sodium bicarbonate solution (10 mL) at this temperature, and after being warmed to room temperature, the system was extracted with ethyl acetate (15 mL × 3). The combined organic phases were dried over anhydrous sodium sulfate and filtered, and the filtrate was concentrated under reduced pressure. The residue was purified by silica gel column chromatography to give compound L018-3 (900 mg, yield: 49.5%). LC-MS: [M-OH]⁺ = 512.00.

### 18.4: Synthesis of compound L018-4

L018-3 (900 mg, 1.71 mmol) was dissolved in dichloromethane (20 mL) and cooled to -70 °C. Triethylsilane (237 mg, 2.04 mmol) and boron trifluoride diethyl ether coordination complex (290 mg, 2.04 mmol) were added under nitrogen atmosphere, and the reaction liquid was stirred at -70 °C for 0.5 h. The reaction liquid was supplemented with triethylsilane (237 mg, 2.04 mmol) and boron trifluoride diethyl ether coordination complex (290 mg, 2.04 mmol) and stirred at -70 °C for another 2 h. The reaction liquid was quenched by adding a saturated aqueous sodium bicarbonate solution (10 mL) at this temperature, and after being warmed to room temperature, the system was extracted with dichloromethane (15 mL × 3). The combined organic phases were dried over anhydrous sodium sulfate and filtered, and the filtrate was concentrated under reduced pressure. The residue was purified by silica gel column chromatography to give compound L018-4 (450 mg, yield: 38.8%). LC-MS: [M-tBu+H]⁺ = 457.90.

### 18.5: Synthesis of compound L018-5

L018-4 (450 mg, 0.88 mmol) was dissolved in methanol (10 mL) and water (10 mL), and then lithium hydroxide monohydrate (110 mg, 2.63 mmol) was added. The reaction liquid was stirred at 25 °C for 4 h. The reaction liquid was directly concentrated under reduced pressure, and the residue was added with water (10 mL) for dilution and washed with ethyl acetate (10 mL × 3). The organic phase was discarded. The aqueous phase was adjusted to pH = 4-5 with diluted hydrochloric acid (1 M) and extracted with dichloromethane (10 mL × 3). The combined organic phases were dried over anhydrous sodium sulfate and filtered, and the filtrate was concentrated under reduced pressure to give compound L018-5 (350 mg, crude product), which could be used directly in the next step. LC-MS: [M-tBu+H]⁺ = 367.95.

### 18.6: Synthesis of compound L018-6

L018-5 (150 mg, 0.35 mmol) was dissolved in N,N-dimethylformamide (2 mL). 1-Hydroxybenzotriazole (57 mg, 0.42 mmol) and 1-(3-dimethylaminopropyl)-3-ethylcarbodiimide hydrochloride (82 mg, 0.42 mmol) were added. The reaction liquid was stirred at room temperature for 10 min. A solution of L001-7 (91 mg, 0.35 mmol) and N,N-diisopropylethylamine (129 mg, 1.77 mmol) in N,N-dimethylformamide (2 mL) was added. The reaction liquid was stirred at room temperature for another 1.5 h. The reaction liquid was directly poured into water (10 mL) and extracted with ethyl acetate (15 mL × 3). The combined organic phases were washed with a saturated aqueous ammonium chloride solution (10 mL × 3), dried over anhydrous sodium sulfate and filtered, and the filtrate was concentrated under reduced pressure to give compound L018-6 (200 mg, crude product). LC-MS: [M+H]⁺ = 626.05.

### 18.7: Synthesis of compound L018

L018-6 (200 mg, 0.32 mmol) was dissolved in dichloromethane (2 mL), and a solution of hydrogen chloride in ethyl acetate (2 mL, 4 M) was added at room temperature. The reaction liquid was stirred for 0.5 h, and a solid was precipitated. The reaction liquid was concentrated under reduced pressure, and the residue was purified by preparative high performance liquid chromatography (basic condition) and lyophilized to give compound L018 (50.3 mg, yield: 29.94%).

¹H NMR (400 MHz, DMSO-*d₆*): δ 8.28 - 8.13(m, 2 H), 7.54 - 7.52(m, 1 H), 7.50 -7.44(m, 1 H), 7.31 - 7.27(m, 1 H), 7.14 -7.09(m, 1 H), 6.24 - 6.22(m, 1 H), 5.83(s, 2 H), 5.14 - 5.02(m, 1 H), 4.26 - 4.15(m, 1 H), 3.62(dd, *J*₁ = 8.0, *J*₂ = 4.0 Hz, 1 H), 2.89 - 2.51(m, 7 H), 2.39 - 2.26(m, 1 H), 2.20 - 2.11(m, 1 H), 1.78 - 1.62(m, 3 H), 1.24 -1.18(m, 3 H); LC-MS: [M+H]⁺= 526.00.

### Example 19: Synthesis of (2R)-N-((2S)-1-((2-amino-6,7-dihydro-5H-cyclopenta[b]pyridin-5-yl) amino)-1-oxopropan-2-yl)-4-phenyl-2,5-dihydro-1H-pyrrole-2-carboxamide (Compound L019) Hydrochloride

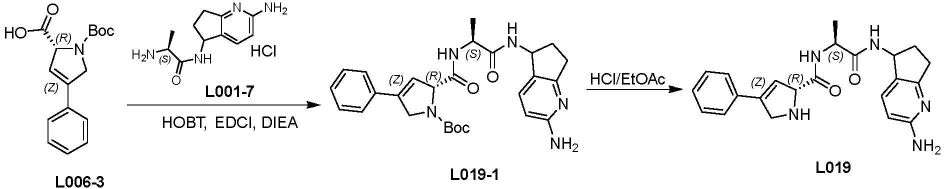

### 19.1: Synthesis of compound L019-1

L006-3 (100 mg, 0.35 mmol), 1-hydroxybenzotriazole (53 mg, 0.38 mmol), N,N-dimethylformamide (3 mL), N,N-diisopropylethylamine (178 mg, 1.38 mmol) and 1-(3-dimethylaminopropyl)-3-ethylcarbodiimide hydrochloride (73 mg, 0.38 mmol) were added to a reaction flask. The reaction liquid was stirred at room temperature for 0.5 h. L001-7 (92 mg, 0.42 mmol) was further added. The reaction liquid was reacted at room temperature for another 1 h. The reaction liquid was added with water (40 mL) for dilution, and the mixture was extracted with ethyl acetate (30 mL × 3). The combined organic phases were washed with a saturated aqueous sodium chloride solution (40 mL), dried over anhydrous sodium sulfate and filtered, and the filtrate was concentrated under reduced pressure. The residue was purified by preparative high performance liquid chromatography to give compound L019-1 (68 mg, yield: 40.1%). LC-MS: [M+H]⁺ = 492.10.

### 19.2: Synthesis of compound L019 hydrochloride

L019-1 (68 mg, 0.14 mmol) was dissolved in dichloromethane (2 mL), and a solution of hydrogen chloride in ethyl acetate (1 mL, 4 M) was added. The reaction liquid was stirred at room temperature for 0.5 h. A solid was precipitated in the reaction liquid. The reaction liquid was filtered, and the filter cake was washed with ethyl acetate (10 mL). The filter cake was dissolved in deionized water (10 mL) and lyophilized to give compound L019 hydrochloride (24.0 mg, yield: 37.4%).

¹H NMR (400 MHz, DMSO-*d₆*): δ 7.85 - 7.79(m, 1H), 7.57 - 7.46(m, 2H), 7.46 - 7.37(m, 3H), 6.95 - 6.74(m, 1H), 6.50 - 6.28(m, 1H), 5.33(m, 2H), 4.57 - 4.52(m, 2H), 4.39 - 4.24(m, 1H), 3.22 - 3.07(m, 1H), 3.06 - 2.95(m, 1H), 2.77 - 2.56(m, 1H), 2.25 - 1.98(m, 1H), 1.46 - 1.44(m, 3H); LC-MS: [M+H]⁺ = 392.10.

### Example 20: Synthesis of N-((2S)-1-((2-amino-6,7-dihydro-SH-cyclopenta[b]pyridin-5-yl)amino)-1-oxopropan-2-yl)-4-phenyl-1H-pyrrole-2-carboxamide (Compound L020) and N-((S)-1-(((R)-2-amino-6,7-dihydro-5H-cyclopenta[b]pyridin-5-yl)amino)-1-oxopropan-2-yl)-4-phenyl-1H-pyrrole-2-carboxamide (Compound L020-IS-01 or L020-IS-02), and N-((S)-1-(((S)-2-amino-6,7-dihydro-5H-cyclopenta[b]pyridin-5-yl)amino)-1-oxopropan-2-yl)-4-phenyl-1H-pyrrole-2-carboxamide (Compound L020-IS-02 or L020-IS-01)

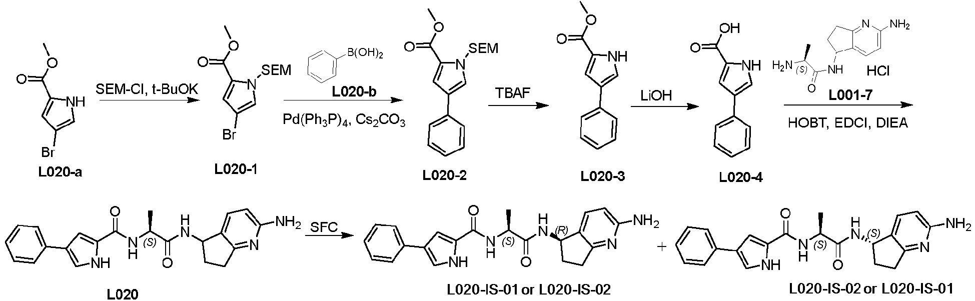

### 20.1: Synthesis of compound L020-1

L020-a (2 g, 9.85 mmol) was dissolved in N,N-dimethylformamide (20 mL), and potassium tert-butoxide (1.3 g, 11.76 mmol) was added. The mixture was stirred at 0 °C for 0.5 h. 2-(Trimethylsilyl)ethoxymethyl chloride (1.8 g, 10.77 mmol) was then added, and the reaction liquid was reacted at 0 °C for 2 h. A small amount of insoluble substance was precipitated in the reaction liquid. The reaction liquid was filtered, and the filtrate was added with water (50 mL) for dilution and extracted with ethyl acetate (50 mL × 3). The combined organic phases were washed with saturated brine (50 mL), dried over anhydrous sodium sulfate and filtered, and the filtrate was concentrated under reduced pressure. The residue was purified by silica gel column chromatography to give compound L020-1 (2.4 g, yield: 73%). LC-MS: [M+H]⁺ = 334.28.

### 20.2: Synthesis of compound L020-2

L020-1 (1.7 g, 5.01 mmol), tetrakis(triphenylphosphine)palladium(0) (576 mg, 0.50 mmol), phenylboronic acid (917 mg, 7.52 mmol) and cesium carbonate (3.3 g, 10.02 mmol) were added to a reaction flask. The reaction liquid was purged with nitrogen, dissolved by adding 1,4-dioxane (24 mL) and water (6 mL), and refluxed at 90 °C for 5 h. The reaction liquid was cooled to room temperature, added with (50 mL) for dilution, and extracted with ethyl acetate (50 mL × 3). The combined organic phases were washed with saturated brine (50 mL), dried over anhydrous sodium sulfate and filtered, and the filtrate was concentrated under reduced pressure. The residue was purified by silica gel column chromatography to give compound L020-2 (1.45 g, yield: 87%). LC-MS: [M+H]⁺ = 332.05.

### 20.3: Synthesis of compound L020-3

L020-2 (1.45 g, 4.38 mmol) and tetrabutylammonium fluoride (2.29 g, 8.76 mmol) were added to a reaction flask, dissolved by adding tetrahydrofuran (10 mL), and the reaction liquid stirred at 80 °C for 6 h. The reaction liquid was cooled to room temperature, added with water (50 mL) for dilution, and extracted with ethyl acetate (50 mL × 3). The combined organic phases were washed with saturated brine (50 mL), dried over anhydrous sodium sulfate and filtered, and the filtrate was concentrated under reduced pressure. The residue was purified by silica gel column chromatography to give compound L020-3 (570 mg, yield: 65%). LC-MS: [M+H]⁺ = 202.10

### 20.4: Synthesis of compound L020-4

L020-3 (570 mg, 2.83 mmol) was added to a reaction flask and dissolved by adding tetrahydrofuran (2 mL), methanol (2 mL) and water (1 mL). Lithium hydroxide monohydrate (357 mg, 8.49 mmol) was added at room temperature, and the reaction liquid was stirred at 80 °C for 3 h. The reaction liquid was cooled to room temperature and concentrated under reduced pressure to give a crude product. The crude product was added with water (30 mL) for dilution. The solution was adjusted to pH = 5-6 with an aqueous hydrochloric acid solution (1 M) and extracted with ethyl acetate (50 mL × 3). The combined organic phases were washed with saturated brine (50 mL), dried over anhydrous sodium sulfate and filtered, and the filtrate was concentrated under reduced pressure to give compound L020-4 (511 mg, yield: 96.3%). LC-MS: [M+H]⁺ = 188.10.

### 20.5: Synthesis of compound L020

L020-4 (100 mg, 0.53 mmol), 1-hydroxybenzotriazole (81 mg, 0.58 mmol), N,N-dimethylformamide (2 mL), N,N-diisopropylethylamine (290 mg, 2.12 mmol) and 1-(3-dimethylaminopropyl)-3-ethylcarbodiimide hydrochloride (111 mg, 0.58 mmol) were added to a reaction flask. The reaction liquid was reacted at room temperature for 0.5 h. Compound L001-7 (141 mg, 0.64 mmol) was further added. The reaction liquid was reacted at room temperature for another 1 h. The reaction liquid was added with water (40 mL) for dilution, and the mixture was extracted with ethyl acetate (30 mL × 3).

The combined organic phases were washed with saturated sodium chloride (40 mL), dried over anhydrous sodium sulfate and filtered, and the filtrate was concentrated under reduced pressure. The residue was purified by preparative high performance liquid chromatography to give compound L020 (50.8 mg, yield: 24.4%).

¹H NMR (400 MHz, DMSO-*d₆*): δ 11.67(s, 1H), 8.32-8.26(m, 1H), 8.17 - 8.02(m, 1H), 7.55(d, *J=* 8.0 Hz, 2H), 7.39 - 7.27(m, 4H), 7.25(s, 1H), 7.18 - 7.07(m, 2H), 6.83(s, 1H), 6.51(d, *J=* 8.8 Hz, 1H), 5.15(d, *J =* 6.8 Hz, 1H), 4.61 - 4.24(m, 1H), 2.99 - 2.82(m, 1H), 2.81 - 2.65(m, 1H), 2.48 - 2.29(m, 1H), 1.96 -1.72(m, 1H), 1.34(d, *J=* 7.2 Hz, 3H); LC-MS: [M+H]⁺ = 390.05.

### 20.6: Preparation of compound L020-IS-01 or L020-IS-02

Compound L020 (50.8 mg) was separated by supercritical fluid chromatography (SFC) (method: Cellulose-2-3-MeOH + CAN (DEA)-40-3 mL-35T, column: Lux Cellulose-2, 50 × 25 mm, 10 µm, mobile phase, A: carbon dioxide, B: methanol, flow rate: 75 g/min) to give compounds L020-IS-01 (retention time Rt = 0.952 min, 14.9 mg) and L020-IS-02 (retention time Rt = 1.418 min, 8.5 mg).
L020-IS-01:
   ¹H NMR (400 MHz, DMSO-*d₆*): δ 11.63(s, 1H), 8.14(d, *J =* 8.2 Hz, 1H), 8.00(d, *J =* 7.6 Hz, 1H), 7.54(d, *J* = 7.2 Hz, 2H), 7.37 - 7.24(m, 4H), 7.18(d, *J* = 8.0 Hz, 1H), 7.12(t, *J* = 7.2 Hz, 1H), 6.21(d, *J* = 8.4 Hz, 1H), 5.79(s, 2H), 5.11(q, *J =* 7.6 Hz, 1H), 4.44 - 4.36(m, 1H), 2.73 - 2.68(m, 1H), 2.65 - 2.57(m, 1H), 2.35 - 2.27(m, 1H), 1.76 -1.67(m, 1H), 1.31(d, *J =* 7.2 Hz, 3H); LC-MS: [M+H]⁺ = 390.05.
L020-IS-02:
   ¹H NMR (400 MHz, DMSO-*d₆*): δ 11.64(s, 1H), 8.13(d, *J =* 8.4 Hz, 1H), 8.02(d, *J =* 7.6 Hz, 1H), 7.55(d, *J =* 7.4 Hz, 2H), 7.35 - 7.29(m, 4H), 7.16 - 7.13(m, 2H), 6.24(d, *J =* 8.4 Hz, 1H), 5.81(s, 2H), 5.13(q, *J =* 7.6 Hz, 1H), 4.47- 4.40(m, 1H), 2.77 - 2.69(m, 1H), 2.67 - 2.59(m, 1H), 2.38 - 2.30(m, 1H), 1.81 - 1.72(m, 1H), 1.33(d, *J =* 7.2 Hz, 3H); LC-MS: [M+H]⁺= 390.05.

### Example 21: Synthesis of N-((2S)-1-((2-amino-6,7-dihydro-SH-cyclopenta[b]pyridin-5-yl)amino)-1-oxopropan-2-yl)-4-phenylpiperidine-2-carboxamide (Compound L021) Hydrochloride

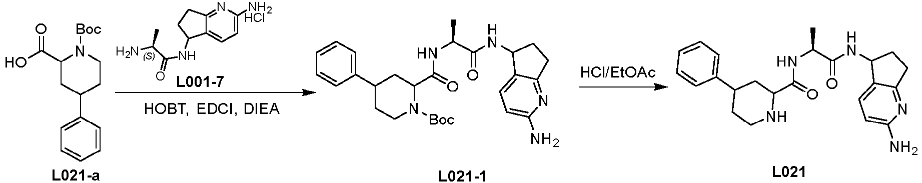

### 21.1: Synthesis of compound L021-1

L021-a (100 mg, 0.33 mmol) was dissolved in N,N-dimethylformamide (2 mL), and 1-(3-dimethylaminopropyl)-3-ethylcarbodiimide hydrochloride (69 mg, 0.36 mmol) and 1-hydroxybenzotriazole (49 mg, 0.36 mmol) were added. After the mixture was stirred at 20 °C for 10 min, N,N-diisopropylethylamine (211 mg, 1.64 mmol) and a solution of L001-7 (106 mg, 0.36 mmol) in N,N-dimethylformamide (2 mL) were added, and the reaction liquid was reacted at 20 °C for 30 min. The reaction liquid was poured into water (10 mL), and the mixture was extracted with ethyl acetate (15 mL × 3). The combined organic phases were washed with a saturated aqueous ammonium chloride solution (10 mL × 3), dried over anhydrous sodium sulfate and filtered, and the filtrate was concentrated under reduced pressure. The residue was purified by preparative high performance liquid chromatography (acidic condition) and lyophilized to give compound L021-1 (60 mg, yield: 36.09%). LC-MS: [M+H]⁺ = 508.15.

### 21.2: Synthesis of compound L021 hydrochloride

L021-1 (60 mg, 0.12 mmol) was dissolved in dichloromethane (2 mL), and a solution of hydrogen chloride in ethyl acetate (2 mL, 4 M) was added. The reaction liquid was reacted at 25 °C for 1 h, and a solid was precipitated. The reaction liquid was filtered, and the filter cake was dissolved in deionized water (10 mL) and lyophilized to give compound L021 hydrochloride (38.7 mg, yield: 68.3%).

¹H NMR (400 MHz, DMSO-*d₆*): δ 9.93 - 9.38 (m, 1H), 9.02 - 8.69 (m, 2H), 8.59 - 8.48 (m, 1H), 8.04 (s, 2H), 7.74 - 7.61 (m, 1H), 7.55 - 7.09 (m, 6H), 6.87 - 6.81 (m, 1H), 5.24 - 4.97 (m, 1H), 4.36 - 4.17 (m, 1H), 3.95 (t, *J =* 12.0 Hz, 1H), 3.34 (d, *J =* 12.0 Hz, 1H), 3.21 - 2.78 (m, 4H), 2.49 - 2.21 (m, 2H), 2.01 - 1.98 (m, 3H), 1.80 - 1.55 (m, 1H), 1.27 - 1.23 (m, 3H); LC-MS: [M+H]⁺= 408.10.

### Example 22: Synthesis of (2R)-N-((2S)-1-((2-amino-6,7-dihydro-5H-cyclopenta[b]pyridin-5-yl)amino)-1-oxopropan-2-yl)-4-(naphthalen-1-yl)piperidine-2-carboxamide (Compound L022) Hydrochloride

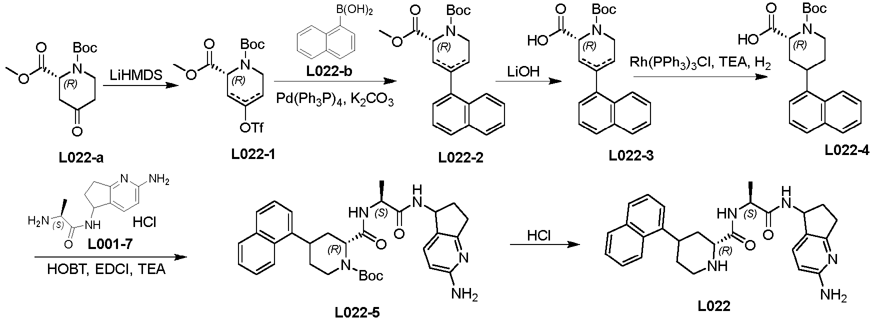

### 22.1: Synthesis of compound L022-1

L022-a (1.0 g, 3.85 mmol) was added to tetrahydrofuran (10 mL) at room temperature, and the reaction liquid was cooled to -78 °C. Lithium bis(trimethylsilyl)amide (4.2 mL, 4.2 mmol, 1 M in tetrahydrofuran) was added dropwise under nitrogen atmosphere, and after the dropwise addition was completed, the reaction liquid was stirred at -78 °C for 10 min. A solution of N-(5-chloro-2-pyridinyl)bis(trifluoromethanesulfonamide) (1.58 g, 4.04 mmol) in tetrahydrofuran (5 mL) was then added at this temperature, and after the addition was completed, the reaction liquid was warmed to room temperature and stirred for another 1.5 h. The reaction liquid was quenched by adding a saturated aqueous ammonium chloride solution (20 mL) and extracted with ethyl acetate (20 mL × 3). The combined organic phases were dried over anhydrous sodium sulfate and filtered, and the filtrate was concentrated under reduced pressure. The residue was purified by silica gel column chromatography to give compound L022-1 (600 mg, crude product, the product was a double bond isomeric mixture).

### 22.2: Synthesis of compound L022-2

L022-1 (440 mg, 1.13 mmol), L022-b (233 mg, 1.36 mmol), tetrakis(triphenylphosphine)palladium(0) (130 mg, 0.11 mmol) and potassium carbonate (311 mg, 2.26 mmol) were added to 1,4-dioxane (10 mL) and water (1 mL). The reaction liquid was stirred at 75 °C for 16 h under nitrogen atmosphere. The reaction liquid was cooled to room temperature, added with water (20 mL) for dilution, and then extracted with ethyl acetate (20 mL × 3). The combined organic phases were dried over anhydrous sodium sulfate and filtered, and the filtrate was concentrated under reduced pressure. The residue was purified by silica gel column chromatography to give L022-2 (430 mg, crude product) as a double bond isomeric mixture. LC-MS: [M-Boc+H]⁺ = 268.05.

### 22.3: Synthesis of compound L022-3

L022-2 (430 mg, 1.17 mmol) was dissolved in tetrahydrofuran (5 mL), methanol (1 mL) and water (1 mL), and lithium hydroxide monohydrate (147 mg, 3.50 mmol) was added. The reaction liquid was stirred at room temperature for 3 h. The reaction liquid was added with water (10 mL) for dilution and extracted with ethyl acetate (20 mL × 3). The organic phase was discarded. The aqueous phase was adjusted to pH = 5-6 with diluted hydrochloric acid (1 M), and then extracted with ethyl acetate (20 mL × 3). The combined organic phases were dried over anhydrous sodium sulfate and filtered, and the filtrate was concentrated under reduced pressure to give L022-3 (260 mg, crude product) as a double bond isomeric mixture. LC-MS: [M-Boc+H]⁺ = 254.10.

### 22.4: Synthesis of compound L022-4

L022-3 (200 mg, 0.56 mmol), tris(triphenylphosphine)rhodium chloride (92 mg, 0.10 mmol) and triethylamine (57 mg, 0.56 mmol) were dissolved in tetrahydrofuran (2.5 mL) and methanol (2.5 mL). The reaction liquid was stirred at room temperature for 3 days under hydrogen atmosphere. The reaction liquid was filtered, and the filtrate was directly concentrated under reduced pressure to give compound L022-4 (200 mg, crude product). LC-MS: [M-Boc+H]⁺ = 256.10.

### 22.5: Synthesis of compound L022-5

L022-4 (120 mg, 0.34 mmol), L001-7 (100 mg, 0.34 mmol), 1-(3-dimethylaminopropyl)-3-ethylcarbodiimide hydrochloride (88 mg, 0.46 mmol), 1-hydroxybenzotriazole (69 mg, 0.46 mmol) and triethylamine (93 mg, 0.92 mmol) were added to dichloromethane (2 mL). The reaction liquid was stirred at room temperature for 0.5 h. The reaction liquid was added with water (20 mL) for dilution and extracted with dichloromethane (10 mL × 3). The combined organic phases were dried over anhydrous sodium sulfate and filtered, and the filtrate was concentrated under reduced pressure. The residue was purified by preparative high performance liquid chromatography to give compound L022-5 (150 mg, yield: 79%), LC-MS: [M+H]⁺= 558.10.

### 22.6: Synthesis of compound L022 hydrochloride

L022-5 (50 mg, 0.09 mmol) was dissolved in dichloromethane (1 mL), and a solution of hydrogen chloride in 1,4-dioxane (1 mL, 4 M) was added dropwise. The reaction liquid was stirred at room temperature for 1 h. A solid was precipitated. The reaction liquid was filtered, and the filter cake was washed with dichloromethane (3 mL). The filter cake was dissolved in deionized water (15 mL) and lyophilized to give compound L022 hydrochloride (11.1 mg, yield: 25.1%).

¹H NMR (400 MHz, DMSO-*d₆*): δ 9.91 (s, 1H), 9.05 - 9.04 (m, 1H), 8.94 - 8.70 (m, 1H), 8.55 (d, *J =* 8.0 Hz, 1H), 8.34 - 7.98 (m, 1H), 7.92 - 7.80 (m, 1H), 7.85 - 7.78 (m, 1H), 7.67 - 7.45 (m, 5H), 7.38 (d, *J =* 7.2 Hz, 1H), 6.78 - 6.57 (m, 1H), 5.26 - 5.09 (m, 1H), 4.48 - 4.24 (m, 2H), 3.58 (m, 2H), 2.97 - 2.87 (m, 2H), 2.44 - 2.34 (m, 1H), 2.13 (s, 2H), 2.06 - 1.88 (m, 2H), 1.42 - 1.21 (m, 4H); LC-MS: [M+H]⁺= 458.10.

### Example 23: Synthesis of (2R)-N-((2S)-1-((2-amino-6,7-dihydro-5H-cyclopenta[b]pyridin-5-yl) amino)-1-oxopropan-2-yl)-4-phenyl-1,2,5,6-tetrahydropyridine-2-carboxamide (L023-IS-01 or L023-IS-02) and (2R)-N-((2S)-1-((2-amino-6,7-dihydro-5H-cyclopenta[b]pyridin-5-yl)amino)-1-oxopropan-2-yl)-4-phenyl-1,2,3,6-tetrahydropyridine-2-carboxamide (L023-IS-02 or L023-IS-01) Hydrochloride

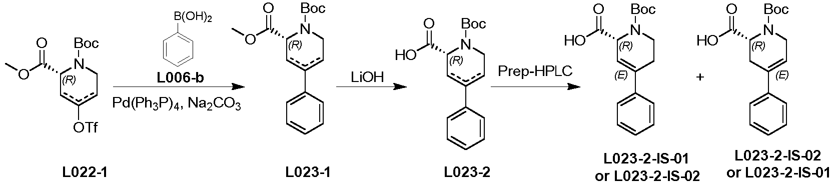

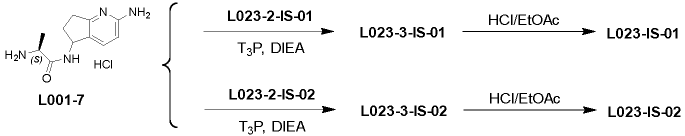

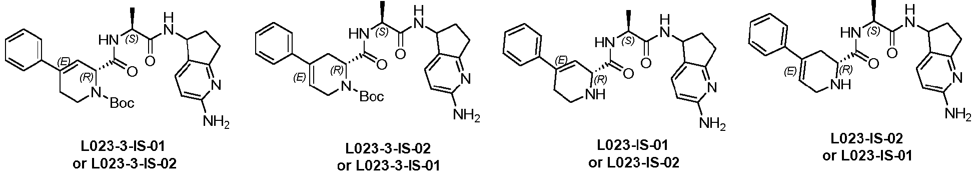

### 23.1: Synthesis of compound L023-1

L022-1 (2.5 g, 6.42 mmol), L006-b (1.17 g, 9.63 mmol), tetrakis(triphenylphosphine)palladium(0) (226 mg, 0.32 mmol) and sodium carbonate (1.36 g, 12.84 mmol) were dissolved in tetrahydrofuran (75 mL) and water (15 mL) at room temperature. The reaction liquid was stirred at 40 °C for 4 h. The reaction liquid was cooled to room temperature, added with water (50 mL) for dilution, and extracted with ethyl acetate (50 mL × 3). The combined organic phases were dried over anhydrous sodium sulfate and filtered, and the filtrate was concentrated under reduced pressure. The residue was purified by silica gel column chromatography to give compound L023-1 (2.1 g, a double bond isomeric mixture). LC-MS: [M-Boc+H]⁺ = 218.10.

### 23.2: Synthesis of compound L023-2

L023-1 (1.5 g, 4.73 mmol) was dissolved in tetrahydrofuran (20 mL), methanol (10 mL) and water (10 mL), and then lithium hydroxide monohydrate (600 mg, 14.2 mmol) was added at room temperature. The reaction liquid was stirred for 2 h. The reaction liquid was directly concentrated under reduced pressure, and the residue was added with water (30 mL) for dilution and washed with ethyl acetate (30 mL × 3). The organic phase was discarded. The aqueous phase was adjusted to pH = 5-6 with diluted hydrochloric acid (1 M) and extracted with ethyl acetate (30 mL × 3). The combined organic phases were dried over anhydrous sodium sulfate and filtered, and the filtrate was concentrated under reduced pressure to give compound L023-2 (1.2 g, a double bond isomeric mixture). LC-MS: [M-Boc+H]⁺ =204.10.

### 23.3: Synthesis of compounds L023-2-IS-01 and L023-2-IS-02

Compound L023-2 (1.2 g, 3.96 mmol) was separated by preparative high performance liquid chromatography (Prep-HPLC) (column: CHIRAL ART Amylose-C NEO, 30 × 250 mm, 10 µm, mobile phase, A: n-hexane (0.1% acetic acid), B: isopropanol, flow rate: 30 mL/min) to give compound L023-2-IS-01 (retention time Rt = 10.854 min, 400 mg) and L023-2-IS-02 (retention time Rt = 17.612 min, 540 mg).
L023-2-IS-01:
   ¹H NMR (400 MHz, CH₃OH-*d₄*): δ 7.45 - 7.28 (m, 5H), 6.34 - 6.07 (m, 1H), 5.04 - 4.98 (m, 1H), 4.26 - 4.21 (m, 1H), 3.31 - 3.15 (m, 1H), 2.67 - 2.46 (m, 2H), 1.53 - 1.49 (m, 9H); LC-MS: [M-Boc+H]⁺= 204.10.
L023-2-IS-02:
   ¹H NMR (400 MHz, CH₃OH-*d₄*): δ 7.58 - 7.16 (m, 5H), 6.41 - 6.19 (m, 1H), 5.06 - 4.94 (m, 1H), 4.27 - 4.21 (m, 1H), 3.23 - 3.11 (m, 1H), 2.59 - 2.47 (m, 2H), 1.53 - 1.49 (m, 9H); LC-MS: [M-Boc+H]⁺= 204.10.

### 23.4: Synthesis of compounds L023-3-IS-01 and L023-3-IS-02

L023-2-IS-01 (100 mg, 0.33 mmol) was dissolved in dichloromethane (3 mL) at room temperature. 1-Propylphosphoric cyclic anhydride (315 mg, 0.49 mmol, 50% in ethyl acetate), N,N-diisopropylethylamine (213 mg, 1.65 mmol) and L001-7 (85 mg, 0.33 mmol) were added, and the reaction liquid was stirred at 20 °C for 1 h. The reaction liquid was added with water (10 mL) for dilution and extracted with dichloromethane (10 mL × 3). The combined organic phases were dried over anhydrous sodium sulfate and filtered, and the filtrate was concentrated under reduced pressure. The residue was purified by high performance liquid chromatography (acidic condition) and lyophilized to give compound L023-3-IS-01 (60 mg, yield: 36%). LC-MS: [M-Boc+H]⁺ = 406.10.

L023-2-IS-02 (100 mg, 0.33 mmol) was dissolved in dichloromethane (3 mL) at room temperature. 1-Propylphosphoric cyclic anhydride (315 mg, 0.49 mmol, 50% in ethyl acetate), N,N-diisopropylethylamine (213 mg, 1.65 mmol) and L001-7 (85 mg, 0.33 mmol) were added, and the reaction liquid was stirred at 20 °C for 1 h. The reaction liquid was added with water (10 mL) for dilution and extracted with dichloromethane (10 mL × 3). The combined organic phases were dried over anhydrous sodium sulfate and filtered, and the filtrate was concentrated under reduced pressure. The residue was purified by high performance liquid chromatography (acidic condition) and lyophilized to give compound L023-3-IS-02 (75 mg, yield: 45%). LC-MS: [M-Boc+H]⁺ = 406.10.

### 23.5: Synthesis of compounds L023-IS-01 hydrochloride and L023-IS-02 hydrochloride

L023-3-IS-01 (60 mg, 0.12 mmol) was dissolved in ethyl acetate (2 mL), and then a solution of hydrogen chloride in ethyl acetate (2 mL, 4 M) was added. The reaction liquid was reacted at room temperature for 2 h, and a white solid was precipitated. The reaction liquid was directly concentrated under reduced pressure, and the residue was dissolved by adding deionized water (10 mL) and lyophilized to give compound L023-IS-01 hydrochloride (48.6 mg, yield: 92.66%).

L023-3-IS-02 (75 mg, 0.15 mmol) was dissolved in ethyl acetate (2 mL), and then a solution of hydrogen chloride in ethyl acetate (2 mL, 4 M) was added. The reaction liquid was reacted at room temperature for 2 h, and a white solid was precipitated. The reaction liquid was directly concentrated under reduced pressure. The residue was dissolved by adding deionized water (10 mL) and lyophilized to give compound L023-IS-02 hydrochloride (43.2 mg, yield: 65.89%).
L023-IS-01 hydrochloride:
   ¹H NMR (400 MHz, DMSO-*d₆*): δ 10.09 (s, 1 H), 9.07 (dd, *J =* 7.2, 3.6 Hz, 1 H), 8.94 (s, 1 H), 8.54 (dd, *J =* 24.4, 8.0 Hz, 1 H), 7.83 (d, *J =* 24.4 Hz, 2 H), 7.59 (dd, *J =* 32.0, 8.8 Hz, 1 H), 7.50 - 7.31 (m, 5 H), 6.71 (t, *J =* 9.6 Hz, 1 H), 6.34 (d, *J =* 8.8 Hz, 1 H), 5.20 - 5.09 (m, 1 H), 4.77 (s, 1 H), 4.33 - 4.26 (m, 1 H), 3.43 (s, 2 H), 3.05 - 2.81 (m, 2 H), 2.69 (s, 2 H), 2.48 - 2.37 (m, 1 H), 2.04 - 1.79 (m, 1 H), 1.35-1.32 (m, 3 H); LC-MS: [M+H]⁺ = 406.10.
L023-IS-02 hydrochloride:
   ¹H NMR (400 MHz, DMSO-*d₆*): δ 10.09 (d, *J =* 38.4 Hz, 1 H), 9.08 (dd, *J =* 7.2, 4.4 Hz, 1 H), 8.96 - 8.94 (m, 1 H), 8.61 - 8.48 (m, 1 H), 7.93 - 7.52 (m, 3 H), 7.51 - 7.44 (m, 2 H), 7.43 - 7.30 (m, 3 H), 6.75 (d, *J =* 8.0 Hz, 1 H), 6.30 (s, 1 H), 5.17 (q, *J =* 7.6 Hz, 1 H), 4.78 (s, 1 H), 4.35 - 4.22 (m, 1 H), 3.44 - 3.38 (m, 2 H), 3.16 - 2.96 (m, 1 H), 2.94 - 2.81 (m, 1 H), 2.81 - 2.54 (m, 2 H), 2.46 - 2.39 (m, 1 H), 2.00 - 1.93 (m, 1 H), 1.31 (d, *J =* 7.2 Hz, 3 H); LC-MS: [M+H]⁺ = 406.10.

### Example 24: Synthesis of (2R)-N-((2S)-1-((2-amino-6,7-dihydro-5H-cyclopenta[b]pyridin-5-yl)amino)-1-oxopropan-2-yl)-4-benzylpiperazine-2-carboxamide (Compound L024) Hydrochloride

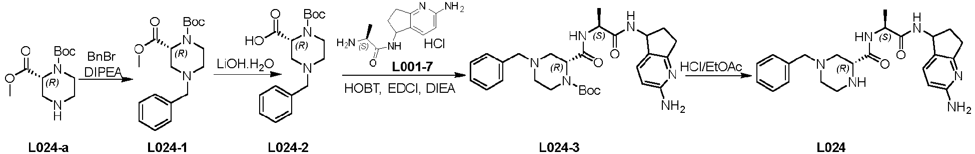

### 24.1: Synthesis of compound L024-1

L024-a (300 mg, 1.23 mmol) was dissolved in N,N-dimethylformamide (3 mL), and N,N-diisopropylethylamine (239 mg, 1.85 mmol) and benzyl bromide (221 mg, 1.29 mmol) were added to the system. The reaction liquid was stirred at room temperature for 1 h. The reaction liquid was added with water (10 mL) for dilution and extracted with ethyl acetate (20 mL × 3). The combined organic phases were washed with a saturated aqueous ammonium chloride solution (30 mL), dried over anhydrous sodium sulfate and filtered, and the filtrate was concentrated under reduced pressure. The residue was purified by silica gel column chromatography to give compound L024-1 (344 mg, yield: 83.7%). LC-MS: [M+H]⁺ = 335.10.

### 24.2: Synthesis of compound L024-2

L024-1 (344 mg, 1.03 mmol) was dissolved in tetrahydrofuran (4 mL) and water (4 mL), and lithium hydroxide monohydrate (169 mg, 4.12 mmol) was added. The reaction liquid was stirred at room temperature for 16 h. The reaction liquid was added with water (5 mL) and extracted with ethyl acetate (10 mL). The organic phase was discarded. The aqueous phase was adjusted to pH = about 5-6 with formic acid and then extracted with ethyl acetate (15 mL × 3). The combined organic phases were dried over anhydrous sodium sulfate and filtered, and the filtrate was concentrated under reduced pressure to give compound L024-2 (343 mg, crude product), which could be used directly in the next step. LC-MS: [M+H]⁺ = 321.10.

### 24.3: Synthesis of compound L024-3

L024-2 (90 mg, 0.28 mmol) was dissolved in N,N-dimethylformamide (2 mL), and 1-hydroxybenzotriazole (41.6 mg, 0.31 mmol) and 1-(3-dimethylaminopropyl)-3-ethylcarbodiimide hydrochloride (59.1 mg, 0.31 mmol) were added. The reaction liquid was reacted at room temperature for 0.5 h. Meanwhile, L001-7 (100 mg, 0.34 mmol) and N,N-diisopropylethylamine (145 mg, 1.12 mmol) were dissolved in N,N-dimethylformamide (2 mL), and the solution was stirred at room temperature for 0.5 h. The solution was then added to the reaction liquid described above and stirred at room temperature for 0.5 h. The reaction liquid was added with water (10 mL) for dilution, and extracted with ethyl acetate (20 mL × 3). The combined organic phases were washed with a saturated aqueous ammonium chloride solution (30 mL), dried over anhydrous sodium sulfate and filtered, and the filtrate was concentrated under reduced pressure. The residue was purified by preparative high performance liquid chromatography (acidic condition) to give compound L024-3 (61.0 mg, yield: 41.5%). LC-MS: [M+H]⁺= 523.10.

### 24.4: Synthesis of compound L024 hydrochloride

L024-3 (61.0 mg, 0.12 mmol) was dissolved in dichloromethane (2 mL), and a solution of hydrogen chloride in ethyl acetate (2 mL, 4 M) was added at room temperature. The reaction liquid was stirred for 0.5 h, and a solid was precipitated. The reaction liquid was filtered, and the filter cake was washed with dichloromethane (15 mL). The filter cake was dissolved in deionized water (15 mL) and lyophilized to give compound L024 hydrochloride (46 mg, yield: 93.1%). LC-MS: [M+H]⁺ = 423.15. ¹H NMR (400 MHz, DMSO-*d₆*): δ 10.63 (s, 1H), 9.50 - 9.27 (m, 2H), 8.63 - 8.38 (m, 1H), 8.03 (s, 2H), 7.73 - 7.52 (m, 3H), 7.45 - 7.41 (m, 4H), 6.83 (d, *J* = 4.0 Hz, 1H), 5.16 - 5.14 (m, 1H), 4.48 - 4.14 (m, 4H), 4.10 - 3.80 (m, 5H), 3.11 - 2.84 (m, 4H), 2.50 - 2.43 (m, 1H), 2.02 - 2.00 (m, 1H), 1.23 (d, *J* = 4.0 Hz, 3H); LC-MS: [M+H]⁺= 423.15.

### Example 25: Synthesis of (2R)-N-(2S)-1-(2-amino-6,7-dihydro-5H-cyclopenta[b]pyridin-5-yl) amino)-1-oxopropanon-2-yl)-4-(naphthalen-1-yl)piperazine-2-carboxamide (Compound L025) Hydrochloride

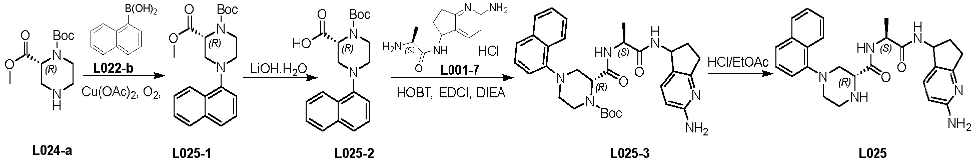

### 25.1: Synthesis of compound L025-1

L024-a (600 mg, 2.46 mmol) was dissolved in dichloromethane (6 mL). Copper(II) acetate (489 mg, 2.70 mmol), L022-b (847 mg, 4.92 mmol), 4Å molecular sieves (300 mg) and pyridine (389 mg, 4.92 mmol) were added. The reaction liquid was stirred at room temperature for 16 h under oxygen atmosphere. The reaction liquid was directly concentrated under reduced pressure, and the residue was purified by silica gel column chromatography to give compound L025-1 (167 mg, yield: 18.3%). LC-MS: [M+H]⁺=371.05.

### 25.2: Synthesis of compound L025-2

L025-1 (167 mg, 0.45 mmol) was dissolved in tetrahydrofuran (2 mL) and water (2 mL), and lithium hydroxide monohydrate (74 mg, 1.81 mmol) was added. The reaction liquid was stirred at room temperature for 16 h. The reaction liquid was added with water (5 mL) and extracted with ethyl acetate (10 mL). The organic phase was discarded, and the aqueous phase was adjusted to pH = 5-6 with formic acid and then extracted with ethyl acetate (15 mL × 3). The combined organic phases were dried over anhydrous sodium sulfate and filtered, and the filtrate was concentrated under reduced pressure to give compound L025-2 (50 mg, yield: 30.5%). LC-MS: [M+H]⁺ = 357.05.

### 25.3: Synthesis of compound L025-3

L025-2 (50 mg, 0.14 mmol) was dissolved in N,N-dimethylformamide (1 mL), and 1-hydroxybenzotriazole (21 mg, 0.15 mmol) and 1-(3-dimethylaminopropyl)-3-ethylcarbodiimide hydrochloride (30 mg, 0.15 mmol) were added. The reaction liquid was reacted at room temperature for 0.5 h. Meanwhile, L001-7 (49 mg, 0.17 mmol) and N,N-diisopropylethylamine (72.3 mg, 0.56 mmol) were dissolved in N,N-dimethylformamide (1 mL), and the solution was stirred at room temperature for 0.5 h. The solution was then added to the reaction liquid described above and stirred for 0.5 h. The reaction liquid was added with water (5 mL) for dilution, and extracted with ethyl acetate (15 mL × 3). The combined organic phases were washed with a saturated aqueous ammonium chloride solution (20 mL), dried over anhydrous sodium sulfate and filtered, and the filtrate was concentrated under reduced pressure. The residue was purified by preparative high performance liquid chromatography (acidic condition) to give compound L025-3 (38.0 mg, yield: 48.5%). LC-MS: [M+H]⁺ = 559.05.

### 25.4: Synthesis of compound L025 hydrochloride

L025-3 (38 mg, 0.07 mmol) was dissolved in dichloromethane (2 mL), and a solution of hydrogen chloride in ethyl acetate (2 mL, 4 M) was added at room temperature. The reaction liquid was stirred for 0.5 h. A solid was precipitated. The reaction liquid was filtered, and the filter cake was washed with dichloromethane (15 mL). The filter cake was dissolved in deionized water (15 mL) and lyophilized to give compound L025 hydrochloride (9 mg, yield: 28.9%).

¹H NMR (400 MHz, DMSO-*d₆*): δ 10.10 (m, 1H), 9.17 - 9.04 (m, 2H), 8.53 (dd, *J =* 8.0, 4.0 Hz, 1H), 8.23 (s, 1H), 8.02 - 7.76 (m, 3H), 7.69 (d, *J =* 8.0 Hz, 2H), 7.64 - 7.54 (m, 2H), 7.46 (m, 1H), 7.40 - 7.12 (m, 2H), 6.80 - 6.76 (m, 1H), 5.33 - 5.16 (m, 1H), 4.34 - 4.24 (m, 2H), 3.60 (m, 1H), 3.09 (s, 2H), 2.93 - 2.85 (m, 1H), 2.47 - 2.40 (m, 1H), 2.07 - 1.93 (m, 2H), 1.24 (m, 7H); LC-MS: [M+H]⁺= 459.05.

### Example 26: Synthesis of (2R)-N-(2S)-1-(2-amino-6,7-dihydro-5H-cyclopenta[b]pyridin-5-yl) amino)-1-oxopropan-2-yl)-4-(4-fluorophenyl)piperazine-2-carboxamide (Compound L026) Hydrochloride

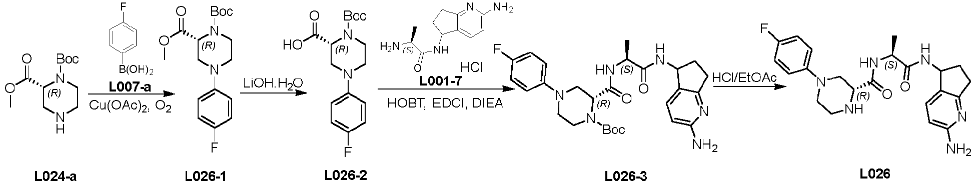

### 26.1: Synthesis of compound L026-1

L024-a (400 mg, 1.64 mmol) and L007-a (459 mg, 3.28 mmol) were dissolved in dichloromethane (3 mL). Copper(II) acetate (327 mg, 1.81 mmol), 4Å molecular sieves (200 mg) and pyridine (259 mg, 3.28 mmol) were added to the system. The reaction liquid was stirred at room temperature for 16 h under oxygen atmosphere. The reaction liquid was directly concentrated under reduced pressure, and the residue was purified by silica gel column chromatography to give compound L026-1 (424 mg, yield: 76.5%). LC-MS: [M+H]⁺ = 339.05.

### 26.2: Synthesis of compound L026-2

L026-1 (424 mg, 1.25 mmol) was dissolved in tetrahydrofuran (4 mL) and water (4 mL), and lithium hydroxide monohydrate (154 mg, 3.76 mmol) was added. The reaction liquid was stirred at room temperature for 16 h. The reaction liquid was added with water (5 mL) for dilution and extracted with ethyl acetate (10 mL). The organic phase was discarded, and the aqueous phase was adjusted to pH = 5-6 with formic acid and then extracted with ethyl acetate (15 mL × 3). The combined organic phases were dried over anhydrous sodium sulfate and filtered, and the filtrate was concentrated under reduced pressure to give compound L026-2 (325 mg). LC-MS: [M+H]⁺ = 325.05.

### 26.3: Synthesis of compound L026-3

L026-2 (81 mg, 0.25 mmol) was dissolved in N,N-dimethylformamide (2 mL), and 1-hydroxybenzotriazole (37 mg, 0.27 mmol) and 1-(3-dimethylaminopropyl)-3-ethylcarbodiimide hydrochloride (53 mg, 0.27 mmol) were added. The reaction liquid was stirred at room temperature for 0.5 h. Meanwhile, L001-7 (80 mg, 0.27 mmol) and N,N-diisopropylethylamine (160.0 mg, 1.24 mmol) were dissolved in N,N-dimethylformamide (2 mL), and the solution was stirred at room temperature for 0.5 h. The solution was then added to the reaction liquid described above and stirred at room temperature for 0.5 h. The reaction liquid was added with water (10 mL) for dilution and extracted with ethyl acetate (20 mL × 3). The combined organic phases were washed with saturated brine (30 mL), dried over anhydrous sodium sulfate and filtered, and the filtrate was concentrated under reduced pressure. The residue was purified by preparative high performance liquid chromatography (acidic condition) to give compound L026-3 (50 mg, yield: 38.2%). LC-MS: [M+H]⁺ = 527.05.

### 26.4: Synthesis of compound L026 hydrochloride

L026-3 (50 mg, 0.10 mmol) was dissolved in dichloromethane (2 mL), and a solution of hydrogen chloride in ethyl acetate (2 mL, 4 M) was added at room temperature. The mixture was stirred for 0.5 h, and a solid was precipitated. The solid was filtered, and the filter cake was washed with dichloromethane (15 mL). The filter cake was dissolved in deionized water (15 mL) and lyophilized to give compound L026 hydrochloride (36.0 mg, yield: 88.8%).

¹H NMR (400 MHz, DMSO-*d₆*): δ 10.08 - 9.95 (m, 1H), 9.14 (d, *J =* 4.0 Hz, 2H), 8.58 (dd, *J*₁ = 8.0 Hz, *J*₂ = 2.0 Hz, 1H), 8.04 (s, 1H), 7.74 - 7.67 (m, 1H), 7.46 - 7.17 (m, 1H), 7.15 - 7.03 (m, 4H), 6.84 (dd, *J*₁ = 12.0 Hz, *J*₂ = 4.0 Hz, 1H), 5.20 - 5.15 (m, 1H), 4.35 - 4.23 (m, 1H), 4.01-3.98 (m, 2H), 3.58 (d, *J =* 12.0 Hz, 1H), 3.32 (d, *J =* 12.0 Hz, 1H), 3.20 - 2.78 (m, 6H), 2.48 - 2.41 (m, 1H), 2.03 - 1.99 (m, 1H), 1.30 - 1.23 (m, 4H); LC-MS: [M+H] ⁺ = 427.05.

### Example 27: Synthesis of (2S)-1-(2R,4S)-4-([1,1'-biphenyl]-4-(4-methyl)pyrrolidine-2-carbonyl)-N-(2-amino-6,7-dihydro-5H-cyclopenta[b]pyridin-5-yl)azetidine-2-carboxamide (Compound L027) Hydrochloride

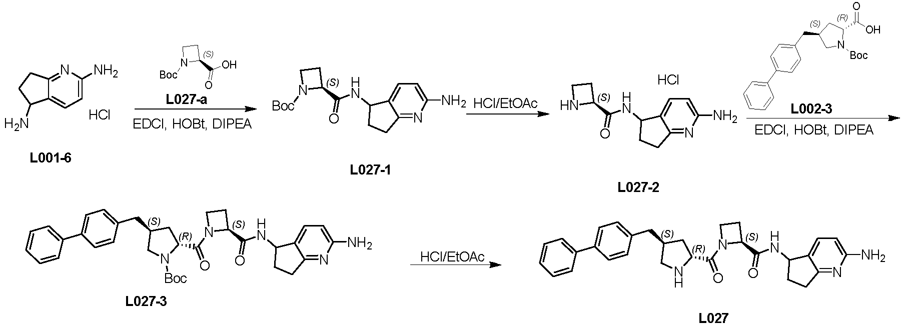

### 27.1: Synthesis of compound L027-1

L027-a (91 mg, 0.45 mmol), 1-hydroxybenzotriazole (67 mg, 0.50 mmol), N,N-dimethylformamide (2 mL) and 1-(3-dimethylaminopropyl)-3-ethylcarbodiimide hydrochloride (95 mg, 0.50 mmol) were added to a reaction flask. The reaction liquid was reacted at room temperature for 0.5 h. Meanwhile, a solution of L001-6 (150 mg, 0.68 mmol) and N,N-diisopropylethylamine (232 mg, 1.80 mmol) in N,N-dimethylformamide (2 mL) was stirred at room temperature for 0.5 h. The solution was then added to the reaction liquid described above at room temperature and stirred for another 0.5 h. The reaction liquid was added with water (10 mL) for dilution and extracted with ethyl acetate (15 mL × 3). The combined organic phases were washed with saturated sodium chloride (20 mL), dried over anhydrous sodium sulfate and filtered, and the filtrate was concentrated under reduced pressure to give compound L027-1 (160 mg, crude product). LC-MS: [M+H]⁺ = 333.10.

### 27.2: Synthesis of compound L027-2

L027-1 (160 mg, crude product) was dissolved in dichloromethane (4 mL), and a solution of hydrogen chloride in ethyl acetate (4 mL, 4 M) was added. The reaction liquid was stirred at room temperature for 0.5 h, and a solid was precipitated. The reaction liquid was filtered, and the filter cake was washed with dichloromethane (5 mL) and dried under reduced pressure to give compound L027-2 hydrochloride (130 mg, crude product). LC-MS: [M+H]⁺ = 233.15.

### 27.3: Synthesis of compound L027-3

L002-3 (141 mg, 0.37 mmol), 1-hydroxybenzotriazole (55 mg, 0.41 mmol), N,N-dimethylformamide (2 mL) and 1-(3-dimethylaminopropyl)-3-ethylcarbodiimide hydrochloride (78 mg, 0.41 mmol) were added to a reaction flask at room temperature. The reaction liquid was stirred at room temperature for 0.5 h. Meanwhile, L027-2 (130 mg, crude product) and N,N-diisopropylethylamine (191 mg, 1.48 mmol) were dissolved in N,N-dimethylformamide (2 mL), and the solution was stirred at room temperature for 0.5 h. The solution was then added to the reaction liquid described above, and the reaction liquid was stirred for another 0.5 h. The reaction liquid was added with water (10 mL) and extracted with ethyl acetate (15 mL × 3). The combined organic phases were washed with saturated sodium chloride (20 mL), dried over anhydrous sodium sulfate and filtered, and the filtrate was concentrated under reduced pressure to give compound L027-3 (260 mg, crude product). LC-MS: [M+H]⁺ = 596.10.

### 27.4: Synthesis of compound L027 hydrochloride

L027-3 (260 mg, crude product) was dissolved in dichloromethane (5 mL), and a solution of hydrogen chloride in ethyl acetate (5 mL, 4 M) was added at room temperature. The reaction liquid was stirred for 0.5 h, and a solid was precipitated. The reaction liquid was filtered, and the filter cake was washed with dichloromethane (5 mL) to give a crude product. The crude product was purified by high performance liquid chromatography (acidic condition) to give compound L027 hydrochloride (10 mg). ¹H NMR (400 MHz, DMSO-*d₆*): δ 10.43 (s, 1H), 9.03 - 8.60 (m, 2H), 8.12 - 7.81 (m, 2H), 7.74 - 7.60 (m, 5H), 7.45 (t, *J =* 8.0 Hz, 2H), 7.35 - 7.29 (m, 3H), 6.84 (t, *J =* 8.0 Hz, 1H), 5.21 - 5.08 (m, 1H), 4.66 - 4.39 (m, 1H), 4.28 - 4.09 (m, 2H), 3.96 - 3.80 (m, 1H), 3.29 (s, 1H), 3.07 - 3.02 (m, 1H), 2.98 - 2.52 (m, 6H), 2.48 - 2.39 (m, 1H), 2.18 - 1.74 (m, 4H); LC-MS: [M+H]⁺= 496.15.

### Example 28: Synthesis of (2S)-1-(2R,4S)-4-([1,1'-biphenyl]-4-(4-ylmethyl)pyrrolidine-2-carbonyl)-N-(2-amino-6,7-dihydro-5H-cyclopenta[b]pyridin-5-yl)pyrrolidine-2-carboxamide (Compound L028) Hydrochloride

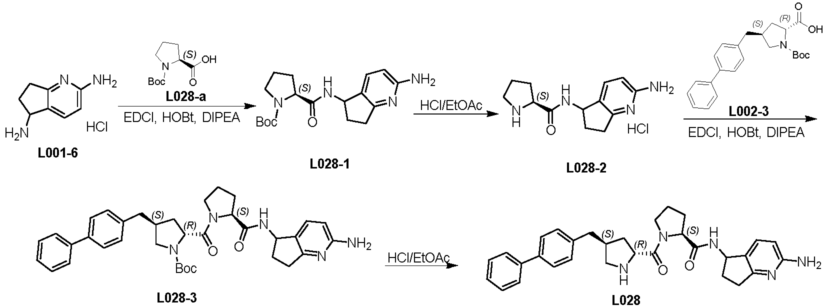

### 28.1: Synthesis of compound L028-1

L028-a (97 mg, 0.45 mmol), 1-hydroxybenzotriazole (67 mg, 0.50 mmol), N,N-dimethylformamide (2 mL) and 1-(3-dimethylaminopropyl)-3-ethylcarbodiimide hydrochloride (95 mg, 0.50 mmol) were added to a reaction flask. The reaction liquid was stirred at room temperature for 0.5 h. Meanwhile, L001-6 (150 mg, 0.68 mmol) and N,N-diisopropylethylamine (232.2 mg, 1.80 mmol) were dissolved in N,N-dimethylformamide (2 mL), and the solution was stirred at room temperature for 0.5 h. The solution was then added to the reaction liquid described above, and the reaction liquid was stirred for another 0.5 h. The reaction liquid was added with water (10 mL) for dilution and extracted with ethyl acetate (15 mL × 3). The combined organic phases were washed with a saturated aqueous sodium chloride solution (20 mL), dried over anhydrous sodium sulfate and filtered, and the filtrate was concentrated under reduced pressure to give compound L028-1 (210 mg, crude product). LCMS: [M+H]⁺ = 347.15.

### 28.2: Synthesis of compound L028-2

L028-1 (210 mg, 0.61 mmol) was dissolved in dichloromethane (4 mL), and a solution of hydrogen chloride in ethyl acetate (4 mL, 4 M) was added. The reaction liquid was stirred at room temperature for 0.5 h, and a solid was precipitated. The reaction liquid was filtered, and the filter cake was washed with dichloromethane (5 mL). The solid was dried under reduced pressure to give compound L028-2 (120 mg, crude product). LC-MS: [M+H]⁺= 247.15.

### 28.3: Synthesis of compound L028-3

L002-3 (125.7 mg, 0.33 mmol), 1-hydroxybenzotriazole (49 mg, 0.36 mmol), N,N-dimethylformamide (2 mL) and 1-(3-dimethylaminopropyl)-3-ethylcarbodiimide hydrochloride (69.7 mg, 0.36 mmol) were added to a reaction flask. The reaction liquid was stirred at room temperature for 0.5 h. Meanwhile, L028-2 (120 mg, crude product) and N,N-diisopropylethylamine (170 mg, 1.32 mmol) were dissolved in N,N-dimethylformamide (2 mL), and the solution was stirred at room temperature for 0.5 h. The solution was then added to the reaction liquid described above, and the reaction liquid was stirred for another 0.5 h. The reaction liquid was added with water (10 mL) for dilution and extracted with ethyl acetate (15 mL × 3). The combined organic phases were washed with a saturated aqueous sodium chloride solution (20 mL), dried over anhydrous sodium sulfate and filtered, and the filtrate was concentrated under reduced pressure to give compound L028-3 (240 mg, crude product). LC-MS: [M+H]⁺= 610.10.

### 28.4: Synthesis of compound L028 hydrochloride

At room temperature, L028-3 (240 mg, crude product) was dissolved in dichloromethane (5 mL), and a solution of hydrogen chloride in ethyl acetate (5 mL, 4 M) was added. The reaction liquid was stirred at room temperature for 0.5 h, and a solid was precipitated. The reaction liquid was filtered, and the filter cake was washed with dichloromethane (5 mL) and dried under reduced pressure to give a crude product. The crude product was purified by high performance liquid chromatography (acidic condition) to give compound L028 hydrochloride (27 mg).

¹H NMR (400 MHz, DMSO-*d₆*): δ 10.53 - 10.50 (m, 1H), 8.93 - 8.58 (m, 2H), 8.09 (s, 2H), 7.77 - 7.58 (m, 5H), 7.45 (t, *J =* 8.0 Hz, 2H), 7.35 - 7.31 (m, 3H), 6.87 - 6.83 (m, 1H), 5.23 - 4.99 (m, 1H), 4.71 - 4.51 (m, 1H), 4.26 - 4.23 (m, 1H), 3.50 - 3.32 (m, 3H), 3.09 - 2.74 (m, 5H), 2.64 - 2.52 (m, 1H), 2.46 - 2.30 (m, 1H), 2.17 - 1.82 (m, 7H); LC-MS: [M+H] ⁺=510.15.

### Example 29: Synthesis of (2S)-1-(2R,4S)-4-([1,1'-biphenyl]-4-(4-ylmethyl)pyrrolidine-2-carbonyl)-N-(2-amino-6,7-dihydro-5H-cyclopenta[b]pyridin-5-yl)pyrrolidine-2-carboxamide (Compound L029) Hydrochloride

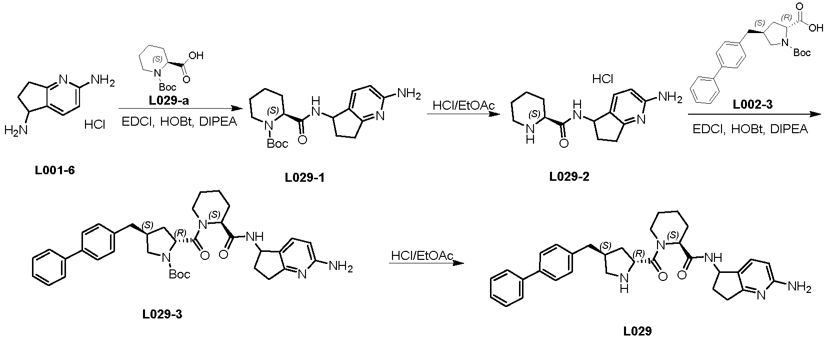

### 29.1: Synthesis of compound L029-1

L029-a (103 mg, 0.45 mmol), 1-hydroxybenzotriazole (67 mg, 0.50 mmol), N,N-dimethylformamide (2 mL) and 1-(3-dimethylaminopropyl)-3-ethylcarbodiimide hydrochloride (95 mg, 0.50 mmol) were added to a reaction flask. The reaction liquid was reacted at room temperature for 0.5 h. Meanwhile, L001-6 (150 mg, 0.68 mmol) and N,N-diisopropylethylamine (232 mg, 1.80 mmol) were dissolved in N,N-dimethylformamide (2 mL), and the solution was stirred at room temperature for 0.5 h. The solution was then added to the reaction liquid described above, and the reaction liquid was stirred for another 0.5 h. The reaction liquid was added with water (10 mL) for dilution and extracted with ethyl acetate (15 mL × 3). The combined organic phases were washed with a saturated aqueous sodium chloride solution (20 mL), dried over anhydrous sodium sulfate and filtered, and the filtrate was concentrated under reduced pressure to give compound L029-1 (300 mg, crude product). LCMS: [M+H]⁺ = 361.10.

### 29.2: Synthesis of compound L029-2

L029-1 (300 mg, crude product) was dissolved in dichloromethane (5 mL), and a solution of hydrogen chloride in ethyl acetate (5 mL, 4 M) was added at room temperature. The reaction liquid was stirred for 0.5 h, and a solid was precipitated. The reaction liquid was filtered, and the filter cake was washed with dichloromethane (5 mL). The filter cake was dried under reduced pressure to give compound L029-2 (140 mg, crude product). LC-MS: [M+H]⁺= 261.15.

### 29.3: Synthesis of compound L029-3

L002-3 (137 mg, 0.36 mmol), 1-hydroxybenzotriazole (54 mg, 0.40 mmol), N,N-dimethylformamide (2 mL) and 1-(3-dimethylaminopropyl)-3-ethylcarbodiimide hydrochloride (76 mg, 0.40 mmol) were added to a reaction flask. The reaction liquid was stirred at room temperature for 0.5 h. Meanwhile, L029-2 (140 mg, 0.54 mmol) and N,N-diisopropylethylamine (185.7 mg, 1.44 mmol) were dissolved in N,N-dimethylformamide (2 mL), and the solution was stirred at room temperature for 0.5 h. The solution was then added to the reaction liquid described above and stirred for another 0.5 h. The reaction liquid was added with water (10 mL) for dilution and extracted with ethyl acetate (15 mL × 3). The combined organic phases were washed with a saturated aqueous sodium chloride solution (20 mL), dried over anhydrous sodium sulfate and filtered, and the filtrate was concentrated under reduced pressure to give compound L029-3 (230 mg, crude product). LC-MS: [M+H]⁺= 624.10.

### 29.4: Synthesis of compound L029 hydrochloride

L029-3 (230 mg, 0.37 mmol) was dissolved in dichloromethane (5 mL), and a solution of hydrogen chloride in ethyl acetate (5 mL, 4 M) was added. The reaction liquid was stirred at room temperature for 0.5 h, and a solid was precipitated. The reaction liquid was filtered, and the filter cake was washed with dichloromethane (5 mL) and purified by high performance liquid chromatography (acidic condition) to give compound L029 hydrochloride (65 mg).

¹H NMR (400 MHz, DMSO-*d₆*): δ 10.50 (s, 1H), 8.61 - 8.54(m, 2H), 8.08 (s, 2H), 7.72 - 7.53 (m, 5H), 7.45 (t, *J* =8.0 Hz, 2H), 7.40 - 7.30 (m, 3H), 6.88 - 6.80 (m, 1H), 5.34 - 5.07 (m, 1H), 4.97 - 4.80 (m, 1H), 4.77 - 4.28 (m, 1H), 3.54 - 3.00 (m, 3H), 2.97 - 2.53 (m, 5H), 2.47 - 2.31 (m, 1H), 2.30 - 2.06 (m, 2H), 2.03 - 1.92 (m, 2H), 1.81 - 1.12 (m, 6H); LC-MS: [M+H] ⁺ = 524.10.

### Example 30: Synthesis of (2S)-N-(2-amino-6,7-dihydro-5H-cyclopenta[b]pyridin-5-yl)-1-((2R,4S)-4-(4-fluorobenzyl)pyrrolidine-2-carbonyl)azetidine-2-carboxamide (Compound L030) Hydrochloride

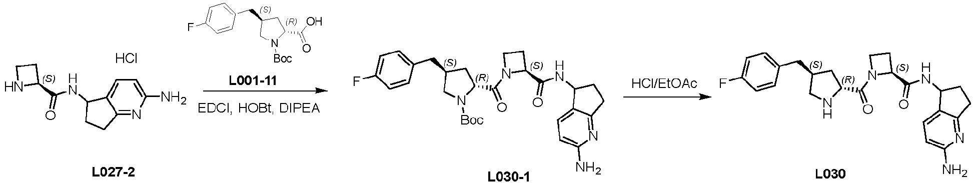

### 30.1: Synthesis of compound L030-1

L001-11 (150 mg, 0.46 mmol) was dissolved in N,N-dimethylformamide (3 mL). 1-Hydroxybenzotriazole (75 mg, 0.56 mmol) and 1-(3-dimethylaminopropyl)-3-ethylcarbodiimide hydrochloride (107 mg, 0.56 mmol) were added. The reaction liquid was stirred at room temperature for 10 min. A solution of L027-2 (162 mg, 0.60 mmol) and N,N-diisopropylethylamine (299 mg, 2.32 mmol) in N,N-dimethylformamide (2 mL) was added. The reaction liquid was reacted at room temperature for 0.5 h. The reaction liquid was poured into water (10 mL), and the mixture was extracted with ethyl acetate (15 mL × 3). The combined organic phases were washed with a saturated aqueous sodium chloride solution (10 mL × 3), dried over anhydrous sodium sulfate and filtered, and the filtrate was concentrated under reduced pressure to give compound L030-1 (180 mg, yield: 72.18%). LCMS: [M+H]⁺ = 538.10.

### 30.2: Synthesis of compound L030 hydrochloride

L030-1 (180 mg, 0.33 mmol) was dissolved in dichloromethane (3 mL), and a solution of hydrogen chloride in ethyl acetate (2 mL, 4 M) was added. The reaction liquid was stirred at room temperature for 1 h. The reaction liquid was concentrated under reduced pressure, and the residue was purified by preparative high performance liquid chromatography (acidic condition) and lyophilized to give compound L030 hydrochloride (43.1 mg, yield: 27.10%).

¹H NMR (400 MHz, DMSO-*d₆*): δ 10.31 (s, 1H), 8.99 - 8.52 (m, 2H), 8.02 (s, 2H), 7.83 - 7.64 (m, 1H), 7.31 - 7.24 (m, 2H), 7.17 - 7.08 (m, 2H), 6.82 (dd, *J =* 8.0, 4.0 Hz, 1H), 5.27 - 4.84 (m, 2H), 4.62 (dd, *J =* 8.0, 4.0 Hz, 1H), 4.39 (dd, *J =* 12.0, 4.0 Hz, 1H), 4.27 - 4.22 (m, 1H), 4.08 (dd, *J =* 12.0, 8.0 Hz, 1H), 3.94 - 3.84 (m, 1H), 3.30 - 3.19 (m, 1H), 3.08 - 3.02 (m, 1H), 2.98 - 2.90 (m, 1H), 2.88 - 2.79 (m, 1H), 2.79 - 2.51 (m, 3H), 2.47 - 2.38 (m, 2H), 2.20 - 2.09 (m, 1H), 2.05 - 1.67 (m, 3H); LC-MS: [M+H] ⁺ = 438.10.

### Example 31: Synthesis of (2S)-N-(2-amino-6,7-dihydro-5H-cyclopenta[b]pyridin-5-yl)-1-((2R,4S)-4-(4-fluorobenzyl)pyrrolidine-2-carbonyl)pyrrolidine-2-carboxamide (Compound L031) Hydrochloride

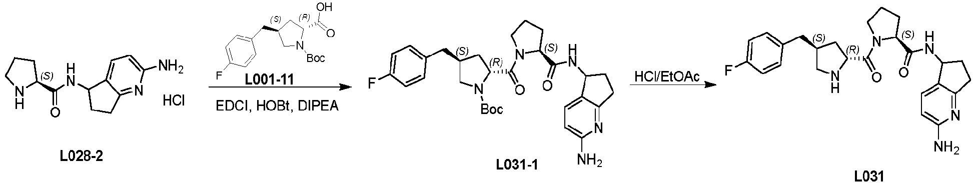

### 31.1: Synthesis of compound L031-1

L001-11 (150 mg, 0.46 mmol) was dissolved in N,N-dimethylformamide (3 mL). 1-Hydroxybenzotriazole (75 mg, 0.56 mmol) and 1-(3-dimethylaminopropyl)-3-ethylcarbodiimide hydrochloride (107 mg, 0.56 mmol) were added. The reaction liquid was stirred at room temperature for 10 min. A solution of L028-2 (171 mg, 0.60 mmol) and N,N-diisopropylethylamine (299 mg, 2.32 mmol) in N,N-dimethylformamide (2 mL) was added. The reaction liquid was stirred at room temperature for another 0.5 h. The reaction liquid was poured into water (10 mL), and the mixture was extracted with ethyl acetate (15 mL × 3). The combined organic phases were washed with a saturated aqueous sodium chloride solution (10 mL × 3), dried over anhydrous sodium sulfate and filtered, and the filtrate was concentrated under reduced pressure to give compound L031-1 (200 mg, yield: 78.16%). LCMS: [M+H]⁺ = 552.10.

### 31.2: Synthesis of compound L031 hydrochloride

L031-1 (200 mg, 0.36 mmol) was dissolved in dichloromethane (3 mL), and a solution of hydrogen chloride in ethyl acetate (2 mL, 4 M) was added. The reaction liquid was stirred at room temperature for 1 h. The reaction liquid was concentrated under reduced pressure, and the residue was purified by preparative high performance liquid chromatography (acidic condition) and lyophilized to give compound L031 hydrochloride (45.7 mg, yield: 25.83%).

¹H NMR (400 MHz, DMSO-*d₆*): δ 10.37-10.35 (m, 1H), 8.88 - 8, 51 (m, 2H), 8.03 (s, 2H), 7.79 - 7.49 (m, 1H), 7.31 - 7.24 (m, 2H), 7.15 - 7.08 (m, 2H), 6.85 - 6.78 (m, 1H), 5.23 - 4.99 (m, 1H), 4.61 - 4.46 (m, 1H), 4.23 (td, *J =* 8.0, 4.0 Hz, 1H), 3.76 - 3.64 (m, 1H), 3.53 - 3.37 (m, 2H), 3.07 - 2.99 (m, 1H), 2.95 - 2.83 (m, 2H), 2.79 - 2.55 (m, 2H), 2.47 - 2.31 (m, 2H), 2.24 - 1.77 (m, 7H); LC-MS: [M+H] ⁺ = 452.10.

### Example 32: Synthesis of (2S)-N-(2-amino-6,7-dihydro-5H-cyclopenta[b]pyridin-5-yl)-1-((2R,4S)-4-(4-fluorobenzyl)pyrrolidine-2-carbonyl)piperidine-2-carboxamide (Compound L032) Hydrochloride

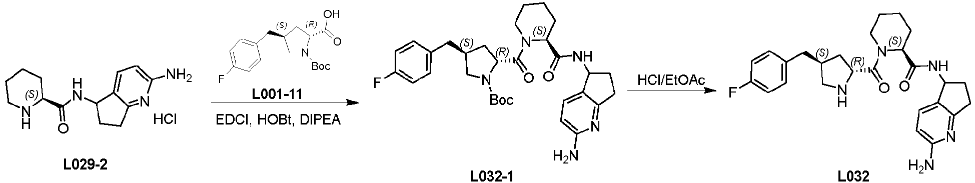

### 32.1: Synthesis of compound L032-1

L001-11 (150 mg, 0.46 mmol) was dissolved in N,N-dimethylformamide (3 mL). 1-Hydroxybenzotriazole (75 mg, 0.56 mmol) and 1-(3-dimethylaminopropyl)-3-ethylcarbodiimide hydrochloride (107 mg, 0.56 mmol) were added. The reaction liquid was stirred at room temperature for 10 min. A solution of L029-2 (179 mg, 0.60 mmol) and N,N-diisopropylethylamine (299 mg, 2.32 mmol) in N,N-dimethylformamide (2 mL) was added. The reaction liquid was stirred at room temperature for another 0.5 h. The reaction liquid was poured into water (10 mL), and the mixture was extracted with ethyl acetate (15 mL × 3). The combined organic phases were washed with a saturated aqueous sodium chloride solution (10 mL × 3), dried over anhydrous sodium sulfate and filtered, and the filtrate was concentrated under reduced pressure to give compound L032-1 (160 mg, crude product), which was used directly in the next step. LCMS: [M+H]⁺ = 566.10.

### 32.2: Synthesis of compound L032 hydrochloride

L032-1 (160 mg, 0.28 mmol) was dissolved in dichloromethane (3 mL), and a solution of hydrogen chloride in ethyl acetate (2 mL, 4 M) was added. The reaction liquid was stirred at room temperature for 1 h. The reaction liquid was concentrated under reduced pressure, and the residue was purified by preparative high performance liquid chromatography (acidic condition) and lyophilized to give compound L032 hydrochloride (29.0 mg, yield: 27.47%) as a white solid.

¹H NMR (400 MHz, DMSO-*d₆*): δ 10.41 (s, 1H), 8.62 - 8.44 (m, 2H), 8.05 (s, 2H), 7.72 - 7.56 (m, 1H), 7.35 - 7.24 (m, 2H), 7.17 - 7.06 (m, 2H), 6.86 - 6.79 (m, 1H), 5.27 - 5.09 (m, 1H), 4.91 - 4.65 (m, 2H), 4.33 - 3.65 (m, 1H), 3.38 - 2.79 (m, 5H), 2.76 - 2.54 (m, 2H), 2.49 - 2.40 (m, 2H), 2.38 - 2.12 (m, 2H), 2.07 - 1.56 (m, 5H), 1.55 - 1.20 (m, 3H); LC-MS: [M+H] ⁺ = 466.10.

### Example 33: Synthesis of (2R,4S)-N-((S)-1-(((R)-2-amino-6,7-dihydro-5H-cyclopenta[b]pyridin-5-yl)amino)-1 -oxopropan-2-yl)-4-(4-fluorobenzyl)pyrrolidine-2-carboxamide (Compound L001-IS-01)

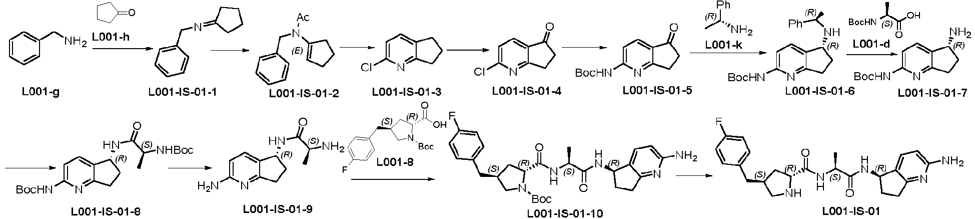

### 33.1: Synthesis of compound L001-IS-01-1

L001-g (500.0 g, 4.67 mol) and L001-h (393.0 g, 4.67 mol) were dissolved in toluene (2750 mL), and anhydrous magnesium sulfate (589.5 g, 5.0 mol) was added at room temperature. The reaction liquid was warmed to 30 °C and stirred for 16 h. The reaction liquid was directly filtered, and the filter cake was washed with toluene (500 mL). The filtrates were combined to give a solution of compound L001-IS-01-1 in toluene, which was used directly in the next step. LC-MS: [M+H]⁺ = 174.10.

### 33.2: Synthesis of compound L001-IS-01-2

The solution of L001-IS-01-1 in toluene was added to a 10 L reactor. Triethylamine (491 g, 4.85 mol) was added slowly at 0 °C with the temperature controlled within 5 °C, and acetic anhydride (485 g, 4.85 mol) was then added dropwise. The reaction liquid was warmed to room temperature and stirred for 16 h. The reaction liquid was added with water (3 L) and stirred for 10 min. The organic phase was then separated, washed with brine (2 L), dried over anhydrous sodium sulfate and filtered, and the filtrate was concentrated under reduced pressure. The residue was purified by silica gel column chromatography (petroleum ether/ethyl acetate = 20/1) to give compound L001-IS-01-2 (580 g). LC-MS: [M+H]⁺ = 216.10.

### 33.3: Synthesis of compound L001-IS-01-3

At room temperature, L001-IS-01-2 (550 g, 2.6 mol) was dissolved in N,N-dimethylformamide (2.2 L) and then added to a 10 L reactor. Phosphorus oxychloride (980 g, 6.4 mol) was added slowly, and the reaction liquid was warmed to 30 °C and stirred for 1.5 h. The reaction liquid was then warmed to 60 °C and stirred for 1 h, warmed to 70 °C and stirred for 1 h, warmed to 80 °C and stirred for 1 h, and warmed to 95 °C and stirred for 16 h. The reaction liquid was cooled to room temperature and added with water (2.2 L) and a sodium hydroxide solution (1.8 L, 30% aq.), so that the solution was adjusted to pH = 9. After methyl tert-butyl ether (4.8 L) was added, the mixture was stirred for 0.5 h. The organic phase was then separated, washed with water (2.2 L × 2), dried over anhydrous sodium sulfate and filtered, and the filtrate was concentrated under reduced pressure to give a crude product. The crude product was dissolved in n-heptane (2.2 L). Water (2.2 L) and concentrated hydrochloric acid (1.1 L, 36%) were then added. The organic phase was separated, and the aqueous phase was extracted once with n-heptane (2.2 L). The organic phase was discarded, and the aqueous phase retained. The aqueous phase was adjusted to pH = 9 with a sodium hydroxide solution (30% aq.) (the temperature of the system was controlled below 25 °C), and then stirred at room temperature for 1 h. A solid was precipitated. The mixture was filtered, and the filter cake was washed with water (500 mL). The filter cake was dried under reduced pressure to give a crude product. The crude product was added to n-heptane (4.4 L), and the mixture was warmed to 80 °C. After the mixture was refluxed for 2 h, the mixture was cooled to 50 °C and filtered while hot to remove a small amount of the insoluble substance. The filtrate was concentrated under reduced pressure to give compound L001-IS-01-3 (224.3 g, yield: 66.9%). LC-MS: [M+H]⁺ = 154.10.

### 33.4: Synthesis of compound L001-IS-01-4

L001-IS-01-3 (50 g, 325.5 mmol), anhydrous magnesium sulfate (78 g, 325.5 mmol) and tert-butanol (1 L) were added to a 10 L reactor at room temperature. The reaction liquid was cooled to 0 °C. A solution of potassium permanganate (103 g, 325.5 mmol) in water (2.8 L) was added dropwise to the reaction liquid, and the temperature of the system was controlled within 15 °C. After the dropwise addition was completed, the reaction liquid was warmed to room temperature and stirred for 4 h. The reaction liquid was added with water (1 L) and ethyl acetate (2 L), and then stirred at room temperature for 0.5 h. The organic phase was separated out, and the aqueous phase was extracted with ethyl acetate (1.5 L × 2). The organic phases were combined and washed with brine (2 L). The organic phase was dried over anhydrous sodium sulfate and filtered, and the filtrate was concentrated under reduced pressure. The residue was purified by silica gel column chromatography (petroleum ether/ethyl acetate = 3/1) to give compound L001-IS-01-4 (30.3 g, yield: 55.6%). LC-MS: [M+H]⁺ = 168.05.

### 33.5: Synthesis of compound L001-IS-01-5

L001-IS-01-4 (40 g, 0.24 mol), tert-butyl carbamate (31 g, 0.26 mol), cesium carbonate (156 g, 0.48 mol), palladium(II) acetate (2.2 g, 9.6 mmol) and 2-dicyclohexylphosphine-2',4',6'-triisopropylbiphenyl (9.1 g, 19.2 mmol) were added to 1,4-dioxane (400 mL) at room temperature. The reaction liquid was stirred at 85 °C for 3 h under nitrogen atmosphere. The reaction liquid was cooled to room temperature, added with ethyl acetate (200 mL), stirred for 10 min, and filtered through celite, and the filter cake was washed with ethyl acetate (200 mL). The filtrate was directly concentrated. The residue was stirred in ethanol (100 mL) for 3 h and filtered. After the filter cake was dried, petroleum ether (200 mL) and methyl tert-butyl ether (200 mL) were added again, and the mixture was stirred for another 3 h and filtered. The filter cake was dried to give compound L001-IS-01-5 (36 g, yield: 55.6%). LC-MS: [M+H]⁺ = 249.10.

### 33.6: Synthesis of compound L001-IS-01-6

At room temperature, L001-IS-01-5 (62 g, 0.25 mol) and L001-k (32 g, 0.26 mol) were added to N,N-dimethylformamide (600 mL), acetic acid (7.5 g, 0.125 mol) was added, and the reaction liquid was stirred at room temperature for 2 h. Sodium borohydride acetate (159 g, 0.75 mol) was added to the above reaction system in 3-4 batches (1 h/time). After the addition was completed, the reaction liquid was stirred at room temperature for 16 h. The reaction liquid was quenched with saturated ammonium chloride (500 mL), and the solution was adjusted to pH = 1 with diluted hydrochloric acid (1 M). The solution was extracted once with ethyl acetate (600 mL). The organic phase was discarded, and the aqueous phase was adjusted to pH = 8-9 with a saturated sodium carbonate solution and extracted with ethyl acetate (600 mL × 2). The combined organic phases were washed with a saturated aqueous sodium chloride solution (800 mL × 3), dried over anhydrous sodium sulfate and filtered, and the filtrate was concentrated under reduced pressure to give compound L001-IS-01-6 (75 g, yield: 85%). LC-MS: [M+H]⁺ = 354.05.

### 33.7: Synthesis of compound L001-IS-01-7

At room temperature, L001-IS-01-6 (75 g, 0.21 mol) and L001-d (48 g, 0.25 mol) were added to methanol (500 mL), palladium on carbon (7.5 g, 10% wt) was then added, and the reaction liquid was stirred for 20 h under hydrogen atmosphere. The reaction liquid was filtered through celite, and the filtrate was concentrated under reduced pressure to give compound L001-IS-01-7 in the form of carboxylate of L001-d, which may used directly in the next step. LC-MS: [M+H]⁺ = 250.10.

### 33.8: Synthesis of compound L001-IS-01-8

At room temperature, the carboxylate of L001-d of L001-IS-01-7 obtained in the previous step was added to dichloromethane (500 mL), and N,N-diisopropylethylamine (81 g, 0.63 mol) was added. 1-Propylphosphoric anhydride (203 g, 0.32 mol, 50% in ethyl acetate) was added at 0 °C. The reaction liquid was stirred at room temperature for 2 h. The reaction liquid was added with water (500 mL) and stirred for dilution and extracted with dichloromethane (300 mL × 2). The organic phases were combined, washed with diluted hydrochloric acid (400 mL × 2, 0.2 M), dried over anhydrous sodium sulfate and filtered, and the filtrate was concentrated under reduced pressure. The residue was purified by silica gel column chromatography to give compound L001-IS-01-8 (71 g, yield: 80.5%, chiral purity: 85%). L001-IS-01-8 (71 g) was dissolved in isopropanol (142 mL), and then the solution was cooled to 0 °C. Purified water (142 mL) was added dropwise while stirring. After the dropwise addition was completed, the solution was stirred at 0 °C for another 2 h, and a large amount of white solid was precipitated and filtered. The obtained solid was dried under reduced pressure to give compound L001-IS-01-8 (36.5 g, chiral purity: 98.2%, yield: 51.4%). LC-MS: [M+H]⁺ = 421.05.

### 33.9: Synthesis of compound L001-IS-01-9

L001-IS-01-8 (32 g, 76.2 mmol) was dissolved in dichloromethane (200 mL), and a solution of hydrogen chloride in 1,4-dioxane (200 mL, 4 M) was added at 0 °C. The reaction liquid was warmed to room temperature and stirred for 16 h. The reaction liquid was concentrated under reduced pressure to give compound L001-IS-01-9 hydrochloride (22 g). LC-MS: [M+H]⁺ = 221.10.

### 33.10: Synthesis of compound L001-IS-01-10

The L001-IS-01-9 hydrochloride (3.8 g, 14.84 mmol) and diisopropylethylamine (8.0 g, 61.85 mmol) were dissolved in dichloromethane (50 mL) at room temperature. The reaction liquid was stirred at room temperature for 30 min and added to a solution of L001-8 (4 g, 12.37 mmol) and 1-propylphosphoric anhydride (15.7 g, 24.74 mmol, 50% in ethyl acetate) in dichloromethane (50 mL) at 0 °C. The reaction liquid was stirred at room temperature for another 1 h. The reaction liquid was poured into a saturated aqueous sodium chloride solution (200 mL) and extracted with dichloromethane (200 mL × 3). The organic phases were combined, dried over anhydrous sodium sulfate and filtered, and the filtrate was concentrated under reduced pressure. The residue was added with a mixed solution of petroleum ether (20 mL) and ethyl acetate (10 mL), stirred for 1 h, and filtered, and the filter cake was dried under reduced pressure to give compound L001-IS-01-10 (6.15 g, yield: 98%). LC-MS: [M+H]⁺ = 526.05.

### 33.11: Synthesis of compound L001-IS-01

At room temperature, L001-IS-01-10 (32 g, 60.88 mmol) was dissolved in dichloromethane (300 mL), and a solution of hydrogen chloride in 1,4-dioxane (200 mL, 4 M) was added. The reaction liquid was stirred at room temperature for 2 h. The reaction liquid was concentrated under reduced pressure, and the residue was purified by high performance liquid chromatography (0.1% hydrochloric acid condition) and then lyophilized to give compound L001-IS-01 hydrochloride. The hydrochloride was dissolved in deionized water (200 mL), added with a strongly basic deionized resin (32 g), stirred and freed. A white solid was precipitated. The mixture was filtered, and the filter cake was washed with deionized water (100 mL). The obtained solid mixture was added to acetonitrile (200 mL), stirred for 0.5 h and filtered, and the filtrate was concentrated under reduced pressure to give a crude product. The crude product was added to ethyl acetate (100 mL), stirred for 0.5 h and filtered, and the filter cake was dried to give compound L001-IS-01 (15.563 g, yield: 60.09%).

¹H NMR (400 MHz, DMSO-*d₆*): δ 8.16 (d, *J* = 8.0 Hz, 2H), 7.22 - 7.18 (m, 2H), 7.16 - 7.04 (m, 3H), 6.23 (d, *J=* 8.4 Hz, 1H), 5.85 (s, 2H), 5.07 (dd, *J =* 14.4, 7.6 Hz, 1H), 4.27 - 4.15 (m, 1H), 3.62 (dd, *J* = 9.2, 4.8 Hz, 1H), 2.85 (dd, *J* = 10.0, 6.4 Hz, 1H), 2.77 - 2.69 (m, 1H), 2.66 - 2.52 (m, 4H), 2.37 - 2.26 (m, 1H), 2.16 - 2.07 (m, 1H), 1.78 - 1.61 (m, 3H), 1.19 (d, *J* = 6.8 Hz, 3H). LC-MS: [M+H]⁺ = 426.00.

### Example 34: Synthesis of (2R,4S)-N-((S)-1-(((R)-2-amino-6,7-dihydro-5H-cyclopenta[b]pyridin-5-yl)amino)-1-oxopropan-2-yl)-4-(4-chlorobenzyl)pyrrolidine-2-carboxamide (Compound L003-IS-01)

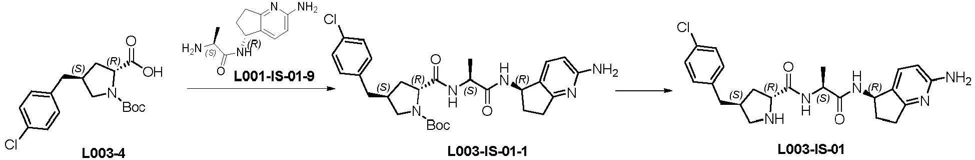

For the synthesis of compound L003-IS-01, reference was made to the synthesis method of compound L001-IS-01, and L001-8 was replaced with L003-4; the crude product obtained by reaction was purified by high performance liquid chromatography (0.1% hydrochloric acid condition) and normal phase chromatography (basic condition) to give compound L003-IS-01 (97.2 mg, yield: 26.55%).

¹H NMR (400 MHz, DMSO-*d₆*): δ 8.18 - 8.15 (m, 2H), 7.33 (d, *J =* 8.0 Hz, 2H), 7.21 (d, *J =* 8.0 Hz, 2H), 7.14 (d, *J* = 8.0 Hz, 1H), 6.23 (d, *J* = 8.0 Hz, 1H), 5.84 - 5.83 (m, 2H), 5.07 (dd, *J =* 12.0, 8.0 Hz, 1H), 4.24 - 4.17 (m, 1H), 3.64 (dd, *J =* 8.0, 4.0 Hz, 1H), 2.88 - 2.84 (m, 1H), 2.77 - 2.69 (m, 1H), 2.67 - 2.52 (m, 4H), 2.36 - 2.27 (m, 1H), 2.19 - 2.07 (m, 1H), 1.78 - 1.61 (m, 3H), 1.19 (d, *J =* 4.0 Hz, 3H); LC-MS [M+H]⁺ = 442.00.

### Example 35: Synthesis of (2R,4S)-N-((S)-1-(((R)-2-amino-6,7-dihydro-5H-cyclopenta[b]pyridin-5-yl)amino)-1-oxopropan-2-yl)-4-phenylpyrrolidine-2-carboxamide (Compound L006-IS-03)

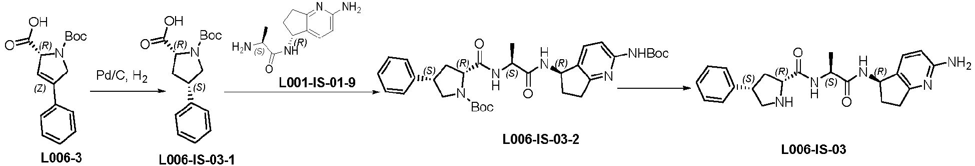

### 35.1: Synthesis of compound L006-IS-03-1

At room temperature, L006-3 (440 mg, 1.52 mmol) was dissolved in methanol (10 mL), palladium on carbon (88 mg, 10% wt) was added, and then the reaction liquid was stirred for 2 h under hydrogen atmosphere. The reaction liquid was filtered through celite, and the filter cake was washed with methanol (10 mL). The filtrate was concentrated under reduced pressure to give compound L006-IS-03-1 (360 mg, yield: 81.4%). LC-MS: [M-tBu+H]⁺ = 236.05.

### 35.2: Synthesis of compound L006-IS-03

For the synthesis of compound L006-IS-03, reference was made to the synthesis method of compound L001-IS-01, and L001-8 was replaced with L006-IS-03-1; the crude product obtained by reaction was purified by high performance liquid chromatography (0.1% aqueous ammonia condition) and then lyophilized to give compound L006-IS-03 (184.1 mg).

¹H NMR (400 MHz, DMSO-*d₆*): δ 8.25 - 8.21 (m, 2H), 7.29 - 7.15 (m, 6H), 6.24 - 6.20 (m, 1H), 5.84 (s, 2H), 5.13 - 5.07 (m, 1H), 4.30 - 4.23 (m, 1H), 3.79 - 3.75 (m, 1H), 3.32 - 3.27 (m, 2H), 3.20 - 3.07 (m, 1H), 2.80 - 2.67 (m, 1H), 2.68 - 2.56 (m, 2H), 2.40 - 2.28 (m, 1H), 1.75 - 1.65 (m, 2H), 1.24 (d, *J* = 4.0 Hz, 3H); LC-MS [M+H]⁺ = 394.05.

### Example 36: Synthesis of (2R,4S)-N-((S)-1-(((R)-2-amino-6,7-dihydro-5H-cyclopenta[b]pyridin-5-yl)amino)-1-oxopropan-2-yl)-4-(3-chloro-4-fluorobenzyl)pyrrolidine-2-carboxamide (Compound L012-IS-01) Hydrochloride

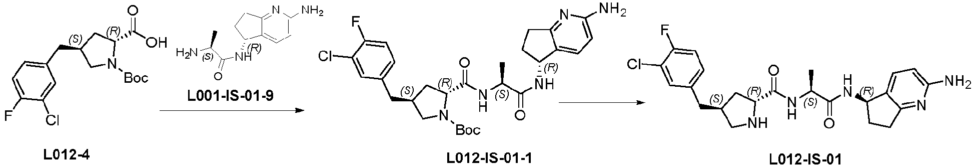

For the synthesis of compound L012-IS-01 hydrochloride, reference was made to the synthesis method of compound L001-IS-01, and L001-8 was replaced with L012-4; the crude product obtained by reaction was purified by high performance liquid chromatography (0.1% hydrochloric acid condition) and then lyophilized to give compound L012-IS-01 hydrochloride (61 mg, yield: 40.6%).

¹H NMR (400 MHz, DMSO-*d₆*): δ 10.47 (s, 1H), 8.86 (d, *J =* 7.2 Hz, 1H), 8.56 -8.53 (m, 2H), 8.04 (s, 1H), 7.71 (d, *J* = 8.8 Hz, 1H), 7.53 (dd, *J* = 7.2, 2.4 Hz, 1H), 7.39 - 7.26 (m, 2H), 6.84 (d, *J* = 8.8 Hz, 1H), 5.18 - 5.14 (m, 1H), 4.40 - 4.26 (m, 1H), 4.24 (p, *J* = 7.2 Hz, 1H), 3.33 - 3.24 (m, 1H), 3.04 (m, 1H), 2.91 (m, 2H), 2.72 (m, 2H), 2.52 (s, 1H), 2.44 (dd, *J* = 8.8, 4.4 Hz, 1H), 2.12 - 2.01 (m, 1H), 1.93 (m, 2H), 1.23 (d, *J =* 7.2 Hz, 3H); LC-MS [M+H]⁺ =460.00.

### Example 37: Synthesis of (2R,4S)-N-((S)-1-(((R)-2-amino-6,7-dihydro-5H-cyclopenta[b]pyridin-5-yl)amino)-1-oxopropan-2-yl)-4-(3-chlorobenzyl)pyrrolidine-2-carboxamide (Compound L014-IS-01) Hydrochloride

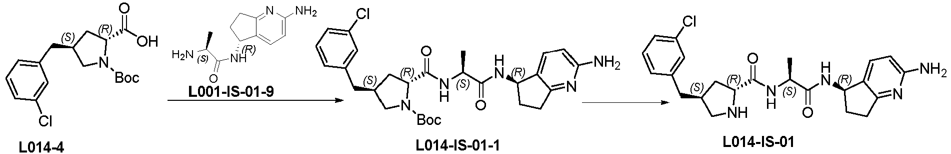

For the synthesis of compound L014-IS-01 hydrochloride, reference was made to the synthesis method of compound L001-IS-01, and L001-8 was replaced with L014-4; the crude product obtained by reaction was purified by high performance liquid chromatography (0.1% hydrochloric acid condition) and then lyophilized to give compound L014-IS-01 hydrochloride (311.2 mg, yield: 73.82%).

¹H NMR (400 MHz, DMSO-*d₆*): δ 10.35 (s, 1H), 8.85 (d, *J* = 7.2 Hz, 1H), 8.55 (t, *J* = 9.2 Hz, 2H), 8.01 (s, 2H), 7.70 (d, *J* = 8.8 Hz, 1H), 7.49 - 7.17 (m, 4H), 6.83 (d, *J* = 8.8 Hz, 1H), 5.15 (q, *J* = 7.6 Hz, 1H), 4.55 - 4.33 (m, 1H), 4.24 (p, *J* = 7.2 Hz, 1H), 3.28 (d, *J =* 4.4 Hz, 1H), 3.05 - 3.01 (m, 1H), 2.93 - 2.89 (m, 2H), 2.76 - 2.72 (m, 2H), 2.49 - 2.39 (m, 2H), 2.16 - 2.02 (m, 1H), 1.99 - 1.84 (m, 2H), 1.23 (d, *J =* 7.2 Hz, 3H); LC-MS [M+H]⁺ = 442.00.

### Example 38: Synthesis of (2R,4S)-N-((S)-1-(((R)-2-amino-6,7-dihydro-5H-cyclopenta[b]pyridin-5-yl)amino)-1-oxopropan-2-yl)-4-(3-methylbenzyl)pyrrolidine-2-carboxamide (Compound L016-IS-01) Hydrochloride

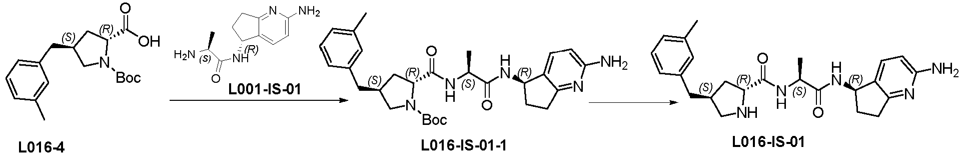

For the synthesis of compound L016-IS-01 hydrochloride, reference was made to the synthesis method of compound L001-IS-01, and L001-8 was replaced with L016-4; the crude product obtained by reaction was purified by high performance liquid chromatography (0.1% hydrochloric acid condition) and then lyophilized to give compound L016-IS-01 hydrochloride (140.4 mg, yield: 57.92%).

¹H NMR (400 MHz, DMSO-*d₆*): δ 10.39 (s, 1H), 8.87 (d, *J* = 7.2 Hz, 1H), 8.57 (d, *J* = 7.6 Hz, 1H), 8.52 (d, *J* = 4.8 Hz, 1H), 8.03 (s, 2H), 7.71 (d, *J* = 8.8Hz, 1H), 7.19 (t, *J=* 7.6 Hz, 1H), 7.06 - 7.00 (m, 3H), 6.83 (d, *J=* 8.8 Hz, 1H), 5.15 (d, *J=* 6.8 Hz, 1H), 4.39 - 4.34 (m, 1H), 4.26 - 4.20 (m, 2H), 3.27 (d, *J* = 4.0 Hz, 1H), 3.07 - 3.00 (m, 1H), 2.87-2.93 (m, 2H), 2.66 (t, *J* = 7.6 Hz, 2H), 2.48 - 2.44 (m, 1H), 2.28 (s, 3H), 2.03-2.10 (m, 1H), 1.89-1.95 (m, 2H), 1.23 (d, *J* = 7.2Hz, 3H); LC-MS [M+H]⁺ = 422.05.

### Example 39: Synthesis of (2R,4S)-N-((2S)-1-((2-amino-6,7-dihydro-5H-cyclopenta[b]pyridin-5-yl) amino)-1-oxopropan-2-yl)-4-phenylpiperidine-2-carboxamide (Compound L021-IS-0 1)

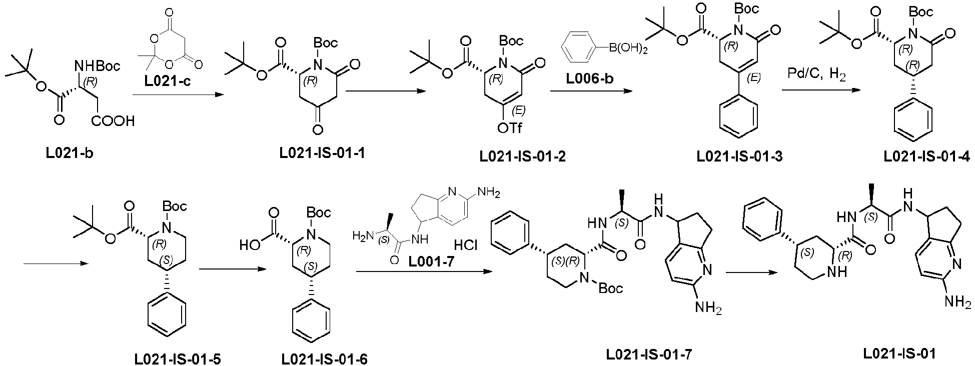

### 39.1: Synthesis of compound L021-IS-01-1

L021-b (25 g, 86.42 mmol) and L021-c (12.5 g, 86.42 mmol) were dissolved in dichloromethane (360 mL), and 1-(3-dimethylaminopropyl)-3-ethylcarbodiimide hydrochloride (24.8 g, 129.35 mmol) and 4-dimethylaminopyridine (15.8 g, 129.35 mmol) were added at 0 °C. The reaction liquid was stirred at 0 °C for 1 h, warmed to room temperature and stirred for another 2 h. The reaction liquid was washed with an aqueous potassium hydrosulfide solution (1 M) (100 mL × 4). The organic phase was dried over anhydrous sodium sulfate and filtered, and the filtrate was concentrated under reduced pressure. The residue was added with toluene (800 mL) and refluxed at 110 °C for 16 h. The reaction liquid was cooled to room temperature and concentrated under reduced pressure. The residue was added with toluene/petroleum ether (100 mL, V/V = 1/10) and stirred for 2 h. The solid was filtered, and the filter cake was dried to give compound L021-IS-01-1 (16 g, yield: 59.08%). LC-MS: [M-Boc+H]⁺ = 214.10.

### 39.2: Synthesis of compound L021-IS-01-2

L021-IS-01-1 (1.0 g, 3.19 mmol) and N,N-diisopropylethylamine (825 mg, 6.38 mmol) were dissolved in dichloromethane (10 mL), and trifluoromethanesulfonic anhydride (1.4 g, 4.80 mmol) was added dropwise at 0 °C. After the addition was completed, the reaction liquid was stirred at room temperature for 1 h. The reaction liquid was quenched by adding a saturated aqueous sodium bicarbonate solution (15 mL) and extracted with ethyl acetate (30 mL × 3). The combined organic phases were dried over anhydrous sodium sulfate and filtered, and the filtrate was concentrated under reduced pressure. The residue was purified by silica gel column chromatography to give compound L021-IS-01-2 (1.1 g, yield: 77.39%). LC-MS: [M-Boc+H]⁺ = 345.95.

### 39.3: Synthesis of compound L021-IS-01-3

L021-IS-01-2 (1 g, 2.33 mmol), L006-b (427 mg, 3.50 mmol), potassium carbonate (387 mg, 2.80 mmol) and bis(triphenylphosphine)palladium(II) dichloride (164 mg, 0.23 mmol) were added to tetrahydrofuran (8 mL) and water (1 mL). The reaction liquid was stirred at 40 °C for 2 h under nitrogen atmosphere. The reaction liquid was added with water (10 mL) for dilution and extracted with ethyl acetate (20 mL × 3). The combined organic phases were dried over anhydrous sodium sulfate and filtered, and the filtrate was concentrated under reduced pressure. The residue was purified by silica gel column chromatography to give compound L021-IS-01-3 (800 mg, yield: 95.41%). LC-MS: [M-Boc+H]⁺ = 274.10.

### 39.4: Synthesis of compound L021-IS-01-4

L021-IS-01-3 (800 mg, 2.14 mmol) and palladium on carbon (200 mg, 10% palladium content) were added to ethyl acetate (10 mL). The mixture was stirred at room temperature for 2 h under hydrogen atmosphere. After the mixture was filtered through celite, the filtrate was concentrated under reduced pressure, and the residue was purified by silica gel column chromatography to give compound L021-IS-01-4 (600 mg, yield: 74.6%). LC-MS: [M-Boc+H]⁺ = 276.10.

### 39.5: Synthesis of compound L021-IS-01-5

L021-IS-01-4 (950 mg, 2.53 mmol) was dissolved in tetrahydrofuran (5 mL), and then borane tetrahydrofuran coordination complex (7.6 mL, 7.6 mmol, 1 M in tetrahydrofuran) was added dropwise at 0 °C. After the addition was completed, the reaction liquid was stirred at room temperature for 3 h. The reaction liquid was quenched by adding methanol (15 mL) dropwise, stirred at room temperature for 1 h, and then concentrated under reduced pressure, and the residue was purified by silica gel column chromatography to give compound L021-IS-01-5 (550 mg, yield: 60.13%). LC-MS: [M+H]⁺ = 362.10.

### 39.6: Synthesis of compound L021-IS-01-6

Trifluoroacetic acid/dichloromethane (2 mL/1 mL) was added to L021-IS-01-5 (300 mg, 0.83 mmol) at 0 °C. The reaction liquid was warmed to room temperature and stirred for 1 h. The reaction liquid was concentrated under reduced pressure, and the residue was dissolved in dichloromethane (3 mL), and di-tert-butyl dicarbonate (217 mg, 1.0 mmol) and triethylamine (168 mg, 1.66 mmol) were added. The mixture was stirred at room temperature for 16 h. The reaction liquid was concentrated under reduced pressure, and the residue was purified by silica gel column chromatography to give compound L021-IS-01-6 (100 mg, yield: 39.46%). LC-MS: [M-Boc+H]⁺ = 206.10.

### 39.7: Synthesis of compound L021-IS-01-7

L021-IS-01-6 (80 mg, 0.26 mmol), 1-hydroxybenzotriazole (53 mg, 0.39 mmol) and 1-(3-dimethylaminopropyl)-3-ethylcarbodiimide hydrochloride (75 mg, 0.39 mmol) were dissolved in N,N-dimethylformamide (2 mL). The reaction liquid was stirred at room temperature for 0.5 h. L001-7 (71 mg, 0.27 mmol) and N,N-diisopropylethylamine (135 mg, 1.05 mmol) were dissolved in N,N-dimethylformamide (2 mL), and the solution was stirred at room temperature for 0.5 h. The latter solution was added to the former reaction liquid, and the reaction liquid was stirred for another 1 h. The reaction liquid was added with water (10 mL) for dilution and extracted with ethyl acetate (10 mL × 3). The combined organic phases were washed with saturated brine (20 mL), dried over anhydrous sodium sulfate and filtered, and the filtrate was concentrated under reduced pressure. The residue was purified by silica gel column chromatography to give compound L021-IS-01-7 (80 mg, yield: 60.16%). LC-MS: [M+H]⁺ = 508.15.

### 39.8: Synthesis of compound L021-IS-01

L021-IS-01-7 (80 mg, 0.16 mmol) was dissolved in dichloromethane (2 mL), and a solution of hydrogen chloride in ethyl acetate (2 mL, 4 M) was added. The reaction liquid was stirred at room temperature for 0.5 h. The reaction liquid was concentrated under reduced pressure, and the residue was purified by preparative high performance liquid chromatography (basic condition) and lyophilized to give compound L021-IS-01 (25.4 mg, yield: 39.55%).

¹H NMR (400 MHz, DMSO-*d₆*): δ 8.13 (d, *J =* 8.0 Hz, 1H), 7.72 (d, *J =* 7.6 Hz, 1H), 7.33 - 7.26 (m, 2H), 7.24 - 7.09 (m, 4H), 6.22 (d, *J* = 8.4 Hz, 1H), 5.83 (d, *J* = 3.6 Hz, 2H), 5.09 (q, *J* = 7.6 Hz, 1H), 4.30 - 4.19 (m, 1H), 3.19 (d, *J* = 11.2 Hz, 1H), 3.10 (d, *J* = 12.4 Hz, 1H), 2.74 - 2.59 (m, 4H), 2.38 - 2.27 (m, 1H), 1.94 (d, *J* = 12.4 Hz, 1H), 1.70 (d, *J* = 12.0 Hz, 2H), 1.54 - 1.41 (m, 1H), 1.39 - 1.27 (m, 1H), 1.24 - 1.20 (m, 3H). LC-MS: [M+H]⁺ = 408.10.

### Example 40: Synthesis of (2R,4R)-N-((2S)-1-((2-amino-6,7-dihydro-5H-cyclopenta[b]pyridin-5-yl) amino)-1-oxopropan-2-yl)-4-phenylpiperidine-2-carboxamide (Compound L021-IS-02)

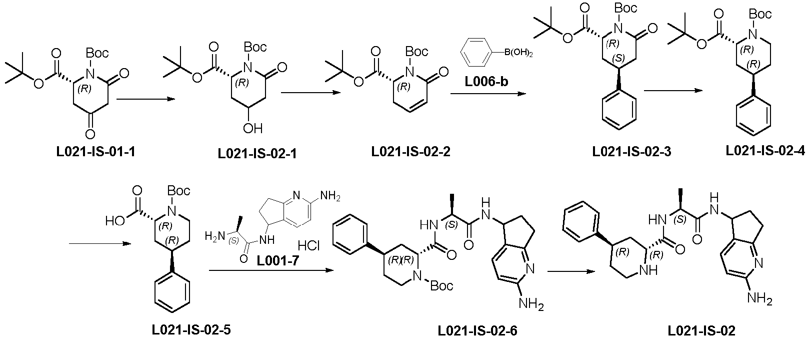

### 40.1: Synthesis of compound L021-IS-02-1

L021-IS-01-1 (5 g, 16 mmol) was dissolved in dichloromethane (40 mL) and acetic acid (4 mL), and then sodium borohydride (607 mg, 16 mmol) was added in batches at 0 °C. The reaction liquid was stirred at 0 °C for 0.5 h, warmed to room temperature and stirred for 48 h. The reaction liquid was quenched by adding a saturated aqueous ammonium chloride solution (30 mL) and extracted with dichloromethane (40 mL × 3). The combined organic phases were dried over anhydrous sodium sulfate and filtered, and the filtrate was concentrated under reduced pressure. The residue was purified by silica gel column chromatography to give compound L021-IS-02-1 (3 g, yield: 59.62%). LC-MS: [M-Boc+H]⁺ = 216.15.

### 40.2: Synthesis of compound L021-IS-02-2

L021-IS-02-1 (3 g, 9.5 mmol) was dissolved in toluene (20 mL), and triethylamine (2.9 g, 28.5 mmol) was added. Methylsulfonyl chloride (1.6 g, 14.3 mmol) was added dropwise at 0 °C. The temperature was controlled to be not exceeding 5 °C. After the addition was completed, the reaction liquid was stirred at 0 °C for 0.5 h, warmed to room temperature and stirred for 1.5 h. The reaction liquid was quenched by adding a saturated sodium bicarbonate solution (15 mL), and extracted with ethyl acetate (20 mL × 3). The combined organic phases were dried over anhydrous sodium sulfate and filtered, and the filtrate was concentrated under reduced pressure. The residue was purified by silica gel column chromatography to give compound L021-IS-02-2 (1.7 g, yield: 60.10%). LC-MS: [M-Boc+H]⁺ = 198.15.

### 40.3: Synthesis of compound L021-IS-02-3

(1,5-Cyclooctadiene)chlororhodium (I) dimer (83 mg, 0.17 mmol) and (S)-1,1'-binaphthyl-2,2'-bisdiphenylphosphine (105 mg, 0.17 mmol) were added to a reaction flask at room temperature. The mixture was purged with nitrogen, added with toluene (5 mL) and purged with nitrogen for 0.5 h. L021-IS-02-2 (500 mg, 1.68 mmol), L006-b (307 mg, 2.52 mmol) and triethylamine (340 mg, 3.36 mmol) were dissolved in toluene (4 mL) and water (1 mL) at room temperature, and then the reaction liquid was added to the reaction flask described above containing the catalyst and stirred at 50 °C for 1.5 h under nitrogen atmosphere. The reaction liquid was added with water (10 mL) for dilution and extracted with ethyl acetate (20 mL × 3). The combined organic phases were washed with saturated brine (10 mL), dried over anhydrous sodium sulfate and filtered, and the filtrate was concentrated under reduced pressure. The residue was purified by silica gel column chromatography to give compound L021-IS-02-3 (500 mg, yield: 79.20%) as a yellow oil. LC-MS: [M+H]⁺ = 376.10.

### 40.4: Synthesis of compound L021-IS-02

For the synthesis of compound L021-IS-02, reference was made to the synthesis method of compound L021-IS-01, and L021-IS-01-4 was replaced with L021-IS-02-3; the crude product obtained by reaction was purified by high performance liquid chromatography (basic condition) and then lyophilized to give compound L021-IS-02 (71.8 mg. yield: 44.72%).

¹H NMR (400 MHz, DMSO-*d₆*): δ 8.27 - 8.06 (m, 2H), 7.34 - 7.26 (m, 2H), 7.24 - 7.12 (m, 4H), 6.23 (t, *J* = 7.6 Hz, 1H), 5.84 (s, 2H), 5.17 - 5.07 (m, 1H), 4.42 - 4.29 (m, 1H), 3.54 (d, *J* = 4.0 Hz, 1H), 2.95 - 2.57 (m, 5H), 2.42 - 2.32 (m, 1H), 2.29 (d, *J =* 13.6 Hz, 1H), 1.80 - 1.68 (m, 1H), 1.63 - 1.49 (m, 2H), 1.49 - 1.36 (m, 1H), 1.28 - 1.20 (m, 4H). LC-MS: [M+H]⁺ = 408.10.

### Example 41: Synthesis of (2R,4R)-N-((2S)-1-((2-amino-6,7-dihydro-5H-cyclopenta[b]pyridin-5-yl) amino)-1-oxopropan-2-yl)-4-(4-fluorophenyl)piperidine-2-carboxamide (Compound L033-IS-01) Hydrochloride

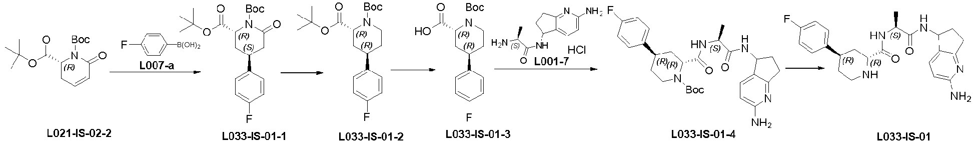

For the synthesis of compound L033-IS-01 hydrochloride, reference was made to the synthesis method of compound L021-IS-02, and L006-b was replaced with L007-a; the crude product obtained by reaction was purified by high performance liquid chromatography (acidic condition) to give compound L033-IS-01 hydrochloride (50.2 mg).

¹H NMR (400 MHz, DMSO-*d₆*): δ 9.93 (m, 1H), 9.04 (s, 1H), 8.87 - 8.84 (m, 1H), 8.57 - 8.53 (m, 1H), 8.02 (s, 1H), 7.72-7.65 (m, 1H), 7.36 - 7.24 (m, 2H), 7.17 (t, *J =* 8.8 Hz, 2H), 6.83 (d, *J =* 8.0 Hz, 1H), 5.17 - 5.16 (m, 1H), 4.40 - 4.14 (m, 2H), 3.41 - 3.39 (m, 1H), 3.15 - 3.03 (m, 2H), 2.98 - 2.77 (m, 2H), 2.48 - 2.38 (m, 1H), 2.36 - 2.13 (m, 2H), 2.07 - 1.85 (m, 3H), 1.30 - 1.23 (m, 4H). LC-MS: [M+H]⁺ =426.15.

### Example 42: Synthesis of (2R,4S)-N-((2S)-1-((2-amino-6,7-dihydro-5H-cyclopenta[b]pyridin-5-yl) amino)-1-oxopropan-2-yl)-4-(4-fluorophenyl)piperidine-2-carboxamide (Compound L033-IS-02) Hydrochloride

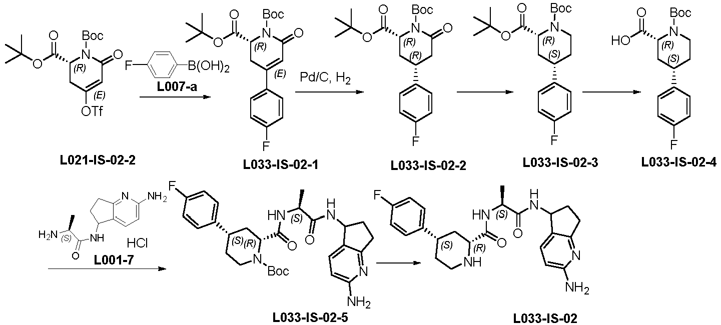

For the synthesis of compound L033-IS-02 hydrochloride, reference was made to the synthesis method of compound L021-IS-01, and L006-b was replaced with L007-a; the crude product obtained by reaction was purified by high performance liquid chromatography (acidic condition) to give compound L033-IS-02 hydrochloride (37.3 mg, yield: 32.5%).

¹H NMR (400 MHz, DMSO-*d₆*): δ 9.61 - 9.46(m, 1H), 8.81 - 8.80 (m, 2H), 8.54 - 8.50 (m, 1H), 7.98 (s, 1H), 7.73 - 7.65 (m, 1H), 7.31 - 7.22 (m, 2H), 7.19 - 7.15 (m, 2H), 6.82 (t, *J =* 8.0 Hz, 1H), 5.17 - 5.16 (m, 1H), 4.34 - 4.16 (m, 1H), 3.93 (s, 1H), 3.33 - 3.28 (m, 1H), 3.14 - 3.00 (m, 2H), 2.95 - 2.88 (m, 2H), 2.46 - 2.44 (m, 1H), 2.30 (d, *J =* 12.0 Hz, 1H), 2.02 - 1.81 (m, 3H), 1.71 - 1.62 (m, 1H), 1.24 - 1.22 (m, 4H). LC-MS: [M+H]⁺ =426.05.

### Example 43: Synthesis of (2R,4S)-N-((2S)-1-((5-amino-2,3-dihydro-1H-inden-1-yl)amino)-1-oxopropan-2-yl)-4-(4-fluorobenzyl)pyrrolidine-2-carboxamide (Compound L034)

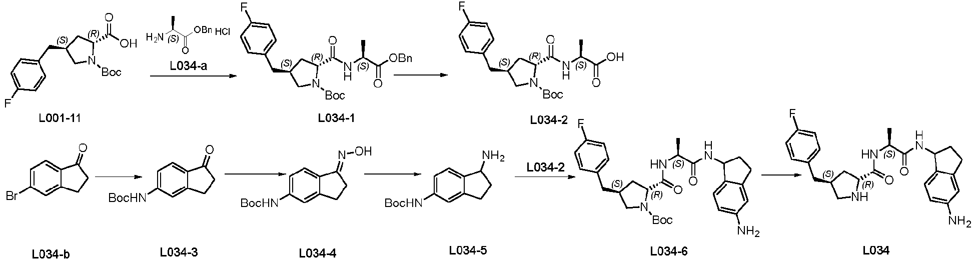

### 43.1: Synthesis of compound L034-1

L001-11 (500 mg, 1.55 mmol), 1-hydroxybenzotriazole (231 mg, 1.71 mmol) and 1-(3-dimethylaminopropyl)-3-ethylcarbodiimide hydrochloride (328 mg, 1.71 mmol) were dissolved in N,N-dimethylformamide (3 mL). The reaction liquid was reacted at room temperature for 0.5 h. Meanwhile, L034-a (400 mg, 1.86 mmol) and N,N-diisopropylethylamine (800 mg, 6.2 mmol) were dissolved in N,N-dimethylformamide (2 mL), and the reaction liquid was reacted at room temperature for 0.5 h. The latter reaction liquid was added to the former reaction liquid, and the reaction liquid was reacted for another 0.5 h. The reaction liquid was added with water (20 mL) for dilution and extracted with ethyl acetate (30 mL × 3). The organic phases were combined, washed with saturated brine (40 mL), dried over anhydrous sodium sulfate and filtered, and the filtrate was concentrated under reduced pressure to give compound L034-1 (750 mg), which was used directly in the next step. LC-MS: [M+H]⁺ = 485.05.

### 43.2: Synthesis of compound L034-2

L034-1 (750 mg, 1.55 mmol) and palladium on carbon (75 mg, 10% wt) were added to methanol (8 mL). The reaction liquid was reacted for 2 h under hydrogen atmosphere. The reaction liquid was filtered through celite, and the filtrate was concentrated under reduced pressure to give compound L034-2 (520 mg), which could be used directly in the next step. LC-MS: [M+H]⁺ = 395.05.

### 43.3: Synthesis of compound L034-3

L034-b (2 g, 9.5 mmol) and tert-butyl carbamate (2.2 g, 19.0 mmol) were dissolved in 1,4-dioxane (20 mL) at room temperature, and then palladium(II) acetate (213 mg, 0.95 mmol), 4,5-bis(diphenylphosphino)-9,9-dimethylxanthene (549 mg, 0.95 mmol) and cesium carbonate (6.2 g, 19.0 mmol) were added. The reaction liquid was purged with nitrogen 3 times, warmed to 100 °C and reacted for 16 h. The reaction liquid was cooled to room temperature, added with water (20 mL) for dilution, and extracted with ethyl acetate (40 mL × 3). The organic phase was dried over anhydrous sodium sulfate and filtered, and the filtrate was concentrated under reduced pressure. The residue was purified by silica gel column chromatography to give compound L034-3 (2 g, yield: 85.5%). LCMS: [M+H]⁺ = 248.10.

### 43.4 Synthesis of compound L034-4

L034-3 (500 mg, 2.02 mmol) was dissolved in methanol (6 mL) and water (3 mL), and then hydroxylamine hydrochloride (281 mg, 4.04 mmol) and potassium carbonate (419 mg, 3.03 mmol) were added. The reaction liquid was reacted at room temperature for 16 h. The reaction liquid was added with water (10 mL) for dilution and extracted with ethyl acetate (20 mL × 3). The organic phase was dried over anhydrous sodium sulfate and filtered, and the filtrate was concentrated under reduced pressure to give compound L034-4 (500 mg). LC-MS: [M+H]⁺ = 263.10.

### 43.5 Synthesis of compound L034-5

L034-4 (500 mg, 1.91 mmol) and palladium on carbon (303 mg, 10% wt) were added to methanol (5 mL), and then concentrated hydrochloric acid (0.2 mL) was added. The reaction liquid was reacted at room temperature for 4 h under hydrogen atmosphere. The reaction liquid was filtered through celite, and then the filtrate was added with water (10 mL) for dilution. The pH was adjusted to 7-8 with an aqueous sodium bicarbonate solution, and the mixture was extracted with ethyl acetate (20 mL × 3). The organic phase was dried over anhydrous sodium sulfate and filtered, and the filtrate was concentrated under reduced pressure to give compound L034-5 (350 mg, crude product), which could be used directly in the next step. LC-MS: [M-NH₂]⁺ = 232.10.

### 43.6 Synthesis of compound L034-6

L034-2 (442 mg, 1.12 mmol), 1-hydroxybenzotriazole (166 mg, 1.23 mmol), and 1-(3-dimethylaminopropyl)-3-ethylcarbodiimide hydrochloride (236 mg, 1.23 mmol) were dissolved in N,N-dimethylformamide (3 mL), and the reaction liquid was reacted at room temperature for 0.5 h. Meanwhile, L034-5 (350 mg, 1.4 mmol) and N,N-diisopropylethylamine (578 mg, 4.48 mmol) were dissolved in N,N-dimethylformamide (2 mL), and the reaction liquid was reacted at room temperature for 0.5 h. The latter reaction liquid was added to the former reaction liquid, and the reaction liquid was reacted for another 0.5 h. The reaction liquid was added with water (10 mL) for dilution and extracted with ethyl acetate (20 mL × 3). The organic phases were combined, washed with saturated brine (30 mL), dried over anhydrous sodium sulfate and filtered, and the filtrate was concentrated under reduced pressure. The residue was purified by silica gel column chromatography to give compound L034-6 (410 mg, yield: 69.8%). LC-MS: [M+H]⁺ = 525.05.

### 43.7 Synthesis of compound L034

L034-6 (410 mg, 0.78 mmol) was dissolved in dichloromethane (5 mL), and then a solution of hydrogen chloride in ethyl acetate (5 mL, 4M) was added. The mixture was reacted at room temperature for 0.5 h. The solid was filtered, and the filter cake was washed with dichloromethane (5 mL) and dried under reduced pressure. The residue was purified by high performance liquid chromatography to give compound L034 (89 mg).

¹H NMR (400 MHz, DMSO-*d₆*): δ 8.20 - 8.07 (m, 2H), 7.25 - 7.18 (m, 2H), 7.12 - 7.07 (m, 2H), 6.81 - 6.75 (m, 1H), 6.44 - 6.36 (m, 2H), 5.12 - 5.04 (m, 1H), 4.95 (s, 2H), 4.27 - 4.19 (m, 1H), 3.64 - 3.61 (m, 1H), 2.88 - 2.71 (m, 2H), 2.67 - 2.54 (m, 4H), 2.30 - 2.28 (m, 1H), 2.20 - 1.97 (m, 1H), 1.81 - 1.58 (m, 3H), 1.24 - 1.16 (m, 4H). LC-MS: [M+H]⁺ = 425.15.

### Example 44: Synthesis of (2R,4S)-N-((2S)-1-((2-amino-3-methyl-6,7-dihydro-5H-cyclopenta[b] pyridin-5-yl)amino)-1-oxopropan-2-yl)-4-(4-fluorobenzyl)pyrrolidine-2-carboxamide (Compound L035)

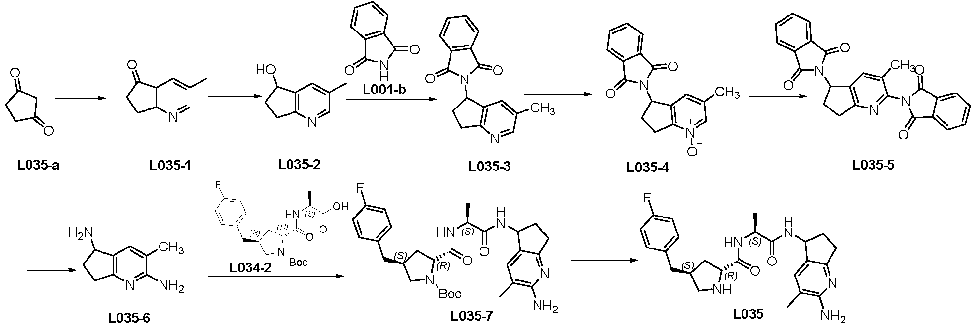

### 44.1 Synthesis of compound L035-1

L035-a (20.0 g, 203.8 mmol), 2-methacrolein (15.7 g, 224.2 mmol), and ammonium acetate (31.4 g, 407.6 mmol) were dissolved in toluene (100 mL) at room temperature. The reaction liquid was refluxed for 24 h. The reaction liquid was cooled to room temperature and then filtered through celite, and the filtrate was concentrated under reduced pressure. The residue was purified by silica gel column chromatography to give compound L035-1 (1.8 g, yield: 6.0%). LC-MS: [M+H]⁺ = 148.10.

### 44.2 Synthesis of compound L035-2

L035-1 (1.7 g, 11.5 mmol) was dissolved in methanol (20 mL) at room temperature, and then sodium borohydride (0.87 g, 23.0 mmol) was added in batches at 0 °C. The reaction temperature was controlled to be not exceeding 5 °C. At this temperature, the reaction liquid was reacted for 2 h. The reaction was quenched by dropwise adding methanol (15 mL) to the reaction liquid. The reaction liquid was concentrated under reduced pressure, and the residue was separated and purified by silica gel column chromatography to give compound L035-2 (1.5 g, yield: 88%). LC-MS: [M+H]⁺ = 150.15.

### 44.3 Synthesis of compound L035-3

L035-2 (1.4 g, 9.3 mmol) was dissolved in tetrahydrofuran (10 mL), and then phthalimide (1.37 g, 9.3 mmol) and triphenylphosphine (2.05 g, 12.09 mmol) were added under an ice bath. Diisopropyl azodicarboxylate (2.4 g, 12.09 mmol) was added dropwise and slowly under nitrogen atmosphere. After the dropwise addition was completed, the reaction liquid was warmed to room temperature and reacted for another 2 h. The reaction liquid was concentrated under reduced pressure, and the residue was purified by silica gel column chromatography to give compound L035-3 (2.1 g, yield: 80.7%). LC-MS: [M+H]⁺ = 279.00.

### 44.4 Synthesis of compound L035-4

L035-3 (2.0 g, 7.0 mmol) was dissolved in dichloromethane (20 mL) at room temperature, and then m-chloroperoxybenzoic acid (1.86 g, 10.6 mmol) was added slowly. The reaction liquid was reacted at room temperature for 2 h. The reaction liquid was quenched by adding saturated sodium bicarbonate (30 mL) and extracted with ethyl acetate (40 mL × 3). The combined organic phases were washed with saturated brine (50 mL). The organic phase was dried over anhydrous sodium sulfate and filtered, and the filtrate was concentrated under reduced pressure. The residue was purified by silica gel column chromatography to give compound L035-4 (900 mg, yield: 42.4%). LC-MS: [M+H]⁺ = 295.05.

### 44.5 Synthesis of compound L035-5

L035-4 (700 mg, 2.5 mmol) was dissolved in dichloromethane (10 mL) at room temperature, and then p-toluenesulfonyl chloride (739 mg, 3.9 mmol), N,N-diisopropylethylamine (969 mg, 7.5 mmol), and phthalimide (442 mg, 3.0 mmol) were added. The reaction liquid was reacted at room temperature for 2 h. The reaction liquid was concentrated under reduced pressure, and the residue was purified by silica gel column chromatography to give compound L035-5 (780 mg, yield: 72.2%). LC-MS: [M+H]⁺ = 423.90.

### 44.6 Synthesis of compound L035-6

L035-5 (700 mg, 4.28 mmol) was dissolved in ethanol (10 mL) at room temperature, and then hydrazine monohydrate (5 mL, 98%) was added. The reaction liquid was reacted at 80 °C for 2 h, and a white solid was precipitated. The reaction liquid was cooled to room temperature and filtered, and the filtrate was concentrated under reduced pressure. The residue was purified by high performance liquid chromatography and then lyophilized to give compound L035-6 (250 mg, yield: 92.6%). LC-MS: [M+H]⁺ = 163.1.

### 44.7 Synthesis of compound L035-7

L035-6 (50 mg, 0.3 mmol) and L034-2 (120 mg, 0.3 mmol) were dissolved in N,N-dimethylformamide (2 mL), and then triethylamine (121.3 mg, 1.2 mmol) and O-(7-azabenzotriazol-1-yl)-N,N,N',N'-tetramethyluronium hexafluorophosphate (125 mg, 0.33 mmol) were added. The reaction liquid was reacted at room temperature for 2 h. The reaction liquid was purified by high performance liquid chromatography and then lyophilized to give compound L035-7 (18.0 mg, yield: 10.9%). LC-MS: [M+H]⁺ = 540.0.

### 44.6 Synthesis of compound L035

L035-7 (18.0 mg, 0.033 mmol) was added to a solution of hydrogen chloride in 1,4-dioxane (1 mL, 4 M). The reaction liquid was reacted at room temperature for 1 h. The reaction liquid was concentrated, and then deionized water (5 mL) was added. The mixture was lyophilized to give compound L035 (11.0 mg, yield: 92.3%).

¹H NMR (400 MHz, DMSO-*d₆*): δ 10.46 -10.37 (m, 2H), 8.85 (t, *J =* 6.4 Hz, 1H), 8.54 (t, *J =* 8.0 Hz, 2H), 7.65 (s, 2H), 7.55 (d, *J =* 18.8 Hz,1H), 7.42 (s, 1H), 7.29 (t, *J =* 8.0 Hz, 2H), 7.16 - 7.10 (m, 2H), 5.14 (d, *J =* 6.8 Hz, 1H), 4.38 (s, 1H), 4.28 - 4.23 (m, 1H), 3.48 - 3.38 (m, 2H), 3.03-2.97 (m, 1H), 2.89 - 2.84 (m, 2H), 2.72 - 2.67 (m,2H), 2.50 - 2.43 (m, 1H) 2.14(s, 3H), 2.07 - 2.05 (m, 1H), 1.91 - 1.88(m, 2H), 1.23 (d, *J =* 4.4 Hz, 3H). LC-MS: [M+H]⁺ = 440.0.

### Example 45: Synthesis of (2R,4S)-4-(4-fluorobenzyl)-N-((2S)-1-((2-hydroxy-6,7-dihydro-5H-cyclopenta[b]pyridin-5-yl)amino)-1-oxopropan-2-yl)pyrrolidine-2-carboxamide (Compound L036)

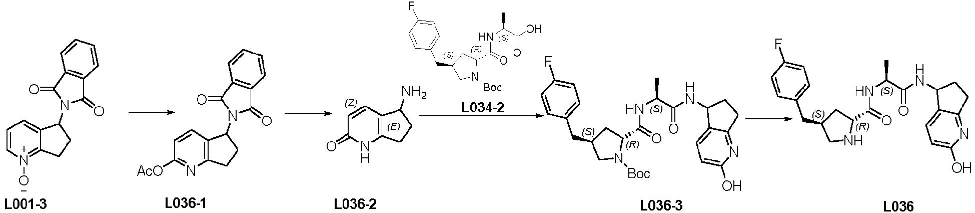

### 45.1 Synthesis of compound L036-1

L001-3 (1 g, 3.6 mmol) was dissolved in acetic anhydride (10 mL) at room temperature. The reaction liquid was warmed to 100 °C and reacted for 2 h. The reaction liquid was concentrated under reduced pressure, and the residue was purified by silica gel column chromatography to give compound L036-1 (800 mg, yield: 69.57%). LC-MS: [M+H]⁺ = 323.00.

### 45.2 Synthesis of compound L036

For the synthesis of compound L036, reference was made to the synthesis method of compound L035, and L035-5 was replaced with L036-1; the crude product obtained by reaction was purified by high performance liquid chromatography (basic condition) and then lyophilized to give compound L036 (18.6 mg, yield: 22.97%).

¹H NMR (400 MHz, DMSO-*d₆*): δ 8.47 (d, *J =* 4.0 Hz, 1H), 8.32 (d, *J =* 8.0 Hz, 1H), 8.21 - 8.13 (m, 1H), 7.60 - 7.50 (m, 1H), 7.30 - 7.16 (m, 3H), 7.09 (t, *J* = 8.8 Hz, 2H), 5.48 - 5.36 (m, 2H), 5.01 (s, 1H), 4.27 - 4.16 (m, 1H), 3.62 (dd, *J* = 9.2, 4.8 Hz, 1H), 2.89 - 2.78 (m, 1H), 2.63 - 2.52 (m, 3H), 2.27 - 2.18 (m, 1H), 2.17 - 2.09 (m, 1H), 2.09 - 2.01 (m, 1H), 1.79 - 1.61 (m, 2H), 1.27 - 1.19 (m, 4H). LC-MS: [M+H]⁺ = 427.05.

### Example 46: Synthesis of (2R,4S)-N-((2S)-1-((2-amino-5,6,7,8-tetrahydroquinolin-5-yl)amino)-1-oxopropan-2-yl)-4-(4-fluorobenzyl)pyrrolidine-2-carboxamide (Compound L037) Trifluoroacetate

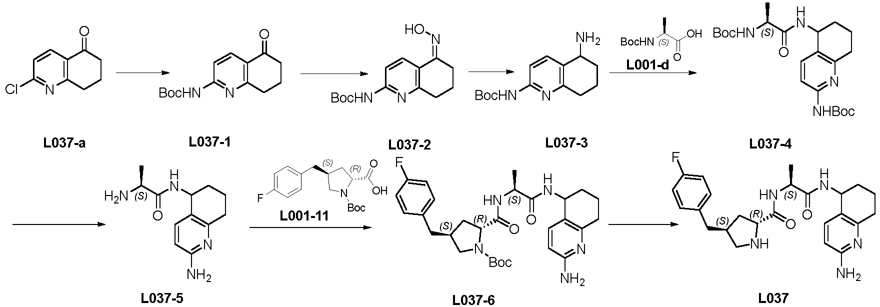

### 46.1 Synthesis of compound L037-1

L037-a (900 mg, 4.95 mmol) and tert-butyl carbamate (1.2 g, 9.91 mmol) were dissolved in 1,4-dioxane (10 mL) at room temperature, and then palladium(II) acetate (110.8 mg, 0.50 mmol), 4,5-bis(diphenylphosphino)-9,9-dimethylxanthene (286.1 mg, 0.50 mmol) and cesium carbonate (3.2 g, 9.91 mmol) were added. The reaction liquid was reacted at 90 °C for 3 h under nitrogen atmosphere. The reaction liquid was cooled to room temperature, added with water (30 mL) for dilution, and extracted with ethyl acetate (30 mL × 3). The organic phases were combined, dried over anhydrous sodium sulfate and filtered, and the filtrate was concentrated under reduced pressure. The residue was purified by silica gel column chromatography to give compound L037-1 (1.3 g, yield: 89%). LC-MS: [M+H]⁺ = 263.10.

### 46.2 Synthesis of compound L037-2

L037-1 (200 mg, 0.76 mmol) was dissolved in ethanol (2 mL) and water (1 mL), and then hydroxylamine hydrochloride (105.6 mg, 1.52 mmol) and potassium carbonate (157.6 mg, 1.14 mmol) were added. The reaction liquid was reacted at room temperature for 16 h. The reaction liquid was added with water (10 mL) for dilution and extracted with ethyl acetate (20 mL × 3). The combined organic phases were dried over anhydrous sodium sulfate and filtered, and the filtrate was concentrated under reduced pressure to give compound L037-2. LC-MS: [M+H]⁺ = 278.10.

### 46.3 Synthesis of compound L037-3

L037-2 (190 mg, crude product) was dissolved in methanol (2 mL), and then palladium on carbon (73 mg, 10% wt) was added. The reaction liquid was purged with hydrogen three times and reacted at room temperature for 4 h. The reaction liquid was filtered through celite, and the filter cake was washed with methanol (10 mL). The filtrate was concentrated under reduced pressure to give compound L037-3 (85 mg, crude product). LC-MS: [M+H]⁺ = 264.15.

### 46.4 Synthesis of compound L037-4

L001-d (60.5 mg, crude product) was dissolved in N,N-dimethylformamide (1 mL), and then 1-hydroxybenzotriazole (47.5 mg, 0.35 mmol) and 1-(3-dimethylaminopropyl)-3-ethylcarbodiimide hydrochloride (67.6 mg, 0.35 mmol) were added. The reaction liquid was reacted at room temperature for 10 min. A solution of L037-3 (85 mg, 0.32 mmol) and N,N-diisopropylethylamine (165.1 mg, 1.28 mmol) in N,N-dimethylformamide (1 mL) was then added to the above reaction system. The reaction liquid was reacted at room temperature for another 1 h. The reaction liquid was added with water (20 mL) for dilution and extracted with ethyl acetate (30 mL × 3). The organic phases were combined and washed with saturated brine (40 mL). The organic phase was dried over anhydrous sodium sulfate and filtered, and the filtrate was concentrated under reduced pressure to give compound L037-4 (70 mg, crude product). LC-MS: [M+H]⁺ = 435.10.

### 46.5 Synthesis of compound L037-5

L037-4 (128 mg, crude product) was dissolved in dichloromethane (2 mL), and then a solution of hydrogen chloride in ethyl acetate (2 mL, 4 M) was added. The reaction liquid was reacted at room temperature for 0.5 h. The reaction liquid was concentrated under reduced pressure, and the residue was added with dichloromethane (10 mL). The mixture was stirred for 10 min and filtered, and the filter cake was dried under reduced pressure to give compound L037-5 (150 mg). LC-MS: [M+H]⁺ = 235.15.

### 46.6 Synthesis of compound L037 trifluoroacetate

For the synthesis of compound L037, reference was made to the synthesis method of compound L001, and L001-7 was replaced with L037-5; the crude product obtained by reaction was purified by high performance liquid chromatography (trifluoroacetic acid condition) to give compound L037 trifluoroacetate (34 mg).

¹H NMR (400 MHz, DMSO-*d₆*): δ 9.61 - 9.56(m, 1H), 8.77(d, *J* = 8.0 Hz, 1H), 8.54(s, 1H), 8.39(d, *J* = 12.0 Hz, 1H), 8.00 - 7.89(m, 1H), 7.67 - 7.52(m, 1H), 7.28 - 7.25(m, 2H), 7.14(t, *J =* 8.0 Hz, 2H), 6.79(t, *J* = 12.0 Hz, 1H), 4.81(s, 1H), 4.35 - 4.21(m, 2H), 3.31(s, 1H), 2.91(s, 1H), 2.71 - 2.62(m, 4H), 2.49 - 2.40(m, 2H), 2.08 - 1.63(m, 6H), 1.24(dd, *J* = 8.0, 4.0 Hz, 3H). LC-MS: [M+H]⁺ = 440.05.

### Example 47: Synthesis of (2R,4R)-N-((2S)-1-((2-amino-6,7-dihydro-5H-cyclopenta[b]pyridin-5-yl) amino)-1-oxopropan-2-yl)-4-(2-fluorophenyl)piperidine-2-carboxamide (Compound L038-IS-01)

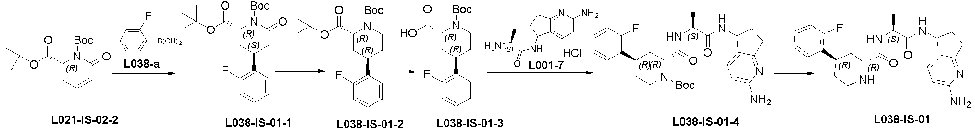

For the synthesis of compound L038-IS-01, reference was made to the synthesis method of compound L021-IS-02, and L006-b was replaced with L038-a; the crude product obtained by reaction was purified by high performance liquid chromatography (basic condition) and then lyophilized to give compound L038-IS-01 (60.6 mg. yield: 29.94%).

¹H NMR (400 MHz, DMSO-*d₆*): δ 7.91 (s, 1H), 7.38 (d, *J* = 8.0 Hz, 1H), 7.22 - 6.95 (m, 4H), 6.73 (s, 1H), 6.39 - 6.15 (m, 1H), 5.36 (s, 1H), 4.59 - 4.50 (m, 1H), 4.45 (s, 2 H), 3.74 (s, 1H), 3.08 - 2.76 (m, 5H), 2.64 - 2.49 (m, 2H), 2.06 - 1.50 (m, 5H), 1.43 (d, *J* = 7.2 Hz, 3H). LC-MS: [M+H]⁺ = 426.10.

### Example 48: Synthesis of (2R,4S)-N-((2S)-1-((2-amino-6,7-dihydro-5H-cyclopenta[b]pyridin-5-yl) amino)-1-oxopropan-2-yl)-4-(2-fluorophenyl)piperidine-2-carboxamide (Compound L038-IS-02) Hydrochloride

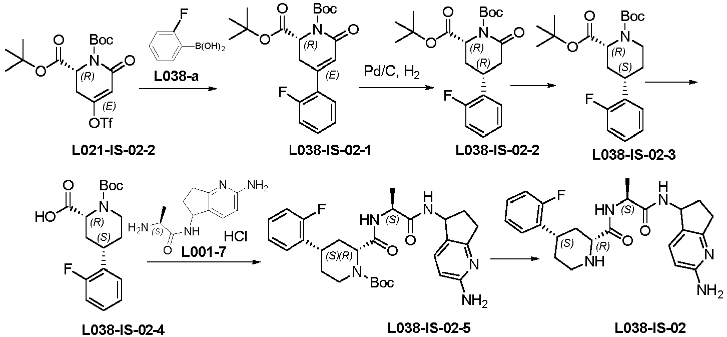

For the synthesis of compound L038-IS-02 hydrochloride, reference was made to the synthesis method of compound L021-IS-01, and L006-b was replaced with L038-a; the crude product obtained by reaction was purified by high performance liquid chromatography (acidic condition) and then lyophilized to give compound L038-IS-02 hydrochloride (56.2 mg. yield: 29.06%).

¹H NMR (400 MHz, DMSO-*d₆*): δ 9.76 - 9.62 (m, 1H), 8.86 (d, J = 7.6 Hz, 2H), 8.55 (dd, J = 16.0, 8.0 Hz, 1H), 8.03 (s, 2H), 7.72 - 7.66 (m, 1H), 7.39 - 7.13 (m, 4H), 6.84 (t, J = 9.2 Hz, 1H), 5.25 - 5.06 (m, 1H), 4.36 - 4.13 (m, 1H), 3.98 (t, J = 10.8 Hz, 1H), 3.33 (d, J = 11.6 Hz, 1H), 3.25 - 3.02 (m, 3H), 2.96 - 2.81 (m, 1H), 2.47 - 2.41 (m, 1H), 2.26 (d, J = 13.2 Hz, 1H), 2.03 - 1.74 (m, 4H), 1.23 (d, J = 6.8 Hz, 3H). LC-MS: [M+H]⁺ = 426.10.

Reference was made to the synthesis method of L021-IS-01/L021-IS-02 to synthesize the compounds in Table A below, wherein for the synthesis of Examples 49, 51, 52, 53, 55, 57, and 59, reference was made to the synthesis method of compound L021-IS-01, and for the synthesis of Examples 50, 54, 56, 58, 60, 61, 62, and 63, reference was made to the synthesis method of compound L021-IS-02.

| Example | Intermediate number and structure | Compound structure, number, and name | Characterization data |
|---|---|---|---|
| 49 | 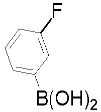 L039-a | 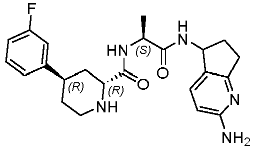 L039-IS-01 | ¹H NMR (400 MHz, DMSO-*d₆*): δ 8.21 - 8.09 (m, 2H), 7.35 - 7.30 (m, 1H), 7.20 - 7.12 (m, 1H), 7.01 - 6.93 (m, 3H), 5.83 (t, *J* = 8.0 Hz, 1H), 5.82 (s, 2H), 5.15 - 5.07 (m, 1H), 4.36 - 4.31 (m, 1H), 3.53 (d, *J* = 3.6 Hz, 1H), 2.88 - 2.82 (m, 1H), 2.78 - 2.64 (m, 4H), 2.39 - 2.26 (m, 2H), 1.75 - 1.69 (m, 1H), 1.61 - 1.49 (m, 2H), 1.43 - 1.37(m, 1H), 1.24 - 1.21 (m, 3H); LC-MS: [M+H]⁺ =426.10 |
| | | (2R,4R)-N-((2S)-1-((2-amino-6,7-dihydro-5H-cyclopenta[b]pyridin-5-yl)amino)-1-oxopropan-2 yl)-4-(3-fluorophenyl)piperidine-2-carboxamide | |
| 50 | 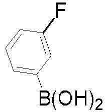 L039-a | 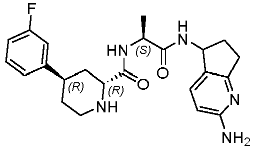 L039-IS-02 | ¹H NMR (400 MHz, DMSO-*d₆*): δ 9.66 - 9.52 (m, 1H), 8.82 (s, 2H), 8.56 - 8.51 (m, 1H), 7.96 (s, 2H), 7.68 (dd, *J =* 28.0, 8.0 Hz, 1H), 7.41 - 7.39 (m, 1H), 7.08 - 7.03 (m, 3H), 6.83 - 6.80 (m, 1H), 5.16 (s, 1H), 4.29 - 4.22 (m, 1H), 3.94 (s, 1H), 3.05 - 2.92 (m, 5H), 2.34 (d, *J =* 16.0 Hz, 1H), 2.05 - 1.82 (m, 3H), 1.71 - 1.68 (m, 1H), 1.24 (d, *J* = 8.0 Hz, 4H); |
| | | (2R,4S)-N-((2S)-1-((2-amino-6,7-dihydro-5H-cyclopenta[b]pyridin-5-yl)amino)-1-oxopropan-2 yl)-4-(3-fluorophenyl)piperidine-2-carboxamide | |
| | | | LC-MS: [M+H]⁺ =426.10 |
| 51 | 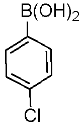 L040-a | 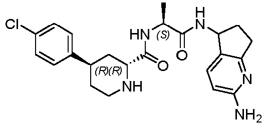 L040-IS-01 | ¹H NMR (400 MHz, DMSO-*d₆*): δ 8.27 - 8.09 (m, 2H), 7.36 -7.34 (m, 2H), 7.23 - 7.13 (m, 3H), 6.23 (t, *J =* 16.0 Hz, 1H), 5.84 (s, 2H), 5.19 - 5.06 (m, 1H), 4.35 - 4.33 (m, 1H), 3.55 (d, *J* = 4.0 Hz, 1H), 2.95 - 2.57 (m, 5H), 2.35 - 2.25 (m, 2H), 1.82 - 1.69 (m, 1H), 1.57-1.51 (m, 2H), 1.45 - 1.35 (m, 1H), 1.26 - 1.23 (m, 4H); LC-MS: [M+H]⁺=442.10 |
| | | (2R,4R)-N-((2S)-1-((2-amino-6,7-dihydro-5H-cyclopenta[b]pyridin-5-yl)amino)-1-oxopropan-2-yl)-4-(4-chlorophenyl)piperidine-2-carboxamide | |
| 52 | 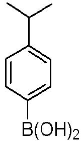 L041-a | 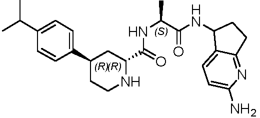 L041-IS-01 | ¹H NMR (400 MHz, DMSO-*d₆*): δ 9.81 - 9.67 (m, 1H), 8.97 (t, *J =* 12.8 Hz, 1H), 8.82 (d, *J =* 7.2 Hz, 1H), 8.52 (dd, *J*₁ = 11.2 Hz, *J*₂ = 8.0 Hz, 1H), 7.99 (s, 2H), 7.68 (dd, *J*₁ = 15.2 Hz, *J*₂ *=* 8.8 Hz, 1H), 7.25 - 7.12 (m, 4H), 6.82 (d, *J =* 8.8 Hz, 1H), 5.26 - 5.03 (m, 1H), 4.44 - 4.10 (m, 2H), 3.46 - 3.35 (m, 1H), 3.20 - 2.99 (m, 2H), 2.96 - 2.74 (m, 3H), 2.47 - 2.42 (m, 1H), 2.35 - 2.59 (m, 1H), 2.23 - 2.15 (m, 1H), 2.02 - 1.88 (m, 3H), 1.29 (d, *J =* 7.2 Hz, 3H), 1.18 (d, *J* = 6.8 Hz, 6H); |
| | | (2R,4R)-N-((2S)-1-((2-amino-6,7-dihydro-5H-cyclopenta[b]pyridin-5-yl)amino)-1-oxopropan-2-yl)-4-(4-isopropylphenyl)piperidine-2-carboxamide | |
| | | | LC-MS: \|M+H]⁺ = 450.10 |
| 53 | 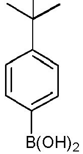 L042-a | 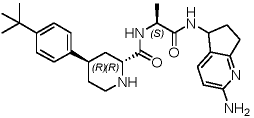 L042-IS-01 | ¹H NMR (400 MHz, DMSO-*d₆*): δ 9.75 - 9.67 (m, 1H), 8.97 (t, *J =* 16.0 Hz, 1H), 8.82 (d, *J =* 7.2 Hz, 1H), 8.52 (dd, *J*₁ = 11.6 Hz, *J*₂ = 8.0 Hz, 1H), 7.97 (s, 2H), 7.68 (dd, *J*₁ = 14.4 Hz, *J*₂ *=* 8.8 Hz, 1H), 7.36 (d, *J* = 8.4 Hz, 2H), 7.17 (d, *J* = 8.4 Hz, 2H), 6.81 (d, *J* = 8.8 Hz, 1H), 5.26 - 5.03 (m, 1H), 4.38 - 4.25 (m, 1H), 4.20 (s, 1H), 3.40 - 3.32 (m, 1H), 3.19 - 3.00 (m, 2H), 2.97 - 2.86 (m, 1H), 2.82 - 2.75 (m, 1H), 2.48 - 2.38 (m, 1H), 2.33 - 2.28 (m, 1H), 2.24 - 2.13 (m, 1H), 2.02 - 1.88 (m, 3H), 1.29 (d, *J =* 7.2 Hz, 3H), 1.27 (s, 9H); |
| | | (2R,4R)-N-((2S)-1-((2-amino-6,7-dihydro-5H-cyclopenta[b]pyridin-5-yl)amino)-1-oxopropan-2-yl)-4-(4-(tert-butyl)phenyl)piperidine-2-carboxamide | |
| | | | LC-MS: [M+H]⁺ = 464.15 |
| 54 | 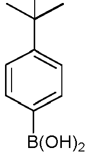 L042-a | 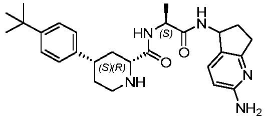 L042-IS-02 | ¹H NMR (400 MHz, DMSO-*d*₆) δ: 9.60 - 9.46 (m, 1H), 8.80 (dd, *J* = 7.2, 2.4 Hz, 2H), 8.61 - 8.46 (m, 1H), 7.96 (s, 2H), 7.68 (dd, *J =* 24, 12 Hz, 1H), 7.35 (d, *J* = 8.4 Hz, 2H), 7.13 (d, *J* = 8.4 Hz, 2H), 6.81 (t*, J =* 8.4 Hz, 1H), 5.19 - 5.13 (m, 1H), 4.33 - 4.16 (m, 1H), 3.93 (t, *J =* 11.2 Hz, 1H), 3.33 (d, *J =* 13.2 Hz, 1H), 3.10 - 3.02 (m, 2H), 2.94 - 2.78 (m, 2H), 2.48 - 2.36 (m, 1H), 2.29 (d, *J =* 12.8 Hz, 1H), 2.02 - 1.93 (m, 1H), 1.88 - 1.83 (m, 2H), 1.69 - 1.58 (m, 1H), 1.28 - 1.18 (m, 12H); |
| | | | |
| | | | |
| | | (2R,4S)-N-((2S)-1-((2-amino-6,7-dihydro-5H-cyclopenta[b]pyridin-5-yl)amino)-1-oxopropan-2-yl)-4-(4-(tert-butyl)phenyl)piperidine-2-carboxamide | |
| | | | LC-MS: [M+H]⁺ = 464.15 |
| 55 | 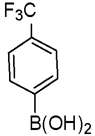 L008-a | 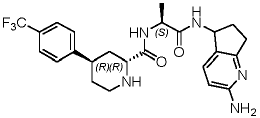 L043-IS-01 | ¹H NMR (400 MHz, DMSO-*d₆*): δ 8.23 - 8.1 (m, 2H), 7.68 - 7.64 (m, 2H), 7.39 (t, *J* = 8.0 Hz, 2H), 7.22 - 7.14 (m, 1H), 6.28 - 6.18 (m, 1H), 5.84 (s, 2H), 5.16 - 5.08 (m, 1H), 4.40 - 4.31 (m, 1H), 3.56 (d, *J =* 4.0 Hz, 1H), 2.88 (t, *J* = 12.0 Hz, 1H), 2.80 - 2.56 (m, 5H), 2.41 - 2.25 (m, 2H), 1.82 - 1.68 (m, 1H), 1.65 - 1.50 (m, 2H), 1.50 - 1.39 (m, 1H), 1.26-1.23 (m, 3H); |
| | | (2R,4R)-N-((2S)-1-((2-amino-6,7-dihydro-5H-cyclopenta[b]pyridin-5-yl)amino)-1-oxopropan-2 yl)-4-(4-(trifluoromethyl)phenyl)piperidine-2-carboxamide | |
| | | | LC-MS: [M+H]⁺ = 476.05 |
| 56 | 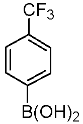 L008-a | 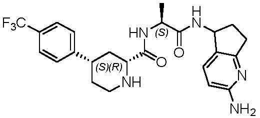 L043-IS-02 | ¹H NMR (400 MHz, DMSO-*d*₆) δ: 9.07 (d, *J =* 12.0 Hz, 1H), 8.76 - 8.71 (m, 2H), 8.42 (dd, *J =* 8.0, 8.0 Hz, 1H), 7.73 (d, *J =* 8.0 Hz, 2H), 7.54 (s, 1H), 7.46 (d, *J =* 8.0 Hz, 2H), 6.65 (s, 1H), 5.35 - 5.12 (m, 1H), 4.29 (dd, *J =* 20.0, 8.0 Hz, 1H), 3.97 (t, *J =* 24.0 Hz, 1H), 3.21 - 2.61 (m, 5H), 2.37 (s, 1H), 2.06 - 1.92 (m, 2H), 1.84 (d, *J =* 12.0 Hz, 2H), 1.62 (m, 1H), 1.23 (s, 4H); |
| | | (2R,4S)-N-((2S)-1-((2-amino-6,7-dihydro-5H-cyclopenta[b]pyridin-5-yl)amino)-1-oxopropan-2 yl)-4-(4-(trifluoromethyl)phenyl)piperidine-2-carboxamide | |
| | | | LC-MS: [M+H]⁺ = 476.05 |
| 57 | 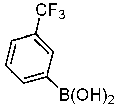 L044-a | 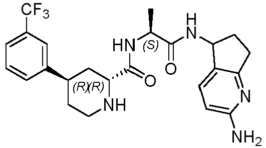 L044-IS-01 | ¹H NMR (400 MHz, DMSO-*d₆*): δ 8.25 - 8.13 (m, 2H), 7.55 (d, *J* = 4.0 Hz, 2H), 7.51 - 7.45 (m, 2H), 7.22 - 7.14 (m, 1H), 6.23 (t, *J* = 8.0 Hz, 1H), 5.83 (d, *J =* 8.0 Hz, 2H), 5.20 - 5.06 (m, 1H), 4.35 (m, 1H), 3.56 (d*, J =* 4.0 Hz, 1H), 2.79 - 2.56 (m, 5H), 2.39 -2.34 (m, 2H), 1.80 - 1.67 (m, 1H), 1.69 - 1.52 (m, 2H), 1.50 - 1, 42 (m, 1H), 1.26 - 1.23 (m, 4H); |
| | | (2R,4R)-N-((2S)-1-((2-amino-6,7-dihydro-5H-cyclopenta[b]pyridin-5-yl)amino)-1-oxopropan-2-yl)-4-(3-(trifluoromethyl)phenyl)piperidine-2-carboxamide | |
| | | | LC-MS: [M+H]⁺ = 476.10 |
| 58 | 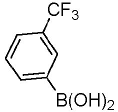 L044-a | 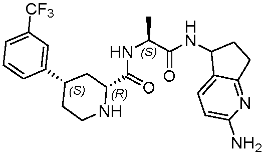 L044-IS-02 | ¹H NMR (400 MHz, DMSO-*d*₆) δ: 8.15 (d, *J =* 8.2 Hz, 1H), 7.73 (d, *J* = 7.6 Hz, 1H), 7.55 (s, 4H), 7.16 (d, *J* = 8.0 Hz, 1H), 6.22 (d, *J =* 8.0 Hz, 1H), 5.82 (s, 2H), 5.23 - 5.02 (m, 1H), 4.37 - 4.17 (m, 1H), 3.55 (s, 1H), 2.96 - 2.54 (m, 5H), 2.45 - 2.23 (m, 1H), 1.98 (dd, *J =* 8.0, 8.0 Hz, 1H), 1.79 - 1.63 (m, 2H), 1.56 - 1.50 (m, 1H),1.43 - 1.32 (m, 1H), 1.25 (m, 4H); |
| | | (2R,4S)N((2S)1-((2-amino-6,7-dihydro-5H-cyclopenta[b]pyridin-5-yl)amino)-1-oxopropan-2-yl)-4-(3-(trifluoromethyl)phenyl)piperidine-2-carboxamide | |
| | | | LC-MS: [M+H]⁺ = 476.05 |
| 59 | 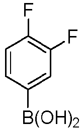 L045-a | 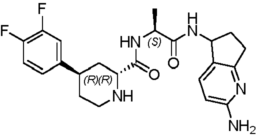 L045-IS-01 | ¹H NMR (400 MHz, DMSO-*d₆*): δ 8.22 - 8.09 (m, 2H), 7.37 - 7.30 (m, 1H), 7.24 -7.13 (m, 2H), 6.95 - 6.04 (m, 1H), 6.22 (t, *J* = 8.8 Hz, 1H), 5.83 (s, 2H), 5.13 - 5.10 (m, 1H), 4.36 - 4.31 (m, 1H), 3.53 (d, *J* = 3.6 Hz, 1H), 2.87 - 2.82 (m, 1H), 2.78 - 2.59 (m, 4H), 2.40 - 2.50 (m, 1H), 2.28 - 2.25 (m, 1H), 1.75 - 1.68 (m, 1H), 1.60 - 1.37(m, 2H), 1.41- 1.34(m, 1H), 1.25 - 1.22 (m, 3H); |
| | | (2R,4R)-N-((2S)-1-((2-amino-6,7-dihydro-5H-cyclopenta[b]pyridin-5-yl)amino)-1-oxopropan-2-yl)-4-(3,4-difluorophenyl)piperidine-2-carboxamide | |
| | | | LC-MS: [M+H]⁺ = 444.10 |
| 60 | 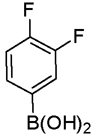 L045-a | 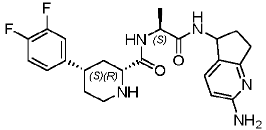 L045-IS-02 | ¹H NMR (400 MHz, DMSO-*d*₆) δ: 9.52 - 9.48 (m, 1H), 8.80 - 8.78 (m, 2H), 8.52 - 8.50 (m, 1H), 7.80 (s, 1H), 7.67 - 7.63 (m, 1H), 7.45 - 7.37 (m, 1H), 7.35 (s, 1H), 7.32 -7.25 (m, 1H), 7.22 (s, 1H), 7.10 - 7.06 (m, 2H), 6.81 - 6.60 (m, 1H), 5.22 - 5.11 (m, 1H), 4.34 - 4.18 (m, 1H), 3.94 (t, *J =* 11.2 Hz, 1H), 3.13 - 2.82 (m, 5H), 2.49 - 2.38 (m, 1H), 2.34 (d, *J =* 11.6 Hz, 1H), 2.04 - 1.88 (m, 3H), 1.85 - 1.74 (m, 1H), 1.67 (q, *J =* 12.4 Hz, 1H), 1.23 (d, *J* = 7.2 Hz, 3H); |
| | | (2R,4S)-N-((2S)-1-((2-amino-6,7-dihydro-5H-cyclopenta[b]pyridin-5-yl)amino)-1-oxopropan-2-yl)-4-(3,4-difluorophenyl)piperidine-2-carboxamide | |
| | | | LC-MS: [M+H]⁺ = 444.10 |
| 61 | 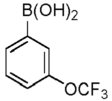 L046-a | 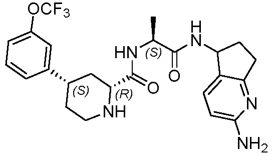 L046-IS-02 | ¹H NMR (400 MHz, DMSO-*d*₆) δ 8.16-8.13 (m, 1H), 7.73 (d, *J =* 8.0 Hz, 1H), 7.48 - 7.41 (m, 1H), 7.31 - 7.23 (m, 1H), 7.22 - 7.09 (m, 3H), 6.22 (d, *J =* 8.0 Hz, 1H), 5.82 (s, 2H), 5.14 - 5.02 (m, 1H), 4.27-4.21 (m, 1H), 3.20-3.09 (m, 2H), 2.79 - 2.69 (m, 2H), 2.67 - 2.58 (m, 2H), 2.40 - 2.25 (m, 2H), 1.97-1.94 (m, 1H), 1.71 - 1.67 (m, 2H), 1.51-1.45 (m, 1H), 1.23-1.20 (m, 4H); |
| | | (2R,4S)-N-((2S)-1-((2-amino-6,7-dihydro-5H-cyclopenta[b]pyridin-5-yl)amino)-1-oxopropan-2yl)-4-(3-(trifluoromethoxy)phenyl)piperidine-2-carboxamide | |
| | | | LC-MS: [M+H]⁺ = 492.05 |
| 62 | 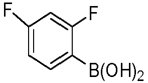 L047-a | 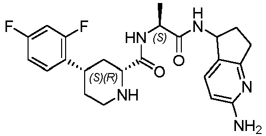 L047-IS-02 | ¹H NMR (400 MHz, DMSO-*d*₆) δ: 8.13 (d, *J =* 8.0 Hz, 1H), 7.74 (d, *J* = 7.6 Hz, 1H), 7.36-7.27 (m, 1H), 7.22 - 6.95 (m, 3H), 6.22 (d, *J* = 8.4 Hz, 1H), 5.83 (d, *J =* 1.6 Hz, 2H), 5.10 - 5.08 (m, 1H), 4.26 - 4.22 (m, 7.2 Hz, 1H), 3.21 (d, *J* = 11.2 Hz, 1H), 3.10 (d, *J* = 12.0 Hz, 1H), 2.92 (t, *J =* 12.0 Hz, 1H), 2.77 - 2.69 (m, 1H), 2.68 - 2.57 (m, 2H), 2.46-2.28 (m, 2H), 1.89 (d, *J* = 12.4 Hz, 1H), 1.76 - 1.60 (m, 2H), 1.57 - 1.47 (m, 1H), 1.43 - 1.31 (m, 1H), 1.22 - 1.19 (m, 3H); |
| | | (2R,4S)-N-((2S)-1-((2-amino-6,7-dihydro-5H-cyclopenta[b]pyridin-5-yl)amino)-1-oxopropan-2-yl)-4-(2,4-difluorophenyl)piperidine-2-carboxamide | |
| | | | LC-MS: [M+H]⁺ = 444.05 |
| 63 | 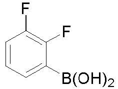 L048-a | 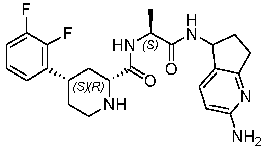 L048-IS-02 | ¹H NMR (400 MHz, DMSO-*d*₆) δ: 9.63 - 9.60 (m, 1H), 8.84 (d, *J =* 7.1 Hz, 2H), 8.53 - 8.52 (m, 1H), 7.98 (s, 2H), 7.70 - 7.68 (m, 1H), 7.33 - 7.30 (m, 1H), 7.22 - 720 (m, 1H), 7.09 (t, *J* = 6.9 Hz, 1H), 6.82 (t, *J* = 8.6 Hz, 1H), 5.16 - 5.12 (m, 1H), 4.33 - 4.14 (m, 1H), 3.98 (t, *J =* 10.5 Hz, 1H), 3.35 - 3.19 (m, 2H), 3.17 - 2.99 (m, 2H), 2.95 - 2.83 (m, 1H), 2.45 (dd, *J*₁ = 8.3 Hz, *J*₂ = 6.1 Hz, 1H), 2.28 (d, *J* = 13.2 Hz, 1H), 2.04 - 1.85 (m, 3H), 1.79 - 1.76 (m, 1H), 1.22 (d, *J* = 7.2 Hz, 3H); |
| | | (2R,4S)-N-((2S)-1-((2-amino-6,7-dihydro-5H-cyclopenta[b]pyridin-5-yl)amino)-1-oxopropan-2-yl)-4-(2,3-difluorophenyl)piperidine-2-carboxamide | |
| | | | LC-MS: [M+H]⁺ = 444.05 |

### Example 64: Synthesis of N-((2S)-1-((2-amino-6,7-dihydro-SH-cyclopenta[b]pyridin-5-yl)amino)-1-oxopropan-2-yl)-4-((4,4-dimethylpiperidin-1-yl)methyl)pyrrolidine-2-carboxamide (Compound L049) Hydrochloride

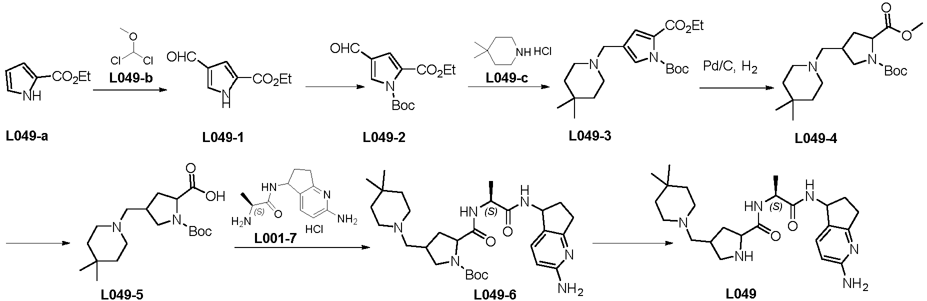

### 64.1 Synthesis of compound L049-1

L049-a (1.00 g, 7.19 mmol) was dissolved in dichloromethane (6 mL) and nitromethane (6 mL) at room temperature, aluminum trichloride (1.92 g, 14.38 mmol) was added in batches at -20 °C under nitrogen atmosphere, and then L049-b (1.65 g, 14.38 mmol) was added dropwise. The reaction liquid was maintained at -20 °C and reacted for 1 h. The reaction liquid was slowly poured into ice water (20 mL) to quench the reaction. The organic phase was separated out, and the aqueous phase was extracted with dichloromethane (15 mL × 3). The organic phases were combined and washed with a saturated aqueous sodium bicarbonate solution (30 mL) and a saturated sodium chloride solution (30 mL). The organic phase was dried over anhydrous sodium sulfate and filtered, and the filtrate was concentrated under reduced pressure to give compound L049-1 (1.10 g, crude product), which was used directly in the next step. LC-MS: [M+H]⁺ = 168.10.

### 64.2 Synthesis of compound L049-2

L049-1 (1.10 g, crude product) was dissolved in dichloromethane (10 mL), and then di-tert-butyl dicarbonate (2.15 g, 9.87 mmol), 4-dimethylaminopyridine (161 mg, 1.32 mmol), and triethylamine (1.33 g, 13.16 mmol) were added. The reaction liquid was reacted at room temperature for 4 h. The reaction liquid was added with water (20 mL) for dilution and extracted with dichloromethane (15 mL × 3). The organic phases were combined and washed with a saturated aqueous sodium chloride solution (20 mL), dried over anhydrous sodium sulfate and filtered, and the filtrate was concentrated under reduced pressure. The residue was purified by silica gel column chromatography (petroleum ether/ethyl acetate = 4/1) to give compound L049-2 (660 mg). LC-MS: [M+H]⁺ = 268.10.

### 64.3 Synthesis of compound L049-3

L049-2 (650 mg, 2.43 mmol) and L049-c (437 mg, 2.92 mmol) were dissolved in dichloromethane (10 mL), and acetic acid (146 mg, 2.43 mmol) was added. The reaction liquid was reacted at room temperature for 2 h. Sodium triacetoxyborohydride (1.29 g, 6.08 mmol) was then added, and the reaction liquid was reacted at room temperature for another 16 h. The reaction liquid was poured into ice water (20 mL) to quench the reaction and extracted with dichloromethane (15 mL × 3). The organic phases were combined, washed with a saturated aqueous sodium chloride solution (20 mL), dried over anhydrous sodium sulfate and filtered, and the filtrate was concentrated under reduced pressure. The residue was purified by silica gel column chromatography (petroleum ether/ethyl acetate = 2/1) to give compound L049-3 (450 mg, yield: 50.3%). LC-MS: [M+H]⁺ = 365.05.

### 64.4 Synthesis of compound L049-4

L049-3 (450 mg, 1.23 mmol) was dissolved in ethanol (10 mL), and palladium on carbon (200 mg, 10% wt) was added. The reaction liquid was reacted at room temperature for 16 h under hydrogen atmosphere. The reaction liquid was filtered through celite, and the filter cake was washed with ethanol (10 mL). The filtrate was concentrated under reduced pressure to give compound L049-4 (450 mg, crude product). LC-MS: [M+H]⁺ = 369.10.

### 64.5 Synthesis of compound L049-5

L049-4 (450 mg, 1.22 mmol) was dissolved in tetrahydrofuran (10 mL), methanol (5 mL) and water (5 mL), and then lithium hydroxide monohydrate (154 mg, 3.66 mmol) was added. The reaction liquid was reacted at room temperature for 5 h. The reaction liquid was concentrated under reduced pressure, and the residue was added with water (10 mL) for dissolution. The aqueous phase was adjusted to pH = 6-7 with diluted hydrochloric acid (1 M) and extracted with dichloromethane (15 mL × 3). The organic phases were combined and washed with a saturated aqueous sodium chloride solution (20 mL). The organic phase was dried over anhydrous sodium sulfate and filtered, and the filtrate was concentrated under reduced pressure to give compound L049-5 (450 mg, crude product). LC-MS: [M+H]⁺ = 341.05.

### 64.6 Synthesis of compound L049-6

L049-5 (450 mg, crude product), L001-7 (407 mg, 1.58 mmol), and N,N-diisopropylethylamine (668 mg, 6.60 mmol) were dissolved in dichloromethane (5 mL), and then 1-propylphosphoric anhydride (630 mg, 1.98 mmol, 50% in ethyl acetate) was added. The reaction liquid was reacted at room temperature for 3 h. The reaction liquid was added with water (20 mL) for dilution and extracted with dichloromethane (15 mL × 3). The organic phases were combined and washed with a saturated aqueous sodium chloride solution (20 mL). The organic phase was dried over anhydrous sodium sulfate and filtered, and the filtrate was concentrated under reduced pressure to give compound L049-6 (370 mg, crude product). LC-MS: [M+H]⁺ = 543.15.

### 64.7 Synthesis of compound L049 hydrochloride

L049-6 (370 mg, crude product) was dissolved in dichloromethane (6 mL), and then a solution of hydrogen chloride in ethyl acetate (4 mL, 4 M) was added. The reaction liquid was reacted at room temperature for 0.5 h. The reaction liquid was concentrated under reduced pressure, and the residue was purified by high performance liquid chromatography (0.1% hydrochloric acid condition) to give compound L049 hydrochloride (23.4 mg).

¹H NMR (400 MHz, DMSO-*d₆*): δ 10.50 (s, 1H), 10.13 (s, 1H), 8.86 (dd, *J* = 6.8, 3.2 Hz, 1H), 8.78 (s, 1H), 8.56 (d, *J* = 7.6 Hz, 1H), 8.01 (s, 2H), 7.75 - 7.62 (m, 1H), 6.84 (t, *J* = 9.2 Hz, 1H), 5.19 - 5.09 (m, 1H), 4.35 - 4.16 (m, 2H), 3.55 (s, 2H), 3.32 - 3.16 (m, 4H), 3.13 - 2.73 (m, 6H), 2.67 - 2.59 (m, 1H), 1.92 (d, *J=* 6.7 Hz, 1H), 1.80 - 1.76(m, 2H), 1.73 - 1.57 (m, 1H), 1.48 - 1.45 (m, 2H), 1.27 (t, *J* = 6.4 Hz, 3H), 0.99 - 0.96 (m, 6H). LC-MS: [M+H]⁺ = 443.10.

### Example 65: Synthesis of (2R)-N-((S)-1-(((R)-2-amino-6,7-dihydro-5H-cyclopenta[b]pyridin-5-yl) amino)-1-oxopropan-2-yl)-4-(difluoro(4-fluorophenyl)methyl)pyrrolidine-2-carboxamide (Compound L050)

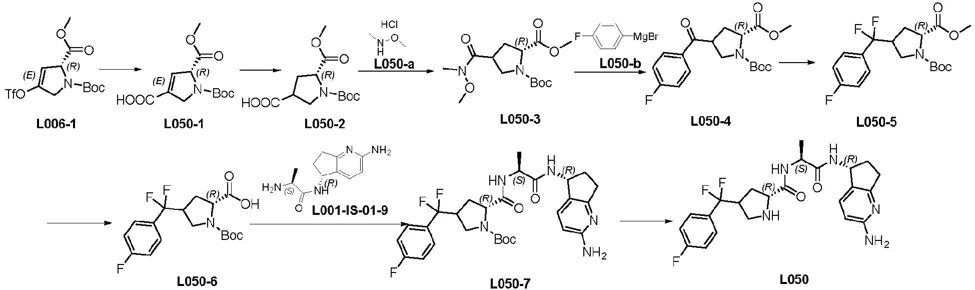

### 65.1 Synthesis of compound L050-1

L006-1 (2.70 g, 7.19 mmol) was dissolved in tetrahydrofuran (20 mL) and water (5 mL), and then tetrakis(triphenylphosphine)palladium(0) (1.66 g, 1.44 mmol) was added. The reaction liquid was purged with nitrogen and then reacted at room temperature for 16 h under carbon monoxide atmosphere. The reaction liquid was added with water (30 mL) for dilution and extracted with ethyl acetate (20 mL × 2). The combined organic phases were washed with a saturated aqueous sodium bicarbonate solution (20 mL), and the organic phase was discarded. All aqueous phases were combined. The aqueous phase was adjusted to pH = 2 with diluted hydrochloric acid (2 M) and extracted with ethyl acetate (25 mL × 3). The organic phases were combined and washed with a saturated aqueous sodium chloride solution (20 mL). The organic phase was dried over anhydrous sodium sulfate and filtered, and the filtrate was concentrated under reduced pressure to give compound L050-1 (1.42 g, crude product). LC-MS: [M-tBu+H]⁺ = 216.00.

### 65.2 Synthesis of compound L050-2

L050-1 (1.42 g, crude product) was dissolved in methanol (10 mL) and tetrahydrofuran (10 mL) at room temperature, and then triethylamine (529 mg, 5.23 mmol) and tris(triphenylphosphine)rhodium chloride (968 mg, 1.05 mmol) were added. The reaction liquid was purged with nitrogen and then purged with hydrogen. The reaction liquid was reacted for 72 h under hydrogen atmosphere. The reaction liquid was concentrated under reduced pressure, and the residue was added to a saturated aqueous sodium bicarbonate solution (15 mL). The mixture was adjusted to pH = 10 with an aqueous sodium hydroxide solution (1 M) and extracted with ethyl acetate (15 mL), and the organic phase was washed with a saturated aqueous sodium bicarbonate solution (15 mL). The organic phase was discarded. All aqueous phases were combined, and the pH was adjusted to 4-5 with diluted hydrochloric acid (1 M). The mixture was extracted with ethyl acetate (30 mL × 3). The combined organic phases were washed with a saturated aqueous sodium chloride solution (40 mL), dried over anhydrous sodium sulfate and filtered, and the filtrate was concentrated under reduced pressure to give compound L050-2 (1.1 g, crude product). LC-MS: [M-Boc+H]⁺ = 174.10.

### 65.3 Synthesis of compound L050-3

L050-2 (1.1 g, crude product) and L050-a (588 mg, 6.03 mmol) were dissolved in N,N-dimethylformamide (10 mL), and then O-(7-azabenzotriazol-1-yl)-N,N,N',N'-tetramethyluronium hexafluorophosphate (1.83 g, 4.82 mmol) and N,N-diisopropylethylamine (1.56 g, 12.06 mmol) were added. The reaction liquid was reacted at room temperature for 16 h. The reaction liquid was added with water (50 mL) for dilution and extracted with ethyl acetate (30 mL × 3). The combined organic phases were washed with a saturated aqueous sodium chloride solution (20 mL), dried over anhydrous sodium sulfate and filtered, and the filtrate was concentrated under reduced pressure. The residue was purified by silica gel column chromatography (petroleum ether/ethyl acetate = 2/1) to give compound L050-3 (800 mg). LC-MS: [M+H]⁺ = 317.05.

### 65.4 Synthesis of compound L050-4

L050-3 (700 mg, 2.21 mmol) was dissolved in anhydrous tetrahydrofuran (10 mL), and the reaction liquid was cooled to 0 °C under nitrogen atmosphere. L050-b (3.3 mL, 3.32 mmol, 1 M) was added dropwise, and then the reaction liquid was warmed to room temperature and reacted for 3 h. The reaction liquid was added to a saturated aqueous ammonium chloride solution (15 mL) to quench the reaction, and the mixture was extracted with ethyl acetate (15 mL × 3). The combined organic phases were washed with a saturated aqueous sodium chloride solution (20 mL), dried over anhydrous sodium sulfate and filtered, and the filtrate was concentrated under reduced pressure. The residue was purified by silica gel column chromatography (petroleum ether/ethyl acetate = 4/1) to give compound L050-4 (144 mg). LC-MS: [M-Boc+H]⁺ = 252.05.

### 65.5 Synthesis of compound L050-5

L050-4 (130 mg, 0.37 mmol) was added to bis(2-methoxyethyl)aminosulfur trifluoride (5 mL), and the reaction liquid was reacted at 90 °C for 6 h under nitrogen atmosphere. The reaction liquid was cooled to room temperature and added dropwise to a saturated aqueous sodium bicarbonate solution (20 mL) that was cooled to 0 °C to quench the reaction, and the mixture was extracted with ethyl acetate (15 mL × 3). The combined organic phases were washed with a saturated aqueous sodium chloride solution (20 mL), dried over anhydrous sodium sulfate and filtered, and the filtrate was concentrated under reduced pressure. The residue was purified by silica gel column chromatography (petroleum ether/ethyl acetate = 3/1) to give compound L050-5 (80 mg). LC-MS: [M-tBu+H]⁺ = 317.95.

### 65.6 Synthesis of compound L050-6

L050-5 (80 mg, 0.21 mmol) was dissolved in methanol (2 mL), tetrahydrofuran (2 mL) and water (1 mL), and then lithium hydroxide monohydrate (27 mg, 0.64 mmol) was added. The reaction liquid was reacted at room temperature for 5 h. The reaction liquid was concentrated under reduced pressure, and the residue was poured into water (5 mL). The pH was adjusted to 4-5 with an aqueous diluted hydrochloric acid solution (1 M), and the mixture was extracted with ethyl acetate (10 mL × 3). The combined organic phases were washed with a saturated aqueous sodium chloride solution (10 mL), dried over anhydrous sodium sulfate and filtered, and the filtrate was concentrated under reduced pressure to give compound L050-6 (80 mg, crude product). LC-MS: [M-tBu+H]⁺ = 303.95.

### 65.7 Synthesis of compound L050

For the synthesis of compound L050 hydrochloride, reference was made to the synthesis method of compound L001-IS-01, and L001-8 was replaced with L050-6; the crude product obtained by reaction was purified by high performance liquid chromatography (0.1% hydrochloric acid condition) and then lyophilized to give compound L050 hydrochloride (42.5 mg).

¹H NMR (400 MHz, DMSO-*d₆*): δ 10.47 (s, 1H), 8.94 - 8.88 (m, 2H), 8.55 (s, 1H), 8.00 (s, 2H), 7.69 - 7.62 (m, 3H), 7.38 (t, *J* = 8.8 Hz, 2H), 6.82 (d, *J* = 8.8 Hz, 1H), 5.18 - 5.12 (m, 1H), 4.37 - 4.20 (m, 2H), 3.26 (s, 1H), 3.10 - 2.99 (m, 1H), 2.96 - 2.85 (m, 1H), 2.47 - 2.44 (m, 1H), 2.41 - 2.39(m, 3H), 2.03 - 1.73 (m, 2H), 1.24 (t, *J =* 5.6 Hz, 3H). LC-MS: [M+H]⁺ = 462.00.

### Example 66: Synthesis of (2R,4S)-N-((2S)-1-((3-amino-6,7-dihydro-5H-cyclopenta[c]pyridin-7-yl) amino)-1-oxopropan-2-yl)-4-(4-fluorobenzyl)pyrrolidine-2-carboxamide (Compound L051) Hydrochloride

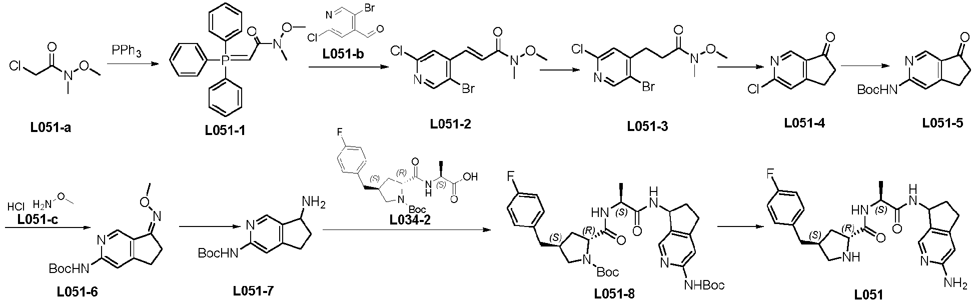

### 66.1 Synthesis of compound L051-1

L051-a (5 g, 36.35 mmol) and triphenylphosphine (9.5 g, 36.35 mmol) were dissolved in toluene (100 mL) at room temperature. The reaction liquid was purged with nitrogen, warmed to 80 °C and reacted for 16 h, and a large amount of white solid was precipitated. The reaction liquid was cooled to room temperature and filtered, and the filter cake was washed with toluene (20 mL) and dried under reduced pressure to give compound L051-1 (6.7 g, yield: 50.72%). LC-MS: [M+H]⁺ = 363.95.

### 66.2 Synthesis of compound L051-2

L051-1 (6.7 g, 18.44 mmol) and L051-b (4.1 g, 18.44 mmol) were dissolved in dichloromethane (100 mL), and the reaction liquid was reacted at room temperature for 16 h. The reaction liquid was concentrated under reduced pressure, and the residue was purified by silica gel column chromatography (petroleum ether/ethyl acetate = 3/1) to give compound L051-2 (3.5 g, yield: 62.17%). LC-MS: [M+H]⁺ = 306.85.

### 66.3 Synthesis of compound L051-3

L051-2 (3.5 g, 11.45 mmol) was dissolved in ethyl acetate (50 mL), and then rhodium/aluminum oxide coordination complex (2 g) was added. The reaction liquid was purged with hydrogen and reacted at room temperature for 5 days under hydrogen atmosphere. The reaction liquid was filtered, and the filtrate was concentrated under reduced pressure. The residue was purified by silica gel column chromatography (petroleum ether/ethyl acetate = 3/1) to give compound L051-3 (3.5 g, yield: 99.43%). LC-MS: [M+H]⁺ = 308.85.

### 66.4 Synthesis of compound L051-4

L051-3 (3.5 g, 11.44 mmol) was dissolved in tetrahydrofuran (50 mL), and the reaction system was cooled to -70 °C. n-Butyllithium (14 mL, 22.4 mmol, 1.6 M in n-hexane) was added dropwise and slowly. After the dropwise addition was completed, the reaction liquid was reacted at -70 °C for another 2 h. The reaction liquid was quenched by adding a saturated aqueous ammonium chloride solution (20 mL) and extracted with ethyl acetate (50 mL × 3). The organic phases were combined, dried over anhydrous sodium sulfate and filtered, and the filtrate was concentrated under reduced pressure. The residue was purified by silica gel column chromatography (petroleum ether/ethyl acetate = 3/1) to give compound L051-4 (1.2 g, yield: 62.83%). LC-MS: [M+H]⁺ = 168.05.

### 66.5 Synthesis of compound L051-5

L051-4 (1.2 g, 7.16 mmol) and tert-butyl carbamate (1.7 g, 14.32 mmol) were dissolved in 1,4-dioxane (50 mL) at room temperature, and then palladium(II) acetate (161 mg, 0.72 mmol), 2-dicyclohexylphosphine-2',4',6'-triisopropylbiphenyl (683 mg, 1.43 mmol), and cesium carbonate (4.7 g, 14.32 mmol) were added. The reaction liquid was reacted at 80 °C for 16 h under nitrogen atmosphere. The reaction liquid was cooled to room temperature, poured into water (50 mL) for dilution, and extracted with ethyl acetate (50 mL × 3). The organic phases were combined, dried over anhydrous sodium sulfate and filtered, and the filtrate was concentrated under reduced pressure. The residue was purified by silica gel column chromatography (petroleum ether/ethyl acetate = 3/1) to give compound L051-5 (1.2 g, yield: 67.42%). LC-MS: [M+H]⁺ = 249.05.

### 66.6 Synthesis of compound L051-6

L051-5 (1.17 g, 4.71 mmol) was dissolved in ethanol (20 mL) and water (10 mL), and then sodium acetate (590 mg, 7.07 mmol) and L051-c (580 mg, 7.07 mmol) were added. The reaction liquid was reacted at room temperature for 16 h. The reaction liquid was poured into water (20 mL) for dilution and extracted with ethyl acetate (20 mL × 3). The organic phases were combined, dried over anhydrous sodium sulfate and filtered, and the filtrate was concentrated under reduced pressure. The residue was purified by silica gel column chromatography (petroleum ether/ethyl acetate = 2/1) to give compound L051-6 (600 mg, yield: 45.80%). LC-MS: [M+H]⁺ = 278.05.

### 66.7 Synthesis of compound L051-7

L051-6 (600 mg, 2.16 mmol) was dissolved in methanol (20 mL) and aqueous ammonia (2 mL), and then Raney nickel (100 mg) was added. The reaction liquid was reacted for 16 h under hydrogen atmosphere. The reaction liquid was filtered through celite, and the filtrate was concentrated under reduced pressure to give compound L051-7 (450 mg, crude product), which was used directly in the next step. LC-MS: [M+H]⁺ = 250.05.

### 66.8 Synthesis of compound L051-8

L051-7 (200 mg, 0.53 mmol) was dissolved in dichloromethane (8 mL), and then N,N-diisopropylethylamine (194 mg, 1.53 mmol) was added. The reaction liquid was reacted at room temperature for 10 min, and then the above reaction system was added to a solution of L034-2 (154 mg, 0.61 mmol) and 1-propylphosphoric anhydride (647 mg, 1.0 mmol, 50% in ethyl acetate) in dichloromethane (8 mL). The reaction liquid was reacted at room temperature for another 1 h. The reaction liquid was added with a saturated aqueous sodium chloride solution (20 mL) for dilution, and the mixture was extracted with dichloromethane (20 mL × 3). The organic phases were combined, dried over anhydrous sodium sulfate and filtered, and the filtrate was concentrated under reduced pressure. The residue was purified by silica gel column chromatography (petroleum ether/ethyl acetate = 1/1) to give compound L051-8 (250 mg, yield: 43.31%). LC-MS: [M+H]⁺ = 626.10.

### 66.9 Synthesis of compound L051 hydrochloride

L051-8 (230 mg, 0.35 mmol) was dissolved in dichloromethane (2 mL), and then a solution of hydrogen chloride in 1,4-dioxane (2 mL, 4 M) was added. The reaction liquid was reacted at room temperature for 2 h. The reaction liquid was concentrated under reduced pressure, and the crude product was purified by high performance liquid chromatography (0.1% hydrochloric acid condition) to give compound L051 hydrochloride (98.5 mg, yield: 53.37%). LC-MS: [M+H]⁺ = 426.05.

¹H NMR (400 MHz, DMSO-*d₆*): δ 10.42 (s, 1H), 8.94 - 8.89 (m, 1H), 8.71 - 8.41 (m, 2H), 8.05 - 8.02 (m, 2H), 7.72 - 7.68 (m, 1H), 7.28 (dd, *J* = 8.4, 5.6 Hz, 2H), 7.11 (t, *J* = 8.8 Hz, 2H), 6.85 (s, 1H), 5.12 (q, *J* = 7.6 Hz, 1H), 4.40 - 4.35 (m, 1H), 4.23 - 4.21 (m, 1H), 3.33 - 3.19 (m, 1H), 3.03 - 2.96 (m, 1H), 2.92 - 2.78 (m, 2H), 2.74 - 2.62 (m, 2H), 2.47 - 2.39 (m, 1H), 2.38 - 2.26 (m, 1H), 2.09 - 2.01 (m, 1H), 1.95 - 1.83 (m, 2H), 1.22 (d, *J =* 7.2 Hz, 3H).

### Example 67: Synthesis of (2R,4S)-N-((2S)-1-((2-amino-3-methyl-6,7-dihydro-5H-cyclopenta[b] pyridin-5-yl)amino)-1-oxopropan-2-yl)-4-(4-fluorophenyl)piperidine-2-carboxamide (Compound L052) Hydrochloride

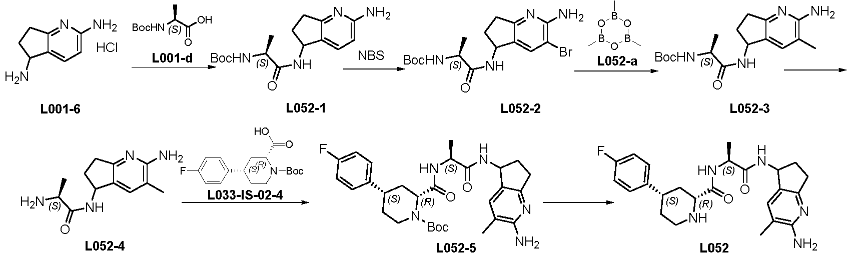

### 67.1 Synthesis of compound L052-1

L001-d (1.5 g, 8.08 mmol) was dissolved in N,N-dimethylformamide (20 mL), and then 1-(3-dimethylaminopropyl)-3-ethylcarbodiimide hydrochloride (1.7 g, 8.89 mmol) and 1-hydroxybenzotriazole (1.2 g, 8.89 mmol) were added sequentially. The reaction liquid was reacted at 25 °C for 10 min, and then a solution of N,N-diisopropylethylamine (5.2 g, 40.40 mmol) and L001-6 (1.5 g, 8.08 mmol) in N,N-dimethylformamide (10 mL) was added. The reaction liquid was reacted at 25 °C for another 30 min. The reaction liquid was poured into water (30 mL) and extracted with ethyl acetate (30 mL × 3). The organic phase was washed with a saturated aqueous ammonium chloride solution (20 mL × 3), dried over anhydrous sodium sulfate and filtered, and the filtrate was concentrated under reduced pressure. The residue was purified by silica gel column chromatography to give compound L052-1 (1.5 g).

### 67.2 Synthesis of compound L052-2

L052-1 (200 mg, 0.66 mmol) was dissolved in acetonitrile (5 mL), and then N-bromosuccinimide (134 mg, 0.75 mmol) was added. The reaction liquid was reacted at room temperature for 2 h. The reaction liquid was added with water (10 mL) for dilution and extracted with dichloromethane (10 mL × 3). The combined organic phases were dried over anhydrous sodium sulfate and filtered, and the filtrate was concentrated under reduced pressure. The residue was purified by silica gel column chromatography (methanol/dichloromethane = 1/20) to give compound L052-2 (160 mg, yield: 60%). LC-MS: [M+H]⁺ = 398.95.

### 67.3 Synthesis of compound L052-3

L052-2 (180 mg, 0.45 mmol) and trimethylboroxine (282 mg, 2.25 mmol) were dissolved in N,N-dimethylformamide (5 mL) and water (0.1 mL) at room temperature, and then potassium carbonate (187 mg, 1.35 mmol) and [1,1'-bis(diphenylphosphino)ferrocene]dichloropalladium(II) (33 mg, 0.045 mmol) were added. The reaction liquid was purged with nitrogen three times and reacted at 110 °C for 16 h. The reaction liquid was cooled to room temperature, added with water (30 mL) for dilution, and extracted with ethyl acetate (30 mL × 3). The organic phases were combined, dried over anhydrous sodium sulfate and filtered, and the filtrate was concentrated under reduced pressure. The residue was purified by silica gel column chromatography (methanol/dichloromethane = 1/10) to give compound L052-3 (130 mg, yield: 86.7%). LC-MS: [M+H]⁺ = 335.10.

### 67.4 Synthesis of compound L052-4

L052-3 (130 mg, 0.39 mmol) was dissolved in dichloromethane (1 mL), and then a solution of hydrogen chloride in 1,4-dioxane (2 mL, 4 M) was added. The reaction liquid was reacted at room temperature for 1 h. The reaction liquid was concentrated under reduced pressure to give compound L052-4 (crude product), which was used directly in the next step. LC-MS: [M+H]⁺ = 235.15.

### 67.5 Synthesis of compound L052 hydrochloride

For the synthesis of compound L052, reference was made to the synthesis method of compound L001, L001-11 was replaced with L033-IS-02-4, and L001-7 was replaced with L052-4; the crude product obtained by reaction was purified by high performance liquid chromatography (0.1% hydrochloric acid condition) and then lyophilized to give compound L052 hydrochloride (8.3 mg).

¹H NMR (400 MHz, DMSO-*d₆*): δ 9.59 - 9.42 (m, 1H), 8.92 - 8.69 (m, 2H), 8.54 - 8.48 (m, 1H), 7.70 (s, 2H), 7.58 (d, *J* = 8.0 Hz, 1H), 7.27 (d, *J* = 8.0 Hz, 2H), 7.17 - 7.15 (m, 2H), 5.19 - 5.14 (m, 1H), 4.28 (m, 1H), 3.94 (s, 1H), 3.08 - 3.03 (m, 2H), 2.91 - 2.89(m, 2H), 2.44 - 2.40 (m, 1H), 2.32 - 2.30 (m, 1H), 2.15 (d, *J* = 8.0 Hz, 2H), 2.04 - 1.61 (m, 5H), 1.24 (d, *J* = 8.0 Hz, 4H). LC-MS: [M+H]⁺ = 440.05.

### Example 68: Synthesis of (2R,4S)-N-((2S)-1-((2-amino-3-chloro-6,7-dihydro-5H-cyclopenta[b] pyridin-5-yl)amino)-1-oxopropan-2-yl)-4-(4-fluorobenzyl)pyrrolidine-2-carboxamide (Compound L053) Hydrochloride

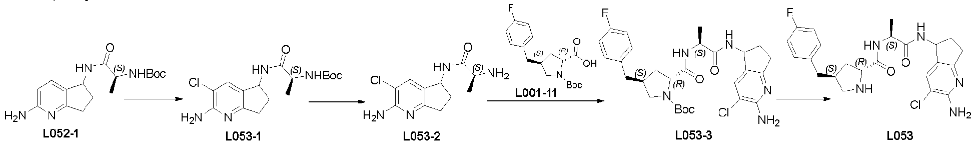

### 68.1 Synthesis of compound L053-1

L052-1 (400 mg, 1.2 mmol) was dissolved in acetonitrile (10 mL), and then N-chlorosuccinimide (250 mg, 1.8 mmol) was added. The reaction liquid was reacted at room temperature for 3 h. The reaction liquid was added with water (20 mL) for dilution and extracted with dichloromethane (20 mL × 3). The organic phases were combined, dried over anhydrous sodium sulfate and filtered, and the filtrate was concentrated under reduced pressure. The residue was purified by high performance liquid chromatography (0.1% aqueous ammonia condition) and then lyophilized to give compound L053-1 (90 mg, yield: 21%). LC-MS: [M+H]⁺ = 355.05.

### 68.2 Synthesis of compound L053-2

L053-1 (90 mg, 0.23 mmol) was dissolved in dichloromethane (2 mL), and then a solution of hydrogen chloride in 1,4-dioxane (4 M, 4 mL) was added. The reaction liquid was reacted at room temperature for 2 h. The reaction liquid was concentrated under reduced pressure to give compound L053-2 hydrochloride (67 mg), which was used directly in the next step. LC-MS: [M+H]⁺ = 255.05.

### 68.3 Synthesis of compound L053 hydrochloride

For the synthesis of compound L053, reference was made to the synthesis method of compound L001, and L001-7 was replaced with L053-2; the crude product obtained by reaction was purified by high performance liquid chromatography (0.1% hydrochloric acid condition) and then lyophilized to give compound L053 hydrochloride (26.3 mg).

¹H NMR (400 MHz, DMSO-*d₆*): δ 10.13 (s, 1H), 8.81 (d, *J* = 8.0 Hz, 1H), 8.52 (t, *J* = 8.0 Hz, 2H), 7.66 (d, *J* = 12 Hz, 1H), 7.29 - 7.27 (m, 2H), 7.13 (t, *J* = 8.4 Hz, 2H), 5.13 (dd, *J* = 8.0, 4.0 Hz, 1H), 4.41 - 4.32 (m, 3H), 4.32 - 4.23 (m, 3H), 3.34 - 3.21 (m, 1H), 2.98 - 2.61 (m, 5H), 2.48 - 2.37 (m, 1H), 2.12 - 1.98 (m, 1H), 1.92 - 1.90 (m, 2H), 1.23 (d, *J =* 4.0 Hz, 3H). LC-MS: [M+H]⁺ = 460.00.

### Example 69: Synthesis of (2R)-N-((S)-1-(((R)-2-amino-6,7-dihydro-5H-cyclopenta[b]pyridin-5-yl) amino)-1-oxopropan-2-yl)-4-fluoro-4-(4-fluorophenyl)piperidine-2-carboxamide (Compound L054)

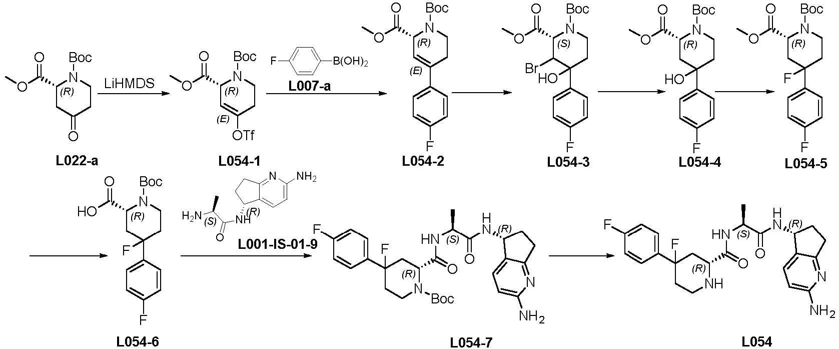

### 69.1 Synthesis of compound L054-1

L022-a (1.0 g, 3.85 mmol) was added to tetrahydrofuran (10 mL) at room temperature, and the reaction liquid was cooled to -78 °C. Lithium bis(trimethylsilyl)amide (4.2 mL, 4.2 mmol, 1 M in tetrahydrofuran) was added dropwise under nitrogen atmosphere, and after the dropwise addition was completed, the reaction liquid was stirred at -78 °C for 10 min. A solution of N-(5-chloro-2-pyridinyl)bis(trifluoromethanesulfonamide) (1.58 g, 4.04 mmol) in tetrahydrofuran (5 mL) was added at this temperature, and after the addition was completed, the reaction liquid was warmed to room temperature and stirred for another 1.5 h. The reaction liquid was quenched by adding a saturated aqueous ammonium chloride solution (20 mL) and extracted with ethyl acetate (20 mL × 3). The organic phase was dried over anhydrous sodium sulfate and filtered, and the filtrate was concentrated under reduced pressure. The residue was purified by silica gel column chromatography to give compound L054-1 (600 mg, crude product).

### 69.2 Synthesis of compound L054-2

L054-1 (2.9 g, 7.45 mmol), L007-a (2.1 g, 14.90 mmol), bis(triphenylphosphine)palladium(II) dichloride (523 mg, 0.74 mmol), and potassium carbonate (2.1 g, 14.90 mmol) were dissolved in tetrahydrofuran (50 mL) and water (10 mL). The reaction liquid was reacted at 40 °C for 3 h under nitrogen atmosphere. The reaction liquid was poured into water (30 mL) and extracted with ethyl acetate (30 mL × 3). The organic phases were combined, dried over anhydrous sodium sulfate and filtered, and the filtrate was concentrated under reduced pressure. The residue was purified by silica gel column chromatography (ethyl acetate/petroleum ether = 1/3) to give compound L054-2 (2.4 g, yield: 95.56%). LC-MS: [M+H]⁺ = 336.05.

### 69.3 Synthesis of compound L054-3

L054-2 (1.0 g, 2.98 mmol) was dissolved in acetone (10 m) and water (2 mL) at room temperature, and then a solution of N-bromosuccinimide (584 mg, 3.28 mmol) in acetone (10 mL) was added dropwise and slowly. After the dropwise addition was completed, the reaction liquid was reacted at room temperature for 16 h. The reaction liquid was poured into water (10 mL) and extracted with ethyl acetate (20 mL × 3). The organic phase was dried over anhydrous sodium sulfate and filtered, and the filtrate was concentrated under reduced pressure. The residue was purified by silica gel column chromatography (petroleum ether/ethyl acetate = 1/2) to give compound L054-3 (867 mg, yield: 67.18%). LC-MS: [M-Boc+H]⁺ = 331.85.

### 69.4 Synthesis of compound L054-4

L054-3 (1.2 g, 2.78 mmol) was dissolved in tetrahydrofuran (30 mL), and then sodium acetate (685 mg, 8.35 mmol) and palladium on carbon (100 mg, 10%) were added. The reaction liquid was reacted at room temperature for 16 h under hydrogen atmosphere. The reaction liquid was filtered through celite, and the filtrate was concentrated under reduced pressure. The residue was purified by high performance liquid chromatography (0.1% aqueous ammonia) and then lyophilized to give compound L054-4 (388 mg, yield: 39.55%). LC-MS: [M-Boc-OH]⁺ = 236.05.

### 69.5 Synthesis of compound L054-5

L054-4 (280 mg, 0.79 mmol) was dissolved in dichloromethane (10 mL) at room temperature, and the reaction liquid was cooled to -70 °C. Diethylaminosulfur trifluoride (153 mg, 0.95 mmol) was added dropwise, and after the dropwise addition was completed, the reaction liquid was warmed to room temperature and reacted for 5 h. The reaction liquid was quenched with a saturated aqueous sodium bicarbonate solution (10 mL) and extracted with dichloromethane (10 mL × 3). The organic phases were combined, dried over anhydrous sodium sulfate and filtered, and the filtrate was concentrated under reduced pressure to give compound L054-5 (300 mg, crude product), which was used directly in the next step. LC-MS: [M-Boc+H]⁺ = 256.05.

### 69.6 Synthesis of compound L054-6

L054-5 (280 mg, 0.79 mmol) was dissolved in tetrahydrofuran (5 mL), methanol (2 mL) and water (2 mL), and then lithium hydroxide monohydrate (99 mg, 2.36 mmol) was added. The reaction liquid was reacted at room temperature for 1 h. The reaction liquid was adjusted to pH = 3-4 with diluted hydrochloric acid (1 N), and the mixture was extracted with dichloromethane (10 mL × 3). The organic phases were combined, dried over anhydrous sodium sulfate and filtered, and the filtrate was concentrated under reduced pressure. The residue was separated and purified by high performance liquid chromatography (0.1% aqueous ammonia) and then lyophilized to give compound L054-6 (89 mg, yield: 33.09%). LC-MS: [M-Boc+H]⁺ = 242.05.

### 69.7 Synthesis of compound L054

For the synthesis of compound L054, reference was made to the synthesis method of compound L001-IS-01, and L001-8 was replaced with L054-6; the crude product obtained by reaction was purified by high performance liquid chromatography (0.1% aqueous ammonia condition) and then lyophilized to give compound L054 (12.3 mg, yield: 21.54%).

¹H NMR (400 MHz, DMSO-*d₆*): δ 8.13 (d, *J* = 8.4 Hz, 1H), 7.93 (s, 1H), 7.45 - 7.41 (m, 2H), 7.26 - 7.14 (m, 3H), 6.22 (d, *J =* 8.0 Hz, 1H), 5.81 (s, 2H), 5.08 (dd, *J =* 14.4, 7.6 Hz, 1H), 4.25 - 4.18 (m, 1H), 3.50 (d, *J =* 11.6 Hz, 1H), 3.05 - 3.01 (m, 1H), 2.91 - 2.85 (m, 1H), 2.76 - 2.67 (m, 1H), 2.65 - 2.56 (m, 1H), 2.35 - 2.27 (m, 1H), 2.14 - 1.64 (m, 5H), 1.21 (d, *J* = 10.0 Hz, 3H). LC-MS: [M+H]⁺ = 444.05.

### Example 70: Synthesis of (1R,2S,5S)-N-((R)-2-amino-6,7-dihydro-5H-cyclopenta[b]pyridin-5-yl)-3-((2R,4S)-4-(4-fluorobenzyl)pyrrolidine-2-carbonyl)-3-azabicyclo[3.1.0]hexane-2-carboxamide (Compound L055-IS-01 or L055-IS-02) and (1S,2R,5R)-N-((R)-2-amino-6,7-dihydro-5H-cyclopenta [b]pyridin-5-yl)-3-((2R,4S)-4-(4-fluorobenzyl)pyrrolidine-2-carbonyl)-3-azabicyclo[3.1.0]hexane-2-carboxamide (Compound L055-IS-02 or L055-IS-01)

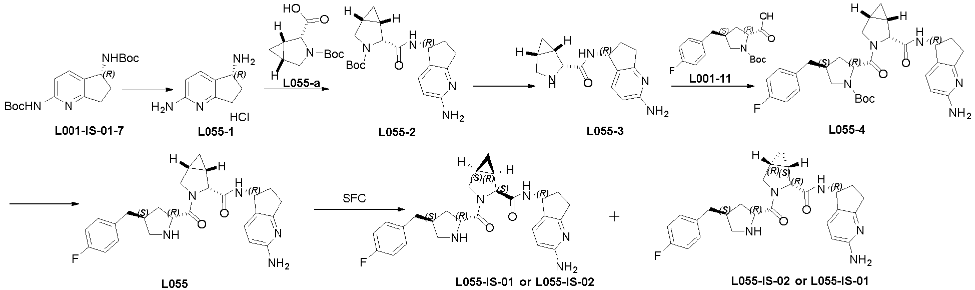

### 70.1 Synthesis of compound L055-1

L001-IS-01-7 (27.0 g, 77.3 mmol) was dissolved in dichloromethane (300 mL), and then a solution of hydrogen chloride in 1,4-dioxane (150 mL, 4 M) was added. The reaction liquid was reacted at room temperature for 16 h. The reaction liquid was concentrated under reduced pressure, and the residue was slurried with dichloromethane (100 mL) for 1 h and then filtered. The filter cake was dried under vacuum to give compound L055-1 (18.0 g). LC-MS: [M+H]⁺ = 150.15.

### 70.2 Synthesis of compound L055-2

L055-a (100 mg, 0.44 mmol) was dissolved in dichloromethane (3 mL), and then 1-(3-dimethylaminopropyl)-3-ethylcarbodiimide hydrochloride (101 mg, 0.53 mmol), 1-hydroxybenzotriazole (71 mg, 0.53 mmol), N,N-diisopropylethylamine (284 mg, 2.20 mmol), and L055-1 (163 mg, 0.88 mmol) were added. The reaction liquid was reacted at room temperature for 2 h. The reaction liquid was poured into water (10 mL) and extracted with dichloromethane (20 mL × 3). The organic phases were combined, dried over anhydrous sodium sulfate and filtered, and the filtrate was concentrated under reduced pressure. The residue was purified by silica gel column chromatography (dichloromethane/methanol = 10/1) to give compound L055-2 (150 mg, yield: 95.10%). LC-MS: [M+H]⁺ = 359.05.

### 70.3 Synthesis of compound L055-3

L055-2 (150 mg, 0.42 mmol) was dissolved in dichloromethane (2 mL), and then a solution of hydrogen chloride in 1,4-dioxane (2 mL, 4 M) was added. The reaction liquid was reacted at room temperature for 2 h. The reaction liquid was concentrated under reduced pressure to give compound L055-3 hydrochloride (crude product), which was used directly in the next step. LC-MS: [M+H]⁺ = 259.10.

### 70.4 Synthesis of compounds L055-IS-01 and L055-IS-02

For the synthesis of compound L055, reference was made to the synthesis method of compound L001, and L001-7 was replaced with L055-3; the crude product obtained by reaction was purified by high performance liquid chromatography (0.1% hydrochloric acid condition) and then lyophilized to give compound L055 hydrochloride (80 mg), and L055 hydrochloride (80 mg) was separated by preparative normal phase chromatography (basic condition) to give compounds L055-IS-01 (33 mg, crude product, retention time Rt = 12.462 min) and L055-IS-02 (30.1 mg, retention time Rt = 13.912 min). Compound L055-IS-01 was purified by high performance liquid chromatography (0.1% hydrochloric acid) and then lyophilized to give compound L055-IS-01 hydrochloride (6.8 mg).
L055-IS-01 (retention time Rt = 13.912 min):
   ¹H NMR (400 MHz, DMSO-*d₆*): δ 10.07 (s, 1H), 8.59 - 8.35 (m, 1H), 8.36 (d, *J* = 8.0 Hz, 1H), 7.99 (s, 2H), 7.63 (d, *J* = 8.8 Hz, 1H), 7.30 - 7.21 (m, 2H), 7.13 (t, *J* = 8.8 Hz, 2H), 6.81 (d, *J* = 8.8 Hz, 1H), 5.13 (dd, *J* = 14.4, 7.6 Hz, 1H), 4.50 - 4.44 (m, 1H), 4.40 (d, *J =* 5.6 Hz, 1H), 3.82 (dd, *J* = 12.4, 5.2 Hz, 1H), 3.52 (s, 1H), 3.28 - 3.20 (m, 1H), 3.06 - 2.79 (m, 3H), 2.76 - 2.67 (m, 2H), 2.47 - 2.36 (m, 2H), 2.14 - 2.03 (m, 1H), 2.00 - 1.82 (m, 3H), 1.80 - 1.73 (m, 1H), 0.73 - 0.61 (m, 2H). LC-MS: [M+H]⁺ = 464.00.
L055-IS-02 (retention time Rt = 12.462 min):
   ¹H NMR (400 MHz, DMSO-*d₆*): δ 8.34 (s, 1H), 7.90 (d, *J =* 4.0 Hz, 1H), 7.22 - 7.08 (m, 4H), 5.80 (s, 2H), 5.10 (d, *J =* 8.0 Hz, 1H), 4.23 (s, 2H), 3.70 - 3.58 (m, 3H), 3.03 - 2.93 (m, 2H), 2.66 - 2.59 (m, 3H), 2.31 - 2.03 (m, 3H), 1.89 - 1.67 (m, 4H), 1.38 - 1.07 (m, 2H), 0.72 - 0.61 (m, 2H). LC-MS: [M+H]⁺ = 464.00.

### Example 71: Synthesis of (S)-N-((R)-2-amino-6,7-dihydro-5H-cyclopenta[b]pyridin-5-yl)-2-((2R,4S)-4-(4-fluorobenzyl)pyrrolidine-2-carbonyl)-2-azaspiro[3.3]heptane-1-carboxamide (L056) Hydrochloride

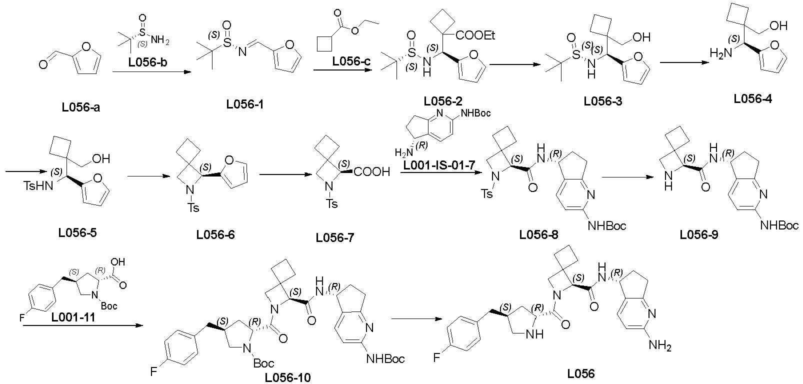

### 71.1 Synthesis of compound L056-1

L056-a (10 g, 104 mmol) and L056-b (18.92 g, 156 mmol) were dissolved in dichloromethane (100 mL), and then cesium carbonate (68 g, 208 mmol) was added. The reaction liquid was reacted at room temperature for 16 h. The reaction liquid was concentrated under reduced pressure, and the residue was purified by silica gel column chromatography (petroleum ether/ethyl acetate = 5/1) to give compound L056-1 (20 g, yield: 96%). LC-MS [M+H]⁺ = 200.05.

### 71.2 Synthesis of compound L056-2

L056-1 (9.0 g, 45 mmol) and L056-c (17 g, 135 mmol) were dissolved in anhydrous tetrahydrofuran (100 mL), and the mixture was cooled to -78 °C. Lithium bis(trimethylsilyl)amide (112 mL, 112 mmol, 1 M in tetrahydrofuran) was added dropwise. After the dropwise addition was completed, the reaction liquid was reacted at this temperature for another 3 h. The reaction liquid was poured into a saturated aqueous ammonium chloride solution (100 mL) to quench the reaction, and the mixture was extracted with ethyl acetate (100 mL × 3). The organic phases were combined and washed with saturated brine (100 mL). The organic phase was dried over anhydrous sodium sulfate and filtered, and the filtrate was concentrated under reduced pressure. The residue was purified by silica gel column chromatography (petroleum ether/ethyl acetate = 5/1) to give compound L056-2 (10 g, yield: 97.62%) as a colorless oil. LC-MS [M+H]⁺ = 328.

### 71.3 Synthesis of compound L056-3

L056-2 (10.0 g, 30.5 mmol) was dissolved in anhydrous tetrahydrofuran (100 mL), and then lithium aluminum hydride (2.32 g, 61.0 mmol) was added in batches at 0 °C. After the addition was completed, the reaction liquid was reacted at room temperature for 2 h. Water (2 mL), a sodium hydroxide solution (10%, 6 mL), and water (2 mL) were added sequentially to the reaction liquid to quench the reaction, and a flocculent solid was precipitated. The mixture was filtered, and the filtrate was concentrated under reduced pressure to give compound L056-3 (8 g, crude product), which could be used directly in the next step. LC-MS: [M+H]⁺ = 286.05.

### 71.4 Synthesis of compound L056-4

L056-3 (3.5 g, crude product) was dissolved in tetrahydrofuran (15 mL), and then a solution of hydrogen chloride in 1,4-dioxane (15 mL, 4 M) was added at 0 °C. After the addition was completed, the reaction liquid was warmed to room temperature and reacted for another 4 h. The reaction liquid was concentrated under reduced pressure to give compound L056-4 (3.0 g, crude product), which was used directly in the next step. LC-MS [M-OH+H]⁺ = 165.10.

### 71.5 Synthesis of compound L056-5

L056-4 (2.2 g, 12.26 mmol) was dissolved in tetrahydrofuran (20 mL), and then triethylamine (6.2 g, 61.31 mmol) and p-toluenesulfonyl chloride (2.8 g, 14.71 mmol) were added at 0 °C. After the addition was completed, the reaction liquid was reacted at room temperature for 16 h. The reaction liquid was added with water (20 mL) for dilution and extracted with ethyl acetate (20 mL × 3). The organic phases were combined and washed with saturated brine (20 mL). The organic phase was dried over anhydrous sodium sulfate and filtered, and the filtrate was concentrated under reduced pressure to give compound L056-5 (3.2 g, crude product). LC-MS: [M-OH+H]⁺ = 320.00.

### 71.6 Synthesis of compound L056-6

L056-5 (3.2 g, crude product) was dissolved in tetrahydrofuran (30 mL), and then sodium hydride (3.8 g, 96.47 mmol, 60% in mineral oil) and p-toluenesulfonyl chloride (2.7 g, 14.47 mmol) were added at 0 °C. After the addition was completed, the reaction liquid was reacted at room temperature for 16 h. The reaction liquid was quenched by adding water (20 mL) and extracted with ethyl acetate (20 mL × 3). The organic phases were combined and washed with saturated brine (20 mL). The organic phase was dried over anhydrous sodium sulfate and filtered, and the filtrate was concentrated under reduced pressure. The residue was purified by silica gel column chromatography (petroleum ether/ethyl acetate = 8/1) to give compound L056-6 (1.9 g, yield: 62.24%) as a light yellow oil. LC-MS: [M+H]⁺ = 317.95.

### 71.7 Synthesis of compound L056-7

Sodium periodate (1.2 g, 6.31 mmol) was added to ethyl acetate (2 mL), acetonitrile (3.2 mL), and water (1.6 mL), and then ruthenium trichloride (5.2 mg, 0.02 mmol) was added at 0 °C. The reaction liquid was reacted for 0.5 h, and then a solution of L056-6 (200 mg, 0.63 mmol) in ethyl acetate (2 mL) was added dropwise. The reaction liquid was reacted at 0 °C for another 3 h. The reaction liquid was added with water (10 mL) for dilution and extracted with ethyl acetate (20 mL × 3). The organic phases were combined and washed with saturated brine (20 mL). The organic phase was dried over anhydrous sodium sulfate and filtered, and the filtrate was concentrated under reduced pressure to give compound L056-7 (180 mg, crude product). LC-MS: [M+H]⁺ = 295.95.

### 71.8 Synthesis of compound L056-8

L056-7 (180 mg, 0.61 mmol) was dissolved in dichloromethane (2 mL), and then L001-IS-01-7 (152 mg, 0.61 mmol), N,N-diisopropylethylamine (236 mg, 1.83 mmol), and 1-propylphosphoric anhydride (581 mg, 0.91 mmol, 50% in ethyl acetate) were added. The reaction liquid was reacted at room temperature for 1 h. The reaction liquid was quenched by adding water (10 mL) and extracted with dichloromethane (10 mL × 3). The organic phases were combined and washed with a saturated aqueous sodium chloride solution (20 mL). The organic phase was dried over anhydrous sodium sulfate and filtered, and the filtrate was concentrated under reduced pressure. The residue was purified by silica gel column chromatography (dichloromethane/methanol = 20/1) to give compound L056-8. LC-MS: [M+H]⁺ = 527.00.

### 71.9 Synthesis of compound L056-9

L056-8 (500 mg, 0.95 mmol) was dissolved in methanol (2 mL), and then magnesium chips (456 mg, 19.01 mmol) were added at 0 °C. The reaction liquid was warmed to room temperature and reacted for 2 h. The reaction liquid was filtered, and the filtrate was concentrated under reduced pressure. The residue was added with water (10 mL) for dilution and extracted with dichloromethane (10 mL × 3). The organic phases were combined, dried over anhydrous sodium sulfate and filtered, and the filtrate was concentrated under reduced pressure to give compound L056-9 (240 mg, crude product). LC-MS: [M+H]⁺ = 373.05.

### 71.10 Synthesis of compound L056 hydrochloride

For the synthesis of compound L056, reference was made to the synthesis method of compound L001, and L001-7 was replaced with L056-9; the crude product obtained by reaction was purified by high performance liquid chromatography (0.1% hydrochloric acid condition) and then lyophilized to give compound L056 hydrochloride (9.9 mg).

¹H NMR (400 MHz, DMSO-*d₆*): δ 10.21 (s, 1H), 8.893 - 8.573 (m, 2H), 7.98 (s, 2H), 7.73 - 7.69 (m, 1H), 7.33 - 7.22 (m, 2H), 7.14 - 7.12 (m, 2H), 6.82 (d, *J* = 8.4 Hz, 1H), 5.21 - 5.19(m, 1H), 4.94 - 4.45 (m, 1H), 4.38 (s, 1H), 4.19 - 4.11(m, 2H), 3.96 - 3.816 (m, 1H), 3.25 (s, 1H), 3.15 - 2.97 (m, 1H), 2.91 - 2.89 (m, 2H), 2.77 - 2.64 (m, 2H), 2.23- 2.21 (m, 2H), 2.07 - 1.86 (m, 5H), 1.82 - 1.72 (m, 3H). LC-MS: [M+H]⁺ = 478.05.

### Example 72: Synthesis of (2S)-N-(2-amino-6,7-dihydro-5H-cyclopenta[b]pyridin-5-yl)-4,4-difluoro-1-((2R,4S)-4-(4-fluorobenzyl)pyrrolidine-2-carbonyl)pyrrolidine-2-carboxamide (Compound L057) Hydrochloride

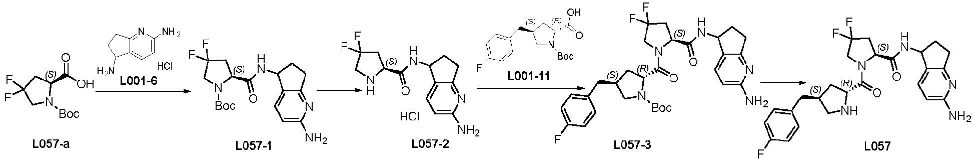

For the synthesis of compound L057, reference was made to the synthesis method of compound L001, and L001-d was replaced with L057-a; the crude product obtained by reaction was purified by high performance liquid chromatography (0.1% hydrochloric acid condition) and then lyophilized to give compound L057 hydrochloride (54.4 mg).

¹H NMR (400 MHz, DMSO-*d₆*): δ 10.17 (s, 1H), 8.69 (s, 2H), 7.95 (s, 2H), 7.65 (dd, *J* = 8.8, 6.4 Hz, 1H), 7.28 (dt, *J =* 13.6, 7.2 Hz, 2H), 7.13 (dd, *J =* 18.0, 9.2 Hz, 2H), 6.80 (d, *J =* 8.4 Hz, 1H), 5.28 - 4.96 (m, 1H), 4.60 (s, 1H), 4.51 (ddd, *J* = 10.8, 7.2, 3.6 Hz, 1H), 4.18 (dd, *J* = 22.4, 12.4 Hz, 1H), 4.00 (d, *J* = 11.6 Hz, 1H), 3.29 (s, 1H), 3.01 (s, 1H), 2.97 - 2.81 (m, 3H), 2.74 (dd, *J* = 18.8, 10.4 Hz, 2H), 2.45 (d, *J =* 4.0 Hz, 3H), 2.20 - 1.73 (m, 3H). LC-MS: [M+H]⁺ = 488.05.

### Example 73: Synthesis of (2R,4S)-N-((2S)-1-((2-amino-6,7-dihydro-5H-cyclopenta[b]pyridin-5-yl) amino)-1-oxopropan-2-yl)-4-((2-fluoropyridin-4-yl)methyl)pyrrolidine-2-carboxamide (Compound L058) Hydrochloride

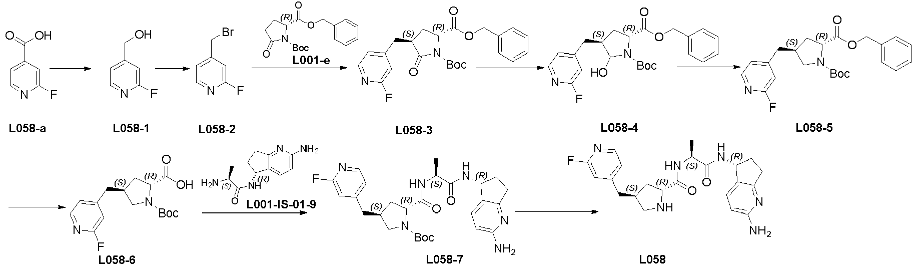

### 73.1 Synthesis of compound L058-1

L058-a (5 g, 35.4 mmol) was dissolved in tetrahydrofuran (55 mL), and the mixture was cooled to 0 °C. Lithium aluminum hydride (1.73 g, 44.3 mmol) was added in batches under nitrogen atmosphere, and the reaction liquid was warmed to room temperature and reacted for 1 h. The reaction liquid was quenched by adding an aqueous sodium hydroxide solution (15%, 1.7 mL) and water (1.7 mL), and then anhydrous sodium sulfate (6 g) was added. The reaction liquid was stirred at room temperature for 10 min. The reaction liquid was filtered, and the filtrate was concentrated under reduced pressure. The residue was purified by silica gel column chromatography (ethyl acetate/petroleum ether = 1/5) to give compound L058-1 (2.4 g, yield: 53.3%). LC-MS: [M+H]⁺ = 128.1.

### 73.2 Synthesis of compound L058-2

L058-1 (2.4 g, 18.8 mmol) was dissolved in dichloromethane (30 mL), and then phosphorus tribromide (6.29 g, 23.2 mmol) was added dropwise at 0 °C under nitrogen atmosphere. After the addition was completed, the reaction liquid was warmed and reacted for 1 h. A saturated aqueous sodium bicarbonate solution (30 mL) was added to the reaction liquid at 0 °C, and the mixture was extracted with dichloromethane (20 mL × 3). The organic phases were combined, dried over anhydrous sodium sulfate and filtered, and the filtrate was concentrated under reduced pressure. The residue was purified by silica gel column chromatography (ethyl acetate/petroleum ether = 1/10) to give compound L058-2 (2.6 g, yield: 53.3%). LC-MS: [M+H]⁺ = 189.95.

### 73.3 Synthesis of compound L058 hydrochloride

For the synthesis of compound L058, reference was made to the synthesis method of compound L001, L001-f was replaced with L058-2, and L001-7 was replaced with L001-IS-01-9; the crude product obtained by reaction was purified by high performance liquid chromatography (0.1% hydrochloric acid condition) and then lyophilized to give compound L058 hydrochloride (50.3 mg, yield: 34.3%). ¹H NMR (400 MHz, DMSO-*d₆*): δ 10.63 (s, 1H), 8.90 - 8.88 (m, 1H), 8.60 (d, *J* = 8.0 Hz, 2H), 8.16 (d, *J* = 5.2 Hz, 1H), 8.07 (s, 1H), 7.71 (d, *J* = 8.8 Hz, 1H), 7.30 - 7.23 (m, 1H), 7.15 - 7.08 (m, 1H), 6.85 (d, *J* = 8.8 Hz, 1H), 5.16 - 5.14 (m, 1H), 4.45 - 4.37 (m, 1H), 4.27 - 4.25 (m, 1H), 3.30 (d, *J =* 6.4 Hz, 1H), 3.08-3.01 (m, 1H), 2.95 - 2.75 (m, 5H), 2.59 - 2.56 (m, 1H), 2.47-2.41 (m, 1H), 2.17 - 2.06 (m, 1H), 2.04 - 1.89 (m, 2H), 1.23 (d, *J =* 7.2 Hz, 3H). LC-MS: [M+H]⁺ = 427.05.

### Example 74: Synthesis of N-((S)-1-(((R)-2-amino-6,7-dihydro-5H-cyclopenta[b]pyridin-5-yl) amino)-1-oxopropan-2-yl)-4-(4-fluorophenyl)-5-oxopiperazine-2-carboxamide (Compound L059) Hydrochloride

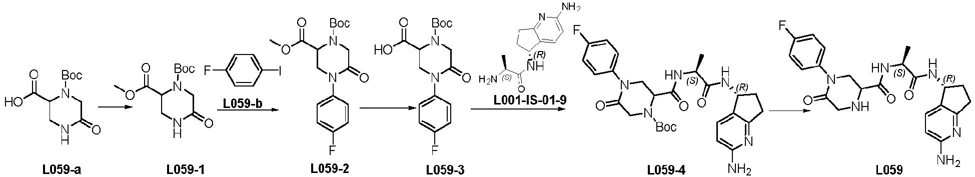

### 74.1 Synthesis of compound L059-1

L059-a (1.0 g, 4.09 mmol) was dissolved in tetrahydrofuran (10 mL) and methanol (10 mL), and the mixture was cooled to -78 °C. (Trimethylsilyl)diazomethane in n-hexane (8.2 mL, 16.36 mmol, 2 M) was added dropwise. After the addition was completed, the reaction liquid was warmed to 0 °C and reacted for 2 h. The reaction liquid was concentrated under reduced pressure, and the residue was stirred in methyl tert-butyl ether/petroleum ether (1/1, 10 mL) for 2 h. The mixture was filtered and dried under reduced pressure to give compound L059-1 (780 mg, yield: 73.58%). LC-MS: [M-tBu+H]⁺ = 203.05.

### 74.2 Synthesis of compound L059-2

L059-1 (320 mg, 1.24 mmol) and L059-b (330 mg, 1.49 mmol) were dissolved in dry toluene (10 mL), and then cesium carbonate (807 mg, 2.47 mmol), copper(I) iodide (22.4 mg, 0.12 mmol), and N,N'-dimethylethylenediamine (22 mg, 0.25 mmol) were added. The reaction liquid was reacted at 100 °C for 3 h under nitrogen atmosphere. The reaction liquid was poured into water (20 mL) and extracted with ethyl acetate (15 mL × 3). The organic phases were combined and washed with saturated brine (20 mL). The organic phase was dried over anhydrous sodium sulfate and filtered, and the filtrate was concentrated under reduced pressure. The residue was purified by silica gel column chromatography (petroleum ether/ethyl acetate = 3/1) to give compound L059-2 (250 mg, yield: 57.21%). LC-MS: [M+H]⁺ = 353.00.

### 74.3 Synthesis of compound L059-3

L059-2 (250 mg, 0.71 mmol) was dissolved in tetrahydrofuran (4 mL), water (4 mL) and methanol (2 mL), and then lithium hydroxide monohydrate (119 mg, 2.84 mmol) was added. The reaction liquid was reacted at room temperature for 10 h. The reaction liquid was added with water (10 mL) for dilution, adjusted to pH = 4-5 with diluted hydrochloric acid (1 M), and extracted with ethyl acetate (20 mL × 3). The combined organic phases were dried over anhydrous sodium sulfate and filtered, and the filtrate was concentrated under reduced pressure to give compound L059-3 (200 mg, yield: 83.33%), LC-MS: [M+H]⁺ = 339.00.

### 74.4 Synthesis of compound L059-4

L059-3 (150 mg, 0.44 mmol) was dissolved in dichloromethane (5 mL), and then 1-propylphosphoric anhydride (282 mg, 0.67 mmol, 50% in ethyl acetate), N,N-diisopropylethylamine (229 mg, 1.77 mmol), and L001-IS-01-9 (148 mg, 0.58 mmol) were added. The reaction liquid was reacted at room temperature for 2 h. The reaction liquid was added with water (10 mL) for dilution and extracted with dichloromethane (10 mL × 3). The combined organic phases were dried over anhydrous sodium sulfate and filtered, and the filtrate was concentrated under reduced pressure. The residue was purified by silica gel column chromatography (petroleum ether/ethyl acetate = 3/1) to give compound L059-4 (130 mg, yield: 50.0%). LC-MS: [M+H]⁺ = 541.05.

### 74.5 Synthesis of compound L059 hydrochloride

L059-4 (130 mg, 0.20 mmol) was dissolved in dichloromethane (3 mL), and then a solution of hydrogen chloride in ethyl acetate (3 mL, 1 M) was added. The reaction liquid was reacted at room temperature for 0.5 h. The reaction liquid was concentrated under reduced pressure, and the residue was purified by high performance liquid chromatography (0.1% hydrochloric acid condition) and then lyophilized to give compound L059 hydrochloride (40.1 mg, yield: 43.94%).

¹H NMR (400 MHz, DMSO-*d₆*): δ 10.23 (s, 1H), 9.14 - 9.04(m, 1H), 8.57 (t, *J =* 7.2 Hz, 1H), 8.00 (s, 1H), 7.73 - 7.61 (m, 1H), 7.40 - 7.24 (m, 4H), 6.85 - 6.76 (m, 1H), 5.19 - 5.05 (m, 1H), 4.62 - 4.52 (m, 1H), 4.29 - 4.13 (m, 2H), 4.03 - 3.91 (m, 2H), 3.88 - 3.77 (m, 1H), 3.33 - 3.23 (m, 1H), 3.10 - 2.84 (m, 2H), 2.44 - 2.39 (m, 1H), 2.00 - 1.81 (m, 1H), 1.29 - 1.19 (m, 3H). LC-MS: [M+H]⁺ = 441.00.

### Example 75: Synthesis of (2R)-N-(2S)-1-(2-amino-6,7-dihydro-5H-cyclopenta[b]pyridin-5-yl)amino)-1-oxopropan-2-yl)-4-(4-fluorobenzyl)piperazine-2-carboxamide (Compound L060) Hydrochloride

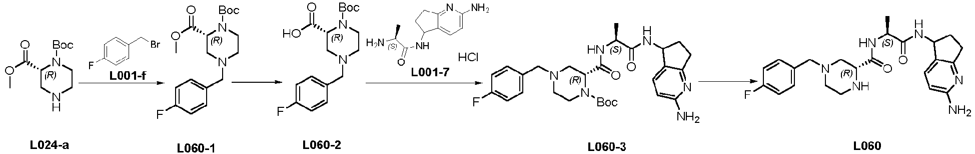

### 75.1 Synthesis of compound L060-1

L024-a (200 mg, 0.82 mmol) was dissolved in N,N-dimethylformamide (3 mL), and then N,N-diisopropylethylamine (159 mg, 1.23 mmol) and L001-f (163 mg, 0.86 mmol) were added. The reaction liquid was reacted at room temperature for 1 h. The reaction liquid was added with water (10 mL) for dilution and extracted with ethyl acetate (20 mL × 3). The organic phases were combined and washed with a saturated aqueous ammonium chloride solution (30 mL). The organic phase was dried over anhydrous sodium sulfate and filtered, and the filtrate was concentrated under reduced pressure. The residue was purified by silica gel column chromatography to give compound L060-1 (240 mg, yield: 83.2%). LC-MS: [M+H]⁺ = 353.05

### 75.2 Synthesis of compound L060 hydrochloride

For the synthesis of compound L060, reference was made to the synthesis method of compound L059, L059-2 was replaced with L060-1, and L001-IS-01-9 was replaced with L001-7; the crude product obtained by reaction was purified by high performance liquid chromatography (0.1% hydrochloric acid condition) and then lyophilized to give compound L060 hydrochloride (10.2 mg).

¹H NMR (400 MHz, DMSO-*d₆*): δ 10.63 (s, 1H), 9.27 (s, 2H), 8.63 - 8.38 (m, 1H), 8.03 (s, 2H), 7.73 - 7.52 (m, 3H), 7.43 (s, 4H), 6.83 (dd, *J =* 8.0, 4.0 Hz, 1H), 5.15 (s, 1H), 4.48 - 4.14 (m, 4H), 3.81 (s, 5H), 3.11 - 2.84 (m, 4H), 2.43 (s, 1H), 2.01 (s, 1H), 1.23 (d, *J* = 4.0 Hz, 3H). LC-MS: [M+H]⁺ = 441.05.

### Example 76: Synthesis of N-((S)-1-(((R)-2-amino-6,7-dihydro-5H-cyclopenta[b]pyridin-5-yl)amino)-1-oxopropan-2-yl)-4-(4-fluorophenyl)-1H-pyrrole-2-carboxamide (Compound L061-IS-01 or L061-IS-02) and N-((S)-1-(((S)-2-amino-6,7-dihydro-5H-cyclopenta[b]pyridin-5-yl)amino)-1-oxopropan-2-yl)-4-(4-fluorophenyl)-1H-pyrrole-2-carboxamide (Compound L061-IS-02 or L061-IS-01) Hydrochloride

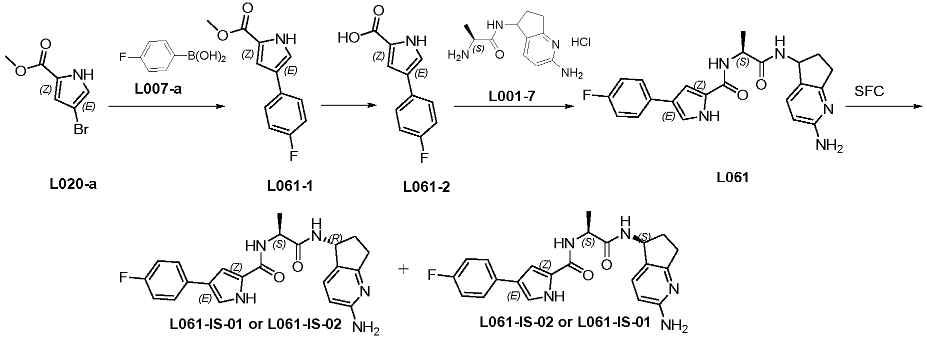

### 76.1 Synthesis of compound L061-1

L020-a (200 mg, 1.0 mmol), [1,1'-bis(diphenylphosphino)ferrocene]dichloropalladium(II) (73 mg, 0.1 mmol), L007-a (182 mg, 1.3 mmol), and potassium carbonate (276 mg, 2.0 mmol) were added to 1,4-dioxane (4 mL) and water (0.5 mL). The reaction liquid was reacted at 100 °C for 12 h under nitrogen atmosphere. The reaction liquid was cooled to room temperature, added with water (10 mL) for dilution, and extracted with ethyl acetate (10 mL ×3). The combined organic phases were washed with saturated brine (10 mL), dried over anhydrous sodium sulfate and filtered, and the filtrate was concentrated under reduced pressure. The residue was purified by silica gel column chromatography to give compound L061-1 (200 mg, yield: 90.9%). LC-MS: [M+H]⁺ = 220.05.

### 76.2 Synthesis of compounds L061, L061-IS-01, and L061-IS-02

For the synthesis of compound L061, reference was made to the synthesis method of compound L020, and L020-3 was replaced with L061-1; the crude product obtained by reaction was purified by high performance liquid chromatography and then lyophilized to give compound L061 (38.0 mg, yield: 19.1 %). L061 was separated by normal phase chromatography to give compounds L061-IS-01 (8.0 mg, retention time Rt = 10.143 min) and L061-IS-02 (8.0 mg, retention time Rt = 13.314 min).
L061-IS-01 (retention time Rt = 10.143 min)
   ¹H NMR (400 MHz, DMSO-*d₆*): δ 11.64 (s, 1H), 8.14 (d, *J* = 8.4 Hz, 1H), 8.01 (d, *J* =8.8 Hz, 1H), 7.59 - 7.53 (m, 2H), 7.33 (s, 1H), 7.28 - 7.24 (m, 1H), 7.18 -7.15 (m, 3H), 6.23 (d, *J* = 8.4Hz, 1H), 5.83 - 5.81 (m, 2H), 5.14 - 5.11 (m, 1H), 4.45 - 4.40 (m, 1H), 2.76 - 2.72 (m, 1H), 2.69 - 2.63(m, 1H), 2.59 - 2.50 (m, 1H), 1.81 - 1.75 (m, 1H), 1.32 (d, *J* = 7.2 Hz, 3H). LC-MS: [M+H]⁺ = 408.05.
L061-IS-02 (retention time Rt = 13.314 min)
   ¹H NMR (400 MHz, DMSO-*d₆*): δ 11.65 (s, 1H), 8.16 (d, *J* = 8.0 Hz, 1H), 8.01 (d, *J* =8.8 Hz, 1H), 7.59 - 7.56 (m, 2H), 7.34 (s, 1H), 7.33 - 7.26 (m, 1H), 7.18 -7.14 (m, 3H), 6.23 (d, *J* = 8.4Hz, 1H), 5.82 - 5.80 (m, 2H), 5.14 - 5.11 (m, 1H), 4.44 - 4.40 (m, 1H), 2.74 - 2.67 (m, 1H), 2.65 - 2.62(m, 1H), 2.34 - 2.33 (m, 1H), 1.77 - 1.69 (m, 1H), 1.32 (d, *J* = 7.2 Hz, 3H). LC-MS: [M+H]⁺ = 408.05.

### Example 77: Synthesis of (2R,4S)-N-((S)-1-(((R)-2-amino-6,7-dihydro-5H-cyclopenta[b]pyridin-5-yl)amino)-1-oxopropan-2-yl)-4-(4-fluorophenoxy)pyrrolidine-2-carboxamide (Compound L062-IS-01) Hydrochloride

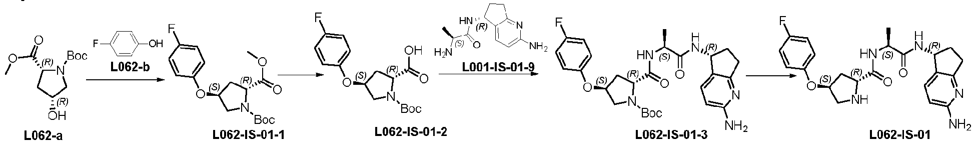

### 77.1 Synthesis of compound L062-IS-01-1

L062-a (1.00 g, 4.07 mmol), L062-b (547 mg, 4.88 mmol), and triphenylphosphine (1.60 g, 6.10 mmol) were dissolved in tetrahydrofuran (20 mL) at room temperature, and then diisopropyl azodicarboxylate (1.23 g, 6.10 mmol) was added dropwise at -10 °C under nitrogen atmosphere. After the dropwise addition was completed, the reaction liquid was further stirred at -10 °C for 1 h, then warmed to room temperature, and stirred for 16 h. The reaction liquid was concentrated under reduced pressure, and the residue was purified by silica gel column chromatography (petroleum ether/ethyl acetate = 5/1) to give compound L062-IS-01-1 (570 mg, yield: 41.3%). LC-MS: [M+H]⁺ = 340.00.

### 77.2 Synthesis of compound L062-IS-01-2

L062-IS-01-1 (500 mg, 1.47 mmol) was dissolved in methanol (0.5 mL), tetrahydrofuran (1 mL) and water (0.5 mL) at room temperature, and then lithium hydroxide monohydrate (190 mg, 4.42 mmol) was added. The reaction liquid was reacted at room temperature for 5 h. The reaction liquid was concentrated under reduced pressure, added with water (10 mL) for dilution, adjusted to pH = 4-5 with diluted hydrochloric acid (1 M), and extracted with ethyl acetate (15 mL × 3). The combined organic phases were washed with saturated sodium chloride (20 mL), dried over anhydrous sodium sulfate and filtered, and the filtrate was concentrated under reduced pressure to give compound L062-IS-01-2 (540 mg, crude product). LC-MS: [M+H]⁺ = 326.05.

### 77.3 Synthesis of compound L062-IS-01 hydrochloride

For the synthesis of compound L062-IS-01, reference was made to the synthesis method of compound L001-IS-01, and L001-8 was replaced with L062-IS-01-2; the crude product obtained by reaction was purified by high performance liquid chromatography (0.1% hydrochloric acid condition) and then lyophilized to give compound L062-IS-01 hydrochloride (157.6 mg).

¹H NMR (400 MHz, DMSO-*d₆*): δ 10.53 (s, 1H), 8.93 (d, *J* = 7.2 Hz, 1H), 8.87 (s, 1H), 8.58 (d, *J* = 7.8 Hz, 1H), 8.03 (s, 2H), 7.71 (d, *J* = 8.8 Hz, 1H), 7.22 - 7.13 (m, 2H), 7.05 - 7.02 (m, 2H), 6.84 (d, *J* = 8.8 Hz, 1H), 5.23 - 5.07 (m, 2H), 4.42 (dd, *J* = 11.2, 6.4 Hz, 1H), 4.27 (t, *J* = 7.2 Hz, 1H), 3.64 (d, *J* = 5.2 Hz, 1H), 3.36 (d, *J* = 12.8 Hz, 1H), 3.07 - 3.03 (m, 1H), 2.96 - 2.85 (m, 1H), 2.61 (dd, *J* = 14.0, 6.8 Hz, 1H), 2.45 (dd, *J* = 8.8, 4.4 Hz, 1H), 2.15 - 2.02 (m, 1H), 1.96 - 1.93 (m, 1H), 1.24 (d, *J* = 7.2 Hz, 3H). LC-MS: [M+H]⁺ = 428.00.

### Example 78: Synthesis of (2R,4R)-N-((S)-1-(((R)-2-amino-6,7-dihydro-5H-cyclopenta[b]pyridin-5-yl)amino)-1 -oxopropan-2-yl)-4-(4-fluorophenoxy)pyrrolidine-2-carboxamide (Compound L062-IS-02) Hydrochloride

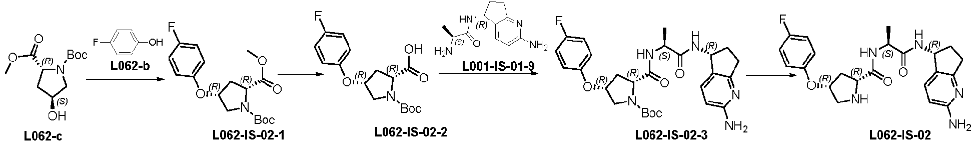

For the synthesis of compound L062-IS-02, reference was made to the synthesis method of compound L062-IS-01, and L062-a was replaced with L062-c; the crude product obtained by reaction was purified by high performance liquid chromatography (0.1% hydrochloric acid condition) and then lyophilized to give compound L062-IS-02 hydrochloride (75.4 mg).

¹H NMR (400 MHz, DMSO-*d₆*): δ 10.45 (s, 1H), 8.77 (d, *J* = 7.2 Hz, 2H), 8.53 (d, *J* = 7.9 Hz, 1H), 7.98 (s, 2H), 7.69 (d, *J* = 8.9 Hz, 1H), 7.27 - 7.04 (m, 2H), 6.90 (ddd, *J* = 6.8, 5.4, 3.1 Hz, 2H), 6.82 (d, *J* = 8.9 Hz, 1H), 5.14 (dd, *J* = 18.5, 11.9 Hz, 2H), 4.39 (s, 1H), 4.24 (p, *J* = 7.1 Hz, 1H), 3.49 (s, 2H), 3.04 (ddd, *J* = 17.4, 9.1, 4.2 Hz, 1H), 2.97 - 2.85 (m, 1H), 2.62 (ddd, *J* = 14.5, 10.2, 5.2 Hz, 1H), 2.49 - 2.41 (m, 1H), 2.26 (d, *J* = 10.9 Hz, 1H), 2.00 - 1.84 (m, 1H), 1.13 (d, *J =* 7.1 Hz, 3H). LC-MS: [M+H]⁺ = 428.00.

### Example 79: Synthesis of (2R)-N-((2S)-1-((2-amino-6,7-dihydro-5H-cyclopenta[b]pyridin-5-yl) amino)-1-oxopropan-2-yl)-4-(4-fluorophenoxy)piperidine-2-carboxamide (Compound L063) Hydrochloride

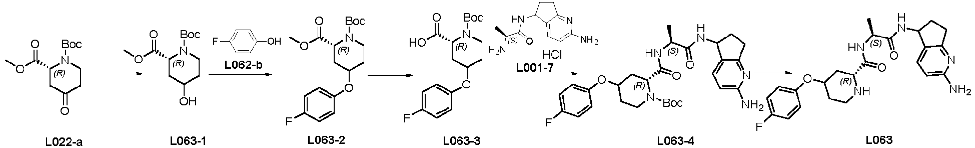

### 79.1 Synthesis of compound L063-1

L022-a (2 g, 7.77 mmol) was dissolved in methanol (30 mL), and then sodium borohydride (300 mg, 7.77 mmol) was added in batches at 0 °C. After the addition was completed, the reaction liquid was warmed to room temperature and reacted for 3 h. The reaction liquid was poured into a saturated aqueous ammonium chloride solution (100 mL) to quench the reaction, and the mixture was extracted with ethyl acetate (50 mL × 3). The combined organic phases were washed with saturated brine (50 mL), dried over anhydrous sodium sulfate and filtered, and the filtrate was concentrated under reduced pressure. The residue was purified by silica gel column chromatography (petroleum ether/ethyl acetate = 2/1) to give compound L063-1 (1.8 g, yield: 88%). LC-MS: [M-Boc+H]⁺ = 160.15.

### 79.2 Synthesis of compound L063

For the synthesis of compound L063, reference was made to the synthesis method of compound L062-IS-01, L062-IS-01-a was replaced with L063-1, and L001-IS-01-9 was replaced with L001-7; the crude product obtained by reaction was purified by high performance liquid chromatography (0.1% hydrochloric acid condition) and then lyophilized to give compound L063 hydrochloride (96.9 mg).

¹H NMR (400 MHz, DMSO-*d₆*): δ 9.75 (d, *J* = 9.6 Hz, 1H), 8.97 - 8.83 (m, 2H), 8.55 (d, *J* = 8.0 Hz, 1H), 8.08 (s, 2H), 7.66 (d, *J =* 8.8 Hz, 1H), 7.21 - 7.02 (m, 4H), 6.85 (d, *J =* 8.8 Hz, 1H), 5.13 (dd, *J* = 14.4, 7.6 Hz, 1H), 4.78 (s, 1H), 4.27 - 4.19 (m, 1H), 4.10 (t, *J* = 10.4 Hz, 1H), 3.52 (d, *J* = 10.8 Hz, 1H), 3.18 - 3.15 (m, 1H), 3.10 - 2.98 (m, 2H), 2.95 - 2.86 (m, 1H), 2.36 - 2.33 (m, 1H), 2.03 - 1.85 (m, 4H), 1.25 (t, *J =* 7.2 Hz, 3H). LC-MS: [M+H]⁺ = 442.00.

### Example 80: Synthesis of (R)-N-((S)-1-(((R)-2-amino-6,7-dihydro-5H-cyclopenta[b]pyridin-5-yl) amino)-1-oxopropan-2-yl)-4-(4-fluorophenyl)-1,2,5,6-tetrahydropyridine-2-carboxamide (Compound L064-IS-01 or L064-IS-02) and (S)-N-((S)-1-(((R)-2-amino-6,7-dihydro-5H-cyclopenta[b]pyridin-5-yl)amino)-1-oxopropan-2-yl)-4-(4-fluorophenyl)-1,2,5,6-tetrahydropyridine-2-carboxamide (Compound L064-IS-02 or L064-IS-01)

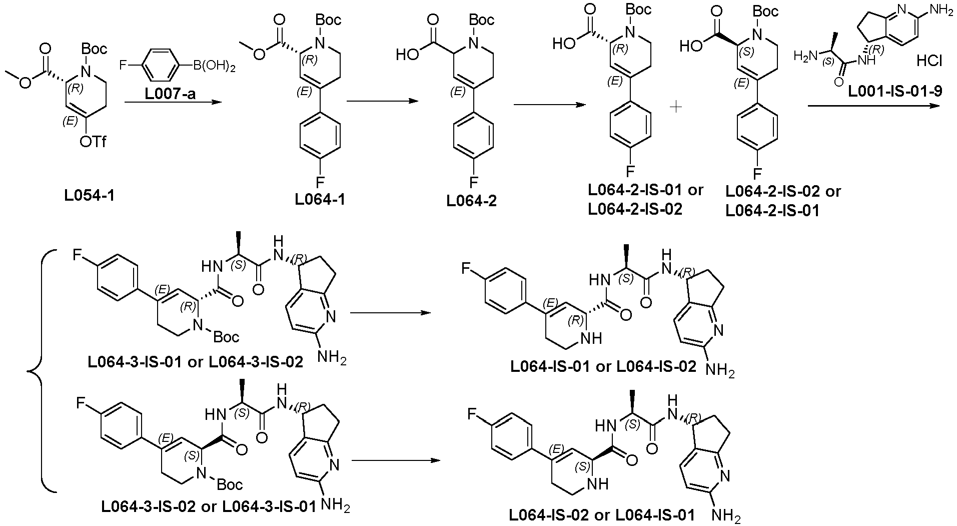

### 80.1 Synthesis of compound L064-1

L054-1 (600 mg, 1.54 mmol), L007-a (323 mg, 2.31 mmol), tetrakis(triphenylphosphine)palladium(0) (108 mg, 0.15 mmol), and potassium carbonate (426 mg, 3.08 mmol) were dissolved in 1,4-dioxane (10 mL) and water (2 mL). The reaction liquid was reacted at 40 °C for 1 h under nitrogen atmosphere. The reaction liquid was filtered, and the filtrate was concentrated under reduced pressure. The residue was purified by silica gel column chromatography (ethyl acetate/petroleum ether = 1/10) to give L064-1 (400 mg, yield: 77.4%). LC-MS: [M-Boc+H]⁺ = 236.10.

### 80.2 Synthesis of compounds L064-2, L064-2-IS-01, and L064-2-IS-02

L064-1 (0.4 g, 1.19 mmol) was dissolved in tetrahydrofuran (4.0 mL), methanol (2.0 mL) and water (2.0 mL), and then lithium hydroxide monohydrate (250 mg, 5.96 mmol) was added. The reaction liquid was reacted at room temperature for 16 h. The reaction liquid was concentrated under reduced pressure, and the residue was added with water (30 mL) for dilution and washed with dichloromethane (30 mL × 3). The organic phase was discarded. The aqueous phase was adjusted to pH = 4-5 with diluted hydrochloric acid (1 M) and extracted with ethyl acetate (30 mL × 3). The combined organic phases were dried over anhydrous sodium sulfate and filtered, and the filtrate was concentrated under reduced pressure to give compound L064-2 (0.37 g, yield: 96.5%). Compound L064-2 (0.37 g) was separated by high performance liquid chromatography (column: CHIRAL ART Amylose-C NEO, 30 × 250 mm, 10 µm, mobile phase, A: n-hexane (0.1% acetic acid), B: isopropanol, flow rate: 30 mL/min) to give compounds (L064-2-IS-01, retention time Rt = 8.021 min, 160 mg) and L064-2-IS-02 (retention time Rt = 10.294 min, 230 mg). LC-MS: [M-Boc+H]⁺ = 222.05.

### 80.3 Synthesis of compounds L064-3-IS-01 and L064-3-IS-02

L064-2-IS-01 (80 mg, 0.249 mmol, retention time Rt = 8.021 min) was dissolved in dichloromethane (2.5 mL) at room temperature, and then 1-propylphosphoric anhydride (396 mg, 0.62 mmol, 50% in ethyl acetate) and N,N-diisopropylethylamine (193 mg, 1.49 mmol) were added. The reaction liquid was stirred at room temperature for 10 min, and L001-IS-01-9 (77 mg, 0.298 mmol) was added. The reaction liquid was stirred at room temperature for another 1 h. The reaction liquid was added with water (10 mL) for dilution and extracted with dichloromethane (10 mL × 3). The organic phase was dried over anhydrous sodium sulfate and filtered, and the filtrate was concentrated under reduced pressure to give compound L064-3-IS-01 (120 mg, crude product). LC-MS: [M+H]⁺ = 524.00. L064-3-IS-02 was synthesized by the same synthesis method.

### 80.4 Synthesis of compounds L064-IS-01 hydrochloride and L064-IS-02 hydrochloride

L064-3-IS-01 (120 mg, 0.23 mmol) was dissolved in dichloromethane (2 mL), and then a solution of hydrogen chloride in 1,4-dioxane (1 mL, 4 M) was added. The reaction liquid was reacted at room temperature for 1 h. The reaction liquid was concentrated under reduced pressure, and the residue was purified by high performance liquid chromatography (0.1% hydrochloric acid condition) and then lyophilized to give compound L064-IS-01 hydrochloride (retention time Rt = 15.888 min). L064-IS-02 hydrochloride (70.0 mg, yield: 21.5%, retention time Rt = 13.664 min) was synthesized by the same synthesis method.
L064-IS-01 :
   ¹H NMR (400 MHz, DMSO-*d₆*): δ 10.01 (s, 1H), 9.07 - 8.89 (m, 2H), 8.57 (d, *J* = 7.6 Hz, 1H), 7.99 (s, 2H), 7.66 (d, *J* = 8.8 Hz, 1H), 7.52 (dd, *J* = 8.8, 5.6 Hz, 2H), 7.26 (t, *J* = 8.8 Hz, 2H), 6.76 (d, *J* = 8.8 Hz, 1H), 6.33 (s, 1H), 5.15 - 5.11 (m, 1H), 4.77 (s, 1H), 4.28 (d, *J* = 7.2 Hz, 1H), 3.33 (s, 2H), 3.05 - 2.86 (m, 2H), 2.69 (s, 2H), 2.46 - 2.38 (m, 1H), 1.89 - 1.88 (m, 1H), 1.32 (d, *J* = 7.2 Hz, 3H). LC-MS: [M+H]⁺ = 424.05.
L064-IS-02:
   ¹H NMR (400 MHz, DMSO-*d₆*): δ 10.09 (s, 1H), 9.10 - 8.93 (m, 2H), 8.55 (d, *J* = 8.0 Hz, 1H), 7.98 (s, 2H), 7.71 (d, *J* = 8.8 Hz, 1H), 7.52 (dd, *J* = 8.8, 5.6 Hz, 2H), 7.24 (t, *J* = 8.8 Hz, 2H), 6.82 (d, *J* = 8.8 Hz, 1H), 6.29 (s, 1H), 5.19 - 5.11 (m, 1H), 4.79 (s, 1H), 4.30 - 4.21 (m, 1H), 3.35 - 3.26 (m, 2H), 3.11 - 3.02 (m, 1H), 2.93 - 2.90 (m, 1H), 2.68 (d, *J* = 6.4 Hz, 2H), 2.47 - 2.41 (m, 1H), 2.03-1.94 (m, 1H), 1.31 (d, *J* = 7.2 Hz, 3H). LC-MS: [M+H]⁺ = 424.05.

### Example 81: Synthesis of (R)-N-((S)-1-(((R)-2-amino-6,7-dihydro-5H-cyclopenta[b]pyridin-5-yl) amino)-1-oxopropan-2-yl)-4-(4-trifluoromethyl)-1,2,5,6-tetrahydropyridine-2-carboxamide (Compound L065-IS-01 or L065-IS-02) Hydrochloride and (S)-N-((S)-1-(((R)-2-amino-6,7-dihydro-5H-cyclopenta [b]pyridin-5-yl)amino)-1-oxopropan-2-yl)-4-(4-trifluoromethyl)-1,2,5,6-tetrahydropyridine-2-carboxamide (Compound L065-IS-02 or L065-IS-01) Hydrochloride

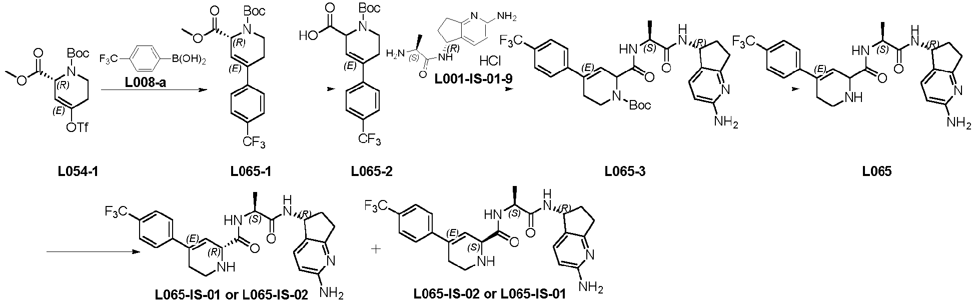

For the synthesis of compounds L065-IS-01 and L065-IS-02, reference was made to the synthesis method of compounds L064-IS-01 and L064-IS-02, L007-a was replaced with L008-a, and intermediate L065-2 was not resolved; the crude product obtained by reaction was purified by high performance liquid chromatography (0.1% hydrochloric acid condition) and then lyophilized to give compound L065-IS-01 hydrochloride (6.7 mg, yield: 2.7%, HPLC retention time Rt = 9.132 min) and compound L065-IS-02 hydrochloride (17.7 mg, yield: 6.9%, HPLC retention time Rt = 11.259 min).
L065-IS-01 hydrochloride (retention time Rt = 9.132 min):
   ¹H NMR (400 MHz, DMSO-*d₆*): δ 10.10 - 9.86 (m, 1H), 9.03 - 9.01 (m, 2H), 8.54 - 8.48 (m, 1H), 7.88 - 7.77 (m, 4H), 7.63 (dd, *J* =8.0, 8.0 Hz, 3H), 6.75 - 6.71 m, 1H), 6.47 (s, 1H), 5.19 - 5.13 (m, 1H), 4.84 (s, 1H), 4.32 - 4.28 (m, 1H), 3.03 - 2.96 (m, 1H), 2.93 - 2.87 (m, 1H), 2.76 - 2.68 (m, 2H), 2.50 - 2.39 (m, 2H), 2.04 - 1.88 (m, 2H), 1.35 (d, *J* = 8.0 Hz, 3H). LC-MS: [M+H]⁺ = 474.00.
L065-IS-02 hydrochloride (retention time Rt = 11.259 min)
   ¹H NMR (400 MHz, DMSO-*d₆*): δ 10.26 - 9.89 (m, 1H), 9.14 - 8.99 (m, 2H), 8.62 - 8.57 (m, 1H), 8.01 (s, 2H), 7.78 - 7.71 (m, 5H), 6.84 (d, *J* = 12.0 Hz, 1H), 6.49 - 6.38 (m, 1H), 5.19 - 5.18 (m, 1H), 4.85 (s, 1H), 4.42 - 4.20 (m, 1H), 3.16 - 3.04 (m, 1H), 2.97 - 2.86 (m, 1H), 2.79 - 2.67 (m, 2H), 2.49 - 2.38 (m, 2H), 2.04 - 1.95 (m, 1H), 1.32 - 1.23 (m, 4H). LC-MS: [M+H]⁺ = 474.00.

Reference was made to the synthesis method of L064-IS-01 and L064-IS-02, or L065-IS-01 and L065-IS-02 to synthesize the compounds in Table B below.

**Table B**

| Example | Intermediate number and structure | Compound structure, number, and name | Characterization data |
|---|---|---|---|
| 82 | 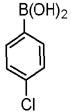 L040-a | 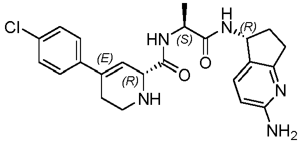 L066-IS-01 or L066-IS-02 | L066-IS-01 (retention time Rt = 17.129 min) |
| | | | ¹H NMR (400 MHz, DMSO-*d₆*): δ 10.02 (s, 1H), 9.07 - 8.93 (m, 2H), 8.57 (d,*J* = 8.0 Hz, 1H), 8.00 (s, 2H), 7.66 (d, *J* = 8.8 Hz, 1H), 7.55 - 7.44 (m, 4H), 6.77 (d, *J* = 8.8 Hz, 1H), 6.39 (s, 1H), 5.12 (dd,*J* = 14.0, 7.6 Hz, 1H), 4.78 (s, 1H), 4.28 (p, *J =* 7.2 Hz, 1H), 3.33 (s, 2H), 3.00 (dd,*J* = 9.2, 4.4 Hz, 1H), 2.91 (dd, *J* = 16.8, 8.8 Hz, 1H), 2.68 (d, *J* = 6.0 Hz, 2H), 2.46 - 2.39 (m, 1H), 1.91 - 1.82 (m, 1H), 1.32 (d, *J =* 7.2 Hz, 3H); |
| | | (R)-N-((S)-1-(((R)-2-amino-6,7-dihydro-5H-cyclopenta [b]pyridin-5-yl)amino)-1-oxopropan-2-yl)-4-(4-chlorophenyl)-1,2,5,6-tetrahydropyridine-2-carboxamide | |
| | | | LC-MS: [M+H]⁺ = 440.00 |
| | | 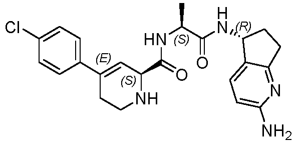 L066-IS-02 or L066-IS-01 | L066-IS-02 (retention time Rt = 28.210 min) |
| | | | ¹H NMR (400 MHz, DMSO-*d₆*): δ 10.12 (s, 1H), 9.08 - 9.06 (m, 2H), 8.55 (d,*J* = 8.0 Hz, 1H), 7.98 (s, 2H), 7.71 (d, *J* = 8.8 Hz, 1H), 7.52 - 7.44 (m, 4H), 6.82 (d, *J* = 8.8 Hz, 1H), 6.36 (d, *J* = 1.6 Hz, 1H), 5.18 (d, *J* = 6.4 Hz, 1H), 4.80 (s, 1H), 4.26 (t, *J* = 7.2 Hz, 1H), 3.36 - 3.28 (m, 2H), 3.10-3.02 (m, 1H), 2.95-2.80 m, 1H), 2.68 (d, *J* = 5.2 Hz, 2H), 2.48 - 2.41 (m, 1H), 2.04 - 1.95 (m, 1H), 1.31 (d, *J* = 7.2 Hz, 3H); |
| | | | |
| | | (S)-N-((S)-1-(((R)-2-amino-6,7-dihydro-5H-cyclopenta [b]pyridin-5-yl)amino)-1-oxopropan-2-yl)-4-(4-chlorophenyl)-1,2,5,6-tetrahydropyridine-2-carboxamide | |
| | | | LC-MS: [M+H]⁺ = 440.00 |
| 83 | 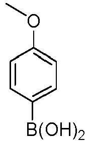 L067-a | 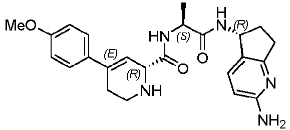 L067-IS-01 or L067-IS-02 | L067-IS-01 (retention time Rt = 15.542 min) |
| | | | ¹H NMR (400 MHz, DMSO-*d*₆): δ 8.15 (d, *J* = 8.0 Hz, 1H), 7.96 (d, *J* = 8.0 Hz, 1H), 7.35 - 7.31 (m, 2H), 7.18 (d, *J* = 8.0 Hz, 1H), 6.92 - 6.88 (m, 2H), 6.23 (d, *J* = 8.0 Hz, 1H), 6.08 (s, 1H), 5.83 (s, 2H), 5.13 - 5.08 (m, 1H), 4.32 - 4.22 (m, 1H), 3.75 (s, 3H), 3.45 - 3.42 (m, 1H), 2.77 - 2.70 (m, 1H), 2.67 - 2.58 (m, 1H), 2.55 - 2.51 (m, 2H), 2.42 - 2.28 (m, 2H), 1.76 - 1.65 (m, 1H), 1.35 - 1.38 (m, 1H), 1.27 - 1.24 (m, 3H); |
| | | (R)-N-((S)-1-(((R)-2-amino-6,7-dihydro-5H-cyclopenta [b]pyridin-5-yl)amino)-1-oxopropan-2-yl)-4-(4-methoxyphenyl)-1,2,5,6-tetrahydropyridine-2-carboxamide | |
| | | | LC-MS: [M+H]⁺ = 436.10 |
| | | 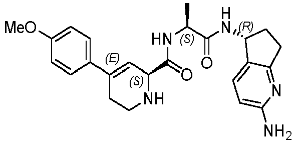 L067-IS-02 or L067-IS-01 | L067-IS-02 (retention time Rt = 21.831 min) |
| | | | ¹H NMR (400 MHz, DMSO-*d₆*): δ 8.18 (dd, *J* = 20.0, 8.0 Hz, 1H), 8.09 (d, *J =* 8.0 Hz, 1H), 7.34 (d, *J* = 8.0 Hz, 2H), 7.20 - 7.14 (m, 1H), 6.90 (d, *J=* 8.0 Hz, 2H), 6.25 - 6.18 (m, 1H), 6.12 (dd, *J =* 7.8, 6.2 Hz, 1H), 5.82 (d, *J* = 6.2 Hz, 2H), 5.14 - 5.04 (m, 1H), 4.31 - 4.22 (m, 1H), 4.01 - 3.92 (m, 1H), 3.75 (s, 3H), 2.93 - 2.91 (m, 2H), 2.76 - 2.67 (m, 1H), 2.64 - 2.56 (m, 1H), 2.36 - 2.26 (m, 2H), 1.76 -1.61 (m, 1H), 1.35 - 1.33 (m, 1H), 1.26 - 1.24 (m, 3H); |
| | | (S)-N-((S)-1-(((R)-2-amino-6,7-dihydro-5H-cyclopenta[b]pyridin-5-yl)amino)-1 - oxopropan-2-yl)-4-(4-methoxyphenyl)-1,2,5,6-tetrahydropyridine-2-carboxamide | |
| | | | LC-MS: [M+H]⁺ = 436.10 |
| 84 | 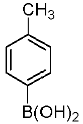 L068-a | 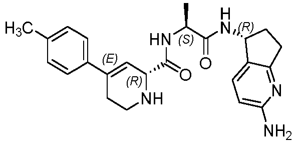 L068-IS-01 or L068-IS-02 | L068-IS-01 (retention time Rt = 13.894 min) |
| | | | ¹H NMR (400 MHz, DMSO-*d₆*): δ 8.22 (d, *J* = 7.8 Hz, 1H), 8.10 (d, *J* = 7.6 Hz, 1H), 7.30 (d, *J =* 8.0 Hz, 2H), 7.16 (t, *J* = 8.4 Hz, 3H), 6.25 - 6.19 (m, 2H), 5.83 (s, 2H), 5.09 (dd, *J* = 14.4, 7.6 Hz, 1H), 4.30 - 4.23 (m, 1H), 3.96 (d, *J* = 2.4 Hz, 1H), 2.92 (t, *J* = 5.2 Hz, 2H), 2.77 - 2.59 (m, 3H), 2.34 (dd, *J* = 12.0, 8.0 Hz, 2H), 2.29 (s, 4H), 1.70 (dd, *J* = 13.6, 7.2 Hz, 1H), 1.21 (d, *J* = 6.8 Hz, 3H); |
| | | (R)-N-((S)-1-(((R)-2-amino-6,7-dihydro-5H-cyclopenta [b]pyridin-5-yl)amino)-1-oxopropan-2-yl)-4-(p-tolyl)-1,2,5,6-tetrahydropyridine-2-carboxamide | |
| | | | LC-MS: [M+H]⁺ = 420.05 |
| | | 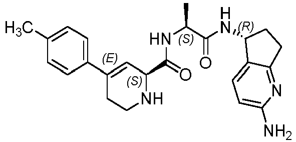 L068-IS-02 or L068-IS-01 | L068-IS-02 (retention time Rt = 10.769 min) |
| | | | ¹H NMR (400 MHz, DMSO-*d₆*): δ8.15 (d, *J* = 8.0 Hz, 1H), 7.96 (d, *J* = 8.0 Hz, 1H), 7.29 (d, *J* = 8.0 Hz, 2H), 7.19-7.13 (m, 3H), 6.23 (d, *J =* 8.0 Hz, 1H), 6.15 (s, 1H), 5.83 (s, 2H), 5.10 (dd, *J* = 12, 4.0 Hz, 1H), 4.31 - 4.27 (m, 1H), 3.43 - 4.32 (m, 3H), 2.52 - 2.50 (m, 3H), 2.32 - 2.31 (m, 1H), 2.30 - 2.28 (m, 3H), 1.73 - 1.71 (m, 1H), 1.25-1,24(m, 6H); |
| | | (S)-N-((S)-1-(((R)-2-amino-6,7-dihydro-5H-cyclopenta[b]pyridin-5-yl)amino)-1- oxopropan-2-yl)-4-(p-tolyl)-1,2,5,6-tetrahydropyridine-2-carboxamide | |
| | | | LC-MS: [M+H]⁺ = 420.05 |
| 85 | 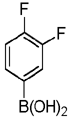 L045-a | 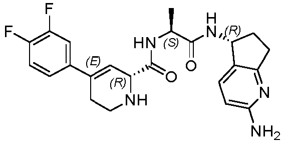 L069-IS-01 or L069-IS-02 | L069-IS-01 (retention time Rt = 10.697 min) |
| | | | ¹H NMR (400 MHz, DMSO-*d₆*): δ 10.10 (s, 1H), 9.06 (d, *J* = 6.8 Hz, 1H), 8.98 (s, 1H), 8.58 (d, *J* = 8.0 Hz, 1H), 7.98 (s, 2H), 7.65 (d, *J* = 8.8 Hz, 1H), 7.62 -7.54 (m, 1H), 7.54 -7.44 (m, 1H), 7.34 (s, 1H), 6.76 (d, *J* = 8.8 Hz, 1H), 6.45 (s, 1H), 5.16 -5.08 (m, 1H), 4.79 (s, 1H), 4.31 -4.22 (m, 1H), 3.30 - 3.23 (m, 1H), 3.06 - 2.94 (m, 1H), 2.94 - 2.83 (m, 1H), 2.68 (s, 2H), 2.47 - 2.36 (m, 2H), 1.91 - 1.80 (m, 1H), 1.32 (d, *J* = 7.2 Hz, 3H) |
| | | (R)-N-((S)-1-(((R)-2-amino-6,7-dihydro-5H-cyclopenta [b]pyridin-5-yl)amino)-1-oxopropan-2-yl)-4-(3,4-difluorophenyl)-1,2,5,6-tetrahydropyridine-2-carboxamide | |
| | | | LC-MS: [M+H]⁺ = 442.00 |
| | | 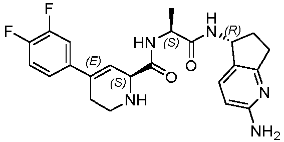 L069-IS-02 or L069-IS-01 | L069-IS-02 (retention time Rt = 21.653 min) |
| | | | ¹H NMR (400 MHz, DMSO-*d₆*): δ 8.20 (d, *J* = 8.0 Hz, 1H), 8.09 (d, *J* = 7.8 Hz, 1H), 7.51-7.44 (m, 1H), 7.43 -7.35 (m, 1H), 7.30 - 723 (m, 1H), 7.14 (d, *J* = 8.4 Hz, 1H), 6.29 (d, *J* = 3.2 Hz, 1H), 6.20 (d, *J* = 8.4 Hz, 1H), 5.82 (s, 2H), 5.12 - 5.04 (m, 1H), 4.31 - 4.21 (m, 1H), 4.01 - 3.95 (m, 1H), 3.31- 3.27 (m, 1H), 2.92 (t, *J* = 5.4 Hz, 2H), 2.76 - 2.67 (m, 1H), 2.65 - 2.57 (m, 1H), 2.40 - 2.19 (m, 3H), 1.72 - 1.61 (m, 1H), 1.23 (d, *J* = 6.8 Hz, 3H); |
| | | (S)-N-((S)-1-(((R)-2-amino-6,7-dihydro-5H-cyclopenta[b]pyridin-5-yl)amino)-1-oxopropan-2-yl)-4-(3,4-difluorophenyl)-1,2,5,6-tetrahydropyridine-2-carboxamide | |
| | | | LC-MS: [M+H]⁺ = 442.00 |
| 86 | 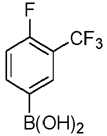 L070-a | 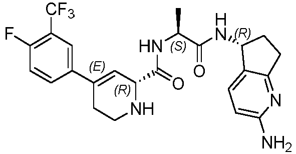 L070-IS-01 or L070-IS-02 | L070-IS-01 (retention time Rt = 11.327 min) |
| | | | ¹H NMR (400 MHz, DMSO-*d₆*): δ10.13 (s, 1H), 9.13 - 8.91 (m, 2H), 8.60 (d, *J* = 8.0 Hz, 1H), 8.00 (s, 2H), 7.89 - 7.77 (m, 2H), 7.69 - 7.56 (m, 2H), 6.76 (d, *J* = 8.0 Hz, 1H), 6.50 (s, 1H), 5.12 (dd, *J* = 16.0, 8.0 Hz, 1H), 4.82 (s, 1H), 4.28 (q, *J* = 8.0 Hz, 1H), 3.34 (d, *J* = 4.0 Hz, 2H), 3.08 - 2.95 (m, 1H), 2.95 - 2.83 (m, 1H), 2.81 - 2.65 (m, 2H), 2.41-2.40 (m, 1H), 1.95 - 1.81 (m, 1H), 1.33 (d, *J* = 8.0 Hz, 3H) |
| | | | |
| | | (R)-N-((S)-1-(((R)-2-amino-6,7-dihydro-5H-cyclopenta[b]pyridin-5-yl)amino)-1-oxopropan-2-yl)-4-(4-fluoro-3-(trifluoromethyl)phenyl)-1,2,5,6-tetrahydropyridine-2-carboxamide | |
| | | | LC-MS: [M+H]⁺ = 492.00 |
| | | 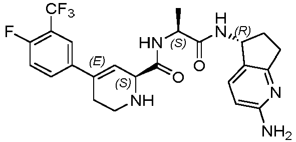 L070-IS-02 or L070-IS-01 | L070-IS-02 (retention time Rt = 16.376 min) |
| | | | ¹H NMR (400 MHz, DMSO-*d₆*): δ10.18 (s, 1H), 9.07 (d, *J* = 8.0 Hz, 2H), 8.55 (d, *J* = 8.0 Hz, 1H), 7.97 (s, 2H), 7.77 - 7.79 (m, 1H), 7.78 (d, *J* = 8.0 Hz, 1H), 7.71 (d, *J* =8.0 Hz, 1H), 7.61 - 7.51 (m, 1H), 6.82 (t, *J* = 8.0 Hz, 1H), 6.47 (s, 1H), 5.18 (q, *J* = 8.0 Hz, 1H), 4.83 (s, 1H), 4.25 (d, *J* = 8.0 Hz, 1H), 3.34 (dd, *J =* 8.0, 4.0 Hz, 2H), 3.08-3.05 (m, 2H), 2.97 - 2.84 (m, 1H), 2.76-2.73 (m, 2H), 2.48 - 2.40 (m, 1H), 1.99 (m, 1H), 1.32 (d, *J* = 8.0 Hz, 4H); |
| | | (S)-N-((S)-1-(((R)-2-amino-6,7-dihydro-5H-cyclopenta[b]pyridin-5-yl)amino)-1- oxopropan-2-yl)-4-(4-fluoro-3-(trifluoromethyl)phenyl)-1,2,5,6-tetrahydropyridine-2-carboxamide | |
| | | | LC-MS: [M+H]⁺ = 492.00 |
| 87 | 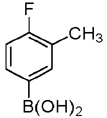 L071-a | 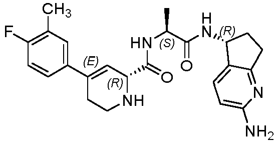 L071-IS-01 or L071-IS-02 | L071-IS-01 (retention time Rt = 9.404 min) |
| | | | ¹H NMR (400 MHz, DMSO-*d₆*): δ 10.04 (s, 1H), 9.05 (d, *J =* 7.2 Hz, 1H), 8.94 (s, 1H), 8.59 (d, *J =* 8.0 Hz, 1H), 7.99 (s,1 1H), 7.66 (d, *J =* 8.8 Hz, 1H), 7.45 - 7.36 (m, 1H), 7.36 - 7.28 (m, 1H), 7.18 (t, *J* =8.8 Hz, 1H), 6.76 (d, *J =* 9.2 Hz, 1H), 6.31 (s, 1H), 5.12 (dd, *J* = 14.4, 7.6 Hz, 1H), 4.76 (s, 1H), 4.33 - 4.23 (m, 1H), 3.34 - 3.24 (m, 2H), 3.05 - 2.97 (m, 1H), 2.93-2.87 (m, 1H), 2.72-2.62 (m, 2H), 2.45 - 2.36 (m, 1H), 2.25 (d, *J =* 1.2 Hz, 3H), 1.92 - 1.81 (m, 1H), 1.32 (d, *J* = 7.2 Hz, 3H) |
| | | (R)-N-((S)-1-(((R)-2-amino-6,7-dihydro-5H-cyclopenta [b]pyridin-5-yl)amino)-1-oxopropan-2-yl)-4-(4-fluoro-3- methylphenyl)-1,2,5,6-tetrahydropyridine-2-carboxamide | |
| | | | LC-MS: [M+H]⁺ = 438.05 |
| | | 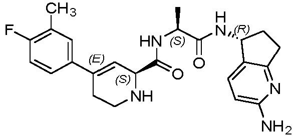 L071-IS-02 or L071-IS-01 | L071-IS-02 (retention time Rt = 13.338 min) |
| | | | ¹H NMR (400 MHz, DMSO-*d₆*): δ 1020 (s, 1H), 9.10 (d, *J* = 7.2 Hz, 1H), 8.98 (s, 1H), 8.56 (d, *J* = 8.0 Hz, 1H), 8.01 (s, 2H), 7.71 (d, *J* = 8.8 Hz, 1H), 7.43 - 7.37 (m, 1H), 7.36 - 7.27 (m, 1H), 7.15 (t, *J* = 9.2 Hz, 1H), 6.82 (d, *J =* 8.8 Hz, 1H), 6.26 (s, 1H), 5.18 (dd, *J* = 14.4, 7.6 Hz, 1H), 4.78 (s, 1H), 4.28 - 4.19 (m, 1H), 3.34 - 3.24 (m, 2H), 3.10 - 3.02 (m, 1H), 2.96 - 2.86 (m, 1H), 2.72-2.62 (m, 2H), 2.47 - 2.41 (m, 1H), 2.25 (d, *J =* 1.2 Hz, 3H), 2.04-1.96 (m, 1H), 1.32 (d, *J* = 7.2 Hz, 3H); |
| | | (S)-N-((S)-1-(((R)-2-amino-6,7-dihydro-5H-cyclopenta [b]pyridin-5-yl)amino)-1-oxopropan-2-yl)-4-(4-fluoro-3- methylphenyl)-1,2,5,6-tetrahydropyridine-2-carboxamide | |
| | | | LC-MS: [M+H]⁺ = 438.05 |
| 88 | 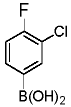 L072-a | 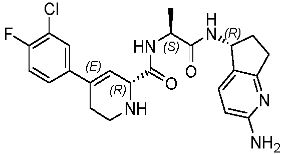 L072-IS-01 or L072-IS-02 | L072-IS-01 (retention time Rt = 18.794 min) |
| | | | ¹H NMR (400 MHz, DMSO-*d₆*): δ10.19 (s, 1H), 9.17 - 8.89 (m, 2H), 8.67 - 8.50 (m, 1H), 8.04 (s, 2H), 7.76 - 7.62 (m, 2H), 7.55 - 7.42 (m, 2H), 6.89 - 6.77 (m, 1H), 6.47 (s, 1H), 5.15 - 5.10 (m, 1H), 4.81 (s, 1H), 4.30 - 4.23 (m, 1H), 3.32 - 3.26 (m, 2H), 3.09 - 2.96 (m, 1H), 2.95 - 2.82 (m, 1H), 2.70 (s, 2H), 2.50 - 2.37 (m, 1H), 1.92 - 1.83 (m, 1H), 1.34 - 1.31 (m, 3H); |
| | | (R)-N-((S)-1-(((R)-2-amino-6,7-dihydro-5H-cyclopenta[b]pyridin-5-yl)amino)-1-oxopropan-2-yl)-4-(3-chloro-4-fluorophenyl)-!,2,5,6-tetrahydropyridine-2-carboxamide | |
| | | | LC-MS: [M+H]⁺ = 457.95 |
| | | 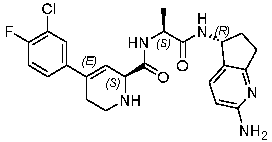 L072-IS-02 or L072-IS-01 | L072-IS-02 (retention time Rt = 24.995 min) |
| | | | ¹H NMR (400 MHz, DMSO-*d₆*): δ10.21 (s, 1H), 9.20 - 8.90 (m, 2H), 8.56 (d, *J* = 12.0 Hz, 1H), 7.99 (s, 2H), 7.73 - 7.66 (m, 2H), 7.48 - 7.42 (m, 2H), 6.84 - 6.80 (m, 1H), 6.41 (s, 1H), 5.18 (dd, *J* = 12.0, 8.0 Hz, 1H), 4.81 (s, 1H), 4.27 - 4.20 (m, 1H), 3.26 (s, 2H), 3.11 - 3.02 (m, 1H), 2.95 - 2.86 (m, 1H), 2.68 (s, 2H), 2.47 - 2.39 (m, 1H), 2.05 - 1.95 (m, 1H), 1.32 (d, *J* = 8.0 Hz, 3H); |
| | | (S)-N-((S)-1-(((R)-2-amino-6,7-dihydro-5H-cyclopenta [b]pyridin-5-yl)amino)-1-oxopropan-2-yl)-4-(3 -chloro-4-fluorophenyl)-1,2,5,6-tetrahydropyridine-2-carboxamide | |
| | | | LC-MS: [M+H]⁺ = 457.95 |
| 89 | 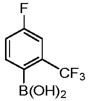 L073-a | 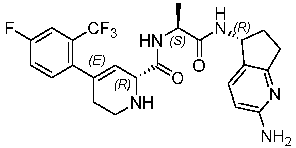 L073-IS-01 or L073-IS-02 | L073-IS-01 (retention time Rt = 10.697 min) |
| | | | ¹H NMR (400 MHz, DMSO-*d*₆): δ10.22 (s, 1H), 9.02 (d, *J* = 8.0 Hz, 2H), 8.58 (d, *J* = 8.0 Hz, 1H), 8.01 (s, 2H), 7.72 (d, *J* = 8.0 Hz, 1H), 7.66 (dd, *J* = 8.0, 4.0 Hz, 1H), 7.59 (td, *J* = 8.0, 4.4 Hz, 1H), 7.44 (dd, *J* = 8.0, 4.0 Hz, 1H), 6.84 (d, *J* = 8.4 Hz, 1H), 5.77 (s, 1H), 5.16 (dd, *J* = 8.0, 4.0 Hz, 1H), 4.75 (s, 1H), 4.28 (p, *J* = 8.0 Hz, 1H), 3.32 (d, *J* = 4.4 Hz, 2H), 3.06 (dd, *J* = 8.0, 4.0 Hz, 1H), 2.97 - 2.86 (m, 1H), 2.54 - 2.51 (m, 2H), 2.49 - 2.40 (m, 2H), 2.04 - 1.89 (m, 1H), 1.23 (d, *J* = 8.0 Hz, 3H); |
| | | (R)-N-((S)-1-(((R)-2-amino-6,7-dihydro-5H-cyclopenta [b]pyridin-5-yl)amino)-1-oxopropan-2-yl)-4-(4-fluoro-2-(trifluoromethyl)phenyl)-1,2,5,6-tetrahydropyridine-2-carboxamide | |
| | | | LC-MS: [M+H]⁺ = 492.00 |
| | | 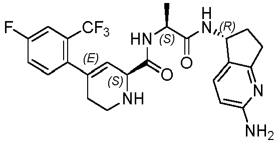 L073-IS-02 or L073-IS-01 | L073-IS-02 (retention time Rt = 21.653 min) |
| | | | Hz, 1H), 3.38 - 3.36 (m, 1H), 3.32 (d, *J* = 8.0 Hz, 1H), 3.06 (dd, *J* = 8.0, 4.0 Hz, 1H), 2.98 - 2.84 (m, 1H), 2.52 (d, *J* = 4.0 Hz, 1H), 2.49 - 2.41 (m, 2H), 2.04 -1.88 (m, 1H), 1.23 (d, *J* =8.0 Hz, 3H); |
| | | (S)-N-((S)-1-(((R)-2-amino-6,7-dihydro-5H-cyclopenta [b]pyridin-5-yl)amino)-1-oxopropan-2-yl)-4-(4-fluoro-2-(trifluoromethyl)phenyl)-1,2,5,6-tetrahydropyridine-2-carboxamide | LC-MS: [M+H]⁺ = 492.00 |
| 90 | 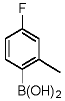 L074-a | 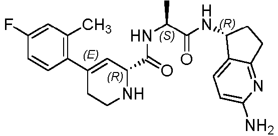 L074-IS-01 or L074-IS-02 | L074-IS-01 (retention time Rt = 13.043 min) |
| | | | ¹H NMR (400 MHz, DMSO-*d₆*): δ 10.04 (s, 1H), 8.99 - 8.94 (m, 2H), 8.54 (d, *J =* 8.0 Hz, 1H), 8.01 (s, 2H), 7.61 (d, *J =* 12.0 Hz, 1H), 7.15 - 7.01 (m, 3H), 6.78 (d, *J* = 12.0 Hz, 1H), 5.74 (s, 1H), 5.13 - 5.07 (m, 1H), 4.71 (s, 1H), 4.31 - 4.24 (m, 1H), 3.04 - 2.85 (m, 2H), 2.67 - 2.56 (m, 2H), 2.50 - 2.41 (m, 3H), 2.25 (s, 3H), 1.95 - 1.78 (m, 1H), 1.29 (d, *J* = 8.0 Hz, 3H); |
| | | (R)-N-((S)-1-(((R)-2-amino-6,7-dihydro-5H-cyclopenta[b]pyridin-5-yl)amino)-1- oxopropan-2-yl)-4-(4-fluoro-2-methylphenyl)-1,2,5,6-tetrahydropyridine-2-carboxamide | |
| | | | LC-MS: [M+H]⁺ = 438.05 |
| | | 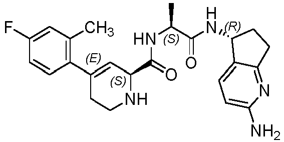 L074-IS-02 or L074-IS-01 | L074-IS-02 (retention time Rt = 14.623 min) |
| | | | ¹H NMR (400 MHz, DMSO-*d₆*): δ 10.17 (s, 1H), 9.01 (d, *J* = 8.0 Hz, 2H), 8.56 (d, *J* = 8.0 Hz, 1H), 8.00 (s, 2H), 7.71 (d, *J* = 8.0 Hz, 1H), 7.14 - 6.99 (m, 3H), 6.83 (d, *J* = 8.0 Hz, 1H), 5.72 (s, 1H), 5.19 - 5.14 (m, 1H), 4.75 (s, 1H), 4.38 - 4.19 (m, 1H), 3.15 - 2.81 (m, 3H), 2.67 - 2.57 (m, 1H), 2.44 - 2.33 (m, 3H), 2.25 (s, 3H), 2.02 - 1.93 (m, 1H), 1.26 (d, *J* = 4.0 Hz, 3H); |
| | | (S)-N-((S)-1-(((R)-2-amino-6,7-dihydro-5H-cyclopenta[b]pyridin-5-yl)amino)-1- oxopropan-2-yl)-4-(4-fluoro-2-methylphenyl)-1,2,5,6-tetrahydropyridine-2-carboxamide | |
| | | | LC-MS: [M+H]⁺ = 438.05 |
| 91 | 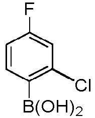 L075-a | 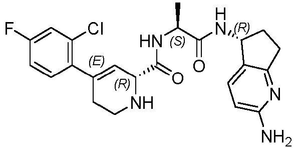 L075-IS-01 or L075-IS-02 | L075-IS-01 (retention time Rt = 11.432 min) |
| | | | ¹H NMR (400 MHz, DMSO-*d₆*): δ 10.13 (s, 2H), 9.02 (d, *J* = 7.2 Hz, 2H), 8.53 (d, *J* = 7.6 Hz, 1H), 8.05-8.04 (m, 2H), 7.61 (d, *J* = 8.8 Hz, 1H), 7.51 (dd, *J* = 12.0, 4.0 Hz, 1H), 7.33 - 7.27 (m, 2H), 6.76 (d, *J* = 8.8 Hz, 1H), 5.91 (s, 1H), 5.10 (dd, *J* = 14.4, 7.6 Hz, 1H), 4.75 (s, 1H), 4.34 - 4.22 (m, 1H), 3.05 - 2.83 (m, 2H), 2.70 - 2.54 (m, 2H), 2.45 - 2.38 (m, 2H), 2.09 - 1.79 (m, 2H), 1.29 (d, *J* = 7.2 Hz, 3H); |
| | | (R)-N-((S)-1-(((R)-2-amino-6,7-dihydro-5H-cyclopenta [b]pyridin-5-yl)amino)-1-oxopropan-2-yl)-4-(2-chloro-4-fluorophenyl)-1,2,5,6-tetrahydropyridine-2-carboxamide | |
| | | | LC-MS: [M+H]⁺ = 457.90 |
| | | 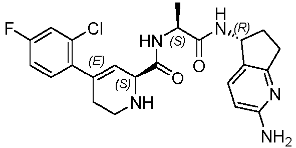 L075-IS-02 or L075-IS-01 | L075-IS-02 (retention time Rt = 12.460 min) |
| | | | ¹H NMR (400 MHz, DMSO-*d₆*): δ 10.09 (s, 1H), 9.01 (d, *J* = 7.2 Hz, 2H), 8.47 (d, *J* = 8.0 Hz, 1H), 7.54 (d, *J* = 8.8 Hz, 1H), 7.50 (dd, *J* = 8.8, 2.4 Hz, 1H), 7.34 - 7.24 (m, 3H), 6.64 (d, *J* = 8.8 Hz, 1H), 5.89 (d, *J* = 1.2 Hz, 1H), 5.15 (dd, *J =* 14.4, 7.6 Hz, 1H), 4.78 (s, 1H), 4.27 (m, 1H), 3.05 - 2.75 (m, 2H), 2.69 - 2.53 (m, 2H), 2.45 - 2.37(m, 2H), 2.02 - 1.86 (m, 2H), 1.26 (d, *J* = 7.2 Hz, 3H); |
| | | (S)-N-((S)-1-(((R)-2-amino-6,7-dihydro-5H-cyclopenta [b]pyridin-5-yl)amino)-1-oxopropan-2-yl)-4-(2-chloro-4-fluorophenyl)-1,2,5,6-tetrahydropyridine-2-carboxamide | |
| | | | LC-MS: [M+H]⁺ = 457.90 |
| 92 | 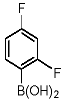 L047-a | 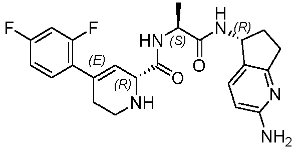 L076-IS-01 or L076-IS-02 | L076-IS-01 (retention time Rt = 9.459 min) |
| | | | 8.4, 2.4 Hz, 1H), 6.14 (s, 1H), 5.12 (dd, *J* = 14.4, 7.6 Hz, 1H), 4.77 (s, 1H), 4.29 (dd, *J* = 14.4, 7.2 Hz, 1H), 2.98 - 2.86 (m, 2H), 2.76 - 2.55 (m, 2H), 2.46 - 2.36 (m, 2H), 2.06 - 1.79 (m, 2H), 1.30 (d, *J* = 7.2 Hz, 3H); |
| | | (R)-N-((S)-1-(((R)-2-amino-6,7-dihydro-5H-cyclopenta [b]pyridin-5-yl)amino)-1-oxopropan-2-yl)-4-(2,4-difluorophenyl)-1,2,5,6-tetrahydropyridine-2-carboxamide | LC-MS: [M+H]⁺ = 441.95 |
| | | 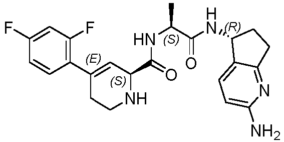 L076-IS-02 or L076-IS-01 | L076-IS-02 (retention time Rt = 11.039 min) |
| | | | ¹H NMR (400 MHz, DMSO-*d₆*): δ 10.06 (s, 1H), 9.24 - 8.95 (m, 2H), 8.55 (d, *J =* 7.6 Hz, 1H), 7.98 (s, 2H), 7.71 (d, *J* = 8.8 Hz, 1H), 7.42 (td, *J* = 8.8 6.4 Hz, 1H), 7.34 - 7.27 (m, 1H), 7.14 (td, *J* = 8.4, 2.4 Hz, 1H), 6.82 (d, *J =* 8.8 Hz, 1H), 6.12 (s, 1H), 5.17 (dd, *J* = 14.4, 7.6 Hz, 1H), 4.80 (s, 1H), 4.26 (m, *J* = 7.1 Hz, 1H), 3.16 - 2.84 (m, 2H), 2.75 - 2.56 (m, 2H), 2.47 - 2.41 (m, 2H), 1.98 (m, 2H), 1.28 (d, *J* = 7.2 Hz, 3H); |
| | | (S)-N-((S)-1-(((R)-2-amino-6,7-dihydro-5H-cyclopenta [b]pyridin-5-yl)amino)-1-oxopropan-2-yl)-4-(2,4-difluorophenyl)-1,2,5,6-tetrahydropyridine-2-carboxamide | |
| | | | LC-MS: [M+H]⁺ = 441.95 |

### Example 93: Synthesis of (R)-N-((S)-1-(((R)-2-amino-6,7-dihydro-5H-cyclopenta[b]pyridin-5-yl) amino)-1-oxopropan-2-yl)-4-(4-fluoro-3-(fluoromethyl)phenyl)-1,2,5,6-tetrahydropyridine-2-carboxamide (L077-IS-01 or L077-IS-02) and (S)-N-((S)-1-(((R)-2-amino-6,7-dihydro-5H-cyclopenta[b] pyridin-5-yl)amino)-1-oxopropan-2-yl)-4-(4-fluoro-3-(fluoromethyl)phenyl)-1,2,5,6-tetrahydropyridine-2-carboxamide (L077-IS-02 or L077-IS-01)

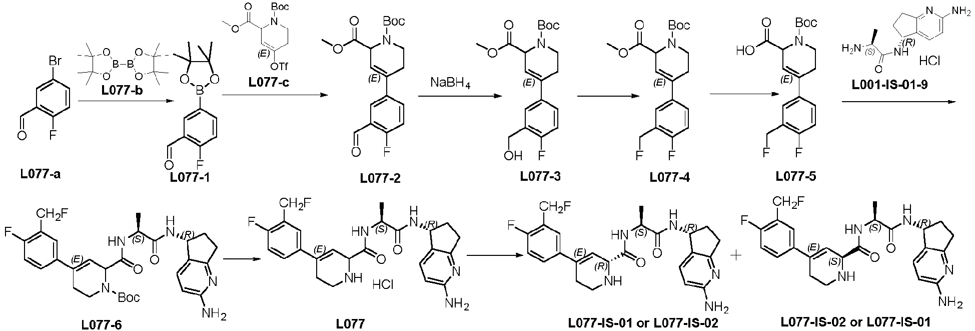

### 93.1 Synthesis of compound L077-1

L077-a (2.0 g, 9.8 mmol), L077-b (2.5 g, 9.8 mmol), [1,1'-bis(diphenylphosphino)ferrocene] dichloropalladium(II) (716 mg, 0.98 mmol), and potassium acetate (2.88 g, 29.4 mmol) were dissolved in 1,4-dioxane (20 mL). The reaction liquid was reacted at 80 °C for 1 h under nitrogen atmosphere. The reaction liquid was cooled to room temperature, quenched by adding a saturated aqueous sodium chloride solution (40 mL), and extracted with ethyl acetate (40 mL × 3). The organic phase was dried over anhydrous sodium sulfate and filtered, and the filtrate was concentrated under reduced pressure. The residue was purified by silica gel column chromatography (ethyl acetate/petroleum ether = 1/10) to give compound L077-1 (2.1 g, yield: 85.7%). LC-MS: [M-Boc+H]⁺ = 251.10.

### 93.2 Synthesis of compound L077-2

L077-c (2.6 g, 6.7 mmol, for the synthesis of compound L077-c, reference was made to the synthesis of compound L054-1), L077-1 (1.8 g, 7.4 mmol), bis(triphenylphosphine)palladium(II) dichloride (490 mg, 0.67 mmol), and potassium carbonate (1.85 mg, 13.4 mmol) were dissolved in tetrahydrofuran (20 mL) and water (10 mL). The reaction liquid was reacted at 40 °C for 2 h under nitrogen atmosphere. The reaction liquid was cooled to room temperature, quenched by adding a saturated aqueous sodium chloride solution (40 mL), and extracted with ethyl acetate (40 mL × 3). The organic phases were combined, dried over anhydrous sodium sulfate and filtered, and the filtrate was concentrated under reduced pressure. The residue was purified by silica gel column chromatography (ethyl acetate/petroleum ether = 1/10) to give compound L077-2 (2.3 g, yield: 94.0%). LC-MS: [M-Boc+H]⁺ = 264.05.

### 93.3 Synthesis of compound L077-3

L077-2 (2.3 g, 6.3 mmol) was dissolved in ethanol (20 mL), and then sodium borohydride (480 mg, 12.6 mmol) was added in batches at 0 °C. After the addition was completed, the reaction liquid was reacted at 0 °C for 1 h, warmed to room temperature, and then reacted for 0.5 h. The reaction liquid was quenched by adding a saturated aqueous sodium chloride solution (30 mL), adjusted to pH = 6-7 with diluted hydrochloric acid (1 M), and extracted with ethyl acetate (40 mL × 2). The organic phase was dried over anhydrous sodium sulfate and filtered, and the filtrate was concentrated under reduced pressure to give compound L077-3 (1.6 g, yield: 69.5%). LC-MS: [M-Boc+H]⁺ = 266.00.

### 93.4 Synthesis of compound L077-4

L077-3 (1.6 g, 4.4 mmol) was dissolved in dichloromethane (20 mL), and then diethylaminosulfur trifluoride (850 mg, 5.3 mmol) was added at 0 °C. After the addition was completed, the reaction liquid was reacted at 0 °C for 0.5 h. The reaction liquid was quenched by adding an aqueous sodium bicarbonate solution (30 mL) and extracted with dichloromethane (30 mL × 2). The combined organic phases were dried over anhydrous sodium sulfate and filtered, and the filtrate was concentrated under reduced pressure. The residue was purified by silica gel column chromatography (ethyl acetate/petroleum ether = 1/7) to give compound L077-4 (1.0 g, yield: 61.3%). LC-MS: [M-Boc+H]⁺ = 268.00.

### 93.5 Synthesis of compound L077-5

L077-4 (1.0 g, 3.7 mmol) was dissolved in acetic acid (10 mL) and concentrated hydrochloric acid (36%, 0.5 mL). The reaction liquid was reacted at 60 °C for 16 h. The reaction liquid was cooled to room temperature and then directly lyophilized to give a crude product. The crude product was dissolved in water (20 mL), and then potassium carbonate (2.0 g, 14.8 mmol) and di-tert-butyl dicarbonate (1.6 g, 7.4 mmol) were added. The reaction liquid was stirred at room temperature for 1 h. The reaction liquid was added with water (10 mL) for dilution, adjusted to pH = 4-5 with diluted hydrochloric acid (1 M), and extracted with ethyl acetate (50 mL × 3). The organic phases were combined and dried over anhydrous sodium sulfate, and the filtrate was concentrated under reduced pressure. The residue was purified by preparative high performance liquid chromatography (0.1% TFA condition) to give compound L077-5 (120 mg, yield: 9.2%). LC-MS: [M-Boc+H]⁺ = 254.05.

### 93.6 Synthesis of compounds L077-IS-01 and L077-IS-02

For the synthesis of compounds L077-IS-01 and L077-IS-02, reference was made to the synthesis method of compounds L065-IS-01 and L065-IS-02, and L065-1 was replaced with L077-5. The crude product obtained by reaction was purified by high performance liquid chromatography (0.1% hydrochloric acid condition) and then lyophilized to give compound L077-IS-01 hydrochloride (26.6 mg, yield: 13.3%, retention time Rt = 10.274 min) and compound L077-IS-02 hydrochloride, and L077-IS-02 hydrochloride was then separated and purified by high performance liquid chromatography (0.1% aqueous ammonia condition) to give compound L077-IS-02 (7.7 mg, yield: 3.8%, retention time Rt = 10.822 min).
L077-IS-01 hydrochloride:
   ¹H NMR (400 MHz, DMSO-*d₆*): δ 10.03 (s, 1H), 9.04 (d, *J* = 6.8 Hz, 2H), 8.54 (d, *J* = 8.0 Hz, 1H), 7.75-7.60 (m, 4H), 7.35 (t, *J* = 9.2 Hz, 1H), 6.67 (d, *J* = 8.4 Hz, 1H), 6.38 (s, 1H), 5.58 (s, 1H), 5.46 (s, 1H), 5.12 (dd, *J* = 14.4, 7.2 Hz, 1H), 4.79 (s, 1H), 4.29 (t, *J* = 7.2 Hz, 1H), 3.50-3.42 (m, 2H), 2.99-2.95 (m, 1H), 2.85-2.83 (m, 1H), 2.73-2.70 (m, 2H), 2.44 - 2.35 (m, 1H), 1.93 - 1.77 (m, 1H), 1.33 (d, *J* = 7.2 Hz, 3H). LC-MS: [M+H]⁺ = 456.05.
L077-IS-02:
   ¹H NMR (400 MHz, DMSO-*d₆*): δ 8.19-8.16 (m, 2H), 7.57 (dd, *J* = 4.8, 2.0 Hz, 1H), 7.52 - 7.44 (m, 1H), 7.23 (t, *J =* 9.2 Hz, 1H), 7.12 (d, *J* = 8.0 Hz, 1H), 6.28 - 6.22 (m, 1H), 6.17 (d, *J* = 8.0 Hz, 1H), 5.80 (s, 2H), 5.47 - 5.45 (m, 2H), 5.05 (dd, *J* = 14.4, 7.2 Hz, 1H), 4.24 - 4.23 (m, 1H), 4.02 (s, 1H), 2.94 (d, *J* = 5.6 Hz, 2H), 2.69- 2.68 (m, 1H), 2.62 - 2.56 (m, 1H), 2.41 - 2.24 (m, 3H), 1.71 - 1.59 (m, 1H), 1.22 (d, *J* = 7.2 Hz, 3H). LC-MS: [M+H]⁺ = 456.05.

### Example 94: Synthesis of (R)-N-((S)-1-(((R)-2-amino-6,7-dihydro-5H-cyclopenta[b]pyridin-5-yl) amino)-1-oxopropan-2-yl)-4-(3-(difluoromethyl)-4-fluorophenyl)-1,2, 5, 6-tetrahydropyridine-2-carboxamide (L078-IS-01 or L078-IS-02) Hydrochloride and (S)-N-((S)-1-(((R)-2-amino-6,7-dihydro-5H-cyclopenta[b]pyridin-5-yl)amino)-1-oxopropan-2-yl)-4-(3-(difluoromethyl)-4-fluorophenyl)-1,2,5,6-tetrahydropyridine-2-carboxamide (L078-IS-02 or L078-IS-01) Hydrochloride

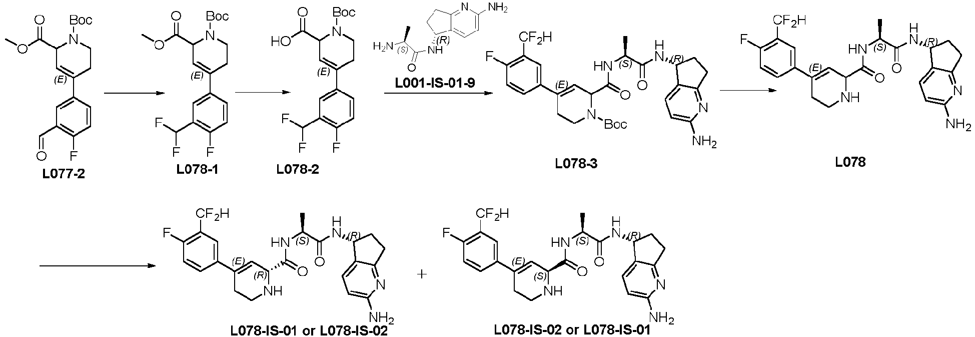

### 94.1 Synthesis of compound L078-1

L077-2 (400 mg, 1.1 mmol) was dissolved in dichloromethane (4 mL), and then diethylaminosulfur trifluoride (372 mg, 2.3 mmol) was added. The mixture was reacted at room temperature for 16 h. An aqueous sodium bicarbonate solution (30 mL) was added to quench the reaction, and the mixture was extracted with ethyl acetate (30 mL × 2). The organic phase was dried over anhydrous sodium sulfate and filtered, and the filtrate was concentrated under reduced pressure to give compound L078-1 (350 mg, yield: 82.6 %). LCMS: [M-Boc+H]⁺ = 289.96.

### 94.2 Synthesis of compounds L078-IS-01 and L078-IS-02

For the synthesis of compounds L078-IS-01 and L078-IS-02, reference was made to the synthesis method of compounds L077-IS-01 and L077-IS-02, and L077-5 was replaced with L078-1. The crude product obtained by reaction was purified by high performance liquid chromatography (0.1% hydrochloric acid condition) and then lyophilized to give compound L078-IS-01 hydrochloride (46.5 mg, retention time Rt = 15.791 min) and compound L078-IS-02 hydrochloride (36.2 mg, retention time Rt = 24.608 min).
L078-IS-01 hydrochloride:
   ¹H NMR (400 MHz, DMSO-*d₆*): δ 10.11 (s, 1H), 9.02 (d, *J* = 6.4 Hz, 2H), 8.50 (d, *J* = 7.6 Hz, 1H), 7.70 (s, 2H), 7.56 - 7.39 (m, 3H), 7.23 (t, *J* = 54.0 Hz, 1H), 6.61 (d, *J* = 8.4 Hz, 1H), 6.40 (s, 1H), 5.09 (dd, *J* = 13.6, 7.2 Hz, 1H), 4.78 (s, 1H), 4.40 - 4.16 (m, 1H), 3.39 (d, *J* = 10.0 Hz, 2H), 2.90 (d, *J* = 5.2 Hz, 1H), 2.80 (dd, *J* = 16.0, 8.4 Hz, 1H), 2.68 - 2.65 (m, 2H), 2.38-2.36 (m, 1H), 1.83-1.80 (m, 1H), 1.31 (d, *J=* 6.8 Hz, 3H). LC-MS: [M+H]⁺ = 474.00.
L078-IS-02 hydrochloride:
   ¹H NMR (400 MHz, DMSO-*d₆*): δ 10.11 (s, 1H), 9.02 (d, *J* = 6.4 Hz, 2H), 8.50 (d, *J* = 7.6 Hz, 1H), 7.70 (s, 2H), 7.56 - 7.39 (m, 3H), 7.24 - 7.25 (m, 1H), 6.61 (d, *J* = 8.5 Hz, 1H), 6.40 (s, 1H), 5.09 (dd, *J* = 13.6, 7.2 Hz, 1H), 4.78 (s, 1H), 4.40 - 4.16 (m, 1H), 3.39 (d, *J =* 10.0 Hz, 2H), 2.90 (d, *J =* 5.2 Hz, 1H), 2.80 (dd, *J* = 16.4, 8.4 Hz, 1H), 2.67 (d, *J* = 16.4 Hz, 2H), 2.37 (d, *J* = 8.4 Hz, 1H), 1.83-1.80 (m, 1H), 1.31 (d, *J* = 6.8 Hz, 3H). LC-MS: [M+H]⁺ = 474.00.

### Example 95: Synthesis of (R)-N-((S)-1-(((R)-2-amino-6,7-dihydro-5H-cyclopenta[b]pyridin-5-yl) amino)-1-oxopropan-2-yl)-4-(4-fluoro-2-(fluoromethyl)phenyl)-1,2,5,6-tetrahydropyridine-2-carboxamide (L079-IS-01 or L079-IS-02) and (S)-N-((S)-1-(((R)-2-amino-6,7-dihydro-5H-cyclopenta[b] pyridin-5-yl)amino)-1-oxopropan-2-yl)-4-(4-fluoro-2-(fluoromethyl)phenyl)-1,2,5,6-tetrahydropyridine-2-carboxamide (L079-IS-02 or L079-IS-01)

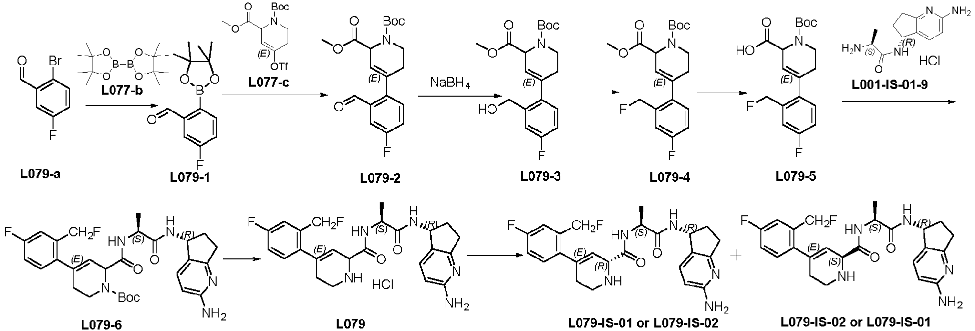

For the synthesis of compounds L079-IS-01 and L079-IS-02, reference was made to the synthesis method of compounds L077-IS-01 and L077-IS-02, and L077-a was replaced with L079-a. The crude product obtained by reaction was purified by high performance liquid chromatography (0.1% trifluoroacetic acid condition) and then lyophilized to give compound L079-IS-01 trifluoroacetate and compound L079-IS-02 trifluoroacetate, which were then freed with a saturated aqueous sodium bicarbonate solution, respectively, to give L079-IS-01 (12.4 mg, retention time Rt = 9.476 min) and compound L079-IS-02 (28 mg, retention time Rt = 9.214 min).
L079-IS-01:
   ¹H NMR (400 MHz, DMSO-*d₆*): δ 8.24 (d, *J* = 8.0 Hz, 1H), 8.11 (d, *J* = 7.6 Hz, 1H), 7.33 - 7.21 (m, 3H), 7.20 - 7.15 (m, 1H), 6.26 - 6.19 (m, 1H), 5.82 (d, *J* = 6.4 Hz, 2H), 5.71 - 5.64 (m, 1H), 5.43 (s, 1H), 5.31 (s, 1H), 5.11 (d, *J* = 7.6 Hz, 1H), 4.29 - 4.27 (m, 1H), 3.95 (d, *J* = 2.8 Hz, 1H), 3.31 - 3.28 (m, 2H), 2.93 (t, *J* = 5.6 Hz, 2H), 2.75 - 2.73 (m, 1H), 2.65 - 2.63 (m, 1H), 2.52 (s, 1H), 2.39 - 2.28 (m, 1H), 2.18 (d, *J* = 2.4 Hz, 2H), 1.75 - 1.66 (m, 1H), 1.22 (d, *J* = 6.8 Hz, 4H). LC-MS: [M+H]⁺ = 456.05.
L079-IS-02:
   ¹H NMR (400 MHz, DMSO-*d₆*): δ 8.22 (d, *J* = 8.0 Hz, 1H), 8.10 (d, *J* = 8.0 Hz, 1H), 7.30 (dd, *J* = 10.0, 2.4 Hz, 1H), 7.27 - 7.19 (m, 2H), 7.16 (d, *J* = 8.4 Hz, 1H), 6.22 (d, *J =* 8.4 Hz, 1H), 5.83 (d,*J* = 9.2 Hz, 2H), 5.72 - 5.68 (m, 1H), 5.40 (s, 1H), 5.32 (s, 1H), 5.10 (dd, *J =* 14.4, 7.6 Hz, 1H), 4.28 (dd, *J* = 14.4, 7.0 Hz, 1H), 3.95 (d, *J* = 2.8 Hz, 1H), 3.31 (s, 1H), 2.92 (dd, *J* = 9.6, 5.4 Hz, 2H), 2.75 - 2.73 (m, 1H), 2.62 (dt, *J* = 11.2, 8.0 Hz, 1H), 2.54 - 2.51 (m, 1H), 2.37 - 2.28 (m, 1H), 2.16 (s, 2H), 1.73 - 1.66 (m, 1H), 1.23 (d, J = 6.8 Hz, 4H). LC-MS: [M+H]⁺ = 456.05.

### Example 96: Synthesis of (R)-N-((S)-1-(((R)-2-amino-6,7-dihydro-5H-cyclopenta[b]pyridin-5-yl) amino)-1-oxopropan-2-yl)-4-(2-(difluoromethyl)-4-fluorophenyl)-1,2,5,6-tetrahydropyridine-2-carboxamide (L080-IS-01 or L080-IS-02) Hydrochloride and (S)-N-((S)-1-(((R)-2-amino-6,7-dihydro-5H-cyclopenta[b]pyridin-5-yl)amino)-1-oxopropan-2-yl)-4-(2-(difluoromethyl)-4-fluorophenyl)-1,2,5,6-tetrahydropyridine-2-carboxamide (L080-IS-02 or L080-IS-01) Hydrochloride

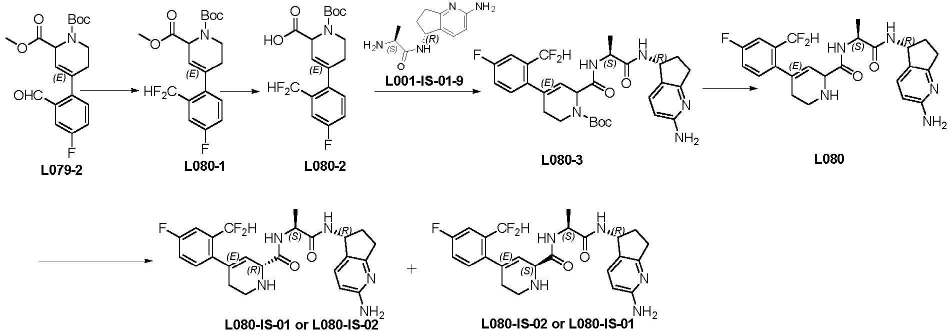

For the synthesis of compounds L080-IS-01 and L080-IS-02, reference was made to the synthesis method of compounds L078-IS-01 and L078-IS-02, and L077-2 was replaced with L079-2. The crude product obtained by reaction was purified by high performance liquid chromatography (0.1% hydrochloric acid condition) and then lyophilized to give compound L080-IS-01 hydrochloride (112.8 mg, retention time Rt = 13.370 min) and compound L080-IS-02 hydrochloride (73.8 mg, retention time Rt = 12.572 min).
L080-IS-01 hydrochloride:
   ¹H NMR (400 MHz, DMSO-*d₆*): δ 10.22 (s, 1H), 9.06 (s, 1H), 8.96 (d, *J* = 7.2 Hz, 1H), 8.54 (d, *J* = 7.6 Hz, 1H), 8.03 (s, 2H), 7.63 (d, *J* = 8.8 Hz, 1H), 7.48 (t, *J* = 8.8 Hz, 2H), 7.43 - 7.36 (m, 1H), 7.09 -7.08 (m, 1H), 6.78 (d, *J* = 8.8 Hz, 1H), 5.81 (d, *J* = 1.2 Hz, 1H), 5.10 (m, 1H), 4.73 (s, 1H), 4.28 (m, 1H), 3.39 (m, 4H), 3.07 - 2.84 (m, 3H), 2.54 (d, *J=* 3.2 Hz, 2H), 2.43 (m, 1H), 1.93 - 1.82 (m, 1H), 1.28 (d, *J* = 7.2 Hz, 3H). LC-MS: [M+H]⁺ = 474.00.
L080-IS-02 hydrochloride:
   ¹H NMR (400 MHz, DMSO-*d₆*): δ 10.22 (s, 1H), 9.06 (s, 1H), 8.96 (d, *J* = 7.2 Hz, 1H), 8.54 (d, *J* = 7.6 Hz, 1H), 8.03 (s, 2H), 7.63 (d, *J* = 8.8 Hz, 1H), 7.48 (t, *J* = 8.8 Hz, 2H), 7.43 - 7.36 (m, 1H), 7.09 -7.08 (m, 1H), 6.78 (d, *J* = 8.8 Hz, 1H), 5.81 (d, *J* = 1.2 Hz, 1H), 5.10 (m, 1H), 4.73 (s, 1H), 4.28 (m, 1H), 3.39 (m, 4H), 3.07 - 2.84 (m, 3H), 2.54 (d, *J* = 3.2 Hz, 2H), 2.43 (m, 1H), 1.93 - 1.82 (m, 1H), 1.28 (d, *J* = 7.2 Hz, 3H). LC-MS: [M+H]⁺ = 474.00.

### Example 97: Synthesis of (R)-N-((S)-1-(((R)-2-amino-6,7-dihydro-5H-cyclopenta[b]pyridin-5-yl) amino)-1-oxopropan-2-yl)-4-(3-chlorophenyl)-1,2,5,6-tetrahydropyridine-2-carboxamide (L081-IS-01 or L081-IS-02) and (S)-N-((S)-1-(((R)-2-amino-6,7-dihydro-5H-cyclopenta[b]pyridin-5-yl)amino)-1-oxopropan-2-yl)-4-(3-chlorophenyl)-1,2,5,6-tetrahydropyridine-2-carboxamide (L081-IS-02 or L081-IS-01)

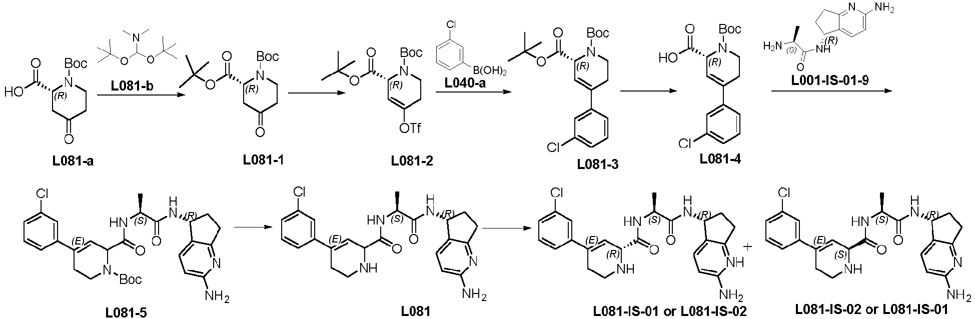

### 97.1 Synthesis of compound L081-1

L081-a (2.0 g, 8.222 mmol) was dissolved in toluene (30 mL), and the reaction liquid was heated to 80 °C under nitrogen atmosphere. L081-b (6.7 g, 32.887 mmol) was added dropwise, and after the addition was completed, the reaction liquid was reacted at 80 °C for another 2 h. The reaction liquid was cooled to room temperature, added with water (30 mL) for dilution, and extracted with ethyl acetate (30 mL ×3). The combined organic phases were washed with saturated brine (30 mL), dried over anhydrous sodium sulfate and filtered, and the filtrate was concentrated under reduced pressure. The residue was purified by silica gel column chromatography (petroleum ether/ethyl acetate = 20/1) to give compound L081-1 (1.4 g, yield: 56.9%).

### 97.2 Synthesis of compound L081-3

For the synthesis of compound L081-3, reference was made to the synthesis method of compound L021-IS-01-3, L021-IS-01-1 was replaced with L081-1, and L006-b was replaced with L040-a. LC-MS: [M-Boc-tBu+H]⁺ = 238.05.

### 97.3 Synthesis of compounds L081-IS-01 and L081-IS-02 hydrochloride

For the synthesis of compounds L081-IS-01 and L081-IS-02 hydrochloride, reference was made to the synthesis method of compound L021-IS-01, L021-IS-01-5 was replaced with L081-3, and L001-7 was replaced with L001-IS-01-9. The crude product obtained by reaction was purified by high performance liquid chromatography (0.1% hydrochloric acid condition) and then lyophilized to give compound L081-IS-01 hydrochloride (8.0 mg, retention time Rt = 16.226 min) and compound L081-IS-02 hydrochloride (13.0 mg, retention time Rt = 20.168 min).
L081-IS-01 hydrochloride:
   ¹H NMR (400 MHz, DMSO-*d₆*): δ 9.97 (s, 1H), 9.40 - 8.646 (m, 2H), 8.56 (d, *J* = 8.0 Hz, 1H), 7.93 (s, 2H), 7.65 (d, *J* = 8.8Hz, 1H), 7.55 (s, 1H), 7.51 - 7.35 (m, 3H), 6.74 (d, *J* = 8.8 Hz, 1H), 6.47 (s, 1H), 5.16 - 5.10 (m, 1H), 4.81 (s, 1H), 4.33 - 4.23 (m, 1H), 3.39 (s, 2H), 3.05 - 2.96 (m, 1H), 2.94 - 2.84 (m, 1H), 2.70 (s, 2H), 2.45 - 2.38 (m, 1H), 1.91 - 1.81 (m, 1H), 1.33 (d, *J* = 7.2 Hz, 3H). LC-MS: [M+H]⁺ = 439.95.
L081-IS-02 hydrochloride:
   ¹H NMR (400 MHz, DMSO-*d₆*): δ 10.10 (s, 1H), 9.05 (d, *J* = 5.6 Hz, 2H), 8.54 (d, *J* = 8.0 Hz, 1H), 7.96 (s, 2H), 7.70 (d, *J* = 8.8 Hz, 1H), 7.53 (s, 1H), 7.48 - 7.37 (m, 3H), 6.81 (d, *J* = 8.8 Hz, 1H), 6.43 (s, 1H), 5.18 (dd, *J* = 14.4, 7.6 Hz, 1H), 4.82 (s, 1H), 4.26 (dd, *J* = 14.4, 7.2 Hz, 1H), 3.34 - 3.25 (m, 2H), 3.10 - 3.01 (m, 1H), 2.92 (dd, *J* = 16.8, 8.4Hz, 1H), 2.69 (d, *J* = 4.8 Hz, 2H), 2.47 - 2.41 (m, 1H), 1.99 (dd, *J* = 13.2, 6.8 Hz, 1H), 1.32 (d, *J* = 7.2 Hz, 3H). LC-MS: [M+H]⁺ = 439.95.

### Example 98: Synthesis of (R)-N-((S)-1-(((R)-2-amino-6,7-dihydro-5H-cyclopenta[b]pyridin-5-yl) amino)-1-oxopropan-2-yl)-4-(2-fluorophenyl)-1,2,5,6-tetrahydropyridine-2-carboxamide (L082) Hydrochloride

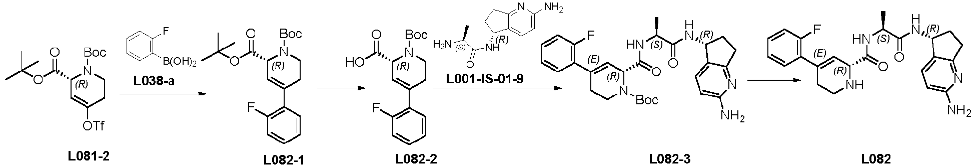

For the synthesis of compound L082, reference was made to the synthesis method of compound L081, and L040-a was replaced with L038-a. The crude product obtained by reaction was purified by high performance liquid chromatography (0.1% hydrochloric acid condition) and then lyophilized to give compound L082 hydrochloride (56.2 mg, yield: 46.1%).

¹H NMR (400 MHz, DMSO-*d₆*): δ 10.23 (s, 1H), 9.12 (d, *J* = 7.2 Hz, 1H), 9.03 (s, 1H), 8.58 (d, *J* = 8.0 Hz, 1H), 8.04 (s, 2H), 7.72 (d, *J* = 8.8 Hz, 1H), 7.47 - 7.32 (m, 2H), 7.25 (dd, *J* = 13.6, 5.2 Hz, 2H), 6.85 (d, *J* = 8.8 Hz, 1H), 6.14 (s, 1H), 5.17 (dd, *J* = 14.4, 7.6 Hz, 1H), 4.81 (s, 1H), 4.26 (p, *J* = 7.2 Hz, 1H), 3.33 (d, *J* = 5.6 Hz, 2H), 3.07 (m, 1H), 2.99 - 2.79 (m, 1H), 2.84 - 2.59 (m, 2H), 2.44 (dd, *J* = 8.4, 4.4 Hz, 1H), 1.99 (m, 1H), 1.29 (d, *J* = 7.2 Hz, 3H). LC-MS: [M+H]⁺ = 424.05.

### Example 99: Synthesis of (R)-N-((S)-1-(((R)-2-amino-6,7-dihydro-5H-cyclopenta[b]pyridin-5-yl) amino)-1-oxopropan-2-yl)-4-(3-fluorophenyl)-1,2,5,6-tetrahydropyridine-2-carboxamide (L083) Hydrochloride

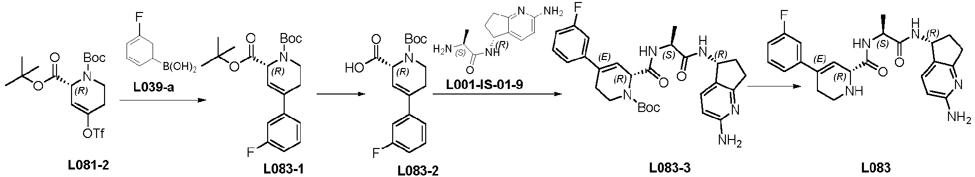

For the synthesis of compound L083, reference was made to the synthesis method of compound L081, and L040-a was replaced with L039-a. The crude product obtained by reaction was purified by high performance liquid chromatography (0.1% hydrochloric acid condition) and then lyophilized to give compound L083 hydrochloride (19.8 mg).

¹H NMR (400 MHz, DMSO-*d₆*): δ 8.19 (d, *J* = 8.0 Hz, 1H), 8.12 (d, *J* = 7.6 Hz, 1H), 7.40 - 7.34 (m, 1H), 7.28 - 7.16 (m, 2H), 7.15 - 7.02 (m, 2H), 6.36 - 6.27 (m, 1H), 6.19 (t, *J* = 8.4 Hz, 1H), 5.79 (s, 2H), 5.09 - 5.05 (m, 1H), 4.25 - 4.22 (m, 1H), 4.01 (s, 1H), 2.93 (t, *J* = 5.6 Hz, 2H), 2.74 - 2.65 (m, 1H), 2.62 - 2.54 (m, 1H), 2.43 - 2.21 (m, 3H), 1.72 - 1.58 (m, 1H), 1.22 (d, *J* =6.8 Hz, 3H). LC-MS: [M+H]⁺ = 424.10.

### Example 100: Synthesis of (R)-N-((S)-1-(((R)-2-amino-6,7-dihydro-5H-cyclopenta[b]pyridin-5-yl) amino)-1-oxopropan-2-yl)-4-(2,3-difluorophenyl)-1,2,5,6-tetrahydropyridine-2-carboxamide (L084) Hydrochloride

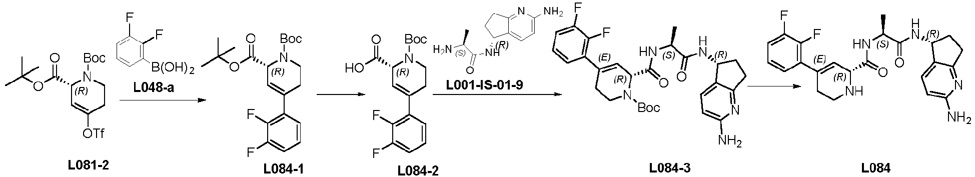

For the synthesis of compound L084, reference was made to the synthesis method of compound L081, and L040-a was replaced with L048-a. The crude product obtained by reaction was purified by high performance liquid chromatography (0.1% hydrochloric acid condition) and then lyophilized to give compound L084 hydrochloride (52.2 mg).

¹H NMR (400 MHz, DMSO-*d₆*): δ 8.21 (d, *J* = 8.0 Hz, 2H), 7.35 - 7.28 (m, 1H), 7.22 - 7.07 (m, 3H), 6.21 - 6.16 (m, 2H), 5.80 (s, 2H), 5.07 (dd, *J* = 14.4, 7.6 Hz, 1H), 4.35 - 4.18 (m, 1H), 4.05 (s, 1H), 2.92 (s, 2H), 2.80 - 2.66 (m, 1H), 2.65 - 2.54 (m, 1H), 2.43 - 2.21 (m, 3H), 1.71 - 1.62 (m, 1H), 1.22 (d, *J* = 6.8 Hz, 3H). LC-MS: [M+H]⁺ = 442.05.

### Example 101: Synthesis of (R)-N-((S)-1-(((R)-2-amino-6,7-dihydro-5H-cyclopenta[b]pyridin-5-yl) amino)-1-oxopropan-2-yl)-4-(3-chlorophenyl)piperazine-2-carboxamide (L085) Hydrochloride

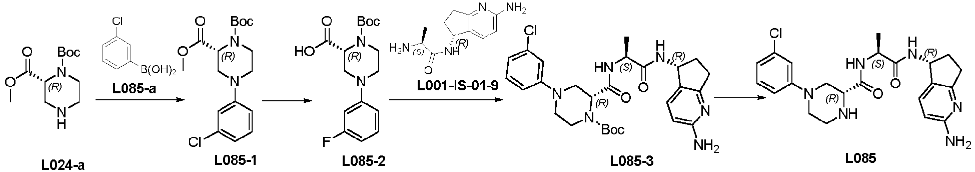

### 101.1 Synthesis of compound L085-1

L024-a (500 mg, 2.05 mmol) and L085-a (640 mg, 4.10 mmol) were dissolved in dichloromethane (10 mL) at room temperature, and then copper acetate (449 mg, 2.26 mmol), 4 Å molecular sieves (500 mg), and pyridine (324 mg, 4.10 mmol) were added. The reaction liquid was purged with oxygen three times and reacted for 16 h under oxygen atmosphere. The reaction liquid was poured into water (10 mL) and extracted with dichloromethane (10 mL × 3). The organic phases were combined, dried over anhydrous sodium sulfate and filtered, and the filtrate was concentrated under reduced pressure. The residue was purified by silica gel column chromatography (ethyl acetate/petroleum ether = 1/10) to give compound L085-1 (720 mg, yield: 99%). LC-MS: [M+H]⁺ = 355.00.

### 101.2 Synthesis of compound L085 hydrochloride

For the synthesis of compound L085, reference was made to the synthesis method of compound L060, and L060-1 was replaced with L085-1. The crude product obtained by reaction was purified by high performance liquid chromatography (0.1% hydrochloric acid condition) and then lyophilized to give compound L085 hydrochloride (20.5 mg, yield: 19.3%).

¹H NMR (400 MHz, DMSO-*d₆*): δ 9.99 - 9.97 (m, 1H), 9.25 - 9.13(m, 2H), 8.56 (d, *J* = 8.0 Hz, 1H), 8.01 (s, 2H), 7.72 (t, *J =* 8.0 Hz, 1H), 7.27 (t, *J =* 8.0 Hz, 1H), 7.11 (s, 1H), 7.02 - 7.01 (m, 1H), 6.92 - 6.86 (m, 1H), 6.83 (d, *J* = 8.8 Hz, 1H), 5.14 - 5.07 (m, 1H), 4.28 - 4.23 (m, 1H), 4.13 - 4.10 (m, 2H), 3.74 (d, *J* = 8.0 Hz, 1H), 3.32 (d, *J* = 8.4 Hz, 1H), 2.99 - 2.93 (m, 5H), 2.49 - 2.40 (m, 1H), 2.03 - 1.91 (m, 1H), 1.30 (d, *J* = 7.2 Hz, 3H). LC-MS: [M+H]⁺ = 443.00.

### Example 102: Synthesis of (R)-N-((S)-1-(((R)-2-amino-6,7-dihydro-5H-cyclopenta[b]pyridin-5-yl) amino)-1-oxopropan-2-yl)-4-(2,3-difluorophenyl)piperazine-2-carboxamide (L086) Hydrochloride

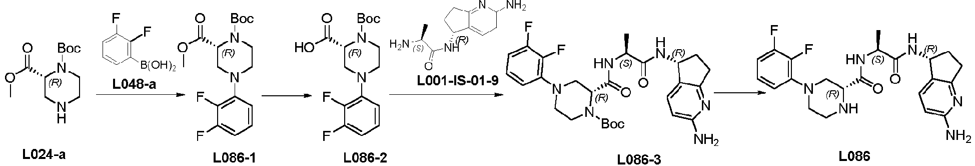

For the synthesis of compound L086, reference was made to the synthesis method of compound L085, and L085-a was replaced with L048-a. The crude product obtained by reaction was purified by high performance liquid chromatography (0.1% hydrochloric acid condition) and then lyophilized to give compound L086 hydrochloride (26.6 mg, yield: 25.3%).

¹H NMR (400 MHz, DMSO-*d₆*): δ 10.11 (s, 1H), 9.19 (s, 1H), 9.04 (d, *J* = 7.2 Hz, 1H), 8.52 (d, *J* = 8.0 Hz, 1H), 8.01 (s, 2H), 7.72 (d, *J* = 8.8 Hz, 1H), 7.18 - 6.97 (m, 3H), 6.83 (d, *J* = 8.8 Hz, 1H), 5.17 (m, 1H), 4.24 (p, *J* = 7.2 Hz, 1H), 4.12 (s, 1H), 3.72 (d, *J* = 12.8 Hz, 1H), 3.34 (s, 1H), 3.22 - 3.02 (m, 5H), 2.98 - 2.86 (m, 1H), 2.47 - 2.39 (m, 1H), 1.99 (m, 1H), 1.27 (d, *J* = 7.2 Hz, 3H). LC-MS: [M+H]⁺ = 445.00.

### Example 103: Synthesis of (2R,4R)-N-((S)-1-(((R)-2-amino-6,7-dihydro-5H-cyclopenta[b]pyridin-5-yl) amino)-1-oxopropan-2-yl)-4-(2-fluorophenyl)-6-carbonylpiperidine-2-carboxamide (Compound L087-IS-01) Hydrochloride

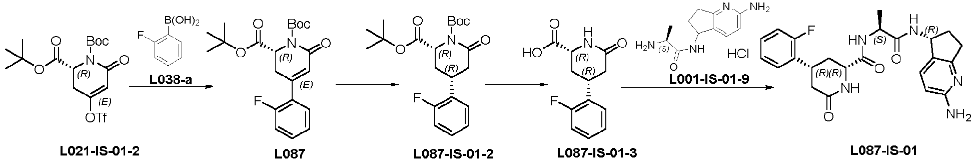

### 103.1 Synthesis of compound L087-IS-01-2

For the synthesis of compound L087-IS-01-2, reference was made to the synthesis method of compound L021-IS-01-4, and L006-b was replaced with L038-a. LC-MS: [M+H]⁺ = 294.05.

### 103.2 Synthesis of compound L087-IS-01-3

L087-IS-01-2 (1.1 g, 2.70 mmol) was dissolved in dichloromethane (4 mL) at room temperature, and then trifluoroacetic acid (8 mL) was added. The reaction liquid was stirred at room temperature for 16 h. The reaction liquid was concentrated under reduced pressure to give compound L087-IS-01-3 (720 mg, yield: 75.2%), which was used directly in the next step. LC-MS: [M+H]⁺ = 238.05.

### 103.3 Synthesis of compound L087-IS-01

L001-IS-01-9 (364 mg, 1.42 mmol) was dissolved in dichloromethane (7 mL) at room temperature, and then diisopropylethylamine (1520 mg, 11.8 mmol) was added. The mixture was stirred at room temperature for 10 min and then added to a solution of L087-IS-01-3 (280 mg, 1.18 mmol) and 1-propylphosphoric anhydride (563 mg, 1.77 mmol, 50% in ethyl acetate) in dichloromethane (7 mL). The reaction liquid was stirred at room temperature for 1 h. The reaction liquid was added with water (50 mL) for dilution and extracted with dichloromethane (50 mL × 3). The organic phases were combined, dried over anhydrous sodium sulfate and filtered, and the filtrate was concentrated under reduced pressure. The residue was separated and purified by high performance liquid chromatography (0.1% hydrochloric acid condition) and then lyophilized to give compound L087-IS-01 (170.5 mg, yield: 56.5%).

¹H NMR (400 MHz, DMSO-*d₆*): δ 8.11 - 8.09 (m, 2H), 7.62 (s, 1H), 7.43 - 7.34 (m, 1H), 7.34 - 7.25 (m, 1H), 7.19 - 7.17 (m, 3H), 6.24 (d, *J =* 8.4 Hz, 1H), 5.83 (s, 2H), 5.13 - 5.12 (m, 1H), 4.25 - 4.23 (m, 1H), 4.13 - 4.10 (m, 1H), 2.81 - 2.69 (m, 1H), 2.69 - 2.57 (m, 2H), 2.45 - 2.25 (m, 3H), 2.16 - 2.14 (m, 1H), 2.05 - 1.63 (m, 2H), 1.21 (d, *J* = 7.6 Hz, 3H). LC-MS: [M+H]⁺ =440.00.

### Example 104: Synthesis of (2R,4S)-N-((S)-1-(((R)-2-amino-6,7-dihydro-5H-cyclopenta[b]pyridin-5-yl) amino)-1-oxopropan-2-yl)-4-(2-fluoro-3-(trifluoromethyl)phenyl)piperidine-2-carboxamide (Compound L088-IS-01) Hydrochloride

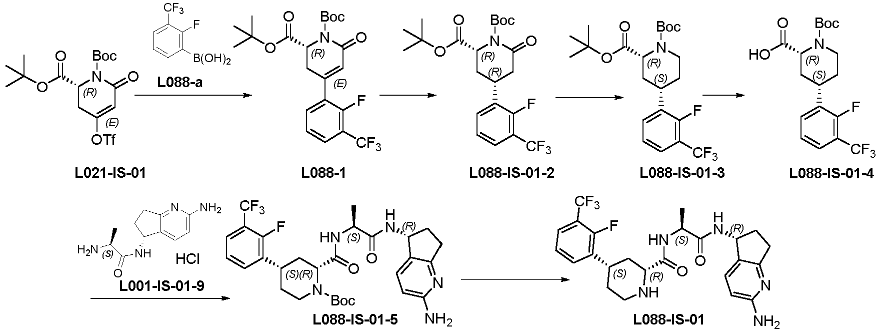

For the synthesis of compound L088-IS-01 hydrochloride, reference was made to the synthesis method of compound L021-IS-01, L006-b was replaced with L088-a, and L001-7 was replaced with L001-IS-01-9; the crude product was separated and purified by high performance liquid chromatography (0.1% hydrochloric acid condition) and then lyophilized to give compound L088-IS-01 hydrochloride (18.6 mg).

¹H NMR (400 MHz, DMSO-*d₆*): δ 9.62 (d, *J* = 8.0 Hz, 1H), 8.84 (m, 2H), 8.51 (d, *J* = 8.4 Hz, 1H), 7.99 (s, 2H), 7.71 (m, 2H), 7.64 (t, *J* = 8.8 Hz, 1H), 7.43 (t, *J* = 8.0 Hz, 1H), 6.83 (d, *J* = 8.4 Hz, 1H), 5.16 (m, 1H), 4.21 (m, 1H), 4.03 - 3.97 (m, 1H), 3.16 (d, *J =* 12.4 Hz, 1H), 3.06 (m, 1H), 2.99 - 2.86 (m, 1H), 2.35 - 2.24 (m, 2H), 2.04 - 1.73 (m, 5H), 1.24 (s, 3H), 1.24 (s, 6H). LC-MS: [M+H]⁺ =494.05.

### Example 105: Synthesis of (5R,7R)-N-((S)-1-(((R)-2-amino-6,7-dihydro-5H-cyclopenta[b]pyridin-5-yl)amino)-1-oxopropan-2-yl)-7-(2-fluorophenyl)-4-azaspiro[2.5]octane-5-carboxamide (Compound L089) Hydrochloride

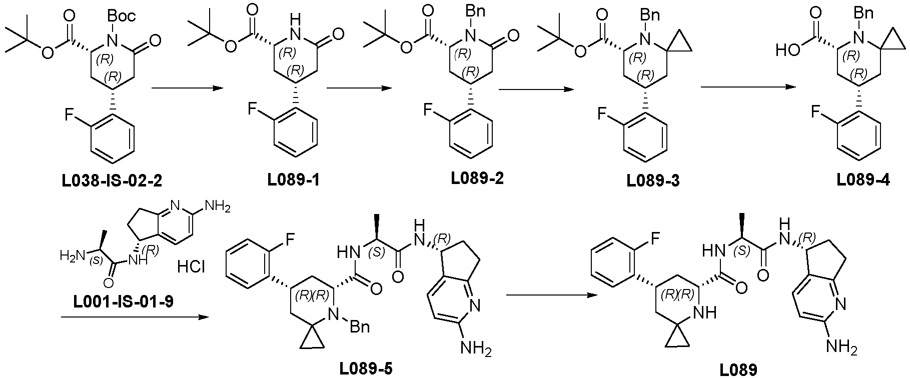

### 105.1: Synthesis of compound L089-1

L038-IS-02-2 (2.7 g, 6.86 mmol) was dissolved in dichloromethane (60 mL), and then a solution of hydrogen chloride in 1,4-dioxane (20 mL, 4 M) was added dropwise at 0 °C. After the dropwise addition was completed, the reaction liquid was reacted at 0 °C for 1 h. The reaction liquid was poured into an aqueous sodium bicarbonate solution (50 mL) to quench the reaction. The organic phase was separated out, and the aqueous phase was extracted with dichloromethane (30 mL×2). The organic phases were combined, washed with saturated brine (50 mL), dried over anhydrous sodium sulfate, and filtered, and the filtrate was concentrated under reduced pressure to give compound L089-1 (2.0 g, yield: 99.36%). LC-MS: [M+H]⁺ = 294.00.

### 105.2: Synthesis of compound L089-2

L089-1 (2.0 g, 6.82 mmol) was dissolved in N,N-dimethylformamide (20 mL), and then sodium hydride (545 mg, 13.64 mmol) was added in batches at 0 °C. The reaction liquid was stirred at 0 °C for 0.5 h, and then benzyl bromide (1.4 g, 8.18 mmol) was added dropwise to the reaction liquid. After the addition was completed, the reaction liquid was warmed to room temperature and reacted for another 16 h. The reaction liquid was poured into a saturated aqueous ammonium chloride solution (30 mL) to quench the reaction and extracted with ethyl acetate (30 mL × 3). The combined organic phases were washed with water (30 mL × 3), washed with saturated brine (30 mL), dried over anhydrous sodium sulfate, and filtered, and the filtrate was concentrated under reduced pressure. The residue was purified by silica gel column chromatography (petroleum ether/ethyl acetate = 5/1) to give compound L089-2 (1.7 g, yield: 65.02%). LC-MS: [M+H]⁺ = 383.95.

### 105.3: Synthesis of compound L089-3

A solution of ethylmagnesium bromide in tetrahydrofuran (12.5 mL, 12.5 mmol, 1M) was added to a reaction flask at room temperature, and then a solution of tetraisopropyl titanate (1.07 g, 3.76 mmol) in tetrahydrofuran (5 mL) was added slowly at -78 °C under nitrogen atmosphere. The temperature was controlled to be not exceeding -70 °C. After the dropwise addition was completed, the reaction liquid was stirred at -78 °C for 10 min. A solution of L089-2 (1.20 g, 3.13 mmol) in tetrahydrofuran (5 mL) was then added dropwise and slowly. After the dropwise addition was completed, the reaction liquid was stirred at -78 °C for another 10 min. The reaction liquid was naturally warmed to room temperature and then stirred for another 2 h. The reaction liquid was quenched by adding a saturated aqueous ammonium chloride solution (10 mL) and extracted with ethyl acetate (30 mL × 3). The organic phases were combined, washed with water (30 mL ×3), washed with saturated brine (30 mL), dried over anhydrous sodium sulfate, and filtered, and the filtrate was concentrated under reduced pressure. The residue was purified by silica gel column chromatography (petroleum ether/ethyl acetate = 5/1) to give compound L089-3 (300 mg, yield: 24.24%). LC-MS: [M+H]⁺ = 396.05.

### 105.4: Synthesis of compound L089-4

L089-3 (250 mg, 0.63 mmol) was dissolved in dichloromethane (3 mL), and then trifluoroacetic acid (1.5 mL) was added dropwise. After the addition was completed, the reaction liquid was reacted at 40 °C for 6 h. The reaction liquid was concentrated under reduced pressure to give compound L089-4 (250 mg, crude product), which was used directly in the next step. LC-MS: [M+H]⁺ = 340.05.

### 105.5: Synthesis of compound L089-5

L089-4 (250 mg, crude product) and L001-IS-01-9 (283 mg, 1.10 mmol) were dissolved in dichloromethane (4 mL), and then N,N-diisopropylethylamine (476 mg, 3.68 mmol) and 1-propylphosphoric anhydride (703 mg, 1.10 mmol, 50% in ethyl acetate) were added. The reaction liquid was reacted at room temperature for 1 h. The reaction liquid was added with a saturated aqueous sodium bicarbonate solution (10 mL) for dilution and extracted with dichloromethane (10 mL × 3). The combined organic phases were dried over anhydrous sodium sulfate and filtered, and the filtrate was concentrated under reduced pressure. The residue was purified by silica gel column chromatography (methanol/dichloromethane = 1/20) to give compound L089-5 (220 mg, two-step yield: 64.44%). LC-MS: [M+H]⁺ = 542.10.

### 105.6: Synthesis of compound L089 hydrochloride

L089-5 (200 mg, 0.37 mmol) was dissolved in methanol (4 mL), and then palladium on carbon (100 mg, 10% wt) was added. The reaction liquid was reacted at 40 °C for 6 h under hydrogen atmosphere. The reaction liquid was filtered through celite, the filter cake was rinsed with methanol (5 mL), and the filtrate was concentrated under reduced pressure. The residue was purified by high performance liquid chromatography (0.1% hydrochloric acid condition) and lyophilized to give compound L089 hydrochloride (27.7 mg).

¹H NMR (400 MHz, DMSO-*d₆*): δ 10.11 (s, 1H), 9.24 (s, 1H), 8.80 (d, *J* = 6.8 Hz, 1H), 8.48 (d, *J* = 8.0 Hz, 1H), 7.92 (s, 2H), 7.68 (d, *J* = 8.8 Hz, 1H), 7.50 (t, *J* = 7.6 Hz, 1H), 7.32 - 7.32 (m, 1H), 7.25 - 7.11 (m, 2H), 6.77 (d, *J* = 8.8 Hz, 1H), 5.16 - 5.14 (m, 1H), 4.36 (s, 1H), 4.28 - 4.28 (m, 1H), 3.47 (s, 1H), 3.07 - 2.81 (m, 2H), 2.45 - 2.38 (m, 1H), 2.34 - 2.22 (m, 2H), 2.02 - 1.79 (m, 3H), 1.28 (d, *J* = 7.2 Hz, 3H), 1.17 - 0.95 (m, 2H), 0.69 - 0.67(m, 1H), 0.39 - 0.37 (m, 1H). LC-MS: [M+H]⁺ = 452.05.

### Example 106: Synthesis of (R)-N-((S)-1-(((R)-2-amino-6,7-dihydro-5H-cyclopenta[b]pyridin-5-yl) amino)-1-oxopropan-2-yl)-4-(4-fluorophenyl)-1,2,3,6-tetrahydropyridine-2-carboxamide (Compound L090) Hydrochloride

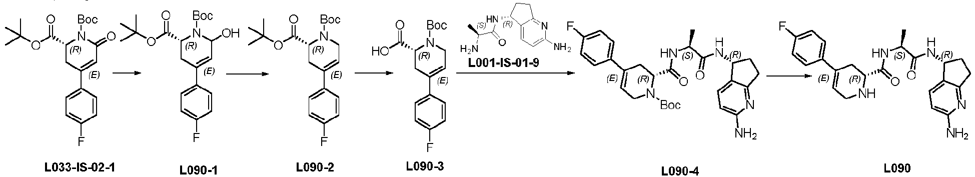

### 106.1: Synthesis of compound L090-1

L033-IS-02-1 (2.0 g, 5.11 mmol) was dissolved in tetrahydrofuran (20 mL), and then the reaction liquid was cooled to -78 °C. Lithium triethylborohydride (7.7 mL, 7.71 mmol, 1 M in tetrahydrofuran) was added dropwise. After the addition was completed, the reaction liquid was reacted at -78 °C for 1 h. The reaction liquid was quenched by adding a saturated ammonium chloride solution (20 mL) and naturally warmed to room temperature under stirring. The reaction liquid was extracted with ethyl acetate (20 mL × 3). The combined organic phases were dried over anhydrous sodium sulfate, and filtered, and the filtrate was concentrated under reduced pressure. The residue was purified by silica gel column chromatography to give compound L090-1 (1.3 g, yield: 64.7%). LC-MS: [M-OH+H]⁺ = 376.00.

### 106.2: Synthesis of compound L090-2

L090-1 (1.2 g, 3.05 mmol) was dissolved in tetrahydrofuran (60 mL), and then the reaction liquid was cooled to -78 °C. Triethylsilylhydride (715 mg, 6.10 mmol) and boron trifluoride-diethyl ether (1.12 g, 3.66 mmol, 46.5% purity) were added dropwise. After the addition was completed, the reaction liquid was reacted at -78 °C for 1 h. The reaction liquid was quenched by adding a saturated aqueous sodium bicarbonate solution (20 mL), naturally warmed to room temperature under stirring, and extracted with ethyl acetate (30 mL × 3). The combined organic phases were dried over anhydrous sodium sulfate and filtered, and the filtrate was concentrated under reduced pressure. The residue was purified by silica gel column chromatography to give compound L090-2 (760 mg, yield: 66.1%). LC-MS: [M+H]⁺ =378.46.

### 106.3: Synthesis of compound L090-3

L090-2 (760 mg, 2.01 mmol) was dissolved in dichloromethane (6 mL), and then trifluoroacetic acid (3 mL) was added. The reaction liquid was reacted at room temperature for 6 h. The reaction liquid was concentrated under reduced pressure, and the residue was redissolved in dichloromethane (10 mL). N,N-diisopropylethylamine (1.04 g, 8.05 mmol) and di-tert-butyl dicarbonate (880 mg, 4,03 mmol) were added. The reaction liquid was reacted at room temperature for 3 h. The reaction liquid was added with water (20 mL) for dilution, and the pH of the solution was adjusted to 4-5 with diluted hydrochloric acid (1 M). The mixture was extracted with dichloromethane (20 mL × 3). The combined organic phases were dried over anhydrous sodium sulfate and filtered, and the filtrate was concentrated under reduced pressure. The residue was purified by silica gel column chromatography to give compound L090-3 (360 mg, yield: 55.4%). LC-MS: [M+Na]⁺ =344.00.

### 106.4: Synthesis of compound L090 hydrochloride

For the synthesis of compound L090, reference was made to the synthesis method of compound L001-IS-01, and L001-8 was replaced with L090-3; the crude product obtained by reaction was purified by high performance liquid chromatography (0.1% hydrochloric acid condition) and then lyophilized to give compound L090 hydrochloride (170.3 mg. yield: 47.29%).

¹H NMR (400 MHz, DMSO-*d₆*): δ 9.92 (d, J = 9.6 Hz, 1H), 9.35 (s, 1H), 8.96 (d, *J* = 6.8 Hz, 1H), 8.58 (d, *J* = 8.0 Hz, 1H), 8.02 (s, 2H), 7.73 (d, *J* = 8.8 Hz, 1H), 7.58 - 7.46 (m, 2H), 7.22 (t, *J* = 8.8 Hz, 2H), 6.83 (d, *J* = 8.8 Hz, 1H), 6.17 (s, 1H), 5.21 - 5.12 (m, 1H), 4.32 - 4.22 (m, 1H), 4.12 - 4.02 (m, 1H), 3.77 (s, 2H), 3.14 - 3.02 (m, 2H), 2.96 - 2.84 (m, 1H), 2.67 - 2.54 (m, 1H), 2.47 - 2.39 (m, 1H), 2.07 - 1.94 (m, 1H), 1.29 (d, *J* = 7.2 Hz, 3H). LC-MS: [M+H]⁺ =424.00.

### Example 107: Synthesis of (2S)-N-(2-amino-6,7-dihydro-5H-cyclopenta[b]pyridin-5-yl)-1-((R)-4-(4-fluorophenyl)piperazine-2-carbonyl)azetidine-2-carboxamide (Compound L091) Hydrochloride

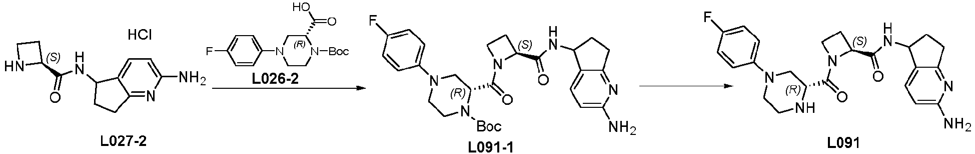

For the synthesis of compound L091, reference was made to the synthesis method of compound L027, and L002-3 was replaced with L026-2; the crude product obtained by reaction was purified by high performance liquid chromatography (0.1% hydrochloric acid condition) and then lyophilized to give compound L091 hydrochloride (5.1 mg. yield: 8.3%).

¹H NMR (400 MHz, DMSO-*d₆*): δ 10.07 (s, 1H), 9.14 (s, 1H), 8.91-8.88 (m, 1H), 8.67 (d, *J* =8.0 Hz, 1H), 7.93 (s, 2H), 7.74-7.70 (m, 1H), 7.48 (d, J=8.0 Hz, 1H), 7.15 - 6.97 (m, 4H), 6.88 - 6.52 (m, 1H), 5.33 - 4.56 (m, 2H), 4.36-4.30 (m, 1H), 4.14 - 3.81 (m, 3H), 3.70-3.68 (m, 1H), 3.30 (s, 1H), 3.19 - 3.03 (m, 2H), 2.98 - 2.77 (m, 3H), 2.20 (d, *J* = 8.0 Hz, 1H), 2.02 - 1.85 (m, 1H), 1.23 (s, 3H). LC-MS: [M+H]⁺ =439.00.

### Example 108: Synthesis of (2R,4R)-N-((S)-1-(((R)-2-amino-6,7-dihydro-5H-cyclopenta[b]pyridin-5-yl)amino)-1-oxopropan-2-yl)-4-fluoro-4-(4-fluorobenzyl)pyrrolidine-2-carboxamide (Compound L092)

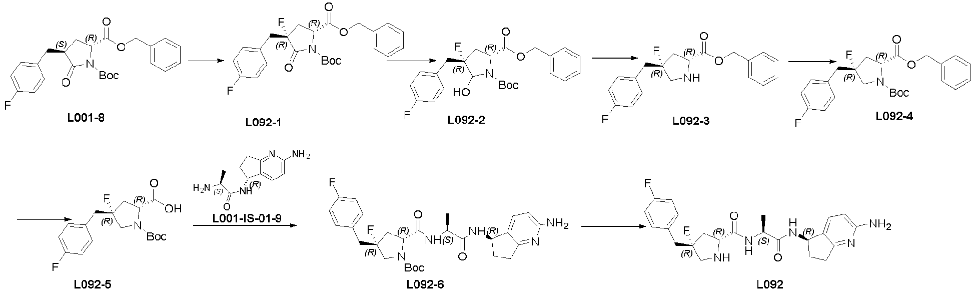

### 108.1: Synthesis of compound L092-1

L001-8 (2.0 g, 4.70 mmol) was dissolved in tetrahydrofuran (20 mL), and then the reaction liquid was purged with nitrogen and cooled to -70 °C. A solution of lithium diisopropylamide in tetrahydrofuran (3.5 mL, 7.00 mmol, 2 M) was then added dropwise. After the addition was completed, the reaction liquid was reacted at -70 °C for 0.5 h. A solution of N-fluorobisbenzenesulfonamide (2.2 g, 7.00 mmol) in tetrahydrofuran (2 mL) was added to the above reaction system, and the reaction liquid was reacted at -70 °C for another 2 h. The reaction liquid was quenched by adding a saturated aqueous ammonium chloride solution (50 mL) and extracted with ethyl acetate (50 mL × 3). The organic phases were combined, dried over anhydrous sodium sulfate, and filtered, and the filtrate was concentrated under reduced pressure. The residue was purified by silica gel column chromatography (petroleum ether/ethyl acetate = 5/1) to give compound L092-1 (500 mg, yield: 24.02%). LC-MS: [M-Boc+H]⁺ =345.95.

### 108.2: Synthesis of compound L092-2

L092-1 (500 mg, 1.10 mmol) was dissolved in tetrahydrofuran (10 mL), and then a solution of lithium triethylborohydride in tetrahydrofuran (1.2 mL, 1.20 mmol, 1 M) was added dropwise at -70 °C. After the addition was completed, the reaction liquid was reacted at -70 °C for 0.5 h. The reaction liquid was quenched by adding a saturated aqueous ammonium chloride solution (20 mL) and extracted with ethyl acetate (20 mL × 3). The organic phases were combined, dried over anhydrous sodium sulfate, and filtered, and the filtrate was concentrated under reduced pressure. The residue was purified by silica gel column chromatography (petroleum ether/ethyl acetate = 5/1) to give L092-2 (409 mg, yield: 81.45%). LC-MS: [M -Boc+H]⁺ = 348.00.

### 108.3: Synthesis of compound L092-3

L092-2 (410 mg, 9.23 mmol) was dissolved in dichloromethane (5 mL), and then triethylsilylhydride (143.5 mg, 1.23 mmol) and boron trifluoride diethyl ether (164 mg, 1.23 mmol) were added dropwise at -70 °C. After the addition was completed, the reaction liquid was reacted at -70 °C for 0.5 h. Triethylsilylhydride (143.5 mg, 1.23 mmol) and boron trifluoride diethyl ether (164 mg, 1.23 mmol) were then added, and the reaction liquid was reacted at 70 °C for 1 h, warmed to room temperature, and then reacted for another 16 h. The reaction liquid was quenched by adding a saturated aqueous sodium bicarbonate solution (30 mL) and extracted with dichloromethane (50 mL × 3). The organic phases were combined, dried over anhydrous sodium sulfate, and filtered, and the filtrate was concentrated under reduced pressure to give compound L092-3 (365 mg, crude product), which was used directly in the next step. LC-MS: [M+H]⁺ = 332.00.

### 108.4: Synthesis of compound L092-4

L092-3 (160 mg, 0.48 mmol) was dissolved in tetrahydrofuran (10 mL) and water (10 mL), and then sodium carbonate (102 mg, 0.97 mmol) and di-tert-butyl dicarbonate (211 mg, 0.97 mmol) were added. The reaction liquid was reacted at room temperature for 1 h. The reaction liquid was extracted with ethyl acetate (20 mL × 3). The organic phases were combined, dried over anhydrous sodium sulfate, and filtered, and the filtrate was concentrated under reduced pressure. The residue was purified by silica gel column chromatography (petroleum ether/ethyl acetate = 3/1) to give compound L092-4 (240 mg, yield: 46.08%). LC-MS: [M-tBu+H]⁺ = 375.95.

### 108.5: Synthesis of compound L092

For the synthesis of compound L092, reference was made to the synthesis method of compound L001, L001-10 was replaced with L092-4, and L001-7 was replaced with L001-IS-01-9. The crude product obtained by reaction was purified by high performance liquid chromatography (0.1% aqueous ammonia condition) and then lyophilized to give compound L092 (68.6 mg, yield: 40.04%).

¹H NMR (400 MHz, DMSO-*d₆*): δ 8.20 (dd, *J* = 10.8, 8.0 Hz, 2H), 7.27 (dd, *J* = 8.4, 5.6 Hz, 2H), 7.20 - 7.06 (m, 3H), 6.24 (d, *J* = 8.4 Hz, 1H), 5.85(s, 2H), 5.12 - 5.06 (m, 1H), 4.26 - 4.19 (m, 1H), 3.77 (t, *J =* 8.4 Hz, 1H), 3.30 (s, 1H), 3.03 (d, *J* = 23.6 Hz, 2H), 2.97 - 2.57 (m, 4H), 2.40 - 2.27 (m, 1H), 2.14 - 2.00 (m, 1H), 1.93 - 1.64 (m, 2H), 1.21 (d, *J*= 6.8 Hz, 3H). LC-MS: [M+H]⁺ = 444.00.

### Example 109: Synthesis of (2R,4S)-N-((S)-1-(((R)-2-amino-6,7-dihydro-5H-cyclopenta[b]pyridin-5-yl)amino)-1-oxopropan-2-yl)-4-(3-fluorobenzyl)pyrrolidine-2-carboxamide (Compound L093)

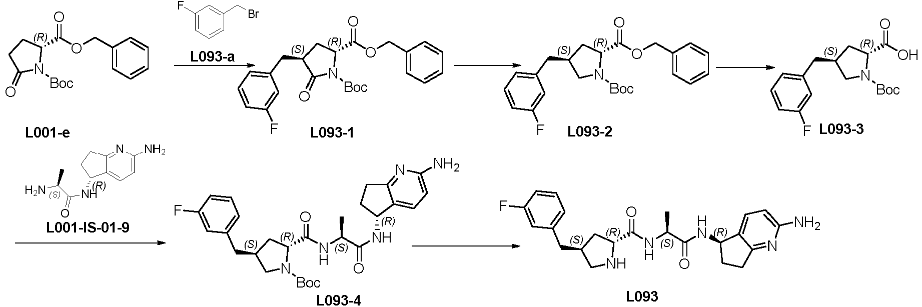

For the synthesis of compound L093, reference was made to the synthesis method of compound L001, L001-f was replaced with L093-a, and L001-7 was replaced with L001-IS-01-9. The crude product obtained by reaction was purified by high performance liquid chromatography (0.1% aqueous ammonia condition) and then lyophilized to give compound L093 (119.4 mg).

¹H NMR (400 MHz, DMSO-*d₆*): δ 8.18 - 8.16 (m, 2H), 7.36 - 7.27 (m, 1H), 7.14 (d, *J=* 8.0 Hz, 1H), 7.03 - 6.98 (m, 3H), 6.23 - 6.17 (m, 1H), 5.84 - 5.82 (m, 2H), 5.10 - 5.04 (m, 1H), 4.27 - 4.16 (m, 1H), 3.66 - 3.64 (m, 1H), 2.93 - 2.82 (m, 1H), 2.78 - 2.68 (m, 1H), 2.67 - 2.53 (m, 4H), 2.39 - 2.26 (m, 1H), 2.21 - 2.14 (m, 1H), 1.84 - 1.60 (m, 3H), 1.19 (d, *J* = 8.0 Hz, 3H). LC-MS: [M+H]⁺ =426.00. Reference was made to the synthesis method of compound L001-1 to synthesize the compounds in the table below.

| Example | Intermediate number and structure | Compound structure, number, and name | Characterization data |
|---|---|---|---|
| 110 | 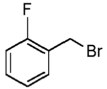 L094-a | 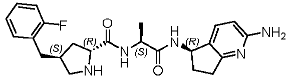 L094 | ¹H NMR (400 MHz, DMSO-*d₆*): δ 10.28 (s, 1H), 8.85 (d, *J* = 7.2 Hz, 1H), 8.54 (d, *J* = 8.0 Hz, 1H), 7.99 (s, 1H), 7.70 (d, *J* = 8.8 Hz, 1H), 7.38 (m, 1H), 7.30 (m, 1H), 7.25 -7.09 (m, 2H), 6.82 (d, *J* = 8.8 Hz, 1H), 5.14 (m, 1H), 4.38 (m, 1H), 4.23 (m, 1H), 3.36 - 3.28 (m, 2H), 3.04 (m, 1H), 2.98 - 2.85 (m, 2H), 2.84 - 2.66 (m, 2H), 2.49 - 2.37 (m, 1H), 2.08 (m, 1H), 1.92 (m, 2H), 1.24 (t, *J* = 6.8 Hz, 2H); |
| | | (2R,4S)-N-((S)-1-(((R)-2-amino-6,7-dihydro-5H-cyclopenta[b]pyridin-5-yl)amino)-1-oxopropan-2-yl)-4-(2-fluorobenzyl)pyrrolidine-2-carboxamide | |
| | | | LC-MS:[M+H]⁺= 426.05 |
| 111 | 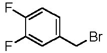 L095-a | 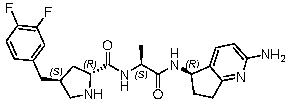 L095 | ¹H NMR (400 MHz, DMSO-*d₆*): δ10.45 (s, 1H), 8.85 (s, 1H), 8.56 (s, 2H), 8.01 (s, 2H), 7.71 (d, *J* = 8.8 Hz, 1H), 7.38 - 7.33 (m, 2H), 7.13 (s, 1H), 6.83 (d, *J* = 8.8 Hz, 1H), 5.17 - 5.12 (m, 1H), 4.38 (s, 1H), 4.26 -4.22 (m, 1H), 3.44 (s, 2H), 3.27 (s, 1H), 3.07 - 3.03 (m, 1H), 2.95 - 2.92 (m, 2H), 2.80 - 2.63 (m, 2H), 2.15 - 2.01 (m, 1H), 1.99 - 1.86 (m, 2H), 1.24 (t, *J* = 7.2 Hz, 3H); |
| | | (2R,4S)-N-((S)-1-(((R)-2-amino-6,7-dihydro-5H-cyclopenta[b]pyridin-5-yl)amino)-1-oxopropan-2-yl)-4-(3,4-difluorobenzyl)pyrrolidine-2-carboxamide | |
| | | | LC-MS:[M+H]⁺= 443.95 |
| 112 | 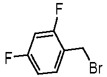 L096-a | 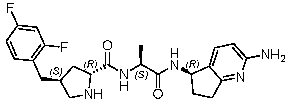 L096 | ¹H NMR (400 MHz, DMSO-*d₆*): δ 8.16 (dd, *J* = 8.0, 2.8 Hz, 2H), 7.33 (td, *J* = 8.8, 6.8 Hz, 1H), 7.21 - 7.14 (m, 1H), 7.13 (d, *J* = 8.4 Hz, 1H), 7.03 - 7.01 (m, 1H), 6.22 (d, *J* = 8.4 Hz, 1H), 5.84 (s, 2H), 5.07 - 5.06 (m, 1H), 4.24 - 4.18 (m, 1H), 3.63 - 3.62 (m, 1H), 2.87 - 2.85 (m, 1H), 2.76-2.69 (m, 1H), 2.65 - 2.55 (m, 4H), 2.38 - 2.25 (m, 1H), 2.15 - 2.13 (m, 1H), 1.80 - 1.59 (m, 3H), 1.18 (d, *J* = 7.2 Hz, 3H); |
| | | (2R,4S)-N-((S)-1-(((R)-2-amino-6,7-dihydro-5H-cyclopenta[b]pyridin-5-yl)amino)-1-oxopropan-2-yl)-4-(2,4-difluorobenzyl)pyrrolidine-2-carboxamide | |
| | | | LC-MS:[M+H]⁺= 444.00 |
| 113 | 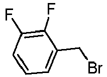 L097-a | 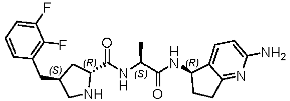 L097 | - 3.28 (m, 1H), 2.89 - 2.87 (m, 1H), 2.77 - 2.56 (m, 5H), 2.35 - 2.28 (m, 1H), 2.24 - 2.14 (m, 1H), 1.79 - 1.60 (m, 3H), 1.19 (d, *J* = 6.8 Hz, 3H); |
| | | (2R,4S)-N-((S)-1-(((R)-2-6,7-dihydro-5H-cyclopenta[b]pyridin-5-yl)amino)-1-oxopropan-2-yl)-4-(2,3-difluorobenzyl)pyrrolidine-2-carboxamideamino- | LC-MS: [M+H]⁺= 444.00 |
| 114 | 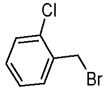 L098-a | 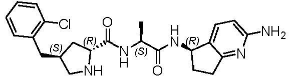 L098 | ¹H NMR (400 MHz, DMSO-*d₆*): δ 8.16 (d, *J* = 8.0 Hz, 2H), 7.40 (d, *J* = 7.6 Hz, 1H), 7.35 - 7.19 (m, 3H), 7.11 (d, *J* = 8.0 Hz, 1H), 620 (d, *J* = 8.4 Hz, 1H), 5.81 (s, 2H), 5.06 - 5.04 (m, 1H), 4.22 - 4.15 (m, 1H), 3.65 (s, 1H), 2.88 - 2.82 (m, 1H), 2.78 - 2.55 (m, 5H), 2.38 - 2.18 (m, 2H), 1.81 - 1.51 (m, 3H), 1.17 (d, *J* = 6.8 Hz, 3H); |
| | | (2R,4S)-N-((S)-1-(((R)-2-amino-6,7-dihydro-5H-cyclopenta[b]pyridin-5-yl)amino)-1-oxopropan-2-yl)-4-(2-chlorobenzyl)pyrrolidine-2-carboxamide | |
| | | | LC-MS:[M+H]⁺= 442.00 |

### Example 115: Synthesis of N-((S)-1-(((R)-2-amino-6,7-dihydro-5H-cyclopenta[b]pyridin-5-yl) amino)-1-oxopropan-2-yl)-4-(3-fluorophenyl)-1H-pyrrole-2-carboxamide (Compound L099)

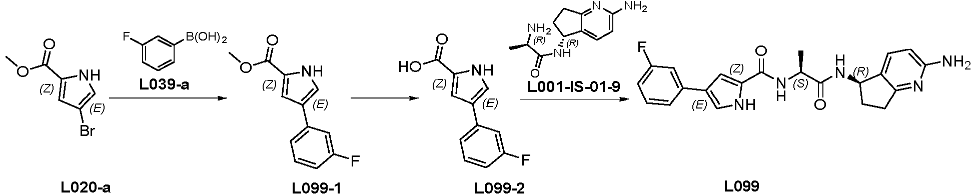

For the synthesis of compound L099, reference was made to the synthesis method of compound L061, L007-a was replaced with L039-a, and L001-7 was replaced with L001-IS-01-9. The crude product obtained by reaction was purified by high performance liquid chromatography (0.1% aqueous ammonia condition) and then lyophilized to give compound L099 (119.4 mg).

¹H NMR (400 MHz, DMSO-*d₆*): δ 11.74 (s, 1H), 8.18 (d, *J* = 8.0 Hz, 1H), 8.00 (d, *J* = 7.6 Hz, 1H), 7.45 (s, 1H), 7.42 - 7.29 (m, 4H), 7.20 (d, *J* = 8.4 Hz, 1H), 6.95 (t, *J* = 8.0 Hz, 1H), 6.23 (d, *J* = 8.4 Hz, 1H), 5.82 (s, 2H), 5.13 (dd, *J* = 8.0, 4.0Hz, 1H), 4.45 - 4.38 (m 1H), 2.78 - 2.70 (m, 1H), 2.67 - 2.59 (m, 1H), 2.37-2.29 (m, 1H), 1.78-1.69 (m, 1H), 1.33 (d, *J =* 7.2 Hz, 3H). LC-MS: [M+H]⁺ =408.00.

### Example 116: Synthesis of (2R,4S)-N-((2S)-1-((2-amino-3-fluoro-6,7-dihydro-5H-cyclopenta[b] pyridin-5-yl)amino)-1-oxopropan-2-yl)-4-(4-fluorobenzyl)pyrrolidine-2-carboxamide (Compound L100) Hydrochloride

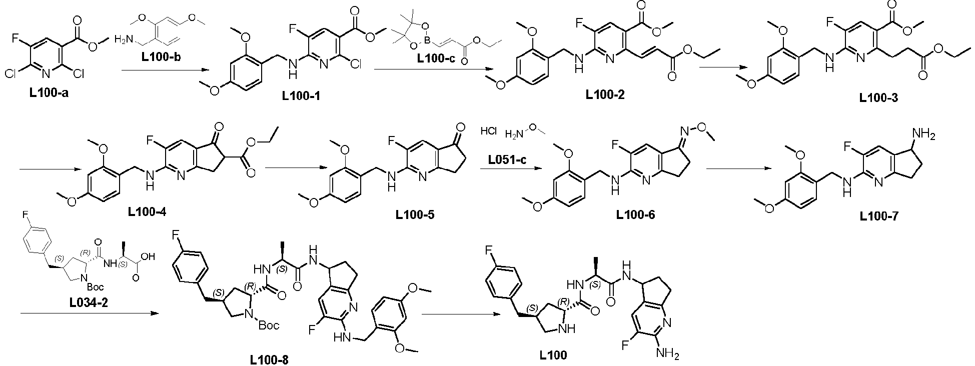

### 116.1: Synthesis of compound L100-1

L100-a (10 g, 44.8 mmol) was dissolved in isopropanol (160 mL), and then N,N-diisopropylethylamine (17.4 g, 134.5 mmol) and L001-b (22.5 g, 134.5 mmol) were added. The reaction liquid was reacted at room temperature for 16 h. The reaction liquid was filtered under reduced pressure. The filter cake was washed with ethanol (100 mL), and the filtrate was concentrated under reduced pressure to give compound L100-1 (14.2 g, yield: 99.0%). LC-MS: [M+H]⁺ = 354.95.

### 116.2: Synthesis of compound L100-2

L100-1 (12.8 g, 36.2 mmol) and L100-c (18.1 g, 72.3 mmol) were dissolved in 1,4-dioxane (180 mL) and water (18 mL), and then potassium acetate (10.6 g, 108.6 mmol) and [1,1'-bis(diphenylphosphino)ferrocene]dichloropalladium(II) (2.6 g, 3.62 mmol) were added. The reaction liquid was reacted at 100 °C for 48 h under nitrogen atmosphere. The reaction liquid was cooled to room temperature, added with water (100 mL) for dilution, and extracted with ethyl acetate (100 mL × 3). The combined organic phases were dried over anhydrous sodium sulfate and filtered, and the filtrate was concentrated under reduced pressure. The residue was purified by silica gel column chromatography (petroleum ether/ethyl acetate = 1/2) to give compound L100-2 (5.3 g, yield: 87%). LC-MS: [M-Boc+H]⁺ = 418.95.

### 116.3: Synthesis of compound L100-3

L100-2 (5.2 g, 12.4 mmol) was added to ethanol (140 mL), and then palladium on carbon (5.2 g, 10% wt) was added. The reaction liquid was reacted for 1.5 h under hydrogen atmosphere. The reaction liquid was filtered through celite. The filter cake was washed with ethanol (150 mL), and the filtrate was concentrated under reduced pressure to give compound L100-3 (3.8 g, yield: 73.0%). LC-MS: [M+H]⁺ = 421.00.

### 116.4: Synthesis of compound L100-4

L100-3 (3.8 g, 9.05 mmol) was dissolved in tetrahydrofuran (40 mL), and then the mixture was cooled to -78 °C. Sodium bis(trimethylsilyl)amide (18.1 mL, 36.2 mmol, 2 M in tetrahydrofuran) was added dropwise under nitrogen atmosphere. After the addition was completed, the reaction liquid was reacted at this temperature for 2 h. The reaction liquid was poured into a saturated aqueous ammonium chloride solution (100 mL) at 0 °C, stirred for another 10 min, and then extracted with ethyl acetate (50 mL × 3). The combined organic phases were dried over anhydrous sodium sulfate and filtered, and the filtrate was concentrated under reduced pressure. The residue was purified by silica gel column chromatography (ethyl acetate/petroleum ether = 1/5) to give compound L100-4 (900 mg, yield: 26%). LC-MS: [M+H]⁺ = 389.00.

### 116.5: Synthesis of compound L100-5

L100-4 (750 mg, 1.95 mmol) and lithium chloride (136.5 mg, 9.75 mmol) were dissolved in dimethyl sulfoxide (20 mL) and water (1 mL), and then the reaction liquid was reacted at 100 °C for 16 h. The reaction liquid was cooled to room temperature, added with a saturated aqueous sodium chloride solution (50 mL) for dilution, and extracted with ethyl acetate (50 mL × 3). The combined organic phases were dried over anhydrous sodium sulfate and filtered, and the filtrate was concentrated under reduced pressure. The residue was purified by silica gel column chromatography (ethyl acetate/petroleum ether = 1/2) to give compound L100-5 (200 mg, yield: 32.4%). LC-MS: [M+H]⁺ = 317.00.

### 116.6: Synthesis of compound L100-6

L100-5 (220 mg, 0.696 mmol) was dissolved in ethanol (4.2 mL) and water (2.1 mL), and then L051-c (87.2 mg, 1.044 mmol) and sodium acetate (85.6 mg, 1.044 mmol) were added. The reaction liquid was reacted at 60 °C for 16 h. The reaction liquid was cooled to room temperature, added with a saturated aqueous sodium chloride solution (10 mL) for dilution, and extracted with ethyl acetate (10 mL × 3). The combined organic phases were dried over anhydrous sodium sulfate and filtered, and the filtrate was concentrated under reduced pressure. The residue was purified by silica gel column chromatography (ethyl acetate/petroleum ether = 1/4) to give compound L100-6 (160 mg, yield: 64%). LC-MS: [M+H]⁺ = 346.00.

### 116.7: Synthesis of compound L100-7

L100-6 (160 mg, 0.446 mmol) was dissolved in methanol (5 mL), and then aqueous ammonia (0.5 mL) was added, followed by addition of Raney nickel (160 mg). The reaction liquid was reacted for 1.5 h under hydrogen atmosphere. The reaction liquid was filtered through celite, and the filter cake was washed with methanol (10 mL). The filtrate was concentrated under reduced pressure, and the residue was added with acetonitrile (2 mL) and water (10 mL) and lyophilized to give compound L100-7 (160 mg, crude product). LC-MS: [M+H]⁺ = 318.05.

### 116.8: Synthesis of compound L100-8

L100-7 (100 mg, 0.315 mmol) was dissolved in dichloromethane (2 mL), and then N,N-diisopropylethylamine (122 mg, 0.945 mmol) was added. The reaction liquid was reacted at room temperature for 10 min. A solution of L034-2 (124 mg, 0.315 mmol) and 1-propylphosphoric anhydride (319 mg, 0.473 mmol, 50% in ethyl acetate) in dichloromethane (2 mL) was added to the above reaction system, and the reaction liquid was reacted at room temperature for another 1 h. The reaction liquid was added with a saturated aqueous sodium chloride solution (10 mL) for dilution and extracted with dichloromethane (10 mL × 3). The combined organic phases were dried over anhydrous sodium sulfate and filtered, and the filtrate was concentrated under reduced pressure. The residue was purified by silica gel column chromatography (methanol/dichloromethane = 1/20) to give compound L100-8 (125 mg, yield: 57.4%). LC-MS: [M+H]⁺ = 694.00.

### 116.9: Synthesis of compound L100 hydrochloride

L100-8 (80 mg, 0.115 mmol) was dissolved in ethyl acetate (1 mL), and then a solution of hydrogen chloride in ethyl acetate (6 mL, 4 M) was added. The reaction liquid was reacted at room temperature for 4 h. The reaction liquid was concentrated under reduced pressure, and the crude product was purified by high performance liquid chromatography (0.1% hydrochloric acid condition) and then lyophilized to give compound L100 hydrochloride (18.7 mg). LC-MS: [M+H]⁺ = 444.00.

¹H NMR (400 MHz, DMSO-*d₆*): δ 10.21 (s, 1H), 8.82 (d, *J* = 7.2 Hz, 1H), 8.65 - 8.44 (m, 2H), 7.49 - 7.39 (m, 1H), 7.29 (m, 3H), 7.13 (t, *J* = 8.8 Hz, 2H), 5.15 - 5.11 (m, 1H), 4.37 (m, 2H), 4.25 (m, 2H), 3.33 - 3.24 (m, 1H), 2.98 - 2.85 (m, 3H), 2.85 - 2.63 (m, 4H), 2.52 (d, *J* = 2.0 Hz, 14H), 2.47 - 2.40 (m, 2H), 2.06 (m, 1H), 1.96 - 1.83 (m, 2H), 1.23 (m, 4H).

### Example 117: Synthesis of (2R,4S)-N-((2S)-1-((2-amino-3-cyano-6,7-dihydro-5H-cyclopenta[b]pyridin-5-yl)amino)-1-oxopropan-2-yl)-4-(4-fluorobenzyl)pyrrolidine-2-carboxamide (Compound L101) Hydrochloride

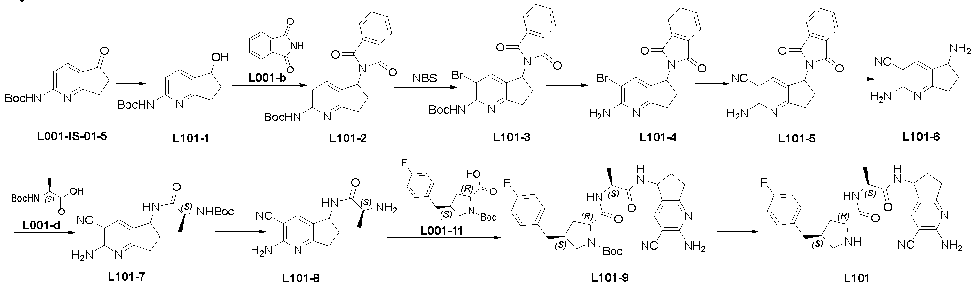

### 117.1: Synthesis of compound L101-1

L001-IS-01-5 (6.0 g, 24.1 mmol) was dissolved in methanol (40 mL) at room temperature, and then sodium borohydride (12.2 g, 322 mmol) was added in batches at 0 °C. After the addition was completed, the reaction liquid was warmed to room temperature and reacted for 2 h. The reaction liquid was quenched by adding a saturated aqueous ammonium chloride solution (20 mL) and concentrated under reduced pressure, and the residue was extracted with dichloromethane (20 mL × 3). The organic phases were combined, washed with a saturated aqueous sodium chloride solution (20 mL × 2), dried over anhydrous sodium sulfate, and filtered, and the filtrate was concentrated under reduced pressure to give compound L101-1 (4.0 g). LC-MS: [M +H]⁺ = 251.10.

### 117.2: Synthesis of compound L101-2

L101-1 (2.0 g, 8.0 mmol) and L001-b (1.4 g, 9.6 mmol) were dissolved in tetrahydrofuran (20 mL), and then triphenylphosphine (1.9 g, 9.6 mmol) was added. Diisopropyl azodicarboxylate (2.5 g, 9.6 mmol) was added dropwise at 0 °C. After the addition was completed, the reaction liquid was reacted at 0 °C for 0.5 h, warmed to room temperature, and then reacted for 1 h. The reaction liquid was quenched by adding water (20 mL) and extracted with ethyl acetate (30 mL×3). The organic phases were combined, washed with saturated brine (30 mL), dried over anhydrous sodium sulfate, and filtered, and the filtrate was concentrated under reduced pressure. The residue was purified by silica gel column chromatography (petroleum ether/ethyl acetate = 5/1) to give compound L101-2 (2.1 g, yield: 69.3%). LC-MS: [M+H]⁺ =380.00.

### 117.3: Synthesis of compound L101-3

L101-2 (2.1 g, 5.54 mmol) was dissolved in acetonitrile (20 mL), and then N-bromosuccinimide (2.9 g, 16.62 mmol) was added. The reaction liquid was reacted at room temperature for 16 h. The reaction liquid was added with water (20 mL) for dilution and extracted with ethyl acetate (20 mL × 3). The organic phases were combined, dried over anhydrous sodium sulfate, and filtered, and the filtrate was concentrated under reduced pressure. The residue was purified by silica gel column chromatography (petroleum ether/ethyl acetate = 4/1) to give compound L101-3 (1.4 g, yield: 55.2%). LC-MS: [M+H]⁺ = 457.80.

### 117.4: Synthesis of compound L101-4

L101-3 (1.4 g, 3.05 mmol) was dissolved in dichloromethane (10 mL), and then a solution of hydrogen chloride in 1,4-dioxane (10 mL, 4 M) was added. The reaction liquid was reacted at room temperature for 1 h. The reaction liquid was concentrated under reduced pressure, and the residue was added with diluted hydrochloric acid (10 mL, 1 M) for dissolving and the solution was extracted with ethyl acetate (10 mL). The organic phase was discarded. The aqueous phase was adjusted to pH = 8 with a saturated aqueous sodium bicarbonate solution and extracted with ethyl acetate (20 mL × 3). The organic phases were combined, dried over anhydrous sodium sulfate, and filtered, and the filtrate was concentrated under reduced pressure to give compound L101-4 (500 mg, yield: 45.9%). LC-MS: [M+H]⁺ = 359.80. 117.5: Synthesis of compound L101-5

L101-4 (500 mg, 1.40 mmol) was dissolved in N,N-dimethylformamide (10 mL), and then zinc cyanide (492 mg, 4.19 mmol), zinc powder (9 mg, 0.14 mmol), 1,1'-bis(diphenylphosphino)ferrocene (78 mg, 0.14 mmol), and tris(dibenzylideneacetone)dipalladium (128 mg, 0.14 mmol) were added. The reaction liquid was reacted at 120 °C for 16 h under nitrogen atmosphere. The reaction liquid was cooled to room temperature, added with water (20 mL) for dilution, and extracted with ethyl acetate (20 mL × 3). The organic phases were combined, dried over anhydrous sodium sulfate, and filtered, and the filtrate was concentrated under reduced pressure. The residue was purified by silica gel column chromatography (petroleum ether/ethyl acetate = 1/1) to give compound L101-5 (280 mg, yield: 65.8%). LC-MS: [M+H]⁺ =304.95.

### 117.6: Synthesis of compound L101-6

L101-5 (280 mg, 0.92 mmol) was dissolved in ethanol (3 mL), and then hydrazine hydrate (59 mg, 1.84 mmol) was added. The reaction liquid was reacted at room temperature for 16 h. The reaction liquid was filtered. The filter cake was rinsed with ethanol (5 mL), and the filtrate was concentrated under reduced pressure. The residue was added with ethanol (10 mL). The mixture was stirred for 0.5 h, and a small amount of the solid was not dissolved. The mixture was filtered, and the filtrate was concentrated under reduced pressure. The above operation was repeated twice to give compound L101-6 (100 mg, crude product), which was used directly in the next step. LC-MS: [M+H]⁺ =175.10.

### 117.7: Synthesis of compound L101 hydrochloride

For the synthesis of compound L101 hydrochloride, reference was made to the synthesis method of compound L001, and L001-6 was replaced with L101-6. The crude product obtained by reaction was purified by high performance liquid chromatography (0.1% hydrochloric acid condition) and then lyophilized to give compound L101 hydrochloride (10.2 mg).

¹H NMR (400 MHz, DMSO-*d₆*): δ 10.21 (s, 1H), 8.82 (d, *J* = 7.2 Hz, 1H), 8.65 - 8.44 (m, 2H), 7.49 - 7.39 (m, 1H), 7.29 (m, 3H), 7.13 (t, *J* = 8.8 Hz, 2H), 5.15 - 5.11 (m, 1H), 4.37 (m, 2H), 4.25 (m, 2H), 3.33 - 3.24 (m, 1H), 2.98 - 2.85 (m, 3H), 2.85 - 2.63 (m, 4H), 2.52 (d, *J*= 2.0 Hz, 14H), 2.47 -
2.40 (m, 2H), 2.06 (m, 1H), 1.96 - 1.83 (m, 2H), 1.23 (m, 4H). LC-MS: [M+H]⁺ = 451.00.

### Example 118: Synthesis of (2R,4S)-N-((2S)-1-((2-amino-4-methyl-6,7-dihydro-5H-cyclopenta[b] pyridin-5-yl)amino)-1-oxopropan-2-yl)-4-(4-fluorobenzyl)pyrrolidine-2-carboxamide (Compound L 102)

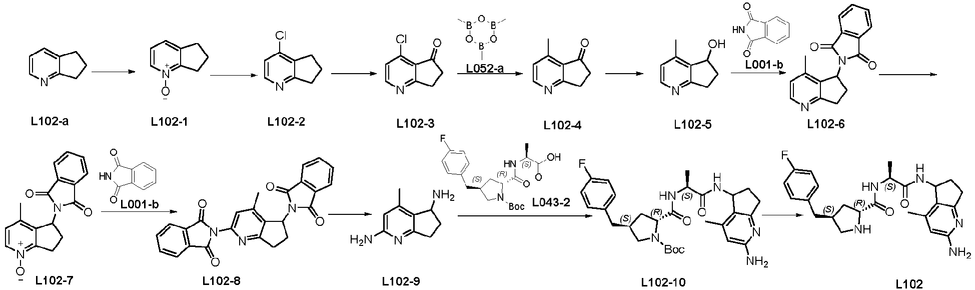

### 118.1: Synthesis of compound L102-1

L102-a (10.0 g, 84.0 mmol) was dissolved in dichloromethane (200 mL), and then m-chloroperoxybenzoic acid (17.1 g, 84.0 mmol) was added in batches at 0 °C. After the addition was completed, the reaction liquid was warmed to room temperature and reacted for 1 h. The reaction liquid was quenched by adding a saturated aqueous sodium thiosulfate solution (100 mL), and then an aqueous sodium carbonate solution was added to adjust the pH to 9-10. The aqueous phase was separated out and directly lyophilized to give a crude product. The crude product was added to dichloromethane (200 mL). The mixture was stirred for 2 h and filtered, and the filtrate was concentrated under reduced pressure. The residue was purified by silica gel column chromatography (ethyl acetate/petroleum ether = 1/10) to give compound L102-1 (9.5 g, yield: 83.8%). LC-MS: [M+H]⁺ = 136.10.

### 118.2: Synthesis of compound L102-2

L102-1 (9.5 g, 70.4 mmol) and lithium chloride (2.96 g, 70.4 mmol) were dissolved in acetonitrile (200 mL), and then phosphorus oxychloride (32.3 g, 211.2 mmol) was added slowly at 0 °C. After the addition was completed, the reaction liquid was warmed to 80 °C and reacted for 12 h. The reaction liquid was poured into ice water (200 mL) to quench the reaction, adjusted to pH = 9-10 with a saturated aqueous sodium bicarbonate solution, and extracted with ethyl acetate (200 mL × 3). The organic phases were combined, dried over anhydrous sodium sulfate, and filtered, and the filtrate was concentrated under reduced pressure. The residue was purified by silica gel column chromatography (ethyl acetate/petroleum ether = 1/1) to give compound L102-2 (9.5 g, yield: 88.2%). LC-MS: [M+H]⁺ = 154.10.

### 118.3: Synthesis of compound L102-3

L102-2 (9.0 g, 58.8 mmol) and anhydrous magnesium sulfate (17.6 g, 147.0 mmol) were dissolved in tert-butanol (90 mL) and water (270 mL), and then potassium permanganate (23.2 g, 147.0 mmol) was added in batches at 0 °C. After the addition was completed, the reaction liquid was warmed to room temperature and reacted for 12 h. The reaction liquid was filtered, and the filter cake was washed with ethyl acetate (100 mL). The organic phase was separated out, and the aqueous phase was extracted with ethyl acetate (200 mL × 2). The organic phases were combined, dried over anhydrous sodium sulfate, and filtered, and the filtrate was concentrated under reduced pressure. The residue was purified by silica gel column chromatography (ethyl acetate/petroleum ether = 1/1) to give compound L102-3 (2.7 g, yield: 27.5%). LC-MS: [M+H]⁺ = 168.10.

### 118.4: Synthesis of compound L102-4

L102-3 (1.9 g, 11.4 mmol) and L052-a (4.8 g, 22.8 mmol) were dissolved in 1,4-dioxane (20 mL) and water (2 mL), and then [1,1'-bis(diphenylphosphino)ferrocene]dichloropalladium(II) (840 mg, 1.15 mmol) and cesium carbonate (7.4 g, 22.8 mmol) were added. The reaction liquid was reacted at 90 °C for 2 h under nitrogen atmosphere. The reaction liquid was cooled to room temperature, added with water (40 mL) for dilution, and extracted with ethyl acetate (40 mL × 3). The combined organic phases were dried over anhydrous sodium sulfate and filtered, and the filtrate was concentrated under reduced pressure. The residue was purified by silica gel column chromatography (ethyl acetate/petroleum ether = 1/1) to give compound L102-4 (1.5 g, yield: 89.3%). LC-MS: [M+H]⁺ = 148.10.

### 118.5: Synthesis of compound L102

For the synthesis of compound L102, reference was made to the synthesis method of compound L035, and L035-1 was replaced with L102-4. The crude product obtained by reaction was purified by high performance liquid chromatography (0.1% hydrochloric acid condition) and normal phase chromatography (0.1% ethylenediamine condition) and then lyophilized to give compound L102 (7.7 mg, yield: 13.5%).

¹H NMR (400 MHz, DMSO-*d₆*): δ 8.34-8.27 (m, 1H), 8.16 (dd, *J* = 8.0, 4.0 Hz, 1H), 7.22 - 7.19 (m, 2H), 7.13 - 7.05 (m, 2H), 6.03 (d, *J* = 4.4 Hz, 1H), 5.73 (s, 2H), 5.22 - 5.11 (m, 1H), 4.24 - 4.11 (m, 1H), 3.82 (d, *J* = 7.2 Hz, 1H), 3.01 - 2.89 (m, 1H), 2.79 - 2.77 (m, 1H), 2.67 - 2.63 (m, 1H), 2.57 - 2.55 (m, 3H), 2.33 - 2.13 (m, 2H), 2.01-1.92 (m, 3H), 1.76 (t, *J* = 7.2 Hz, 2H), 1.67 -1.64 (m, 1H), 1.18 - 1.13 (m, 3H). LC-MS: [M+H]⁺ = 440.05.

### Example 119: Synthesis of (2R,4S)-N-((2S)-1-((2-amino-4-chloro-6,7-dihydro-5H-cyclopenta[b] pyridin-5-yl)amino)-1-oxopropan-2-yl)-4-(4-fluorobenzyl)pyrrolidine-2-carboxamide (Compound L103) Hydrochloride

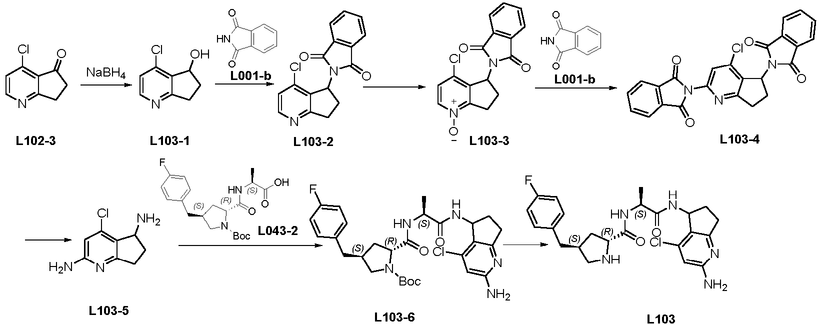

For the synthesis of compound L103, reference was made to the synthesis method of compound L035, and L035-1 was replaced with L102-3. The crude product obtained by reaction was purified by high performance liquid chromatography (0.1% hydrochloric acid condition) and then lyophilized to give compound L103 hydrochloride (20.5 mg, yield: 51.5%).

¹H NMR (400 MHz, DMSO-*d₆*): δ 10.24 (s, 1H), 8.79 (t, *J =* 7.6 Hz, 1H), 8.58 - 8.51 (m, 2H), 7.99 (s, 1H), 7.28 (dd, *J =* 8.4, 5.6 Hz, 2H), 7.12 (t, *J* = 8.8 Hz, 2H), 6.85 (s, 1H), 5.33 - 5.17 (m, 1H), 4.37 - 4.35 (m, 1H), 4.22 - 4.20 (m, 1H), 3.25 (d, *J* = 6.7 Hz, 1H), 3.19 - 3.08 (m, 1H), 2.94 - 2.83 (m, 2H), 2.69 - 2.64 (m, 3H), 2.47 - 2.40 (m, 1H), 2.11 - 1.97 (m, 1H), 1.94 - 1.78 (m, 2H), 1.23 - 1.17 (m, 3H). LC-MS: [M+H]⁺ = 459.95.

### Example 120: Synthesis of (1R,3S,5R)-N-((R)-2-amino-6,7-dihydro-5H-cyclopenta[b]pyridin-5-yl)-2-((2R,4S)-4-(4-fluorobenzyl)pyrrolidine-2-carbonyl)-2-azabicyclo[3.1.0]hexane-3-carboxamide (Compound L104-IS-01) Hydrochloride and (1S,3S,5S)-N-((R)-2-amino-6,7-dihydro-5H-cyclopenta [b]pyridin-5-yl)-2-((2R,4S)-4-(4-fluorobenzyl)pyrrolidine-2-carbonyl)-2-azabicyclo[3.1.0]hexane-3-carboxamide (Compound L104-IS-02) Hydrochloride

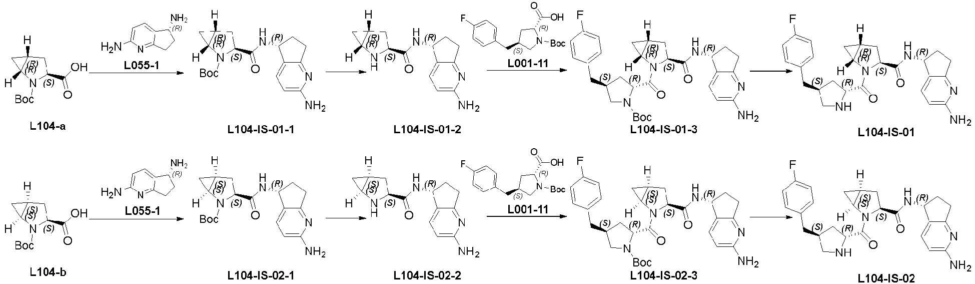

For the synthesis of compound L104-IS-01 hydrochloride, reference was made to the synthesis method of compound L001, L001-d was replaced with L104-a, and L001-6 was replaced with L055-1. The crude product obtained by reaction was purified by high performance liquid chromatography (0.1% hydrochloric acid condition) and then lyophilized to give compound L104-IS-01 hydrochloride (227.1 mg, yield: 39.6%).

¹H NMR (400 MHz, DMSO-*d₆*): δ 10.51 (s, 1H), 8.61 (d, *J* = 5.2 Hz, 1H), 8.50 (d, *J* = 8.0 Hz, 1H), 8.04 (s, 2H), 7.75 - 7.68 (m, 1H), 7.28 (m, 2H), 7.13 (dd, *J* = 14.8, 6.0 Hz, 2H), 6.82 (dd, *J* = 15.2, 9.2 Hz, 1H), 5.15 (q, *J* = 7.6 Hz, 1H), 4.98 - 4.83 (m, 1H), 4.18 - 4.05 (m, 1H), 3.77 - 3.70 (m, 1H), 3.26 (d, *J* = 6.0 Hz, 1H), 3.02 (m, 1H), 2.97 - 2.85 (m, 2H), 2.76 (d, *J* = 7.6 Hz, 2H), 2.49 - 2.37 (m, 3H), 2.31 - 2.04 (m, 4H), 1.96 (m, 1H), 1.88 - 1.76 (m, 1H), 0.90 - 0.81 (m, 1H), 0.55 (m, 1H). LC-MS: [M+H]⁺ = 464.10.

For the synthesis of compound L104-IS-02 hydrochloride, reference was made to the synthesis method of compound L001, L001-d was replaced with L104-b, and L001-6 was replaced with L055-1. The crude product obtained by reaction was purified by high performance liquid chromatography (0.1% hydrochloric acid condition) and then lyophilized to give compound L104-IS-02 hydrochloride (227.1 mg, yield: 39.6%).

¹H NMR (400 MHz, DMSO-*d₆*): δ 10.70 - 10.59 (m, 1H), 8.99 - 8.52 (m, 2H), 8.10 (s, 2H), 7.76 - 7.68 (m, 1H), 7.32 - 7.24 (m, 2H), 7.14 - 7.08 (m, 2H), 6.87 - 6.82 (m, 1H), 5.14 - 5.09 (m, 1H), 4.64 - 4.57 (m, 1H), 3.86 - 3.84 (m, 1H), 3.53 - 3.49 (m, 1H), 3.09 - 2.85 (m, 3H), 2.83 - 2.66 (m, 2H), 2.62 - 2.51 (m, 2H), 2.45 - 2.38 (m, 2H), 2.34 - 2.27 (m, 1H), 2.09 - 2.00 (m, 1H), 1.98 - 1.80 (m, 2H), 1.75 - 1.69 (m, 1H), 1.16 - 1.14 (m, 1H), 0.88 - 0.76 (m, 1H). LC-MS: [M+H]⁺ = 464.00.

### Example 121: Synthesis of (2R,4S)-N-((S)-1-(((R)-2-amino-6,7-dihydro-5H-cyclopenta[b]pyridin-5-yl)amino)-1-oxobutan-2-yl)-4-(4-fluorobenzyl)pyrrolidine-2-carboxamide (Compound L105) Hydrochloride

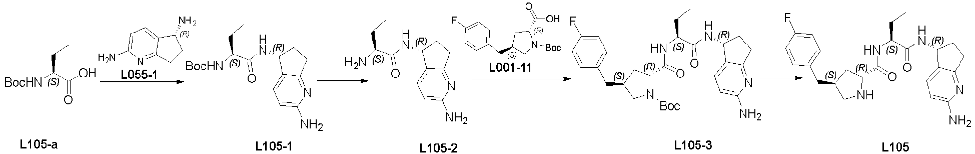

For the synthesis of compound L105, reference was made to the synthesis method of compound L001, L001-6 was replaced with L055-1, and L001-d was replaced with L105-a. The crude product obtained by reaction was purified by high performance liquid chromatography (0.1% hydrochloric acid condition) and then lyophilized to give compound L105 hydrochloride (14.8 mg, yield: 12.0%).

¹H NMR (400 MHz, DMSO-*d₆*): δ 10.70 - 10.59 (m, 1H), 8.99 - 8.52 (m, 2H), 8.10 (s, 2H), 7.76 - 7.68 (m, 1H), 7.32 - 7.24 (m, 2H), 7.14 - 7.08 (m, 2H), 6.87 - 6.82 (m, 1H), 5.14 - 5.09 (m, 1H), 4.64 - 4.57 (m, 1H), 3.86 - 3.84 (m, 1H), 3.53 - 3.49 (m, 1H), 3.09 - 2.85 (m, 3H), 2.83 - 2.66 (m, 2H), 2.62 - 2.51 (m, 2H), 2.45 - 2.38 (m, 2H), 2.34 - 2.27 (m, 1H), 2.09 - 2.00 (m, 1H), 1.98 - 1.80 (m, 2H), 1.75 - 1.69 (m, 1H), 1.16 - 1.14 (m, 1H), 0.88 - 0.76 (m, 1H). LC-MS: [M+H]⁺ = 440.05.

### Example 122: Synthesis of (2R,4S)-N-((R)-1-(((R)-2-amino-6,7-dihydro-5H-cyclopenta[b]pyridin-5-yl)amino)-3-fluoro-1-oxopropan-2-yl)-4-(4-fluorobenzyl)pyrrolidine-2-carboxamide (Compound L106) Hydrochloride

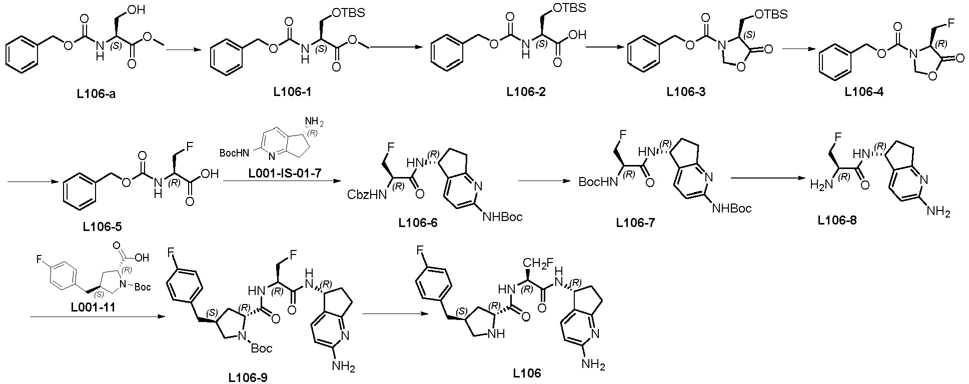

### 122.1: Synthesis of compound L106-1

L106-a (5.0 g, 19.8 mmol) was dissolved in dichloromethane (150 mL), and then imidazole (2.7 g, 39.6 mmol), 4-dimethylaminopyridine (0.48 g, 3.96 mmol), and tert-butyldimethylchlorosilane (4.5 g, 29.7 mmol) were added under an ice-water bath. After the addition was completed, the reaction liquid was warmed to room temperature and reacted for 16 h. The reaction liquid was added with water (100 mL) for dilution and extracted with dichloromethane (50 mL × 2). The organic phases were combined, washed with a saturated aqueous sodium chloride solution (100 mL), dried over anhydrous sodium sulfate, and filtered, and the filtrate was concentrated under reduced pressure. The residue was purified by silica gel column chromatography (ethyl acetate/petroleum ether = 1/10) to give compound L106-1 (7.2 g, yield: 99.0%). LC-MS: [M+H]⁺ = 368.00.

### 122.2: Synthesis of compound L106-2

L106-1 (7.0 g, 19.1 mmol) was dissolved in methanol (50 mL) and tetrahydrofuran (50 mL), and then a solution of lithium hydroxide monohydrate (1.6 g, 38.2 mmol) in water (12.5 mL) was added dropwise to the above reaction liquid. The reaction liquid was reacted at room temperature for 16 h. Petroleum ether (50 mL) was added to the reaction liquid. Liquid separation was performed, and the organic phase was discarded. The aqueous phase was adjusted to pH = 4-5 with diluted hydrochloric acid (1 M) and extracted with ethyl acetate (50 mL×3). The organic phases were combined, washed with a saturated aqueous sodium chloride solution (100 mL), dried over anhydrous sodium sulfate, and filtered, and the filtrate was concentrated under reduced pressure. The residue was purified by silica gel column chromatography (ethyl acetate/petroleum ether = 3/7) to give compound L106-2 (2.3 g, yield: 34.3%). LC-MS: [M+H]⁺ = 354.00.

### 122.3: Synthesis of compound L106-3

L106-2 (2.3 g, 6.5 mmol) was added to toluene (130 mL) at room temperature, and then paraformaldehyde (1.8 g, 19.5 mmol) and p-toluenesulfonic acid (118 mg, 0.65 mmol) were added. The reaction liquid was reacted at 60 °C for 0.5 h, warmed to 80 °C and reacted for 0.5 h, and then reacted at 120 °C for 2 h. The reaction liquid was cooled to 60 °C, supplemented with paraformaldehyde (1.8 g, 19.5 mmol), and warmed to 120 °C and reacted for another 2 h. The operation was repeated three times. The reaction liquid was cooled to room temperature, poured into an aqueous sodium bicarbonate solution (150 mL), and extracted with ethyl acetate (50 mL × 3). The organic phases were combined, washed with saturated brine (150 mL), dried over anhydrous sodium sulfate, and filtered, and the filtrate was concentrated under reduced pressure. The residue was purified by silica gel column chromatography (ethyl acetate/petroleum ether = 1/10) to give compound L106-3 (500 mg, yield: 20.8%). LC-MS: [M+H]⁺ = 366.00.

### 122.4: Synthesis of compound L106-4

L106-3 (500 mg, 1.37 mmol) was dissolved in dichloromethane (6 mL), and then pyridine hydrofluoride (0.28 mL, 21.9 mmol) and bis(2-methoxyethyl)aminosulfur trifluoride (0.5 mL, 5.48 mmol) were added dropwise at 0 °C. After the addition was completed, the reaction liquid was warmed to room temperature and reacted for 16 h. Water (10 mL) was added to the reaction liquid, and the mixture was stirred for another 10 min. The organic phase was separated out, and the aqueous phase was extracted with dichloromethane (10 mL × 2). The organic phases were combined, washed with a saturated aqueous sodium chloride solution (20 mL), dried over anhydrous sodium sulfate, and filtered, and the filtrate was concentrated under reduced pressure. The residue was purified by silica gel column chromatography (ethyl acetate/petroleum ether = 1/4) to give compound L106-4 (180 mg, yield: 51.0%).

### 122.5: Synthesis of compound L106-5

L106-4 (180 mg, 0.71 mmol) was dissolved in 1,4-dioxane (2 mL), and an diluted aqueous hydrochloric acid solution (2 mL, 2 M) was added. The reaction liquid was reacted at 60 °C for 2 h. The reaction liquid was poured into an aqueous ammonium chloride solution (5 mL) and extracted with ethyl acetate (3 mL × 3). The organic phase was dried over anhydrous sodium sulfate and filtered, and the filtrate was concentrated under reduced pressure to give compound L106-5 (47 mg, yield: 97.9%). LC-MS: [M+H]⁺ = 263.95.

### 122.6: Synthesis of compound L106-6

L001-IS-01-7 (63 mg, 0.254 mmol) was dissolved in dichloromethane (1 mL), and then N,N-diisopropylethylamine (76 mg, 0.585 mmol) was added. The reaction liquid was reacted at room temperature for 10 min. Then the above reaction system was added to a solution of L106-5 (47 mg, 0.195 mmol) and 1-propylphosphoric anhydride (197 mg, 0.293 mmol, 50% in ethyl acetate) in dichloromethane (1 mL). The reaction liquid was reacted at room temperature for another 1 h. The reaction liquid was added with a saturated aqueous sodium chloride solution (3 mL) for dilution and extracted with dichloromethane (3 mL × 3). The organic phase was dried over anhydrous sodium sulfate and filtered, and the filtrate was concentrated under reduced pressure. The residue was purified by silica gel thin-layer chromatography to give compound L106-6 (67 mg, yield: 72.8%). LC-MS: [M+H]⁺ = 473.00.

### 122.7: Synthesis of compound L106-7

L106-6 (67 mg, 0.142 mmol) was dissolved in methanol (2 mL), and then di-tert-butyl dicarbonate (135 mg, 0.426 mmol) and palladium on carbon (67 mg, 10% wt) were added. The reaction liquid was reacted for 2 h under hydrogen atmosphere. The reaction liquid was filtered through celite. The filter cake was washed with methanol (10 mL), and the filtrate was concentrated under reduced pressure to give compound L106-7 (62 mg, crude product), which could be used directly in the next step. LC-MS: [M+H]⁺ = 439.05.

### 122.8: Synthesis of compound L106-8

L106-7 (62 mg, crude product) was dissolved in dichloromethane (2 mL), and then a solution of hydrogen chloride in 1,4-dioxane (2 mL, 4 M) was added. The reaction liquid was reacted at room temperature for 1 h. The reaction liquid was concentrated under reduced pressure to give compound L106-8 (58 mg, crude product), which was used directly in the next step. LC-MS: [M+H]⁺ = 239.05.

### 122.9: Synthesis of compound L106 hydrochloride

For the synthesis of compound L106, reference was made to the synthesis method of compound L001, and L001-7 was replaced with L106-8. The crude product obtained by reaction was purified by high performance liquid chromatography (0.1% hydrochloric acid condition) and then lyophilized to give compound L106 hydrochloride (9.6 mg, yield: 26.8%).

¹H NMR (400 MHz, DMSO-*d₆*): δ 10.32 (s, 1H), 9.13 (d, *J* = 7.2 Hz, 1H), 8.81 (d, *J* = 7.6 Hz, 1H), 8.57 (s, 1H), 7.93 (s, 2H), 7.68 (d, *J* = 8.8 Hz, 1H), 7.29 (m, 2H), 7.13 (t, *J* = 8.8 Hz, 2H), 6.80 (d, *J* = 8.8 Hz, 1H), 5.18 (m, 1H), 4.74 - 4.41 (m, 4H), 3.26 (d, *J* = 6.0 Hz, 1H), 3.10 - 2.99 (m, 1H), 2.91 (m, 2H), 2.70 (m, 2H), 2.45 (d, *J* = 4.8 Hz, 2H), 2.09 (m, 1H), 2.00 - 1.87 (m, 2H). LC-MS: [M+H]⁺ = 444.00.

### Example 123: Synthesis of (2R,4S)-N-(3-(((R)-2-amino-6,7-dihydro-5H-cyclopenta[b]pyridin-5-yl) amino)-1,1,1-trifluoro-3-oxopropan-2-yl)-4-(4-fluorobenzyl)pyrrolidine-2-carboxamide (Compound L 107)

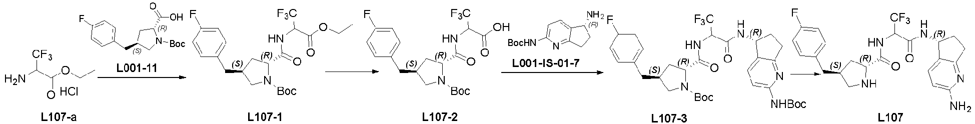

### 123.1: Synthesis of compound L107-1

L107-a (1.0 g, 4.82 mmol) and L001-11 (1.42 g, 4.38 mmol) were dissolved in dichloromethane (20 mL), and then N,N-diisopropylethylamine (1.42 g, 10.95 mmol) was added. 1-Propylphosphoric anhydride (1.34 g, 2.11 mmol, 50% in ethyl acetate) was then added at 0 °C. After the addition was completed, the reaction liquid was warmed to room temperature and reacted for 3 h. The reaction liquid was added with water (30 mL) for dilution and extracted with dichloromethane (20 mL × 3). The organic phases were combined, dried over anhydrous sodium sulfate, and filtered, and the filtrate was concentrated under reduced pressure. The residue was purified by silica gel column chromatography (petroleum ether/ethyl acetate = 5/1) to give compound L107-1 (1.68 g, yield: 80.51%). LC-MS: [M-Boc+H]⁺ = 376.95.

### 123.2: Synthesis of compound L107-2

L107-1 (500 mg, 1.07 mmol) was added to acetic acid (5 mL), and then concentrated hydrochloric acid (1 mL, 12 M) was added. The reaction liquid was reacted at 60 °C for 16 h. The reaction liquid was cooled to room temperature, added with water (2 mL) for dilution, and adjusted to pH = 8-9 with a saturated aqueous potassium carbonate solution, and then di-tert-butyl dicarbonate (460 mg, 2.14 mmol) was added. The reaction liquid was stirred at room temperature for another 1 h. The reaction liquid was extracted with ethyl acetate (10 mL × 2), and the organic phase was discarded. The aqueous phase was adjusted to pH = 3-4 with diluted hydrochloric acid (0.5 M) and extracted with ethyl acetate (10 mL × 3). The combined organic phases were dried over anhydrous sodium sulfate and filtered, and the filtrate was concentrated under reduced pressure. The residue was lyophilized to give compound L107-2 (280 mg, yield: 59.6%). LC-MS: [M-Boc+H]⁺ = 348.95.

### 123.3: Synthesis of compound L107-3

L107-2 (55 mg, 0.13 mmol) and L001-IS-01-7 (36.7 mg, 0.15 mmol) were dissolved in dichloromethane (3 mL), and then N,N-diisopropylethylamine (21 mg, 0.16 mmol) was added. 1-Propylphosphoric anhydride (117 mg, 0.18 mmol, 50% in ethyl acetate) was then added at 0 °C. The reaction liquid was reacted at room temperature for 3 h. The reaction liquid was added with water (10 mL) for dilution and extracted with dichloromethane (10 mL × 3). The organic phases were combined, dried over anhydrous sodium sulfate, and filtered, and the filtrate was concentrated under reduced pressure. The residue was purified by silica gel column chromatography (dichloromethane/methanol = 15/1) to give compound L107-3 (36 mg, yield: 43.4%). LC-MS: [M-Boc+H]⁺ = 680.05.

### 123.4: Synthesis of compound L107 hydrochloride

L107-3 (36 mg, 0.075 mmol) was dissolved in dichloromethane (1 mL), and then a solution of hydrogen chloride in 1,4-dioxane (1 mL, 4 M) was added. The reaction liquid was reacted at room temperature for 1 h. The reaction liquid was concentrated under reduced pressure, and the crude product was purified by high performance liquid chromatography (0.1% hydrochloric acid condition) and then lyophilized to give compound L107 hydrochloride (6.9 mg, yield: 25%).

¹H NMR (400 MHz, DMSO-*d₆*): δ 10.40 (s, 1H), 9.69 (d, *J* = 8.8 Hz, 1H), 9.28 (d, *J* = 7.2 Hz, 1H), 8.61 (s, 1H), 7.98 (s, 2H), 7.74 (d, *J* = 8.8 Hz, 1H), 7.29 (dd, *J* = 8.4, 5.7 Hz, 2H), 7.13 (dd, *J* = 12.4, 5.4 Hz, 2H), 6.83 (d, *J* = 8.8 Hz, 1H), 5.49 - 5.26 (m, 1H), 5.18 (dd, *J* = 13.2, 7.6 Hz, 1H), 4.50 (s, 1H), 3.30 (d, *J* = 5.6 Hz, 2H), 3.14 - 2.99 (m, 1H), 2.99 - 2.86 (m, 2H), 2.81 - 2.62 (m, 2H), 2.59 - 2.52 (m, 1H), 2.22 - 2.07 (m, 1H), 1.98 - 1.76 (m, 2H). LC-MS: [M+H]⁺ = 479.95.

### Example 124: Synthesis of (2R,4S)-N-((S)-2-(((R)-2-amino-6,7-dihydro-5H-cyclopenta[b]pyridin-5-yl)amino)-1-cyclopropyl-2-oxoethyl)-4-(4-fluorobenzyl)pyrrolidine-2-carboxamide (Compound L108) Hydrochloride

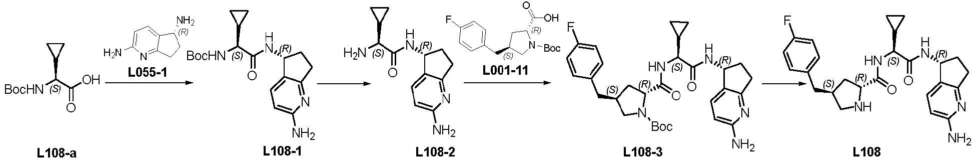

For the synthesis of compound L108, reference was made to the synthesis method of compound L001, L001-d was replaced with L108-a, and L001-6 was replaced with L055-1. The crude product obtained by reaction was purified by high performance liquid chromatography (0.1% hydrochloric acid condition) and then lyophilized to give compound L108 hydrochloride (125.5 mg).

¹H NMR (400 MHz, DMSO-*d₆*): δ 10.52 (s, 1H), 8.97 (d, *J* = 4.0 Hz, 1H), 8.63 - 8.50 (m, 2H), 8.01 (s, 2H), 7.70 (d, *J* = 8.0 Hz, 1H), 7.32 - 7.29 (m, 2H), 7.15 - 7.11 (m, 2H), 6.83 (d, *J* = 8.0 Hz, 1H), 5.16 (dd, *J* = 16.0, 8.0 Hz, 1H), 4.40 (s, 1H), 3.77 - 3.73 (m, 1H), 3.30 - 3.23 (m, 1H), 3.08 - 3.00 (m, 1H), 2.95 - 2.86 (m, 2H), 2.77 - 2.64 (m, 2H), 2.47 - 2.43 (m, 1H), 2.14 - 2.07 (m, 1H), 1.99 - 1.84 (m, 2H), 1.30 - 1.19 (m, 1H), 1.11 - 0.97 (m, 1H), 0.48 - 0.37 (m, 3H), 0.28 - 0.18 (m, 1H). LC-MS: [M+H]⁺ = 452.05.

### Example 125: Synthesis of (2R,4S)-N-(1-(((R)-2-amino-6,7-dihydro-5H-cyclopenta[b]pyridin-5-yl) carbamoyl)cyclopropyl)-4-(4-fluorobenzyl)pyrrolidine-2-carboxamide (Compound L109) Hydrochloride

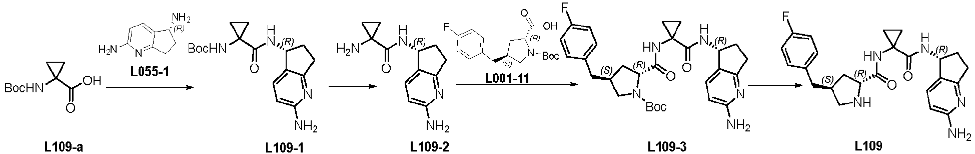

For the synthesis of compound L109, reference was made to the synthesis method of compound L001, L001-d was replaced with L109-a, and L001-6 was replaced with L055-1. The crude product obtained by reaction was purified by high performance liquid chromatography (0.1% hydrochloric acid condition) and then lyophilized to give compound L109 hydrochloride (53 mg, yield: 35.8%).

¹H NMR (400 MHz, DMSO-*d₆*): δ 10.20 (s, 1H), 9.09 (s, 1H), 8.64 (d, *J* = 5.2 Hz, 1H), 8.30 (d, *J* = 8.4 Hz, 1H), 7.99 (s, 1H), 7.63 (d, *J* = 8.8 Hz, 1H), 7.26 (dd, *J* = 8.4, 5.7 Hz, 2H), 7.12 (dd, *J* = 12.4, 5.2 Hz, 2H), 6.80 (d, *J* = 8.8 Hz, 1H), 5.21 (dd, *J* = 14.4, 8.2 Hz, 1H), 4.42 - 4.31 (m, 1H), 3.24 (d, *J* = 6.8 Hz, 1H), 3.07 (m, 1H), 2.88 (m, 2H), 2.75 - 2.61 (m, 2H), 2.47 - 2.36 (m, 2H), 2.09 - 1.97 (m, 1H), 1.92 (t, *J* = 7.6 Hz, 2H), 1.45 - 1.34 (m, 1H), 1.30 - 1.18 (m, 2H), 1.00 - 0.91 (m, 1H), 0.91 - 0.77 (m, 1H). LC-MS: [M+H]⁺ = 438.00.

### Example 126: Synthesis of (2R,4S)-N-((S)-1-(((R)-2-amino-6,7-dihydro-5H-cyclopenta[b]pyridin-5-yl)amino)-1-oxopropan-2-yl)-4-((4-fluorobenzyl)oxy)pyrrolidine-2-carboxamide (Compound L110)

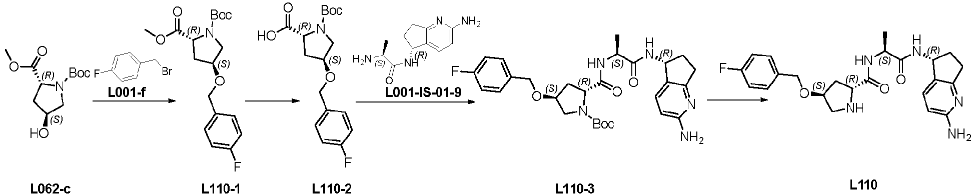

### 126.1: Synthesis of compound L110-1

L062-c (1.0 g, 4.1 mmol) was dissolved in N,N-dimethylformamide (10 mL), and then silver oxide (2.1 g, 9.02 mmol) was added. L001-f (1.7 g, 9.02 mmol) was added dropwise at 0 °C. After the addition was completed, the reaction liquid was warmed to room temperature and reacted for 72 h. Ethyl acetate (10 mL) was added to the reaction liquid. The mixture was stirred for 10 min and filtered to remove the insoluble substance, and the filtrate was added with water (40 mL) for dilution and extracted with ethyl acetate (30 mL × 3). The organic phases were combined, washed with saturated sodium chloride (30 mL), dried over anhydrous sodium sulfate, and filtered, and the filtrate was concentrated under reduced pressure. The residue was purified by high performance liquid chromatography to give compound L110-1 (500 mg, yield: 34.5%). LC-MS: [M-Boc+H]⁺ = 254.05.

### 126.2: Synthesis of compound L110

For the synthesis of compound L110, reference was made to the synthesis method of compound L062-IS-01, and L062-IS-01-1 was replaced with L110-1. The crude product obtained by reaction was purified by high performance liquid chromatography (basic condition) and then lyophilized to give compound L110 (116.3 mg, yield: 35.6%).

¹H NMR (400 MHz, DMSO-*d₆*): δ 8.20 - 8.16 (m, 2H), 7.39 - 7.36 (m, 2H), 7.22 - 7.09 (m, 3H), 6.23 (d, *J* = 8.0 Hz, 1H), 5.83 - 5.82 (m, 2H), 5.09 - 5.06 (m, 1H), 4.42 (s, 2H), 4.26 - 4.19 (m, 1H), 4.04 (s, 1H), 3.74 - 3.70 (m, 1H), 3.01 (d, *J* = 12.0 Hz, 1H), 2.87 - 2.55 (m, 3H), 2.39 - 2.26 (m, 1H), 2.20 - 2.06 (m, 1H), 1.78 - 1.61 (m, 2H), 1.22 (d, *J* = 8.0 Hz, 3H). LC-MS: [M+H]⁺ = 442.00.

### Example 127: Synthesis of N-((S)-1-(((R)-2-amino-6,7-dihydro-5H-cyclopenta[b]pyridin-5-yl)amino)-1-oxopropan-2-yl)-4-benzylpiperidine-2-carboxamide (Compound L111)

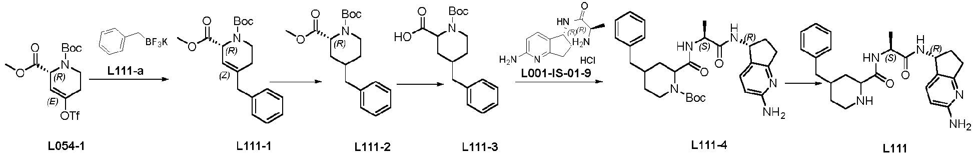

### 127.1: Synthesis of compound L111-1

L054-1 (1.5 g, 3.85 mmol) and L111-a (1.0 g, 5.01 mmol) were dissolved in 1,4-dioxane (20 mL) and water (2 mL), and then potassium carbonate (1.1 g, 7.71 mmol) and [1,1'-bis(diphenylphosphino)ferrocene]dichloropalladium(II) (280 mg, 0.39 mmol) were added. The reaction liquid was reacted at 60 °C for 3 h under nitrogen atmosphere. The reaction liquid was cooled to room temperature, added with water (60 mL) for dilution, and extracted with ethyl acetate (20 mL ×3). The organic phases were combined, washed with saturated brine (20 mL), dried over anhydrous sodium sulfate, and filtered, and the filtrate was concentrated under reduced pressure. The residue was purified by silica gel column chromatography (petroleum ether/ethyl acetate = 15/1) to give compound L111-1 (850 mg, yield: 66.5%). LC-MS: [M-Boc+H]⁺ = 232.10.

### 127.2: Synthesis of compound L111-2

L111-1 (770 mg, 2.32 mmol) was dissolved in methanol (10 mL), and then palladium on carbon (80 mg, 10% wt) was added. The reaction liquid was reacted at room temperature for 4 h under hydrogen atmosphere. The reaction liquid was filtered through celite. The filter cake was washed with methanol (5 mL), and the filtrate was concentrated under reduced pressure to give compound L111-2 (700 mg, yield: 90.4%). LC-MS: [M-Boc+H]⁺ = 234.10.

### 127.3: Synthesis of compound L111-3

L111-2 (500 mg, 1.50 mmol) was dissolved in methanol (4 mL) and water (4 mL), and then lithium hydroxide monohydrate (250 mg, 6.01 mmol) was added. The reaction liquid was reacted at room temperature for 16 h. The reaction liquid was added with water (10 mL) for dilution, adjusted to pH = 4-5 with diluted hydrochloric acid (1 M), and extracted with ethyl acetate (15 mL × 3). The organic phases were combined, dried over anhydrous sodium sulfate, and filtered, and the filtrate was concentrated under reduced pressure to give compound L111-3 (450 mg, yield: 93.9%). LC-MS: [M-Boc+H]⁺ = 220.00.

### 127.4: Synthesis of compound L111

For the synthesis of compound L111, reference was made to the synthesis method of compound L001, L001-11 was replaced with L111-3, and L001-7 was replaced with L001-IS-01-9. The crude product obtained by reaction was purified by high performance liquid chromatography (basic condition) and then lyophilized to give compound L111 (93 mg).

¹H NMR (400 MHz, DMSO-*d₆*): δ 8.20 (d, *J* = 8.0 Hz, 1H), 7.99 (d, *J* = 8.0 Hz, 1H), 7.28 - 7.25 (m, 2H), 7.20 - 7.11 (m, 4H), 6.22 (d, *J* = 8.0 Hz, 1H), 5.83 - 5.81 (m, 2H), 5.08 (dd, *J* = 16.0, 8.0 Hz, 1H), 4.30 - 4.22 (m, 1H), 3.43 (s, 1H), 2.85 - 2.69 (m, 2H), 2.67 - 2.57 (m, 1H), 2.56 - 2.52 (m, 1H), 2.48 - 2.43 (m, 1H), 2.38 - 2.26 (m, 1H), 2.06 - 1.98 (m, 1H), 1.76 - 1.59 (m, 2H), 1.45 - 1.37 (m, 1H), 1.35 - 1.30 (m, 1H), 1.26 - 1.23 (m, 2H), 1.21 (d, *J* = 8.0 Hz, 3H), 1.06 - 0.99 (m, 1H). LC-MS: [M+H]⁺ = 422.10.

### Example 128: Synthesis of (2R)-N-((S)-1-(((R)-2-amino-6,7-dihydro-5H-cyclopenta[b]pyridin-5-yl) amino)-1-oxopropan-2-yl)-4-(3-cyanophenyl)pyrrolidine-2-carboxamide (Compound L112), (2R,4S)-N-((S)-1-(((R)-2-amino-6,7-dihydro-5H-cyclopenta[b]pyridin-5-yl)amino)-1-oxopropan-2-yl)-4-(3-cyanophenyl)pyrrolidine-2-carboxamide (Compound L112-IS-01 or L112-IS-02) Trifluoroacetate, and (2R,4R)-N-((S)-1-(((R)-2-amino-6,7-dihydro-5H-cyclopenta[b]pyridin-5-yl) amino)-1-oxopropan-2-yl)-4-(3-cyanophenyl)pyrrolidine-2-carboxamide (Compound L112-IS-02 or L112-IS-01) Trifluoroacetate

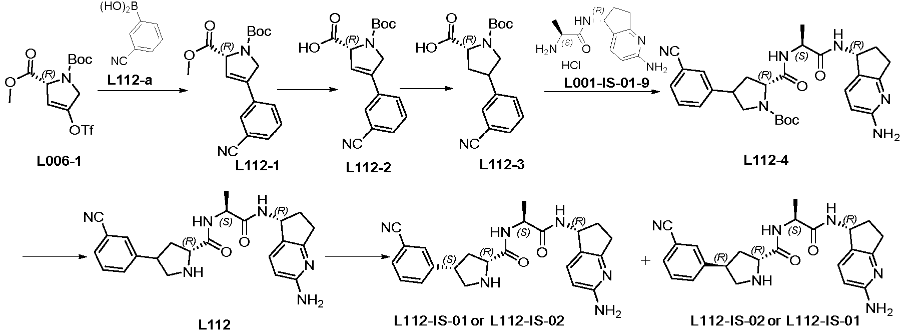

### 128.1: Synthesis of compound L112-2

For the synthesis of compound L112-2, reference was made to the synthesis method of compound L006-3, and L006-b was replaced with L112-a. LC-MS: [M-tBu+H]⁺ = 259.00.

### 128.2: Synthesis of compound L112-3

L112-2 (250 mg, 0.79 mmol) was dissolved in methanol (3 mL), and then palladium on carbon (25 mg, 10% wt) was added. The reaction liquid was reacted at room temperature for 2 h under hydrogen atmosphere. The reaction liquid was filtered through celite. The filter cake was washed with methanol (10 mL), and the filtrate was concentrated under reduced pressure to give compound L112-3 (160 mg, yield: 90.4%). LC-MS: [M- tBu+H]⁺ = 261.00.

### 128.3: Synthesis of compounds L112, L112-IS-01 trifluoroacetate, and L112-IS-02 trifluoroacetate

For the synthesis of compound L112, reference was made to the synthesis method of compound L001-IS-01, and L001-8 was replaced with L112-3. The crude product of compound L 112 was purified by preparative high performance liquid chromatography (0.1% trifluoroacetic acid condition) and then lyophilized to give compound L112-IS-01 trifluoroacetate (87.4 mg, retention time Rt = 13.679 min) and compound L112-IS-02 trifluoroacetate (20.4 mg, retention time Rt = 11.758 min)
L112-IS-01 trifluoroacetate:
   ¹H NMR (400 MHz, DMSO-*d₆*): δ 9.77 (s, 1H), 8.86 - 8.82 (m, 2H), 8.48 (d, *J*= 8.0 Hz, 1H), 7.96 - 7.89 (m, 2H), 7.77 - 7.75 (m, 1H), 7.71 - 7.65 (m, 2H), 7.60 - 7.55 (m, 1H), 6.75 (d, *J* = 8.0 Hz, 1H), 5.16 - 5.13 (m, 1H), 4.37 (s, 1H), 4.33 - 4.26 (m, 1H), 3.77 - 3.73 (m, 2H), 3.26 - 3.21 (m, 1H), 3.03 - 2.80 (m, 3H), 2.48 - 2.40 (m, 1H), 2.02 - 1.85 (m, 2H), 1.26 (d, *J =* 4.0 Hz, 3H). LC-MS: [M+H]⁺ = 419.05.
L112-IS-02 trifluoroacetate:
   ¹H NMR (400 MHz, DMSO-*d₆*): δ 9.66 (s, 1H), 8.92 - 8.78 (m, 2H), 8.45 (d, *J* = 8.0 Hz, 1H), 7.87 (s, 2H), 7.80 - 7.65 (m, 2H), 7.65 - 7.53 (m, 2H), 6.69 (d, *J* = 8.0 Hz, 1H), 5.16 - 5.13 (m, 1H), 4.40 - 4.36 (m, 1H), 4.33 - 4.26 (m, 1H), 3.79 - 3.72 (m, 1H), 3.31 - 3.18 (m, 2H), 3.01 - 2.81 (m, 3H), 2.48 - 2.44 (m, 1H), 2.02 - 1.80 (m, 2H), 1.28 (d, *J* = 8.0 Hz, 3H). LC-MS: [M+H]⁺ = 419.05.

### Example 129: Synthesis of (2R,4S)-N-((2S)-1-((2-amino-7,7-dimethyl-6,7-dihydro-5H-cyclopenta [b]pyridin-5-yl)amino)-1-oxopropan-2-yl)-4-(4-fluorobenzyl)pyrrolidine-2-carboxamide (Compound L113), (2R,4S)-N-((S)-1-(((R)-2-amino-7,7-dimethyl-6,7-dihydro-5H-cyclopenta[b]pyridin-5-yl) amino)-1-oxopropan-2-yl)-4-(4-fluorobenzyl)pyrrolidine-2-carboxamide (Compound L113-IS-01 or L113-IS-02) Hydrochloride, and ((2R,4S)-N-((S)-1-(((S)-2-amino-7,7-dimethyl-6,7-dihydro-5H-cyclopenta[b]pyridin-5-yl)amino)-1-oxopropan-2-yl)-4-(4-fluorobenzyl)pyrrolidine-2-carboxamide (Compound L113-IS-02 or L113-IS-01) Hydrochloride

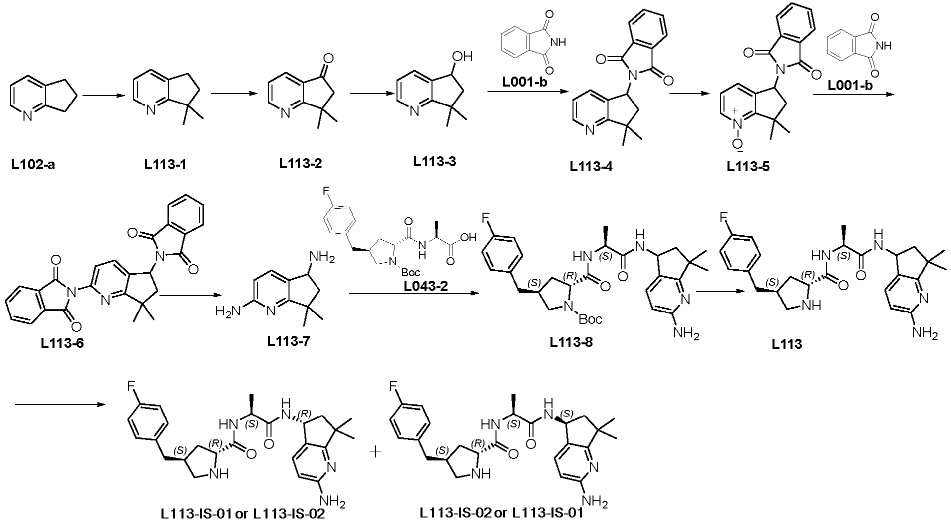

### 129.1: Synthesis of compound L113-1

L102-a (11.9 g, 100.00 mmol) was dissolved in tetrahydrofuran (100 mL) at room temperature, and then the mixture was cooled to -20 °C. Lithium diisopropylamide (55 mL, 110.00 mmol, 2 M) was then added dropwise under nitrogen atmosphere. After the addition was completed, the mixture was reacted at -20 °C for 0.5 h. Iodomethane (15.6 g, 110.00 mmol) was added dropwise to the reaction system. After the addition was completed, the mixture was warmed to room temperature and reacted for 0.5 h. The reaction system was cooled to -20 °C again, and then lithium diisopropylamide (55 mL, 110.00 mmol, 2 M) was added dropwise. After the addition was completed, the mixture was reacted at -20 °C for 0.5 h. Iodomethane (15.6 g, 110.00 mmol) was added dropwise to the reaction system. After the addition was completed, the mixture was warmed to room temperature and reacted for 1 h. The reaction was quenched with a saturated aqueous ammonium chloride solution (100 mL), and the mixture was extracted with ethyl acetate (50 mL × 3). The organic phases were combined, washed with saturated brine (100 mL), dried over anhydrous sodium sulfate, and filtered, and the filtrate was concentrated under reduced pressure. The residue was purified by silica gel column chromatography (petroleum ether/ethyl acetate = 5/1) to give compound L113-1 (4.0 g, yield: 27.2%). LC-MS: [M+H]⁺ = 148.11.

### 129.2: Synthesis of compound L113-2

L113-1 (2.5 g, 17.00 mmol) was dissolved in tert-butanol (60 mL) at room temperature, and then magnesium sulfate (4.1 g, 34.00 mmol) was added. A solution of potassium permanganate (5.4 g, 34.00 mmol) in water (140 mL) was added dropwise at 0 °C. After the addition was completed, the reaction liquid was reacted at room temperature for 2 h. The reaction liquid was added with water (200 mL) for dilution and extracted with ethyl acetate (200 mL × 3). The organic phases were combined, washed with water (200 mL × 3), washed with saturated brine (200 mL), dried over anhydrous sodium sulfate, and filtered, and the filtrate was concentrated under reduced pressure to give compound L113-2 (2.4 g, crude product). LC-MS: [M+H]⁺ = 162.09.

### 129.3: Synthesis of compounds L113, L113-IS-01 hydrochloride, and L113-IS-02 hydrochloride

For the synthesis of compound L113, reference was made to the synthesis method of compound L102, and L102-4 was replaced with L113-2. The crude product of compound L113 was purified by preparative high performance liquid chromatography (0.1% hydrochloric acid condition) and then lyophilized to give compound L113-IS-01 hydrochloride (30.4 mg, retention time Rt = 8.048 min) and compound L113-IS-02 hydrochloride (31.4 mg, retention time Rt = 10.864 min).
L113-IS-01 hydrochloride:
   ¹H NMR (400 MHz, DMSO-*d₆*): δ 10.32 (d, *J =* 8.0 Hz, 1H), 8.84 (d, *J =* 8.0 Hz, 1H), 8.58 - 8.45 (m, 2H), 8.07 (s, 2H), 7.63 (d, *J =* 8.0 Hz, 1H), 7.29 - 7.26 (m, 2H), 7.11 (t, *J =* 8.0 Hz, 2H), 6.84 (d, *J =* 12.0 Hz, 1H), 5.20 - 5.14 (m, 1H), 4.37 - 4.34 (m, 1H), 4.29 - 4.25 (m, 1H), 3.26 - 3.24 (m, 1H), 2.91 - 2.83 (m, 1H), 2.74 - 2.62 (m, 2H), 2.45 - 2.41 (m, 1H), 2.35 - 2.30 (m, 1H), 2.08 - 2.01 (m, 1H), 1.91 - 1.82 (m, 2H), 1.44 (s, 3H), 1.29 (s, 3H), 1.23 (d, *J =* 8.0 Hz, 3H). LC-MS: [M+H]⁺ = 454.26.
L113-IS-02 hydrochloride:
   ¹H NMR (400 MHz, DMSO-*d₆*): δ 10.55 (d, *J* = 8.0 Hz, 1H), 8.91 (d, *J =* 8.0 Hz, 1H), 8.57 - 8.51 (m, 2H), 8.13 (s, 2H), 7.70 (d, *J =* 8.0 Hz, 1H), 7.34 - 7.28 (m, 2H), 7.12 (t, *J =* 8.8 Hz, 2H), 6.88 (d, *J =* 12.0 Hz, 1H), 5.21 - 5.15 (m, 1H), 4.42 - 4.36 (m, 1H), 4.29 - 4.22 (m, 1H), 3.29 - 3.25 (m, 1H), 2.93 - 2.86 (m, 1H), 2.76 - 2.64 (m, 2H), 2.46 - 2.43 (m, 1H), 2.35 - 2.29 (m, 1H), 2.11 - 2.04 (m, 1H), 1.94 - 1.85 (m, 2H), 1.45 (s, 3H), 1.30 (s, 3H), 1.23 (d, *J =* 8.0 Hz, 3H). LC-MS: [M+H]⁺ = 454.26.

### Example 130: Synthesis of (2R,4S)-N-((2S)-1-((2-amino-6,7-dihydro-5H-cyclopenta[d]pyrimidin-5-yl)amino)-1-oxopropan-2-yl)-4-(4-fluorobenzyl)pyrrolidine-2-carboxamide (Compound L114), (2R,4S)-N-((S)-1-(((R)-2-amino-6,7-dihydro-5H-cyclopenta[d]pyrimidin-5-yl)amino)-1-oxopropan-2-yl)-4-(4-fluorobenzyl)pyrrolidine-2-carboxamide (Compound L114-IS-01 or L114-IS-02) Hydrochloride, and (2R,4S)-N-((S)-1-(((S)-2-amino-6,7-dihydro-5H-cyclopenta[d]pyrimidin-5-yl) amino)-1-oxopropan-2-yl)-4-(4-fluorobenzyl)pyrrolidine-2-carboxamide (Compound L114-IS-02 or L114-IS-01) Hydrochloride

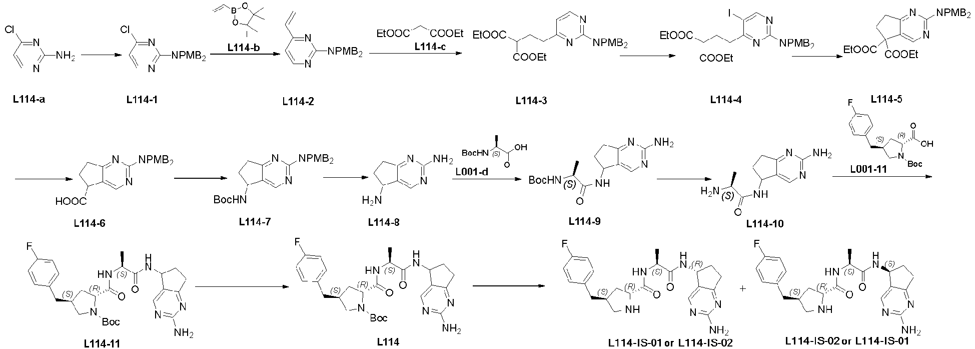

### 130.1: Synthesis of compound L114-1

L114-a (9.0 g, 69.47 mmol) was dissolved in N,N-dimethylformamide (100 mL), and then 4-methoxybenzyl chloride (22.8 g, 145.89 mmol) was added dropwise at 0 °C. The internal temperature of the reaction liquid was controlled to be not exceeding 5 °C. Sodium tert-butoxide (16.0 g, 166.73 mmol) was then added in batches. After the addition was completed, the reaction liquid was reacted at 0 °C for 2 h. The reaction liquid was quenched by adding water (100 mL) and extracted with ethyl acetate (100 mL×3). The organic phases were combined, washed with a saturated aqueous sodium chloride solution (50 mL × 3), dried over anhydrous sodium sulfate, and filtered, and the filtrate was concentrated under reduced pressure. The residue was purified by silica gel column chromatography (petroleum ether/ethyl acetate = 3/1) to give compound L114-1 (13.3 g, yield: 51.77%).

### 130.2: Synthesis of compound L114-2

L114-1 (7.3 g, 19.8 mmol) was dissolved in 1,4-dioxane (60 mL) and water (60 mL), and then L114-b (3.7 g, 23.8 mmol), 1,1-bis(diphenylphosphino)ferrocene dichloropalladium(II) (807 mg, 0.99 mmol) and sodium carbonate (8.4 g, 79.2 mmol) were added. The reaction liquid was reacted at 80 °C for 7 h under nitrogen atmosphere. The reaction liquid was cooled to room temperature, added with water (150 mL) for dilution, and extracted with ethyl acetate (100 mL × 3). The organic phases were combined, dried over anhydrous sodium sulfate, and filtered, and the filtrate was concentrated under reduced pressure. The residue was purified by silica gel column chromatography (petroleum ether/ethyl acetate = 3/1) to give compound L114-2 (6.7 g, yield: 93.97%). LC-MS: [M+H]⁺ = 362.00.

### 130.3: Synthesis of compound L114-3

L114-c (14.8 g, 92.75 mmol) was dissolved in N,N-dimethylformamide (60 mL), and then 1,8-diazabicycloundec-7-ene (14.1 g, 92.75 mmol) was added. A solution of L114-2 (6.7 g, 18.55 mmol) in toluene (60 mL) was then added dropwise. After the addition was completed, the reaction liquid was reacted at 50 °C for 16 h. The reaction liquid was cooled to room temperature, poured into an aqueous acetic acid solution (120 mL, 1%), stirred for 10 min, and extracted with ethyl acetate (100 mL × 3). The organic phases were combined, washed with water (100 mL × 3), washed with an aqueous sodium chloride solution (100 mL), dried over anhydrous sodium sulfate, and filtered, and the filtrate was concentrated under reduced pressure. The residue was purified by silica gel column chromatography (petroleum ether/ethyl acetate = 3/1) to give compound L114-3 (8.0 g, yield: 82.7%). LC-MS: [M+H]⁺ = 522.10.

### 130.4: Synthesis of compound L114-4

N-Iodosuccinimide (4.5 g, 20.13 mmol) was dissolved in acetonitrile (70 mL), and then a solution of trifluoroacetic acid (76 mg, 0.67 mmol) in acetonitrile (5 mL) was added at 0 °C. The reaction liquid was reacted for 10 min, and then a solution of L114-3 (7.0 g, 13.42 mmol) in acetonitrile (70 mL) was added. The reaction liquid was reacted at 0 °C for another 4 h. The reaction liquid was poured into a saturated aqueous sodium thiosulfate solution (150 mL), and then a saturated aqueous sodium bicarbonate solution (200 mL) was added. The mixture was extracted with ethyl acetate (200 mL × 3). The combined organic phases were dried over anhydrous sodium sulfate and filtered, and the filtrate was concentrated under reduced pressure. The residue was purified by silica gel column chromatography (petroleum ether/ethyl acetate = 3/1) to give compound L114-4 (7.9 g, yield: 90.91%). LC-MS: [M+H]⁺ = 648.25.

### 130.5: Synthesis of compound L114-5

L114-4 (8.5 g, 13.13 mmol), copper(I) iodide (2.5 g, 13.13 mmol), 2-picolinic acid (3.2 g, 26.25 mmol), cesium carbonate (17.1 g, 52.51 mmol), and water (236 mg, 13.13 mmol) were added to N,N-dimethylacetamide (80 mL), and the reaction liquid was reacted at 60 °C for 16 h under nitrogen atmosphere. The reaction liquid was poured into water (100 mL) and extracted with ethyl acetate (100 mL × 3). The organic phases were combined, washed with a saturated aqueous ammonium chloride solution (50 mL × 3), dried over anhydrous sodium sulfate, and filtered, and the filtrate was concentrated under reduced pressure. The residue was purified by silica gel column chromatography (ethyl acetate/petroleum ether = 1/3) to give compound L114-5 (5.5 g, yield: 80.65%). LC-MS [M+H]⁺ = 520.20.

### 130.6: Synthesis of compound L114-6

L114-5 (5.6 g, 10.78 mmol) was dissolved in N,N-dimethylacetamide (100 mL) and water (10 mL), and then sodium hydroxide (2.8 g, 70.05 mmol) was added. The reaction liquid was reacted at 40 °C for 2 h. The reaction liquid was adjusted to pH = 3-4 with diluted hydrochloric acid (1 M) and extracted with ethyl acetate (100 mL × 3). The organic phases were combined, washed with a saturated aqueous ammonium chloride solution (50 mL×3), dried over anhydrous sodium sulfate, and filtered, and the filtrate was concentrated under reduced pressure. The residue was purified by silica gel column chromatography (methanol/dichloromethane = 1/20) to give compound L114-6 (3.9 g, yield: 86.28%). LC-MS [M+H]⁺ = 420.05.

### 130.7: Synthesis of compound L114-7

L114-6 (4.0 g, 9.54 mmol) was dissolved in tert-butanol (80 mL), and then N,N-diisopropylethylamine (1.8 g, 14.30 mmol) and diphenylphosphoryl azide (3.9 g, 14.30 mmol) were added. The reaction liquid was reacted at 90 °C for 2 h under nitrogen atmosphere. The reaction liquid was poured into a saturated aqueous ammonium chloride solution (100 mL) and extracted with ethyl acetate (100 mL × 3). The combined organic phases were dried over anhydrous sodium sulfate and filtered, and the filtrate was concentrated under reduced pressure. The residue was purified by silica gel column chromatography (ethyl acetate/petroleum ether = 1/2) to give compound L114-7 (2.4 g, yield: 50.00%). LC-MS [M+H]⁺ = 491.10.

### 130.8: Synthesis of compound L114-8

L114-7 (2.4 g, 4.89 mmol) was dissolved in trifluoroacetic acid (50 mL), and then the reaction liquid was reacted at 50 °C for 16 h. The reaction liquid was concentrated under reduced pressure to give compound L114-8 trifluoroacetate (1.7 g, crude product), which was used directly in the next step. LC-MS [M+H]⁺ = 151.15.

### 130.9: Synthesis of compounds L114, L114-IS-01 hydrochloride, and L114-IS-02 hydrochloride

For the synthesis of compound L114, reference was made to the synthesis method of compound L001, and L001-6 was replaced with L114-8. The crude product of compound L114 was purified by preparative high performance liquid chromatography (0.1% hydrochloric acid condition) and then lyophilized to give compound L114-IS-01 hydrochloride (6.6 mg, yield: 6.27%, retention time Rt = 10.975 min) and compound L114-IS-02 hydrochloride (6.2 mg, yield: 5.89%, retention time Rt = 15.054 min).
L114-IS-01 hydrochloride:
   ¹H NMR (400 MHz, DMSO-*d₆*): δ 10.06 (s, 1H), 8.81 (d, *J =* 7.6 Hz, 1H), 8.59 - 8.52 (m, 2H), 8.07 (s, 1H), 7.94 (s, 1H), 7.34 - 7.06 (m, 5H), 5.12 (q, *J =* 7.2 Hz, 1H), 4.38 - 4.33 (m, 1H), 4.30 - 4.22 (m, 1H), 2.95 - 2.77 (m, 3H), 2.75 - 2.61 (m, 2H), 2.45 - 2.30 (m, 3H), 2.08 - 2.01 (m, 1H), 1.92 - 1.83 (m, 2H), 1.23 (t, *J =* 6.8 Hz, 3H). LC-MS: [M+H]⁺ = 427.05.
L114-IS-02 hydrochloride:
   ¹H NMR (400 MHz, DMSO-*d₆*): δ 10.02 (s, 1H), 8.81 (d, *J =* 4.8 Hz, 1H), 8.64 - 8.38 (m, 2H), 8.09 (s, 1H), 7.85 (s, 1H), 7.27 - 7.25 (m, 2H), 7.23 - 7.08 (m, 3H), 5.11 (q, *J =* 4.4 Hz, 1H), 4.36 - 4.32 (m, 1H), 4.25 - 4.20 (m, 1H), 2.90 - 2.78 (m, 3H), 2.71 - 2.61 (m, 2H), 2.45 - 2.30 (m, 3H), 2.06 - 2.01 (m, 1H), 1.90 - 1.82 (m, 2H), 1.21 (d, *J =* 4.8 Hz, 3H). LC-MS: [M+H]⁺ = 427.05.

### Example 131: Synthesis of (2R,4S)-N-((2S)-1-((5-(aminomethyl)-2,3-dihydro-1H-inden-1-yl)amino)-1-oxopropan-2-yl)-4-benzylpyrrolidine-2-carboxamide (Compound L115)

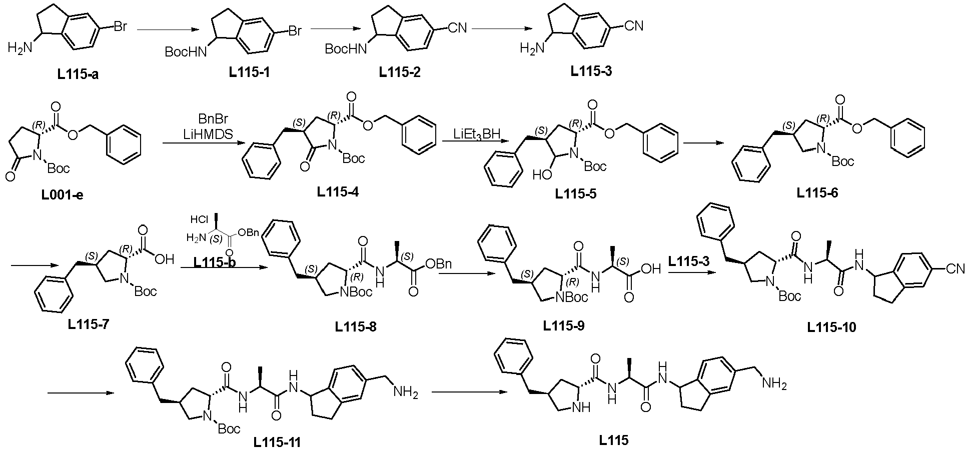

### 131.1: Synthesis of compound L115-1

L115-a (800 mg, 3.77 mmol), di-tert-butyl dicarbonate (822 mg, 3.77 mmol), and triethylamine (910 mg, 9.01 mmol) were added to dichloromethane (8 mL), and then the reaction liquid was reacted at room temperature for 1 h. The reaction liquid was added with water (20 mL) for dilution and extracted with dichloromethane (20 mL × 3). The organic phases were dried over anhydrous sodium sulfate and filtered, and the filtrate was concentrated under reduced pressure. The residue was purified by silica gel column chromatography (petroleum ether/ethyl acetate = 5/1-3/1) to give compound L115-1 (1.0 g, yield: 85%). LC-MS: [M-tBu+H]⁺ = 257.95.

### 131.2: Synthesis of compound L115-2

L115-1 (1.0 g, 3.21 mmol), zinc cyanide (109 mg, 0.48 mmol), and tetrakis(triphenylphosphine) palladium(0) (153 mg, 0.13 mmol) were added to N,N-dimethylformamide (10 mL), and the reaction liquid was reacted at 110 °C for 4 h under nitrogen atmosphere. The reaction liquid was filtered through celite, and the filtrate was added with water (30 mL) for dilution and extracted with ethyl acetate (30 mL × 3). The organic phase was dried over anhydrous sodium sulfate and filtered, and the filtrate was concentrated under reduced pressure. The residue was purified by silica gel column chromatography (petroleum ether/ethyl acetate = 5/1-3/1) to give compound L115-2 (650 mg, yield: 79%). LC-MS: [M+H]⁺ = 259.10.

### 131.3: Synthesis of compound L115-3 hydrochloride

L115-2 (650 mg, 2.52 mmol) was dissolved in dichloromethane (2 mL), and then a solution of hydrogen chloride in 1,4-dioxane (1 mL, 4 M) was added at room temperature. The reaction liquid was reacted at room temperature for 1 h. The reaction liquid was filtered, and the filter cake was rinsed with dichloromethane and dried under reduced pressure to give compound L115-3 hydrochloride (420 mg, yield: 85%). LC-MS: [M+H]⁺ = 159.10.

### 131.4: Synthesis of compound L115-7

For the synthesis of compound L115-7, reference was made to the synthesis of compound L001-11, and L001-f was replaced with benzyl bromide. LC-MS: [M-tBu+H]⁺ = 250.10.

### 131.5: Synthesis of compound L115-8

L115-7 (300 mg, 0.98 mmol) was dissolved in N,N-dimethylformamide (5 mL), and then N,N-diisopropylethylamine (507 mg, 3.93 mmol), 1-hydroxybenzotriazole (146 mg, 1.08 mmol), and 1-(3-dimethylaminopropyl)-3-ethylcarbodiimide hydrochloride (207 mg, 1.08 mmol) were added. The reaction liquid was reacted at room temperature for 10 min, and then L115-b (211 mg, 0.48 mmol) was added. The reaction liquid was reacted for another 1 h. The reaction liquid was added with ethyl acetate (10 mL) for dilution and washed with a saturated aqueous ammonium chloride solution (10 mL × 3). The organic phases were combined, dried over anhydrous sodium sulfate, and filtered, and the filtrate was concentrated under reduced pressure. The residue was purified by silica gel column chromatography (dichloromethane/methanol = 10/1) to give compound L115-8 (257 mg, yield: 56.07%). LC-MS: [M+H]⁺ = 467.15.

### 131.6: Synthesis of compound L115-9

At room temperature, L115-8 (300 mg, 0.64 mmol) was dissolved in methanol (10 mL), palladium on carbon (30 mg, 10% wt) was added, and then the reaction liquid was reacted for 1 h under hydrogen atmosphere. The reaction liquid was filtered through celite. The filter cake was rinsed with methanol (10 mL), and the filtrate was concentrated under reduced pressure to give compound L115-9 (250 mg, crude product), which was used directly in the next step. LC-MS: [M+H]⁺ = 377.10.

### 131.7: Synthesis of compound L115-10

L115-9 (250 mg, 0.66 mmol), L115-3 hydrochloride (155 mg, 0.79 mmol), 1-(3-dimethylaminopropyl)-3-ethylcarbodiimide hydrochloride (253 mg, 1.32 mmol), 1-hydroxybenzotriazole (200 mg, 1.32 mmol), and triethylamine (266 mg, 2.64 mmol) were added to dichloromethane (2 mL), and the reaction liquid was reacted at room temperature for 0.5 h. The reaction liquid was added with water (20 mL) for dilution and extracted with dichloromethane (20 mL × 3). The combined organic phases were concentrated under reduced pressure, and the residue was purified by preparative high performance liquid chromatography to give compound L115-10 (250 mg, yield: 73%). LC-MS: [M+H]⁺ = 517.05.

### 131.8: Synthesis of compound L115-11

L115-10 (250 mg, 0.48 mmol) and cobalt chloride (124 mg, 0.96 mmol) were added to methanol (3 mL), and then sodium borohydride (182 mg, 4.88 mmol) was added at 0 °C. The mixture was reacted for 1 h while maintaining the temperature at 0 °C. The reaction was quenched with diluted hydrochloric acid (5 mL), and then solid sodium carbonate was added to adjust pH to 9-10. The reaction system was added with water (20 mL) for dilution and extracted with ethyl acetate (20 mL × 3). The combined organic phases were dried over anhydrous sodium sulfate and filtered, and the filtrate was concentrated under reduced pressure. The residue was purified by high performance liquid chromatography to give compound L115-11 (150 mg, yield: 60%). LC-MS: [M+H]⁺ = 521.10.

### 131.9: Synthesis of compound L115

L115-11 (150 mg, 0.29 mmol) was dissolved in dichloromethane (1 mL), and then a solution of hydrogen chloride in 1,4-dioxane (1 mL, 4 M) was added. The reaction liquid was reacted at room temperature for 1 h. The reaction liquid was filtered, and the filter cake was washed with dichloromethane and then dried under reduced pressure. The solid was dissolved in deionized water (2 mL) and lyophilized to give compound L115 (62.0 mg, yield: 51%).

¹H NMR (400 MHz, DMSO-*d₆*): δ 10.27 (s, 1H), 8.86-8.83 (m, 1H), 8.50 - 8.45 (m, 4H), 7.31 - 7.12 (m, 8H), 5.26 - 5.24 (m, 1H), 4.35 - 4.28 (m, 2H), 3.97 (s, 2H), 3.28 - 3.27 (m, 1H), 2.91 - 2.85 (m, 2H), 2.71 - 2.68 (m, 4H), 2.36 - 2.35 (m, 1H), 2.16 - 2.10 (m, 1H), 1.91 - 1.81 (m, 2H), 1.27 - 1.24 (m, 3H). LC-MS: [M+H]⁺ =421.10.

### Example 132: Synthesis of (2R,4S)-N-((2S)-1-((5-amino-4,6-difluoro-2,3-dihydro-1H-inden-1-yl)amino)-1-oxopropan-2-yl)-4-benzylpyrrolidine-2-carboxamide (Compound L116) Hydrochloride

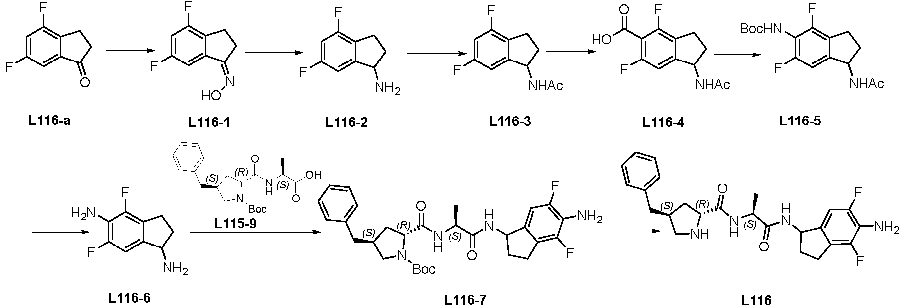

### 132.1: Synthesis of compound L116-1

L116-a (2.2 g, 13.1 mmol) and hydroxylamine hydrochloride (1.8 g, 26.2 mmol) were dissolved in ethanol (25 mL), and then potassium carbonate (3.6 g, 26.2 mmol) was added. The reaction liquid was reacted at 80 °C for 2 h. The reaction liquid was cooled to room temperature and added with water (200 mL) for dilution, and a solid was precipitated. The reaction liquid was filtered, and the filter cake was rinsed with water (20 mL) and dried under reduced pressure to give compound L116-1 (2.5 g). LC-MS: [M+H]⁺ =184.10.

### 132.2: Synthesis of compound L116-2

L116-1 (2.5 g, 13.6 mmol) was dissolved in ethanol (20 mL) and acetic acid (4 mL), and then palladium on carbon (1.0 g, 10% wt) was added. The reaction liquid was reacted at 50 °C for 16 h under hydrogen atmosphere. The reaction liquid was cooled to room temperature and filtered through celite. The filter cake was rinsed with ethanol (10 mL), and the filtrate was concentrated under reduced pressure. The residue was purified by silica gel column chromatography (ethyl acetate/petroleum ether = 3/1) to give compound L116-2 (1.5 g, yield: 65.2%). LC-MS: [M+H]⁺ = 170.10.

### 132.3: Synthesis of compound L116-3

L116-2 (1.5 g, 8.8 mmol) was dissolved in dichloromethane (30 mL), and then acetic anhydride (1.8 g, 17.6 mmol) and triethylamine (3.6 g, 26.2 mmol) were added. The reaction liquid was reacted at room temperature for 0.5 h. The reaction liquid was added with water (20 mL) for dilution and extracted with dichloromethane (50 mL × 3). The organic phases were combined, dried over anhydrous sodium sulfate, and filtered, and the filtrate was concentrated under reduced pressure. Water (20 mL) was added to the residue, and the mixture was stirred at room temperature for 1 h. The resulting suspension was filtered directly, and the filter cake was rinsed with water (5 mL) and dried under vacuum to give compound L116-3 (1.4 g, yield: 75.4%). LC-MS: [M+H]⁺ = 212.10.

### 132.4: Synthesis of compound L116-4

L116-3 (1.4 g, 6.0 mmol) and N,N,N',N'-tetramethylethylenediamine (2.3 g, 19.8 mmol) were dissolved in tetrahydrofuran (20 mL), and the mixture was cooled to -78 °C. n-Butyllithium (12.5 mL, 19.8 mmol, 1.6 M in tetrahydrofuran) was then added dropwise under nitrogen atmosphere. After the addition was completed, the reaction liquid was reacted at this temperature for 1 h. Dry ice (5 g) was added. After the addition was completed, the reaction liquid was slowly warmed to room temperature and reacted for 1 h. The reaction liquid was quenched by adding water (50 mL) and extracted with ethyl acetate (50 mL×2). The organic phase was discarded. The aqueous phase was adjusted to pH = 3-4 with diluted hydrochloric acid (1 M) and extracted with ethyl acetate (50 mL × 3). The organic phases were combined, dried over anhydrous sodium sulfate, and filtered, and the filtrate was concentrated under reduced pressure. The residue was purified by high performance liquid chromatography (0.1% trifluoroacetic acid condition) to give compound L116-4 (700 mg, yield: 45.8%). LC-MS: [M+H]⁺ = 256.05.

### 132.5: Synthesis of compound L116-5

L116-4 (630 mg, 2.47 mmol), diphenylphosphoryl azide (740 mg, 2.9 mmol), and N,N-diisopropylethylamine (374 mg, 2.9 mmol) were dissolved in tert-butanol (20 mL), and the reaction liquid was reacted at 80 °C for 16 h. The reaction liquid was cooled to room temperature, added with a saturated aqueous sodium bicarbonate solution (50 mL) for dilution, and extracted with ethyl acetate (50 mL × 3). The organic phases were combined, dried over anhydrous sodium sulfate, and filtered, and the filtrate was concentrated under reduced pressure. The residue was purified by silica gel column chromatography (methanol/dichloromethane = 1/10) to give compound L116-5 (520 mg, yield: 64.6%). LC-MS: [M+H₂O]⁺ = 344.05.

### 132.6: Synthesis of compound L116-6

L116-5 (300 mg, 0.92 mmol) was dissolved in ethylene glycol (5 mL), and then an aqueous sodium hydroxide solution (5 mL, 10%) was added. The reaction liquid was reacted at 150 °C for 5 h. The reaction liquid was cooled to room temperature, added with a saturated aqueous sodium chloride solution (20 mL) for dilution, and extracted with ethyl acetate (20 mL × 3). The combined organic phases were dried over anhydrous sodium sulfate and filtered, and the filtrate was concentrated under reduced pressure to give compound L116-6 (150 mg, crude product), which was used directly in the next step. LC-MS: [M-NH₂]⁺ = 168.15.

### 132.7: Synthesis of compound L116-7

L116-6 (150 mg, 0.8 mmol) was dissolved in dichloromethane (2 mL), and then triethylamine (244 mg, 2.4 mmol) was added. The reaction liquid was reacted at room temperature for 10 min and then added to a solution of L115-9 (300 mg, 0.8 mmol), 1-(3-dimethylaminopropyl)-3-ethylcarbodiimide hydrochloride (229 mg, 1.2 mmol), and 1-hydroxybenzotriazole (182 mg, 1.2 mmol) in dichloromethane (3 mL). The reaction liquid was reacted at room temperature for another 0.5 h. The reaction liquid was added with a saturated aqueous sodium chloride solution (10 mL) for dilution and extracted with dichloromethane (10 mL × 3). The organic phases were combined, dried over anhydrous sodium sulfate, and filtered, and the filtrate was concentrated under reduced pressure. The residue was purified by silica gel column chromatography (methanol/dichloromethane = 1/20) to give compound L116-7 (130 mg, yield: 25.3%). LC-MS: [M+H]⁺ = 543.10.

### 132.8: Synthesis of compound L116 hydrochloride

L116-7 (130 mg, 0.24 mmol) was dissolved in dichloromethane (2 mL), and then a solution of hydrogen chloride in 1,4-dioxane (2 mL, 4 M) was added. The reaction liquid was reacted at room temperature for 0.5 h, and a solid was precipitated. The reaction liquid was filtered, and the filter cake was rinsed with dichloromethane (2 mL) and dissolved by adding acetonitrile (2 mL) and deionized water (5 mL). The mixture was lyophilized to give compound L116 hydrochloride (106.1 mg, yield: 92.2%).

¹H NMR (400 MHz, DMSO-*d₆*): δ 10.14 (s, 1H), 8.85-8.82 (m, 1H), 8.65 (t, *J =* 8.4 Hz, 1H), 8.49 (s, 1H), 7.29 (t, *J =* 7.6 Hz, 2H), 7.21 (dd, *J =* 13.6, 7.2 Hz, 3H), 6.95-6.94 (m, 1H), 5.27 - 5.14 (m, 1H), 4.34 - 4.30 (m, 1H), 4.28 - 4.25 (m, 1H), 3.25 (s, 1H), 3.03 - 2.84 (m, 2H), 2.80 - 2.60 (m, 3H), 2.44 - 2.35 (m, 1H), 2.06 - 2.04 (m, 1H), 1.97 - 1.83 (m, 2H), 1.22 -1.19 (m, 4H). LC-MS: [M+H]⁺ = 443.05.

### Example 133: Synthesis of (2R,4S)-N-((2S)-1-((7-amino-4,6-difluoro-2,3-dihydro-1H-inden-1-yl) amino)-1-oxopropan-2-yl)-4-benzylpyrrolidine-2-carboxamide (Compound L117) Hydrochloride

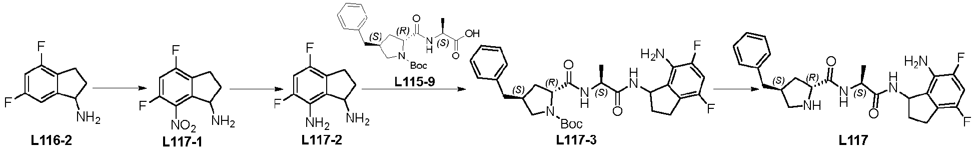

### 133.1: Synthesis of compound L117-1

L116-2 (200 mg, 1.20 mmol) was dissolved in concentrated sulfuric acid (2 mL), and then concentrated nitric acid (149 mg, 1.40 mmol) was added dropwise at 0 °C. After the addition was completed, the reaction liquid was reacted at 0 °C for 0.5 h. The reaction liquid was poured into water (20 mL), and the resulting mixture was adjusted to pH = 8 with a saturated aqueous sodium bicarbonate solution. The reaction liquid was extracted with ethyl acetate (10 mL × 3). The organic phases were combined, dried over anhydrous sodium sulfate, and filtered, and the filtrate was concentrated under reduced pressure to give compound L117-1 (200 mg, crude product). LC-MS: [M+H]⁺ = 215.05.

### 133.2: Synthesis of compound L117-2

L117-1 (200 mg, 0.93 mmol) was dissolved in methanol (2 mL), and then palladium on carbon (100 mg, 10% wt) was added. The reaction liquid was reacted for 0.5 h under hydrogen atmosphere. The reaction liquid was filtered through celite, and the filter cake was rinsed with methanol (10 mL). The filtrate was concentrated under reduced pressure to give compound L117-2 (150 mg, crude product), which was used directly in the next step. LC-MS: [M-NH₃+H]⁺ = 168.05.

### 133.3: Synthesis of compound L117 hydrochloride

For the synthesis of compound L117 hydrochloride, reference was made to the synthesis method of compound L116, and L116-6 was replaced with L117-2. The reaction liquid was filtered, and the filter cake was rinsed with dichloromethane (2 mL) and dissolved by adding acetonitrile (2 mL) and deionized water (5 mL). The mixture was lyophilized to give compound L117 hydrochloride (158 mg, yield: 69.0%).

¹H NMR (400 MHz, DMSO-*d₆*): δ 10.14 (s, 1H), 8.86 - 8.80 (m, 1H), 8.65 (t, *J =* 8.4 Hz, 1H), 8.49 (s, 1H), 7.29 (t, *J =* 7.6 Hz, 2H), 7.21 (dd, *J =* 13.6, 7.2 Hz, 3H), 6.95 - 6.94 (m, 1H), 5.27 - 5.14 (m, 1H), 4.32 (d, *J =* 16.0 Hz, 1H), 4.28 - 4.25 (m, 1H), 3.25 (s, 1H), 3.03 - 2.84 (m, 2H), 2.80 - 2.60 (m, 3H), 2.44 - 2.35 (m, 1H), 2.07 - 1.92 (m, 1H), 1.97 - 1.83 (m, 2H), 1.21 - 0.83 (m, 4H). LC-MS: [M+H]⁺ = 443.05.

### Example 134: Synthesis of (2R,4R)-N-((2S)-1-((5-amino-4,6-difluoro-2,3-dihydro-1H-inden-1-yl) amino)-1 -oxopropan-2-yl)-4-(4-fluorophenyl)piperidine-2-carboxamide (Compound L118) Hydrochloride

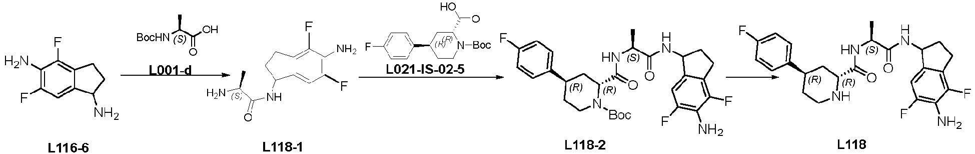

For the synthesis of compound L118 hydrochloride, reference was made to the synthesis method of compound L001, L001-6 was replaced with L116-6, and L001-11 was replaced with L021-IS-02-5. The reaction liquid was filtered, and the filter cake was rinsed with dichloromethane (1 mL) and dissolved by adding acetonitrile (2 mL) and deionized water (5 mL). The mixture was lyophilized to give compound L118 hydrochloride (6.5 mg, yield: 17.7%).

¹H NMR (400 MHz, DMSO-*d₆*): δ 9.65 (s, 1H), 8.73 - 8.59 (m, 1H), 8.36 - 8.34 (m, 1H), 8.30 - 8.25 (m, 1H), 7.15 - 7.11 (m, 4H), 6.72 - 6.62 (m, 1H), 5.31 - 5.15 (m, 1H), 4.35 - 4.32 (m, 1H), 4.30 - 4.21 (m, 1H), 3.37 - 3.34 (m, 1H), 3.16 - 3.14 (m, 1H), 2.91 - 2.90 (m, 1H), 2.79 - 2.77 (m, 1H), 2.68 - 2.66 (m, 1H), 2.31 - 2.29 (m, 2H), 2.01 - 2.00 (m, 1H), 1.84 - 1.80(m, 3H), 1.30 - 1.26 (m, 4H). LC-MS: [M+H]⁺ = 461.05.

### Example 135: Synthesis of (2R,4S)-N-((2S)-1-((5-amino-4,6-difluoro-2,3-dihydro-1H-inden-1-yl) amino)-1-oxopropan-2-yl)-4-(4-fluorobenzyl)pyrrolidine-2-carboxamide (Compound L119) Hydrochloride

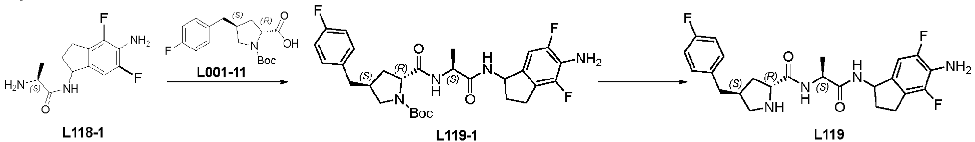

For the synthesis of compound L119 hydrochloride, reference was made to the synthesis method of compound L001, and L001-7 was replaced with L118-1. The reaction liquid was filtered, and the filter cake was rinsed with dichloromethane (2 mL) and dissolved by adding acetonitrile (2 mL) and deionized water (5 mL). The mixture was lyophilized to give compound L119 hydrochloride (71.7 mg, yield: 59.3%).

¹H NMR (400 MHz, DMSO-*d₆*): δ 10.39 (s, 1H), 8.81 (dd, *J =* 7.2, 5.2 Hz, 1H), 8.55 - 8.45 (m, 1H), 8.40 (dd, *J =* 12.0, 8.0 Hz, 1H), 7.28 (dd, *J* = 8.4, 5.6 Hz, 2H), 7.11 (t, *J =* 8.8 Hz, 2H), 6.68 - 6.66 (m, 1H), 5.15 (q, *J =* 7.6 Hz, 1H), 4.44 - 4.19 (m, 2H), 3.31 - 3.18 (m, 1H), 2.97 - 2.82 (m, 2H), 2.76 - 2.60 (m, 3H), 2.47 - 2.27 (m, 2H), 2.08 - 2.05 (m, 1H), 1.93 - 1.73 (m, 2H), 1.26 - 1.17 (m, 3H). LC-MS: [M+H]⁺ = 461.10.

### Biological Activity Assays

### 1. Assay for Inhibition of Activity of Lectin Pathway (MBL) in Human Serum by Compounds

The inhibition of the activity of the lectin pathway (MBL) in human serum by compounds was detected using a WIESLAB^{®} Complement System MBL Pathway kit (SVAR, COMPLMP320). Test compounds were each serially diluted in a buffer (Diluent MP), and 1% human serum was incubated with the diluted compound solution for 15 min at room temperature. 100 µL of the mixture was added to a microplate and incubated at 37 °C for 60 min. The liquid in the wells was discarded, and the microplate was washed three times with a washing solution. A Conjugate antibody solution was added at 100 µL/well, and the resulting mixture was incubated for 30 min at 25 °C. The liquid in the wells was discarded, and the microplate was washed three times with a washing solution; a substrate solution was added at 100 µL/well, and the resulting mixture was incubated for 30 min at 25 °C; the terminator was added at 50 µL/well, and the absorbance at 405 nm was detected within 30 min by using a microplate reader.

Data on the inhibition of the activity of the lectin pathway (MBL) in human serum by compounds are shown in Table 1 below.

### 2. Assay for Inhibition of Thrombin by Compounds

For thrombin assay, a fluorescent peptide substrate (Boc-VPR-AMC (R&D Systems)) was used and reacted in a buffer containing 20 mM Hepes (pH 7.4), 140 mM NaCl, and 0.1% Tween 20 at room temperature. Assay parameters were adjusted to make the assay linearly related to time, enzyme, and substrate concentration.

The assay method for thrombin is as follows. Test compounds were each serially diluted with a buffer, and then 10 µL of compound solution was transferred to an assay plate. A buffer was added to the assay plate at 10 µL/well, and then a thrombin (human α-thrombin (BioPharm Lab.)) solution was added at 15 µL/well. A Boc-VPR-AMC substrate solution was added at 15 µL/well, and the resulting mixture was incubated for 60 min at 25 °C. A terminating solution (0.1 M acetic acid) was added at 15 µL/well, and detection was performed within 30 min by using a SpectraMax i3x microplate reader.

**Table 1. Inhibition of lectin pathway (MBL) in human serum and thrombin by compounds**

| Example | Compound No. | Lectin pathway (MBL) IC₅₀ (nM) | Thrombin IC₅₀ (µM) |
|---|---|---|---|
| 1 | L001 | 2.669 | > 50 |
| 2 | L002 | 2.479 | > 50 |
| 3 | L003 | 5.170 | ND |
| 4 | L004 | 12.82 | ND |
| 9 | L009 | 7.082 | ND |
| 11 | L011 | 7.553 | ND |
| 12 | L012 | 4.056 | ND |
| 13 | L013 | 6.616 | ND |
| 14 | L014 | 2.709 | ND |
| 15 | L015 | 1.994 | ND |
| 16 | L016 | 4.645 | ND |
| 17 | L017 | 6.96 | ND |
| 20 | L020-IS-01 | 15.03 | ND |
| 21 | L021 | 3.75 | ND |
| 23 | L023-IS-02 | 3.36 | ND |
| 24 | L024 | 6.336 | ND |
| 25 | L025 | 5.523 | ND |
| 27 | L027 | 1.43 | 11.91 |
| 28 | L028 | 4.09 | 16.62 |
| 30 | L030 | 1.85 | 10.28 |
| 31 | L031 | 4.49 | 10.14 |
| 33 | L001-IS-01 | 0.52 | 27.45 |
| 36 | L012-IS-01 | 3.88 | ND |
| 39 | L021-IS-01 | 0.26 | ND |
| 42 | L033-IS-02 | 2.26 | ND |
| 48 | L038-IS-02 | 4.76 | ND |
| 50 | L039-IS-02 | 2.36 | ND |
| 58 | L044-IS-02 | 1.83 | ND |
| 60 | L045-IS-02 | 4.11 | ND |
| 61 | L046-IS-02 | 3.71 | ND |
| 62 | L047-IS-02 | 3.06 | ND |
| 63 | L048-IS-02 | 6.08 | ND |
| 65 | L050 | 2.64 | ND |
| 70 | L055-IS-02 | 7.00 | ND |
| 80 | L064-IS-02 | 7.57 | ND |
| 82 | L066-IS-02 | 9.52 | ND |
| 85 | L069-IS-02 | 17.45 | ND |
| 86 | L070-IS-02 | 17.65 | ND |
| 87 | L071-IS-02 | 7.85 | ND |
| 88 | L072-IS-02 | 9.26 | ND |
| 89 | L073-IS-02 | 4.25 | ND |
| 90 | L074-IS-02 | 2.79 | ND |
| 91 | L075-IS-02 | 4.89 | ND |
| 92 | L076-IS-02 | 11.6 | ND |
| 94 | L078-IS-02 | 16.34 | ND |
| 95 | L079-IS-02 | 2.9 | ND |
| 96 | L080-IS-02 | 2.87 | ND |
| 97 | L081-IS-02 | 7.41 | ND |
| 98 | L082 | 8.85 | ND |
| 99 | L083 | 10.64 | ND |
| 100 | L084 | 13.29 | ND |
| 120 | L104-IS-02 | 0.97 | ND |

| | | | |
|---|---|---|---|
| ND: not detected. | | | |

Experimental conclusion: the compounds of the present disclosure have good inhibitory effect on the lectin pathway (MBL) in human serum and have good selectivity relative to thrombin.

### 3. In Vivo Pharmacokinetic Study of Compounds of the Present Disclosure

After adaptive feeding, the SPF SD rats were each subjected to a single administration of 1 mg/kg or 3 mg/kg of the compound of the present disclosure intragastrically or by tail vein bolus injection. Plasma was collected at specific time points after administration, and the concentration of the compound in plasma was detected by LC-MS/MS (AB Sciex Qtrap 4500). Analysis was performed using a non-compartmental model in WinNonlin (version 5.2.1 Pharsight, Mountain View, CA) to obtain PK parameters, which represented the pharmacokinetic properties of the compounds of the present disclosure in the animals.

**Table 2. PK experiment of compounds of the present disclosure in SD rats**

| Compound | Administration dose mg/kg | Route of administration | Cₘₐₓ (ng/mL) | T_{1/2} (hr) | AUCₗₐₛₜ (ng/mL*hr) |
|---|---|---|---|---|---|
| L001 | 1 | PO, fasting, clear | 1483±41.0 | 10.1 | 9822±108 |
| | | IV, not fasting, clear | 7297±154 | 31.1±1.27 | 54837±1386 |
| L003 | 1 | PO, fasting, clear | 1777±394 | 5.89 | 11167±1986 |
| | | IV, not fasting, clear | 25400±1940 | 5.84±0.55 | 95060±770 |
| L012 | 1 | PO, fast, clear | 1777±98.2 | 12.7 | 11618±919 |
| | | IV, not fasting, clear | 26933±1167 | 12.0±3.72 | 97054±1914 |
| L014 | 1 | PO, fasting, clear | 1967±568 | 12.5 | 13180±3323 |
| | | IV, not fasting, clear | 24633±167 | 7.81±1.16 | 89916±4999 |
| L023-IS-02 | 3 | PO, fasting, clear | 4340±162 | 4.20±0.54 | 17996±2579 |
| | 1 | IV, not fasting, clear | 17400±1026 | 3.93±0.18 | 21567±2996 |
| L001-IS-01 | 3 | PO, fasting, clear | 6070±914 | 43.9±8.44 | 188265±20044 |
| | 1 | IV, not fasting, clear | 23267±1538 | 42.4±3.40 | 299766±11465 |
| L050 | 1 | PO, fasting, clear | 1373±162 | 2.21±0.27 | 3638±335 |
| | 1 | IV, not fasting, clear | 27167±1299 | 2.13±0.25 | 16723±860 |
| L064-IS-02 | 1 | PO, fasting, clear | 875±155 | 5.57±1.46 | 2630±245 |
| | 1 | IV, not fasting, clear | 16033±2074 | 4.18±1.02 | 15573±5146 |
| L066-IS-02 | 1 | PO, fasting, clear | 1136±164 | 4.93±0.51 | 3237±233 |
| | 1 | IV, not fasting, clear | 15867±1073 | 4.51±1.29 | 17126±4640 |
| L069-IS-02 | 1 | PO, fasting, clear | 1190±162 | 3.37 | 6931±968 |
| | 1 | IV, not fasting, clear | 15667±1132 | 3.39±0.17 | 19983±3838 |
| L076-IS-02 | 1 | PO, fasting, clear | 1347±423 | 4.29±0.28 | 6973±1213 |
| | 1 | IV, not fasting, clear | 22467±1033 | 5.88±0.27 | 25902±556 |
| L082 | 1 | PO, fasting, clear | 1431±283 | 2.85±0.05 | 6111±1206 |
| | 1 | IV, not fasting, clear | 18633±1965 | 3.21±0.19 | 24715±1031 |
| L083 | 1 | PO, fasting, clear | 1090±295 | 2.97±0.07 | 3361±778 |
| | 1 | IV, not fasting, clear | 18000±850 | 2.71±0.23 | 16417±936 |
| L084 | 1 | PO, fasting, clear | 2140±664 | 3.09±0.05 | 6883±1428 |
| | 1 | IV, not fasting, clear | 18200±929 | 3.68±0.22 | 26008±1479 |

Experimental conclusion: the compounds of the present disclosure can obtain relatively high *in vivo* exposure at a relatively low dose and have relatively good pharmacokinetic properties.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A compound represented by formula (I'), a racemate, a stereoisomer, a tautomer, a nitrogen oxide or a pharmaceutically acceptable salt thereof: 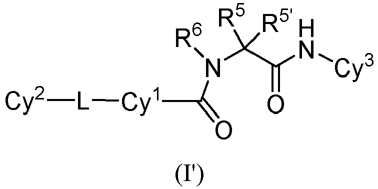 wherein:
Cy³ is selected from 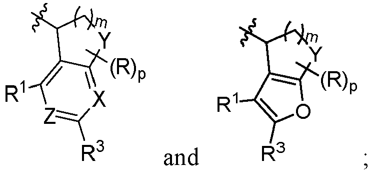
Z is selected from CR² and N;
R¹, R², and R³ are each independently selected from H, halogen, -NH₂, -OH, -NO₂, -CN, C₁₋₆ alkyl, C₁₋₆ aminoalkyl, and C₁₋₆ alkoxy;
X is selected from CR⁴ and N; R⁴ is selected from H, halogen, C₁₋₆ alkyl, and C₁₋₆ alkoxy;
Y is selected from -CH₂-, -C(C₁₋₃ alkyl)₂-, -NH-, -O-, and -S-; m is selected from 0, 1, 2, and 3;
each R is identical or different and is each independently selected from H, halogen, C₁₋₆ alkyl, and C₁₋₆ alkoxy;
p is selected from 0, 1, 2, 3, and 4;
R⁵ is selected from H, C₁₋₆ alkyl, C₂₋₆ alkynyl, C₁₋₆ haloalkyl, and C₃₋₆ cycloalkyl;
R^{5'} is selected from H and C₁₋₆ alkyl; or
R⁵ and R^{5'}, together with the carbon atom attached thereto, form C₃₋₆ cycloalkyl;
R⁶ is selected from H and C₁₋₆ alkyl; or
R⁵ and R⁶, together with the C and N atoms attached thereto, form 3- to 12-membered heterocycloalkyl, wherein the 3- to 12-membered heterocycloalkyl being unsubstituted or optionally substituted with 1, 2, or 3 R^{5a};
each R^{5a} is identical or different and is each independently selected from halogen, C₁₋₆ alkyl, -OH, - NO₂, -CN, oxo (=O), C₂₋₆ alkenyl, C₂₋₆ alkynyl, C₁₋₆ haloalkyl, C₁₋₆ alkoxy, and C₁₋₆ haloalkoxy; or
R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2, or 3 halogens;
Cy¹ is selected from the following groups unsubstituted or optionally substituted with 1, 2, 3, or 4 R⁷: 5- to 8-membered heterocyclyl and 5- to 6-membered heteroaryl;
each R⁷ is identical or different and is each independently selected from H, oxo (=O), halogen, and C₁₋₆ alkyl;
L is selected from -(CR^{a}R^{b})_{q}- and -(CR^{a}R^{b})_{q}-O-; q is selected from 0, 1, and 2, each R^{a} and each R^{b} are identical or different and are each independently selected from H, halogen, and C₁₋₆ alkyl, or R^{a} and R^{b}, together with the carbon atom attached thereto, form C₃₋₆ cycloalkyl;
Cy² is selected from the following groups unsubstituted or optionally substituted with 1, 2, 3, or 4 R⁸: C₆₋₁₀ aryl, 5- to 10-membered heteroaryl, C₃₋₁₀ cycloalkyl, and 5- to 12-membered heterocyclyl;
each R⁸ is identical or different and is each independently selected from -CN, halogen, C₁₋₆ alkyl, C₁₋₆ alkoxy, C₁₋₆ haloalkyl, C₁₋₆ haloalkoxy, C₃₋₁₀ cycloalkyl, C₆₋₁₀ aryl, and 5- to 10-membered heteroaryl.

2. The compound according to claim 1, wherein the compound represented by formula (I') has a structure represented by the following formula (I): 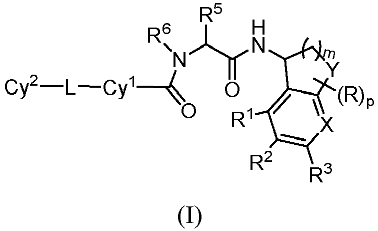 wherein:
R¹, R², and R³ are each independently selected from H, halogen, -NH₂, -OH, -NO₂, -CN, C₁₋₆ alkyl, C₁₋₆ aminoalkyl, and C₁₋₆ alkoxy;
X is selected from CR⁴ and N; R⁴ is selected from H, halogen, C₁₋₆ alkyl, and C₁₋₆ alkoxy;
Y is selected from -CH₂-, -C(C₁₋₃ alkyl)₂-, -NH-, -O-, and -S-; m is selected from 0, 1, 2, and 3;
each R is identical or different and is each independently selected from H, halogen, C₁₋₆ alkyl, and C₁₋₆ alkoxy;
p is selected from 0, 1, 2, 3, and 4;
R⁵ is selected from H, C₁₋₆ alkyl, C₂₋₆ alkynyl, C₁₋₆ haloalkyl, and C₃₋₆ cycloalkyl; R⁶ is selected from H and C₁₋₆ alkyl, or R⁵ and R⁶, together with the C and N atoms attached thereto, form 3- to 12-membered heterocycloalkyl, wherein the 3- to 12- membered heterocycloalkyl being unsubstituted or optionally substituted with 1, 2, or 3 R^{5a};
each R^{5a} is identical or different and is each independently selected from halogen, C₁₋₆ alkyl, -OH, - NO₂, -CN, oxo (=O), C₂₋₆ alkenyl, C₂₋₆ alkynyl, C₁₋₆ haloalkyl, C₁₋₆ alkoxy, and C₁₋₆ haloalkoxy; or
R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2, or 3 halogens;
Cy¹ is selected from the following groups unsubstituted or optionally substituted with 1, 2, 3, or 4 R⁷: 5- to 8-membered heterocyclyl and 5- to 6-membered heteroaryl;
each R⁷ is identical or different and is each independently selected from H, oxo (=O), halogen, and C₁₋₆ alkyl;
L is selected from -(CR^{a}R^{b})_{q}- and -(CR^{a}R^{b})_{q}-O-; q is selected from 0, 1, and 2, each R^{a} and each R^{b} are identical or different and are each independently selected from H, halogen, and C₁₋₆ alkyl, or R^{a} and R^{b}, together with the carbon atom attached thereto, form C₃₋₆ cycloalkyl;
Cy² is selected from the following groups unsubstituted or optionally substituted with 1, 2, 3, or 4 R⁸: C₆₋₁₀ aryl, 5- to 10-membered heteroaryl, C₃₋₁₀ cycloalkyl, and 5- to 12-membered heterocyclyl;
each R⁸ is identical or different and is each independently selected from -CN, halogen, C₁₋₆ alkyl, C₁₋₆ alkoxy, C₁₋₆ haloalkyl, C₁₋₆ haloalkoxy, C₃₋₁₀ cycloalkyl, C₆₋₁₀ aryl, and 5- to 10-membered heteroaryl.

3. The compound according to claim 2, wherein the compound represented by formula (I') has a structure represented by the following formula (II): 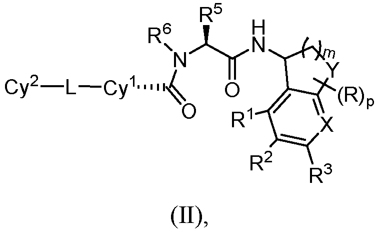 wherein, R⁵ is selected from C₁₋₆ alkyl, C₂₋₆ alkynyl, C₁₋₆ haloalkyl, and C₃₋₆ cycloalkyl; m, p, X, Y, R, R¹, R², R³, R⁶, L, Cy¹, and Cy² are each independently defined as in claim 1.

4. The compound according to claim 3, wherein the compound represented by formula (I') has a structure represented by any one of the following structural formulas (I-a) to (I-j'): 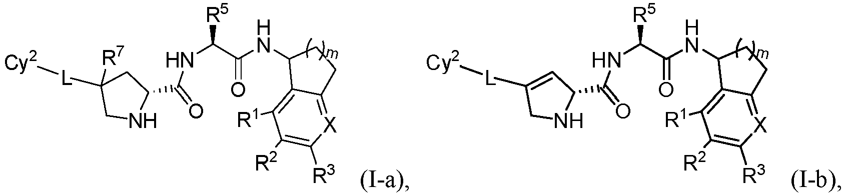 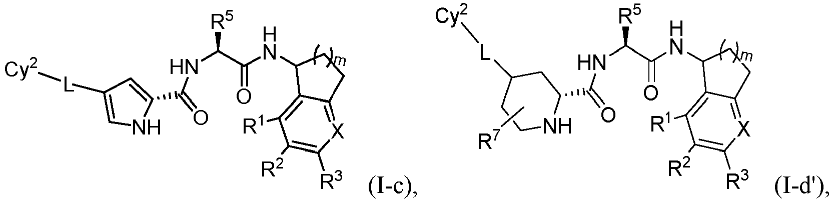 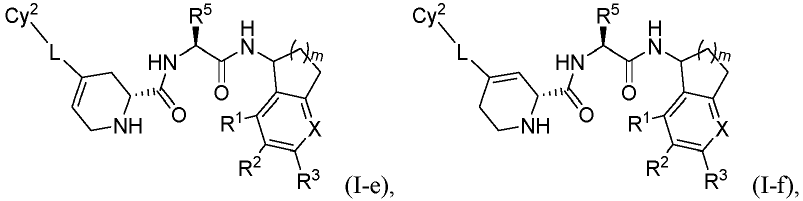 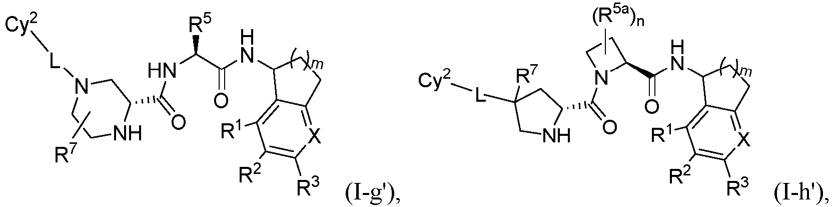 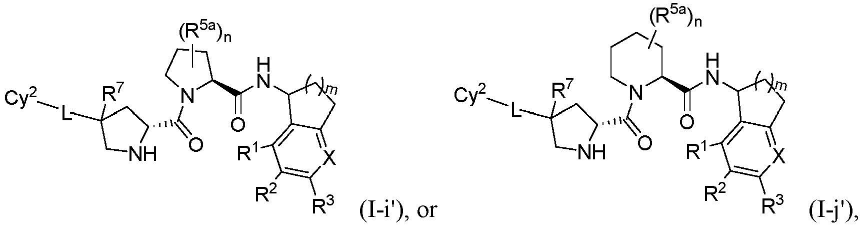
wherein, R⁵ is selected from C₁₋₃ alkyl, C₂₋₃ alkynyl, C₁₋₃ haloalkyl, and C₃₋₄ cycloalkyl; R^{5a} is each independently selected from halogen, C₁₋₃ alkyl, -OH, -NO₂, -CN, oxo (=O), C₁₋₃ haloalkyl, C₁₋₃ alkoxy, and C₁₋₃ haloalkoxy; or R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2, or 3 halogens; n is 0, 1, 2, or 3; m, X, R¹, R², R³, R⁷, L, and Cy² are each independently defined as in claim 2; preferably, the compound represented by formula (I') has a structure represented by any one of the following structural formulas (I-1) to (I-u'): 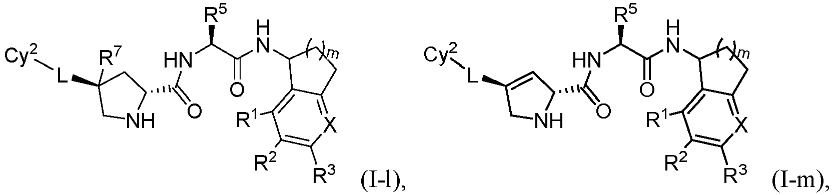 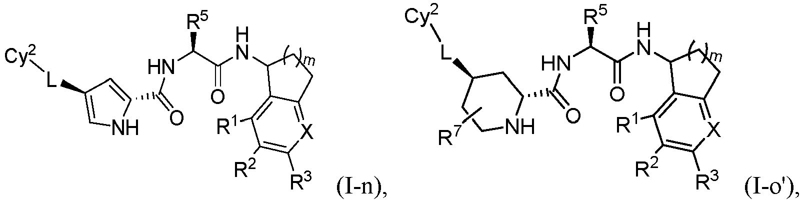 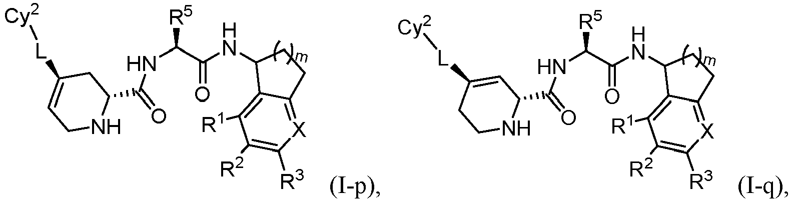 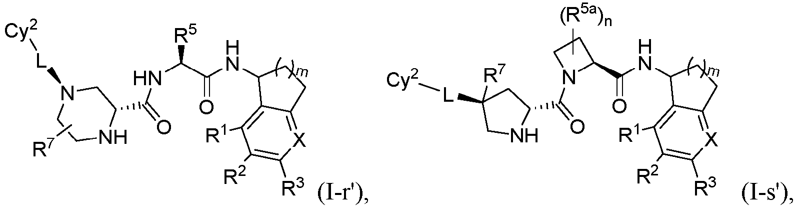 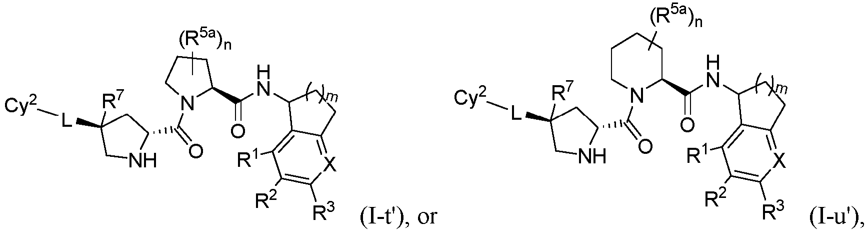
wherein, R⁵ is selected from C₁₋₃ alkyl, C₂₋₃ alkynyl, C₁₋₃ haloalkyl, and C₃₋₄ cycloalkyl; R^{5a} is each independently selected from halogen, C₁₋₃ alkyl, -OH, -NO₂, -CN, oxo (=O), C₁₋₃ haloalkyl, C₁₋₃ alkoxy, and C₁₋₃ haloalkoxy; or R^{5a} on the same carbon atom or on two adjacent carbon atoms, together with the carbon atom attached thereto, form C₃₋₆ cycloalkyl, wherein the C₃₋₆ cycloalkyl being unsubstituted or optionally substituted with 1, 2 or 3 halogens; n is 0, 1, 2, or 3; m, X, R¹, R², R³, R⁷, L, and Cy² are each independently defined as in claim 2;
more preferably, the compound represented by formula (I') has a structure represented by any one of the following structural formulas (I-v) to (I-z'): 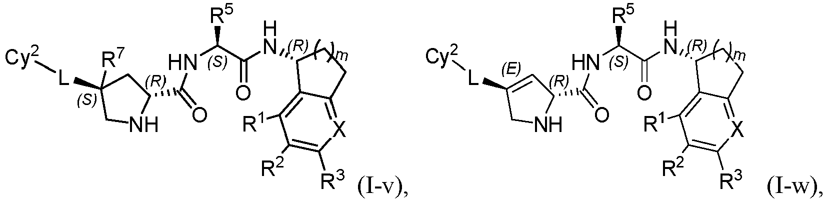 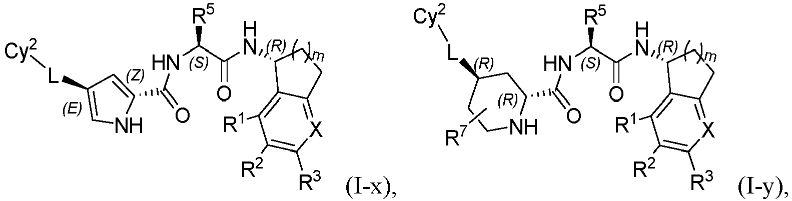 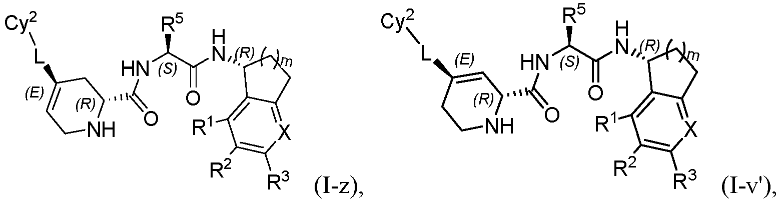 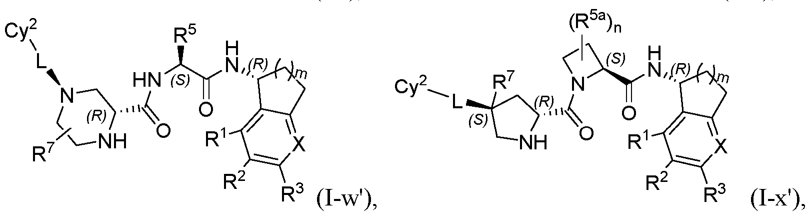 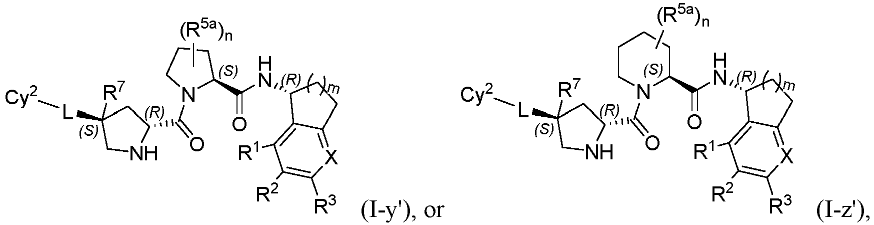
wherein, R⁵ is selected from methyl, ethyl, ethynyl, propynyl, -CH₂F, -CHF₂, -CF₃, and cyclopropyl; R^{5a} is each independently selected from halogen, methyl, ethyl, -CH₂F, -CHF₂, -CF₃, and methoxy; or R^{5a} on the same carbon atom or on two adjacent carbon atoms, together with the carbon atom attached thereto, form cyclopropyl or cyclobutyl, wherein the cyclopropyl and cyclobutyl being unsubstituted or optionally substituted with 1, 2, or 3 halogens; n is 0, 1, 2, or 3; m, X, R¹, R², R³, R⁷, L, and Cy² are each independently defined as in claim 2.

5. The compound according to claim 1, wherein Cy³ is selected from the following structures: 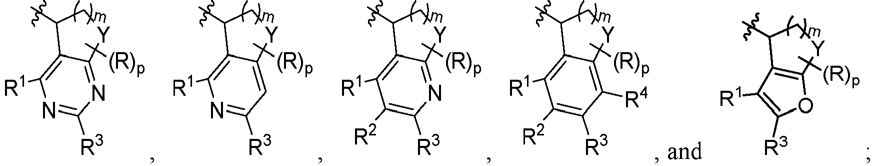 preferably, Cy³ is selected from the following structures: 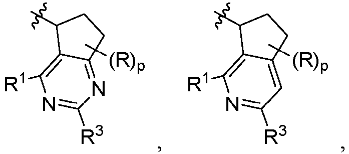 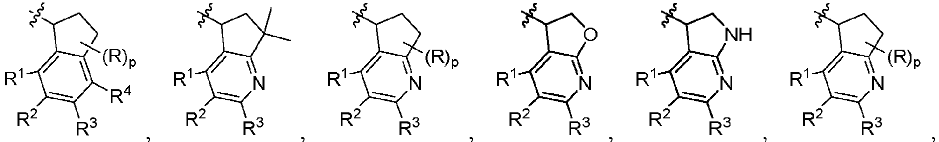 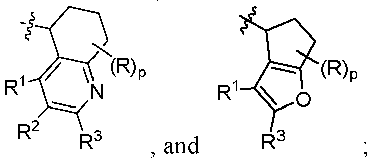 more preferably, Cy³ is selected from the following structures: 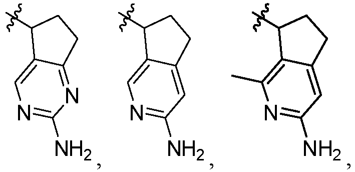 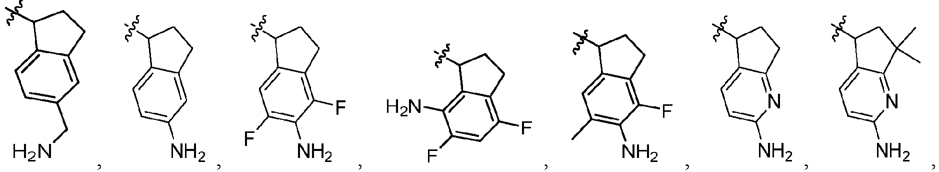 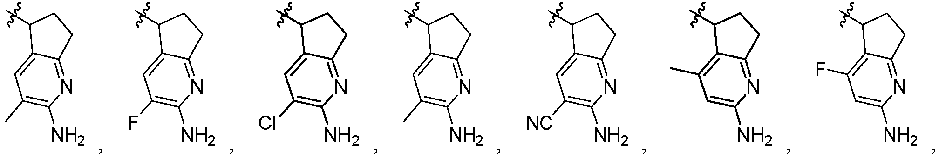 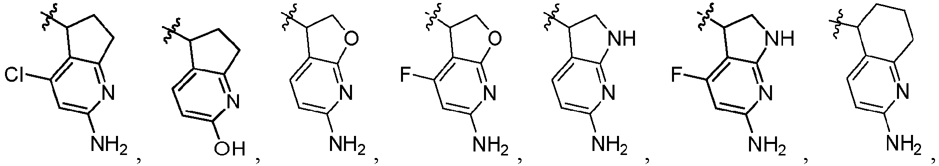 and

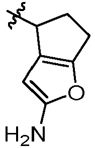

6. The compound according to claim 1, wherein, R⁵ is selected from H, C₁₋₃ alkyl, C₂₋₃ alkynyl, C₁₋₃ haloalkyl, and C₃₋₄ cycloalkyl;
R^{5'} is selected from H and C₁₋₃ alkyl; or
R⁵ and R^{5'}, together with the carbon atom attached thereto, form C₃₋₄ cycloalkyl;
preferably, R⁵ is selected from H, methyl, ethyl, ethynyl, propynyl, -CH₂F, -CHF₂, -CF₃, and cyclopropyl;
R^{5'} is selected from H, methyl, and ethyl; or
R⁵ and R^{5'}, together with the carbon atom attached thereto, form cyclopropyl.

7. The compound according to any one of claims 1 to 6, wherein, R¹, R², and R³ are each independently selected from H, F, Cl, Br, -NH₂, -OH, -CN, methyl, ethyl, -CH₂NH₂, and methoxy;
X is selected from CR⁴ and N; R⁴ is selected from H, halogen, methyl, and ethyl;
Y is selected from -CH₂-, -C(CH₃)₂-, -NH-, and -O-; m is selected from 0, 1, and 2; and/or
each R is identical or different and is each independently selected from H, halogen, C₁₋₃ alkyl, and C₁₋₃ alkoxy; p is selected from 0, 1, and 2.

8. The compound according to any one of claims 1 to 6, wherein R⁶ is selected from H, methyl, and ethyl; or R⁵ and R⁶, together with the C and N atoms attached thereto, form 4-, 5-, or 6-membered heterocycloalkyl, wherein the 4-, 5-, and 6-membered heterocycloalkyl being unsubstituted or optionally substituted with 1, 2, or 3 R^{5a}; R^{5a} is each independently selected from F, Cl, Br, methyl, and ethyl; or R^{5a} on the same carbon atom or on two adjacent carbon atoms, together with the carbon atom attached thereto, form cyclopropyl or cyclobutyl, wherein the cyclopropyl and cyclobutyl being unsubstituted or optionally substituted with 1, 2, or 3 halogens;
preferably, R⁵ and R⁶, together with the C and N atoms attached thereto, form the following structures: 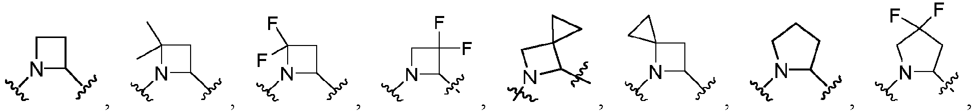 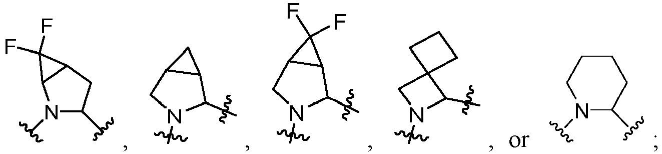 the structures formed are more preferably selected from:

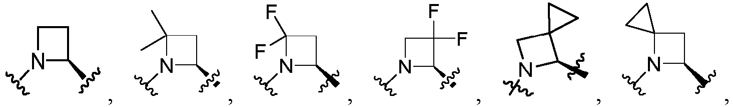

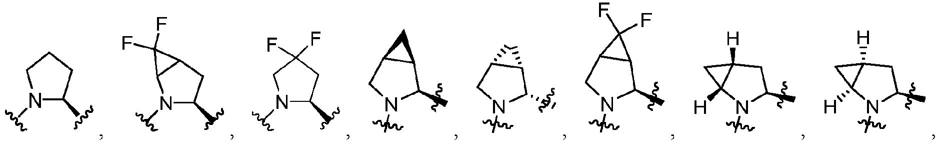

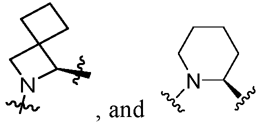

9. The compound according to any one of claims 1 to 6, wherein,
L is absent or selected from -C(R^{a})(R^{b})-, -O-, and -C(R^{a})(R^{b})-O-; R^{a} and R^{b} are each independently selected from H, halogen, methyl, and ethyl, or R^{a} and R^{b}, together with the carbon atom attached thereto, form cyclopropyl or cyclobutyl;
preferably, L is absent or selected from -CH₂-, -CH(CH₃)-, -CF₂-, -O-, -CH₂-O-, -CH(CH₃)-O-, and

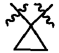

10. The compound according to any one of claims 1 to 6, wherein,
Cy¹ is selected from the following groups unsubstituted or substituted with 1 or 2 R⁷: tetrahydropyrrolyl, 2,5-dihydro-1H-pyrrolyl, pyrrolyl, piperidyl, 1,2,3,6-tetrahydropyridinyl, 1,2,5,6-tetrahydropyridinyl, piperazinyl, 4-azaspiro[2.5]octyl, 1,2-dihydropyridinyl, and 1,4-dihydropyrazinyl; each R⁷ is identical or different and is each independently selected from H, oxo (=O), F, Cl, and -CH₃;
preferably, Cy¹ is selected from the following structures: 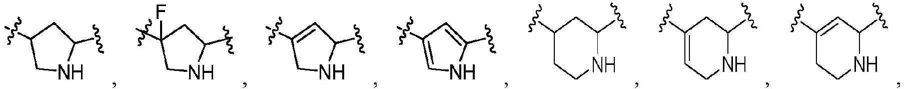 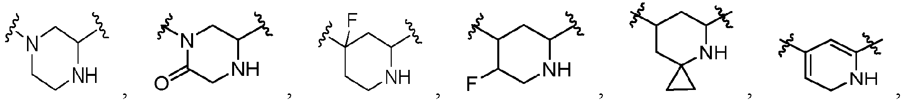 and 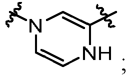
more preferably, Cy¹ is selected from the following structures: 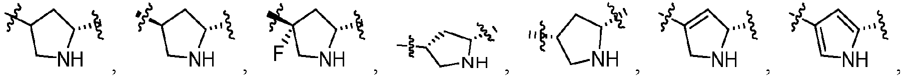 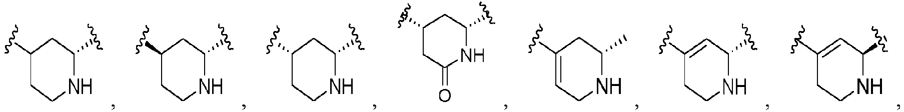 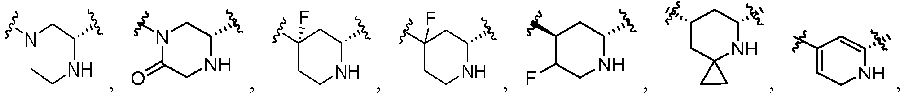
and

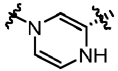

11. The compound according to any one of claims 1 to 6, wherein,
Cy² is selected from the following groups unsubstituted or optionally substituted with 1, 2, 3, or 4 R⁸: C₆₋₁₀ aryl, 5- to 9-membered heteroaryl, and 6- to 11-membered heterocyclyl; each R⁸ is identical or different and is each independently selected from -CN, F, Br, Cl, -CH₂F, -CHF₂, -CF₃, methyl, ethyl, n-propyl, isopropyl, n-butyl, tert-butyl, methoxy, ethoxy, phenyl, pyridinyl, and trifluoromethoxy;
preferably, Cy² is selected from the following groups unsubstituted or substituted with 1, 2, 3, or 4 R⁸: phenyl, naphthyl, pyrrolyl, pyridinyl, benzothiazolyl, piperidyl, 6-azaspiro[2.5]octyl, 7-azaspiro[3.5]nonyl, 8-azaspiro[4.5]decyl, and 3-azaspiro[5.5]undecyl; each R⁸ is identical or different and is each independently selected from -CN, F, Br, Cl, CH₂F, CHF₂, CF₃, methyl, ethyl, n-propyl, isopropyl, n-butyl, tert-butyl, methoxy, ethoxy, phenyl, pyridinyl, and trifluoromethoxy;
more preferably, Cy² is selected from the following structures: 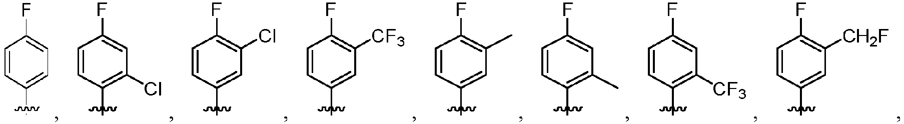 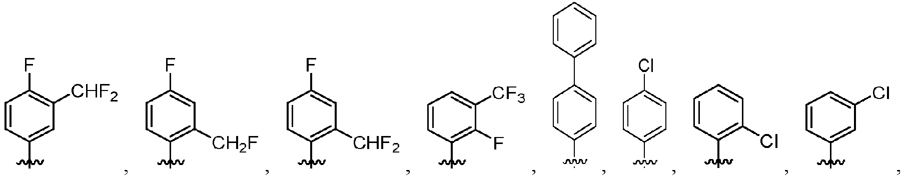 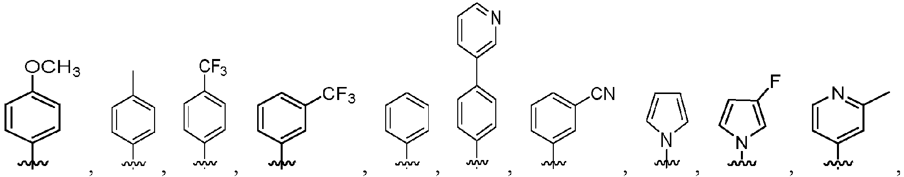 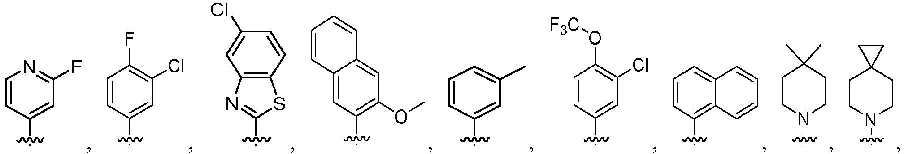 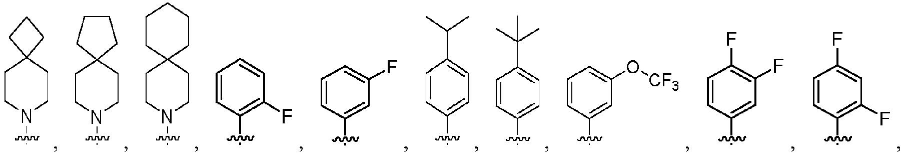
and

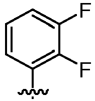

12. The compound according to claim 1, wherein,
Cy³ is selected from the following structures: 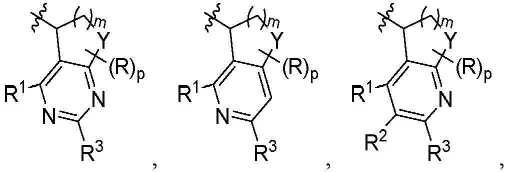 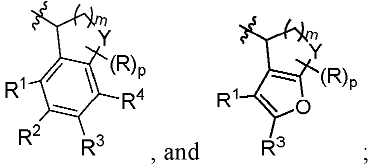
R¹, R², and R³ are each independently selected from H, F, Cl, Br, -NH₂, -OH, -CN, C₁₋₃ alkyl, and C₁₋₃ aminoalkyl;
R⁴ is selected from H, halogen, and C₁₋₃ alkyl;
Y is selected from -CH₂-, -C(CH₃)₂-, -NH-, -O-, and -S-; m is selected from 0, 1, and 2;
each R is identical or different and is each independently selected from H, halogen, and C₁₋₃ alkyl; p is selected from 0, 1, and 2;
R⁵ is selected from H, C₁₋₃ alkyl, C₂₋₃ alkynyl, C₁₋₃ haloalkyl, and C₃₋₄ cycloalkyl;
R^{5'} is selected from H and C₁₋₃ alkyl; or
R⁵ and R^{5'}, together with the carbon atom attached thereto, form C₃₋₄ cycloalkyl;
R⁶ is selected from H and C₁₋₃ alkyl; or
R⁵ and R⁶, together with the C and N atoms attached thereto, form 4-, 5-, or 6-membered heterocycloalkyl, wherein the 4-, 5-, and 6-membered heterocycloalkyl being unsubstituted or optionally substituted with 1, 2, or 3 R^{5a}; R^{5a} is independently selected from F, Cl, Br, methyl, and ethyl; or R^{5a} on the same carbon atom or on two adjacent carbon atoms, together with the carbon atom attached thereto, form cyclopropyl or cyclobutyl, wherein the cyclopropyl and cyclobutyl being unsubstituted or optionally substituted with 1, 2, or 3 halogens;
L is absent or selected from -C(R^{a})(R^{b})-, -O-, and -C(R^{a})(R^{b})-O-; R^{a} and R^{b} are each independently selected from H, halogen, and C₁₋₃ alkyl, or R^{a} and R^{b}, together with the carbon atom attached thereto, form cyclopropyl;
Cy¹ is selected from the following groups unsubstituted or substituted with 1 or 2 R⁷: tetrahydropyrrolyl, 2,5-dihydro-1H-pyrrolyl, pyrrolyl, piperidyl, 1,2,3,6-tetrahydropyridinyl, 1,2,5,6-tetrahydropyridinyl, piperazinyl, 4-azaspiro[2.5]octyl, 1,2-dihydropyridinyl, and 1,4-dihydropyrazinyl; each R⁷ is identical or different and is each independently selected from H, oxo (=O), F, and Cl;
Cy² is selected from the following groups unsubstituted or substituted with 1, 2, 3, or 4 R⁸: phenyl, naphthyl, pyrrolyl, pyridinyl, benzothiazolyl, piperidyl, 6-azaspiro[2.5]octyl, 7-azaspiro[3.5]nonyl, 8-azaspiro[4.5]decyl, and 3-azaspiro[5.5]undecyl;
each R⁸ is identical or different and is each independently selected from -CN, F, Cl, -CH₂F, -CHF₂, CF₃, methyl, ethyl, n-propyl, isopropyl, tert-butyl, methoxy, phenyl, pyridinyl, and trifluoromethoxy;
preferably, Cy³ is selected from the following structures: 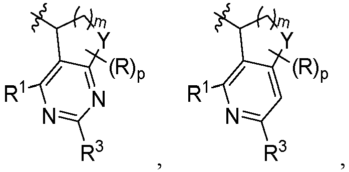 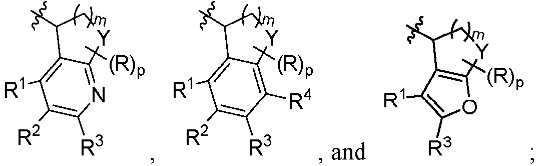
R¹ is selected from H, F, Cl,-NH₂, and methyl;
R² is selected from H, F, Cl, -CN, and methyl;
R³ is selected from H, F, -NH₂, -OH, -CH₂NH₂, and methoxy;
R⁴ is selected from H and F;
Y is selected from -CH₂-, -C(CH₃)₂-, -NH-, and -O-; m is 1 or 2;
R is selected from H, methyl, and ethyl;
R⁵ is selected from H, methyl, ethyl, ethynyl, propynyl, -CH₂F, -CHF₂, -CF₃, and cyclopropyl;
R^{5'} is selected from H, methyl, and ethyl; or
R⁵ and R^{5'}, together with the carbon atom attached thereto, form cyclopropyl;
R⁶ is selected from H; or
R⁵ and R⁶, together with the C and N atoms attached thereto, form the following structures: 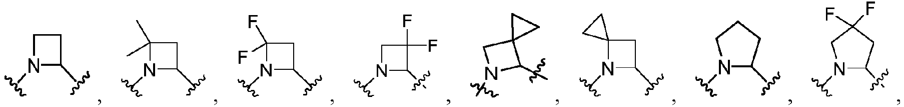 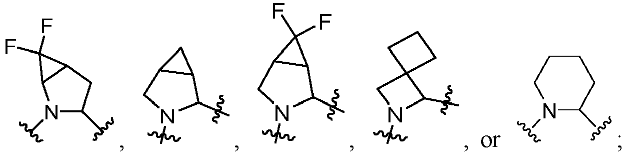 the structures formed are preferably selected from: 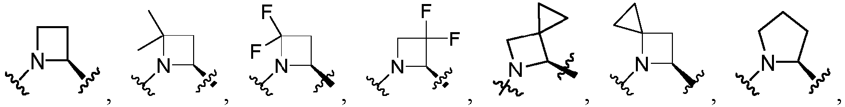 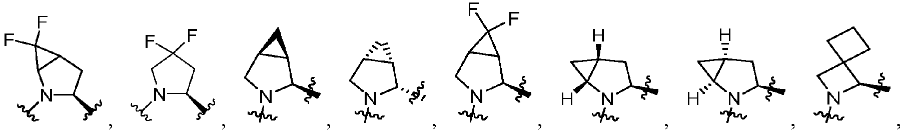 and 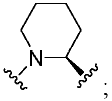
preferably, L is absent or selected from -CH₂-, -CH(CH₃)-, -CF₂-, -O-, -CH₂-O-, and 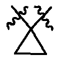 ;
preferably, Cy¹ is selected from the following structures: 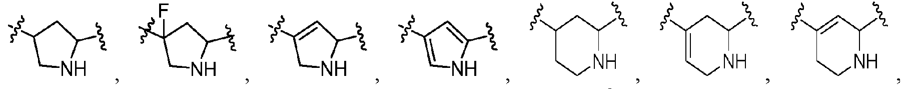 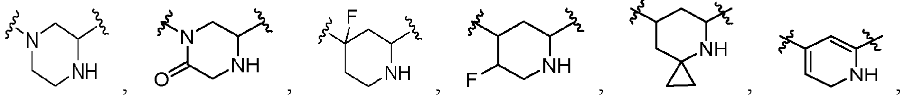 and 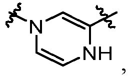
preferably selected from: 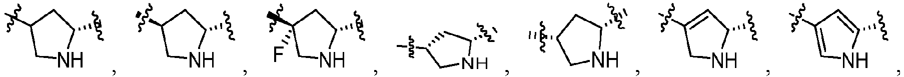 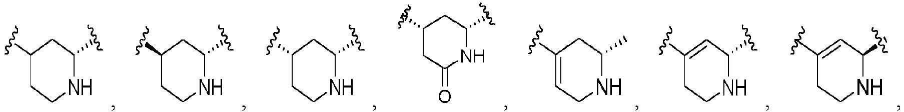 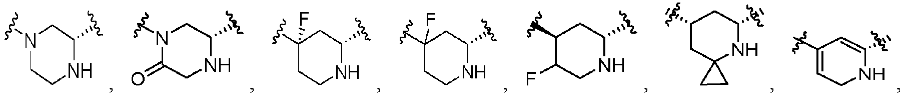 and 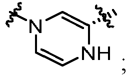
preferably, Cy² is selected from the following structures:

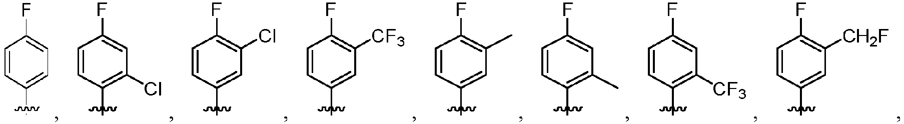

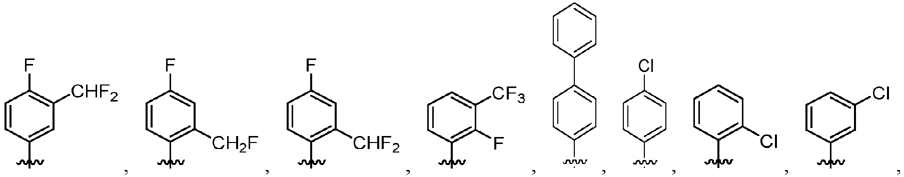

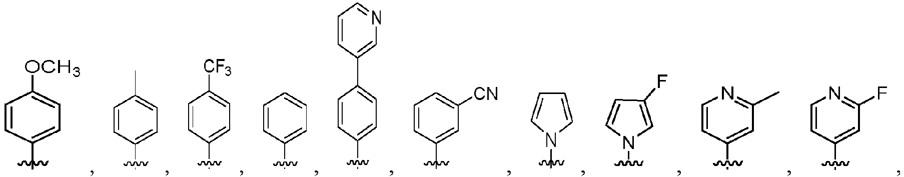

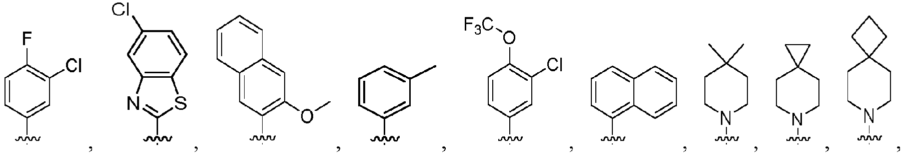

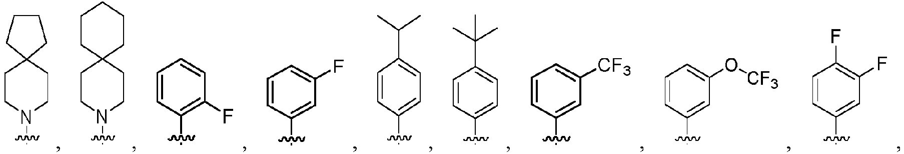

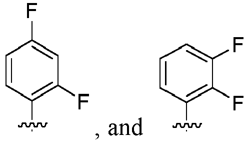

13. The compound according to any one of claims 1 to 12, wherein the compound is selected from the following structures: 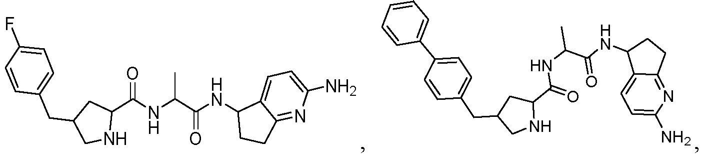 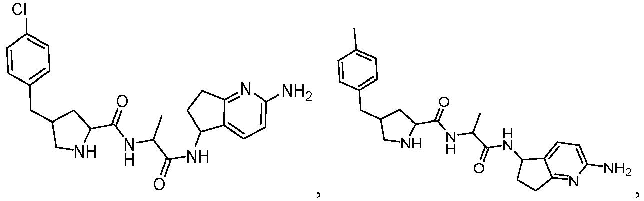 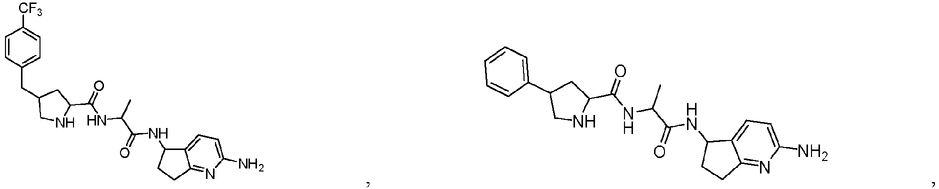 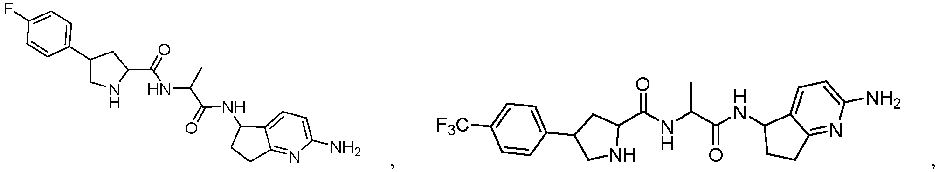 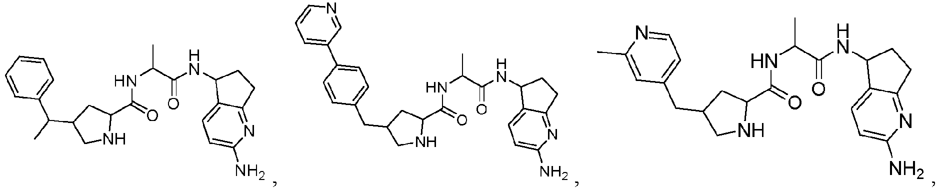 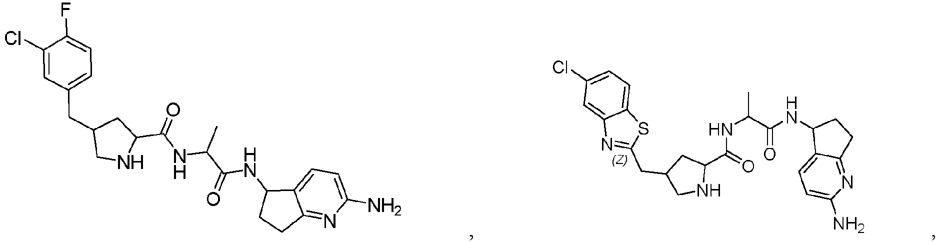 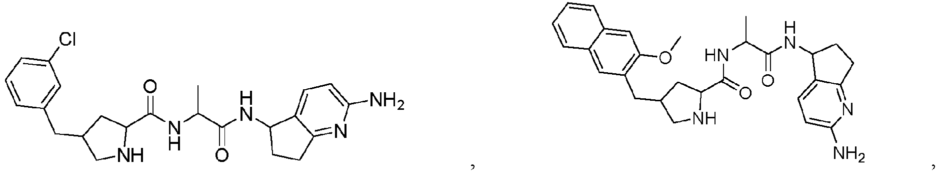 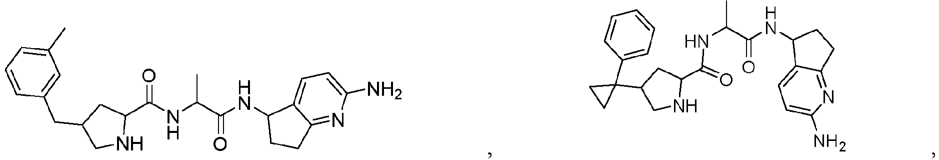 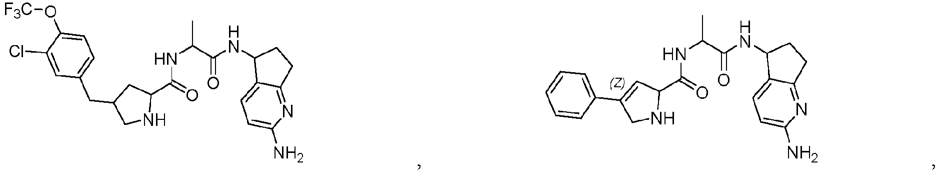 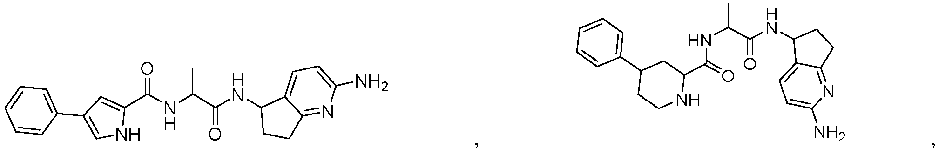 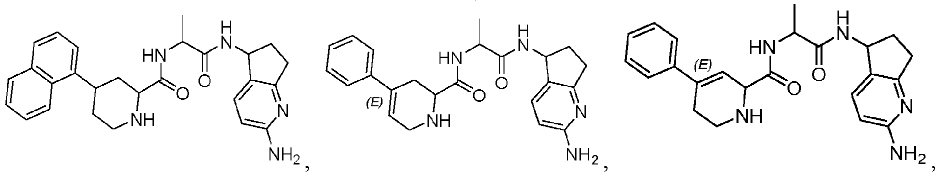 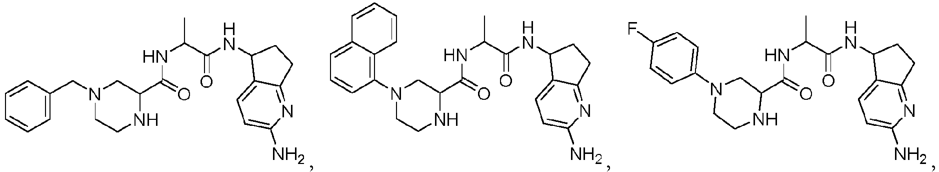 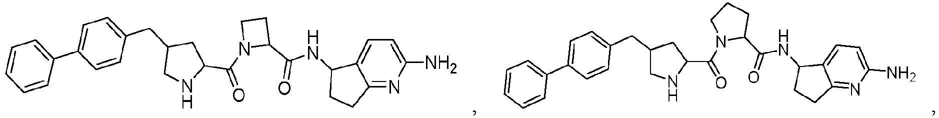 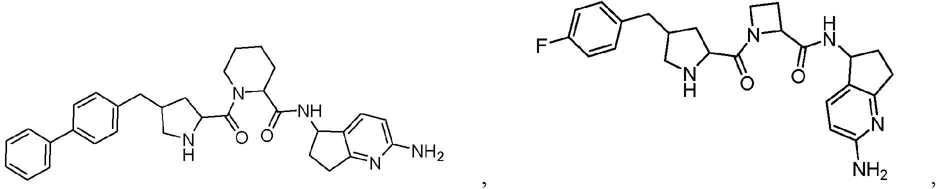 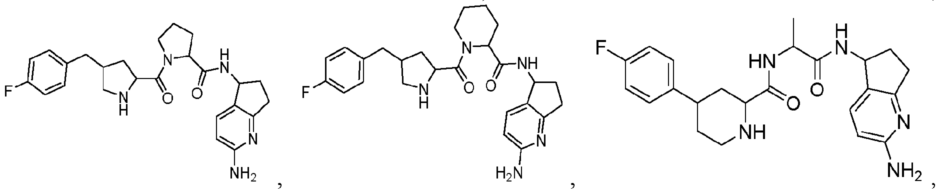 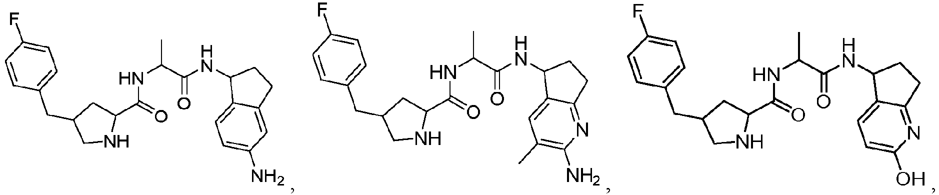 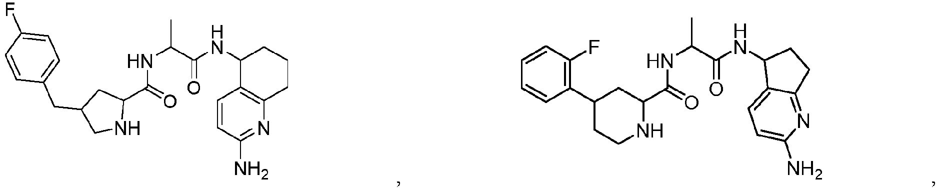 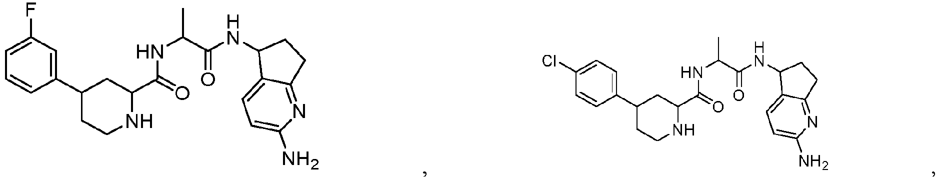 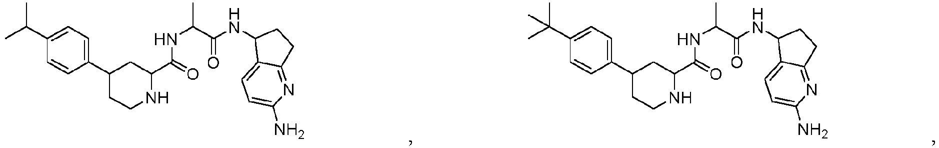 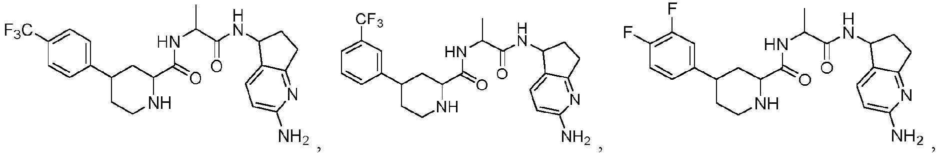 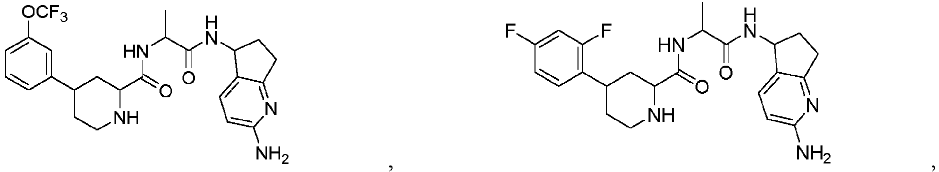 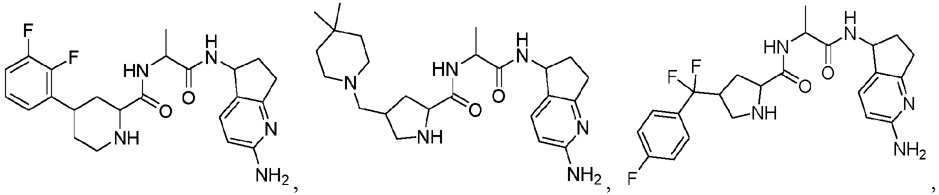 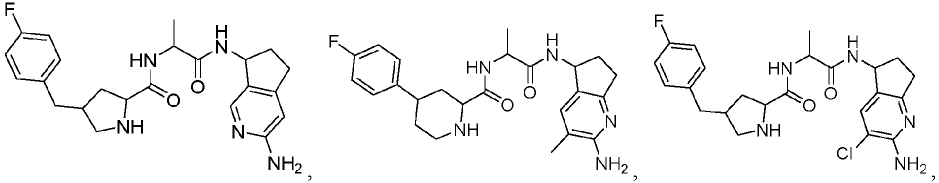 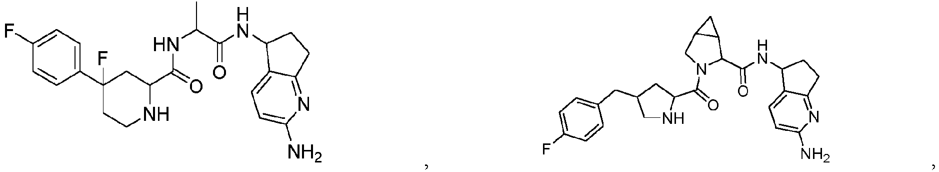 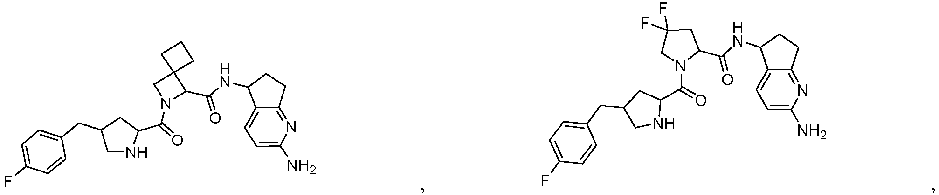 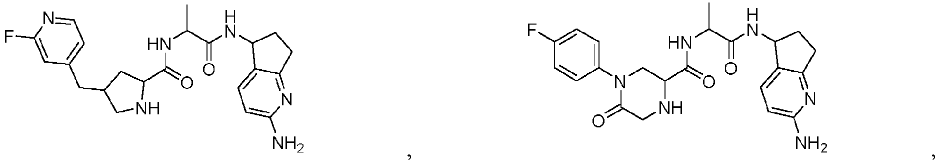 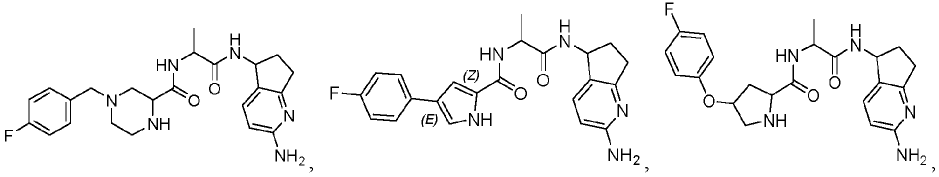 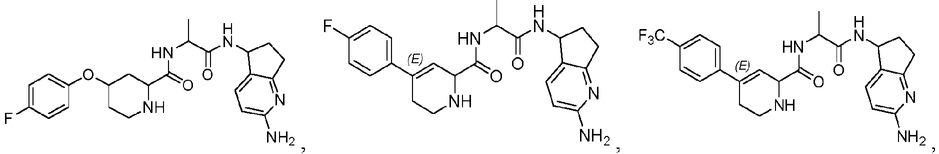 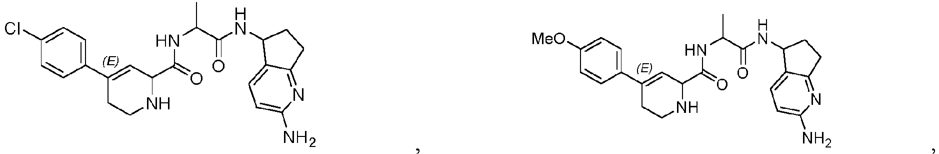 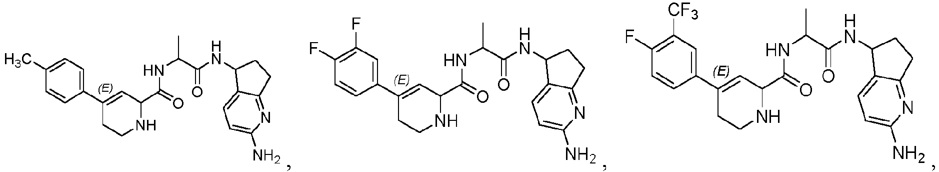 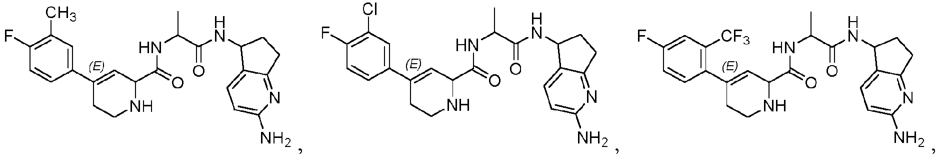 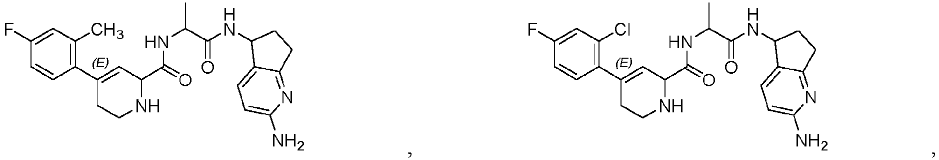 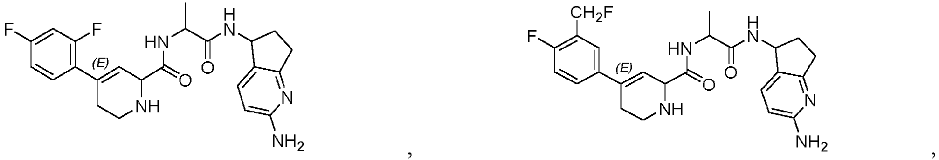 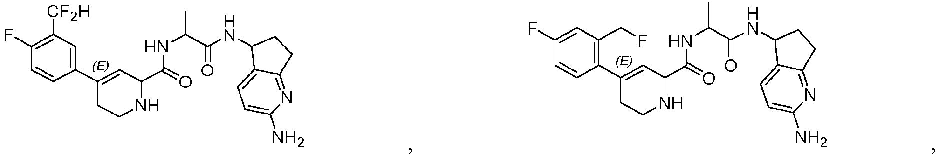 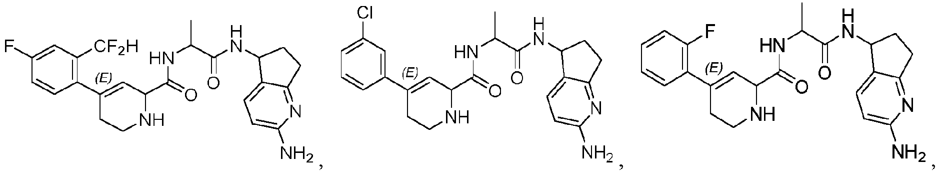 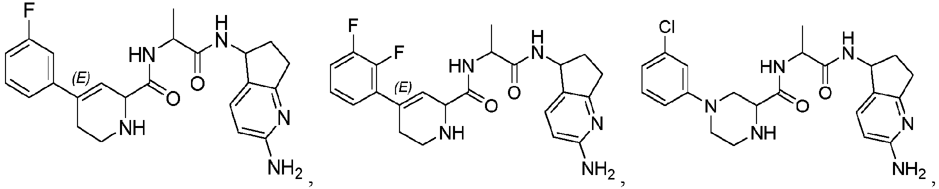 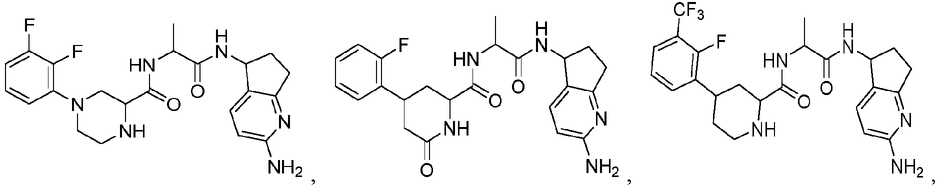 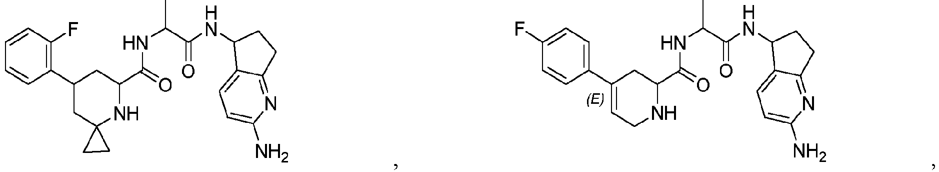 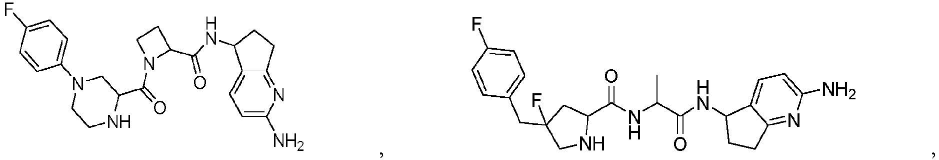 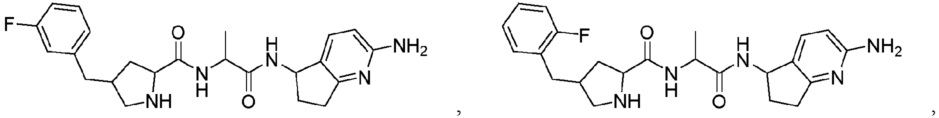 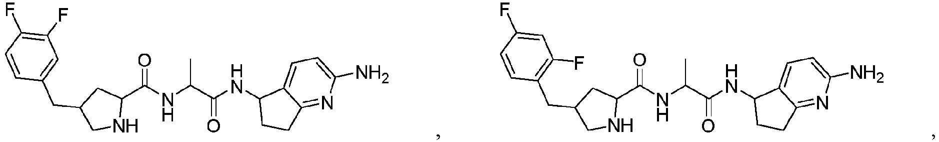 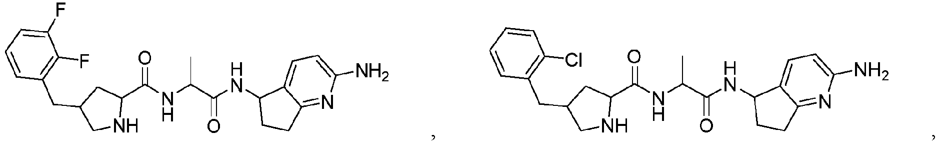 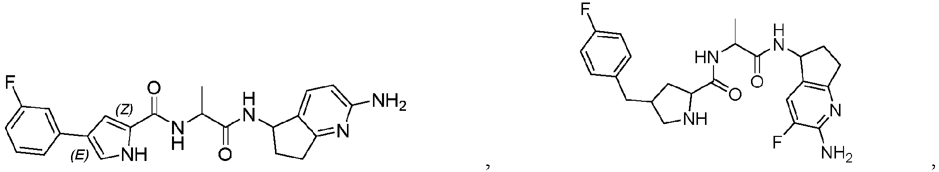 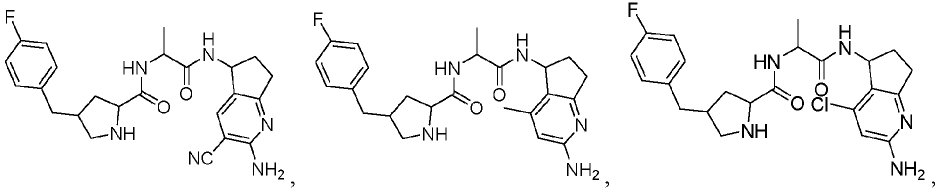 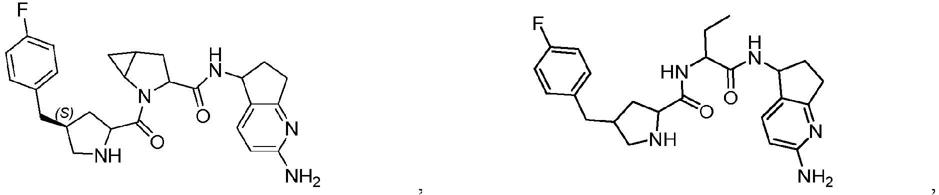 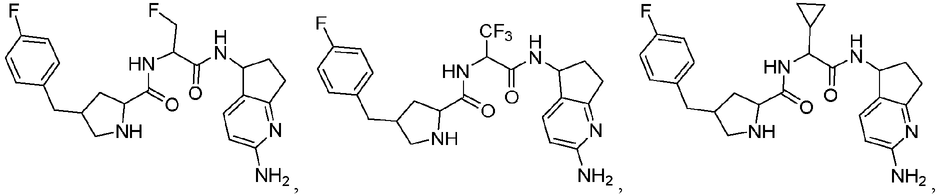 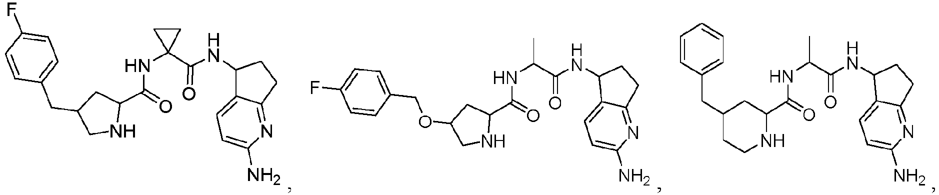 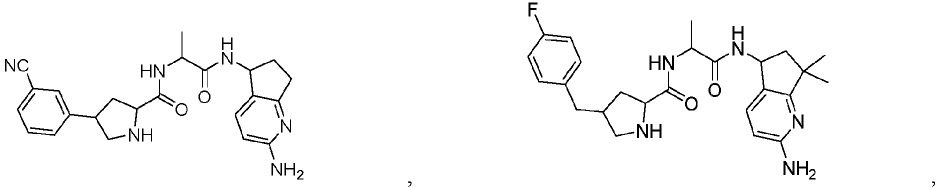 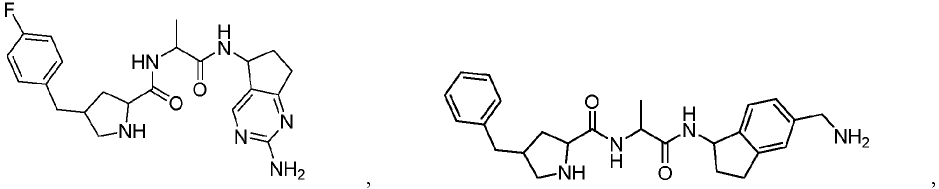 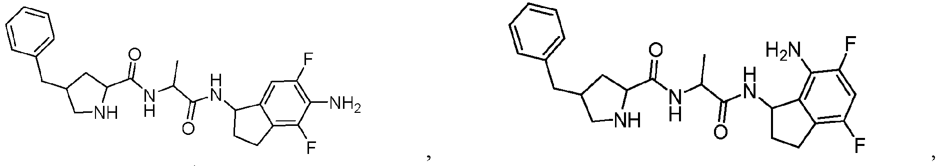 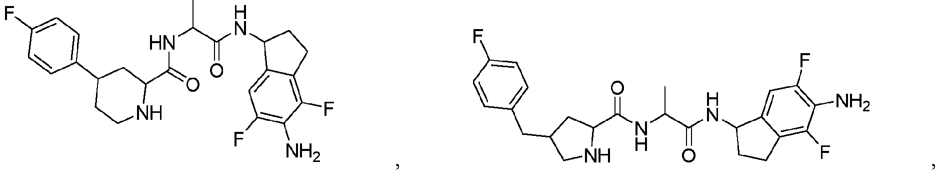 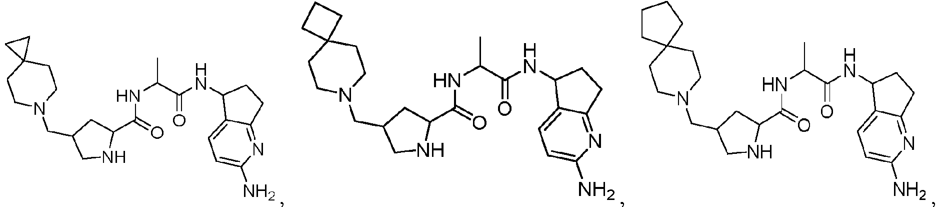 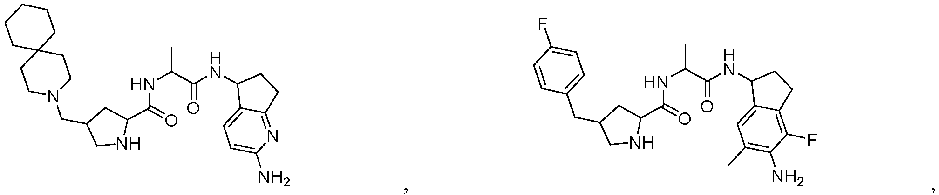 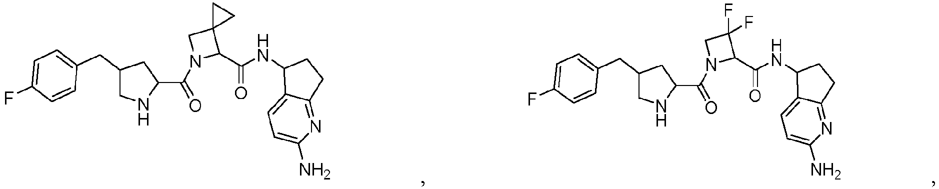 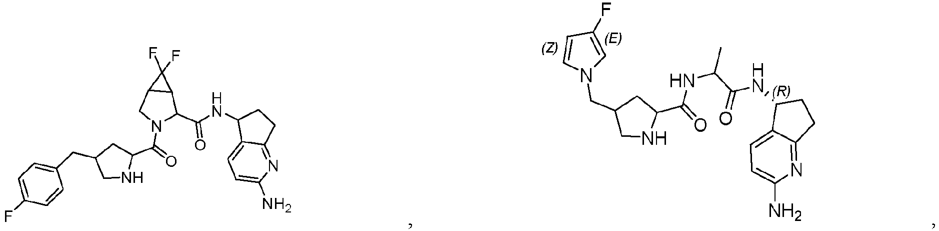 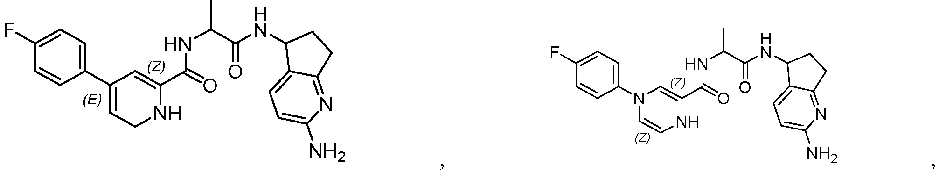 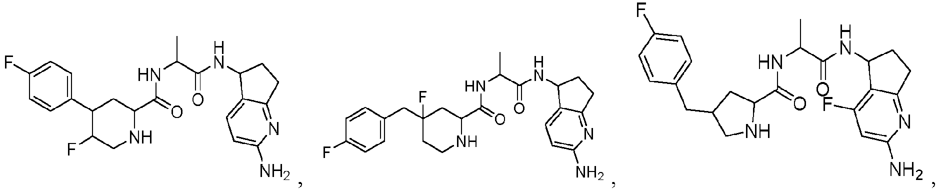 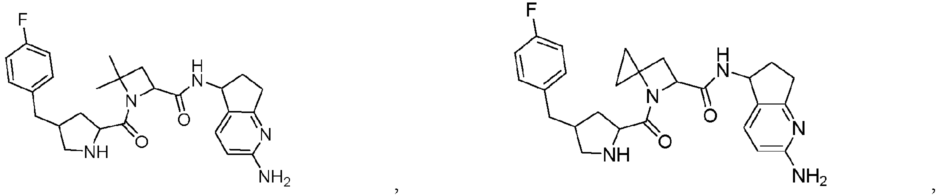 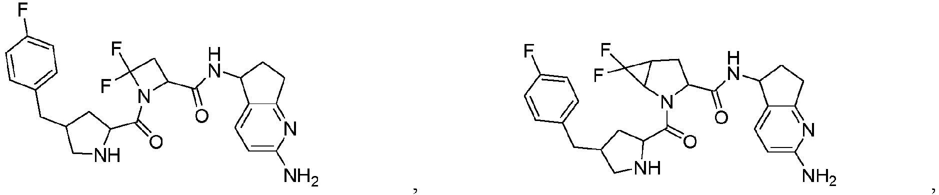 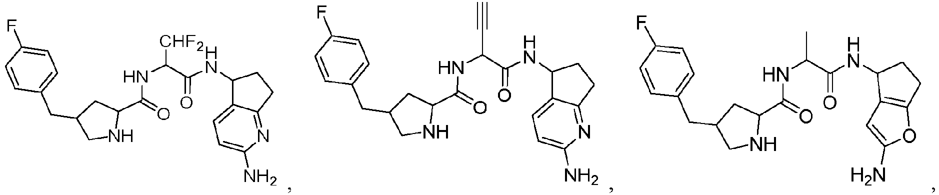 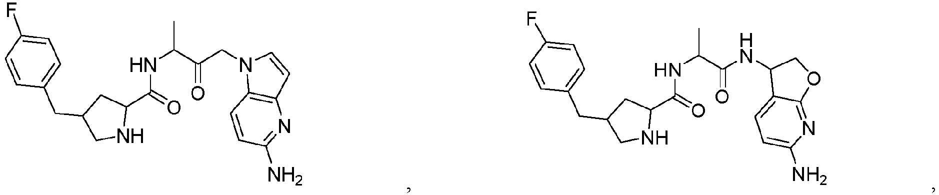 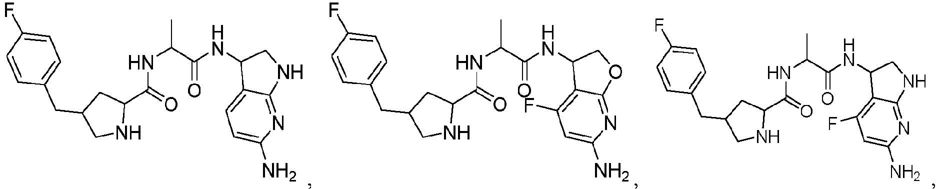 and 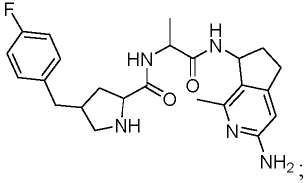 preferably, the compound is selected from the following structures:

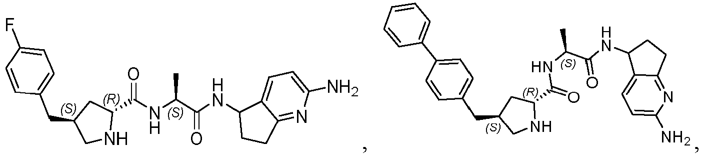

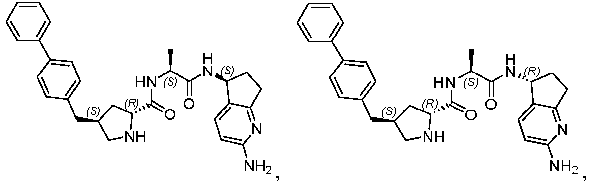

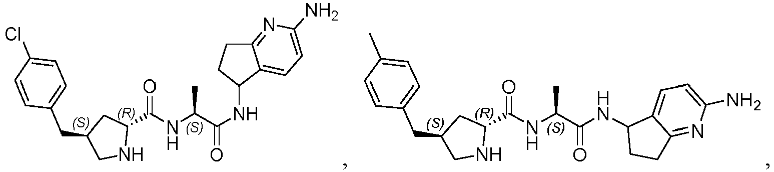

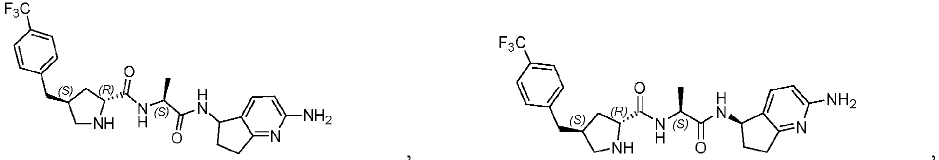

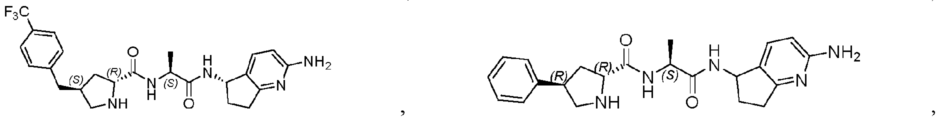

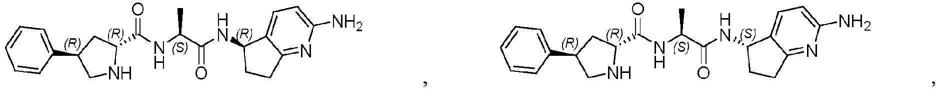

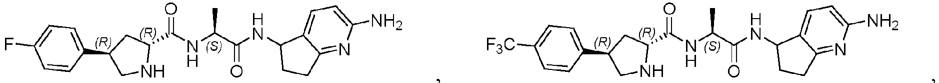

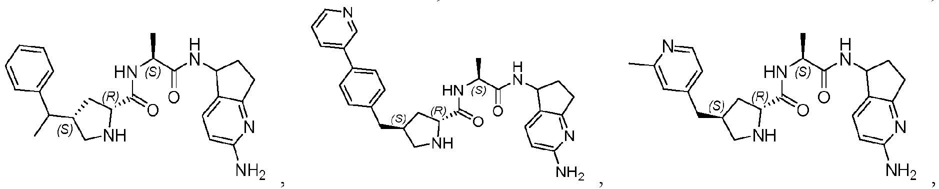

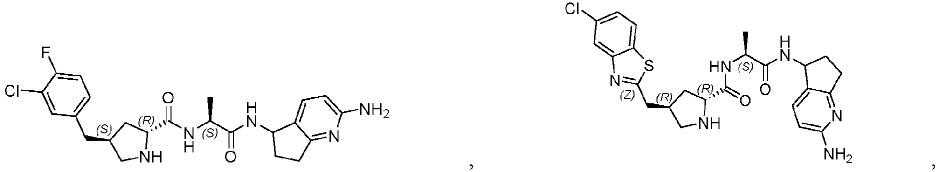

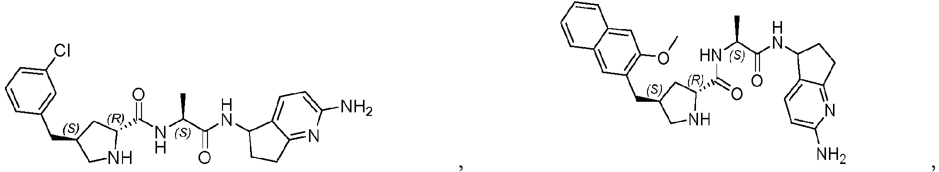

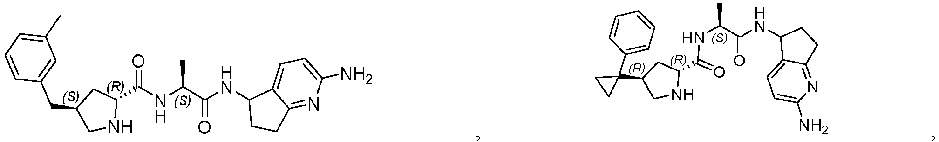

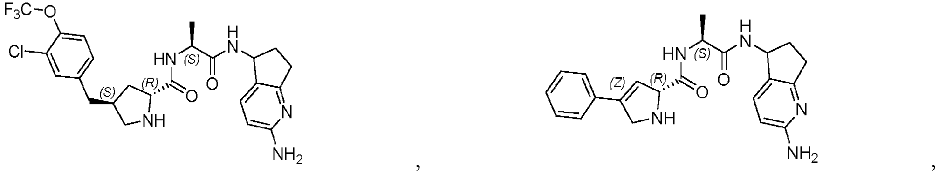

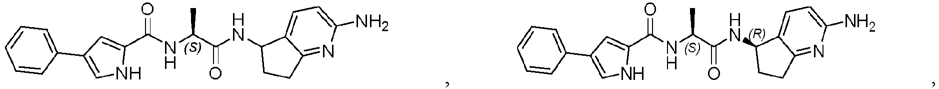

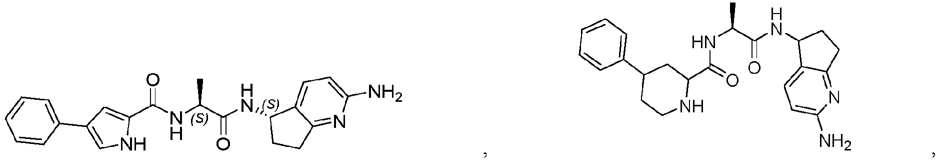

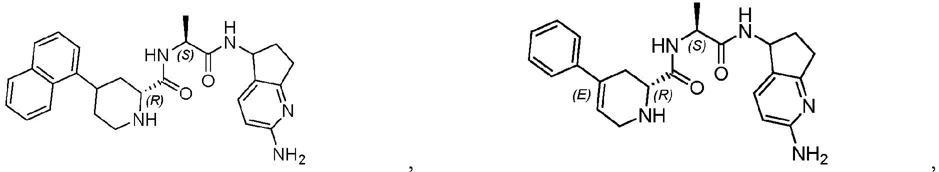

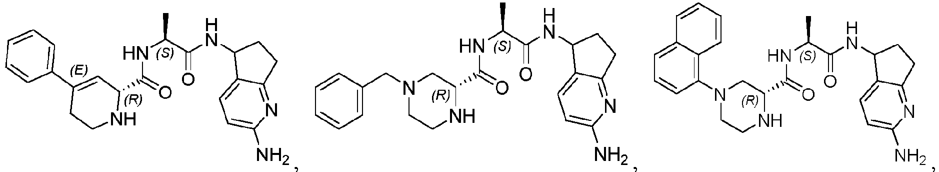

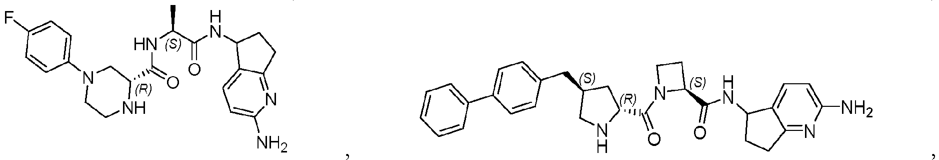

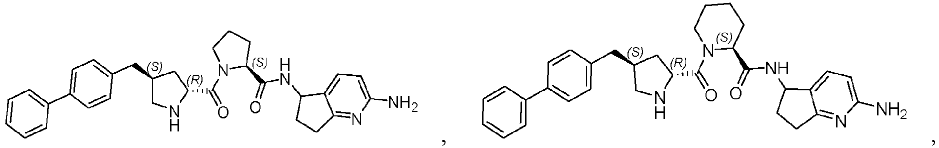

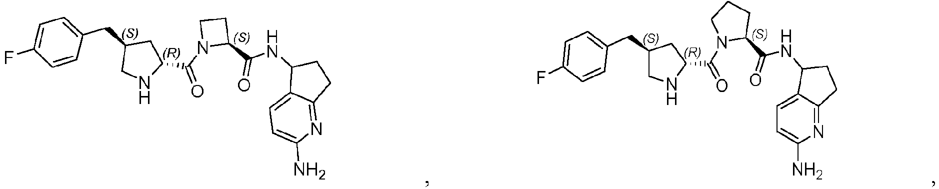

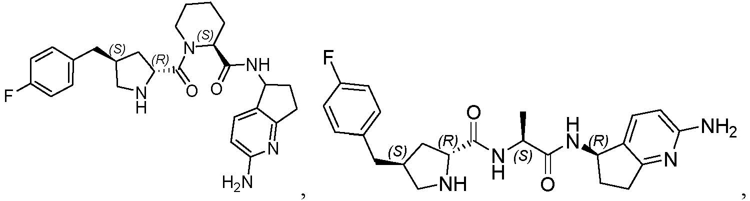

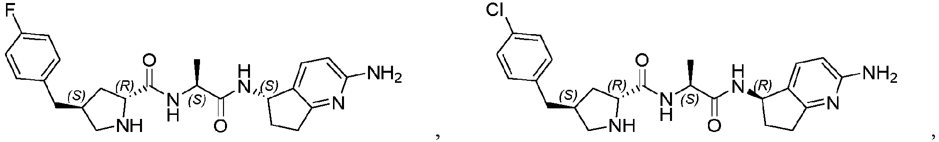

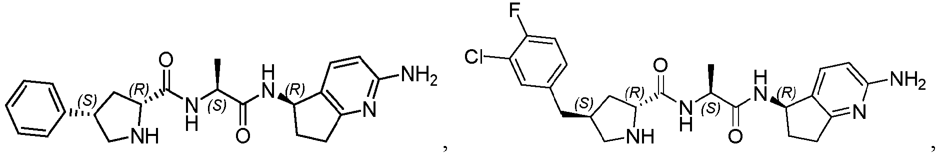

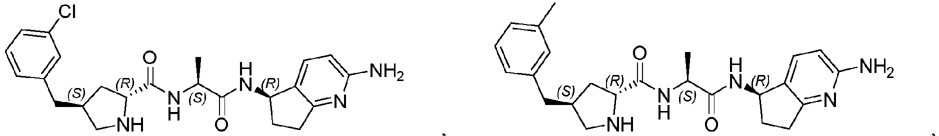

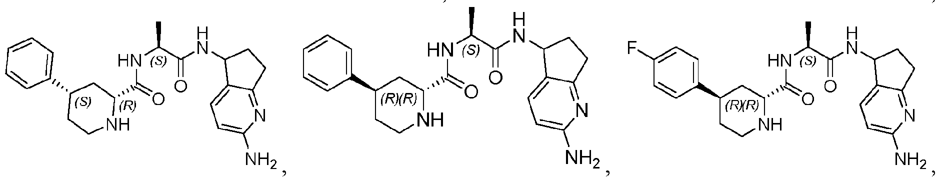

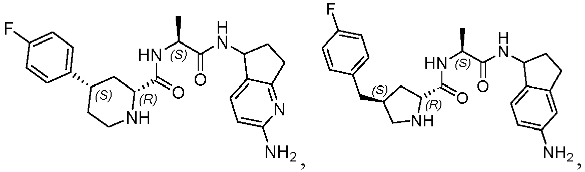

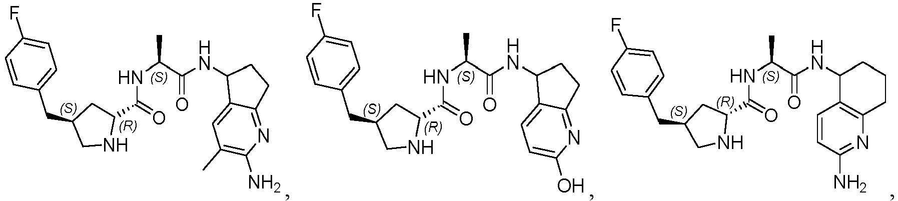

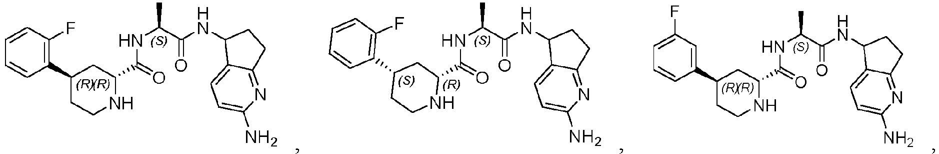

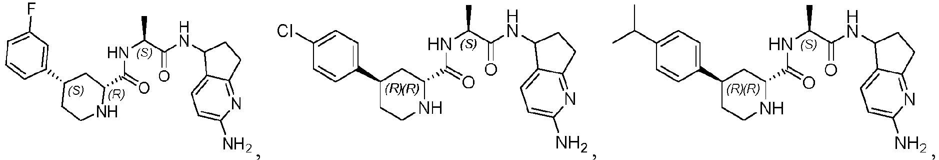

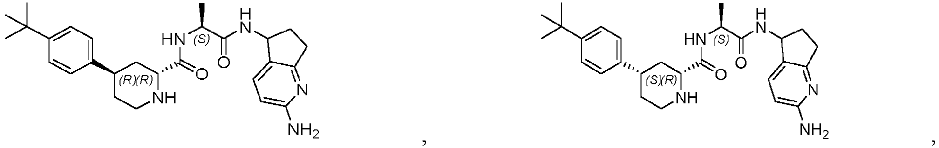

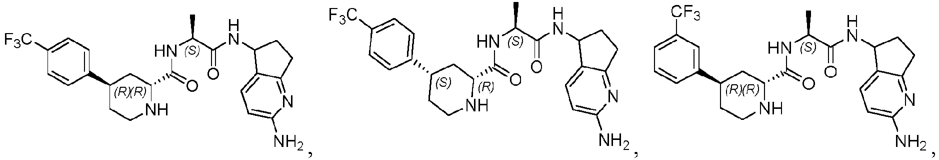

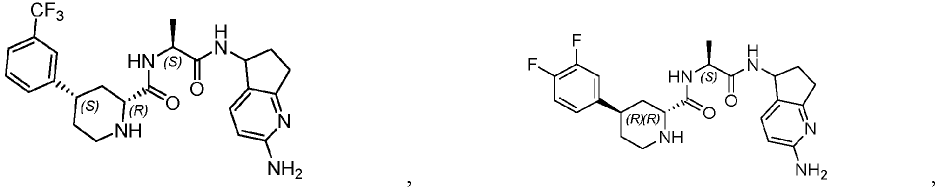

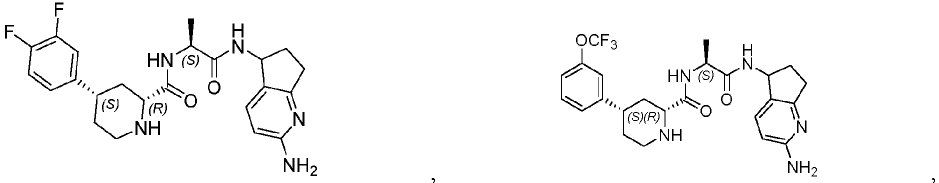

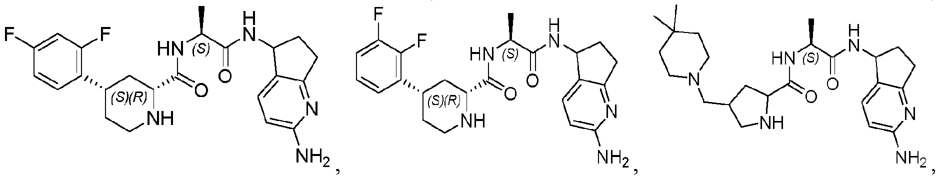

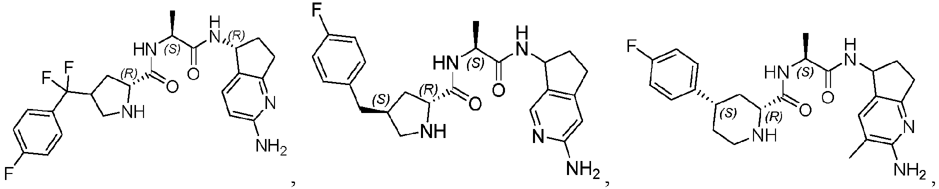

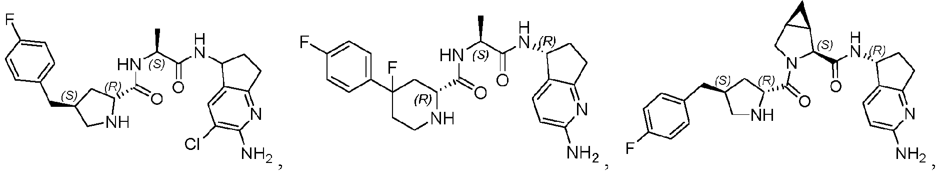

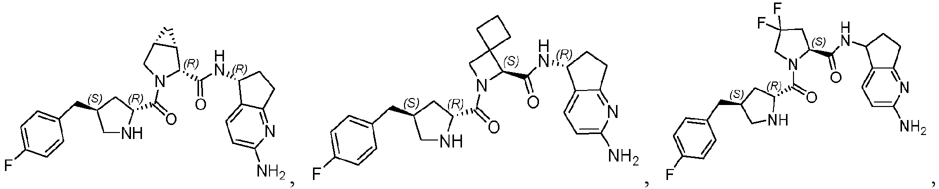

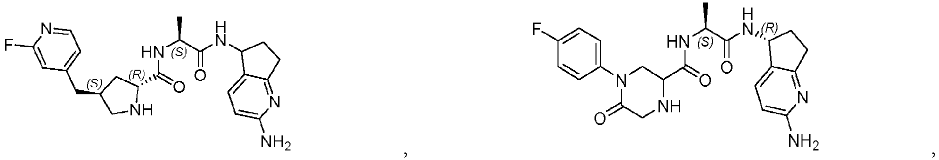

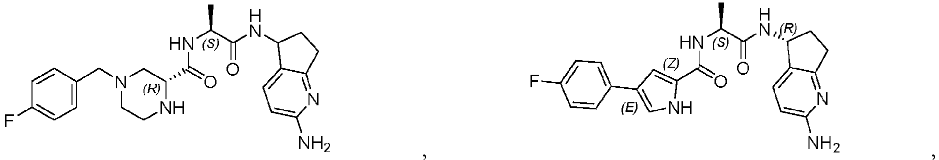

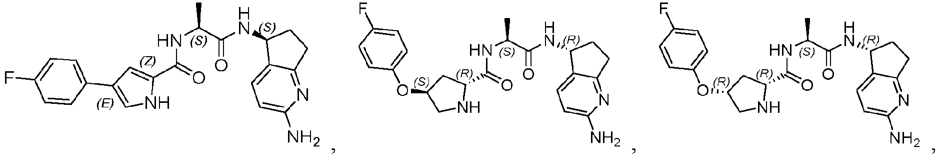

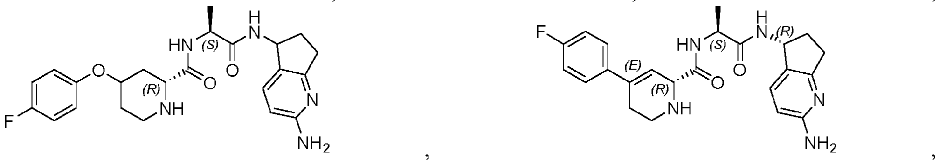

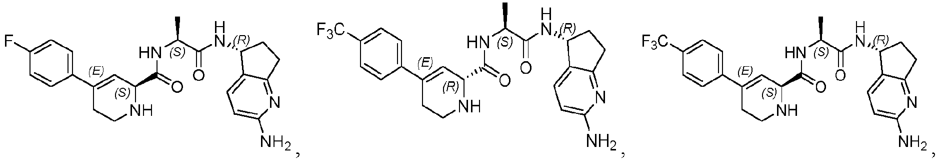

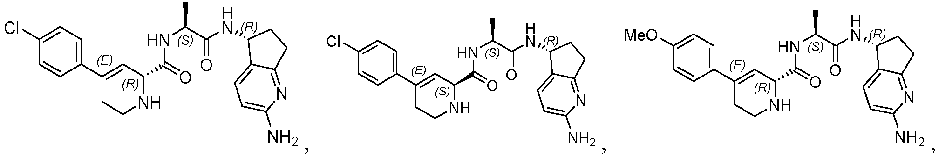

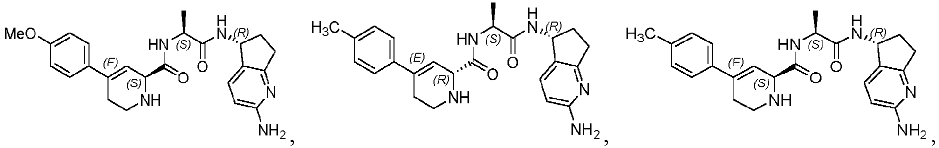

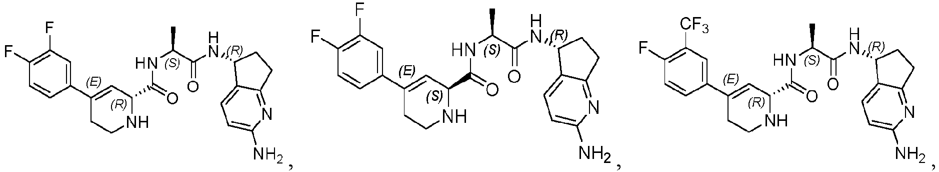

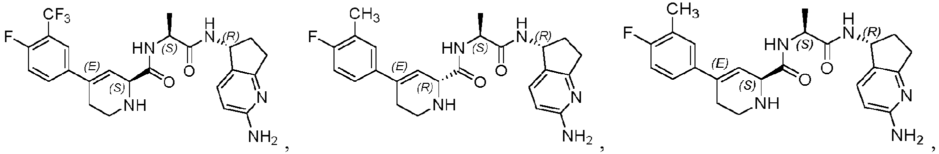

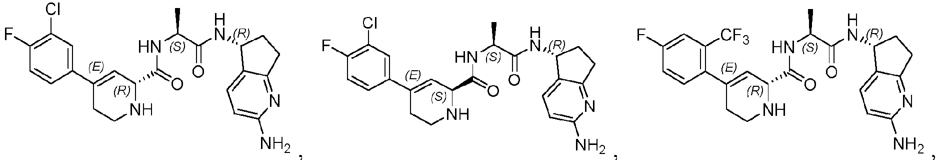

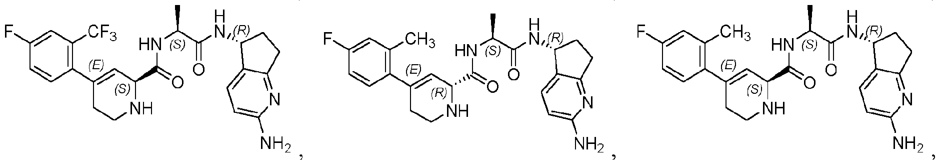

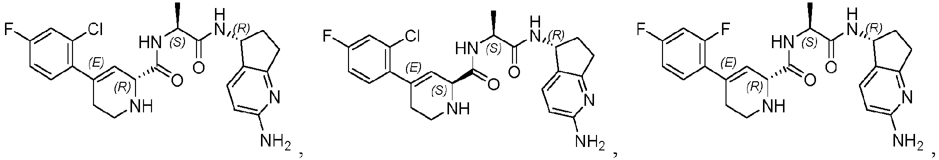

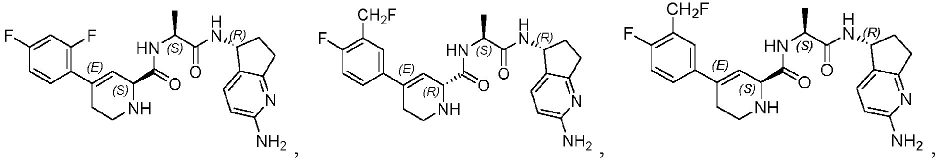

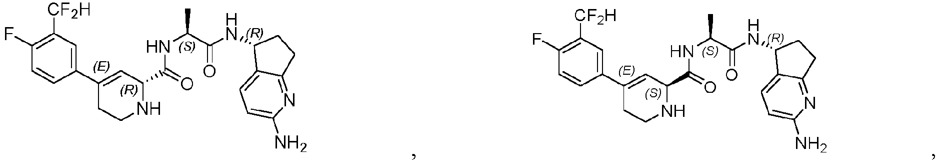

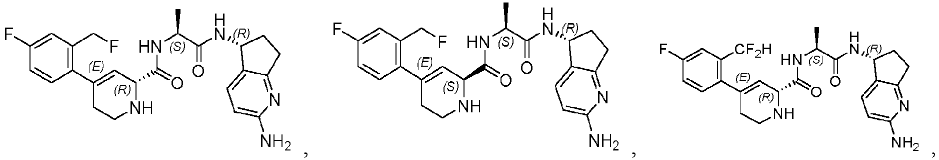

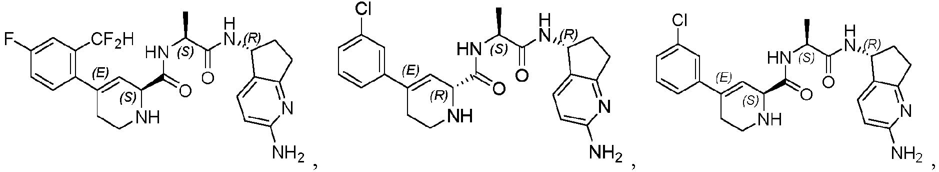

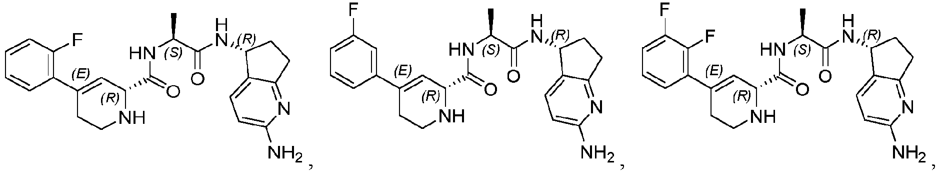

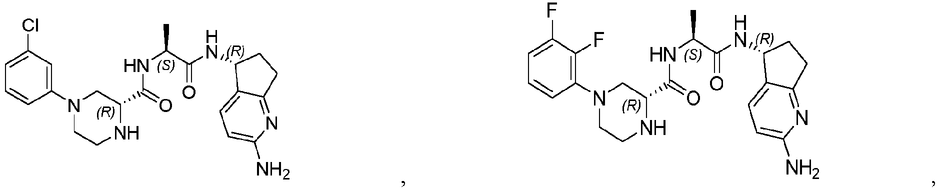

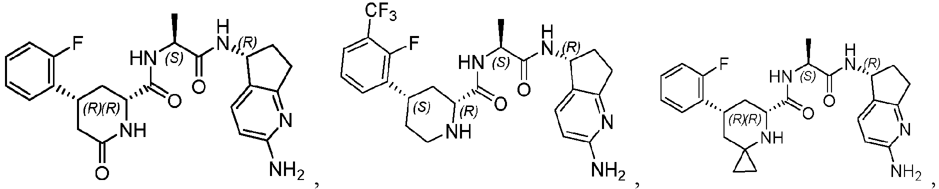

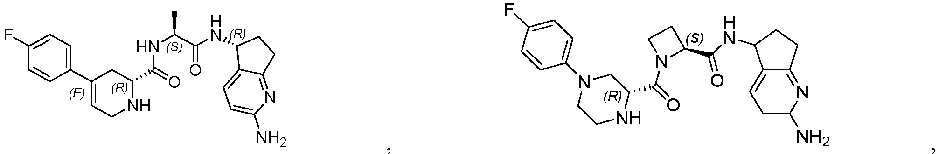

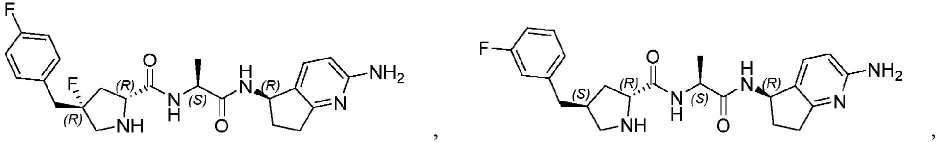

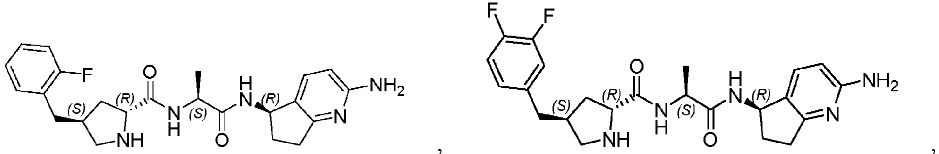

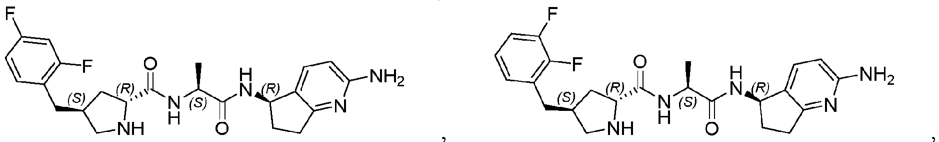

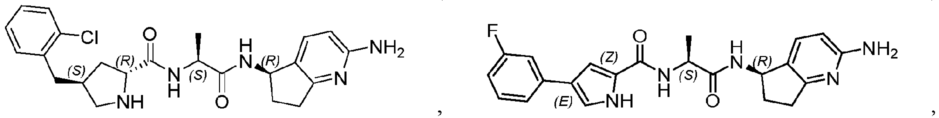

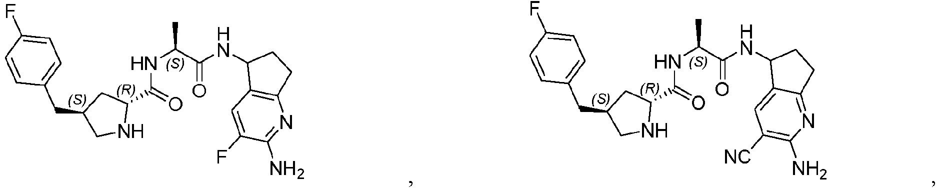

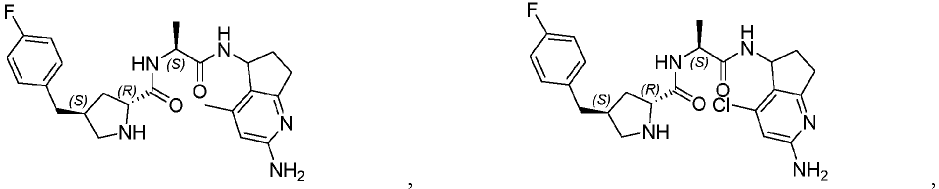

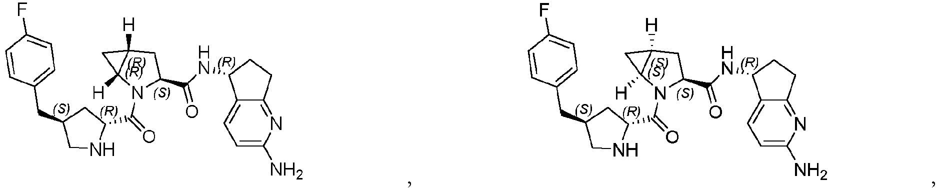

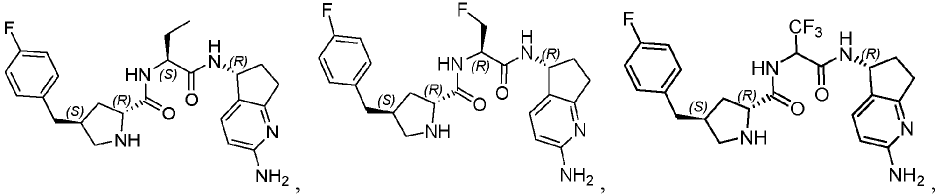

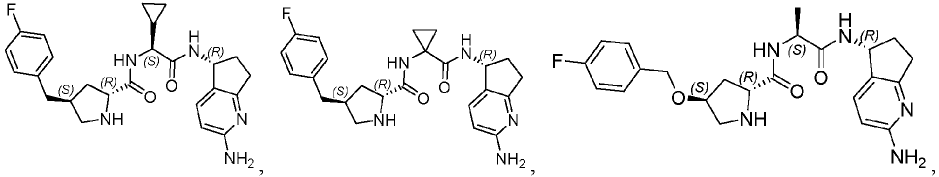

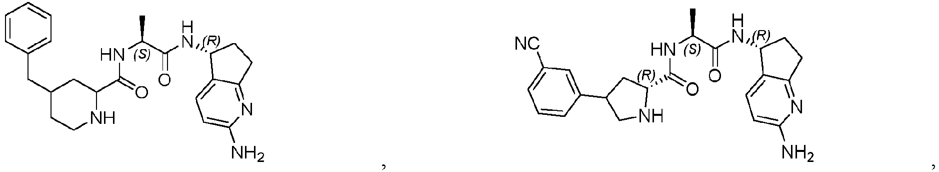

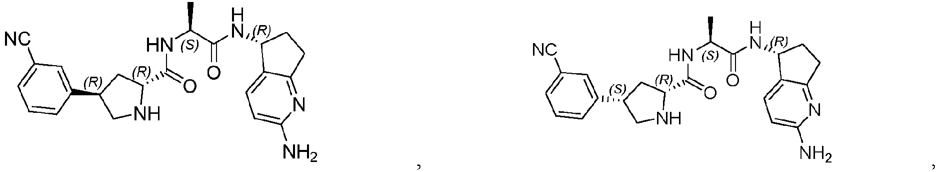

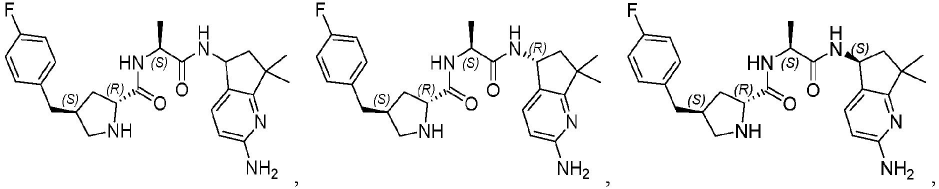

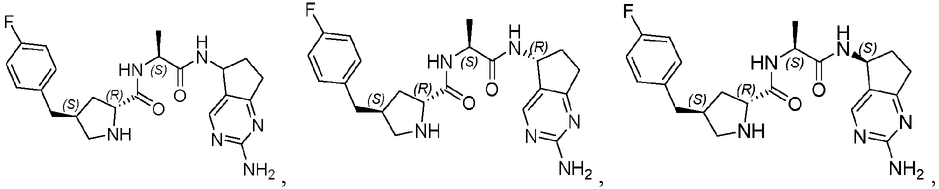

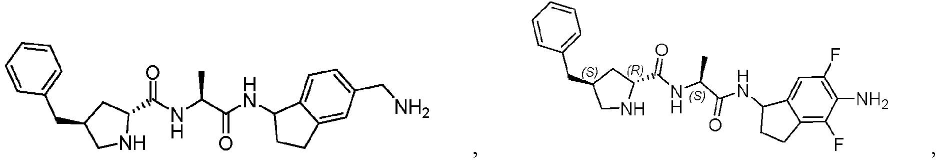

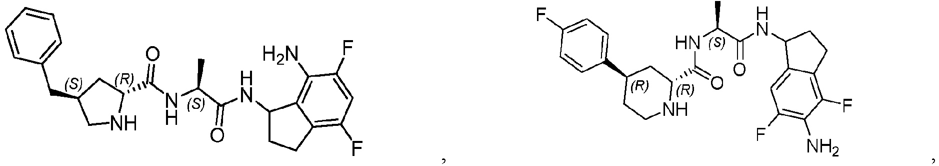

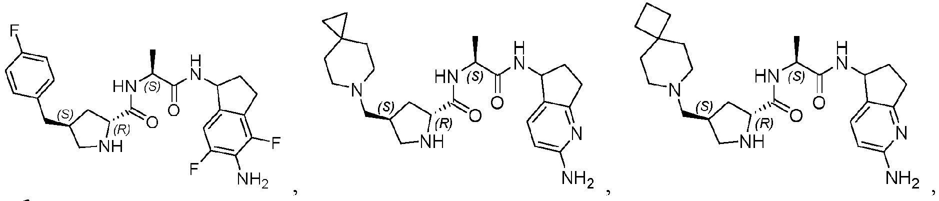

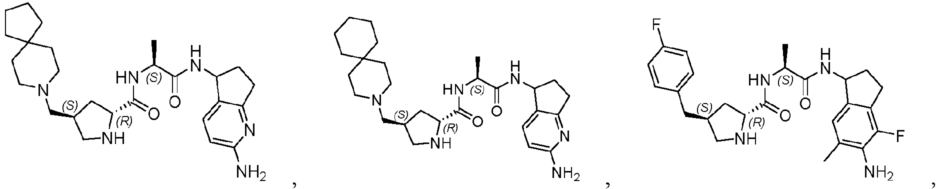

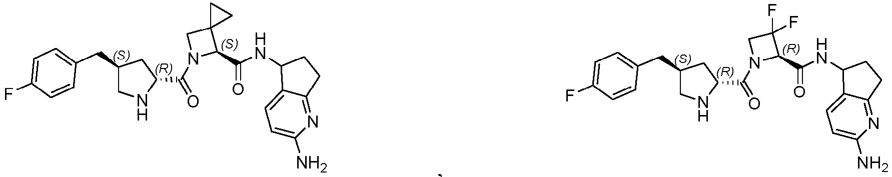

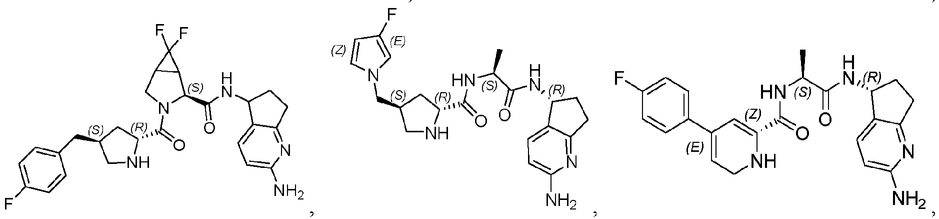

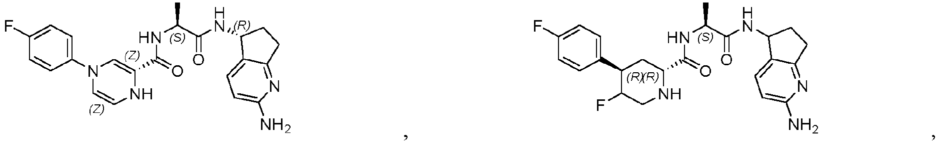

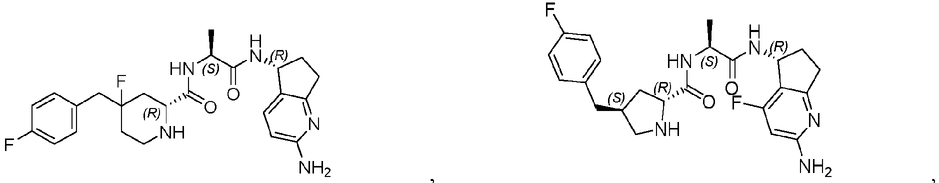

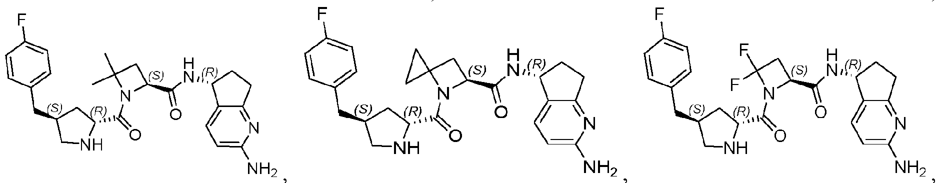

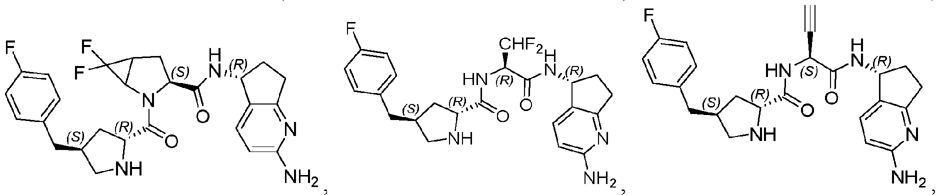

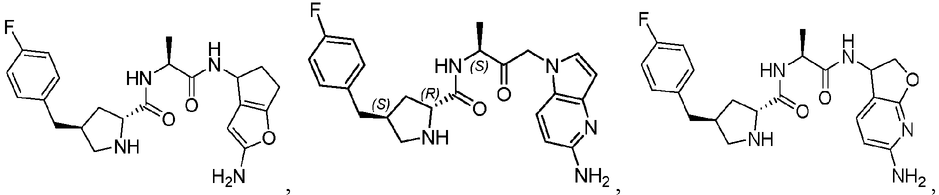

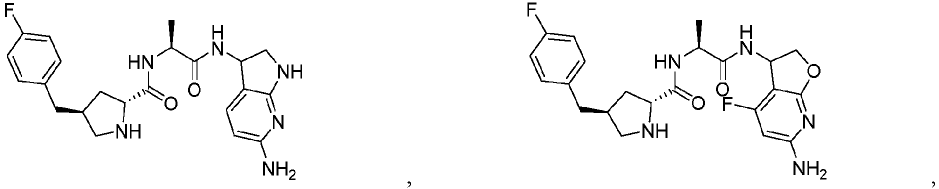

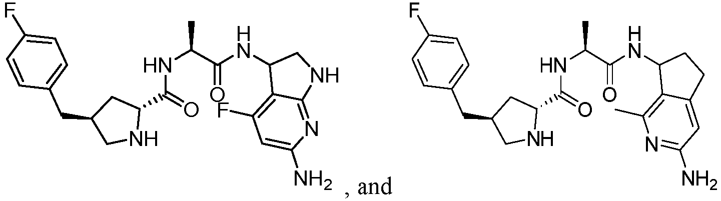

14. A preparation method for the compound according to any one of claims 1 to 12, wherein the preparation method comprises the following steps:
subjecting compound I-A' and compound I-B' to a condensation reaction to give compound I-1', and then deprotecting the compound 1-1' to give the compound represented by formula (I'); 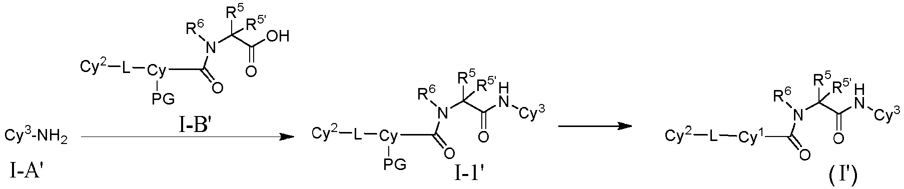 or
subjecting compound I-C' and compound I-D' to a condensation reaction to give compound I-1', and then deprotecting the compound I-1' to give the compound represented by formula (I'); 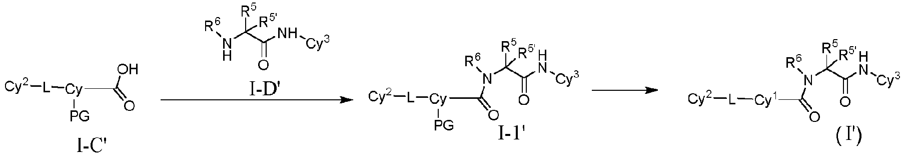
wherein R⁵, R^{5'}, R⁶, L, Cy¹, Cy², and Cy³ are each independently defined as in any one of claims 1 to 12; Cy is a group resulting from protection of an active group in Cy¹ by PG; PG is a protecting group, e.g., an amino protecting group such as tert-butyloxycarbonyl; preferably, the preparation method comprises the following steps:
subjecting compound I-A and compound I-B to a condensation reaction to give compound I-1, and then deprotecting the compound I-1 to give the compound represented by formula (I); 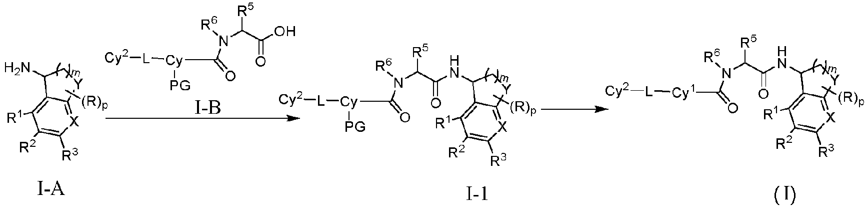 or
subjecting compound I-C and compound I-D to a condensation reaction to give compound I-1, and then deprotecting the compound I-1 to give the compound represented by formula (I); 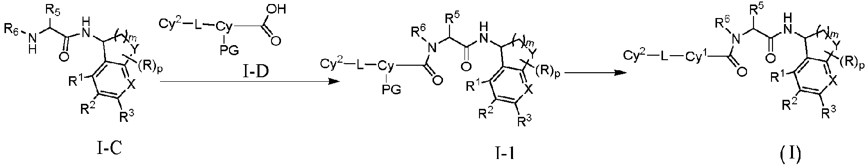
wherein R¹, R², R³, R⁵, R⁶, R, X, Y, L, Cy¹, Cy², m, and p are each independently defined as in any one of claims 1 to 12; Cy is a group resulting from protection of an active group in Cy¹ by PG; PG is a protecting group, e.g., an amino protecting group such as tert-butyloxycarbonyl.

15. Compounds represented by formulas (I-A)-(I-G), racemates, stereoisomers or hydrochlorides thereof: 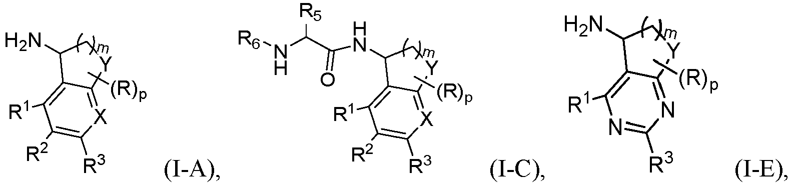 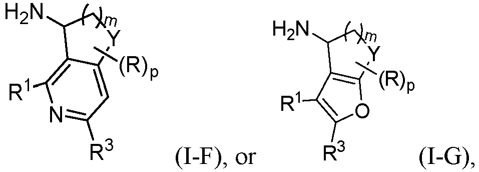
wherein, R¹, R², R³, R⁵, R⁶, R, X, Y, m, and p are each independently defined as in any one of claims 1 to 9;
preferably, the compounds represented by formulas (I-A)-(I-G) are selected from the following compounds:

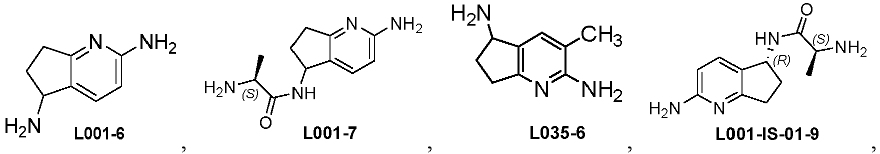

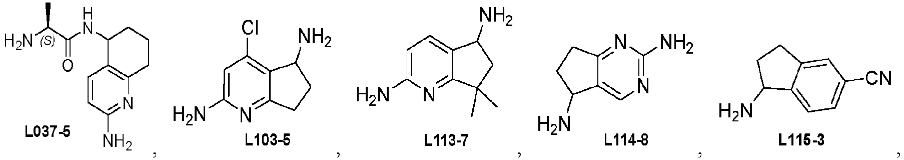

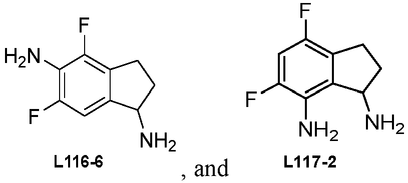

16. A pharmaceutical composition, comprising a therapeutically effective amount of at least one of the compounds, the racemates, the stereoisomers, the tautomers, the nitrogen oxides or the pharmaceutically acceptable salts thereof according to any one of claims 1 to 13.

17. Use of the compound, the racemate, the stereoisomer, the tautomer, the nitrogen oxide or the pharmaceutically acceptable salt thereof according to any one of claims 1 to 13 and the pharmaceutical composition according to claim 16 for the manufacture of a medicament, wherein,
preferably, the medicament is used for preventing and/or treating a disease associated with the lectin pathway of complement;
preferably, the medicament is a MASP-2 inhibitor;
preferably, the disease associated with the lectin pathway of complement is selected from thrombotic microangiopathy (TMA), a renal disease, respiratory distress syndrome caused by coronavirus infection, a hematologic system disease, a complication caused by type I or type II diabetes mellitus, a cardiovascular disease or disorder, an inflammatory gastrointestinal disorder, a pulmonary disorder, an ophthalmic disease or disorder, an ocular angiogenic disease or disorder, disseminated intravascular coagulation (DIC) or other complement-mediated coagulation disorders, angiogenesis-dependent cancer or tumor, a peripheral nervous system and/or central nervous system disorder or injury, sepsis or a disorder caused by sepsis, inflammatory response caused by tissue or organ transplantation, ischemia reperfusion injury, graft-versus-host disease, diffuse alveolar hemorrhage (DAH), venous occlusive disease (VOD), a skin disorder, an endocrine disorder, atherosclerosis, or an autoimmune disease;
the thrombotic microangiopathy is selected from thrombotic thrombocytopenic purpura (TTP), refractory TTP, Upshaw Schulman syndrome (USS), hemolytic uremic syndrome (HUS), atypical hemolytic uremic syndrome (aHUS), non-Factor H-dependent atypical hemolytic uremic syndrome, aHUS secondary to an infection, plasma therapy-resistant aHUS, TMA secondary to cancer, TMA secondary to chemotherapy, TMA secondary to transplantation, or TMA associated with hematopoietic stem cell transplantation (HSCT-TMA);
the renal disease is selected from IgA nephropathy, mesangioproliferative glomerulonephritis, membranous glomerulonephritis, membranoproliferative glomerulonephritis, acute post-infectious glomerulonephritis, C3 glomerulopathy, cryoglobulinemic glomerulonephritis, pauci-immune necrotizing crescentic glomerulonephritis, lupus nephritis, or Henoch-Schonlein purpura nephritis; the respiratory distress syndrome caused by coronavirus infection is selected from respiratory distress syndrome caused by influenza A virus, influenza B virus or influenza C virus and respiratory distress syndrome caused by SARS-COV-2, SARS-COV or MERS-COV;
the hematologic system disease is selected from paroxysmal nocturnal hemoglobinuria (PNH), cold agglutinin syndrome, disseminated intravascular coagulation, or autoimmune hemolytic anemia; the inflammatory gastrointestinal disorder is selected from pancreatitis, Crohn's disease, ulcerative colitis, irritable bowel syndrome, or inflammatory bowel disease (IBD);
the peripheral nervous system and/or central nervous system disorder or injury is selected from multiple sclerosis (MS), myasthenia gravis (MG), Huntington's disease (HD), amyotrophic lateral sclerosis (ALS), Guillain Barre syndrome, reperfusion following stroke, degenerative discs, cerebral trauma, Parkinson's disease (PD), Alzheimer's disease (AD), Miller-Fisher syndrome, cerebral trauma and/or hemorrhage, traumatic brain injury, demyelination, or meningitis;
the autoimmune disease is selected from systemic lupus erythematosus, rheumatoid arthritis, psoriatic arthritis, polymyositis, or psoriasis.
